# EUROPEAN PATENT SPECIFICATION

(11) **EP 4 182 299 B1**
(45) Date of publication and mention of the grant of the patent: **17.06.2026**
(21) Application number: 21745991.6
(22) Date of filing: 15.07.2021
(51) Int. Cl.: C07C 255/46, A61P 25/00, C07D 205/12, C07D 221/20, C07D 295/185, C07D 307/94, C07D 311/96, C07D 403/02, C07D 413/02, C07D 491/048, A61K 31/4747, A61K 31/277

(54) **CYCLIC CYANOENONE DERIVATIVES AS MODULATORS OF KEAP1**
ZYKLISCHE CYANOENON-DERIVATE ALS MODULATOREN VON KEAP1
DÉRIVÉS DE CYANOÉNONE CYCLIQUE EN TANT QUE MODULATEURS DE KEAP1

(30) Priority: 16.07.2020 EP 20186249
(43) Date of publication of application: 24.05.2023
(73) Proprietor: Merck Sharp & Dohme LLC, Rahway, New Jersey 07065 (US)
(72) Inventor: ALTMAN, Michael, Boston, Massachusetts 02115-5727 (US); CANDITO, David, A., Boston, Massachusetts 02115-5727 (US); CHRISTIAN, Alec, H., Boston, Massachusetts 02115-5727 (US); DI PIETRO, Ornella, London NC1 4AG (GB); LU, Min, Boston, Massachusetts 02115-5727 (US); LIU, Ping, Boston, Massachusetts 02115-5727 (US); MANSOOR, Umar, Faruk, Boston, Massachusetts 02115-5727 (US); MENNIE, Katrina, Marie, Boston, Massachusetts 02115-5727 (US); MUSACCHIO, Andrew, J., Boston, Massachusetts 02115-5727 (US); PALANI, Anandan, Boston, Massachusetts 02115-5727 (US); REUTERSHAN, Michael, H., Boston, Massachusetts 02115-5727 (US); SHAW, David, Matthew, London NC1 4AG (GB); ST-GALLAY, Stephen, London NC1 4AG (GB)
(74) Representative: Merck Sharp & Dohme LLC
(86) International application number: PCT/EP2021/069897
(87) International publication number: WO 2022/013408

(56) References cited:
- WO-A1-2013/040863
- WO-A1-2019/122265

## Description

The present invention relates to cyclic cyanoenone derivatives, to pharmaceutical compositions comprising the cyclic cyanoenone derivatives and to the cyclic cyanoenone derivatives for use in therapy as set out in the appended claims.

Nuclear factor, erythroid 2-related factor 2 (NRF2 (gene name: NFE2L2), is a basic leucine zipper (bZIP) transcription factor and a member of the Cap'n' Collar (CNC) family of transcription factors. It serves as a master regulator of the cellular response to oxidative stress. Oxidative stress occurs when cumulative damage by free radicals, generated in response to physiological stress, is no long adequately neutralised by antioxidants, leading to lipid peroxidation and cellular damage. Oxidative stress is implicated in various pathologies including neurodegenerative disease, cardiovascular disease, cancer, diabetes, most of which are age-related.

Under normal physiological conditions, Kelch-like ECH-associated protein 1 (KEAP1), a cytosolic actin-bound repressor protein, maintains low levels of NRF2 by promoting its CUL3-RBX1-mediated ubiquitination and subsequent proteasomal degradation. Under conditions of oxidative stress, either oxidation of sulfhydryl groups on specific cysteines in KEAP1 or phosphorylation of KEAP1 and/or NRF2 induces KEAP1 to release NRF2. Free of KEAP1, NRF2 is stabilized and translocates from the cytoplasm to the nucleus, through a bipartite nuclear localization signal, where it transactivates expression of over 200 cellular defence, repair and detoxification enzymes, e.g., Heme oxygenase 1, glutathione S-transferases, ferritin, and glutamate-cysteine ligase. NRF2 binds to an antioxidant response element (ARE) located in the promoter regulatory regions of these genes. This coordinated and rapid upregulation of multiple genes ensures a robust response to many types of cellular stress

In addition to its role in responding to oxidative and electrophilic stresses, the KEAP-NRF2 system has been demonstrated to regulate other key processes triggered in response to oxidative stress including: autophagy; mitophagy; mitochondrial dysfunction; inflammation; dysregulation of protein homeostasis leading to protein aggregation. Therefore, the KEAP1/NRF2 system appears to not only trigger the mechanisms to lower the oxidative stress but also to eliminate the oxidatively damaged proteins.

There is substantial evidence of impaired NRF2 activation in neurodegenerative diseases/ageing leading to dysfunction of redox homeostasis, mitochondrial dysfunction, autophagy. There is extensive pharmacology/gene-based validation supporting rescue of dysfunction of these mechanisms via NRF2 activation. Nrf2 nuclear transport has been shown to be compromised in a wide range of neurodegenerative diseases as demonstrated in control and FRDA patient cells under basal conditions and during oxidative stress (Abeti et al., Novel Nrf2-Inducer Prevents Mitochondrial Defects and Oxidative Stress in Friedreich's Ataxia Models. Front. Cell. Neurosci. 2018, 12:188-198).

Over-expression of NRF2, inhibition of KEAP1 and small molecule pharmacological activators of NRF2 have shown protective effects in a wide range of animal models of neurodegenerative diseases. Nrf2-deficient cells and Nrf2 knockout mice are significantly more vulnerable to malonate and 3NP and demonstrate increased antioxidant response element (ARE)-regulated transcription mediated by astrocytes. ARE pre-activation by means of intrastriatal transplantation of Nrf2-overexpressing astrocytes before lesioning conferred dramatic protection against mitochondrial complex II inhibition (Calkins et al., Protection from mitochondrial complex II inhibition in vitro and in vivo by Nrf2-mediated transcription. Proc. Natl. Acad. Sci. U.S.A. 2005, 102(1):244-249). Dopaminergic neurons from Nrf2 -/- deficient mice are more vulnerable to neurotoxicity of MPTP (Burton et al., In vivo modulation of the Parkinsonian phenotype by Nrf2. Neurotoxicology 2006, 27(6):1094-1100; Chen et al., Nrf2-mediated Neuroprotection in the MPTP Mouse Model of Parkinson's Disease: Critical Role for the Astrocyte. Proc. Natl. Acad. Sci. U.S.A., 2009, 106(8): 2933-2938) and 6-OHDA (Jakel *et al*., 2007) mouse models. Astrocytic overexpression of Nrf2 protects against MPTP toxicity (Chen et al. Nrf2-mediated Neuroprotection in the MPTP Mouse Model of Parkinson's Disease: Critical Role for the Astrocyte. Proc. Natl. Acad. Sci. U.S.A., 2009, 106(8): 2933-2938). Overexpression of Nrf2 reduces a-synuclein accumulation in the SN region (Gan et al., Astrocyte-specific overexpression of Nrf2 delays motor pathology and synuclein aggregation throughout the CNS in the alpha-synuclein mutant (A53T) mouse model. J Neurosci. 2012, 32(49):17775-17778). Nrf2 deficiency leads to increased aggregation of a-synuclein, increased inflammation and neuronal death in a rAAV-a-synuclein model (Lastres-Becker et al. Repurposing the NRF2 Activator Dimethyl Fumarate as Therapy Against Synucleinopathy in Parkinson's Disease. Antioxid Redox Signal. 2016, 25(2):61-77). Both overexpression of Nrf2 or its DNA-binding dimerization partner, Maf-S and RNAi downregulation of Keap1 restore the locomotor activity of a-synuclein-expressing Drosophila. a-synuclein-induced dopaminergic neuron loss is suppressed by Maf-S overexpression or keap1 heterozygosity (Barone et al. Genetic activation of Nrf2 signalling is sufficient to ameliorate neurodegenerative phenotypes in a Drosophila model of Parkinson's disease. Dis Model Mech. 2011, 4(5):701-707. An AT-Nrf2 model (model of amyloidopathy and tauopathy) exhibits increased markers of oxidative stress/neuroinflammation in brain, deficits in LTP and spatial memory (Rojo et al., NRF2 deficiency replicates transcriptomic changes in Alzheimer's patients and worsens APP and TAU pathology. Redox Biol. 2017, 13:444-451). Intrahippocampal injection of Nrf-2 expressing lentiviral vector improves spatial learning and reduces astrocytosis in an APPxPS-1 Tg model of AD (Kanninen et al. Intrahippocampal injection of a lentiviral vector expressing Nrf2 improves spatial learning in a mouse model of Alzheimer's disease. Proc Natl Acad Sci USA. 2009, 106(38):16505-16510).

Tecfidera^{®} (dimethyl fumarate - active ingredient monomethyl fumarate) is a weak activator of Nrf2 that is approved for the treatment of relapsing-remitting multiple sclerosis. The Nrf2 activator Omaveloxolone (RTA- met its primary endpoint of change in the modified Friedrich's Ataxia Rating Scale (mFARS) relative to placebo after 48 weeks of treatment in a Phase II trial for the treatment of Friedreich's ataxia.

Small molecules that promote NRF2 activation, either by blocking the NRF2-KEAP1 interaction (protein-protein inhibitors) or mimicking the oxidation of sulfhydryl groups on specific cysteines on KEAP1 (electrophiles) are predicted to target multiple pathways contributing to neuronal dysfunction and loss across a broad range of neurodegenerative disorders including: Alzheimer's disease; amyotrophic lateral sclerosis; Down's syndrome; Friedreich's ataxia; frontotemporal dementia; Huntington's disease; Parkinson's disease (reviewed in Johnson and Johnson, Nrf2--a therapeutic target for the treatment of neurodegenerative diseases. Free Radic Biol Med. 2015, 88(Pt B):253-267; Li et al., Reasonably activating Nrf2: A long-term, effective and controllable strategy for neurodegenerative diseases. Eur. J. Med. Chem. 2020, 185:111862-111880). Use of Nrf2 activators is predicted to delay the onset, modify disease progression and/or reduce the symptoms associated with these diseases.

Small molecules that promote NRF2 activation are known in the art. See, for example, WO 2019/104030 and WO 2019/122265. In spite of the availability of these compounds, there remains a need for further NRF2 activators which are safe and effective.

In one aspect, the present invention provides a compound according to Formula (I): 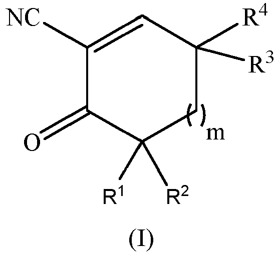
or a pharmaceutically acceptable salt or solvate thereof,
wherein:
   R¹ and R² are each independently methyl;
   R³ and R⁴ together with the carbon to which they are bonded form a cyclobutyl, cyclopentyl, or cyclohexyl said cyclobutyl, cyclopentyl and cyclohexyl being optionally substituted with 1-4 substituents R¹²; or
   R³ and R⁴ together with the carbon to which they are bonded form an azetidine, a piperidine, a pyrrolidine, a morpholine, a tetrahydrofuran or a tetrahydropyran, said azetidine, piperidine, pyrrolidine, morpholine, tetrahydrofuran and tetrahydropyran being optionally substituted with 1-4 substituents R¹²;
   each R¹² is independently selected from:
      (1) -C₁₋₆alkyl,
      (2) halogen,
      (3) oxo,
      (4) -(CH₂)ₚC₃₋₆cycloalkyl,
      (5) -(CH₂)ₚC₆₋₁₀aryl,
      (6) -(CH₂)ₚhet, wherein het denotes a 3- to 9-membered saturated or unsaturated heterocyclic ring comprising 1 to 4 heteroatoms independently selected from O, S and N
      (7) -(CH₂)ₚ-OR¹⁴,
      (8) -(CH₂)ₚ-NR¹⁵R¹⁶,
      (9) -(CH₂)ₚ-C(O)NR¹⁷R¹⁸,
      (10) -(CH₂)ₚ-S(O)ₙNR¹⁷R¹⁸
      (11) -(CH₂)ₚ-NR¹⁷C(O)R¹⁸,
      (12) -(CH₂)ₚ-NR¹⁷SO₂R¹⁸,
      (13) -(CH₂)ₚ-C(O)R¹⁹,
      (14) -(CH₂)ₚ-S(O)ₙR¹⁹
      (15) -(CH₂)ₚ-C(O)OR²⁰ and
      (16) -(CH₂)ₚ-CN;
   wherein said C₁₋₆alkyl, C₃₋₆cycloalkyl C₆₋₁₀aryl and 3- to 9-membered heterocyclic ring are each independently substituted with one or more substituents selected from halogen, hydroxyl, C₁₋₆alkyl, -OC₁₋₆alkyl and -SC₁₋₆alkyl and wherein said -C₁₋₆alkyl, -C₃₋₆cycloalkyl, -OC₁₋₆alkyl and -SC₁₋₆alkyl are optionally substituted with one or more halogens or one or more hydroxyls;
   or two R¹² substituents on adjacent atoms of the cyclobutyl, cyclopentyl, cyclohexyl, azetidine, piperidine, pyrrolidine, morpholine, tetrahydrofuran or tetrahydropyran ring together with the ring atoms to which they are bonded form a fused bicyclic ring system which is optionally substituted with one or more substituents R²¹;
   R¹⁴ is H, -C₁₋₆alkyl or -C₃₋₈cycloalkyl, said -C₁₋₆alkyl and -C₃₋₈cycloalkyl being optionally substituted with one or more halogens;
   R¹⁵ is H or -C₁₋₆alkyl optionally substituted with one or more halogens;
   R¹⁶ is H, C₁₋₆alkyl, C₃₋₆cycloalkyl, C₆₋₁₀aryl or a 5- to 8-membered heteroaryl comprising 1 to 3 heteroatoms independently selected from O, S and N, wherein said C₁₋₆alkyl, C₃₋₆cycloalkyl C₆₋₁₀aryl and 5- to 8-membered heteroaryl are each independently optionally substituted with one or more substituents selected from halogen, hydroxyl, C₁₋₆alkyl and -OC₁₋6alkyl;
   R¹⁷ is H or C₁₋₆alkyl optionally substituted with one or more halogens;
   R¹⁸, R¹⁹and R²⁰ are each independently H, -C₁₋₆alkyl, -C₂₋₆alkenyl, -C₂₋₆alkynyl, -C₃₋₆cycloalkyl -C₆₋₁₂aryl or a 5- to 10-membered heteroaryl comprising 1 to 3 heteroatoms independently selected from O, S and N, wherein said -C₁₋₆alkyl, -C₂₋₆alkenyl, -C₂₋₆alkynyl, - C₃₋₆cycloalkyl -C₆₋₁₂aryl and 5- to 10-membered heteroaryl are each optionally independently substituted with one or more substituents R²⁷;
   each R²¹ is independently selected from H, halogen, hydroxyl, -C₁₋₆alkyl and -OC₁₋₆alkyl, wherein said -C₁₋₆alkyl and -OC₁₋₆alkyl are each optionally independently substituted with one or more substituents selected from halogen, hydroxyl and -OC₁₋₆alkyl,
   R²⁷ is halogen, hydroxyl, nitrile, -C₁₋₆alkyl, -O-C₁₋₆alkyl, -S-C₁₋₆alkyl, C(O)NHC₁₋₆alkyl, -C₃₋₆cycloalkyl, -C₆₋₁₀aryl, -O-C₆₋₁₀aryl, -O-C₁₋₂alkylC₆₋₁₀aryl or a 5- to 8-membered heteroaryl comprising 1 to 3 heteroatoms independently selected from O, S and N, wherein each occurrence of said -C₁₋₆alkyl, -C₃₋₆cycloalkyl -C₆₋₁₀aryl and 5- to 8-membered heteroaryl are independently substituted with one or more substituents selected from halogen, hydroxyl, nitrile, -C₁₋₆alkyl, -OC₁₋₆alkyl, and -N(C₁₋₆alkyl)₂ wherein said -C₁₋₆alkyl and -OC₁₋₆alkyl are each independently substituted with one or more halogens;
   m is 0, 1 or 2;
   each n is independently 0, 1 or 2 and
   each p is independently 0, 1 or 2.

In a further aspect, the present invention provides a pharmaceutical composition comprising a compound according to Formula (I), or a pharmaceutically acceptable salt or solvate thereof, and a pharmaceutically acceptable carrier or diluent.

The compounds of Formula (I) or pharmaceutically acceptable salts or solvates thereof are useful in therapy. In particular, the compounds of Formula (I) or pharmaceutically acceptable salts or solvates thereof are useful in the treatment or prevention of diseases mediated by Nrf2 activation in a patient. As such, compounds of Formula (I) or pharmaceutically acceptable salts or solvates thereof can be useful, for example, for treating or preventing neurodegenerative diseases, inflammatory diseases, autoimmune diseases or cancers in a patient.

In a further aspect, therefore, the present invention provides a compound of Formula (I) or a pharmaceutically acceptable salt or solvate thereof for use in the treatment or prevention of a disease mediated by Nrf2 activation in a patient. The present invention further provides a compound of Formula (I) or a pharmaceutically acceptable salt or solvate thereof for use in treating or preventing a neurological disease, an inflammatory disease, an autoimmune disease or a cancer in a patient. The present invention further provides a compound of Formula (I) or a pharmaceutically acceptable salt or solvate thereof for use in treating or preventing a neurological disease in a patient. The present invention further provides a compound of Formula (I) or a pharmaceutically acceptable salt or solvate thereof for use in treating a neurodegenerative disease selected from Freidreich's ataxia, amyotrophic lateral sclerosis, stroke, Alzheimer's disease, Parkinson's disease, Huntington's disease, peripheral neuropathy and hearing loss in a patient.

The present invention further provides a combination comprising a compound of Formula (I) or a pharmaceutically acceptable salt or solvate thereof and one, two, three or more other therapeutic agents.

The terms used herein have their ordinary meaning and the meaning of such terms is independent at each occurrence thereof. That notwithstanding and except where stated otherwise, the following definitions apply throughout the specification and claims. Chemical names, common names, and chemical structures may be used interchangeably to describe the same structure. If a chemical compound is referred to using both a chemical structure and a chemical name and an ambiguity exists between the structure and the name, the structure predominates. These definitions apply regardless of whether a term is used by itself or in combination with other terms, unless otherwise indicated. Hence, the definition of "alkyl" applies to "alkyl" as well as the "alkyl" portions of "hydroxyalkyl," "haloalkyl," "-Oalkyl," *etc.*

As used herein, and throughout this disclosure, the following terms, unless otherwise indicated, shall be understood to have the following meanings:
The term "alkyl," as used herein, refers to an aliphatic hydrocarbon group having one of its hydrogen atoms replaced with a bond. An alkyl group may be straight or branched. In one embodiment, an alkyl group contains from about 1 to about 6 carbon atoms (C₁₋₆alkyl). Examples of alkyl groups include methyl, ethyl, n-propyl, isopropyl, n-butyl, sec-butyl, isobutyl, tert-butyl, n-pentyl, neopentyl, isopentyl, n-hexyl, isohexyl and neohexyl. In one embodiment, an alkyl group is linear. In another embodiment, an alkyl group is branched. Unless otherwise indicated, an alkyl group is unsubstituted.

The term " OC₁₋₆alkyl " as used herein, refers to a group where the term "C₁₋₆alkyl" is as defined above.

The term "alkenyl," as used herein, refers to an aliphatic hydrocarbon group containing at least one carbon-carbon double bond and having one of its hydrogen atoms replaced with a bond. An alkenyl group may be straight or branched. In one embodiment, an alkenyl group contains from 2 to 6 carbon atoms (C₂-C₆alkenyl). Examples of alkenyl groups include ethenyl, propenyl, n-butenyl, 3-methylbut-2-enyl, n-pentenyl, octenyl and decenyl. Unless otherwise indicated, an alkenyl group is unsubstituted.

The term "alkynyl," as used herein, refers to an aliphatic hydrocarbon group containing at least one carbon-carbon triple bond and having one of its hydrogen atoms replaced with a bond. An alkynyl group may be straight or branched. In one embodiment, an alkynyl group contains from 2 to 6 carbon atoms (C₂-C₆alkynyl). In another embodiment, an alkynyl group contains from 2 to 3 carbon atoms (C₂-C₃alkynyl). Examples of alkynyl groups include ethynyl, propynyl, 2-butynyl and 3-methylbutynyl. Unless otherwise indicated, an alkynyl group is unsubstituted.

The term "aryl," as used herein, refers to an aromatic monocyclic or multicyclic ring system. In one embodiment, an aryl group contains from 6 to 12 carbon atoms (C₆₋₁₂aryl). In another embodiment, an aryl group contains from 6 to 10 carbon atoms (C₆₋₁₀aryl). In one embodiment, an aryl group can be optionally fused to a cycloalkyl or cycloalkanoyl group. Examples of aryl groups include phenyl and naphthyl. In one embodiment, an aryl group is phenyl. The term "aryloxy" as used herein, refers to a group having the formula -Oaryl, where the term "aryl" is defined above herein.

The term "cycloalkyl," as used herein, refers to a non-aromatic mono- or multicyclic ring system. In one embodiment, a cycloalkyl contains from 3 to 12 ring carbon atoms (C3-12cycloalkyl). In another embodiment, a cycloalkyl contains from 3 to 8 ring carbon atoms (C₃₋₈cycloalkyl). In another embodiment, a cycloalkyl contains from 3 to 6 ring atoms (C₃₋₆cycloalkyl). Examples of monocyclic cycloalkyls include cyclopropyl, cyclobutyl, cyclopentyl, cyclohexyl, cycloheptyl and cyclooctyl. Examples of multicyclic cycloalkyls include 1-decalinyl, norbornyl and adamantly. A ring carbon atom of a cycloalkyl group may be functionalized as a carbonyl group. An illustrative example of such a cycloalkyl group (also referred to herein as a "cycloalkanoyl" group) includes cyclobutanoyl:

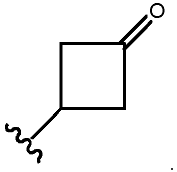

The term "halogen," as used herein, means -F, -Cl, -Br or -I.

The term "haloalkyl," as used herein, refers to an alkyl group as defined above, wherein one or more of the alkyl group's hydrogen atoms have been replaced with a halogen. In one embodiment, a haloalkyl group has from 1 to 6 carbon atoms (C₁₋₆haloalkyl). In another embodiment, a haloalkyl group is substituted with from 1 to 3 F atoms. Examples of haloalkyl groups include -CH₂F, -CHF₂, -CF₃, -CH₂Cl and -CCl₃.

The term "hydroxyalkyl," as used herein, refers to an alkyl group as defined above, wherein one or more of the alkyl group's hydrogen atoms have been replaced with an - OH group. In one embodiment, a hydroxyalkyl group has from 1 to 6 carbon atoms (C₁₋₆hydroxyoalkyl). Examples of hydroxyalkyl groups include -CH₂OH, -CH₂CH₂OH, - CH₂CH₂CH₂OH and -CH₂CH(OH)CH₃.

The term "5- to 12-membered heteroaryl ring comprising 1 to 4 heteroatoms independently selected from O, S and N" as used herein, refers to an aromatic monocyclic or bicyclic ring system comprising from 5 to 12 ring atoms, wherein from 1 to 4 of the ring atoms is independently O, S or N and the remaining ring atoms are carbon atoms. In one embodiment, the heteroaryl ring is a 5- to 8-membered heteroaryl ring comprising 1 to 3 heteroatoms independently selected from O, S and N. The heteroaryl group is joined via a ring carbon atom, and any nitrogen atom of a heteroaryl can be optionally oxidized to the corresponding N-oxide. Examples of 5 or 6-membered monocyclic heteroaryls include pyridyl, pyrazinyl, furanyl, thienyl, pyrimidinyl, pyridone (including N-substituted pyridones), isoxazolyl, isothiazolyl, oxazolyl, oxadiazolyl, thiazolyl, pyrazolyl, furazanyl, pyrrolyl, triazolyl, 1,2,4-thiadiazolyl, pyrazinyl, pyridazinyl, imidazolyl, 1,2,4-triazinyl and the like, and all isomeric forms thereof. In another embodiment, the heteroaryl is an aromatic bicyclic ring system comprising 9 or 10 ring atoms, wherein from 1 to 3 of the ring atoms is independently O, N or S and the remaining ring atoms are carbon atoms. A 9 or 10-membered bicyclic heteroaryl group is joined via a ring carbon atom, and any nitrogen atom of a heteroaryl can be optionally oxidized to the corresponding N-oxide. Examples of 9 or 10-membered bicyclic heteroaryls include imidazo[1,2-a]pyridinyl, imidazo[2,1-b]thiazolyl, benzofurazanyl, indolyl, azaindolyl, benzimidazolyl, benzothienyl, quinolinyl, benzimidazolyl, quinazolinyl, pyrrolopyridyl, imidazopyridyl, isoquinolinyl, benzoazaindolyl, benzothiazolyl, and the like, and all isomeric forms thereof.

The term "3- to 11-membered saturated or unsaturated heterocyclic ring comprising one to four heteroatoms independently selected from O, S and N, wherein the S is optionally oxidized to SO or SO₂," as used herein, refers to a non-aromatic monocyclic or multicyclic saturated or partially unsaturated ring system comprising 3 to 11 ring atoms, wherein from 1 to 4 of the ring atoms are independently O, S, or N, and the remainder of the ring atoms are carbon atoms. A heterocyclic ring can be joined via a ring carbon or ring nitrogen atom. In one embodiment, a heterocyclic ring is monocyclic and has from 3 to 9 ring atoms. In another embodiment, a heterocyclic ring is monocyclic has from 3 to 6 ring atoms. In another embodiment, a heterocyclic ring is bicyclic and has from 7 to 11 ring atoms. In still another embodiment, a heterocyclic ring is monocyclic and has 5 or 6 ring atoms. In one embodiment, a heterocyclic ring is monocyclic. In another embodiment, a heterocyclic ring is bicyclic. There are no adjacent oxygen and/or sulfur atoms present in the ring system. Any -NH group in a heterocyclic ring may exist protected such as, for example, as an -N(BOC), -N(Cbz), -N(Tos) group and the like; such protected heterocyclic groups are considered part of this invention. The heterocyclic ring may also encompass a heterocyclic ring, as defined above, which is fused to an aryl (e.g., benzene) or heteroaryl ring. The nitrogen or sulfur atom of the heterocyclic ring can be optionally oxidized to the corresponding N-oxide, S-oxide or S, S-dioxide. Examples of monocyclic heterocyclic rings include oxetanyl, piperidyl, pyrrolidinyl, piperazinyl, morpholinyl, thiomorpholinyl, thiazolidinyl, 1,4-dioxanyl, tetrahydrofuranyl, tetrahydrothiophenyl, delta-lactam, delta-lactone, silacyclopentane, silapyrrolidine and the like, and all isomers thereof.

A ring carbon atom of a heterocyclic group may be functionalized as a carbonyl group. An illustrative example of such a heterocyclic group is:

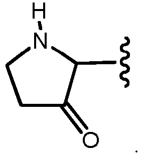

In one embodiment, a heterocyclic group is a 5-membered monocyclic heterocyclic ring. In another embodiment, a heterocyclic group is a 6-membered monocyclic heterocyclic ring. The term "3- to 8-membered monocyclic heterocyclic ring" refers to a monocyclic heterocyclic ring having from 3 to 8 ring atoms. The term "3- to 6-membered monocyclic heterocyclic ring" refers to a monocyclic heterocyclic ring having from 3 to 6 ring atoms. The term "7- to 11-membered bicyclic heterocyclic ring" refers to a bicyclic heterocyclic ring group having from 7 to 11 ring atoms. Unless otherwise indicated, an heterocyclic ring is unsubstituted.

The term "substituted", as used herein, means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" is meant a compound that is sufficiently robust to survive isolation to a useful degree of purity from a reaction mixture, and formulation into an efficacious therapeutic agent.

The term "in substantially purified form," as used herein, refers to the physical state of a compound after the compound is isolated from a synthetic process (e.g., from a reaction mixture), a natural source, or a combination thereof. The term "in substantially purified form," also refers to the physical state of a compound after the compound is obtained from a purification process or processes described herein or well-known to the skilled artisan (e.g., chromatography, recrystallization and the like), in sufficient purity to be characterizable by standard analytical techniques described herein or well-known to the skilled artisan.

It should also be noted that any carbon as well as heteroatom with unsatisfied valences in the text, schemes, examples and tables herein is assumed to have the sufficient number of hydrogen atom(s) to satisfy the valences.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene *et al*, *Protective Groups in Organic Synthesis* (1991), Wiley, New York.

When any substituent or variable (e.g., alkyl, R⁶ etc.) occurs more than one time in any constituent or in Formula (I), its definition on each occurrence is independent of its definition at every other occurrence, unless otherwise indicated.

The term "composition," as used herein, is intended to encompass a product comprising the specified ingredients in the specified amounts, as well as any product which results directly from combination of the specified ingredients in the specified amounts.

IC₅₀, as used herein, refers to an amount, concentration or dosage of a particular test compound that achieves a 50% inhibition of a maximal response in an assay that measures such response.

The terms "subject" and "patient," as used herein, are used interchangeably. The terms "subject" and "subjects" refer to a mammal including a non-primate (e.g., a cow, pig, horse, cat, dog, rat, and mouse) and a primate *(e.g.,* a monkey such as a cynomolgous monkey, a chimpanzee and a human), and for example, a human. The subject may also be a farm animal *(e.g.,* a horse, a cow, a pig, etc.) or a pet *(e.g.,* a dog or a cat).

The terms "therapeutic agent" and "therapeutic agents," as used herein, refer to any agent(s) which can be used in the treatment or prevention of a disorder or one or more symptoms thereof. The term "therapeutic agent" includes a compound provided herein. In some cases, a therapeutic agent can be an agent which is known to be useful for or has been or is currently being used for the treatment or prevention of a disorder or one or more symptoms thereof.

The term "effective amount," as used herein, refers to an amount of a compound of Formula (I) and/or an additional therapeutic agent, or a composition thereof that is effective in producing the desired therapeutic, ameliorative, inhibitory or preventative effect when administered to a patient suffering from a viral infection or virus-related disorder. In the combination therapies of the present invention, an effective amount can refer to each individual agent or to the combination as a whole, wherein the amounts of all agents administered are together effective, but wherein the component agent of the combination may not be present individually in an effective amount. A "therapeutically effective amount" can vary depending on, *inter alia,* the compound, the disease and its severity, and the age, weight, *etc.*, of the subject to be treated.

The term "treating" or "treatment" of any disease or disorder, as used herein, refers to ameliorating a disease or disorder that exists in a subject. In some cases, "treating" or "treatment" includes ameliorating at least one physical parameter, which may be indiscernible by the subject. In other cases, "treating" or "treatment" includes modulating the disease or disorder, either physically *(e.g.,* stabilization of a discernible symptom) or physiologically (*e.g.,* stabilization of a physical parameter) or both. In other cases, "treating" or "treatment" includes delaying the onset of the disease or disorder.

The term "preventing," as used herein with respect to a disease or disorder, refers to reducing the likelihood or severity of the disease or disorder.

In another embodiment, R³ and R⁴ together with the carbon to which they are bonded form a cyclobutyl, cyclopentyl, or cyclohexyl which is optionally substituted with 1-3 substituents R¹², wherein R¹² is as previously defined.

In another embodiment, R³ and R⁴ together with the carbon to which they are bonded form a cyclopentyl which is optionally substituted with 1-3 substituents R¹², wherein R¹² is as previously defined.

In another embodiment, R³ and R⁴ together with the carbon to which they are bonded form a cyclohexyl which is optionally substituted with 1-3 substituents R¹², wherein R¹² is as previously defined.

In another embodiment, R³ and R⁴ together with the carbon to which they are bonded form an azetidine, a piperidine, a pyrrolidine, a morpholine, a tetrahydrofuran or a tetrahydropyran, said azetidine, piperidine, pyrrolidine, morpholine, tetrahydrofuran and tetrahydropyran being optionally substituted with 1-3 substituents R¹², wherein R¹² is as previously defined.

In another embodiment, R³ and R⁴ together with the carbon to which they are bonded form a tetrahydrofuran or a tetrahydropyran, said tetrahydrofuran and tetrahydropyran being optionally substituted with 1-3 substituents R¹², wherein R¹² is as previously defined.

In another embodiment, R³ and R⁴ together with the carbon to which they are bonded form an azetidine, a piperidine, a pyrrolidine or a morpholine, said azetidine, piperidine, pyrrolidine and morpholine being optionally substituted with 1-3 substituents R¹², wherein R¹² is as previously defined.

In another embodiment, R³ and R⁴ together with the carbon to which they are bonded form a spirocyclic carbocyclic or heterocyclic ring selected from: 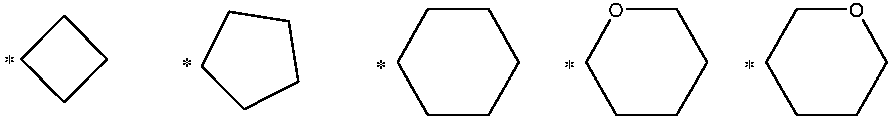 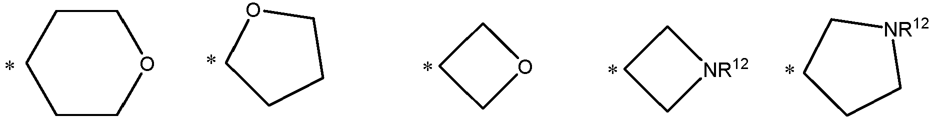 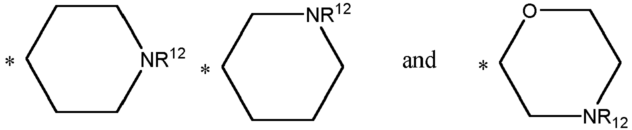 wherein the asterisk denotes the point of attachment of the spirocyclic carbocyclic or heterocyclic ring with the cyanoenone, said spirocyclic carbocyclic or heterocyclic ring being optionally substituted with 1-3 substituents R¹², wherein R¹² is as previously defined. In a sub-embodiment of this embodiment R¹² is selected from hydroxyl, -C₁₋₆alkyl, -C(O)O-C₁₋₆alkyl, - C(O)-C₁₋₆alkyl, -C(O)-C₃₋₆cycloalkyl, -C(O)-C₆₋₁₀aryl, -C(O)-het, -S(O)₂-C₆₋₁₀aryl, -C₆₋₁₀aryl, het, -CH₂-C₆₋₁₀aryl, -CH₂-C₃₋₆cycloalkyl and -CH₂-het, wherein het represents a 3- to 9-membered saturated or unsaturated heterocyclic ring comprising 1 to 3 heteroatoms independently selected from O, S and N, wherein each occurrence of said C₁₋₆alkyl, C₃₋6cycloalkyl, C₆₋₁₀aryl and 3- to 9-membered heterocyclic ring is optionally independently substituted with one or more substituents selected from halogen, hydroxyl, C₁₋₆alkyl, -C₁₋₆haloalkyl, -C₁₋₆hydroxyalkyl, -O-C₁₋₆alkyl and -S-C₁₋₆alkyl.

In another embodiment, R³ and R⁴ together with the carbon to which they are bonded form a spirocyclic carbocyclic or heterocyclic ring selected from: 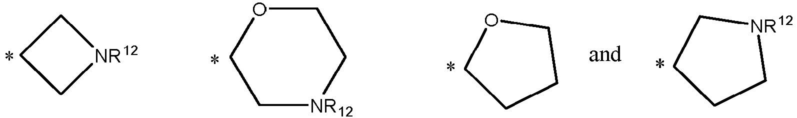 wherein the asterisk denotes the point of attachment of the spirocyclic carbocyclic or heterocyclic ring with the cyanoenone, said spirocyclic carbocyclic or heterocyclic ring being optionally substituted with 1-3 substituents R¹², wherein R¹² is as previously defined. In a sub-embodiment of this embodiment R¹² is selected from hydroxyl, -C₁₋₆alkyl, -C(O)O-C₁₋₆alkyl, - C(O)-C₁₋₆alkyl, -C(O)-C₃₋₆cycloalkyl, -C(O)-C₆₋₁₀aryl, -C(O)-het, -S(O)₂-C₆₋₁₀aryl, -C₆₋10aryl, het, -CH₂-C₆₋₁₀aryl, -CH₂-C₃₋₆cycloalkyl and -CH₂-het, wherein het represents a 3- to 9-membered saturated or unsaturated heterocyclic ring comprising 1 to 3 heteroatoms independently selected from O, S and N, wherein each occurrence of said C₁₋₆alkyl, C₃₋6cycloalkyl, C₆₋₁₀aryl and 3- to 9-membered heterocyclic ring is optionally independently substituted with one or more substituents selected from halogen, hydroxyl, C₁₋₆alkyl, -C₁₋₆haloalkyl, -C₁₋₆hydroxyalkyl, -O-C₁₋₆alkyl and -S-C₁₋₆alkyl.

In another embodiment is a compound having the formula Ia - Id: 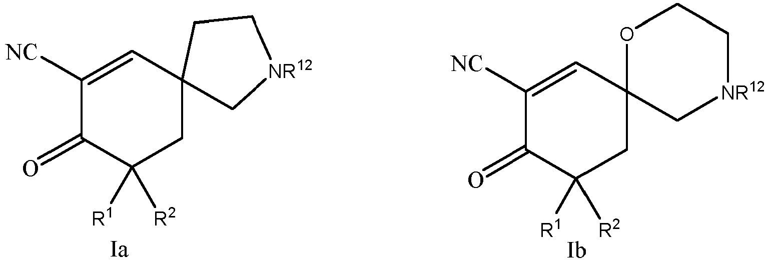 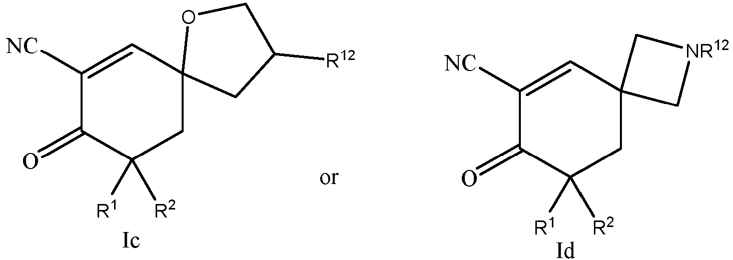 or a pharmaceutically acceptable salt or solvate thereof, wherein R¹, R² and R¹² are as previously defined. In a sub-embodiment of this embodiment R¹² is selected from hydroxyl, -C₁₋₆alkyl, - C(O)O-C₁₋₆alkyl, -C(O)-C₁₋₆alkyl, -C(O)-C₃₋₆cycloalkyl, -C(O)-C₆₋₁₀aryl, -C(O)-het, - S(O)₂-C₆₋₁₀aryl, -C₆₋₁₀aryl, het, -CH₂-C₆₋₁₀aryl, -CH₂-C₃₋₆cycloalkyl and -CH₂-het, wherein het represents a 3- to 9-membered saturated or unsaturated heterocyclic ring comprising 1 to 3 heteroatoms independently selected from O, S and N, wherein each occurrence of said C₁₋₆alkyl, C₃₋₆cycloalkyl, C₆₋₁₀aryl and 3- to 9-membered heterocyclic ring is optionally independently substituted with one or more substituents selected from halogen, hydroxyl, C₁₋₆alkyl, -C₁₋₆haloalkyl, -C₁₋₆hydroxyalkyl, -O-C₁₋₆alkyl and -S-C₁₋₆alkyl.

In another embodiment is a compound having the formula Ia - Id: 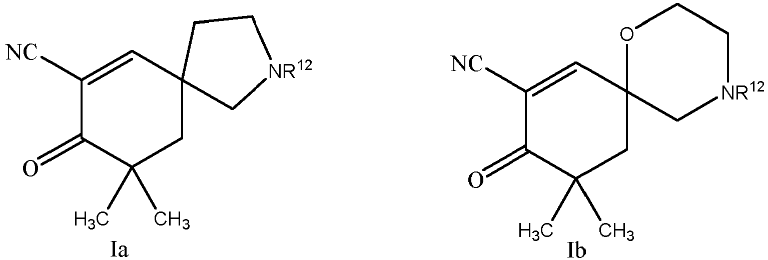 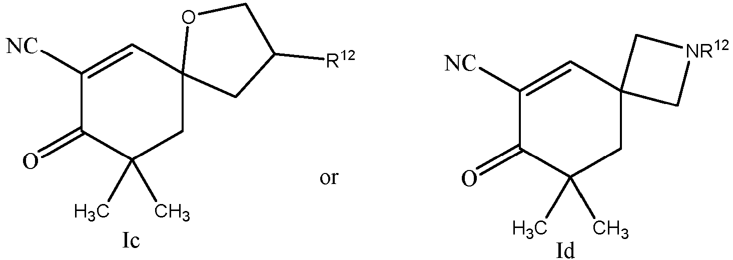 or a pharmaceutically acceptable salt or solvate thereof, wherein R¹² is as previously defined. In a sub-embodiment of this embodiment R¹² is selected from hydroxyl, -C₁₋₆alkyl, -C(O)O-C₁₋₆alkyl, -C(O)-C₁₋₆alkyl, -C(O)-C₃₋₆cycloalkyl, -C(O)-C₆₋₁₀aryl, -C(O)-het, -S(O)₂-C₆₋10aryl, -C₆₋₁₀aryl, het, -CH₂-C₆₋₁₀aryl, -CH₂-C₃₋₆cycloalkyl and -CH₂-het, wherein het represents a 3- to 9-membered saturated or unsaturated heterocyclic ring comprising 1 to 3 heteroatoms independently selected from O, S and N, wherein each occurrence of said C₁₋₆alkyl, C₃₋₆cycloalkyl, C₆₋₁₀aryl and 3- to 9-membered heterocyclic ring is optionally independently substituted with one or more substituents selected from halogen, hydroxyl, C₁₋₆alkyl, -C₁₋₆haloalkyl, -C₁₋₆hydroxyalkyl, -O-C₁₋₆alkyl and -S-C₁₋₆alkyl.

In another embodiment is a compound of Formula Ia: 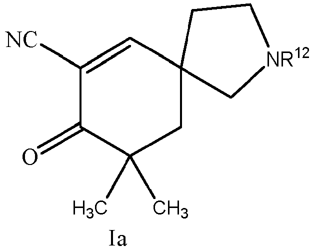 or a pharmaceutically acceptable salt or solvate thereof, wherein R¹² is as previously defined. In a sub-embodiment of this embodiment R¹² is selected from hydroxyl, -C₁₋₆alkyl, -C(O)O-C₁₋₆alkyl, -C(O)-C₁₋₆alkyl, -C(O)-C₃₋₆cycloalkyl, -C(O)-C₆₋₁₀aryl, -C(O)-het, -S(O)₂-C₆₋10aryl, -C₆₋₁₀aryl, het, -CH₂-C₆₋₁₀aryl, -CH₂-C₃₋₆cycloalkyl and -CH₂-het, wherein het represents a 3- to 9-membered saturated or unsaturated heterocyclic ring comprising 1 to 3 heteroatoms independently selected from O, S and N, wherein each occurrence of said C₁₋₆alkyl, C₃₋₆cycloalkyl, C₆₋₁₀aryl and 3- to 9-membered heterocyclic ring is optionally independently substituted with one or more substituents selected from halogen, hydroxyl, C₁₋₆alkyl, -C₁₋₆haloalkyl, -C₁₋₆hydroxyalkyl, -O-C₁₋₆alkyl and -S-C₁₋₆alkyl. In a sub-embodiment of this embodiment R¹² is -C(O)-C₆₋₁₀aryl, -C(O)-het, -C₆₋₁₀aryl, het, -CH₂-C₆₋10aryl, -CH₂-C₃₋₆cycloalkyl and -CH₂-het, wherein het represents a 3- to 9-membered saturated or unsaturated heterocyclic ring comprising 1 to 3 heteroatoms independently selected from O, S and N, wherein each occurrence of said C₃₋₆cycloalkyl, C₆₋₁₀aryl and 3- to 9-membered heterocyclic ring is optionally independently substituted with one or more substituents selected from halogen, hydroxyl, C₁₋₆alkyl, -C₁₋₆haloalkyl, -C₁₋₆hydroxyalkyl, -O-C₁₋₆alkyl and -S-C₁₋₆alkyl. In a sub-embodiment of this embodiment embodiment R¹² is -C(O)-C₆₋₁₀aryl, - C(O)-het, -C₆₋₁₀aryl, het, -CH₂-C₆₋₁₀aryl, -CH₂-C₃₋₆cycloalkyl and -CH₂-het, wherein het represents a heterocyclic ring selected from pyridyl, pyrimidyl, pyrazinyl, pyridazinyl, oxazolyl, imidazolyl and pyrazolyl wherein each occurrence of said C₃₋₆cycloalkyl, C₆₋₁₀aryl and het is optionally independently substituted with one or more substituents selected from halogen,

In another embodiment is a compound of Formula Ib: 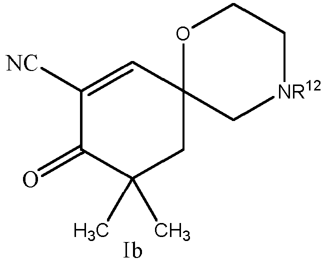 or a pharmaceutically acceptable salt or solvate thereof, wherein R¹² is as previously defined. In a sub-embodiment of this embodiment R¹² is selected from hydroxyl, -C₁₋₆alkyl, -C(O)O-C₁₋₆alkyl, -C(O)-C₁₋₆alkyl, -C(O)-C₃₋₆cycloalkyl, -C(O)-C₆₋₁₀aryl, -C(O)-het, -S(O)₂-C₆₋10aryl, -C₆₋₁₀aryl, het, -CH₂-C₆₋₁₀aryl, -CH₂-C₃₋₆cycloalkyl and -CH₂-het, wherein het represents a 3- to 9-membered saturated or unsaturated heterocyclic ring comprising 1 to 3 heteroatoms independently selected from O, S and N, wherein each occurrence of said C₁₋₆alkyl, C₃₋₆cycloalkyl, C₆₋₁₀aryl and 3- to 9-membered heterocyclic ring is optionally independently substituted with one or more substituents selected from halogen, hydroxyl, C₁₋₆alkyl, -C₁₋₆haloalkyl, -C₁₋₆hydroxyalkyl, -O-C₁₋₆alkyl and -S-C₁₋₆alkyl. In a sub-embodiment of this embodiment R¹² is -C(O)-C₆₋₁₀aryl, -C(O)-het, -C₆₋₁₀aryl, het, -CH₂-C₆₋10aryl, -CH₂-C₃₋₆cycloalkyl and -CH₂-het, wherein het represents a 3- to 9-membered saturated or unsaturated heterocyclic ring comprising 1 to 3 heteroatoms independently selected from O, S and N, wherein each occurrence of said C₃₋₆cycloalkyl, C₆₋₁₀aryl and 3- to 9-membered heterocyclic ring is optionally independently substituted with one or more substituents selected from halogen, hydroxyl, C₁₋₆alkyl, -C₁₋₆haloalkyl, -C₁₋₆hydroxyalkyl, -O-C₁₋₆alkyl and -S-C₁₋₆alkyl. In a sub-embodiment of this embodiment embodiment R¹² is -C(O)-C₆₋₁₀aryl, - C(O)-het, -C₆₋₁₀aryl, het, -CH₂-C₆₋₁₀aryl, -CH₂-C₃₋₆cycloalkyl and -CH₂-het, wherein het represents a heterocyclic ring selected from pyridyl, pyrimidyl, pyrazinyl, pyridazinyl, oxazolyl, imidazolyl and pyrazolyl wherein each occurrence of said C₃₋₆cycloalkyl, C₆₋₁₀aryl and het is optionally independently substituted with one or more substituents selected from halogen, hydroxyl, C₁₋₆alkyl, -C₁₋₆haloalkyl, -C₁₋₆hydroxyalkyl, -O-C₁₋₆alkyl and -S-C₁₋₆alkyl.

In another embodiment, R³ and R⁴ together with the carbon to which they are bonded form a C₃₋₈cycloalkyl or a 3- to 11-membered saturated or unsaturated heterocyclic ring comprising one to four heteroatoms independently selected from O, S and N, wherein the S is optionally oxidized to SO or SO₂, wherein said C₃₋₈cycloalkyl and 3- to 11-membered saturated or unsaturated heterocyclic ring are optionally substituted with 1-3 substituents R¹² and wherein two R¹² substituents on adjacent atoms of the C₃₋₈cycloalkyl or 3- to 11-membered saturated or unsaturated heterocycloalkyl together with the ring atoms to which they are bonded form a fused bicyclic ring system which is optionally substituted with one or more substituents R²¹;

In another embodiment, R³ and R⁴ together with the carbon to which they are bonded form a C₃₋₈cycloalkyl or a 3- to 8-membered saturated or unsaturated heterocyclic ring comprising one to three heteroatoms independently selected from O, S and N, wherein the S is optionally oxidized to SO or SO₂, wherein said C₃₋₈cycloalkyl and 3- to 8-membered saturated or unsaturated heterocyclic ring are optionally substituted with 1-3 substituents R¹² and wherein two R¹² substituents on adjacent atoms of the C₃₋₈cycloalkyl or 3- to 8-membered saturated or unsaturated heterocyclic ring together with the ring atoms to which they are bonded form a fused bicyclic ring system which is optionally substituted with one or more substituents R²¹;

In another embodiment, R³ and R⁴ together with the carbon to which they are bonded form a C₃₋₈cycloalkyl which is optionally substituted with 1-3 substituents R¹² and wherein two R¹² substituents on adjacent atoms of the C₃₋₈cycloalkyl together with the ring atoms to which they are bonded form a fused bicyclic ring system which is optionally substituted with one or more substituents R²¹;

In a further embodiment of the present invention is a compound of Formula (I), or a pharmaceutically acceptable salt or solvate thereof, wherein R³ and R⁴ together with the carbon to which they are bonded form a 3- 11-membered saturated or unsaturated heterocyclic ring comprising one to four heteroatoms independently selected from O, S and N, wherein the S is optionally oxidized to SO or SO₂, wherein said 3- to 11-membered saturated or unsaturated heterocyclic ring is optionally substituted with 1-3 substituents R¹² and wherein two R¹² substituents on adjacent atoms of the 3- to 11-membered saturated or unsaturated heterocyclic ring together with the ring atoms to which they are bonded form a fused bicyclic ring system which is optionally substituted with one or mo substituents R²¹;

In a further embodiment of the present invention is a compound of Formula (I), or a pharmaceutically acceptable salt or solvate thereof, wherein R³ and R⁴ together with the carbon to which they are bonded form a 3- 8-membered saturated or unsaturated heterocyclic ring comprising one to three heteroatoms independently selected from O, S and N, wherein the S is optionally oxidized to SO or SO₂, wherein said 3- to 8-membered saturated or unsaturated heterocyclic ring is optionally substituted with 1-3 substituents R¹² and wherein two R¹² substituents on adjacent atoms of the 3- to 8-membered saturated or unsaturated heterocyclic ring together with the ring atoms to which they are bonded form a fused bicyclic ring system which is optionally substituted with one or mo substituents R²¹;

In another embodiment is a compound of Formula IIa having the structure: 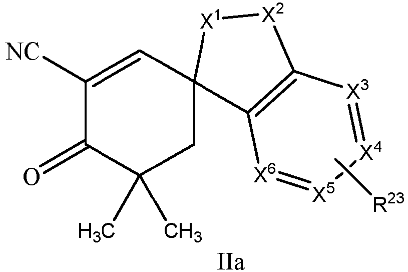
or a pharmaceutically acceptable salt or solvate thereof, wherein,
X¹ is O, OCR^{12a}R^{12a}, CR^{12a}R^{12a}O, CR^{12a}R^{12a}, (CR^{12a}R^{12a})₂, C(O), NR^{12a}, CHR^{12a}NR^{12a} or NR^{12a}CR^{12a}R^{12a};
X² is O, CR^{12a}R^{12a}, C(O) or NR^{12a};
X³, X⁴, X⁵ and X⁶ are each independently N or CR²³, with the proviso that no more than 2 of X³-X⁶ can be N and
each R^{12a} is independently H or C₁₋₆alkyl and
R²³ is 1-3 substituents independently selected from H, halogen, hydroxyl, C₁₋₆alkyl and -OC₁₋₆alkyl, wherein said -C₁₋₆alkyl and -OC₁₋₆alkyl are each independently substituted with one or more halogens, or a pharmaceutically acceptable salt or solvate thereof.

In a further embodiment is a compound of Formula IIa: 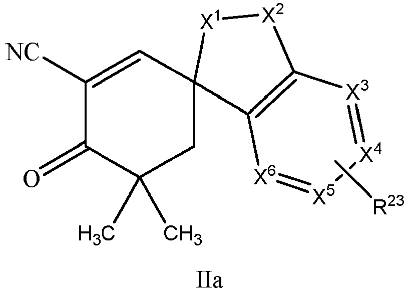
or a pharmaceutically acceptable salt or solvate thereof, wherein,
X¹ is O or CR^{12a}R^{12a};
X² is CR^{12a}R^{12a}, C(O) or NR^{12a};
X³, X⁴, X⁵ and X⁶ are each independently N or CR²³, with the proviso that no more than 2 of X³-X⁶ can be N;
each R^{12a} is independently H, -C(O)C₁₋₆alkyl, or-C₁₋₆alkyl, optionally substituted with hydroxyl and
each R²³ is independently H, halogen, -C₁₋₆alkyl or -C₁₋₆haloalkyl.

In a further embodiment is a compound of Formula IIa: 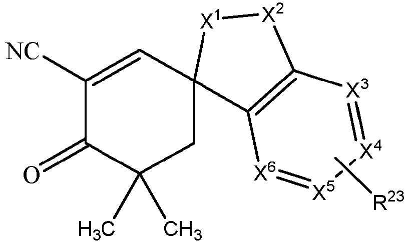 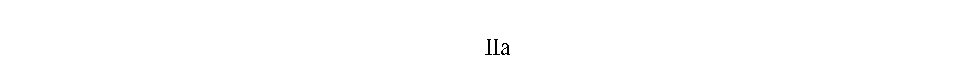
or a pharmaceutically acceptable salt or solvate thereof, wherein,
X¹ is O wherein each R^{12a} and X²-X⁶ are selected independently from the previously defined options. In a further embodiment of this sub-embodiment, X¹ is O and X² is CR^{12a}R^{12a}, wherein each R^{12a} and X³-X⁶ are selected independently from the previously defined options. In a further embodiment of this sub-embodiment, X¹ is O, X² is CR^{12a}R^{12a}, wherein each R^{12a} is selected independently from the previously defined options, X³-X⁵ are CR²³, wherein each R²³ is selected independently from the previously defined options and X⁶ is N. In a further embodiment of this sub-embodiment, X¹ is O, X² is CH₂, X³-X⁵ are CR²³, wherein each R²³ is selected independently from the previously defined options and X⁶ is N.

In a further embodiment is a compound of Formula IIa: 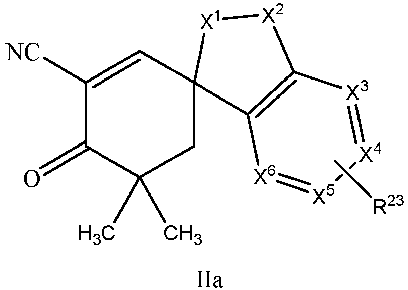 or a pharmaceutically acceptable salt or solvate thereof, wherein, X¹ is O, X² is (CR^{12a}R^{12a})₂, wherein each R^{12a} is selected independently from the previously defined options and each X³-X⁶ are selected independently from the previously defined options. In a further embodiment of this sub-embodiment, X¹ is O, X² is CH₂CH₂, and X³-X⁶ are CR²³, wherein each R²³ is selected independently from the previously defined options.

In a further embodiment is a compound of Formula IIa: 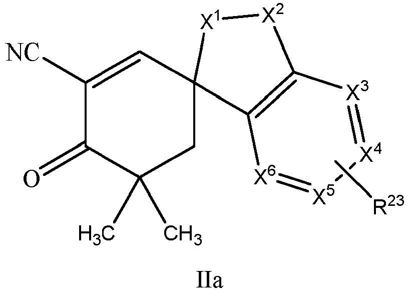 or a pharmaceutically acceptable salt or solvate thereof, wherein, X¹ is CR^{12a}, X² is CR^{12a}, wherein each R^{12a} is selected independently from the previously defined options and X³-X⁶ are each selected independently from the previously defined options. In a further embodiment of this sub-embodiment, X¹ is O, X² is CH₂CH₂, and X³-X⁶ are CR²³, wherein each R²³ is selected independently from the previously defined options.

In a further embodiment is a compound of formula IIa: 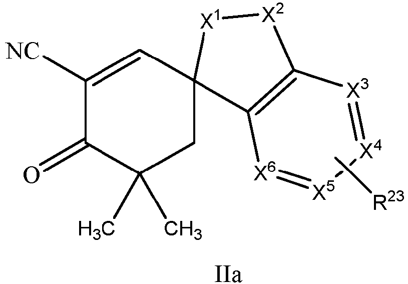 or a pharmaceutically acceptable salt or solvate thereof, wherein, X¹ is C(O) and X² is NR^{12a}, wherein R^{12a} is as previously defined and X³-X⁶ are each independently selected from the previously defined options. In a further embodiment of this sub-embodiment, X¹ is C(O), X² is NR^{12a}, wherein R^{12a} is as previously defined and X³-X⁶ are CR²³, wherein each R²³ is as previously defined. In a further embodiment of this sub-embodiment, X¹ is C(O), X² is NCH₃ and X³-X⁶ are CR²³, wherein each R²³ is as previously defined.

In another embodiment is a compound having the formula: 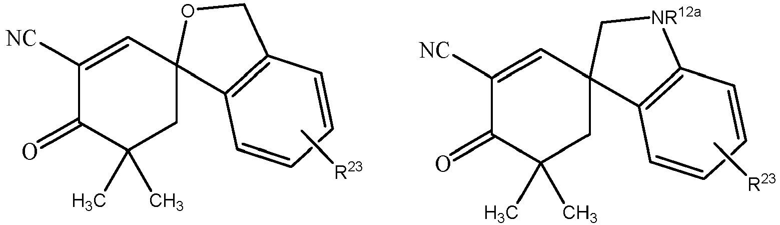 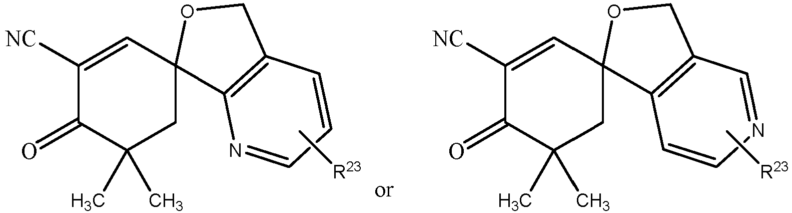 or a pharmaceutically acceptable salt or solvate thereof, wherein R^{12a} and R²³ are as previously defined. In a sub-embodiment of this embodiment R²³ is 1-3 substituents independently selected from H, fluoro, chloro, methyl and CF₃.

In another embodiment is a compound having the formula: 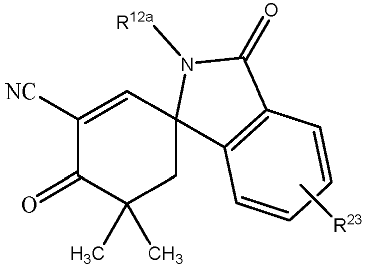 or a pharmaceutically acceptable salt or solvate thereof, wherein R^{12a} is as previously defined and each R²³ is independently selected from the previously defined options. In a sub-embodiment of this embodiment, R^{12a} is methyl and each R²³ is independently selected from H, fluoro, chloro, methyl and CF₃.

In another embodiment of the present invention is a compound of Formula (I), or a pharmaceutically acceptable salt or solvate thereof, wherein R³ and R⁴ together with the carbon to which they are bonded form a C₃₋₈cycloalkyl or a 3- to 9-membered saturated or unsaturated heterocyclic ring comprising one to three heteroatoms independently selected from O, S and N, wherein the S is optionally oxidized to SO or SO₂, wherein said C₃₋₈cycloalkyl and 3- to 9-membered saturated or unsaturated heterocyclic ring are optionally substituted with 1-3 substituents R¹² and wherein two R¹² substituents on the same atom of the C₃₋₈cycloalkyl or 3- to 9-membered saturated or unsaturated heterocyclic ring together with the ring atoms to which they are bonded form a spirocyclic bicyclic ring system which is optionally substituted with one or more substituents R²²;

In another embodiment of the present invention is a compound of Formula (I), or a pharmaceutically acceptable salt or solvate thereof, wherein R³ and R⁴ together with the carbon to which they are bonded form a C₃₋₈cycloalkyl or a 3- to 9-membered saturated or unsaturated heterocyclic ring comprising one to three heteroatoms independently selected from O, S and N, wherein the S is optionally oxidized to SO or SO₂, wherein said C₃₋₈cycloalkyl and 3- to 9-membered saturated or unsaturated heterocyclic ring are optionally substituted with 1-3 substituents R¹² and wherein two R¹² substituents on the C₃₋₈cycloalkyl or 3- to 8-membered saturated or unsaturated heterocyclic ring together with the ring atoms to which they are bonded form a bridged bicyclic ring system which is optionally substituted with one or more substituents R²²;

In another embodiment is a compound of formula (I) which is selected from: 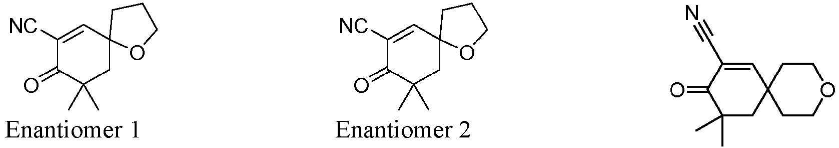 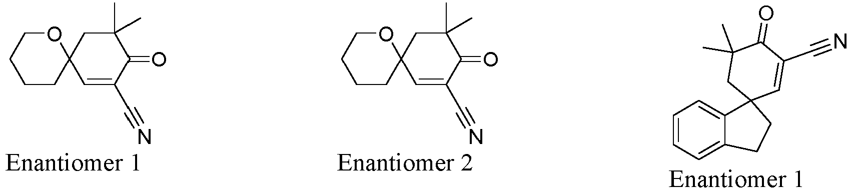 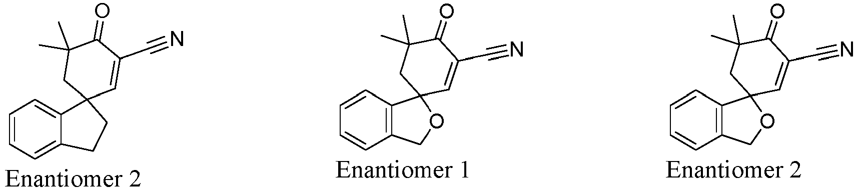 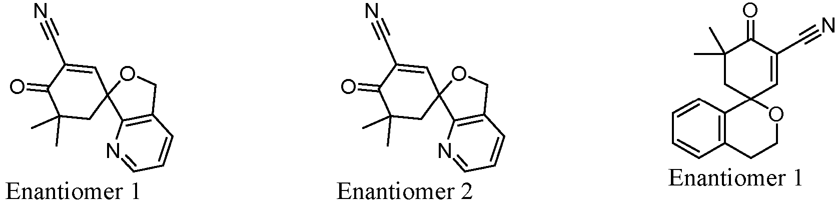 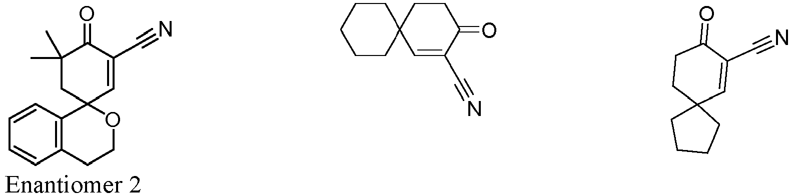 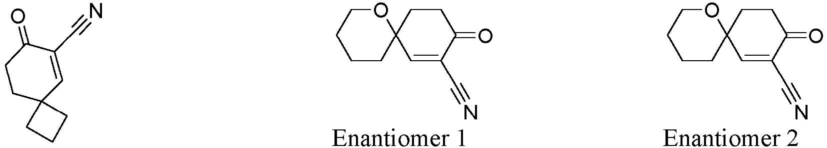 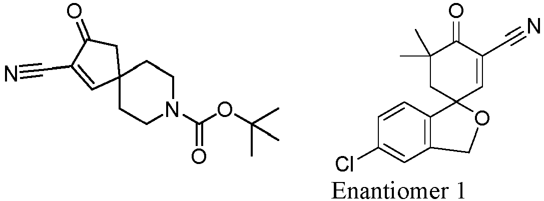 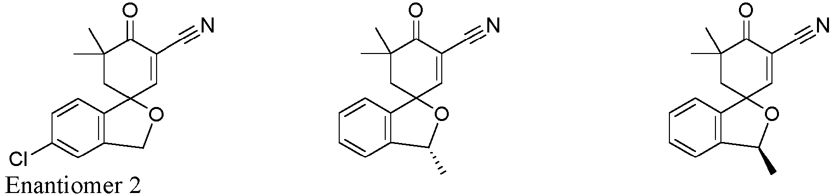 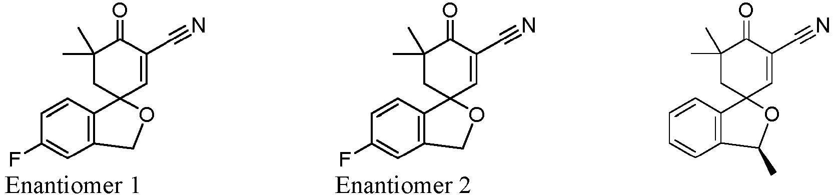 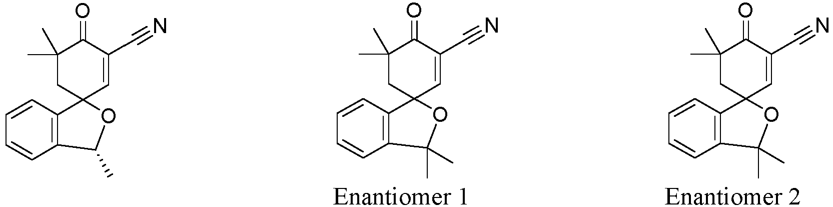 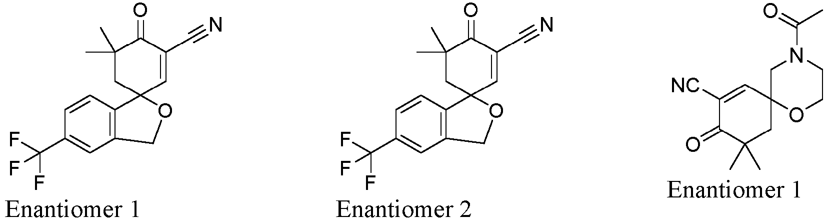 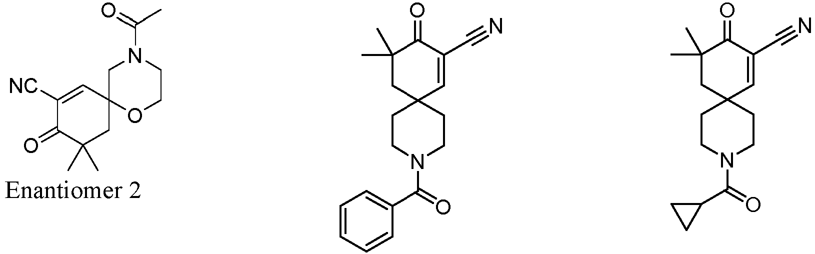 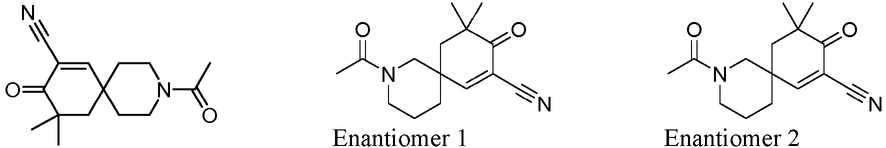 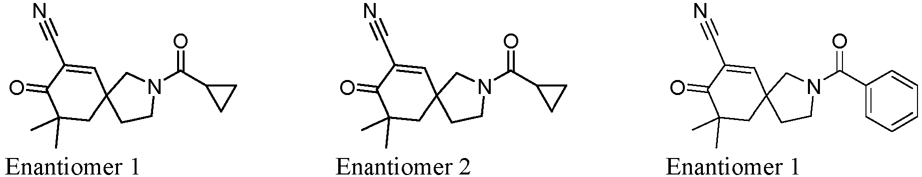 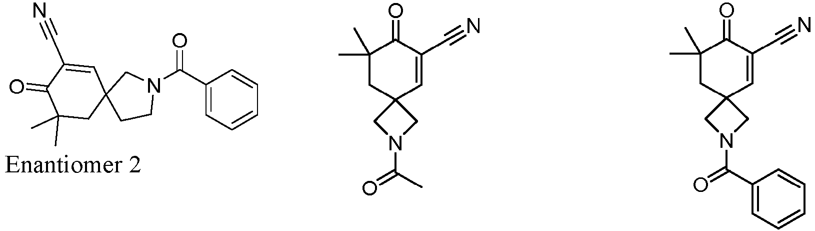 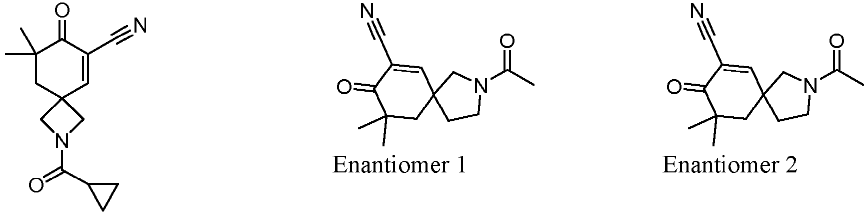 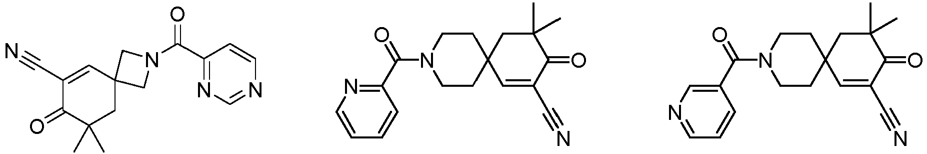 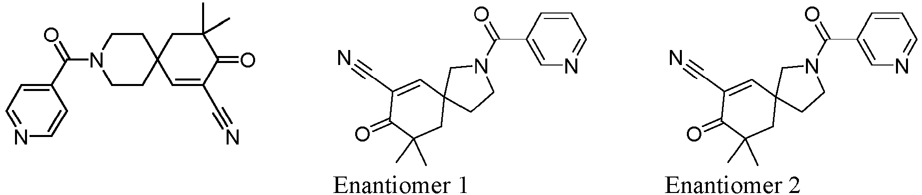 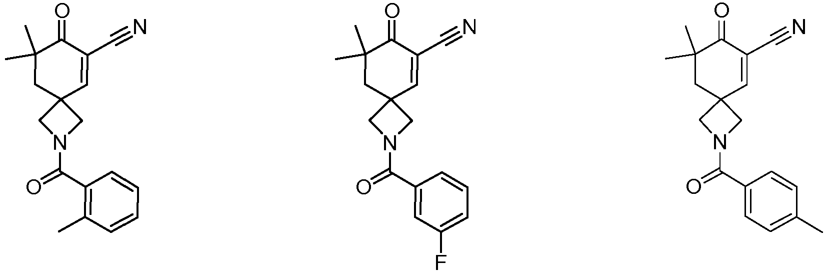 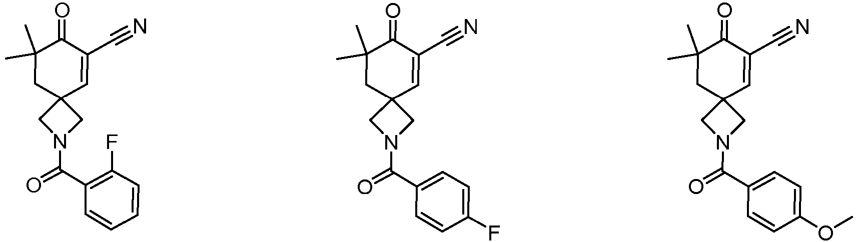 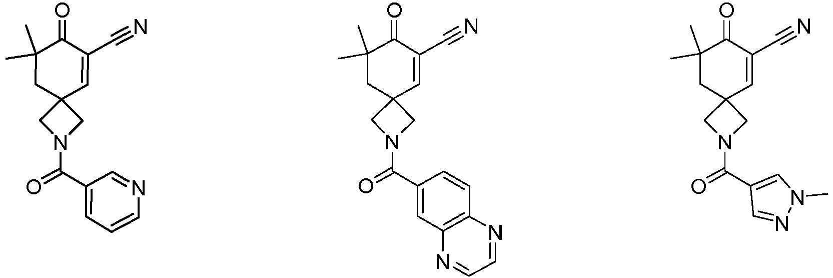 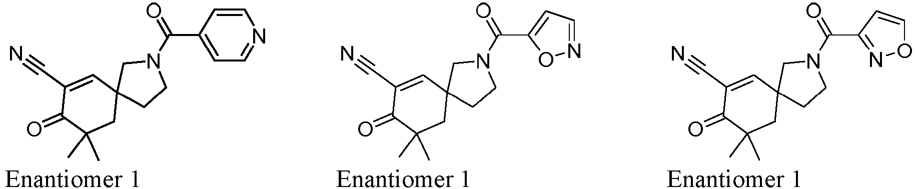 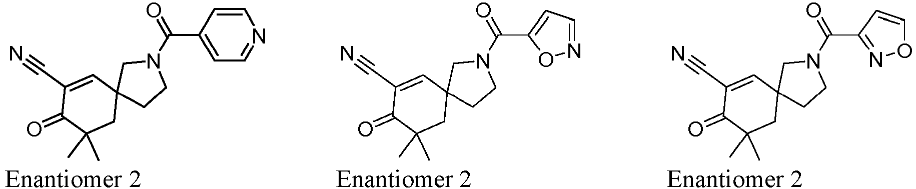 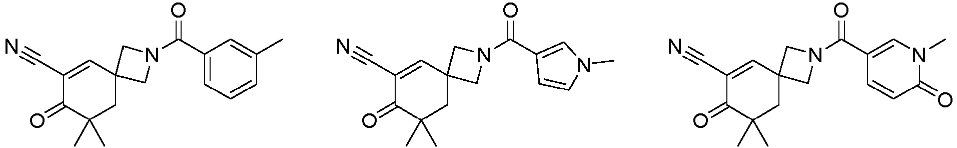 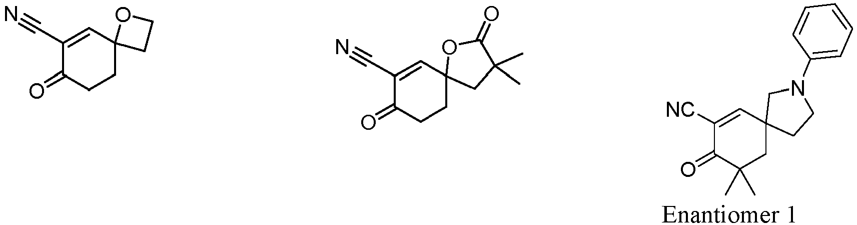 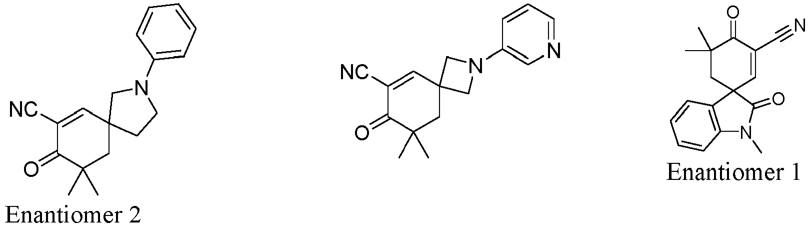 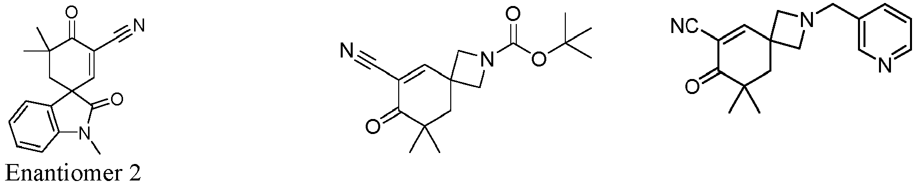 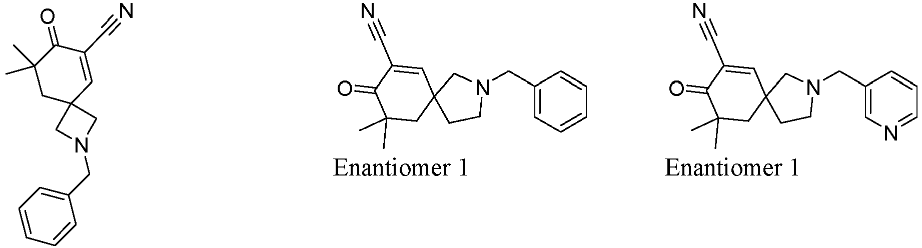 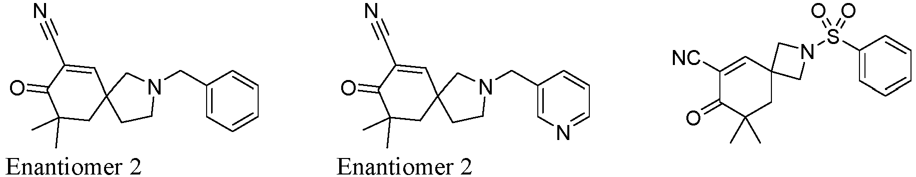 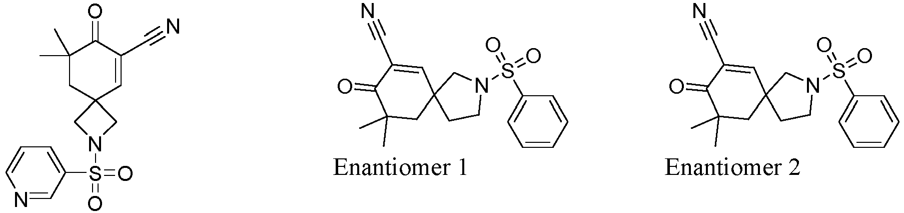 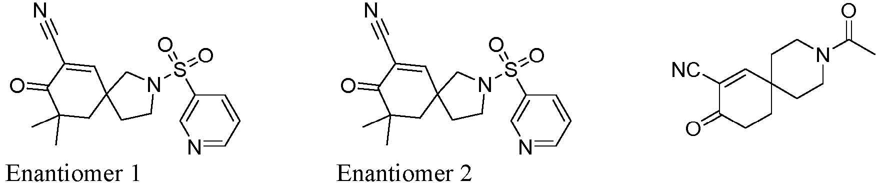 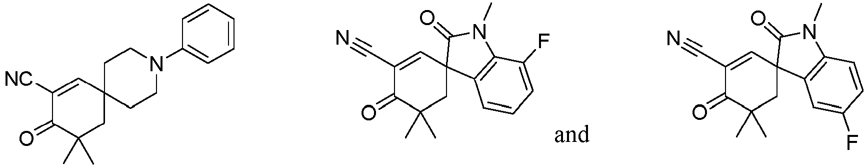 or a pharmaceutically acceptable salt or solvate thereof.

In another embodiment is a compound of formula (I) which is selected from: 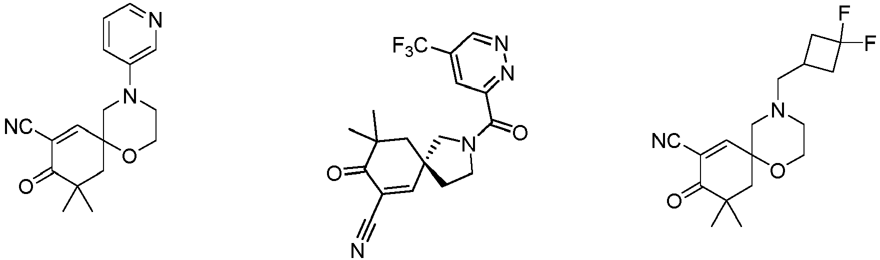 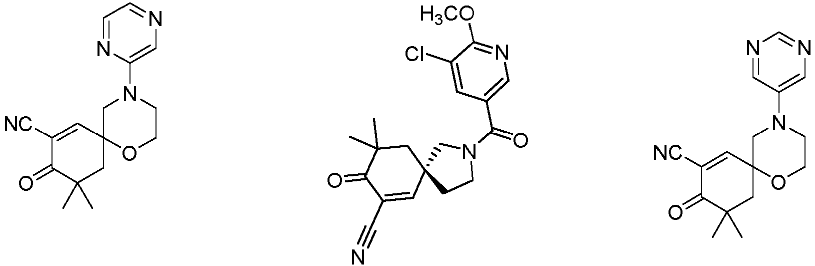 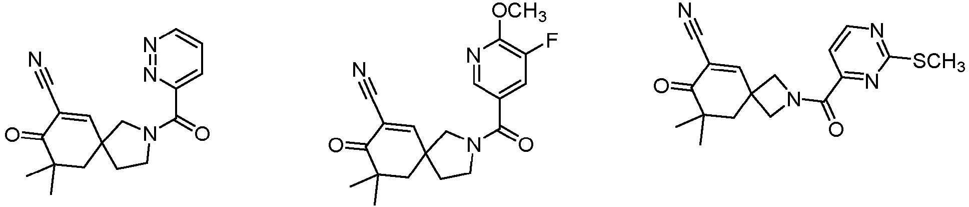 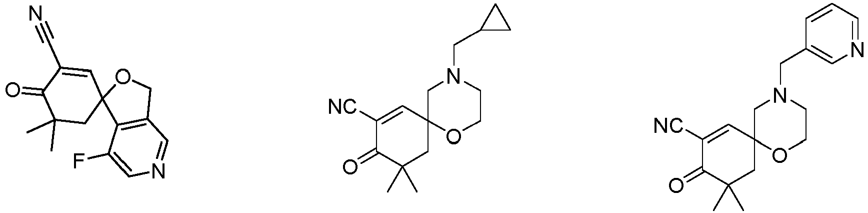 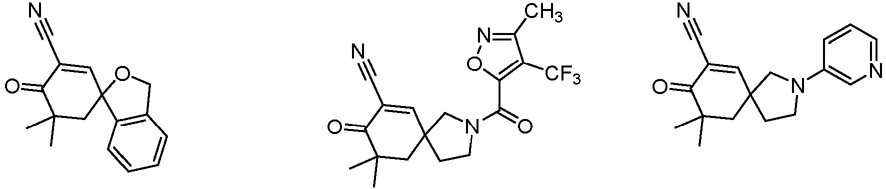 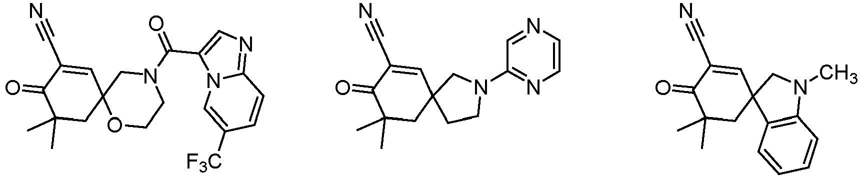 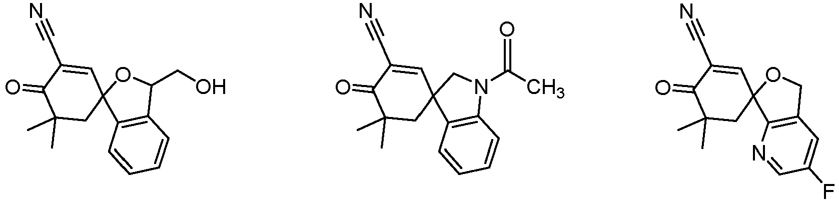 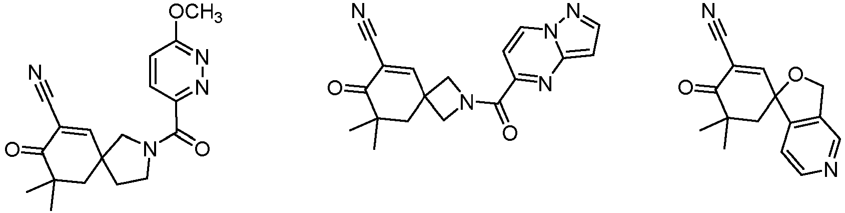 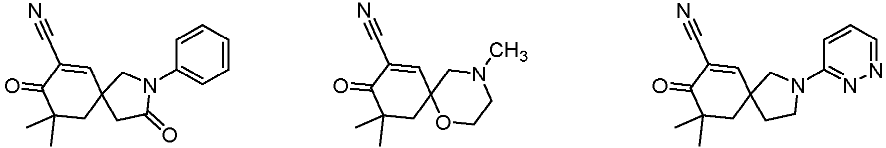 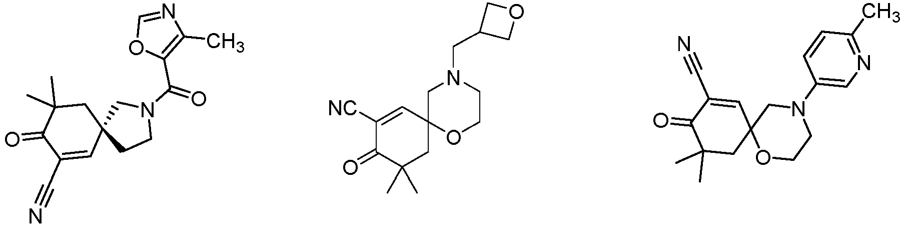 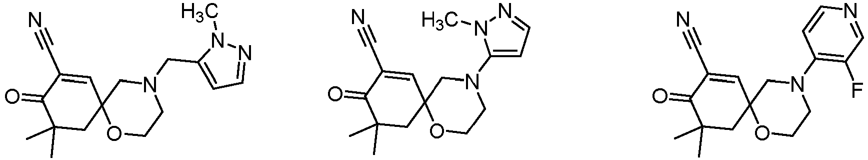 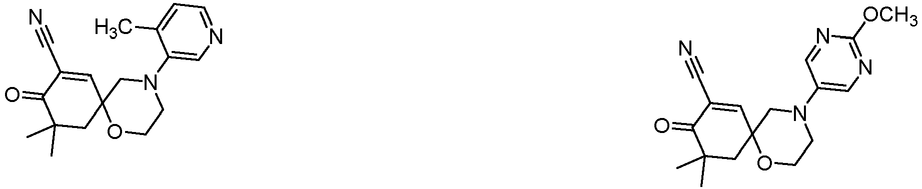 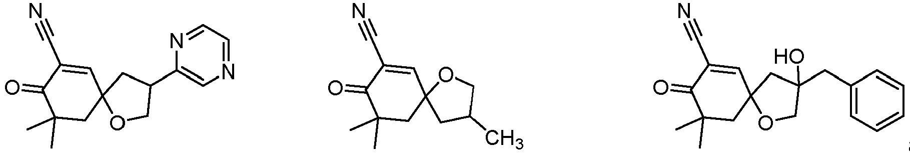 and 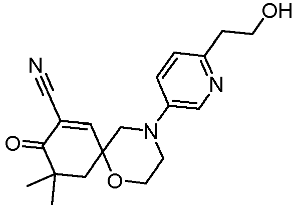 or a pharmaceutically acceptable salt or solvate thereof.

In one embodiment, variables for the Compounds of Formula (I) are selected independently of each other.

In another embodiment, the Compounds of Formula (I) are in substantially purified form.

Prodrugs and solvates of the compounds of the invention are also contemplated herein. Prodrugs of the compounds of the invention are not according to the claims. A discussion of prodrugs is provided in T. Higuchi and V. Stella, Pro-drugs as Novel Delivery Systems (1987) 14 of the A.C.S. Symposium Series, and in Bioreversible Carriers in Drug Design, (1987) Edward B. Roche, ed., American Pharmaceutical Association and Pergamon Press. The term "prodrug" means a compound *(e.g.,* a drug precursor) that is transformed *in vivo* to provide a Compound of Formula (I) or a pharmaceutically acceptable salt of the compound. The transformation may occur by various mechanisms (e.g., by metabolic or chemical processes), such as, for example, through hydrolysis in blood.

For example, if a Compound of Formula (I) contains a carboxylic acid functional group, a prodrug can comprise an ester formed by the replacement of the hydrogen atom of the acid group with a group such as, for example, (C₁-₈)alkyl, (C₂-₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-₂)alkylamino(C₂-₃)alkyl (such as β-dimethylaminoethyl), carbamoyl-(C₁-₂)alkyl, N,N-di (C₁-₂)alkylcarbamoyl-(C₁-₂)alkyl and piperidino-, pyrrolidino- or morpholino(C₂-₃)alkyl, and the like.

Similarly, if a Compound of Formula (I) contains an alcohol functional group, a prodrug can be formed by the replacement of one or more of the hydrogen atoms of the alcohol groups with a group such as, for example, (C₁-₆)alkanoyloxymethyl, 1-((C₁-₆)alkanoyloxy)ethyl, 1-methyl-1-((C₁-₆)alkanoyloxy)ethyl, (C₁-₆)alkoxycarbonyloxymethyl, N-(C₁-₆)alkoxycarbonylaminomethyl, succinoyl, (C₁-₆)alkanoyl, α-amino(C₁-₄)alkyl, α-amino(C₁-C₄)alkylene-aryl, arylacyl and α-aminoacyl, or α-aminoacyl-α-aminoacyl, where each α-aminoacyl group is independently selected from the naturally occurring L-amino acids, or glycosyl (the radical resulting from the removal of a hydroxyl group of the hemiacetal form of a carbohydrate). Other example of alcohol-derived prodrugs include -P(O)(OH)₂; -P(O)(-O-C₁-C₆alkyl)₂; -P(O)(-NH-(α-aminoacyl group))(-O-aryl); -P(O)(-O-(C₁-C₆ alkylene)-S-acyl)(-NH-arylalkyl);and those described in US Patent No. 7,879,815; International Publication Nos. WO2005/003047, WO2008/082602, WO2010/0081628, WO2010/075517 and WO2010/075549; Mehellou, Chem. Med. Chem., 5:1841-1842 (2005); Bobeck et al., Antiviral Therapy 15:935-950 (2010); Furman et al., Future Medicinal Chemistry, 1:1429-1452 (2009); and Erion, Microsomes and Drug Oxidations, Proceedings of the International Symposium, 17th, Saratoga Springs, NY, United States, July 6-10, 2008, 7-12 (2008).

If a Compound of Formula (I) incorporates an amine functional group, a prodrug can be formed by the replacement of a hydrogen atom in the amine group with a group such as, for example, R-carbonyl-, RO-carbonyl-, NRR'-carbonyl- wherein R and R' are each independently (C₁-₁₀)alkyl, (C₃-₇) cycloalkyl, benzyl, a natural α-aminoacyl, -C(OH)C(O)OY¹ wherein Y¹ is H, (C₁-C₆)alkyl or benzyl, -C(OY²)Y³ wherein Y² is (C₁-₄) alkyl and Y³ is (C₁-₆)alkyl; carboxy (C₁-₆)alkyl; amino(C₁-₄)alkyl or mono-N- or di-N,N-(C₁-₆)alkylaminoalkyl; - C(Y⁴)Y⁵ wherein Y⁴ is H or methyl and Y⁵ is mono-N- or di-N,N-(C₁-₆)alkylamino morpholino; piperidin-1-yl or pyrrolidin-1-yl, and the like.

Pharmaceutically acceptable esters of the present compounds include the following groups: (1) carboxylic acid esters obtained by esterification of the hydroxy group of a hydroxyl compound, in which the non-carbonyl moiety of the carboxylic acid portion of the ester grouping is selected from straight or branched chain alkyl (e.g., methyl, ethyl, n-propyl, isopropyl, t-butyl, sec-butyl or n-butyl), alkoxyalkyl *(e.g.,* methoxymethyl), aralkyl (*e.g.,* benzyl), aryloxyalkyl (for example, phenoxymethyl), aryl *(e.g.,* phenyl optionally substituted with, for example, halogen, C₁₋₄alkyl, -O-(C₁₋₄alkyl) or amino); (2) sulfonate esters, such as alkyl- or aralkylsulfonyl (for example, methanesulfonyl); (3) amino acid esters (e.g., L-valyl or L-isoleucyl); (4) phosphonate esters and (5) mono-, di- or triphosphate esters. The phosphate esters may be further esterified by, for example, a C₁₋₂₀ alcohol or reactive derivative thereof, or by a 2,3-di (C₆₋₂₄)acyl glycerol.

One or more compounds of the invention may exist in unsolvated as well as solvated forms with pharmaceutically acceptable solvents such as water, ethanol, and the like, and it is intended that the invention embrace both solvated and unsolvated forms. "Solvate" means a physical association of a compound of this invention with one or more solvent molecules. This physical association involves varying degrees of ionic and covalent bonding, including hydrogen bonding. In certain instances, the solvate will be capable of isolation, for example when one or more solvent molecules are incorporated in the crystal lattice of the crystalline solid. "Solvate" encompasses both solution-phase and isolatable solvates. Examples of solvates include ethanolates, methanolates, and the like. A "hydrate" is a solvate wherein the solvent molecule is water.

One or more compounds of the invention may optionally be converted to a solvate. Preparation of solvates is generally known. Thus, for example, M. Caira et al, J. Pharmaceutical Sci., 93(3), 601-611 (2004) describe the preparation of the solvates of the antifungal fluconazole in ethyl acetate as well as from water. Similar preparations of solvates, hemisolvate, hydrates and the like are described by E. C. van Tonder et al, AAPS PharmSciTechours. , 5(1), article 12 (2004); and A. L. Bingham et al, Chem. Commun., 603-604 (2001). A typical process involves dissolving the inventive compound in desired amounts of the desired solvent (organic or water or mixtures thereof) at a higher than room temperature, and cooling the solution at a rate sufficient to form crystals which are then isolated by standard methods. Analytical techniques such as, for example IR spectroscopy, show the presence of the solvent (or water) in the crystals as a solvate (or hydrate).

The compounds of Formula (I) can form salts which are also within the scope of this invention. The term "salt(s)", as employed herein, denotes acidic salts formed with inorganic and/or organic acids, as well as basic salts formed with inorganic and/or organic bases. In addition, when a compound of Formula (I) contains both a basic moiety, such as, a pyridine or imidazole, and an acidic moiety, such as, a carboxylic acid, zwitterions ("inner salts") may be formed and are included within the term "salt(s)" as used herein. In one embodiment, the salt is a pharmaceutically acceptable *(i.e.,* non-toxic, physiologically acceptable) salt. In another embodiment, the salt is other than a pharmaceutically acceptable salt. Salts of the compounds of Formula (I) may be formed, for example, by reacting a compound of Formula (I) with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates) and the like. Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website).

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamine, t-butyl amine, choline, and salts with amino acids such as arginine, lysine and the like. Basic nitrogen-containing groups may be quarternized with agents such as lower alkyl halides (e.g., methyl, ethyl, and butyl chlorides, bromides and iodides), dialkyl sulfates (e.g., dimethyl, diethyl, and dibutyl sulfates), long chain halides *(e.g.,* decyl, lauryl, and stearyl chlorides, bromides and iodides), aralkyl halides (e.g., benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

All stereoisomers (for example, geometric isomers, optical isomers and the like) of the present compounds (including those of the salts and solvates of the compounds), such as those which may exist due to asymmetric carbons on various substituents, including enantiomeric forms (which may exist even in the absence of asymmetric carbons), rotameric forms, atropisomers (e.g., substituted biaryls) and diastereomeric forms, are contemplated within the scope of this invention. If a compound of Formula (I) incorporates a double bond or a fused ring, both the cis- and trans-forms, as well as mixtures, are embraced within the scope of the invention.

It is also possible that the compounds of Formula (I) may exist in different tautomeric forms, and all such forms are embraced within the scope of the invention. For example, all keto-enol and imine-enamine forms of the compounds are included in the invention.

Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations. The use of the terms "salt", "solvate" and the like, is intended to apply equally to the salt and solvate of enantiomers, stereoisomers, rotamers, tautomers, positional isomers, racemates or prodrugs of the inventive compounds.

Diastereomeric mixtures may be separated into their individual diastereomers on the basis of their physical chemical differences by methods well-known to those skilled in the art, such as, for example, by chromatography and/or fractional crystallization. Enantiomers may be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereomers to the corresponding pure enantiomers. Sterochemically pure compounds may also be prepared by using chiral starting materials or by employing salt resolution techniques. Enantiomers can also be directly separated using chiral chromatographic techniques.

In the compounds of Formula (I), the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of generic Formula (I). For example, different isotopic forms of hydrogen (H) include protium (¹H) and deuterium (²H). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds of Formula (I) may be prepared without undue experimentation by conventional techniques well known to those skilled in the art or by processes analogous to those described in the Schemes and Examples herein using appropriate isotopically-enriched reagents and/or intermediates. In one embodiment, a Compound of Formula (I) has one or more of its hydrogen atoms replaced with deuterium.

Polymorphic forms of the compounds of Formula (I), and of the salts or solvates of the Compounds of Formula (I), are intended to be included in the present invention.

Other embodiments of the present invention include the following:
(a) A pharmaceutical composition comprising an effective amount of a compound of Formula (I), or a pharmaceutically acceptable salt or solvate thereof, and a pharmaceutically acceptable carrier or diluent.
(b) The pharmaceutical composition of (a), further comprising one or more other therapeutic agents selected from neurological or psychiatric drugs, anti-inflammatory drugs, immunomodulatory drugs and anticancer drugs.
(c) A combination comprising a compound of Formula (I), or a pharmaceutically acceptable salt or solvate thereof and one, two, three or more other therapeutic agents.
(d) The combination of (c) that is (i) a Compound of Formula (I), or a pharmaceutically acceptable salt or solvate thereof, and (ii) one or more other therapeutic agents selected from neurological or psychiatric drugs, anti-inflammatory drugs, immunomodulatory drugs and anticancer drugs.
(e) A Compound of Formula (I), or a pharmaceutically acceptable salt or solvate thereof for use in therapy.
(f) The Compound for use of (e), wherein the therapy is treating or preventing a disease mediated by activation of Nrf2 transcription in a subject.
(g) The Compound for use of (e) or (f), wherein the therapy is treating or preventing a neurological disorder, an inflammatory disorder, an autoimmune disorder or a cancer in a subject.
(h) The Compound for use of (g), wherein the therapy is treating or preventing a neurodegenerative disease such as Fredreich's Ataxia, amyotrophic lateral sclerosis, stroke, Alzheimer's disease, Parkinson's disease, Huntingdon's disease, peripheral neuropathy or hearing loss in a subject.
(i) The Compound for use of (e), (f), (g) or (h), wherein said compound is administered in combination with one or more other therapeutic agents.
(j) Use of a Compound of Formula (I), or a pharmaceutically acceptable salt or solvate thereof, for the manufacture of a medicament for use in treating or preventing a neurological disorder, an inflammatory disorder, an autoimmune disorder or a cancer in a subject.
(k) The use of (j), wherein the neurodegenerative disease is Fredreich's Ataxia, amyotrophic lateral sclerosis, stroke, Alzheimer's disease, Parkinson's disease, Huntingdon's disease, peripheral neuropathy or hearing loss in a subject.
(l) The use of (j) or (k), wherein said compound is administered in combination with one or more other therapeutic agents.
(m) A method of treating or preventing a neurological disorder, an inflammatory disorder, an autoimmune disorder or a cancer in a subject in need thereof which comprises administering to the subject an effective amount of a compound of Formula (I) or a pharmaceutically acceptable salt or solvate thereof.
(n) The method of (m), wherein the neurological disorder is a neurodegenerative disease selected from Fredreich's Ataxia, amyotrophic lateral sclerosis, stroke, Alzheimer's disease, Parkinson's disease, Huntingdon's disease, peripheral neuropathy and hearing loss.
(o) The method of (m) or (n) wherein the compound of Formula (I) is administered in combination with an effective amount of one or more other therapeutic agents.

Additional embodiments of the invention include the pharmaceutical compositions, combinations and methods set forth in (a)-(o) above and the uses set forth in the discussion below, wherein the compound of the present invention employed therein is a compound of one of the embodiments, aspects, classes, sub-classes, or features of the compounds described above. **In** all of these embodiments, the compound may optionally be used in the form of a pharmaceutically acceptable salt or solvate as appropriate. It is understood that references to compounds would include the compound in its present form as well as in different forms, such as polymorphs and solvates as applicable.

It is further to be understood that the embodiments of compositions and methods provided as (a) through (o) above are understood to include all embodiments of the compounds, including such embodiments as result from combinations of embodiments.

In a further embodiment is a compound of Formula (I), or a pharmaceutically acceptable salt or solvate thereof, for use in the treatment or prevention of a neurodegenerative disorder in a subject. In one embodiment, the neurodegenerative disorder is selected from Alzheimer's Disease, Parkinson's Disease, Huntington's disease, other CAG-triplet repeat (or polyglutamine) diseases, amyotrophic lateral sclerosis (ALS, Lou Gehrig,'s Disease), diffuse Lewy body disease, chorea-acanthocytosis, primary lateral sclerosis, multiple sclerosis (MS), frontotemporal dementia, Friedrich's ataxia, acute head injury and epilepsy (repression of microglia activation). In a further embodiment, the neurodegenerative disorder is Fredreich's Ataxia, amyotrophic lateral sclerosis, stroke, Alzheimer's disease, Parkinson's disease, Huntingdon's disease, peripheral neuropathy or hearing loss. In a further embodiment, the neurodegenerative disorder is Alzheimer's disease. In a further embodiment, the neurodegenerative disorder is Parkinson's disease. In a further embodiment the neurodegenerative disorder is amyotrophic lateral sclerosis.

In a further embodiment is a compound of Formula (I), or a pharmaceutically acceptable salt or solvate thereof, for use in the treatment or prevention of an opthalmologic disorder in a subject. In one embodiment, the opthalmologic disorder is selected from Retinal diseases such as macular degeneration, diabetic retinopathy, diabetic macular edema, retinitis pigmentosa, Stargardt disease, Usher syndrome, Leber congenital amaurosis, choroidemia, rod-cone or cone-rod dystrophy, ciliopathy, progressive retinal atrophy, degenerative retinal diseases, retinopathy of prematurity, retinal vascular diseases, cataracts, glaucoma, glaucomatous retinal neurodegeneration; ischemic optic neuropathy and ophthalmic vascular diseases.

In a further embodiment is a compound of Formula (I), or a pharmaceutically acceptable salt or solvate thereof, for use in the treatment of a renal kidney disease in a subject. In one embodiment, the renal kidney disease is chronic or acute kidney failure, acute kidney injury from prerenal, intrarenal and postrenal causes, chronic kidney disease, diabetic nephropathy, focal segmental glomerulosclerosis (FSGS), nephrotic syndrome or non-diabetic chronic kidney disease.

Accordingly, the invention also provides methods for treating or preventing a neurological disorder and/or reducing the likelihood or severity of symptoms of a neurological disorder in a patient, the methods comprising administering to the patient an effective amount of at least one compound of Formula (I) or a pharmaceutically acceptable salt or solvate thereof.

In a further embodiment is a compound of Formula (I), or a pharmaceutically acceptable salt or solvate thereof, for use in the inhibition, the treatment and/or reduction of the likelihood or severity of symptoms of an inflammatory disorder in a subject. In one embodiment, the inflammatory disorder is selected from (i) respiratory diseases, such as chronic obstructive pulmonary disease (COPD), asthma, idiopathic pulmonary fibrosis and radiation pneumonitis; (ii) cardiovascular diseases, such as atherosclerotic disease; (iii) metabolic diseases, such as acute kidney injury, chronic kidney disease, acute liver injury and chronic liver failure; (iv) multiple organ failure arising as a consequence of systemic sepsis and/or major trauma; (v) inflammatory arthritis, such as gout; (vi) inflammatory bowel disease (IBD), such as Crohns's disease or ulcerative colitis and (vii) inflammatory skin diseases, such as atopic dermatitis.

Accordingly, the invention also provides methods for treating or preventing an inflammatory disorder and/or reducing the likelihood or severity of symptoms of an inflammatory disorder in a patient, the methods comprising administering to the patient an effective amount of at least one compound of Formula (I) or a pharmaceutically acceptable salt or solvate thereof.

In a further embodiment is a compound of Formula (I), or a pharmaceutically acceptable salt or solvate thereof, for use in the inhibition, the treatment and/or reduction of the likelihood or severity of symptoms of an autoimmune disorder in a subject. In one embodiment, the autoimmune disorder is selected from systemic lupus erythematosus (SLE), Sjogren's syndrome, rheumatoid arthritis, psoriatic arthritis, psoriasis and myositis.

Accordingly, the invention also provides methods for treating or preventing an autoimmune disorder and/or reducing the likelihood or severity of symptoms of an autoimmune disorder in a patient, the methods comprising administering to the patient an effective amount of at least one compound of Formula (I) or a pharmaceutically acceptable salt or solvate thereof.

In a further embodiment is a compound of Formula (I), or a pharmaceutically acceptable salt or solvate thereof, for use in the inhibition, the treatment and/or reduction of the likelihood or severity of symptoms of a cancer in a subject. In one embodiment, the cancer is selected from liver cancer, breast cancer, gastric cancer, colorectal cancer, colon cancer, prostate cancer, gall-bladder cancer, ovarian cancer, lung cancer, esophageal cancer, glioma, esophageal squameous cell carcinoma, endometrial cancer, papillary cancer, head and kneck cancers, skin cancer, hepatocellular carcinoma, renal and urinary bladder carcinoma, pancreatic cancer and leukemic cancer. In particular embodiments, the cancer is a metastasis which has originated from other cancers (such as colon cancer, pancreatic cancer, *etc).*

Accordingly, the invention also provides methods for treating or preventing cancer and/or reducing the likelihood or severity of symptoms of cancer in a patient, the methods comprising administering to the patient an effective amount of at least one compound of Formula (I) or a pharmaceutically acceptable salt or solvate thereof.

In another aspect of the present invention, the therapeutic use of the present compounds and compositions can further comprise the administration of one or more additional therapeutic agents which are not compounds of Formula (I).

In one embodiment, the compound of Formula (I) is administered in combination with one additional therapeutic agent. In a further embodiment, the compound of Formula (I) is administered in combination with two additional therapeutic agents. In a still further embodiment, the compound of Formula (I) is administered in combination with two or more additional therapeutic agents.

In one embodiment, the additional therapeutic agent is a neurological or psychiatric drug such as a cognitive or memory enhancing agent.

In another embodiment, the additional therapeutic agent is an anti-inflammatory agent.

In another embodiment, the additional therapeutic agent is an immunomodulatory agent, such as an immunosuppressive agent.

In another embodiment, the additional therapeutic agent is an anticancer agent.

In one embodiment, the additional therapeutic agent is a beta-secretase inhibitor, for example, verubecestat; a M1 mAChR agonist or PAM; a M4 mAChR agonist or PAM; a mGluR2 antagonist or NAM or PAM; an ADAM 10 or activator; a gamma-secretase inhibitor, such as LY450139 and TAK 070; a gamma secretase modulator; a tau phosphorylation inhibitor; a glycine transport inhibitor; a LXR β agonist; an ApoE4 conformational modulator; a NR2B antagonist; an androgen receptor modulator; a blockers of Aβ oligomer formation; a 5-HT4 agonist, such as PRX-03140; a 5-HT6 antagonist, such as GSK 742467, SGS-518, FK-962, SL-65.0155, SRA-333 and xaliproden;a 5-HT1a antagonist, such as lecozotan; a p25/CDK5 inhibitor; a NK1/NK3 receptor antagonist; a COX-2 inhibitora ; LRRK2 inhibitor; a CB-1 receptor antagonist or CB-1 receptor inverse agonist, such as AVE1625; a N-methyl-D-aspartate (NMDA) receptor antagonista, such as memantine, neramexane and EVT101; a cholinesterase inhibitor such as galantamine, rivastigmine, donepezil, tacrine, phenserine, ladostigil and ABT-089; a growth hormone secretagogue such as ibutamoren, ibutamoren mesylate, and capromorelin; a histamine H₃ receptor antagonist such as ABT-834, ABT 829, GSK 189254 and CEP16795; an AMPA agonist or AMPA modulator, such as CX-717, LY 451395, LY404187 and S-18986; a PDE IV inhibitor, including MEM1414, HT0712 and AVE8112; a GABAA inverse agonist; a GSK3β inhibitor, including AZD1080, SAR502250 and CEP16805; a neuronal nicotinic agonist; a selective M1 agonist; a HDAC inhibitor; and a microtubule affinity regulating kinase (MARK) ligands.

Examples of combinations of the compounds include combinations with agents for the treatment of schizophrenia, for example in combination with sedatives, hypnotics, anxiolytics, antipsychotics, antianxiety agents, cyclopyrrolones, imidazopyridines, pyrazolopyrimidines, minor tranquilizers, melatonin agonists and antagonists, melatonergic agents, benzodiazepines, barbiturates, 5HT-2 antagonists, and the like, such as: adinazolam, allobarbital, alonimid, aiprazolam, amisulpride, amitriptyline, amobarbital, amoxapine, aripiprazole, bentazepam, benzoctamine, brotizolam, bupropion, busprione, butabarbital, butalbital, capuride, carbocloral, chloral betaine, chloral hydrate, clomipramine, clonazepam, cloperidone, clorazepate, chlordiazepoxide, clorethate, chlorpromazine, clozapine, cyprazepam, desipramine, dexclamol, diazepam, dichloralphenazone, divalproex, diphenhydramine, doxepin, estazolam, ethchlorvynol, etomidate, fenobam, flunitrazepam, flupentixol, fluphenazine, flurazepam, fluvoxamine, fluoxetine, fosazepam, glutethimide, halazepam, haloperidol, hydroxyzine, imipramine, lithium, lorazepam, lormetazepam, maprotiline, mecloqualone, melatonin, mephobarbital, meprobamate, methaqualone, midaflur, midazolam, nefazodone, nisobamate, nitrazepam, nortriptyline, olanzapine, oxazepam, paraldehyde, paroxetine, pentobarbital, perlapine, perphenazine, phenelzine, phenobarbital, prazepam, promethazine, propofol, protriptyline, quazepam, quetiapine, reclazepam, risperidone, roletamide, secobarbital, sertraline, suproelone, temazepam, thioridazine, thiothixene, tracazolate, tranylcypromaine, trazodone, triazolam, trepipam, tricetamide, triclofos, trifluoperazine, trimetozine, trimipramine, uldazepam, venlafaxine, zaleplon, ziprasidone, zolazepam, zolpidem, and salts thereof, and combinations thereof, and the like, or the subject compound may be administered in conjunction with the use of physical methods such as with light therapy or electrical stimulation.

In another embodiment, the additional therapeutic agent is levodopa (with or without a selective extracerebral decarboxylase inhibitor such as carbidopa or benserazide), an anticholinergic such as biperiden (optionally as its hydrochloride or lactate salt) and trihexyphenidyl (benzhexol) hydrochloride; a COMT inhibitor such as entacapone, a MAO-B inhibitor, an antioxidant, a A2a adenosine receptor antagonist, a cholinergic agonist, a NMDA receptor antagonist, a serotonin receptor antagonist or dopamine receptor agonist such as alentemol, bromocriptine, fenoldopam, lisuride, naxagolide, pergolide and pramipexole. It will be appreciated that the dopamine agonist may be in the form of a pharmaceutically acceptable salt, for example, alentemol hydrobromide, bromocriptine mesylate, fenoldopam mesylate, naxagolide hydrochloride and pergolide mesylate.

In another embodiment, the additional therapeutic agent is an anti-inflammatory agent. Anti-inflammatory agents include non-steroidal anti-inflammatory drugs (NSAIDs), nonspecific and COX-2 specific cyclooxgenase enzyme inhibitors, gold compounds, corticosteroids, methotrexate, tumor necrosis factor receptor (TNF) receptors antagonists, immunosuppressants and methotrexate.

Examples of NSAIDs include ibuprofen, flurbiprofen, naproxen and naproxen sodium, diclofenac, combinations of diclofenac sodium and misoprostol, sulindac, oxaprozin, diflunisal, piroxicam, indomethacin, etodolac, fenoprofen calcium, ketoprofen, sodium nabumetone, sulfasalazine, tolmetin sodium, and hydroxychloroquine. Examples of NSAIDs also include COX-2 specific inhibitors such as celecoxib, valdecoxib, lumiracoxib and/or etoricoxib.

In some embodiments, the anti-inflammatory agent is a salicylate. Salicylates include by are not limited to acetylsalicylic acid or aspirin, sodium salicylate, and choline and magnesium salicylates.

The anti-inflammatory agent may also be a corticosteroid. For example, the corticosteroid may be cortisone, dexamethasone, methylprednisolone, prednisolone, prednisolone sodium phosphate, or prednisone.

In additional embodiments the anti-inflammatory agent is a gold compound such as gold sodium thiomalate or auranofin.

The invention also includes embodiments in which the anti-inflammatory agent is a metabolic inhibitor such as a dihydrofolate reductase inhibitor, such as methotrexate or a dihydroorotate dehydrogenase inhibitor, such as leflunomide.

Other embodiments of the invention pertain to combinations in which at least one anti-inflammatory agent is an anti-C5 monoclonal antibody (such as eculizumab or pexelizumab), a TNF antagonist, such as entanercept, or infliximab, which is an anti-TNF alpha monoclonal antibody.

In a further embodiment, the additional therapeutic agent is an anticancer agent. Examples of such agents include sorafenib tosylate (Nexavar), radiation therapy, selective internal radiation therapy (e.g., SIR-Spheres and TheraSphere), ethiodized oil (Lipidol), pexastimogene devacirepvec (Pexa-Vec, JX-594, Jennarex), Quinacrine (Clevelane BioLabs), CC-223 (Celgene), CF102 (Can-Fite), SGI-110 (Astex), and G-202 (Genspera).

In one embodiment, the anticancer agent is selected from the group consisting of vascular endothelial growth factor (VEGF) receptor inhibitors, topoisomerase II inhibitors, smoothen inhibitors, alkylating agents, anti-tumor antibiotics, anti-metabolites, retinoids, immunomodulatory agents including anti-cancer vaccines, CTLA-4, LAG-3, PD-1 antagonists and BET bromodomain inhibitors.

As used herein, the term "in combination" includes the use of more than one therapy (e.g., one or more prophylactic and/or therapeutic agents). The use of the term "in combination" does not restrict the order in which therapies (e.g., prophylactic and/or therapeutic agents) are administered to a subject with a disorder. A first therapy *(e.g.,* a prophylactic or therapeutic agent such as a compound provided herein) can be administered prior to (*e.g.*, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to *(e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy *(e.g.,* a prophylactic or therapeutic agent) to a subject with a disorder.

As used herein, the term "synergistic" includes a combination of a compound provided herein and another therapy *(e.g.,* a prophylactic or therapeutic agent) which has been or is currently being used to prevent, manage or treat a disorder, which is more effective than the additive effects of the therapies. A synergistic effect of a combination of therapies (e.g., a combination of prophylactic or therapeutic agents) permits the use of lower dosages of one or more of the therapies and/or less frequent administration of said therapies to a subject with a disorder. The ability to utilize lower dosages of a therapy *(e.g.,* a prophylactic or therapeutic agent) and/or to administer said therapy less frequently reduces the toxicity associated with the administration of said therapy to a subject without reducing the efficacy of said therapy in the prevention or treatment of a disorder). In addition, a synergistic effect can result in improved efficacy of agents in the prevention or treatment of a disorder. Finally, a synergistic effect of a combination of therapies (e.g., a combination of prophylactic or therapeutic agents) may avoid or reduce adverse or unwanted side effects associated with the use of either therapy alone.

When administering a combination therapy of the invention to a patient, therapeutic agents in the combination, or a pharmaceutical composition or compositions comprising therapeutic agents, may be administered in any order such as, for example, sequentially, concurrently, together, simultaneously and the like. The amounts of the various actives in such combination therapy may be different amounts (different dosage amounts) or same amounts (same dosage amounts). Thus, for illustration purposes, a Compound of Formula (I) and an additional therapeutic agent may be present in fixed amounts (dosage amounts) in a single dosage unit *(e.g.,* a capsule, a tablet and the like).

In one embodiment, the at least one compound of Formula (I) is administered during a time when the additional therapeutic agent(s) exert their prophylactic or therapeutic effect, or *vice versa.*

In another embodiment, the at least one compound of Formula (I) and the additional therapeutic agent(s) are administered in doses commonly employed when such agents are used as monotherapy.

In another embodiment, the at least one compound of Formula (I) and the additional therapeutic agent(s) are administered in doses lower than the doses commonly employed when such agents are used as monotherapy.

In still another embodiment, the at least one compound of Formula (I) and the additional therapeutic agent(s) act synergistically and are administered in doses lower than the doses commonly employed when such agents are used as monotherapy.

In one embodiment, the at least one compound of Formula (I) and the additional therapeutic agent(s) are present in the same composition. In one embodiment, this composition is suitable for oral administration. In another embodiment, this composition is suitable for intravenous administration. In another embodiment, this composition is suitable for subcutaneous administration. In still another embodiment, this composition is suitable for parenteral administration.

The active compounds provided herein can be administered in combination or alternation with another therapeutic agent. In combination therapy, effective dosages of two or more agents are administered together, whereas in alternation or sequential-step therapy, an effective dosage of each agent is administered serially or sequentially. The dosages given will depend on absorption, inactivation and excretion rates of the drug as well as other factors known to those of skill in the art. It is to be noted that dosage values will also vary with the severity of the condition to be alleviated. It is to be further understood that for any particular subject, specific dosage regimens and schedules should be adjusted over time according to the individual need and the professional judgment of the person administering or supervising the administration of the compositions.

The at least one compound of Formula (I) and the additional therapeutic agent(s) can act additively or synergistically. A synergistic combination may allow the use of lower dosages of one or more agents and/or less frequent administration of one or more agents of a combination therapy. A lower dosage or less frequent administration of one or more agents may lower toxicity of therapy without reducing the efficacy of therapy.

The doses and dosage regimen of the other agents used in the combination therapies of the present invention may be determined using the attending clinician, taking into consideration the approved doses and dosage regimen in the package insert; the age, sex and general health of the patient; and the type and severity of the viral infection or related disease or disorder. When administered in combination, the compound of Formula (I) and the other agent(s) may be administered simultaneously (*i.e.,* in the same composition or in separate compositions one right after the other) or sequentially. This particularly useful when the components of the combination are given on different dosing schedules, e.g., one component is administered once daily and another component is administered every six hours, or when the preferred pharmaceutical compositions are different, *e.g.,* one is a tablet and one is a capsule. A kit comprising the separate dosage forms is therefore advantageous.

Generally, a total daily dosage of the at least one Compound of Formula (I) alone, or when administered as combination therapy, can range from about 1 to about 2500 mg per day, although variations will necessarily occur depending on the target of therapy, the patient and the route of administration. In one embodiment, the dosage is from about 10 to about 1000 mg/day, administered in a single dose or in 2-4 divided doses. In another embodiment, the dosage is from about 1 to about 500 mg/day, administered in a single dose or in 2-4 divided doses. In still another embodiment, the dosage is from about 1 to about 100 mg/day, administered in a single dose or in 2-4 divided doses. In yet another embodiment, the dosage is from about 1 to about 50 mg/day, administered in a single dose or in 2-4 divided doses. In another embodiment, the dosage is from about 500 to about 1500 mg/day, administered in a single dose or in 2-4 divided doses. In still another embodiment, the dosage is from about 500 to about 1000 mg/day, administered in a single dose or in 2-4 divided doses. In yet another embodiment, the dosage is from about 100 to about 500 mg/day, administered in a single dose or in 2-4 divided doses.

Due to their activity, the compounds of Formula (I) are useful in veterinary and human medicine. As described above, the compounds of Formula (I) are useful for treating or preventing neurodegenerative diseases, inflammatory diseases, autoimmune diseases or cancers in a patient.

When administered to a patient, the compounds of Formula (I) may be administered as a component of a composition that comprises a pharmaceutically acceptable carrier or vehicle. The present invention provides pharmaceutical compositions comprising an effective amount of at least one compound of Formula (I) or a pharmaceutically acceptable salt, solvate or enantiomer thereof and a pharmaceutically acceptable carrier or diluent. In the pharmaceutical compositions and uses of the present invention, the active ingredients will typically be administered in admixture with suitable carrier materials suitably selected with respect to the intended form of administration, *i.e.,* oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral non-toxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Solid form preparations include powders, tablets, dispersible granules, capsules, cachets and suppositories. Powders and tablets may be comprised of from about 0.5 to about 95 percent inventive composition. Tablets, powders, cachets and capsules may be used as solid dosage forms suitable for oral administration.

Moreover, when desired or needed, suitable binders, lubricants, disintegrating agents and coloring agents may also be incorporated in the mixture. Suitable binders include starch, gelatin, natural sugars, corn sweeteners, natural and synthetic gums such as acacia, sodium alginate, carboxymethylcellulose, polyethylene glycol and waxes. Among the lubricants there may be mentioned for use in these dosage forms, boric acid, sodium benzoate, sodium acetate, sodium chloride, and the like. Disintegrants include starch, methylcellulose, guar gum, and the like. Sweetening and flavoring agents and preservatives may also be included where appropriate.

Liquid form preparations include solutions, suspensions and emulsions and may include water or water-propylene glycol solutions for parenteral injection.

Liquid form preparations may also include solutions for intranasal administration.

Also included are solid form preparations which are intended to be converted, shortly before use, to liquid form preparations for either oral or parenteral administration. Such liquid forms include solutions, suspensions and emulsions.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Additionally, the compositions of the present invention may be formulated in sustained release form to provide the rate controlled release of any one or more of the components or active ingredients to optimize therapeutic effects, *i.e.,* antiviral activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

In one embodiment, the one or more compounds of Formula (I) are administered orally.

In another embodiment, the one or more compounds of Formula (I) are administered intravenously.

In one embodiment, a pharmaceutical preparation comprising a compound of Formula (I) is in unit dosage form. In such form, the preparation is subdivided into unit doses containing effective amounts of the active components.

Compositions may be prepared according to conventional mixing, granulating or coating methods, respectively, and the present compositions can contain, in one embodiment, from about 0.1% to about 99% of the compound of Formula (I) by weight or volume. In various embodiments, the present compositions can contain, in one embodiment, from about 1% to about 70% or from about 5% to about 60% of the compound of Formula (I) by weight or volume.

The quantity of compound of Formula (I) in a unit dose of preparation may be varied or adjusted from about 1 mg to about 2500 mg. In various embodiments, the quantity is from about 10 mg to about 1000 mg, 1 mg to about 500 mg, 1 mg to about 100 mg, and 1 mg to about 100 mg.

For convenience, the total daily dosage may be divided and administered in portions during the day if desired. In one embodiment, the daily dosage is administered in one portion. In another embodiment, the total daily dosage is administered in two divided doses over a 24 hour period. In another embodiment, the total daily dosage is administered in three divided doses over a 24 hour period. In still another embodiment, the total daily dosage is administered in four divided doses over a 24 hour period.

The amount and frequency of administration of the compounds of Formula (I) will be regulated according to the judgment of the attending clinician considering such factors as age, condition and size of the patient as well as severity of the symptoms being treated. Generally, a total daily dosage of the compounds of Formula (I) range from about 0.1 to about 2000 mg per day, although variations will necessarily occur depending on the target of therapy, the patient and the route of administration. In one embodiment, the dosage is from about 1 to about 200 mg/day, administered in a single dose or in 2-4 divided doses. In another embodiment, the dosage is from about 10 to about 2000 mg/day, administered in a single dose or in 2-4 divided doses. In another embodiment, the dosage is from about 100 to about 2000 mg/day, administered in a single dose or in 2-4 divided doses. In still another embodiment, the dosage is from about 500 to about 2000 mg/day, administered in a single dose or in 2-4 divided doses.

### EXAMPLES

The invention is illustrated by the following examples. For all of the examples, standard work-up and purification methods known to those skilled in the art can be utilized. Unless otherwise indicated, all temperatures are expressed in °C (degrees Centigrade). All reactions are conducted at room temperature unless otherwise noted. Synthetic methodologies illustrated herein are intended to exemplify the applicable chemistry through the use of specific examples.

Abbreviations used are those conventional in the art or the following:
- ACN: acetonitrile
- Ar: aryl
- Aq: aqueous
- Boc: *tert*-butyloxycarbonyl protecting group
- °C: degree Celsius
- CDCl₃: deuterated chloroform
- CHCl₃: chloroform
- CO₂: carbon dioxide
- DBDMH: 1,3-dibromo-5,5-dimethylhydantoin
- DCM: dichloromethane
- DDQ: 2,3-dichloro-5,6-dicyano-1,4-benzoquinone
- DIEA: N,N-diisopropylethylamine
- DMAP: 4-dimethylaminopyridine
- DMF: N,N-dimethylformamide
- DMSO: dimethyl sulfoxide
- DMSO-*d*₆: deuterated dimethyl sulfoxide
- Et₂O: diethyl ether
- EtOAc: ethyl acetate
- EtOH: ethanol
- Eq: equivalents
- g: gram
- h: hour
- H₂O: water
- H₂O₂: hydrogen peroxide
- HATU: *N-[(dimethylamino)-1H-1,2,3-triazolo-[4,5-b]pyridin-1-ylmethylene]-N-*methylmethanaminium hexafluorophosphate N-oxide
- HCl: hydrochloric acid
- HPLC: high performance liquid chromatography
- KOtBu: potassium tert-butoxide
- L: liter
- LCMS: liquid chromatography and mass spectrometry
- LDA: lithium diisopropylamide
- LiHMDS: lithium bis(trimethylsilyl)amide
- M: molar
- MHz: megahertz
- MeI: methyl iodide
- MeOH: methanol
- MS: mass spectrometry
- mmol: millimole
- mg: milligram
- min: minute
- mL: milliliter
- N₂: nitrogen
- NaH: sodium hydride
- NaHCO₃: sodium bicarbonate
- NaOMe: sodium methoxide
- NaOH: sodium hydroxide
- NaOtBu: sodium tert-butoxide
- nBuLi: n-butyllithium solution
- nM: nanomolar
- N: normal
- NH₄Cl: ammonium chloride
- NH₃H₂O: ammonia in water
- NH₄OH: ammonium hydroxide
- NMR: nuclear magnetic resonance
- HO-NH₂ HCl: hydroxylamine hydrochloride
- Pd(OAc)₂: palladium(II) acetate
- Pd-PEPPSI-IHepCl: Dichloro[1,3-bis(2,6-di-4-heptylphenyl)imidazol-2-ylidene](3-chloropyridyl)palladium(II)
- PhSeCl: phenylselenyl chloride
- Pyr: pyridine
- rt: room temperature
- RuPhos Pd G2: chloro(2-dicyclohexylphosphino-2',6'-diisopropoxy-1,1'-biphenyl)[2-(2'-amino-1,1'-biphenyl)]palladium(II)
- sat.: saturated
- SM: starting material
- SFC: supercritical fluid chromatography
- SPhos Pd G4: methanesulfonato(2-dicyclohexylphosphino-2',6'-dimethoxy-1,1'-biphenyl)(2'-methylamino-1,1'-biphenyl-2-yl)palladium(II)
- tBuOH: tert-butanol
- T3P: propylphosphonic anhydride
- TEA: triethylamine
- TFA: trifluoroacetic acid
- THF: tetrahydrofuran
- TLC: thin layer chromatography
- Prep. TLC: preparative TLC
- TsCl: p-toluenesulfonyl chloride (tosyl chloride)
- TsCN: p-toluenesulfonyl cyanide (tosyl cyanide)
- µL: microliter
- vol: volume
- VT: variable temperature

### GENERAL SYNTHETIC SCHEMES

While the present invention has been described in conjunction with the specific examples set forth below, many alternatives, modifications, and variations thereof will be apparent to those of ordinary skill in the art. In some cases, the order of carrying out the steps of the reaction schemes may be varied to facilitate the reaction or to avoid unwanted reaction products. Starting materials and intermediates are purchased from commercial sources, made from known procedures, or are otherwise illustrated.

Several methods for preparing the compounds of this invention are described in the following Schemes and Examples. Unless otherwise indicated, all variables are as previously defined.

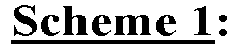

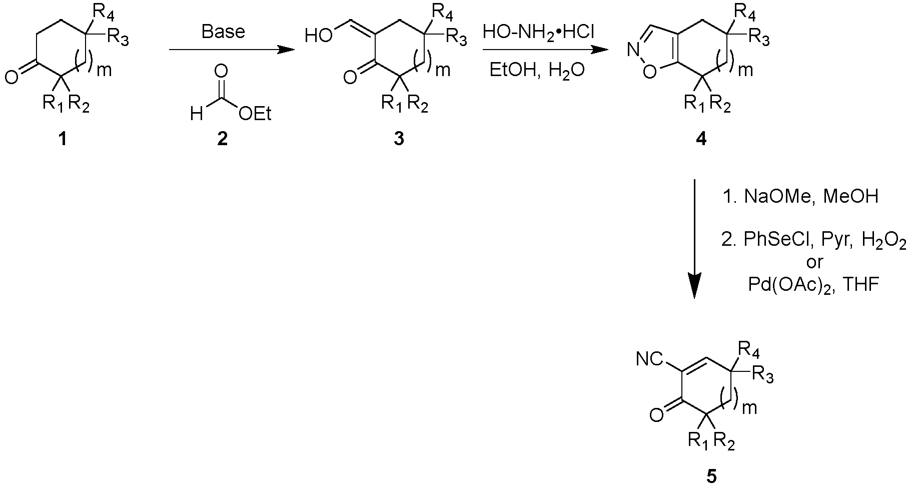

In scheme 1, optionally substituted cyanoenones **5** can be synthesized using a four-step procedure. Formylation of optionally substituted ketones **1** affords **3,** followed by ring closure affords isoxazole **4.** Ring opening followed by oxidation affords cyanoenones **5.**

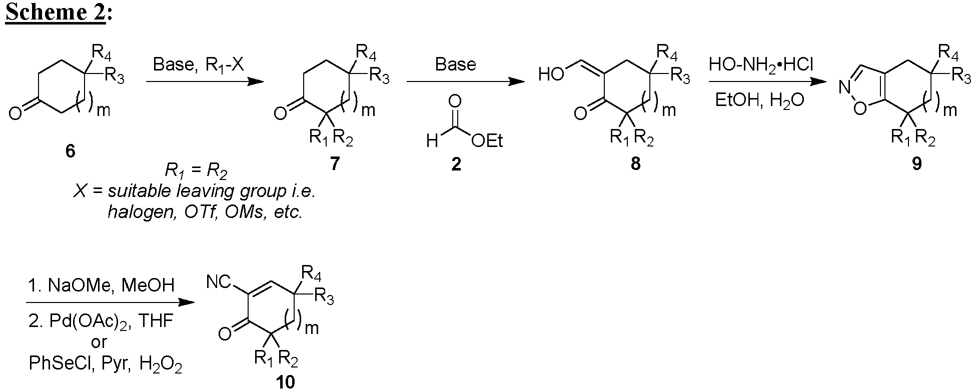

In scheme 2, optionally substituted cyanoenones **10** can be synthesized using a five-step procedure. Optionally substituted ketones **6** can be alkylated under basic conditions to afford **7.** Formylation followed by ring closure affords isoxazole **9.** Ring opening followed by oxidation affords cyanoenones **10.**

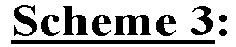

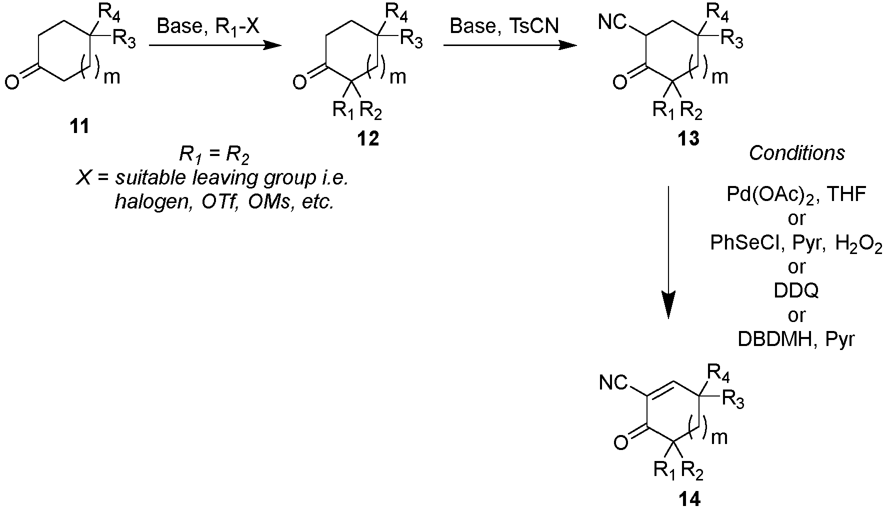

In scheme 3, optionally substituted cyanoenones **14** can be synthesized using a three-step procedure. Optionally substituted ketones **11** can be alkylated under basic conditions to afford **12.** Alpha cyanation, followed by oxidation affords cyanoenones **14.**

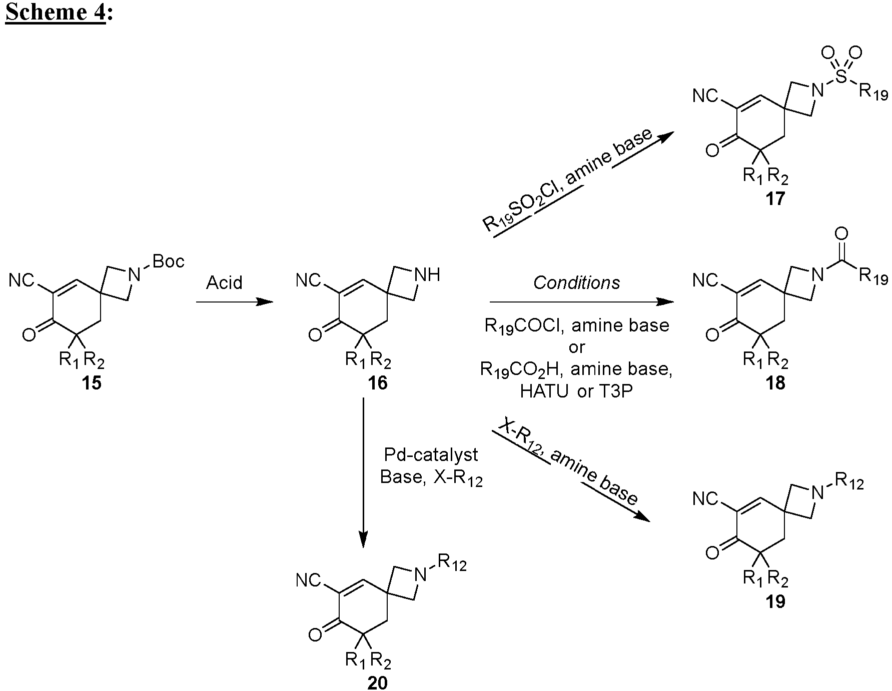

In scheme 4, the spiro-azetidine is used for illustrative purposes. Other amines can be functionalized using analogous chemistry. When R₃ and R₄ are a spirocyclic amine, the amine substituent can be functionalized using a two-step procedure. Protected amine **15** can be deprotected under acidic conditions to afford amine **16.** Amine 16 can be used in a multitude of reactions. Sulfonylation using sulfonyl chlorides afford sulfonamides **17.** Alternatively, amides **18** can be formed with either acyl chlorides or carboxylic acids. Alternatively, alkylation with alkyl halides affords alkyl amines **19.** Finally, a palladium-catalyzed C-N coupling reaction with aryl halides affords aryl amines **20.**

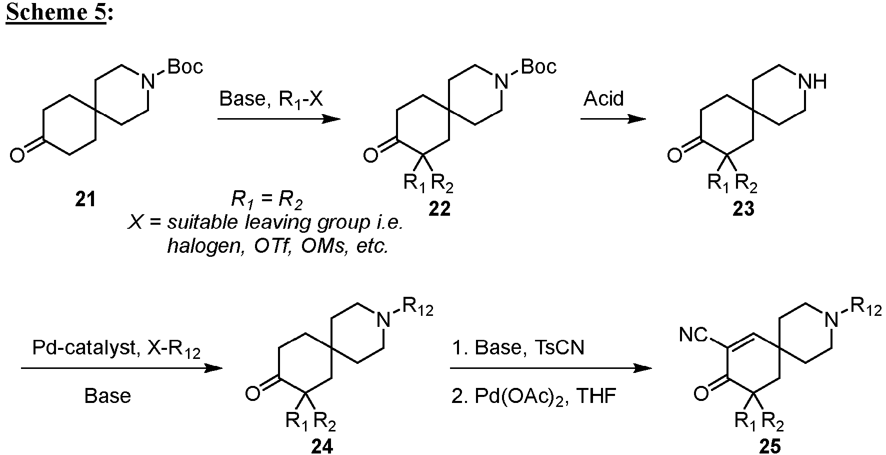

In scheme 5, optionally substituted spirocyclic cyanoenones **25** can be synthesized using a 5-step procedure. Optionally substituted spirocyclic ketones **21** can be alkylated under basic conditions to afford **22.** The amine can be deprotected under acidic conditions to afford amine **23.** A palladium-catalyzed C-N coupling reaction with aryl halides affords aryl amines **24.** Alpha cyanation, followed by oxidation affords spirocyclic cyanoenones **25.**

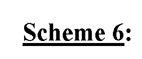

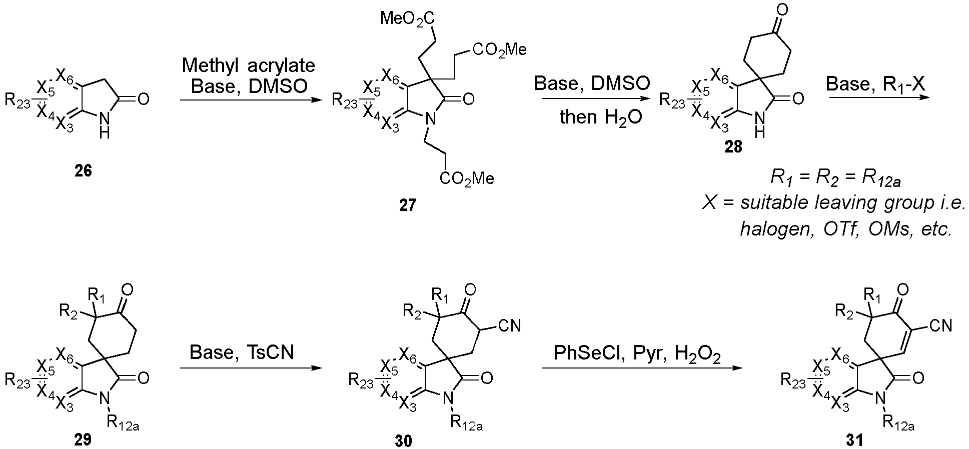

In scheme 6, optionally substituted spirocyclic indolinones **31** can be synthesized using a 5-step procedure. Optionally substituted indolinones **26** can be alkylated under basic conditions with methyl acrylate to afford **27,** which can be cyclized under basic conditions to give **28.** The ketone can be alkylated under basic conditions to afford **29.** Alpha cyanation, followed by oxidation affords spirocyclic indolinones **31.**

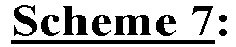

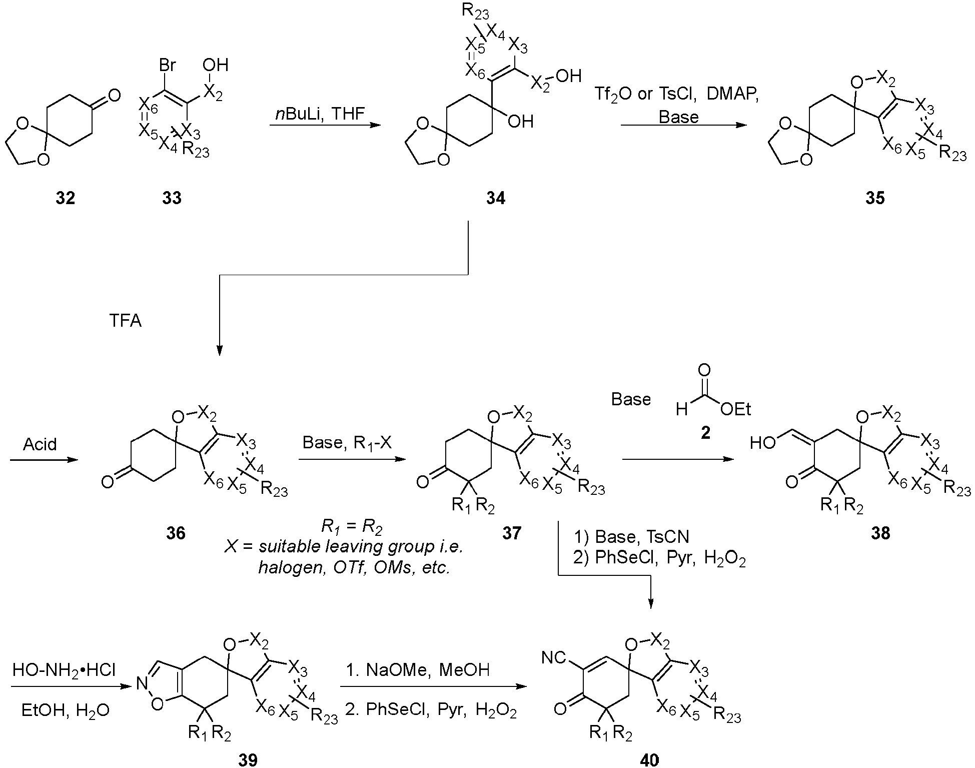

In scheme 7, optionally substituted spirocyclic dihydroisobenzofurans **40** can be synthesized using an 8-step procedure. Lithiation of optionally substituted bromophenyl **33** with ketone **32** affords diol **34,** which can be closed to a spirocyclic dihydroisobenzofuran under basic conditions with TsCl or Tf₂O to give **35.** Deprotection under acidic conditions affords ketone **36.** Alternatively, diol **34** can be closed and deprotected in one pot using TFA to afford ketone **36.** Ketone **36** can be alkylated under basic conditions to afford **37.** Formylation followed by ring closure affords isoxazole **39.** Ring opening followed by oxidation affords spirocyclic dihydroisobenzofurans **40.** Optionally, alpha cyanation of **37,** followed by oxidation affords spirocyclic dihydroisobenzofurans **40.** Alternatively, to **32,** ketone with R1 and R2 preinstalled as methyl can be used omitting the transformation from **36** to **37.**

### SYNTHESIS OF INTERMEDIATES

Racemic compounds purified by reverse phase or normal phase chromatography were further purified by chiral SFC. Where mentioned, enantiomer 1 refers to the first eluting peak and enantiomer 2 refers to the second eluting peak. Otherwise, compounds are achiral or racemic.

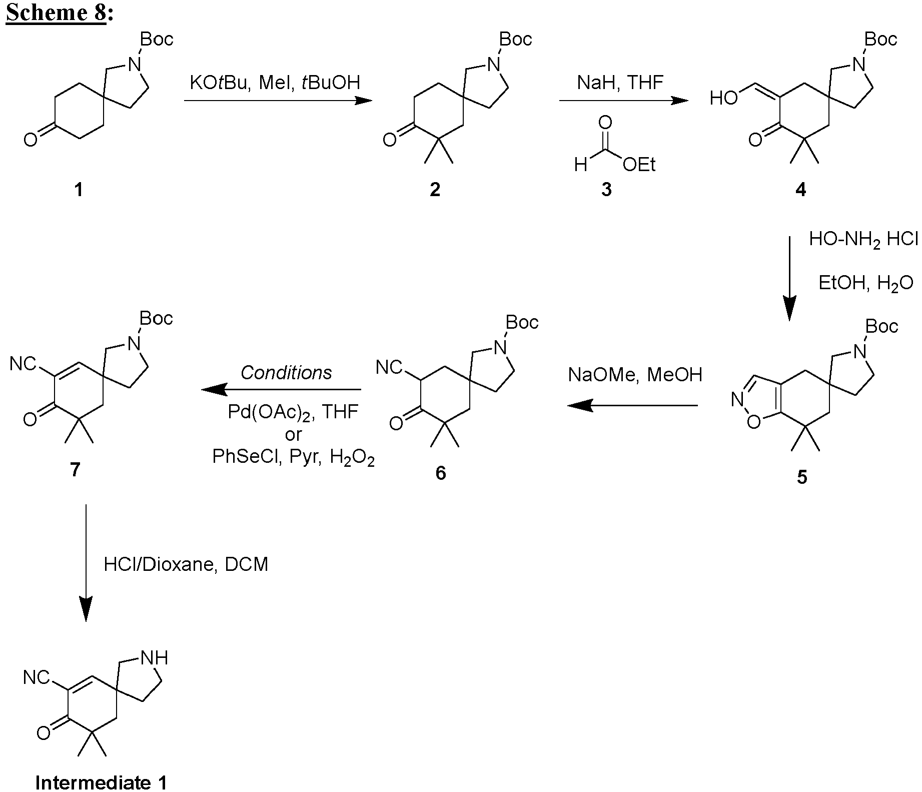

### Intermediate 1: 9,9-dimethyl-8-oxo-2-azaspiro[4.5]dec-6-ene-7-carbonitrile, HCl

**Step 1:** *Tert-*butyl 8-oxo-2-azaspiro[4.5]decane-2-carboxylate (829 mg, 3.27 mmol) was dissolved in tBuOH (16 mL). Potassium tert-butoxide (918 mg, 8.18 mmol) was added slowly under a nitrogen atmosphere, and the mixture was stirred at room temperature for 1 h. Methyl iodide (0.430 mL, 6.87 mmol) was added dropwise, and the mixture was stirred at room temperature overnight. Water (20 mL) was added and the aqueous phase was extracted with EtOAc (3x). The combined organic layers were washed with brine, dried over anhydrous sodium sulfate, filtered, and concentrated under reduced pressure. The residue was purified by column chromatography on silica (0-50% Et₂O/Hexanes) to give *tert*-butyl 7,7-dimethyl-8-oxo-2-azaspiro[4.5]decane-2-carboxylate as an oil. MS: 226 (M + H - *t*Bu).

**Step 2:** To a suspension of sodium hydride (151 mg, 3.76 mmol) in THF (5.5 mL) at 0 °C was added a solution of *tert*-butyl 7,7-dimethyl-8-oxo-2-azaspiro[4.5]decane-2-carboxylate (921 mg, 3.27 mmol) in THF (5.5 mL). The reaction mixture was stirred for 20 minutes at 0 °C. Ethyl formate (0.793 mL, 9.82 mmol) was then added and the reaction mixture was allowed to warm to room temperature and stirred overnight. To the mixture was added 0.5 eq of NaH, and the reaction was stirred for 2 h at room temperature. The mixture was quenched with sat. NH₄Cl (aq) and extracted with EtOAc (2x). The combined organic layers were dried over anhydrous sodium sulfate, filtered, and concentrated under reduced pressure to afford *tert*-butyl (Z)-9-(hydroxymethylene)-7,7-dimethyl-8-oxo-2-azaspiro[4.5]decane-2-carboxylate as a liquid, which was used without further purification. MS: 254 (M + H - *t*Bu).

**Step 3:** To a solution of *tert*-butyl (Z)-9-(hydroxymethylene)-7,7-dimethyl-8-oxo-2-azaspiro[4.5]decane-2-carboxylate (1.01 g, 3.27 mmol) in 10:1 EtOH:H₂O (8 mL) was added a solution of hydroxylamine hydrochloride (0.25 g, 3.6 mmol) in 10:1 EtOH:H₂O (8 mL). The reaction mixture was stirred at 40 °C for 5 h. The mixture was cooled to room temperature and concentrated under reduced pressure. The residue was basified with sat. NaHCO₃ (aq) and extracted with EtOAc (3x). The combined organic layers were washed with brine, dried over anhydrous sodium sulfate, filtered, and concentrated under reduced pressure to give *tert*-butyl 7,7-dimethyl-6,7-dihydro-4H-spiro[benzo[d]isoxazole-5,3'-pyrrolidine]-1'-carboxylate as an oily solid, which was used without further purification. MS: 251 (M + H - *t*Bu).

**Step 4:** To a solution of *tert*-butyl 7,7-dimethyl-6,7-dihydro-4H-spiro[benzo[d]isoxazole-5,3'-pyrrolidine]-1'-carboxylate (1.0 g, 3.3 mmol) in MeOH (16 mL) was added sodium methoxide (30% in MeOH, 1.87 mL, 9.79 mmol). The reaction mixture was stirred at room temperature overnight. The mixture was concentrated under reduced pressure, diluted with EtOAc, and neutralized with 1 M aq HCl (9.8 mL). The layers were separated, and the aqueous layer was extracted with EtOAc. The combined organic layers were washed with brine, dried over anhydrous sodium sulfate, filtered, and concentrated under reduced pressure. The residue was purified by column chromatography on silica (0-100% Et₂O/Hexanes) to give *tert*-butyl 9-cyano-7,7-dimethyl-8-oxo-2-azaspiro[4.5]decane-2-carboxylate as a foam. MS: 251 (M + H - tBu). **Step 5:** To a solution of *tert*-butyl 9-cyano-7,7-dimethyl-8-oxo-2-azaspiro[4.5]decane-2-carboxylate (843 mg, 2.75 mmol) in THF (5.5 mL) at room temperature was added palladium(II) acetate (618 mg, 2.75 mmol). The mixture was stirred at room temperature overnight. The mixture was concentrated under reduced pressure, and then diluted with DCM (5 mL) and 1:1 water:brine (5 mL). The organic layer was collected via a phase separator and concentrated under reduced pressure. The residue was purified by column chromatography on silica (0-80% Et₂O/Hexanes) to give *tert*-butyl 7-cyano-9,9-dimethyl-8-oxo-2-azaspiro[4.5]dec-6-ene-2-carboxylate as an oily foam. MS: 249 (M + H - tBu).

**Alternate Step 5:** To a solution of pyridine (34 µL, 0.42 mmol) in DCM (0.5 mL) at 0 °C was added a solution of phenylselenyl chloride (80 mg, 0.42 mmol) in DCM (0.5 mL). The reaction mixture was stirred for 20 minutes at 0 °C before the addition of a solution of *tert*-butyl 9-cyano-7,7-dimethyl-8-oxo-2-azaspiro[4.5]decane-2-carboxylate (64 mg, 0.209 mmol) in DCM (1 mL). The reaction mixture was stirred for 1 h at 0 °C, and was subsequently washed with 1 M aqueous HCl (2 mL). The phases were separated, and the organic phase was treated with hydrogen peroxide (30% in water, 430 µL, 4.2 mmol) at 0 °C. The reaction mixture was stirred vigorously for 15 mins, then the phases were separated and the organic phase was concentrated under reduced pressure. The residue was purified by column chromatography on silica (0-100% Et₂O/Hexanes) to give *tert*-butyl 7-cyano-9,9-dimethyl-8-oxo-2-azaspiro[4.5]dec-6-ene-2-carboxylate as an oily foam. MS: 249 (M + H - *t*Bu).

**Alternative Step 5:** To a solution of compound 5 (40.0 g, 124 mmol, 1.0 *eq*) in Tol. (0.4 L) at 25 °C was added DDQ (42.2 g, 186 mmol, 1.5 *eq*)*.* The reaction was stirred at 110°C for 3 hours. The reaction mixture was filtered and concentrate under reduced pressure to give a residue. Purification by column chromatography (SiO₂, Petroleum ether : Ethyl acetate = 100 : 1 to 50 : 1) provided $ (23.0 g, 67.5 mmol, 54.4% yield, 94% purity) as a light yellow solid. ¹HNMR: ET33537-23-P1A1 (400 MHz, CDCl₃) *δ* 7.61 (s, 1 H), 3.72-3.79 (m, 3 H), 3.62 (s, 1 H), 3.21-3.26 (m, 1 H), 3.15-3.19 (m, 1 H), 2.01-2.05 (m, 1 H), 1.82-.86 (m, 1 H), 1.48 (s, 9 H), 1.30 (s, 3 H), 1.20 (s, 3 H).

**Step 6:** *Tert*-butyl 7-cyano-9,9-dimethyl-8-oxo-2-azaspiro[4.5]dec-6-ene-2-carboxylate (443 mg, 1.45 mmol) was dissolved in DCM (14 mL). HCl (4.0 M in dioxane, 3.64 mL, 14.5 mmol) was added, and the mixture was stirred at room temperature overnight. The mixture was concentrated under reduced pressure to give 9,9-dimethyl-8-oxo-2-azaspiro[4.5]dec-6-ene-7-carbonitrile, HCl as a foam, which was used without further purification. MS: 205 (M + H).

**Table 1: The following intermediates were prepared using a similar procedure as described above for intermediate 1. For the oxidation conditions, 1 = Palladium(II) acetate and 2 = PhSeCl, Pyridine, H₂O₂**

| **Int.** # | **Structure** | **Compound Name** | **Oxidation Conditions** | **Exact Mass [M+H]** |
|---|---|---|---|---|
| **2** | 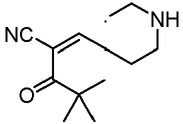 | 10,10-dimethyl-9-oxo-3-azaspiro[5.5]undec-7-ene-8-carbonitrile, HCl | 1 | 219 |
| **3** | 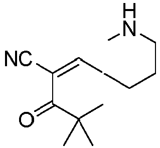 | 10,10-dimethyl-9-oxo-2-azaspiro[5.5]undec-7-ene-8-carbonitrile, HCl | 1 | 219 |
| **4** | 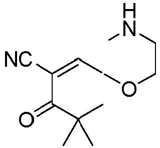 | 10,10-dimethyl-9-oxo-1-oxa-4-azaspiro[5.5]undec-7-ene-8-carbonitrile, HCl | 2 | 221 |

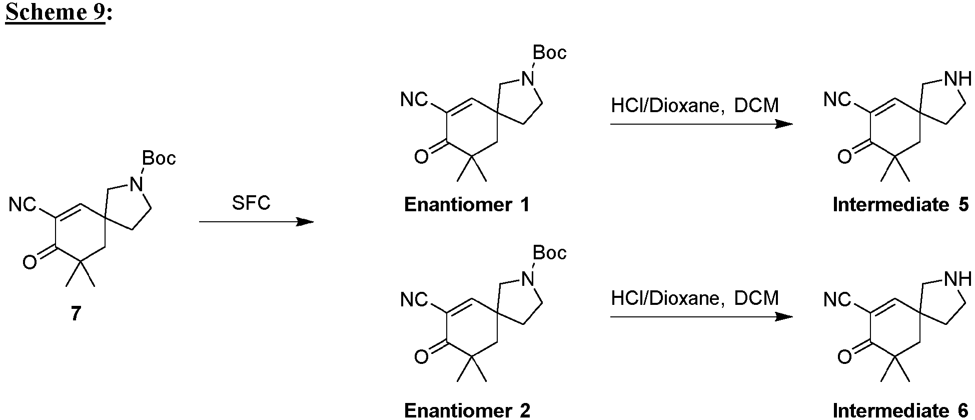

### Intermediate 5: 9,9-dimethyl-8-oxo-2-azaspiro[4.5]dec-6-ene-7-carbonitrile, HCl

### Intermediate 6: 9,9-dimethyl-8-oxo-2-azaspiro[4.5]dec-6-ene-7-carbonitrile, HCl

**Step 1:** *Tert*-butyl 7-cyano-9,9-dimethyl-8-oxo-2-azaspiro[4.5]dec-6-ene-2-carboxylate (1.1 g, 3.6 mmol) was purified by chiral SFC (OJ-H column, 15% MeOH/85% CO₂ with 0.1% NH₄OH modifier) to afford two products as solids:
*Tert*-butyl 7-cyano-9,9-dimethyl-8-oxo-2-azaspiro[4.5]dec-6-ene-2-carboxylate (enantiomer 1). MS: 249 (M + H - *t*Bu).
*Tert*-butyl 7-cyano-9,9-dimethyl-8-oxo-2-azaspiro[4.5]dec-6-ene-2-carboxylate (enantiomer 2). MS: 249 (M + H - *t*Bu).

**Step 2:** To *tert*-butyl 7-cyano-9,9-dimethyl-8-oxo-2-azaspiro[4.5]dec-6-ene-2-carboxylate (enantiomer 1, 405 mg, 1.33 mmol) dissolved in DCM (10 mL) was added HCl (4.0 M in dioxane, 3.33 mL, 13.3 mmol). The mixture was stirred at room temperature overnight. The mixture was concentrated under reduced pressure to give 9,9-dimethyl-8-oxo-2-azaspiro[4.5]dec-6-ene-7-carbonitrile, HCl (Intermediate 5) as a solid which was used without further purification. MS: 205 (M + H).

Note that enantiomer 2 was deprotected using the same procedure described in step 2 and was isolated as Intermediate 6.

**Table 2: The following intermediates were prepared using a similar procedure as described above for intermediate 5 and 6**

| **Int.** | Structure | IUPAC Name | SFC Conditions | Exact Mass [M + H] |
|---|---|---|---|---|
| 7 | 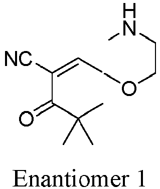 | 10,10-dimethyl-9-oxo-1-oxa-4-azaspiro[5.5]undec-7-ene-8-carbonitrile, HCl | SFC (column: DAICEL CHIRALPAK AS (250mm x 50mm, 10um); | 221 |
| **8** | 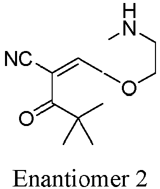 | 10,10-dimethyl-9-oxo-1-oxa-4-azaspiro[5.5]undec-7-ene-8-carbonitrile, HCl | SFC (column: DAICEL CHIRALPAK AS (250mm x 50mm, 10um); | 221 |

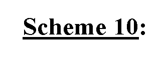

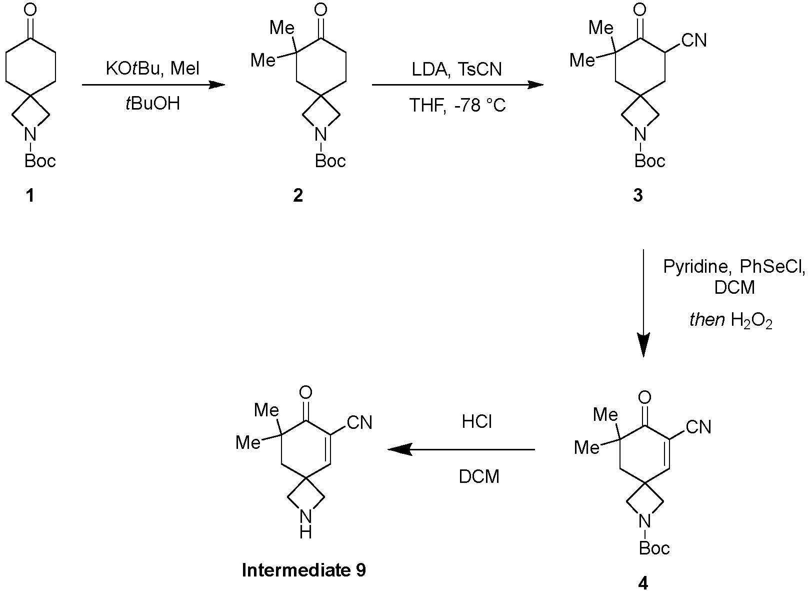

### Intermediate 9: 8,8-dimethyl-7-oxo-2-azaspiro[3.5]non-5-ene-6-carbonitrile, HCl

**Step 1:** To a solution of *tert*-butyl 7-oxo-2-azaspiro[3.5]nonane-2-carboxylate **(1.25 g, 5.22 mmol)** in *t*BuOH (26 mL) was added potassium *tert*-butoxide (1.46 g, 13.1 mmol) at room temperature. The mixture was stirred at room temperature for 1 h. Methyl iodide (0.685 mL, 11.0 mmol) was added to the mixture, and the reaction was stirred overnight at room temperature. The reaction was quenched with water (100 mL) and the mixture was extracted with EtOAc (3 x 75 mL). The combined organic layers were washed with brine (100 mL), dried over anhydrous sodium sulfate, filtered, and concentrated under reduced pressure. The residue was purified by column chromatography on silica (0-30% EtOAc/Hexanes) to afford *tert*-butyl 6,6-dimethyl-7-oxo-2-azaspiro[3.5]nonane-2-carboxylate as a solid. MS: 212 (M + H - *t*Bu).

**Step 2:** To LDA **(2.0 M in THF, 2.32 mL, 4.64 mmol) at -78** °C was added a solution of *tert-*butyl 6,6-dimethyl-7-oxo-2-azaspiro[3.5]nonane-2-carboxylate (620 mg, 2.32 mmol) in THF (4 mL) dropwise. The mixture was stirred at -78 °C for 30 minutes. A solution of 4-methylbenzenesulfonyl cyanide (420 mg, 2.32 mmol) in THF (3 mL) was added dropwise and the mixture was stirred for an additional 30 minutes at -78 °C. The reaction was quenched with 2 M NH₄OH (1.2 mL) and let warm to room temperature. The mixture was then neutralized with 1 M aq HCl to a pH of ~7, and extracted with EtOAc (3 x 5 mL). The combined organic layers were dried over anhydrous sodium sulfate, filtered, and concentrated under reduced pressure. The residue was purified by column chromatography on silica (0-30% EtOAc/Hexanes) to afford *tert*-butyl 8-cyano-6,6-dimethyl-7-oxo-2-azaspiro[3.5]nonane-2-carboxylate as a solid. MS: 237 (M + H -*t*Bu).

**Step 3:** To a solution of pyridine (314 µL, 3.90 mmol) in DCM (2.5 mL) at 0 °C was added a solution of phenylselenyl chloride (747 mg, 3.90 mmol) in DCM (2.5 mL). The reaction mixture was stirred for 20 minutes at 0 °C before a solution of *tert*-butyl 8-cyano-6,6-dimethyl-7-oxo-2-azaspiro[3.5]nonane-2-carboxylate (570 mg, 1.95 mmol) in DCM (5 mL) was added. The reaction mixture was stirred for 1 h at 0 °C, then washed with 1 M aqueous HCl (8 mL) then water (16 mL). The phases were separated, and the organic phase was treated with hydrogen peroxide (30% in water, 4.0 mL, 39 mmol) at 0 °C. The reaction mixture was stirred vigorously for 15 minutes, then the phases were separated and the organic phase was concentrated under reduced pressure. The residue was purified by column chromatography on silica (0-40% EtOAc/Hexanes) to give *tert*-butyl 6-cyano-8,8-dimethyl-7-oxo-2-azaspiro[3.5]non-5-ene-2-carboxylate as a solid. MS: 235 (M + H - tBu).

**Step 4:** To a solution of *tert*-butyl 6-cyano-8,8-dimethyl-7-oxo-2-azaspiro[3.5]non-5-ene-2-carboxylate (465 mg, 1.60 mmol) in DCM (16 mL) was added HCl (4 M in THF, 4.0 mL, 16 mmol). The mixture was stirred at room temperature for 3 h. The reaction was concentrated under reduced pressure to give 8,8-dimethyl-7-oxo-2-azaspiro[3.5]non-5-ene-6-carbonitrile as the HCl salt. MS: 191 (M + H).

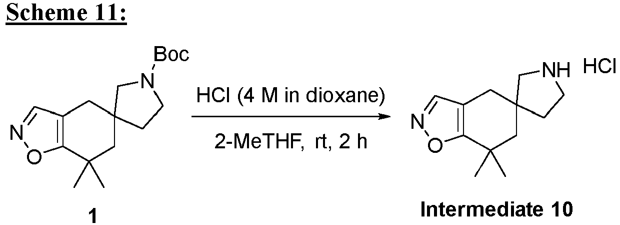

### Intermediate 10: 7,7-dimethyl-6,7-dihydro-4H-spiro[benzo[d]isoxazole-53'-pyrrolidine] hydrochloride

**Step 1:** To a solution of *tert*-butyl 7,7-dimethyl-6,7-dihydro-4*H*-spiro[benzo[*d*]isoxazole-5,3'-pyrrolidine]-1'-carboxylate (2 g, 6.53 mmol) in 2-MeTHF (10 mL) was added hydrochloride (4 M in 1,4-dioxane, 30 mL). The resulted mixture was stirred for 2 hours at 18 °C. After completion of the reaction, the reaction mixture was concentrated in vacuo. The residue was *co-*evaporated with THF (15 mL × 5) to afford 7,7-dimethyl-6,7-dihydro-4*H-*spiro[benzo[*d*]isoxazole-5,3'-pyrrolidine] hydrochloride (1.585 g, 6.53 mmol, 100% yield) as a light yellow solid. MS ESI calculated for C₁₂H₁₈N₂O [M + H]⁺ 207.14 found 207.10.¹H-NMR (300 MHz, DMSO-*d₆*) δ 9.39 (s, 2H), 8.38 (s, 1H), 3.32-3.14 (m, 2H), 3.11-3.01 (m, 1H), 2.96-2.83 (m, 1H), 2.57 (d, *J =* 15.7 Hz, 1H), 2.42 (d, *J =* 15.7 Hz, 1H), 1.91-1.75 (m, 4H), 1.29 (s, 3H), 1.26 (s, 3H).

**Table 3: The following intermediate was prepared using a similar procedure as described above for intermediate 10.**

| **Int. #** | **Structure** | **Compound Name** | **Exact Mass [M+H]⁺** |
|---|---|---|---|
| **11** | 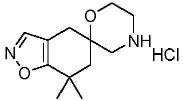 | 7,7-dimethyl-6,7-dihydro-4H-spiro[benzo[d]isoxazole-5,2'-morpholine] hydrochloride | 223 |
| **12** | 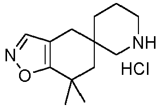 | 7,7-dimethyl-6,7-dihydro-4H-spiro[benzo[d]isoxazole-5,3'-piperidine] hydrochloride | 221 |

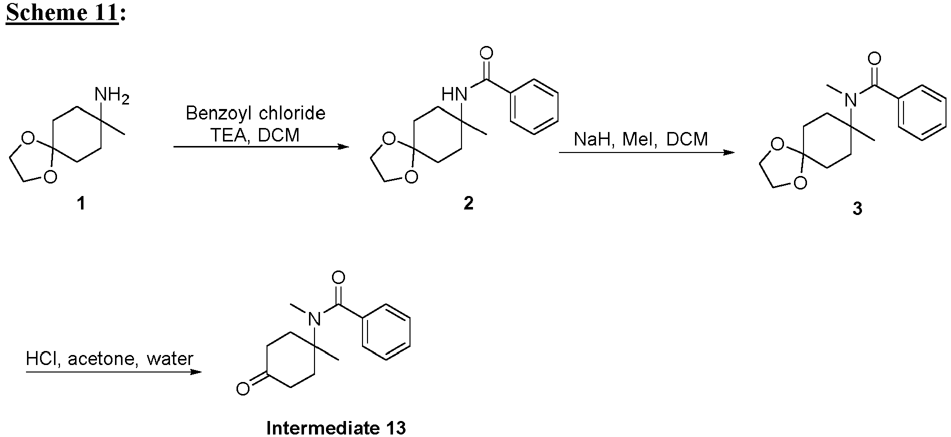

### Intermediate 13: N-methyl-N-(1-methyl-4-oxocyclohexyl)benzamide

**Step 1:** To a solution of 8-methyl-1,4-dioxaspiro[4.5]decan-8-amine (1.89 g, 11.0 mmol) in DCM (10 mL) was added triethylamine (6.12 mL, 44.1 mmol) and benzoyl chloride (2.56 mL, 22.1 mmol). The reaction was stirred overnight at room temperature. The mixture was quenched with aqueous HCl (1 M, 10 mL), and extracted with DCM (2x). The combined organic layers were dried over anhydrous sodium sulfate, filtered, and concentrated under reduced pressure. The residue was purified by column chromatography on silica (0-70% Et₂O/hexanes) to afford *N*-(8-methyl-1,4-dioxaspiro[4.5]decan-8-yl)benzamide as a solid. MS: 276 (M + H).

**Step 2:** To a solution of *N*-(8-methyl-1,4-dioxaspiro[4.5]decan-8-yl)benzamide (2.76 g, 10.0 mmol) in DCM (10 mL) was added sodium hydride (1.00 g, 25.1 mmol) and iodomethane (2.18 mL, 35.1 mmol). The reaction mixture was stirred overnight at room temperature. The reaction mixture was quenched with aqueous HCl (1 M, 10 mL) and extracted with DCM (2x). The combined organic layers were dried over anhydrous sodium sulfate, filtered, and concentrated under reduced pressure to afford *N*-methyl-*N*-(8-methyl-1,4-dioxaspiro[4.5]decan-8-yl)benzamide as a solid, which was used without further purification. MS: 290 (M + H).

**Step 3:** A solution of *N*-methyl-*N*-(8-methyl-1,4-dioxaspiro[4.5]decan-8-yl)benzamide (2.9 g, 10 mmol) in acetone (11 mL), HCl (37 wt. % in H₂O, 5.5 mL), and water (5.5 mL) was stirred at room temperature overnight. The reaction mixture was neutralized with aqueous NaOH (2 M) and extracted with EtOAc (2x). The combined organic layers were dried over anhydrous sodium sulfate, filtered, and concentrated under reduced pressure. The residue was purified by column chromatography on silica (0-100% Et₂O/hexanes) to afford *N*-methyl-*N*-(1-methyl-4-oxocyclohexyl)benzamide as a solid. MS: 246 (M + H).

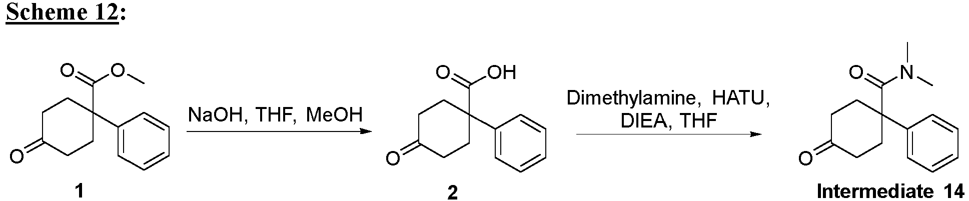

### Intermediate 14: N,N-dimethyl-4-oxo-1-phenylcyclohexane-1-carboxamide

**Step 1:** To a solution of methyl 4-oxo-1-phenylcyclohexane-1-carboxylate (1.4 g, 6.0 mmol) in THF (18 mL) and MeOH (9 mL) was added sodium hydroxide (2.0 M in water, 9.0 mL, 18 mmol). The reaction mixture was stirred overnight at room temperature. The reaction mixture was then quenched with saturated aqueous NH₄Cl (10 mL) and extracted with EtOAc (2x). The combined organic layers were dried over anhydrous sodium sulfate, filtered, and concentrated under reduced pressure to afford 4-oxo-1-phenylcyclohexane-1-carboxylic acid as a solid, which was used without further purification. MS: 219 (M + H).

**Step 2:** A solution of 4-oxo-1-phenylcyclohexane-1-carboxylic acid (1.3 g, 6.0 mmol), dimethylamine (2.0 M in THF, 4.5 mL, 9.0 mmol), N,N-diisopropylethylamine (2.1 mL, 12 mmol), and HATU (2.85 g, 7.50 mmol) in DMF (7.5 mL) was stirred at room temperature overnight. The reaction mixture was diluted with EtOAc, washed with aqueous HCl (1 M), saturated aqueous NaHCO₃, and brine. The organic layer was dried over anhydrous sodium sulfate, filtered, and concentrated under reduced pressure. The residue was purified by column chromatography on silica (20-90% Et₂O/hexanes) to afford *N*,*N-*dimethyl-4-oxo-1-phenylcyclohexane-1-carboxamide as a solid. MS: 246 (M + H).

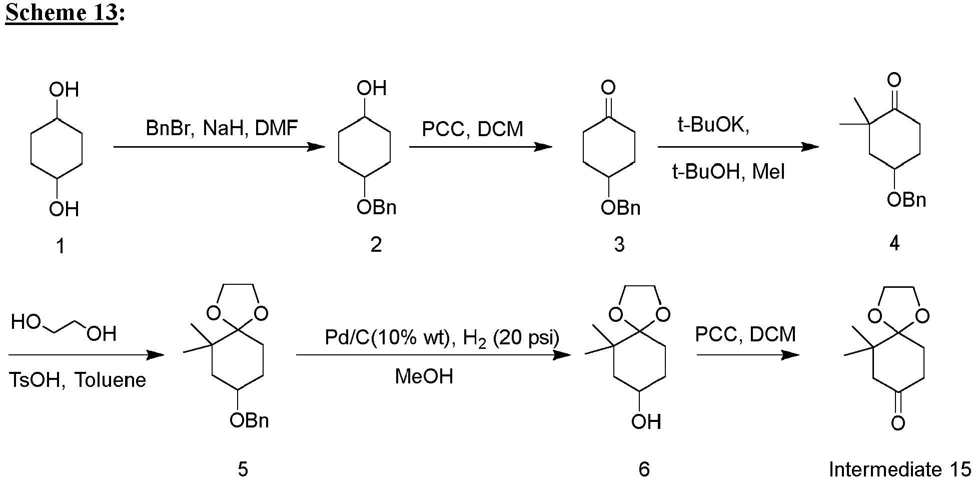

### Intermediate 15: 6,6-dimethyl-1,4-dioxaspiro[4.5]decan-8-one

**Step 1:** To a solution of cyclohexane-1,4-diol (100 g, 860 mmol) in DMF (2.5 L) was added NaH (68.9 g, 1.72 mol) at 0°C. Benzyl bromide (161 g, 946 mmol) was then added dropwise and the resulting solution stirred at ambient temperature for 12 h. The reaction mixture was quenched by addition of a saturated aqueous solution of ammonium chloride and extracted with ethyl acetate (3x). The residue was purified by silica gel chromatography, eluting with a gradient of ethyl acetate : petroleum ether (1 : 50 to 1 : 3) to yield 4-(benzyloxy)cyclohexan-1-ol. 1H NMR (400 MHz, CDCl₃) δ 7.23 - 7.39 (m, 5H), 4.53 (d, J = 5.6 Hz, 2H), 3.63 - 3.79 (m, 1H), 3.34 - 3.52 (m, 1H), 1.96 - 2.10 (m, 2H), 1.85 - 1.94 (m, 1H), 1.63 - 1.77 (m, 2H), 1.54 - 1.62 (m, 1H), 1.24 - 1.48 (m, 3H), 1.24 - 1.48 (m, 1H).

**Step 2:** To a solution of 4-(benzyloxy)cyclohexan-1-ol (7.00 g, 33.9 mmol) in DCM (140.0 mL) was added PCC (10.9 g, 50.9 mmol) and celatom^{®} (10.9 g) at ambient temperature. The resulting mixture was stirred for 12 h. The reaction mixture was filtered and washed with a saturated sodium bicarbonate solution (2x). The organic layer was concentrated under reduced pressure and the residue was purified by silica gel chromatography, eluting with a gradient of ethyl acetate : petroleum ether (1 : 5 to 1 : 3) to afford 4-(benzyloxy)cyclohexan-1-one. MS: 205 (M + H).

**Step 3:** To a solution of 4-(benzyloxy)cyclohexan-1-one (100 g, 489 mmol) in *t*-BuOH (1.0 L) was added potassium tert-butoxide (137 g, 1.22 mol). The resulting mixture was stirred at ambient temperature for 1 h. Iodomethane (145 g, 1.03 mol) was added dropwise and the mixture was stirred at ambient temperature for 1 h. Then the mixture was quenched with saturated aqueous NH₄Cl (5.0 L) and extracted with ethyl acetate (3x). The combined organic layers were washed with brine (1.0 L) and concentrated under reduced pressure. The residue was purified by silica gel chromatography, eluting with a gradient of ethyl acetate : petroleum ether (1 : 100 to 5 : 100) to yield 4-(benzyloxy)-2,2-dimethylcyclohexan-1-one. MS: 233 (M + H).

**Step 4:** To a solution of 4-(benzyloxy)-2,2-dimethylcyclohexan-1-one (132 g, 568 mmol) in toluene (2.0 L) was added 4-methylbenzenesulfonic acid (7.81 g, 45.3 mmol) and ethane-1,2-diol (105 g, 1.70 mol) at ambient temperature. The resulting mixture was refluxed under azeotropic conditions for 12 h. After cooling, the mixture was poured into a saturated sodium bicarbonate solution (3.0 L) and extracted with ethyl acetate (3x). The combined organic layers were washed with brine (800 mL), dried over anhydrous sodium sulfate and filtered. The filtrate was concentrated under reduced pressure and the residue was purified by preparative HPLC (ACN/H2O with 10mM NH₄HCO₃ modifier) to yield 8-(benzyloxy)-6,6-dimethyl-1,4-dioxaspiro[4.5]decane. MS: 277 (M + H).

**Step 5:** To a solution of 8-(benzyloxy)-6,6-dimethyl-1,4-dioxaspiro[4.5]decane (55.0 g, 199 mmol) in methanol (500 mL) was added Pd/C (10%, 25.0 g) at ambient temperature under nitrogen atmosphere. The resulting mixture was stirred at ambient temperature for 1 h under H₂ atmosphere (20 psi). The mixture reaction was filtrated and the filtrate was concentrated under reduced pressure to afford 6,6-dimethyl-1,4-dioxaspiro[4.5]decan-8-ol which was used in the next step without further purification. MS: 187 (M + H).

**Step 6:** To a solution of 6,6-dimethyl-1,4-dioxaspiro[4.5]decan-8-ol (51.0 g, 273 mmol) in dichloromethane (1.0 L) was added PCC (106 g, 493 mmol) and celatom^{®} (106 g) at ambient temperature. The resulting mixture was stirred for 2 h. The reaction mixture was filtered and washed with a saturated sodium bicarbonate solution (3x). The organic layer was concentrated under reduced pressure and the residue was purified by silica gel chromatography, eluting with a gradient of ethyl acetate : petroleum ether (1 : 50 to 1 : 30) to afford 6,6-dimethyl-1,4-dioxaspiro[4.5]decan-8-one. MS: 185 (M + H).

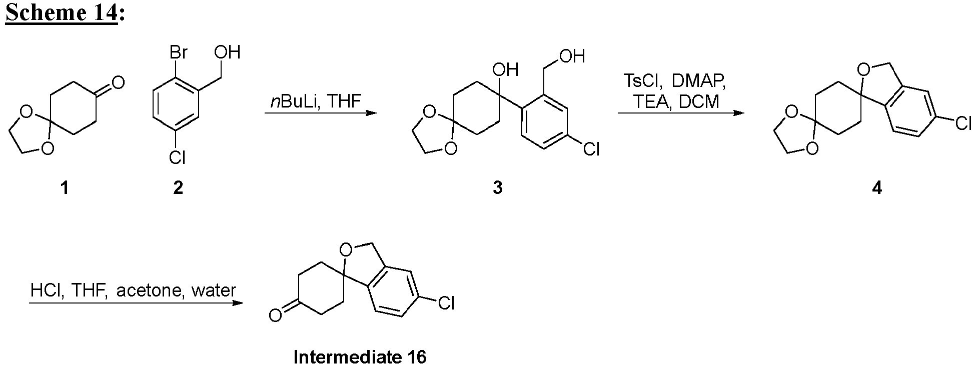

### Intermediate 16: 5'-chloro-3'H-spiro[cyclohexane-1,1'-isobenzofuran]-4-one

**Step 1:** To a solution of (2-bromo-5-chlorophenyl)methanol (2.22 g, 10.0 mmol) in THF (30 mL) at -78 °C was added n-butyllithium (2.5 M in hexanes, 8.0 mL, 20 mmol). The resulting suspension was stirred for 45 minutes at -78 °C. A solution of 1,4-dioxaspiro[4.5]decan-8-one (1.56 g, 10.0 mmol) in THF (3 mL) was added and the reaction mixture was stirred for 1 h at -78 °C. The reaction mixture was then quenched with saturated aqueous NH₄Cl (10 mL), warmed to room temperature, and extracted with EtOAc (3x). The combined organic layers were washed with brine, dried over anhydrous sodium sulfate, filtered, and concentrated under reduced pressure. The residue was purified by column chromatography on silica (0-10% MeOH/DCM) to afford 8-(4-chloro-2-(hydroxymethyl)phenyl)-1,4-dioxaspiro[4.5]decan-8-ol as an oil. MS: 281 (M + H - H₂O).

**Step 2:** To a solution of 8-(4-chloro-2-(hydroxymethyl)phenyl)-1,4-dioxaspiro[4.5]decan-8-ol (1.4 g, 4.7 mmol) in DCM (16 mL) was added *p*-toluenesulfonyl chloride (1.34 g, 7.03 mmol), triethylamine (1.95 mL, 14.1 mmol), and 4-dimethylaminopyridine (0.057 g, 0.47 mmol). The reaction mixture was stirred overnight at room temperature. The reaction mixture was then quenched with aqueous HCl (1 M, 10 mL) and extracted with DCM (2x). The combined organic layers were dried over anhydrous sodium sulfate, filtered, and concentrated under reduced pressure. The residue was purified by column chromatography on silica (0-60% Et₂O/hexanes) to afford 5-chloro-3H-dispiro[isobenzofuran-1,1'-cyclohexane-4',2"-[1,3]dioxolane] as a solid. MS: 281 (M + H).

**Step 3:** A solution of S5-chloro-3H-dispiro[isobenzofuran-1,1'-cyclohexane-4',2"-[1,3]dioxolane] (765 mg, 2.72 mmol) in acetone (3.6 mL), THF (3.6 mL), hydrochloric acid (37 wt. % in H₂O, 1.8 mL), and water (1.8 mL) was stirred overnight at room temperature. The reaction mixture was neutralized with aqueous NaOH (2 M) and extracted with EtOAc (2x). The combined organic layers were dried over anhydrous sodium sulfate, filtered, and concentrated under reduced pressure. The residue was purified by column chromatography on silica (0-70% Et₂O/hexanes) to afford 5'-chloro-3'H-spiro[cyclohexane-1,1'-isobenzofuran]-4-one as a solid. MS: 237 (M + H). ¹H NMR (600 MHz, CDCl₃) δ 7.29 (s, 1H), 7.26 (broad s, 1H), 7.04 (d, *J =* 8.0 Hz, 1H), 5.14 (s, 2H), 2.94 - 2.85 (m, 2H), 2.44 - 2.38 (m, 2H), 2.18 - 2.10 (m, 4H).

**Table 2: The following intermediates were prepared using a similar procedure as described above for intermediate 16.**

| **Int. #** | **Structure** | **Compound Name** | **Exact Mass [M+H]⁺** |
|---|---|---|---|
| **17** | 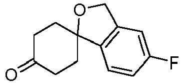 | 5'-fluoro-3'H-spiro[cyclohexane-1,1'-isobenzofuran]-4-one | 203 (M + H - H₂O) |
| **18** | 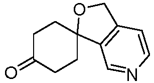 | 1'*H*-spiro[cyclohexane-1,3'-furo[3,4-*c*]pyridin]-4-one | 204 [M + H]⁺ |
| **19** | 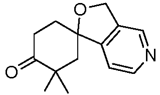 From Intermediate 15 | 3,3-dimethyl-3'*H*-spiro[cyclohexane-1,1'-furo[3,4-*c*]pyridin]-4-one | 232 [M + H]⁺ |

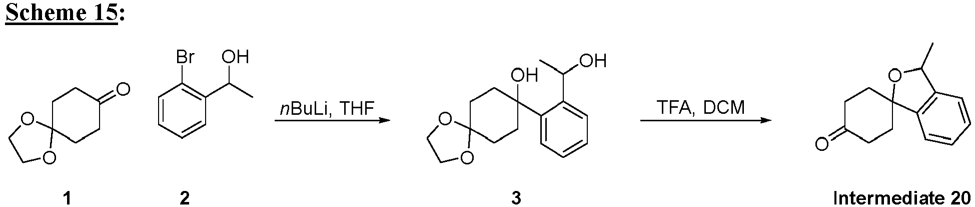

### Intermediate 20: 3'-methyl-3'H-spiro[cyclohexane-1,1'-isobenzofuran]-4-one

**Step 1:** To a solution of 1-(2-bromophenyl)ethan-1-ol (1.97 g, 9.80 mmol) in THF (30 mL) at - 78 °C was added *n*-butyllithium (2.5 M in hexanes, 7.84 mL, 19.6 mmol). The resulting suspension was stirred for 1 h at -78 °C. A solution of 1,4-dioxaspiro[4.5]decan-8-one (1.53 g, 9.80 mmol) in THF (3 mL) was added and the reaction mixture was stirred for 1 h at -78 °C. The reaction was quenched with saturated aqueous NH₄Cl (10 mL) and extracted with EtOAc (3x). The combined organic layers were washed with brine, dried over anhydrous sodium sulfate, filtered, and concentrated under reduced pressure. The residue was purified by column chromatography on silica (0-100% Et₂O/hexanes) to afford 8-(2-(1-hydroxyethyl)phenyl)-1,4-dioxaspiro[4.5]decan-8-ol as an oil. MS: 261 (M + H - H₂O).

**Step 2:** A solution of 8-(2-(1-hydroxyethyl)phenyl)-1,4-dioxaspiro[4.5]decan-8-ol (1.25 g, 4.49 mmol) in DCM (9 mL) and TFA (9 mL) was stirred overnight at room temperature. The reaction mixture was quenched with aqueous NaOH (2 M) and extracted with DCM (3x). The combined organic layers were dried over anhydrous sodium sulfate, filtered, and concentrated under reduced pressure. The residue was purified by column chromatography on silica (0-45% Et₂O/hexanes) to afford 3'-methyl-3'H-spiro[cyclohexane-1,1'-isobenzofuran]-4-one as a solid. MS: 217 (M + H).

**Table 3: The following intermediate was prepared using a similar procedure as described above for intermediate 20.**

| **Int. #** | **Structure** | **Compound Name** | **Exact Mass [M+H]⁺** |
|---|---|---|---|
| **21** | 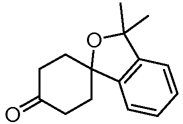 | 3',3'-dimethyl-3'H-spiro[cyclohexane-1,1'-isobenzofuran]-4-one | 231 |

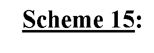

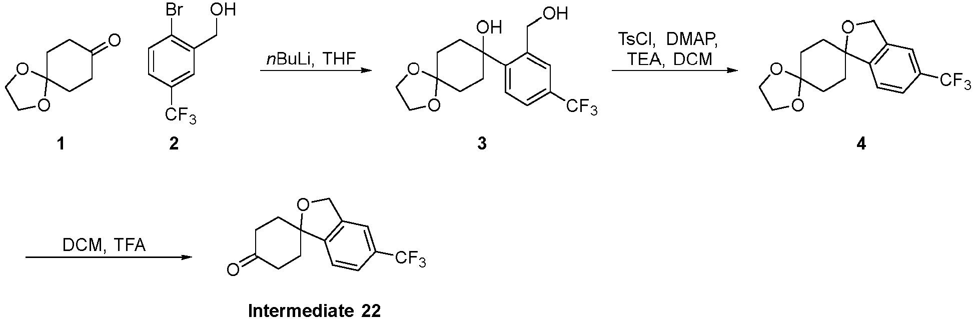

### Intermediate 22: 5'-(trifluoromethyl)-3'H-spiro[cyclohexane-1,1'-isobenzofuran]-4-one

**Step 1:** To a solution of (2-bromo-5-(trifluoromethyl)phenyl)methanol (3 g, 11.8 mmol) in THF (35 mL) at -78 °C was added n-butyllithium (2.5 M in hexanes, 9.41 mL, 23.5 mmol). The resulting suspension was stirred for 1 h at -78 °C. A solution of 1,4-dioxaspiro[4.5]decan-8-one (1.84 g, 11.8 mmol) in THF (4 mL) was added and the reaction mixture was stirred for 3 h at -78 °C. The reaction mixture was quenched with saturated aqueous NH₄Cl (10 mL) and extracted with EtOAc (3x). The combined organic layers were washed with brine, dried over anhydrous sodium sulfate, filtered, and concentrated under reduced pressure. The residue was purified by column chromatography on silica (0-100% Et₂O/hexanes) to afford 8-(2-(hydroxymethyl)-4-(trifluoromethyl)phenyl)-1,4-dioxaspiro[4.5]decan-8-ol as an oil. MS: 315 (M + H - H₂O).

**Step 2:** To a solution of 8-(2-(hydroxymethyl)-4-(trifluoromethyl)phenyl)-1,4-dioxaspiro[4.5]decan-8-ol (2.09 g, 6.29 mmol) in DCM (21 mL) was added*p*-toluenesulfonyl chloride (1.8 g, 9.4 mmol), triethylamine (2.6 mL, 18.8 mmol), and 4-dimethylaminopyridine (77 mg, 0.63 mmol). The reaction mixture was stirred overnight at room temperature. The reaction mixture was then quenched with aqueous HCl (1 M, 10 mL) and extracted with DCM (2x). The combined organic layers were dried over anhydrous sodium sulfate, filtered, and concentrated under reduced pressure. The residue was purified by column chromatography on silica (0-60% Et₂O/hexanes) to afford 5-(trifluoromethyl)-3H-dispiro[isobenzofuran-1,1'-cyclohexane-4',2"-[1,3]dioxolane] as an oil. MS: 315 (M + H).

**Step 3:** A solution of 5-(trifluoromethyl)-3H-dispiro[isobenzofuran-1,1'-cyclohexane-4',2"-[1,3]dioxolane] (1.2 g, 3.8 mmol) in DCM (6.5 mL) and TFA (6.5 mL) was stirred overnight at room temperature. The reaction mixture was quenched with aqueous NaOH (2 M, 10 mL) and extracted with DCM (2x). The combined organic layers were dried over anhydrous sodium sulfate, filtered, and concentrated under reduced pressure. The residue was purified by column chromatography on silica (0-70% Et₂O/hexanes) to afford 5'-(trifluoromethyl)-3'H-spiro[cyclohexane-1,1'-isobenzofuran]-4-one as an oil. MS: 271 (M + H).

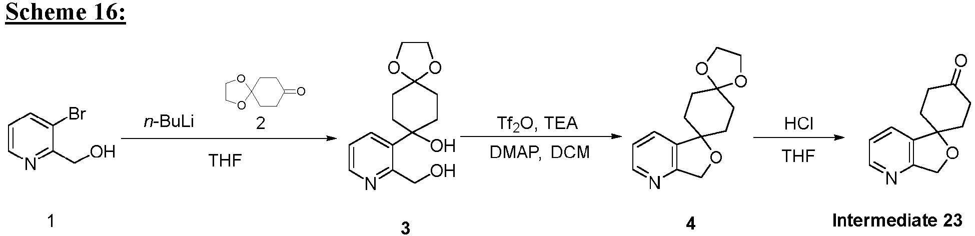

### Intermediate 23: 7'H-spiro[cyclohexane-1,5'-furo[3,4-b]pyridin]-4-one

**Step 1:** To a stirred mixture of (3-bromopyridin-2-yl)methanol (8 g, 42.5 mmol) and 1,4-dioxaspiro[4.5]decan-8-one (6.65 g, 42.5 mmol) in THF (110 mL) was added *N*-butyllithium (2.5 M in hexane, 34.0 mL, 85 mmol) at -78 °C. The mixture was stirred at 0 °C for 2 h. The reaction progress was monitored by LCMS. After completion the reaction mixture was quenched by saturated aq. NH₄Cl (100 mL), extracted with ethyl acetate (3 × 150 mL). The combined organic phase was washed with brine (100 mL), dried over anhydrous sodium sulfate. After filtration, the filtrate was concentrated under reduced pressure. The residue was purified by RP-Combi-Flash chromatography with the following conditions: Column: C 18 gel column (330 g), 20 - 45 µm, 19 x 150 mm; Mobile Phase A: 5 mM aq. NH₄HCO₃; Mobile Phase B: MeCN; Gradient: 20% hold 7 min, up to 35% within 20 min; Flow rate: 100 mL/min; Detector: UV 254 & 210 nm; RT: 15 min to get 8-(2-(hydroxymethyl)pyridin-3-yl)-1,4-dioxaspiro[4.5]decan-8-ol (1.6 g, 6.03 mmol, 14% yield) as a yellow oil. MS: *m*/*z* = 266.15 [M + H]⁺. ¹H NMR (400 MHz, Chloroform-*d*) δ 8.46 - 7.45 (m, 1H), 7.56 - 7.32 (m, 1H), 7.23 - 7.20 (m, 1H), 5.03 (s, 2H), 4.03 - 3.95 (m, 4H), 2.20 - 2.07 (m, 4H), 1.95 - 1.88 (m, 2H), 1.72 - 1.69 (m, 2H).

**Step 2:** To a stirred mixture of 8-(2-(hydroxymethyl)pyridin-3-yl)-1,4-dioxaspiro[4.5]decan-8-ol (1.6 g, 6.03 mmol) in DCM (5 mL) were added 4-dimethylaminopyridine (0.074 g, 0.603 mmol), triethylamine (1.831 g, 18.09 mmol) and trifluoromethanesulfonic anhydride (2.55 g, 9.05 mmol) at ambient temperature. The mixture was stirred at ambient temperature for 2 h. The reaction progress was monitored by LCMS. After completion of the reaction, the mixture was diluted with water (20 mL), extracted with ethyl acetate (3 × 40 mL). The combined organic phase was washed with brine (40 mL), dried over anhydrous sodium sulfate. After filtration, the filtrate was concentrated under reduced pressure. The residue was purified by silica gel column chromatography, using gradient of 60 ~ 75% EA in PE to get 7*H*-dispiro[furo[3,4-*b*]pyridine-5,1'-cyclohexane-4',2"-[1,3]dioxolane] (800 mg, 3.24 mmol, 53% yield) as a yellow oil. MS: *m*/*z* = 248.20 [M + H]⁺. ¹H NMR (400 MHz, Chloroform-*d*) δ 8.48 - 8.47 (m, 1H), 7.49 - 7.47 (m, 1H), 7.20 - 7.17 (m, 1H), 5.06 (s, 2H), 4.04 - 3.97 (m, 4H), 2.13 - 1.94 (m, 4H), 1.90 - 1.76 (m, 2H), 1.75 - 1.72 (m, 2H).

**Step 3:** To a stirred mixture of 7*H*-dispiro[furo[3,4-*b*]pyridine-5,1'-cyclohexane-4',2"-[1,3]dioxolane] (800 mg, 3.24 mmol) in THF (5 mL) was added hydrochloric acid (5 mL, 18.00 mmol, 2 M) at ambient temperature. The mixture was stirred at ambient temperature for 2 h. The reaction progress was monitored by LCMS. After completion of the reaction, the mixture was quenched by saturated NaHCO₃ (15 mL), extracted with ethyl acetate (3 × 30 mL). The combined organic phase was washed with brine (30 mL), dried over anhydrous sodium sulfate. After filtration, the filtrate was concentrated under reduced pressure. The residue was purified by *RP*-Flash chromatography with the following conditions: Column: C 18 gel column (80 g), 20-35 µm, 19 x 150 mm; Mobile Phase A: 5 mM aq. NH₄HCO₃; Mobile Phase B: MeCN; Gradient: 0% hold 5 min, up to 38% within 20 min; 38% hold 2.2 min. Flow rate: 70 mL/min; Detector: UV 254 & 210 nm; RT: 15 min to get 7*'H*-spiro[cyclohexane-1,5'-furo[3,4-*b*]pyridin]-4-one (500 mg, 2.460 mmol, 76% yield) as a white solid. MS: *m*/*z* = 204.20 [M + H]⁺. ¹H NMR (300 MHz, Chloroform-*d*) δ 8.56 - 8.54 (m, 1H), 7.47 - 7.44 (m, 1H), 7.25 - 7.21 (m, 1H), 5.17 (s, 2H), 3.01 - 2.89 (m, 2H), 2.46 - 2.38 (m, 2H), 2.26 - 2.09 (m, 4H).

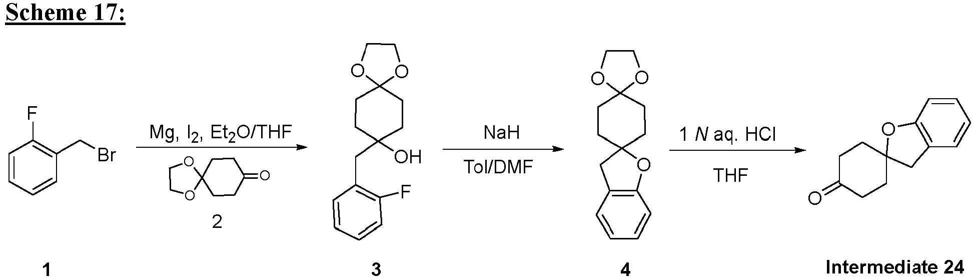

### Intermediate 24: 3H-spiro[benzofuran-2,1'-cyclohexan]-4'-one

**Step 1:** To a suspension of strip magnesium (1.56 g, 64.0 mmol) in diethyl ether (20 mL) was added I₂ (0.163 g, 0.64 mmol) followed by 1-(bromomethyl)-2-fluorobenzene (0.24 g, 1.28 mmol) under nitrogen atmosphere. A high intensity heat gun was applied to initiate the reaction, then 1-(bromomethyl)-2-fluorobenzene (4.84 g, 25.6 mmol) in diethyl ether (5.0 mL) was added slowly at such a speed that a gentle reflux was maintained. After the addition was completed, the reaction mixture was refluxed for 3 hours, cooled to room temperature and a solution of 1,4-dioxaspiro[4.5]decan-8-one (2.0 g, 12.81 mmol) in diethyl ether (5.0 mL) and tetrahydrofuran (5.0 mL) was added slowly with vigorous stirring. After the addition was completed, the reaction mixture was left at room temperature for 3 hours. Aqueous NH₄Cl (50 mL) was added and the mixture was stirred at room temperature overnight, extracted with ethyl acetate (3 x 150 mL), washed with water (200 mL), dried over Na₂SO₄, filtered and concentrated in *vacuo.* The residue was purified by silica gel column chromatography, eluted with EA : PE = 0% - 30% to give 8-(2-fluorobenzyl)-1,4-dioxaspiro[4.5]decan-8-ol (3.00 g, 11.27 mmol, 88% yield) as a light yellow solid. ¹H NMR (300 MHz, Chloroform-*d*): δ 7.28 - 7.21 (m, 2H), 7.14 - 7.04 (m, 2H), 3.97 (d, *J* = 1.8 Hz, 4H), 2.86 (d, *J =* 1.8 Hz, 2H), 1.98 - 1.72 (m, 4H), 1.70 - 1.62 (m, 4H).

**Step 2:** 8-(2-fluorobenzyl)-1,4-dioxaspiro[4.5]decan-8-ol (2.4 g, 9.01 mmol) and sodium hydride (60 wt% dispersion in mineral oil, 511 mg, 11.73 mmol) in toluene (21 mL) was heated at 110°C for 5 minutes. DMF (7 mL) was added and the mixture was stirred at 110 °C for 30 minutes before allowing to cool to room temperature. The reaction mixture was quenched by water (100 mL) and extracted with ethyl acetate (3 x 100 mL). The combined organic layers were washed with brine (2 x 200 mL), dried over anhydrous sodium sulfate and filtered. The filtrate was concentrated under reduced pressure and the residue was purified by silica gel column chromatography, eluted with EA : PE = 5% - 40 % to afford 3*H*-dispiro[1-benzofuran-2,1'-cyclohexane-4',2"-[1,3]dioxolane] (1.9 g, 7.71 mmol, 86% yield) as a light yellow solid. MS: *m*/*z* = 247.10 [M + H]⁺. ¹H NMR (300 MHz, Chloroform-*d*): δ 7.23 - 7.11 (m, 2H), 6.86 - 6.77 (m, 2H), 4.06 - 3.96 (m, 4H), 3.03 (s, 2H), 2.10 - 2.00 (m, 4H), 1.93 - 1.68 (m, 4H).

**Step 3:** To a solution of 3*H*-dispiro[1-benzofuran-2,1'-cyclohexane-4',2"-[1,3]dioxolane] (1.9 g, 7.71 mmol) in tetrahydrofuran (8 mL) was added dropwise 1 *N* aq. HCl (8.0 mL, 97 mmol) at 0 °C under argon atmosphere. The resulting mixture was warmed to ambient temperature and stirred for 3 h. The reaction mixture was quenched by water (50 mL) and the *p*H value of the solution was adjusted to 6 ~ 7 with 1 *N* aq. NaHCO₃. The reaction mixture was extracted with ethyl acetate (3 x 100 mL). The combined organic layers were washed with brine (100 mL), dried over anhydrous sodium sulfate and filtered. The filtrate was concentrated under reduced pressure and the residue was purified by silica gel column chromatography, eluted with EA : PE = 5 - 40% to afford 3*H*-spiro[benzofuran-2,1'-cyclohexan]-4'-one (1.3 g, 6.43 mmol, 83% yield) as a light yellow solid. MS: *m*/*z* = 203.05 [M + H]⁺. ¹H NMR (300 MHz, Chloroform-*d*): δ 7.21 - 7.15 (m, 2H), 6.92 - 6.82 (m, 2H), 3.12 (s, 2H), 2.93 - 2.82 (m, 2H), 2.42 - 2.32 (m, 4H), 2.08 - 1.98 (m, 2H).

**Table 4: The following intermediate was prepared using a similar procedure as described above for intermediate 24.**

| **Int. #** | **Structure** | **Compound Name** | **Exact Mass [M+H]⁺** |
|---|---|---|---|
| **25** | 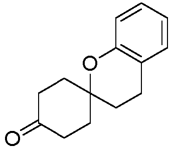 | spiro[chromane-2,1'-cyclohexan]-4'-one | 217 |

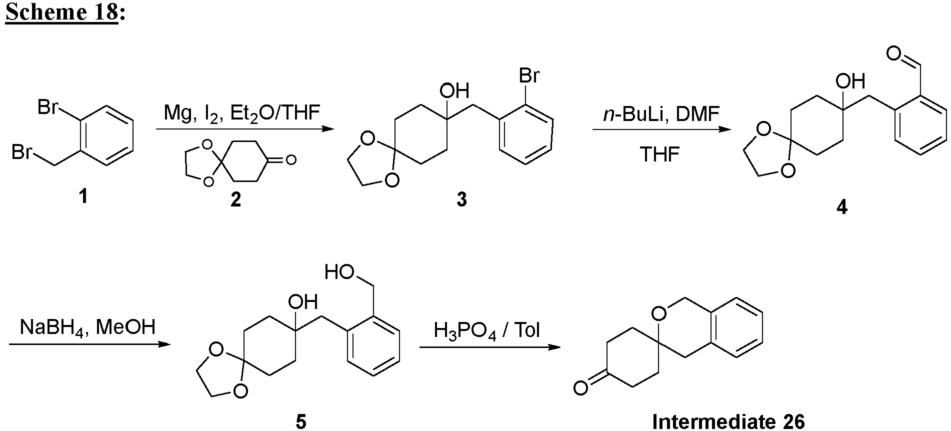

### Intermediate 26: spiro[cyclohexane-1,3'-isochroman]-4-one

**Step 1:** To a suspension of magnesium strip (2.45 g, 101 mmol) in diethyl ether (40.0 mL) was added I₂ (0.26 g, 1.024 mmol) followed by 1-bromo-2-(bromomethyl)benzene (0.34 g, 1.35 mmol) under nitrogen atmosphere. A high intensity heat gun was applied to initiate the reaction, then1-bromo-2-(bromomethyl)benzene (5.04 g, 20.17 mmol) in diethyl ether (10.0 mL) was added slowly at such a speed that a gentle reflux was maintained. After the addition was completed, the reaction mixture was refluxed for 3 hours, cooled to room temperature and a solution of 1,4-dioxaspiro[4.5]decan-8-one (2.1 g, 13.45 mmol) in diethyl ether (10.0 mL) and tetrahydrofuran (10.0 mL) was added slowly with vigorous stirring. After the addition was completed, the reaction mixture was left at room temperature for 3 hours. Aqueous NH₄Cl (40 mL) was added and the mixture was stirred at room temperature overnight, extracted with ethyl acetate (200 mL × 3), washed with H₂O (300 mL), dried over anhydrous sodium sulfate, filtered and the filtrate was concentrated in vacuo. The residue was purified by silica gel column chromatography, eluted with 0% - 30% ethyl acetate in petroleum ether to give 8-(2-bromobenzyl)-1,4-dioxaspiro[4.5]decan-8-ol (3.3 g, 10.09 mmol, 75% yield) as a light yellow solid. ¹H NMR (300 MHz, Chloroform-*d*): δ 7.59 (dd, *J =* 8.0, 1.3 Hz, 1H), 7.38-7.18 (m, 2H), 7.17-7.06 (m, 1H), 3.97 (s, 4H), 3.04 (s, 2H), 1.98-1.58 (m, 8H).

**Step 2:** *n*-Butyllithium (2.5 M in *n*-hexane, 4.89 mL, 12.22 mmol) was added dropwise to a solution of 8-(2-bromobenzyl)-1,4-dioxaspiro[4.5]decan-8-ol (2.0 g, 6.11 mmol) in tetrahydrofuran (15.0 mL) at -78 °C. The resulting solution was stirred at 0 °C for 1 h under nitrogen atmosphere. Dimethylformamide (2.37 mL, 30.6 mmol) was added into mixture at -78 °C and the resulting solution was stirred under 0 °C for 1.5 h. The reaction mixture was quenched by saturated ammonium chloride (50 mL) and extracted with ethyl acetate (100 mL × 3). The combined organic layers were washed with brine (100 mL), dried over anhydrous sodium sulfate and filtered. The filtrate was concentrated under reduced pressure to give crude 2-((8-hydroxy-1,4-dioxaspiro[4.5]decan-8-yl)methyl)benzaldehyde (1.689 g, 6.11 mmol, 100% yield) as a light yellow oil, which was used directly for next step.

**Step 3:** To a mixture of 2-((8-hydroxy-1,4-dioxaspiro[4.5]decan-8-yl)methyl)benzaldehyde (1.689 g, 0.00 mmol) in methanol (8.0 mL) was added sodium borohydride (0.35 g, 9.17 mmol) at ambient temperature and the mixture was stirred at this temperature for 1 h under nitrogen atmosphere. The reaction mixture was quenched by saturated ammonium chloride (50 mL) and extracted with ethyl acetate (100 mL × 3). The combined organic layers were washed with brine (100 mL), dried over anhydrous sodium sulfate and filtered. The filtrate was concentrated under reduced pressure and the residue was purified by silica gel chromatography, eluting with a gradient of 1 : 100 to 1 : 20 methanol in dichloromethane to give 8-(2-(hydroxymethyl)benzyl)-1,4-dioxaspiro[4.5]decan-8-ol (1.3 g, 4.67 mmol, 76% yield) as a colorless oil. MS ESI calculated for C₁₆H₂₂O₄ [M + Na]⁺ 301.14 found 301.15. ¹H NMR (300 MHz, Chloroform-*d*): δ 7.42-7.35 (m, 1H), 7.35-7.23 (m, 2H), 7.23-7.14 (m, 1H), 4.65 (s, 2H), 3.98 (s, 4H), 2.92 (s, 2H), 1.93 - 1.58 (m, 8H).

**Step 4:** To a solution of 8-(2-(hydroxymethyl)benzyl)-1,4-dioxaspiro[4.5]decan-8-ol (1.5 g, 5.39 mmol) in toluene (15 mL) was added phosphoric acid (85% in water, 1.5 mL, 5.39 mmol) at ambient temperature. The mixture was stirred for 1 h at 110 °C. Then toluene was removed by distillation and the resulting residue was diluted with water (50 mL), extracted with ethyl acetate (50 mL × 3), dried over anhydrous sodium sulfate and filtered. The filtrate was concentrated under reduced pressure and the residue was purified by silica gel column chromatography, eluted with 10% - 60% ethyl acetate in petroleum ether to afford spiro[cyclohexane-1,3'-isochroman]-4-one (800 mg, 3.70 mmol, 67% yield) as a light yellow solid. MS ESI calculated for C₁₄H₁₆O₂ [M + H]⁺ 217.12 found 217.10. ¹H NMR (300 MHz, Chloroform-*d*): δ 7.31-7.16 (m, 2H), 7.15-7.05 (m, 2H), 4.87 (s, 2H), 2.79 (s, 2H), 2.78-2.67 (m, 2H), 2.37-2.12 (m, 4H), 1.88-1.76 (m, 2H).

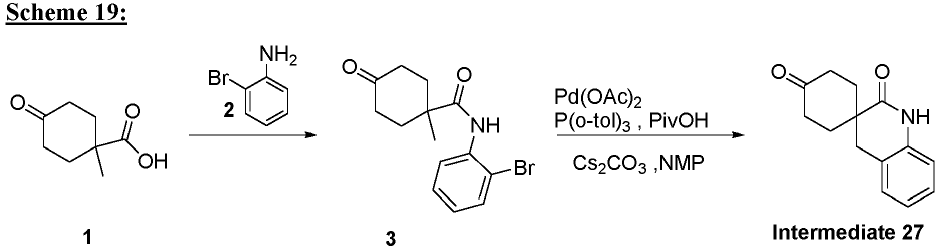

### Intermediate 27: 1',4'-dihydro-2'H-spiro[cyclohexane-13'-quinoline]-2',4-dione

**Step 1:** To a solution of 1-methyl-4-oxocyclohexane-1-carboxylic acid (2 g, 12.81 mmol) in DCM (25 ml) were added *N, N*-dimethylformamide (0.099 ml, 1.281 mmol) and oxalyl chloride (1.625 g, 12.81 mmol) at 0 °C. The resulted mixture was stirred for 2 h at 25 °C. After completion of the reaction, the reaction mixture was concentrated in vacuo. The residue was co-evaporated with DCM three times to afford 1-methyl-4-oxocyclohexane-1-carbonyl chloride (2.237 g, 12.81 mmol, 100% yield) as a light yellow solid.

**Step 2:** To a solution of 1-methyl-4-oxocyclohexane-1-carbonyl chloride (2.237 g, 12.81 mmol) in DCM (25 mL) were added *N,N*-diisopropylethylamine (3.31 g, 25.6 mmol) and 2-bromoaniline (4.41 g, 25.6 mmol) in DCM (1 mL). The resulted mixture was stirred for 16 h 25 °C. The reaction mixture was concentrated in vacuo. The residue was purified by silica gel column chromatography using 10% - 70% gradient of ethyl acetate in petroleum ether as eluent. The fractions containing desired product were combined and concentrated under reduced pressure to afford *N*-(2-bromophenyl)-1-methyl-4-oxocyclohexane-1-carboxamide (3.6 g, 11.61 mmol, 91% yield) as a light yellow solid. MS ESI calculated for C₁₄H₁₆BrNO₂ [M + H]⁺ 310.04 found 310.00. ¹H-NMR (300 MHz, Chloroform-d) δ 8.34 (dd, *J =* 8.3, 1.6 Hz, 1H), 8.04 (brs, 1H), 7.56 (dd, *J =* 8.0, 1.5 Hz, 1H), 7.39-7.30 (m, 1H), 7.07-6.96 (m, 1H), 2.70-2.53 (m, 2H), 2.53-2.33 (m, 4H), 1.96-1.78 (m, 2H), 1.47 (s, 3H).

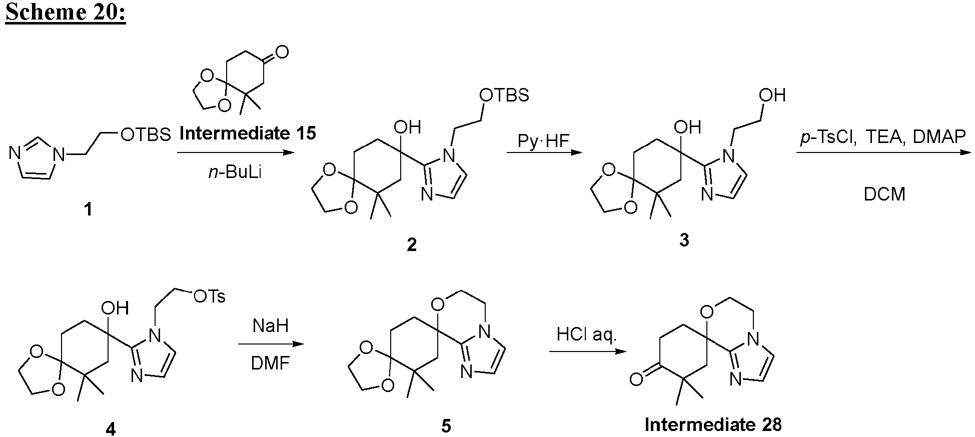

### Intermediate 28: 3,3-dimethyl-5',6'-dihydrospiro[cyclohexane-1,8'-imidazo[2,1-c][1,4]oxazin]-4-one

**Step 1:** To a solution of 1-(2-((*tert*-butyldimethylsilyl)oxy)ethyl)-1*H*-imidazole (5.59 g, 24.69 mmol) in THF (30 mL) was added n-butyllithium (2.5 M in n-hexane, 9.87 mL, 24.69 mmol) at - 78 °C and the mixture was stirred for 30 min. Then a solution of 6,6-dimethyl-1,4-dioxaspiro[4.5]decan-8-one (3.79 g, 20.57 mmol) in THF (21 mL) was added to the above mixture at -78 °C and the resulting mixture was stirred for 4 h at room temperature. The reaction mixture was quenched by water (200 mL) and extracted with ethyl acetate (200 mL × 3). The combined organic layers was washed with brine (400 mL), dried over anhydrous sodium sulfate and filtered. The filtrate was concentrated under reduced pressure to give crude 8-(1-(2-((*tert*butyldimethylsilyl)oxy)ethyl)-1*H*-imidazol-2-yl)-6,6-dimethyl-1,4-dioxaspiro[4.5]decan-8-ol (8.44 g), which was used directly in the next step. MS ESI calculated for C₂₁H₃₈N₂O₄Si [M + H]⁺ 411.26 found 411.25.

**Step 2:** To a solution of 8-(1-(2-((*tert*-butyldimethylsilyl)oxy)ethyl)-1*H*-imidazol-2-yl)-6,6-dimethyl-1,4-dioxaspiro[4.5]decan-8-ol (8.44 g, 20.57 mmol) in THF (66 mL) was added hydrofluoride (70% in pyridine, 25.6 mL) at 0 °C and the solution was stirred for 20 min at 0 °C and for 1 h at room temperature. The reaction mixture was quenched by saturated sodium bicarbonate (100 mL) and filtered. The filtrate was concentrated under reduced pressure and the residue was purified by reverse phase chromatography with the following conditions: column AQ C¹⁸ spherical 20-35 um; Mobile phase: 0%-30% acetonitrile in water, UV 210 nm. The collected fractions were combined and concentrated under reduced pressure to give 8-(1-(2-hydroxyethyl)-1*H*-imidazol-2-yl)-6,6-dimethyl-1,4-dioxaspiro[4.5]decan-8-ol (3.18 g, 10.72 mmol, 52.1% yield) as a white solid. MS ESI calculated for C₁₅H₂₄N₂O₄ [M + H]⁺ 279.17 found 279.20. ¹H-NMR (400 MHz, Chloroform-*d*) δ 7.07 (d, *J =* 1.2 Hz, 1H), 6.69 (d, *J =* 1.2 Hz, 1H), 5.10 (s, 1H), 4.96 (t, 1H), 4.32-4.22 (m, 2H), 3.98-3.76 (m, 4H), 3.72-3.62 (m, 2H), 2.37-2.21 (m, 1H), 2.11-1.76 (m, 5H), 1.11 (s, 3H), 0.67 (s, 3H).

**Step 3:** To a solution of 8-(1-(2-hydroxyethyl)-1*H*-imidazol-2-yl)-6,6-dimethyl-1,4-dioxaspiro[4.5]decan-8-ol (3.177 g, 10.72 mmol) in DCM (78 mL) were added 4-dimethylaminopyridine (0.131 g, 1.072 mmol), triethylamine (3.25 g, 32.2 mmol) and*p-*toluenesulfonyl chloride (3.07 g, 16.08 mmol) at room temperature and the mixture was stirred for 16 h. The reaction mixture was quenched by water (60 mL) and extracted with ethyl acetate (60 mL × 3). The combined organic layers was washed with brine (100 mL ), dried over anhydrous sodium sulfate and filtered. The filtrate was concentrated under reduced pressure. The residue was purified by reverse phase chromatography with the following conditions: column AQ C¹⁸ spherical 20-40 um; Mobile phase: 0% - 50% acetonitrile in water, UV 210 nm. The collected fractions was combined and concentrated under reduced pressure to give 2-(2-(8-hydroxy-6,6-dimethyl-1,4-dioxaspiro[4.5]decan-8-yl)-1H-imidazol-1-yl)ethyl 4-methylbenzenesulfonate (3.3 g, 7.32 mmol, 68.3% yield) as a yellow oil. MS ESI calculated for C₂₂H₃₀N₂O₆S [M + H]⁺ 451.18 found 451.25. ¹H-NMR (400 MHz, Chloroform-*d*) δ 7.68 (d, *J =* 7.9 Hz, 2H), 7.32 (d, *J =* 8.0 Hz, 2H), 6.89 (d, *J =* 1.5 Hz, 1H), 6.87 (d, *J =* 1.4 Hz, 1H), 4.55-4.51 (m, 2H), 4.35-4.25 (m, 2H), 4.01-3.92 (m, 2H), 3.92-3.88 (m, 2H), 2.44 (s, 3H), 2.43-2.32 (m, 1H), 2.16 (d, *J =* 14.3 Hz, 1H), 2.07-2.02 (m, 1H), 1.88-1.80 (m, 1H), 1.74-1.60 (m, 2H), 1.20 (s, 3H), 0.80 (s, 3H).

**Step 4:** To a mixture of sodium hydride (60% in mineral oil, 0.439 g, 10.99 mmol) in DMF (25 mL) was added a solution of 2-(2-(8-hydroxy-6,6-dimethyl-1,4-dioxaspiro[4.5]decan-8-yl)-1*H-*imidazol-1-yl)ethyl 4-methylbenzenesulfonate (3.3 g, 7.32 mmol) in DMF (25 mL) at 0 °C and the mixture was stirred for 30 min at 0 °C and for 4 h at room temperature. The reaction mixture was quenched by NH₄Cl (60 mL) and extracted with ethyl acetate (70 mL × 3). The combined organic layers was washed with brine (200 mL ), dried over anhydrous sodium sulfate and filtered. The filtrate was concentrated under reduced pressure and the residue was purified by silica gel column chromatography, eluted with 1~70% EtOAc in petroleum ether to afford 2',2'-dimethyl-5,6-dihydrodispiro[imidazo[2,1-c][1,4]oxazine-8,4'-cyclohexane-1',2"-[1,3]dioxolane] (786 mg, 2.82 mmol, 38.6% yield) as a white solid. MS ESI calculated for C₁₅H₂₂N₂O₃ [M + H]⁺ 279.16 found 279.15. ¹H NMR (400 MHz, Chloroform-*d*) δ 7.03 (s, 1H), 6.81-6.76 (m, 1H), 4.10-3.90 (m, 8H), 2.49 (t, *J =* 13.3 Hz, 1H), 2.26 (d, *J =* 14.7 Hz, 1H), 2.09 (td, *J =* 13.7, 3.8 Hz, 1H), 1.95 (dd, *J =* 14.0, 3.4 Hz, 1H), 1.85 (dd, *J =* 14.8, 3.2 Hz, 1H), 1.59-1.51 (m, 1H), 1.24 (s, 3H), 0.89 (s, 3H).

**Step 5:** To a solution of HCl (1.5 M in water, 10 mL, 15.00 mmol) was added a solution of 2',2'-dimethyl-5,6-dihydrodispiro[imidazo[2,1-c][1,4]oxazine-8,4'-cyclohexane-1',2"-[1,3]dioxolane] (786 mg, 2.82 mmol) in THF (10 mL) at room temperature and the solution was stirred for 3 h at room temperature. The reaction mixture was quenched by saturated sodium bicarbonate (20 mL) and extracted with ethyl acetate (30 mL × 3). The combined organic layers was washed with brine (80 mL ), dried over anhydrous sodium sulfate and filtered. The filtrate was concentrated under reduced pressure. The residue was purified by silica gel column chromatography, eluted with 1~15% MeOH in DCM to afford 3,3-dimethyl-5',6'-dihydrospiro[cyclohexane-1,8'-imidazo[2,1-c][1,4]oxazin]-4-one (460 mg, 1.963 mmol, 69.5% yield) as a white solid. MS ESI calculated for C₁₃H₁₈N₂O₂ [M + H]⁺ 235.14 found 235.15. ¹H-NMR (300 MHz, Chloroform-*d*) δ 7.02 (d, *J* = 1.3 Hz, 1H), 6.83 (d, *J =* 1.3 Hz, 1H), 4.29-3.87 (m, 4H), 3.07-2.84 (m, 1H), 2.65-2.01 (m, 5H), 1.34 (s, 3H), 1.10 (s, 3H).

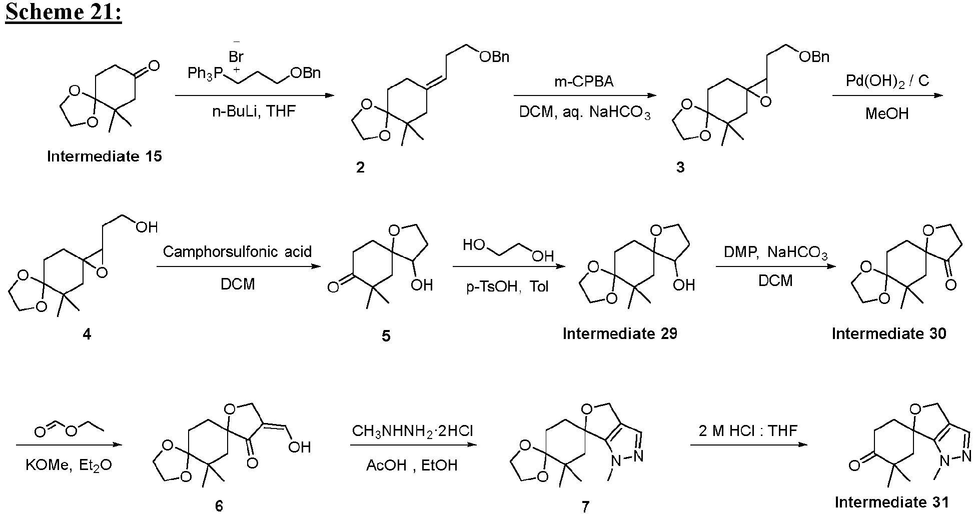

### Intermediate 29: 6,6-dimethyl-1,4,9-trioxadispiro[4.2.4⁸.2⁵]tetradecan-12-ol

### Intermediate 30: 6,6-dimethyl-1,4,9-trioxadispiro[4.2.4⁸.2⁵]tetradecan-12-one

### Intermediate 31: 1',3,3-trimethyl-1',4'-dihydrospiro[cyclohexane-1,6'-furo[3,4-c]pyrazol]-4-one

**Step 1:** To a solution of (3-(benzyloxy)propyl)triphenylphosphonium bromide (10.06 g, 20.46 mmol) in THF (23 mL) was added *n*-butyllithium (2.5 M in hexane, 8.19 mL, 20.46 mmol) at 0 °C and the solution was stirred for 2 hours at 25 °C. Then a solution of 6,6-dimethyl-1,4-dioxaspiro[4.5]decan-8-one (2.9 g, 15.74 mmol) in THF (5 mL) was dropwise added to the above solution at 0 °C. The resulted mixture was stirred for 6 hours at 25 °C. After completion of the reaction, the reaction mixture was quenched with aqueous NH₄Cl and the mixture was concentrated under reduced pressure. The residue was diluted with ethyl acetate (200 mL), then washed with brine (20 mL × 3), dried over anhydrous sodium sulfate and filtered. The filtrate was concentrated under reduced pressure. The residue was purified by silica gel column chromatography using 10% - 40% gradient of ethyl acetate in petroleum ether as eluent. The fractions containing desired product were combined and concentrated under reduced pressure to afford 8-(3-(benzyloxy)propylidene)-6,6-dimethyl-1,4-dioxaspiro[4.5]decane (4.05 g, 12.80 mmol, 81% yield) as a light yellow solid. MS ESI calculated for C₂₀H₂₈O₃ [M + H]⁺ 317.20 found 317.15. ¹H-NMR (400 MHz, Chloroform-*d*) δ 7.40-7.29 (m, 5H), 5.26 (t, *J =* 7.2 Hz, 0.5H), 5.13 (t, *J =* 7.3 Hz, 0.5H), 4.51 (s, 2H), 4.01-3.87 (m, 4H), 3.49-3.40 (m, 2H), 2.40-2.28 (m, 2H), 2.28-2.15 (m, 2H), 2.14 (s, 1H), 2.07 (s, 1H), 1.67-1.55 (m, 2H), 0.94 (s, 3H), 0.92 (s, 3H).

**Step 2:** To a solution of 8-(3-(benzyloxy)propylidene)-6,6-dimethyl-1,4-dioxaspiro[4.5]decane (3.5 g, 11.06 mmol) in DCM (175 mL) and 0.5 M aqueous sodium bicarbonate (53 mL) was added *m*-CPBA (85%, 2.863 g, 14.10 mmol) at 0 °C under argon atmosphere. The resulted mixture was stirred for 3 hours at room temperature. After completion of the reaction, the reaction mixture was diluted with DCM (250 mL), then washed with 0.5 M aqueous sodium bicarbonate (50 mL × 3), dried over anhydrous sodium sulfate and filtered. The filtrate was concentrated under reduced pressure. The residue was purified by RP-Combi-Flash with the following conditions: Column: AQ-C¹⁸ OBD Column, 330 g; Mobile Phase A: 10 mM aqueous NH₄HCO₃, Mobile Phase B: MeCN; Flow rate: 100 mL/min; Gradient: 0% - 100% in 35 min. Detector: UV 254 & 210 nm; The fractions containing desired product were combined and concentrated under reduced pressure to afford 2-(2-(benzyloxy)ethyl)-5,5-dimethyl-1,7,10-trioxadispiro[2.2.4⁶.2³]dodecane (2.96 g, 8.90 mmol, 80.5% yield) as a colorless oil. MS ESI calculated for C₂₀H₂₈O₄ [M + Na]⁺ 355.20 found 355.25. ¹H-NMR (300 MHz, Chloroform-*d*) δ 7.43-7.30 (m, 5H), 4.62-4.47 (m, 2H), 4.10-3.88 (m, 4H), 3.82-3.53 (m, 2H), 2.95-2.72 (m, 1H), 2.26-1.50 (m, 8H), 1.13-0.94 (m, 6H).

**Step 3:** 2-(2-(Benzyloxy)ethyl)-5,5-dimethyl-1,7,10-trioxadispiro[2.2.4⁶.2³]dodecane (1.00 g, 3.01 mmol) was dissolved in MeOH (150 mL) and the resulting mixture was evacuated and an atmosphere of argon was applied at ambient temperature. Then palladium hydroxide on carbon (20%, 800 mg, 1.14 mmol) was added under argon atmosphere. The suspension was degassed under vacuum and purged with hydrogen for three times. The reaction solution was stirred for 4 hours at room temperature under 1 atm of hydrogen. The suspension was filtered and the filtrate was concentrated under reduced pressure. The residue was purified by silica gel column chromatography using 10% - 40% gradient of ethyl acetate in petroleum ether as eluent. The fractions containing desired product were combined and concentrated under reduced pressure to afford to give 2-(5,5-dimethyl-1,7,10-trioxadispiro[2.2.4⁶.2³]dodecan-2-yl)ethan-1-ol (684 mg, 2.82 mmol, 93.8% yield) as colorless oil. MS ESI calculated for C₁₃H₂₂O₄ [M + H]⁺ 243.15 found 243.10. ¹H-NMR (400 MHz, DMSO-*d₆*) δ 4.60 (t, *J =* 4.3 Hz, 0.5H), 5.57 (t, *J =* 4.3 Hz, 0.5H), 3.98-3.77 (m, 4H), 3.63-3.43 (m, 2H), 2.79-2.70 (m, 1H), 1.79-1.34 (m, 8H), 0.98 (s, 1.5H), 0.93 (s, 3H), 0.90 (s, 1.5H).

**Step 4:** To a solution of 2-(5,5-dimethyl-1,7,10-trioxadispiro[2.2.46.23]dodecan-2-yl)ethan-1-ol (510 mg, 2.105 mmol) in DCM (5 mL) was added (1*S*)-(+)-10-camphorsulfonic acid (98 mg, 0.421 mmol). The resulted mixture was stirred for 6 hours at room temperature. After completion of the reaction, the reaction mixture was concentrated under reduced pressure. The residue was purified by silica gel column chromatography using 10% - 40% gradient of ethyl acetate in petroleum ether as eluent. The fractions containing desired product were combined and concentrated under reduced pressure to afford 4-hydroxy-7,7-dimethyl-1-oxaspiro[4.5]decan-8-one (320 mg, 1.614 mmol, 77% yield) as a colorless oil. MS ESI calculated for C₁₁H₁₈O₃ [M + H]⁺ 199.13 found 199.20. ¹H-NMR (400 MHz, DMSO-*d₆*) δ 4.99 (d, *J =* 4.9 Hz, 0.5H), 4.93 (d, *J* = 4.1 Hz, 0.5H), 3.93-3.72 (m, 3H), 2.73 (ddt, *J =* 13.9, 8.4, 5.8 Hz, 1H), 2.24-2.02 (m, 2H), 1.90-1.62 (m, 5H), 1.20 (s, 1.5H), 1.19 (s, 1.5H), 0.97 (s, 1.5H), 0.94 (s, 1.5H).

**Step 5:** To a solution of 4-hydroxy-7,7-dimethyl-1-oxaspiro[4.5]decan-8-one (310 mg, 1.564 mmol) and ethylene glycol (97 mg, 1.564 mmol) in toluene (15 mL) was added*p-*toluenesulfonic acid (269 mg, 1.564 mmol). The resulted mixture was heated to reflux to remove water by Dean-Stark trap for 16 hours. After completion of the reaction, the reaction mixture was diluted with ethyl acetate (100 mL), then washed with saturated aqueous sodium bicarbonate (10 mL × 3), dried over anhydrous sodium sulfate and filtered. The filtrate was concentrated under reduced pressure to give 6,6-dimethyl-1,4,9-trioxadispiro[4.2.4⁸.2⁵]tetradecan-12-ol (400 mg, crude) as a brown oil, which was used directly for next step without further purification. MS ESI calculated for C₁₃H₂₂O₄ [M + H]⁺ 243.15 found 243.20. ¹H-NMR (400 MHz, DMSO-*d₆*) δ 4.90-4.70 (m, 1H), 3.93-3.60 (m, 7H), 2.15-2.00 (m, 1H), 1.83-1.30 (m, 7H), 1.08 (s, 3H), 0.82 (s, 1.5H), 0.78 (s, 1.5H).

**Step 6:** To a solution of 6,6-dimethyl-1,4,9-trioxadispiro[4.2.4⁸.2⁵]tetradecan-12-ol (400 mg, 1.564 mmol, crude) in DCM (10 mL) was added Dess-Martin periodinane (1400 mg, 3.30 mmol). The resulted mixture was stirred for 2 hours at 25 °C. After completion of the reaction, the reaction mixture was filtered. The filtrate was concentrated under reduced pressure. The residue was purified by silica gel column chromatography using 0% - 30% gradient of ethyl acetate in petroleum ether as eluent to afford 6,6-dimethyl-1,4,9-trioxadispiro[4.2.4⁸.2⁵]tetradecan-12-one (220 mg, 0.916 mmol, 58.6% yield for two steps) as a light yellow solid. MS ESI calculated for C₁₃H₂₀O₄ [M + H]⁺ 241.14 found 241.20. ¹H-NMR (400 MHz, Chloroform-*d*) δ 4.28-4.05 (m, 2H), 4.04-3.85 (m, 4H), 2.51 (t, *J =* 7.3 Hz, 2H), 2.00 (td, *J =* 13.4, 4.2 Hz, 1H), 1.85-1.71 (m, 2H), 1.71-1.49 (m, 2H), 1.41 (dd, *J =* 14.1, 2.9 Hz, 1H), 1.22 (s, 3H), 0.90 (s, 3H).

**Step 7:** To a solution of 6,6-dimethyl-1,4,9-trioxadispiro[4.2.4^{8.}2⁵]tetradecan-12-one (240 mg, 0.999 mmol) in ethyl ether (1 mL) were added potassium methanolate (140 mg, 1.998 mmol) and ethyl formate (148 mg, 1.998 mmol). The resulted mixture was stirred for 2 hours at 25 °C. The reaction progress was monitored by LCMS and TLC. After completion of the reaction, the reaction was quenched by saturated aqueous NH₄Cl (2 mL). The mixture was concentrated in vacuo. The residue was purified by RP-Combi-Flash with the following conditions: Column: AQ-C¹⁸ OBD Column, 40 g; Mobile Phase A: water (0.1% TFA), Mobile Phase B: MeCN; Flow rate: 40 mL/min; Gradient: 5% - 60% in 35 min; Detector: UV 254 & 210 nm; The fractions containing desired product were combined and concentrated under reduced pressure to afford 11-(hydroxymethylene)-6,6-dimethyl-1,4,9-trioxadispiro[4.2.4⁸.2⁵]tetradecan-12-one (256 mg, 0.954 mmol, 96% yield) as a yellow oil. MS ESI calculated for C₁₄H₂₀O₅ [M + H]⁺ 269.13 found 269.10

**Step 8:** To a solution of 11-(hydroxymethylene)-6,6-dimethyl-1,4,9-trioxadispiro[4.2.4⁸.2⁵]tetradecan-12-one (256 mg, 0.954 mmol) in ethanol (5 mL) were added methylhydrazine dihydrochloride (227 mg, 1.908 mmol) and AcOH (229 mg, 3.82 mmol). The resulted mixture was stirred for 2 hours at 85 °C. The reaction mixture was concentrated under reduced pressure to give crude 2,2',2'-trimethyl-2,4-dihydrodispiro[furo[3,4-*c*]pyrazole-6,4'-cyclohexane-1',2"-[1,3]dioxolane] (266 mg, 0.954 mmol, 100% yield) as a brown solid, which was used directly for next step without further purification. MS ESI calculated for C₁₅H₂₂N₂O₃ [M + H]⁺ 279.16 found 279.15.

**Step 9:** To a solution of 2,2',2'-trimethyl-2,4-dihydrodispiro[furo[3,4-*c*]pyrazole-6,4'-cyclohexane-1',2"-[1,3]dioxolane] (266 mg, 0.954 mmol) in THF (2 mL) was added hydrochloric acid (2 M in water, 3 mL, 6.00 mmol). The resulted mixture was stirred for 2 hours at 25 °C. The reaction progress was monitored by LCMS and TLC. After completion of the reaction, the reaction was quenched by sodium bicarbonate (0.5 g, 6 mmol) and the mixture was concentrated in vacuo. The residue was purified by RP-Combi-Flash with the following conditions: Column: AQ-C¹⁸ OBD Column, 40 g; Mobile Phase A: water (0.1% TFA), Mobile Phase B: MeCN; Flow rate: 40 mL/min; Gradient: 5% - 60% in 35 min. Detector: UV 254 & 210 nm; The fractions containing desired product were combined and concentrated under reduced pressure to afford 2',3,3-trimethyl-2',4'-dihydrospiro[cyclohexane-1,6'-furo[3,4-*c*]pyrazol]-4-one (90 mg, 0.384 mmol, 40.3% yield) as a yellow oil. MS ESI calculated for C₁₃H₁₈N₂O₂ [M + H]⁺ 235.14 found 235.20. ¹H NMR (400 MHz, Chloroform-*d*) δ 7.18 (s, 1H), 4.94-4.84 (m, 2H), 3.78 (s, 3H), 3.21-3.09 (m, 1H), 2.35-2.27 (m, 1H), 2.24-2.12 (m, 2H), 2.10-1.98 (m, 2H), 1.42 (s, 3H), 1.09 (s, 3H).

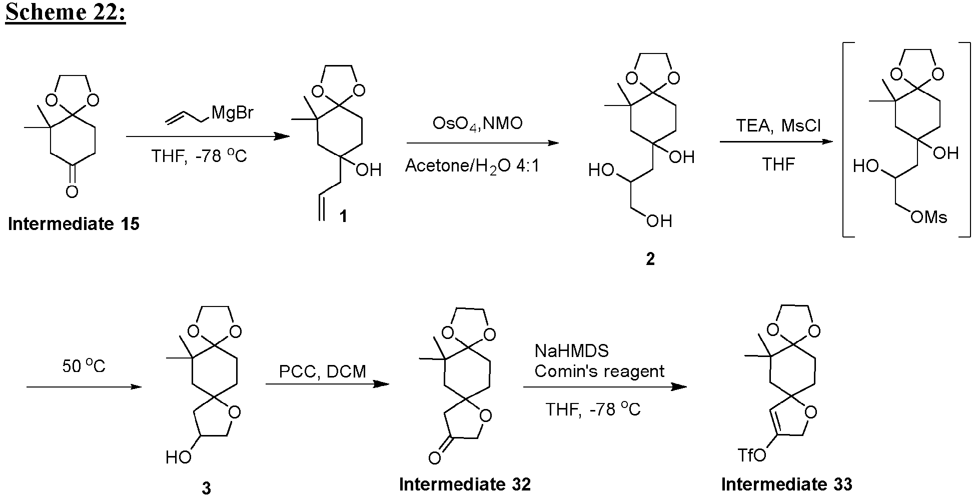

### Intermediate 32: 6,6-dimethyl-1,4,9-trioxadispiro[4.2.4⁸.2⁵]tetradecan-11-one

### Intermediate 33: 6,6-dimethyl-1,4,9-trioxadispiro[4.2.4⁸.2⁵]tetradec-11-en-11-yl trifluoromethanesulfonate

**Step 1:** To a solution of 6,6-dimethyl-1,4-dioxaspiro[4.5]decan-8-one (48.0 g, 260 mmol) in THF (1.0 L) was added allylmagnesium bromide (1 M, 521 mL) dropwise at -78°C. The reaction was stirred for 3 h at -78°C. Then the mixture was quenched with saturated aqueous NH4Cl (1.0 L) and extracted with MTBE (3x). The combined organic layers were washed with water (2x), dried over sodium sulfate and concentrated under reduced pressure to afford 8-allyl-6,6-dimethyl-1,4-dioxaspiro[4.5]decan-8-ol which was used in the next step without further purification. 1H NMR (400 MHz, CDCl₃) δ 5.73 - 5.87 (m, 1H), 4.96 - 5.15 (m, 2H), 3.77 - 4.02 (m, 5H), 2.07 - 2.15 (m, 2H), 1.96 - 2.05 (m, 1H), 1.51 - 1.72 (m, 3H), 1.35 - 1.43 (m, 2H), 1.16 - 1.22 (m, 3H), 0.76 - 0.83 (m, 3H).

**Step 2:** To a solution of 8-allyl-6,6-dimethyl-1,4-dioxaspiro[4.5]decan-8-ol (55.0 g, 243 mmol) in a 4:1 solution of water/acetone (1.0 L) was added OsO4 (0.75 g, 2.95 mmol) at 0°C. The resulting solution was stirred for 12 h at RT. The reaction was then quenched saturated aqueous Na2SO3 (0.5 L) and extracted with ethyl acetate (8x). The combined organic layers were washed with water, dried over sodium sulfate and concentrated under reduced pressure. The residue was purified by silica gel chromatography, eluting with a gradient of ethyl acetate : petroleum ether (1 : 20 to 1 : 1) to yield 3-(8-hydroxy-6,6-dimethyl-1,4-dioxaspiro[4.5]decan-8-yl)propane-1,2-diol. 1H NMR (400 MHz, DMSO-d6) δ 4.76 - 4.83 (m, 1H), 4.53 - 4.64 (m, 1H), 4.34 - 4.42 (m, 1H), 3.76 - 3.97 (m, 5H), 3.20 - 3.34 (m, 2H), 1.89 - 2.03 (m, 1H), 1.57 - 1.73 (m, 2H), 1.34 - 1.56 (m, 5H), 1.16 - 1.24 (m, 3H), 0.79 - 0.89 (m, 3H).

**Step 3:** To a solution of 3-(8-hydroxy-6,6-dimethyl-1,4-dioxaspiro[4.5]decan-8-yl)propane-1,2-diol (41.0.g, 158 mmol) in THF (0.8 L) was added triethylamine (87.7mL, 630 mmol). The mixture was cooled to 0°C and methanesulfonyl chloride (21.7 g, 189 mmol) was added portion wise. The reaction was stirred for 3 h and heated to 50°C for a further 20 h. The mixture was then diluted with ethyl acetate (250 mL), quenched with a saturated sodium bicarbonate solution and extracted with ethyl acetate (3x). The combined organic layers were washed with brine, dried over anhydrous sodium sulfate and concentrated under reduced pressure. The residue was purified by silica gel chromatography, eluting with a gradient of ethyl acetate : petroleum ether (1 : 50 to 1 : 0) to yield 6,6-dimethyl-1,4,9-trioxadispiro[4.2.4.2]tetradecan-11-ol. 1H NMR (400 MHz, DMSO-d6) δ 4.75 - 4.82 (m, 1H), 4.19 - 4.28(m, 1H), 3.73 - 3.91 (m, 6H), 3.48- 3.56 (m, 1H), 1.59 - 1.92 (m, 5H), 1.51 - 1.58 (m, 2H), 1.40 - 1.48 (m, 2H), 1.02 - 1.10 (m, 3H), 0.78 - 0.84 (m, 3H).

**Step 4:** To a solution of 6,6-dimethyl-1,4,9-trioxadispiro[4.2.4.2]tetradecan-11-ol (26.0 g, 107 mmol) ) in dichloromethane (0.8 L) was added PCC (41.6 g, 193. mmol) at ambient temperature. The resulting mixture was stirred for 3 h. The reaction mixture was filtered and concentrated under reduced pressure. The residue was purified by silica gel chromatography, eluting with a gradient of ethyl acetate : petroleum ether (1 : 100 to 1 : 5) to afford 6,6-dimethyl-1,4,9-trioxadispiro[4.2.4.2]tetradecan-11-one. 1H NMR (400 MHz, DMSO-d6) δ 3.87 - 4.00 (m, 6H), 2.24 - 2.54 (m, 2H), 1.67 - 1.92 (m, 4H), 1.45 - 1.64 (m, 2H), 1.04 - 1.14 (m, 3H), 0.80 - 0.90 (m, 3H).

**Step 5:** To a solution of 6,6-dimethyl-1,4,9-trioxadispiro[4.2.4.2]tetradecan-11-one (11.0 g, 45.8 mmol) in THF (0.3 L) was added NaHMDS (2 M, 34.3 mL, 68.6 mmol) at -78°C and the resulting solution stirred for 2 h. Comin's reagent (26.9 g, 68.6 mmol) was then added and the mixture stirred at -78°C for a further 1 h. The reaction was then warmed to RT and stirred for 1 h. Then the mixture was quenched with saturated aqueous NH₄Cl and extracted with ethyl acetate (3x). The combined organic layers were washed with water, dried over anhydrous sodium sulfate and concentrated under reduced pressure. The residue was purified by silica gel chromatography, eluting with a gradient of ethyl acetate : petroleum ether (0 to 30%) to yield 6,6-dimethyl-1,4,9-trioxadispiro[4.2.4.2]tetradec-11-en-11-yl trifluoromethanesulfonate. 1H NMR (400 MHz, CDCl₃) δ 5.52 - 5.62 (m, 1H), 4.47 - 4.60 (m, 2H), 3.78 - 3.92(m, 4H), 1.89 - 1.99 (m, 1H), 1.63 - 1.83 (m, 3H), 1.41 - 1.57 (m, 3H), 1.08 - 1.11 (m, 3H), 0.79 - 0.83 (m, 3H).

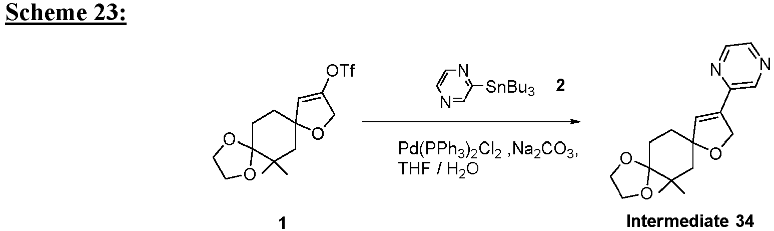

### Intermediate 34: 2-(6,6-dimethyl-1,4,9-trioxadispiro[4.2.4⁸.2⁵]tetradec-11-en-11-yl)pyrazine

**Step 1:** To a stirred solution of 6,6-dimethyl-1,4,9-trioxadispiro[4.2.4⁸.2⁵]tetradec-11-en-11-yl trifluoromethanesulfonate (600 mg, 1.611 mmol) in THF (6 mL) were added bis(triphenylphosphine)palladium(II) dichloride (56.6 mg, 0.081 mmol), 2 N aqueous sodium carbonate (4 mL) and 2-(tributylstannyl)pyrazine (1190 mg, 3.22 mmol). The reaction mixture was degassed with nitrogen for three times and stirred at 80 °C for 16 h under nitrogen atmosphere. The reaction progress was monitored by LCMS and TLC. After completion of the reaction, the reaction mixture was cooled to room temperature and extracted with ethyl acetate (50 mL × 3). Then the combined organic layer was washed with brine (60 mL × 3), dried over anhydrous sodium sulfate and filtered. The filtrate was concentrated in vacuo. The residue was purified by silica gel column chromatography, using 10% - 60% gradient of ethyl acetate in petroleum ether as eluent. The fractions containing desired product were combined and concentrated under reduced pressure to afford 2-(6,6-dimethyl-1,4,9-trioxadispiro[4.2.4⁸.2⁵]tetradec-11-en-11-yl)pyrazine (330 mg, 1.037 mmol, 64.3% yield) as an off-white solid. MS ESI calculated for C₁₇H₂₂N₂O₃ [M + H]⁺ 303.17 found 303.10. ¹H NMR (300 MHz, Chloroform-*d*) δ 8.69 (s, 1H), 8.54 (s, 1H), 8.44 (s, 1H), 6.51 (t, *J =* 2.2 Hz, 1H), 5.10 (d, *J* = 2.1 Hz, 2H), 4.09-3.88 (m, 4H), 2.19-2.01(m, 1H), 2.01-1.76 (m, 3H), 1.69-1.52 (m, 2H), 1.26 (s, 3H), 0.93 (s, 3H).

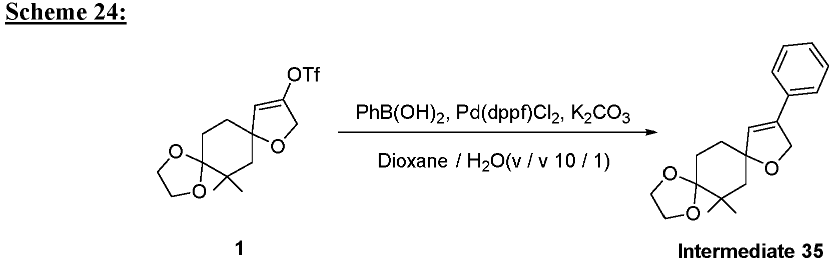

### Intermediate 35: 6,6-dimethyl-11-phenyl-1,4,9-trioxadispiro[4.2.4⁸.2⁵]tetradec-11-ene

**Step 1:** To a solution of 6,6-dimethyl-1,4,9-trioxadispiro[4.2.4⁸.2⁵]tetradec-11-en-11-yl trifluoromethanesulfonate (600 mg, 1.611 mmol) in 1,4-Dioxane (6 mL) were added phenylboronic acid (236 mg, 1.934 mmol), K₂CO₃ (557 mg, 4.03 mmol) and water (0.6 mL) at room temperature. Then Pd(dppf)Cl₂.CH₂Cl₂ (132 mg, 0.161 mmol) was added to above mixture. Replaced the system three times with nitrogen and stirred for 2 h at 100 °C under nitrogen. The reaction mixture was quenched with water (60 mL), extracted with ethyl acetate (3 x 100 mL). The combined organic layers were washed with brine (*sat.,* 2 x 50 mL), dried over Sodium sulfate, filtered and the solvent was removed under reduced pressure. The residue was purified by silica gel column chromatography, eluted with 0 - 20% ethyl acetate in petroleum ether to afford 6,6-dimethyl-11-phenyl-1,4,9-trioxadispiro[4.2.4⁸.2⁵]tetradec-11-ene (450 mg, 1.498 mmol, 93 % yield) as a light yellow oil. MS ESI calculated for C₁₉H₂₄O₃ [M + H]⁺ 301.18 found 301.10. ¹H NMR (300 MHz, Chloroform-*d*) *δ* 7.37 - 7.27 (m, 5H), 6.01 (t, *J =* 2.1 Hz, 1H), 4.99 (d, *J =* 2.1 Hz, 2H), 4.01 - 3.92 (m, 4H), 2.07 (td, *J =* 12.7, 4.5 Hz, 1H), 1.88 (td, *J =* 13.0, 4.1 Hz, 1H), 1.82 - 1.73 (m, 2H), 1.63 - 1.52 (m, 2H), 1.23 (s, 3H), 0.90 (s, 3H).

**Table 5: The following intermediate was prepared using a similar procedure as described above for intermediate 35**

| **Int. #** | **Structure** | **Compound Name** | **Exact Mass [M+H]⁺** |
|---|---|---|---|
| **36** | 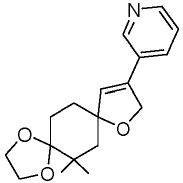 | 3-(6,6-dimethyl-1,4,9-trioxadispiro[4.2.4⁸.2⁵]tetradec-11-en-11-yl)pyridine | 302 |

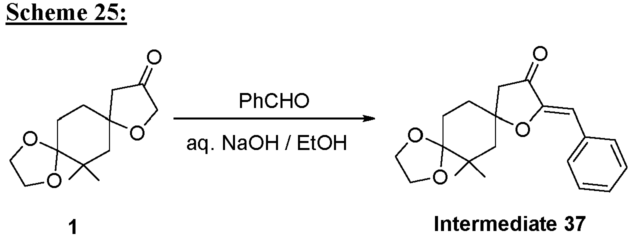

### Intermediate 37: (Z)-10-benzylidene-6,6-dimethyl-1,4,9-trioxadispiro[4.2.4⁸.2⁵]tetradecan-11-one

**Step 1:** To a stirred mixture of 6,6-dimethyl-1,4,9-trioxadispiro[4.2.4⁸.2⁵]tetradecan-11-one (480 mg, 1.998 mmol) and benzaldehyde (212 mg, 1.998 mmol) in EtOH (4 mL) was added *aq.* NaOH (5% in H₂O, 0.8 mL, 1.998 mmol) at 0 °C. The resulting mixture was stirred at 0 °C for 1 hours. The mixture was filtered by EtOH (3 × 10 mL) and the filtrate was concentrated under reduced pressure. The residue was purified by *RP*-flash chromatography, eluted with 0 - 55% acetonitrile in water to afford (*Z*)-10-benzylidene-6,6-dimethyl-1,4,9-trioxadispiro[4.2.4⁸.2⁵]tetradecan-11-one (300 mg, 0.913 mmol, 45.7 % yield) as a yellow oil. MS ESI calculated for C₂₀H₂₄O₄ [M + H]⁺ 329.18 found 329.20. ¹H NMR (300 MHz, Chloroform - *d*) δ 7.83-7.74 (m, 2H), 7.45-7.30 (m, 3H), 6.34 (s, 1H), 4.11-3.92 (m, 4H), 2.58 (s, 2H), 2.42-2.14 (m, 1H), 2.12-1.85 (m, 4H), 1.71-1.57 (m, 1H), 1.36 (s, 3H), 0.98 (s, 3H).

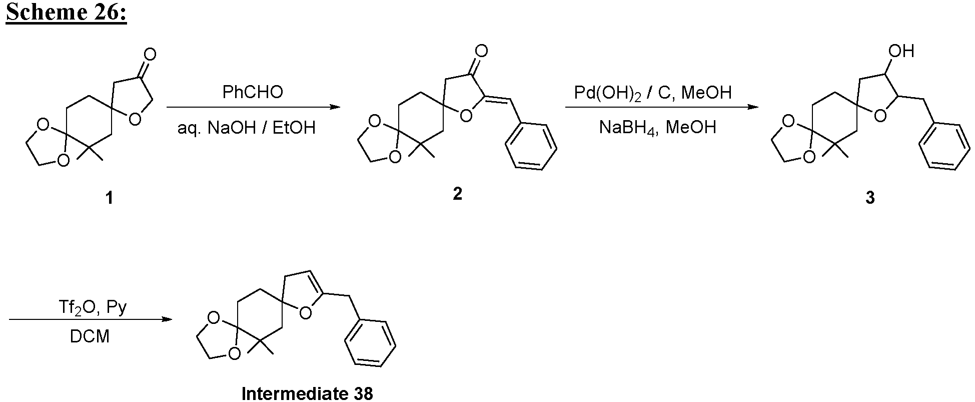

### Intermediate 38: 10-benzyl-6,6-dimethyl-1,4,9-trioxadispiro[4.2.4⁸.2⁵]tetradecan-11-ol

**Step 1:** To a stirred mixture of 6,6-dimethyl-1,4,9-trioxadispiro[4.2.4⁸.2⁵]tetradecan-11-one (480 mg, 1.998 mmol) and benzaldehyde (212 mg, 1.998 mmol) in EtOH (4 mL) was added *aq.* NaOH (5% in H₂O, 0.8 mL, 1.998 mmol) at 0 °C. The resulting mixture was stirred at 0 °C for 1 hours. The mixture was filtered by EtOH (3 × 10 mL) and the filtrate was concentrated under reduced pressure. The residue was purified by *RP*-flash chromatography, eluted with 0 - 55% acetonitrile in water to afford (*Z*)-10-benzylidene-6,6-dimethyl-1,4,9-trioxadispiro[4.2.4⁸.2⁵]tetradecan-11-one (300 mg, 0.913 mmol, 45.7 % yield) as a yellow oil. MS ESI calculated for C₂₀H₂₄O₄ [M + H]⁺ 329.18 found 329.20. ¹H NMR (300 MHz, Chloroform - *d*) δ 7.83-7.74 (m, 2H), 7.45-7.30 (m, 3H), 6.34 (s, 1H), 4.11-3.92 (m, 4H), 2.58 (s, 2H), 2.42-2.14 (m, 1H), 2.12-1.85 (m, 4H), 1.71-1.57 (m, 1H), 1.36 (s, 3H), 0.98 (s, 3H).

**Step 2:** To a stirred mixture of (*Z*)-10-benzylidene-6,6-dimethyl-1,4,9-trioxadispiro[4.2.4⁸.2⁵]tetradecan-11-one (300 mg, 0.913 mmol) in MeOH (5 mL) was added palladium hydroxide on carbon (128 mg, 0.913 mmol) at room temperature under Ar. The resulting mixture was stirred at room temperature under H₂ for 3 h. The solid was filtered out and washed with MeOH (50 mL). The filtrate was concentrated under vacuum. The residue was dissolved in MeOH (3 mL) and added sodium borohydride (46.4 mg, 1.226 mmol) at 0 °C. The resulting mixture was stirred at room temperature for 2 h. The reaction was quenched by addition of *aq.* NH₄Cl (30 mL), diluted with ethyl acetate (30 × 3 mL), the organic layer was washed with water (60 mL) and brine (60 mL). The organic layer was dried over anhydrous Na₂SO₄, filtered and the filtrate was concentrated under reduced pressure. The crude material was purified by *RP-*flash chromatography, eluted with 0 - 50% acetonitrile in water to afford 10-benzyl-6,6-dimethyl-1,4,9-trioxadispiro[4.2.4⁸.2⁵]tetradecan-11-ol (190 mg, 0.572 mmol, 69.9 % yield) as a colorless oil. MS ESI calculated for C₂₀H₂₈O₄ [M + H]⁺ 333.21 found 333.20. ¹H NMR (300 MHz, Chloroform - *d*) δ 7.34-7.29 (m, 3H), 7.28-7.17 (m, 2H), 4.15-4.03 (m, 1H), 3.99-3.90 (m, 4H), 3.09-2.79 (m, 2H), 2.13-1.59 (m, 8H), 1.54-1.39 (m, 2H), 1.25 (s, 1H), 1.19 (s, 0.6H), 1.12 (s, 0.6H), 1.05 (s, 0.6H), 0.91 (s, 1H), 0.90 (s, 0.6H), 0.88 (s, 0.6H), 0.85 (s, 0.6H).

**Step 3:** To a stirred mixture of 10-benzyl-6,6-dimethyl-1,4,9-trioxadispiro[4.2.4⁸.2⁵]tetradecan-11-ol (260 mg, 0.782 mmol) in DCM (3 mL) were added pyridine (93 mg, 1.173 mmol) and trifluoromethanesulfonic anhydride (0.387 mL, 2.346 mmol) at -10 °C. The resulting mixture was stirred at -10 °C for 30 min. The reaction was quenched by addition of *aq.* NaHCO₃ (30 mL), extracted with DCM (3 × 50 mL), the organic layer was washed with water (50 mL) and brine (50 mL). The organic layer was dried over anhydrous Na₂SO₄, filtered and the filtrate was concentrated under reduced pressure to afford crude product 10-benzyl-6,6-dimethyl-1,4,9-trioxadispiro[4.2.4⁸.2⁵]tetradec-10-ene (252 mg, 0.827 mmol, 100% yield) as a yellow oil, which was used in the next step directly. MS ESI calculated for C₂₀H₂₆O₃ [M + H]⁺ 315.20 found 315.30.

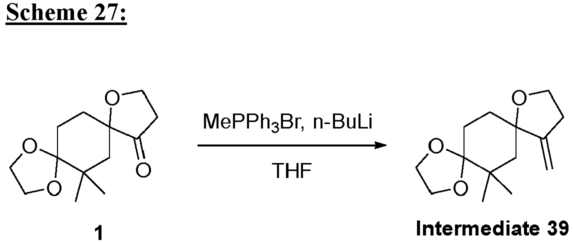

### Intermediate 39: 6,6-dimethyl-12-methylene-1,4,9-trioxadispiro[4.2.4⁸.2⁵]tetradecane

To a solution of methyltriphenylphosphonium bromide (2973 mg, 8.32 mmol) in THF (3 mL) was added n-BuLi (2.5 M in Hexane, 3.33 mL, 8.32 mmol) at 0 °C and the mixture was stirred at 30 °C for 30 min. Then a solution of 6,6-dimethyl-1,4,9-trioxadispiro[4.2.4⁸.2⁵]tetradecan-12-one (500 mg, 2.081 mmol) in THF (3.00 mL) was added to the above mixture at 0 °C. The sealed vial was irradiated in the microwave on a Biotage Smith Synthesizer at 80 °C for 8 hours. The reaction progress was monitored by LCMS and TLC. After completion of the reaction, the reaction mixture was quenched by aqueous NH₄Cl (5 mL). The mixture was concentrated in vacuo. The residue was diluted with ethyl acetate (80 mL), then washed with brine (5 mL × 3) and dried over anhydrous sodium sulfate and filtered. The filtrate was concentrated in vacuo, and the residue was purified by silica gel column chromatography using 0% - 30% gradient of ethyl acetate in petroleum ether as eluent. The fractions containing desired product were combined and concentrated under reduced pressure to afford 6,6-dimethyl-12-methylene-1,4,9-trioxadispiro[4.2.4⁸.2⁵]tetradecane (400 mg, 1.678 mmol, 81% yield) as a colorless oil. MS ESI calculated for C₁₄H₂₂O₃ [M + H]⁺ 239.16 found 239.20. ¹H-NMR (400 MHz, Chloroform-*d*) δ 4.95-4.87 (m, 1H), 4.86-4.78 (m, 1H), 4.05-3.88 (m, 4H), 3.87-3.75 (m, 2H), 2.67-2.49 (m, 2H), 2.14-1.92 (m, 1H), 1.76-1.59 (m, 3H), 1.56-1.43 (m, 2H), 1.21 (s, 3H), 0.85 (s, 3H).

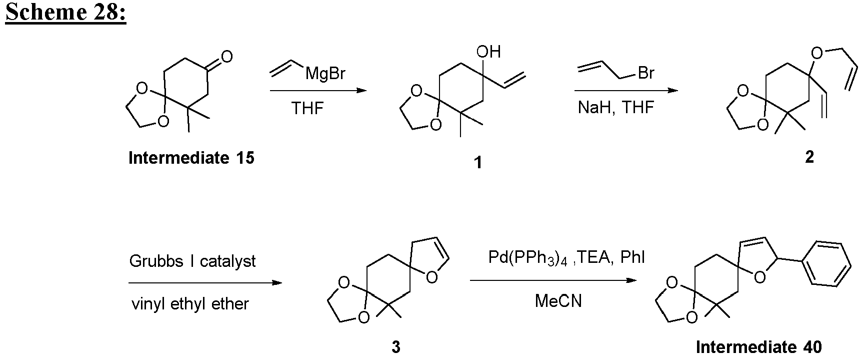

### Intermediate 40: 6,6-dimethyl-10-phenyl-1,4,9-trioxadispiro[4.2.4⁸.2⁵]tetradec-11-ene

**Step 1:** To a solution of 6,6-dimethyl-1,4-dioxaspiro[4.5]decan-8-one (6 g, 32.6 mmol) in tetrahydrofuran (60 mL) was added vinylmagnesium bromide (1 M in THF, 42.3 mL, 42.3 mmol) at 0 °C under atmosphere of argon. The resulting mixture was stirred at 25 °C for 1 h. The solution was neutralized by addition of saturated aqueous NH₄Cl (60 mL) and extracted with ethyl acetate (100 mL × 3). The combined organic layer was washed with brine (100 mL). The organic layer was dried over Na₂SO₄, filtered and the filtrate was concentrated under reduced pressure. The residue was purified by silica gel column chromatography, eluted with ethyl acetate / petroleum ether (0 - 10%) to afford 6,6-dimethyl-8-vinyl-1,4-dioxaspiro[4.5]decan-8-ol (5.2 g, 22.05 mmol, 67.7% yield) as a white solid. ¹H NMR (400 MHz, Chloroform-*d*) δ 5.94 (dd, *J =* 17.3, 10.7 Hz, 1H), 5.26 (dd, *J =* 17.3, 1.2 Hz, 1H), 5.03 (dd, *J =* 10.6, 1.2 Hz, 1H), 4.14- 3.84 (m, 4H), 2.12 (td, *J =* 13.2, 3.9 Hz, 1H), 1.89 (td, *J =* 13.5, 4.1 Hz, 1H), 1.78 (d, *J* = 14.4 Hz, 1H), 1.65-1.48 (m, 2H), 1.42 (dd, *J =* 14.4, 2.9 Hz, 1H), 1.28 (s, 3H), 0.90 (s, 3H).

**Step 2:** To a solution of 6,6-dimethyl-8-vinyl-1,4-dioxaspiro[4.5]decan-8-ol (1 g, 4.71 mmol) in tetrahydrofuran (10 mL) was added sodium hydride (60% in mineral oil, 0.414 g, 10.36 mmol) at 25 °C under atmosphere of argon. The resulting mixture was stirred at 66 °C for 0.5 h. Then the reaction was cooled to room temperature and 3-bromoprop-1-ene (1.425 g, 11.78 mmol) was added into the mixture. The resulting mixture was stirred for another 2 h at 66 °C. The solution was neutralized by addition of aqueous saturated NH₄Cl (10 mL) and extracted with ethyl acetate (30 mL × 3). The combined organic layer was washed with brine (30 mL). The organic layer was dried over Na₂SO₄, filtered and the filtrate was concentrated under reduced pressure. The residu was purified by silica gel column chromatography, eluted with ethyl acetate / petroleum ether (0 - 10%) to afford 8-(allyloxy)-6,6-dimethyl-8-vinyl-1,4-dioxaspiro[4.5]decane (1 g, 3.96 mmol, 84% yield) as a yellow oil. ¹H NMR (300 MHz, Chloroform-*d*) δ 5.92-5.80 (m, 1H), 5.79-5.70 (m, 1H), 5.35-5.21 (m, 1H), 5.20-5.04 (m, 3H), 4.15-3.84 (m, 4H), 3.82-3.81 (m, 2H), 2.10-1.70 (m, 4H), 1.63-1.52 (m, 1H), 1.50-1.48 (m, 1H), 1.24 (s, 3H), 0.90 (s, 3H).

**Step 3:** To a solution of 8-(allyloxy)-6,6-dimethyl-8-vinyl-1,4-dioxaspiro[4.5]decane (1g, 3.96 mmol) in toluene (10 mL) was added benzylidene-bis(tricyclohexylphosphine)dichlororuthenium (0.065 g, 0.079 mmol) at 25 °C under atmosphere of argon. The resulting mixture was stirred at 25 °C for 4 h. Then ethoxyethene (2.86 g, 39.6 mmol) was added. The mixture was stirred for 15 min and then heated to reflux for overnight. After cooling down to room temperature. The solution was diluted with brine (10 mL) and extracted with ethyl acetate (30 mL × 3). The combined organic layer was washed with brine (30 mL). The organic layer was dried over Na₂SO₄, filtered and the filtrate was concentrated under reduced pressure. The residue was purified by silica gel column chromatography, eluted with ethyl acetate / petroleum ether (0 - 10%) to afford 6,6-dimethyl-1,4,9-trioxadispiro[4.2.4⁸.2⁵]tetradec-10-ene (400 mg, 1.783 mmol, 45.0% yield) as a yellow oil. ¹H NMR (400 MHz, Chloroform-*d*) δ 6.20 (t, *J =* 2.5 Hz, 1H), 4.76 (q, *J* = 2.6 Hz, 1H), 4.04-3.81 (m, 4H), 2.37 (t, *J =* 2.4 Hz, 2H), 2.06-1.45 (m, 6H), 1.16 (s, 3H), 0.90 (s, 3H).

**Step 4:** To a solution of 6,6-dimethyl-1,4,9-trioxadispiro[4.2.4⁸.2⁵]tetradec-10-ene (500 mg, 2.229 mmol) in MeCN (5 mL) were added tetrakis(triphenylphosphine)palladium(0) (258 mg, 0.223 mmol), triethylamine (338 mg, 3.34 mmol) and iodobenzene (682 mg, 3.34 mmol) at 25 °C under atmosphere of argon. The resulting mixture was stirred at 100 °C for 16 h. The solution was neutralized by addition of aqueous saturated NH₄Cl (5 mL) and extracted with ethyl acetate (20 mL × 3). The combined organic layer was washed with brine (20 mL). The organic layer was dried over Na₂SO₄, filtered and the filtrate was concentrated under reduced pressure. The residue was purified by Column: XBridge Prep C¹⁸ OBD Column, 19 × 150 mm, 5 um; Mobile Phase A: Water, Mobile Phase B: MeCN; Flow rate: 30 mL/min; Gradient: 0 % B to 70 % B in 30 min, 65% B; detector: UV 220/254 nm to afford 6,6-dimethyl-10-phenyl-1,4,9-trioxadispiro[4.2.4⁸.2⁵]tetradec-11-ene (400 mg, 1.198 mmol, 53.8% yield) as a yellow oil. MS ESI calculated for C₁₉H₂₄O₃ [M + H]⁺ 301.17, found 301.25. ¹H NMR (300 MHz, DMSO-*d*₆) δ 7.36-7.25 (m, 5H), 5.92-5.87 (m, 2H), 5.75 (dd, *J =* 4.0, 2.2 Hz, 1H), 3.93-3.83 (m, 4H), 1.92-1.88 (m, 2H), 1.73-1.55 (m, 2H), 1.55-1.47 (m, 2H), 1.15 (s, 3H), 0.85 (s, 3H).

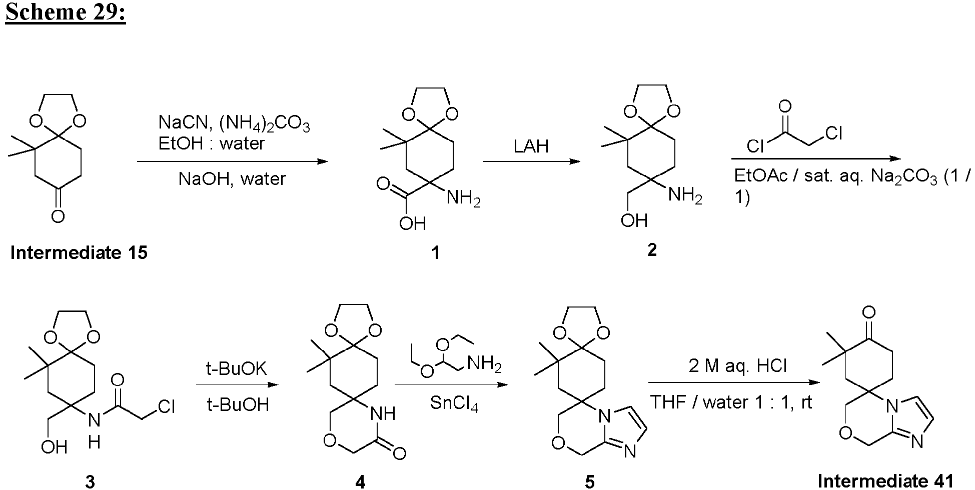

### Intermediate 41: 3,3-dimethyl-6'H,8'H-spiro[cyclohexane-1,5'-imidazo[2,1-c][1,4]oxazin]-4-one

**Step 1:** To a suspension of 6,6-dimethyl-1,4-dioxaspiro[4.5]decan-8-one (500 mg, 2.71 mmol) and ammonium carbonate (1304 mg, 13.57 mmol) in ethanol / water (1 : 1) (300 mL) was added sodium cyanide (133 mg, 2.71 mmol). The reaction mixture was heated at 50 °C for 12 h. After the starting material had disappeared as verified by TLC monitoring, the mixture was heated to 80 °C to let an excess of (NH₄)₂CO₃ decompose. Then NaOH (434 mg, 10.86 mmol) was added and ethanol was removed under reduced pressure and the resulting solution was refluxed overnight. The mixture was cooled to room temperature. The mixture was neutralized by adding 2 M HCl. The mixture was lyophilized twice to give 8-amino-6,6-dimethyl-1,4-dioxaspiro[4.5]decane-8-carboxylic acid (crude 2.71 mmol) as a white solid, which was used directly for next step without further purification. MS ESI calculated for C₁₁H₁₉NO₄ [M + H]⁺ 230.13 found 230.15.

**Step 2:** To a solution of 8-amino-6,6-dimethyl-1,4-dioxaspiro[4.5]decane-8-carboxylic acid (621 mg, 2.71 mmol) in ethyl ether (50 mL) was added LAH (206 mg, 5.42 mmol). The resulted mixture was stirred for 6 hours at 38 °C. The reaction progress was monitored by LCMS. After completion of the reaction, the reaction mixture was cooled to 0 °C then quenched with aqueous NH₄Cl (1 mL) and the mixture was stirred for 5 min, then 15% aq. NaOH (1 mL) was added and the mixture was stirred for another 5 min, then 3 mL water was added, the mixture was stirred for 15 min up to room temperature. Finally 3 g of NaSO₄ was added, the mixture was stirred for 15 min then filtered. The filtrate was concentrated under reduced pressure and the residue was purified by silica gel column chromatography using 0% - 20% gradient of MeOH (10% NH₃.H₂O) in DCM as eluent. The fractions containing desired product were combined and concentrated under reduced pressure to afford (8-amino-6,6-dimethyl-1,4-dioxaspiro[4.5]decan-8-yl)methanol (400 mg, 1.858 mmol, 68.6% yield) as a white solid. MS ESI calculated for C₁₁H₂₁NO₃ [M + H]⁺ 216.15 found 216.20. ¹H-NMR (300 MHz, DMSO-*d₆*) δ 4.08 (s, 1H), 3.93-3.71 (m, 4H), 3.17 (s, 2H), 3.00 (s, 2H), 1.92 (td, *J =* 12.7, 4.0 Hz, 1H), 1.56-1.32 (m, 3H), 1.30-1.16 (m, 1H), 1.19 (s, 3H), 1.11 (dd, *J =* 13.8, 2.4 Hz, 1H), 0.77 (s, 3H).

**Step 3:** A mixture of (8-amino-6,6-dimethyl-1,4-dioxaspiro[4.5]decan-8-yl)methanol (380 mg, 1.765 mmol) in ethyl acetate (7 mL) and saturated aqueous Na₂CO₃ (7 mL) was cooled to 0 °C and 2-chloroacetyl chloride (399 mg, 3.53 mmol) was added. The resulting mixture was stirred at 0 °C for 1 h. Major was desired product on LCMS & TLC. The mixture was extracted with ethyl acetate (50 mL × 3). The combined organic phase was washed with brine (10 mL), dried over anhydrous sodium sulfate. After filtration, the filtrate was concentrated under reduced pressure to give crude 2-chloro-*N*-(8-(hydroxymethyl)-6,6-dimethyl-1,4-dioxaspiro[4.5]decan-8-yl)acetamide (630 mg, 1.765 mmol) as a white solid, which was used for next step without further purification. MS ESI calculated for C₁₃H₂₂ClNO₄ [M + H]⁺ 292.12, 294.12 found 292.05, 294.05. ¹H-NMR (400 MHz, Chloroform-*d*) δ 6.63 (s, 1H), 4.03 (d, *J =* 2.4 Hz, 2H), 4.00-3.88 (m, 4H), 3.68 (s, 2H), 2.11-1.97 (m, 2H), 1.86-1.72 (m, 2H), 1.71-1.54 (m, 2H), 1.14 (s, 3H), 0.92 (s, 3H).

**Step 4:** To a solution of 2-chloro-*N*-(8-(hydroxymethyl)-6,6-dimethyl-1,4-dioxaspiro[4.5]decan-8-yl)acetamide (600 mg, 2.056 mmol) in *t*-BuOH (20 mL) was added potassium 2-methylpropan-2-olate (254 mg, 2.262 mmol) at room temperature. The resulting mixture was stirred at 30 °C for 3 h. Major desired product was detected on LCMS. The reaction mixture was diluted with NH₄Cl (5 mL), then extracted with ethyl acetate (50 mL × 3). The combined organic layers was dried over anhydrous sodium sulfate and filtered. The filtrate was concentrated in vacuo. The residue was purified by silica gel column chromatography using 10% - 40% gradient of ethyl acetate in petroleum ether as eluent. The fractions containing desired product were combined and concentrated under reduced pressure to afford 6,6-dimethyl-1,4,12-trioxa-9-azadispiro[4.2.5⁸.2⁵]pentadecan-10-one (500 mg, 1.958 mmol, 95% yield) as a brown oil. MS ESI calculated for C₁₃H₂₁NO₄ [M + H]⁺ 256.15 found 256.15. ¹H-NMR (400 MHz, Chloroform-*d*) δ 6.19 (s, 1H), 4.22-4.06 (m, 2H), 4.01-3.87 (m, 4H), 3.72 (d, *J =* 11.6 Hz, 1H), 3.52 (d, *J =* 11.6 Hz, 1H), 1.96-1.85 (m, 1H), 1.80-1.61 (m, 5H), 1.06 (s, 3H), 0.97 (s, 3H).

**Step 5:** A mixture of 6,6-dimethyl-1,4,12-trioxa-9-azadispiro[4.2.5⁸.2⁵]pentadecan-10-one (340 mg, 1.332 mmol), 2,2-diethoxyethan-1-amine (1.162 mL, 7.99 mmol) and perchlorostannane (1 M in DCM, 0.40 mL, 0.40 mmol) in toluene (3.6 mL) was heated to 150 °C and the resulting mixture was stirred for 48 hours in a sealed tube. Major was product on LCMS. The reaction mixture was concentrated in vacuo to give a residue (1.332 mmol) as a black solid, which was used directly for next step without further purification. MS ESI calculated for C₁₅H₂₂N₂O₃ [M + H]⁺ 279.16 found 279.10.

**Step 6:** To a solution of 2',2'-dimethyl-6*H,*8*H*-dispiro[imidazo[2,1-*c*][1,4]oxazine-5,4'-cyclohexane-1',2"-[1,3]dioxolane] (371 mg, 1.332 mmol) in THF (3 mL) was added HCl (2 M in water, 4 mL, 8.00 mmol). The mixture was stirred for 3 hours at 25 °C. The mixture was neutralized by adding NaHCO₃. The filtrate was concentrated under reduced pressure and the residue was purified by silica gel column chromatography using 0% - 10% gradient of methanol in DCM as eluent. The fractions containing desired product were combined and concentrated under reduced pressure to afford 3,3-dimethyl-6'*H*,8'*H*-spiro[cyclohexane-1,5'-imidazo[2,1-*c*][1,4]oxazin]-4-one (50 mg, 0.213 mmol, 16.02% yield) as a white solid. MS ESI calculated for C₁₃H₁₈N₂O₂ [M + H]⁺ 235.15 found 235.10. ¹H-NMR (300 MHz, Chloroform-*d*) δ 7.06 (s, 1H), 6.99 (s, 1H), 4.90 (s, 2H), 4.19 (d, *J =* 12.3 Hz, 1H), 4.02 (d, *J =* 12.3 Hz, 1H), 2.80-2.63 (m, 1H), 2.62-2.47 (m, 1H), 2.48-2.28 (m, 2H), 2.24 (s, 2H), 1.27 (s, 3H), 1.23 (s, 3H).

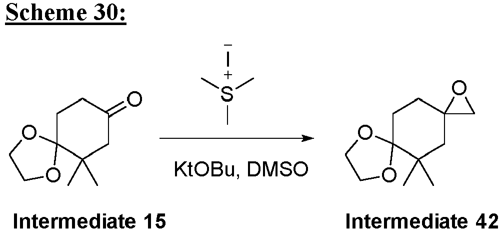

### Intermediate 42: 5,5-dimethyl-1,7,10-trioxadispiro[2.2.4⁶.2³]dodecane

To a solution of trimethylsulfonium iodide (3.59 g, 17.59 mmols) and 6,6-dimethyl-1,4-dioxaspiro[4.5]decan-8-one (2.0 g, 10.86 mmols) in dry DMSO (54.2 mL), potassium tert-butoxyde (1.827 g, 16.28 mmols) was added and the resulting reaction mixture was stirred at *r.t.* overnight under inert atmosphere. The reaction was cooled down to 0 °C and quenched with H₂O (20 mL). The organic phase was extracted with MTBE (3 x 25 mL). The combined organic layers were washed with H₂O (2 x 20 mL) and then dried over anhydrous MgSO4, filtrated and concentrated under reduced pressure to afford 5,5-dimethyl-1,7,10-trioxadispiro[2.2.4⁶.2³]dodecane as a yellowish oil (1.78 g, 83% IY). ¹H NMR (400 MHz, Chloroform-d) δ 3.98 - 3.95 (m, 4H), 2.58 (m, 2H), 1.94 - 1.87 (m, 1H), 1.84 - 1.77 (m, 1H), 1.76 - 1.65 (m, 2H), 1.64 - 1.57 (m, 1H), 1.51 (d, J = 13.6 Hz, 1H), 1.07 (s, 3H), 0.97 (s, 3H).

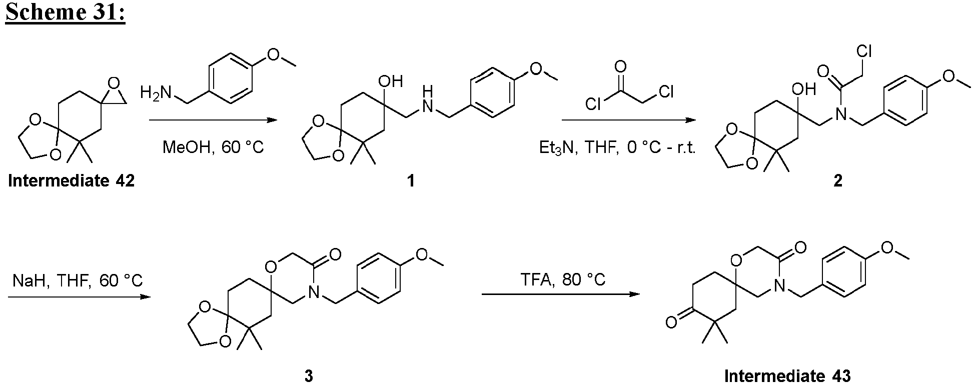

### Intermediate 43: 4-(4-methoxybenzyl)-8,8-dimethyl-1-oxa-4-azaspiro[5.5]undecane-3,9-dione

**Step 1:** To a solution of 5,5-dimethyl-1,7,10-trioxadispiro[2.2.46.23]dodecane (1.0 g, 5.04 mmols) in methanol (10.01 mL), (4-methoxyphenyl)methanamine (1.450 mL, 11.10 mmols) was added and the resulting reaction mixture was stirred at 60 °C overnight. The reaction mixture was allowed to cool down to *r.t.* and concentrated under reduced pressure. The residue was purified by column chromatography on silica (0-5% MeOH/DCM) to afford 8-(((4-methoxybenzyl)amino)methyl)-6,6-dimethyl-1,4-dioxaspiro[4.5]decan-8-ol as a transparent oil (1.11 g, 66% IY). MS = 336 [M + H]. ¹H NMR (400 MHz, Chloroform-d) δ 7.23 (m, 2H), 6.86 (m, 2H), 3.97 - 3.83 (m, 4H), 3.80 (s, 3H), 3.76 (s, 2H), 2.49 (s, 2H), 2.12-2.04 (m, 1H), 1.65 - 1.52 (m, 2H), 1.52 - 1.43 (m, 2H), 1.43 - 1.38 (m, 1H), 1.25 (s, 3H), 0.84 (s, 3H).

**Step 2:** To a solution of 8-(((4-methoxybenzyl)amino)methyl)-6,6-dimethyl-1,4-dioxaspiro[4.5]decan-8-ol (1.11 g, 3.31 mmols) and triethylamine (0.484 mL, 3.47 mmols) in dry THF (44.1 mL) 2-chloroacetyl chloride (0.264 mL, 3.31 mmols) was added at 0 °C under a nitrogen stream. The resulting reaction mixture was stirred at 0 °C for 1h and then at *r.t.* for 3h under inert atmosphere. The reaction mixture was diluted with H₂O (15 mL) and the organic phase was extracted with DCM (3 x 20 mL). The combined organic layers were dried over anhydrous MgSO4, filtrated and concentrated under reduced pressure to afford 2-chloro-N-((8-hydroxy-6,6-dimethyl-1,4-dioxaspiro[4.5]decan-8-yl)methyl)-N-(4-methoxybenzyl)acetamide as a transparent oil. MS = 412 [M + H]. The crude material was used in the next step without any further purification.

**Step 3:** To a solution of 2-chloro-N-((8-hydroxy-6,6-dimethyl-1,4-dioxaspiro[4.5]decan-8-yl)methyl)-N-(4-methoxybenzyl)acetamide (1.36 g, 3.30 mmols) in dry THF (33.3 mL), NaH (60% in mineral oil) (172 mg, 4.29 mmols) was added at 0 °C under a nitrogen stream. The reaction mixture was stirred at 0 °C for 15 min. and then at 60 °C overnight under inert atmosphere. The reaction mixture was quenched at 0 °C with H₂O (20 mL). The organic phase was extracted with DCM (3 x 30 mL). The combined organic layers were dried over anhydrous MgSO4, filtrated and concentrated under reduced pressure. The residue was purified by column chromatography on silica (0-100% MTBE/Heptane) to afford 12-(4-methoxybenzyl)-6,6-dimethyl-1,4,9-trioxa-12-azadispiro[4.2.5⁸.2⁵]pentadecan-11-one as a transparent oil which solidifies in contact with air (822 mg, 66% IY). MS = 376 [M + H]. ¹H NMR (400 MHz, Chloroform-d) δ 7.17 (m, 2H), 6.85 (m, 2H), 4.55 - 4.46 (m, 2H), 4.27 - 4.15 (m, 2H), 3.98 - 3.82 (m, 4H), 3.80 (s, 3H), 3.02 - 2.92 (m, 2H), 1.97 - 1.90 (m, 1H), 1.85 - 1.80 (m, 1H), 1.78 - 1.73 (m, 1H), 1.54 - 1.40 (m, 2H), 1.31 (m, 1H), 1.18 (s, 3H), 0.81 (s, 3H).

**Step 4:** 12-(4-methoxybenzyl)-6,6-dimethyl-1,4,9-trioxa-12-azadispiro[4.2.5⁸.2⁵]pentadecan-11-one (200 mg, 0.533mmols) was dissolved in TFA (4.0 mL, 52.2 mmols) and the resulting reaction mixture was sealed and stirred at 80 °C for 3h. The reaction mixture was allowed to cool down to *r.t.* and neutralized with a sat. aq. NaHCO₃ solution. The organic phase was extracted with DCM (3 x 15 mL). The combined organic layers were dried over anhydrous MgSO4, filtrated and concentrated under reduced pressure. The residue was purified by column chromatography on silica (0-100% MTBE/Heptane) to afford 4-(4-methoxybenzyl)-8,8-dimethyl-1-oxa-4-azaspiro[5.5]undecane-3,9-dione as a transparent oil (142 mg, 80% IY). MS = 332 [M + H]. ¹H NMR (400 MHz, Chloroform-d) δ 7.20 - 7.16 (m, 2H), 6.88 - 6.84 (m, 2H), 4.61 (d, J = 14.4 Hz, 1H), 4.47 (d, J = 14.4 Hz, 1H), 4.36 - 4.22 (m, 2H), 3.80 (s, 3H), 3.10 - 3.00 (m, 2H), 2.84 - 2.76 (m, 1H), 2.26 - 2.15 (m, 2H), 2.06 (dd, J = 14.8, J' = 3.6 Hz, 1H), 1.62 - 1.53 (m, 1H), 1.41 (d, J = 14.8 Hz, 1H), 1.28 (s, 3H), 1.00 (s, 3H).

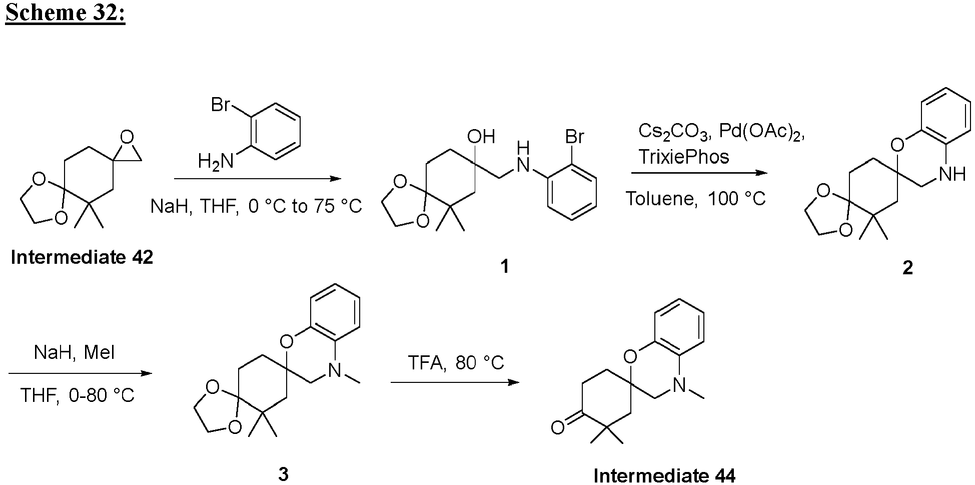

### Intermediate 44: 3',3',4-trimethyl-3,4-dihydrospiro[benzo[b][1,4]oxazine-2,1'-cyclohexan]-4'-one

**Step 1:** In a microwave vial, to a solution of 2-bromoaniline (837 mg, 4.86 mmol) in dry THF (6 mL), sodium hydride (389 mg, 9.73 mmol) (60% in mineral oils) was added at 0 °C under a nitrogen stream and the resulting suspension was stirred at 0 °C for 30'. Then, a solution of 5,5-dimethyl-1,7,10-trioxadispiro[2.2.4⁶.2³]dodecane (643 mg, 3.24 mmol) in dry THF (6 mL) was added. The resulting reaction mixture was sealed and stirred at 75 °C overnight. After LCMS control, an additional aliquot of sodium hydride (389 mg, 9.73 mmol) (60% in mineral oils) was added and the reaction mixture was stirred at 75 °C until completion. The reaction mixture was quenched with H₂O at 0 °C and the organic phase was extracted with EtOAc (3 x 15 mL). The combined organic layers were dried over anhydrous MgSO₄, filtrated and concentrated under reduced pressure. The residue was purified by column chromatography on silica (0-50% MTBE/Heptane) to afford 8-(((2-bromophenyl)amino)methyl)-6,6-dimethyl-1,4-dioxaspiro[4.5]decan-8-ol as a yellowish oil which solidifies in contact with air (1.06 g, 88% IY). MS = 371 [M + H]. ¹H NMR (400 MHz, Chloroform-d) δ 7.43 (d, J = 7.9 Hz, 1H), 7.17 (t, J = 8.0 Hz, 1H), 6.82 (d, J = 8.0 Hz, 1H), 6.62 (t, J = 7.4 Hz, 1H), 4.00 - 3.88 (m, 4H), 3.12 (s, 2H), 2.12 - 2.05 (m, 1H), 1.84 - 1.72 (m, 2H), 1.68 (d, J = 14.2 Hz, 1H), 1.59 - 1.54 (m, 1H), 1.54 - 1.49 (m, 1H), 1.26 (s, 3H), 0.88 (s, 3H).

**Step 2:** In a microwave vial, to a solution of 8-(((2-bromophenyl)amino)methyl)-6,6-dimethyl-1,4-dioxaspiro[4.5]decan-8-ol (1.43 g, 3.86 mmol) in dry toluene (19.31 ml), cesium carbonate (2.52 g, 7.72 mmol) was added and the resulting mixture was degassed for 10 min. Then, palladium (II) acetate (0.130 g, 0.579 mmol) and [1,1'-binaphtalene]-2-yldi-tert-butylphosphine (0.308 g, 0.772 mmol) were added under a nitrogen stream. The reaction mixture was sealed and stirred at 100 °C overnight under inert atmosphere. The reaction mixture was allowed to cool down to *r.t.,* diluted with EtOAc and filtrated under vacuum through celite. The residue was purified by column chromatography on basic alumina (0-50% MTBE/Heptane) to afford 2',2'-dimethyl-3,4-dihydrodispiro[benzo[b][1,4]oxazine-2,4'-cyclohexane-1',2"-[1,3]dioxolane as a pale solid (420 mg, 38% IY). MS = 290 [M + H]. ¹H NMR (400 MHz, Chloroform-d) δ 6.78 (t, J = 7.4 Hz, 2H), 6.70 (t, J = 7.5 Hz, 1H), 6.65 (d, J = 7.5 Hz, 1H), 4.05 - 3.89 (m, 4H), 3.05 (s, 2H), 2.24 - 2.16 (m, 1H), 1.87 - 1.82 (m, 1H), 1.76 - 1.72 (m, 2H), 1.57 - 1.49 (m, 2H), 1.23 (s, 3H), 0.85 (s, 3H).

**Step 3:** In a microwave vial, to a solution of 2',2'-dimethyl-3,4-dihydrodispiro[benzo[b][1,4]oxazine-2,4'-cyclohexane-1',2"-[1,3]dioxolane] (250 mg, 0.864 mmol) in dry THF (8 mL), sodium hydride (138 mg, 3.46 mmol) (60% in mineral oil) was added at 0 °C under a nitrogen stream and the resulting suspension was stirred at 0 °C for 1h. Then, iodomethane (0.161 mL, 2.59 mmol) was added and the resulting reaction mixture was sealed and stirred at 45 °C for 48h. After LCMS control, additional aliquots of sodium hydride (138 mg, 3.46 mmol) and iodomethane (0.161 mL, 2.59 mmol) were added and the reaction was stirred at 80 °C overnight. The reaction mixture was allowed to cool down to *r.t.* and quenched at 0 °C with H₂O (15 mL). The organic phase was extracted with DCM (3 x 15 mL). The combined organic layers were dried over anhydrous MgSO4, filtrated and concentrated under reduced pressure. The residue was purified by column chromatography on silica (0-50% MTBE/Heptane) to afford 2',2',4-trimethyl-3,4-dihydrodispiro[benzo[b][1,4]oxazine-2,4'-cyclohexane-1',2"-[1,3]dioxolane as a transparent oil (212 mg, 81% IY). MS = 304 [M + H]. ¹H NMR (400 MHz, Chloroform-d) δ 6.86 (t, J = 7.6 Hz, 1H), 6.78 (d, J = 7.7 Hz, 1H), 6.71 - 6.67 (m, 2H), 4.02 - 3.88 (m, 4H), 2.92 (d, J = 5.0 Hz, 2H), 2.89 (s, 3H), 2.24 - 2.16 (m, 1H), 1.86 - 1.81 (m, 1H), 1.77 - 1.69 (m, 2H), 1.58 - 1.53 (m, 2H), 1.23 (s, 3H), 0.85 (s, 3H).

**Step 4:** 2',2',4-trimethyl-3,4-dihydrodispiro[benzo[b][1,4]oxazine-2,4'-cyclohexane-1',2"-[1,3]dioxolane] (212 mg, 0.699 mmol) was dissolved in trifluoroacetic acid (5247 µl, 68.5 mmol) and the resulting reaction mixture was sealed and stirred at 80 °C for 3 h. The reaction mixture was allowed to cool down to *r.t.* and neutralized with a sat. aq. NaHCO₃ solution. The organic phase was extracted with DCM (3 x 15 mL). The combined organic layers were dried over anhydrous MgSO₄, filtrated and concentrated under reduced pressure to afford 3',3',4-trimethyl-3,4-dihydrospiro[benzo[b][1,4]oxazine-2,1'-cyclohexan]-4'-one as a dark solid. MS = 260 [M + H]. ¹H NMR (400 MHz, Chloroform-d) δ 6.90 (t, J = 7.6 Hz, 1H), 6.84 (d, J = 7.7 Hz, 1H), 6.75 - 6.70 (m, 2H), 3.15 - 3.06 (m, 1H), 2.99 (s, 2H), 2.92 (s, 3H), 2.29 - 2.20 (m, 2H), 2.16 - 2.11 (m, 1H), 1.87 - 1.78 (m, 1H), 1.63 (d, J = 14.7 Hz, 1H), 1.35 (s, 3H), 1.05 (s, 3H). The crude material was used in the next step without any further purification.

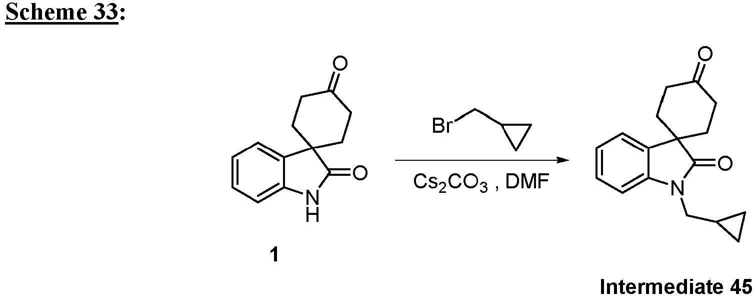

### Intermediate 45: 1'-(cyclopropylmethyl)spiro[cyclohexane-1,3'-indoline]-2',4-dione

**Step 1:** To a stirred solution of spiro[cyclohexane-1,3'-indoline]-2',4-dione (860 mg, 4 mmol) in DMF (6 mL) was added (bromomethyl)cyclopropane (701 mg, 5.19 mmol) and Cs₂CO₃ (2083 mg, 6.39 mmol) at room temperature. Then the reaction mixture was stirred at 50 °C for 1 h. The mixture was cooled to room temperature, diluted with water (10 mL), and extracted with ethyl acetate (100 mL × 2). The organic layer was washed with water (50 mL) and brine (50 mL), dried over anhydrous sodium sulfate and filtered. The filtrate was concentrated under reduced pressure, and the residue was purified by column chromatography on silica (0-50% EtOAc/PE) to afford 1'-(cyclopropylmethyl)spiro[cyclohexane-1,3'-indoline]-2',4-dione as a solid. MS: 270 (M + 1). ¹H-NMR (400 MHz, Chloroform-d) δ 7.33 - 7.29 (m, 1H), 7.26 - 7.24 (m, 1H), 7.10 - 7.08 (m, 1H), 6.99 - 6.97 (m, 1H), 3.64 (d, *J =* 6.8 Hz, 2H), 3.20 - 3.14 (m, 2H), 2.51 - 2.47 (m, 2H), 2.18 - 2.15 (m, 4H), 1.22 - 1.18 (m, 1H), 0.56 - 0.52 (m, 2H), 0.42 - 0.38 (m, 2H).

**Table 6: The following intermediates were prepared using a similar procedure as described above for intermediate 45.**

| **Int. #** | **Structure** | **Compound Name** | **Exact Mass [M+H]⁺** |
|---|---|---|---|
| **46** | 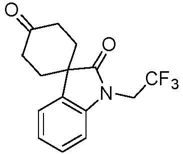 | 1'-(2,2,2-trifluoroethyl)spiro[cyclohexane -1,3'-indoline]-2',4-dione | 298 |
| **47** | 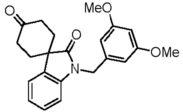 | 1'-(3,5-dimethoxybenzyl)spiro[cyclohex ane-1,3'-indoline]-2',4-dione | 366 |
| **48** | 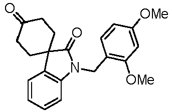 | 1'-(2,4-dimethoxybenzyl)spiro[cyclohex ane-1,3'-indoline]-2',4-dione | 366 |

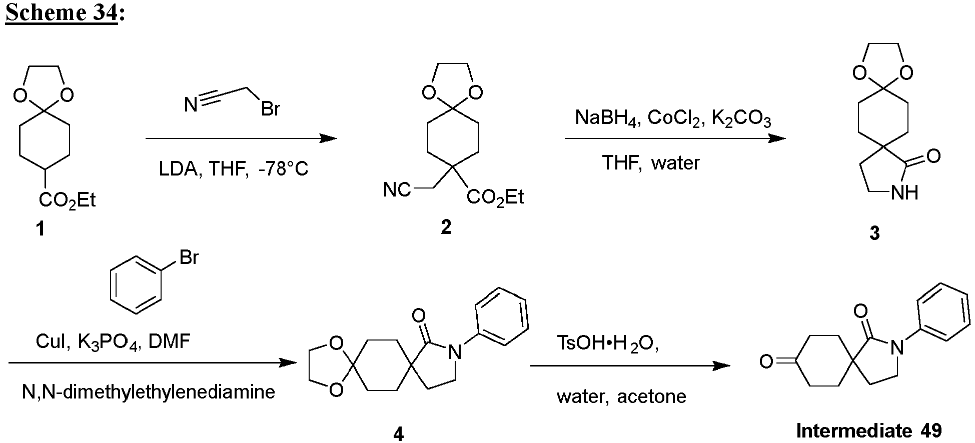

### Intermediate 49: 2-phenyl-2-azaspiro[4.5]decane-1,8-dione

**Step 1:** Added THF (15.5 mL) and LDA (12.8 mL, 25.7 mmol) to a degassed 100 mL RBF and cooled to -78 °C. Added a solution of ethyl 1,4-dioxaspiro[4.5]decane-8-carboxylate (5 g, 23 mmol) in THF (31 mL) to the flask dropwise while stirring. The mixture was stirred at -78 °C for 30 minutes. Added a solution of bromoacetonitrile (1.95 ml, 28.0 mmol) in THF (31 mL) to the flask at -78 °C and the reaction was stirred for 2 h at -78 °C. The reaction was quenched with saturated aqueous ammonium chloride and extracted with EtOAc (3x). The combined organic layers were washed with brine, dried over anhydrous sodium sulfate, filtered, and concentrated under reduced pressure. The residue was purified by column chromatography on silica (0-30% EtOAc/Hexanes) monitoring with ELSD to afford ethyl 8-(cyanomethyl)-1,4-dioxaspiro[4.5]decane-8-carboxylate as an oil. MS: 254 (M + 1).

**Step 2:** Sodium borohydride (2.15 g, 56.7 mmol) was added in portions to a mixture of ethyl 8-(cyanomethyl)-1,4-dioxaspiro[4.5]decane-8-carboxylate (2.87 g, 11.3 mmol) and cobalt(II) chloride (0.736 g, 5.67 mmol) in THF (44 mL) and water (22 mL) at 0 °C. The mixture was stirred overnight at room temperature. The reaction was quenched with 2M NH₄OH (5 mL) and the mixture was filtered. The solid was washed with 2:1 THF:water, and the filtrate was concentrated under reduced pressure. The aqueous solution was extract with DCM (3x), and the combined organic layers were dried over anhydrous sodium sulfate, filtered, and concentrated under reduced pressure. The residue was purified by column chromatography on silica (0-10% MeOH/DCM) to afford 1,4-dioxa-10-azadispiro[4.2.4⁸.2⁵]tetradecan-9-one as a solid. MS: 212 (M + 1).

**Step 3:** 1,4-dioxa-10-azadispiro[4.2.4⁸.2⁵]tetradecan-9-one (908 mg, 4.30 mmol) was dissolved in DMF (43 mL) at room temperature under nitrogen. Bromobenzene (0.675 ml, 6.45 mmol), N,N-dimethylethylenediamine (0.939 ml, 8.60 mmol), copper(I) iodide (1.23 g, 6.45 mmol), and potassium phosphate (1.62 g, 7.61 mmol) were added and the mixture was heated at 100 °C overnight.The reaction was cooled to room temperature, poured into ice water, and extracted with EtOAc (3x). The combined organic layers were washed with brine, dried over anhydrous sodium sulfate, filtered, and concentrated under reduced pressure. The residue was purified by column chromatography on silica (0-100% EtOAc/Hexanes) to afford 10-phenyl-1,4-dioxa-10-azadispiro[4.2.4⁸.2⁵]tetradecan-9-one as a solid. MS: 288 (M + 1).

**Step 4:** To a solution of 10-phenyl-1,4-dioxa-10-azadispiro[4.2.4⁸.2⁵]tetradecan-9-one (1.4 g, 4.9 mmol) in acetone (24 mL) was added *p*-toluenesulfonic acid monohydrate (2.04 g, 10.7 mmol) and water (4.39 ml, 244 mmol). The mixture was heated at 70 °C overnight. The reaction was quenched with saturated aqueous sodium bicarbonate, and the aqueous layer was extracted with DCM (3x). The combined organic layers were dried over anhydrous sodium sulfate, filtered, and concentrated under reduced pressure. The residue was purified by column chromatography on silica (0-100% EtOAc/Hexanes) to afford 2-phenyl-2-azaspiro[4.5]decane-1,8-dione as a solid. MS: 244 (M + 1).

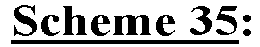

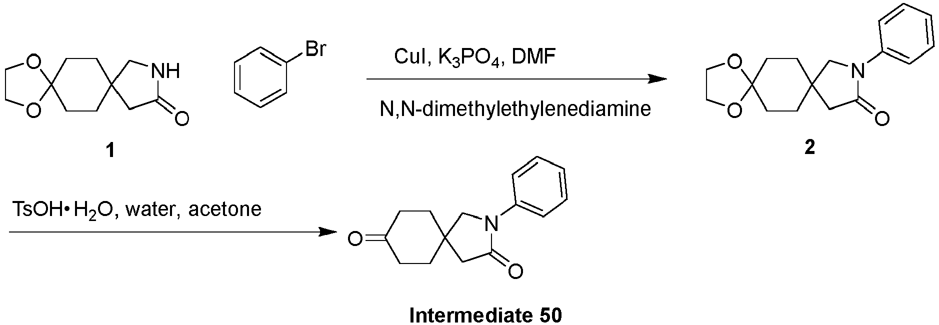

### Intermediate 50: 2-phenyl-2-azaspiro[4.5]decane-3,8-dione

**Step 1:** To a flask was added 1,4-dioxa-10-azadispiro[4.2.4⁸.2⁵]tetradecan-11-one (1 g, 4.7 mmol), copper(I) iodide (1.35 g, 7.1 mmol), potassium phosphate (1.80 g, 8.38 mmol), bromobenzene (0.744 mL, 7.1 mmol), and N,N-dimethylethylenediamine (1.03 mL, 9.46 mmol). DMF (48 mL) were added and the mixture was stirred at 100 °C overnight. The reaction was cooled to room temperature, diluted with water, and extracted with EtOAc (3x). The combined organic layers were washed with brine, dried over anhydrous sodium sulfate, filtered, and concentrated under reduced pressure. The residue was purified by column chromatography on silica (30-100% EtOAc/Hexanes) to afford 10-phenyl-1,4-dioxa-10-azadispiro[4.2.4⁸.2⁵]tetradecan-11-one as a solid. MS: 288 (M + 1).

**Step 2:** To a solution of 10-phenyl-1,4-dioxa-10-azadispiro[4.2.4⁸.2⁵]tetradecan-11-one (1.19 g, 4.16 mmol) in acetone (21 mL) was added *p*-toluenesulfonic acid monohydrate (1.74 g, 9.15 mmol) and water (3.75 ml, 208 mmol). The mixture was heated at 70 °C overnight. The reaction was quenched with saturated aqueous sodium bicarbonate, extract DCM (3x), and the combined organic layers were dried over anhydrous sodium sulfate, filtered, and concentrated under reduced pressure. The residue was purified by column chromatography on silica (20-100% EtOAc/Hexanes) to afford 2-phenyl-2-azaspiro[4.5]decane-3,8-dione as a solid. MS: 244 (M + 1).

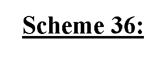

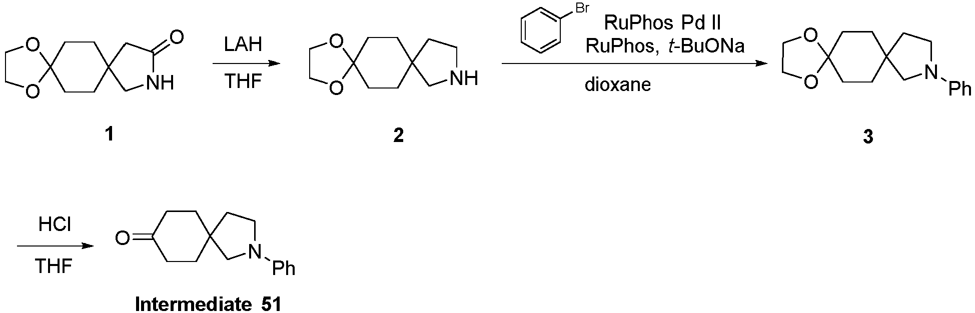

### Intermediate 51: 2-phenyl-2-azaspiro[4.5]decan-8-one

**Step 1:** To a solution of 1,4-dioxa-10-azadispiro[4.2.48.25]tetradecan-11-one (1.08 g, 5.11 mmol) in THF (10 mL) was added lithium aluminium hydride (0.388 g, 10.22 mmol) at 0 °C under N₂ atmosphere. The resulting mixture was stirred at room temperature for 16 h. Then the mixture was quenched by addition of water (0.39 mL), 15% aqueous NaOH (0.39 mL) and water (1.17 mL) at 0 °C. The resulting mixture was stirred at room temperature for 1 h. Then the mixture was filtered. The filtrate was concentrated under reduced pressure to give 1,4-dioxa-10-azadispiro[4.2.48.25]tetradecane (900 mg, 4.56 mmol, 89% yield) as a light yellow oil. MS ESI calculated for C₁₁H₁₉NO₂ [M + H]⁺ 198.14, found 198.25. ¹H NMR (300 MHz, Chloroform-*d*): δ 3.96 (s, 4H), 2.99 (t, *J =* 6.9 Hz, 2H), 2.74 (s, 2H), 1.74-1.56 (m, 10H).

**Step 2:** To a mixture of 1,4-dioxa-10-azadispiro[4.2.48.25]tetradecane (18 g, 91 mmol), [2'-(amino-n)[1,1'-biphenyl]-2-yl-c][[2',6'-bis(1-methylethoxy)[1,1'-biphenyl]-2-yl]dicyclohexylphosphine-p]chloro-palladium (7.09 g, 9.12 mmol) and Ruphos (4.26 g, 9.12 mmol) in 1,4-dioxane (180 mL) were added bromobenzene (17.19 g, 109 mmol) and sodium 2-methylpropan-2-olate (17.54 g, 182 mmol) under N₂ atmosphere. The resulting mixture was heated to 70 °C and stirred for 16 h. Then the mixture was cooled to room temperature, concentrated under reduced pressure and the residue was purified by column chromatography on silica gel, eluted with 0 - 30% ethyl acetate in petroleum ether to give 10-phenyl-1,4-dioxa-10-azadispiro[4.2.48.25]tetradecane (13.5 g, 49.4 mmol, 54.1% yield) as a white solid. MS ESI calculated for C₁₇H₂₃NO₂ [M + H]⁺ 274.17, found 274.20. ¹H NMR (300 MHz, Chloroform-*d*): δ 7.30-7.18 (m, 2H), 6.68 (t, *J =* 7.5 Hz, 1H), 6.57 (d, *J =* 8.1 Hz, 2H), 3.97 (s, 4H), 3.37 (t, *J =* 6.9 Hz, 2H), 3.18 (s, 2H), 1.89 (t, *J =* 6.9 Hz, 2H), 1.80-1.60 (m, 8H).

**Step 3:** To a solution of 10-phenyl-1,4-dioxa-10-azadispiro[4.2.48.25]tetradecane (13.4 g, 49.0 mmol) in THF (123 mL) was added hydrochloric acid (2 M, 123 mL, 245 mmol) at 0 °C. The resulting mixture was stirred at 25°C for 5 h. Then the pH value of the mixture was adjusted to 8 with saturated aqueous NaHCO₃. The mixture was diluted with water (200 mL), extracted with ethyl acetate (200 mL × 3). The combined organic phase was washed with brine (250 mL), dried over anhydrous sodium sulfate. After filtration, the filtrate was concentrated under reduced pressure to give 2-phenyl-2-azaspiro[4.5]decan-8-one (11.24 g, 49.0 mmol, 100% yield) as a yellow solid. MS ESI calculated for C₁₅H₁₉NO [M + H]⁺ 230.15, found 230.20. ¹H NMR (300 MHz, Chloroform-*d*): δ 7.35-7.20 (m, 2H), 6.72 (t, *J =* 7.2 Hz, 1H), 6.66-6.55 (m, 2H), 3.46 (t, *J* = 6.9 Hz, 2H), 3.32 (s, 2H), 2.43 (t, *J =* 6.9 Hz, 4H), 2.12-1.83 (m, 6H).

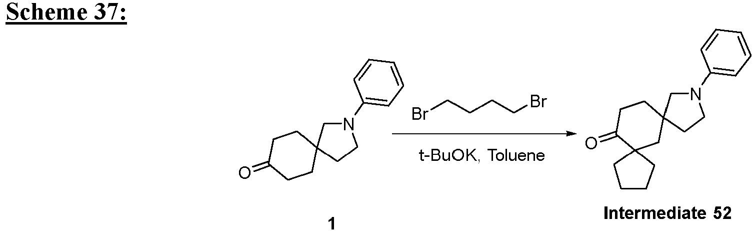

### Intermediate 52: 2-phenyl-2-azadispiro[4.1.47.35]tetradecan-12-one

**Step 1:** A mixture of 2-phenyl-2-azaspiro[4.5]decan-8-one (690 mg, 3.01 mmol), 1,4-dibromobutane (682 mg, 3.16 mmol) and potassium 2-methylpropan-2-olate (709 mg, 6.32 mmol) in toluene (12 mL) was heated to reflux and stirred for 4 h. Then the mixture was cooled to room temperature, diluted with ethyl acetate (40 mL). The mixture was filtered and the filtrate was concentrated under reduced pressure and the residue was purified by reverse phase chromatography with the following conditions: Column: C¹⁸ gel column, 80 g, 20-45 um, 100 A; Mobile Phase A: water, Mobile Phase B: MeCN; Gradient: 0% B hold 5 min, up to 45% B within 5 min, 45% B to 95% B within 30 min, 95%B hold 5 min; Flow rate: 65 mL/min; RT = 25 min; Detector: UV 254 & 210 nm. The product-containing fractions were collected and evaporated to afford 2-phenyl-2-azadispiro[4.1.47.35]tetradecan-12-one (340 mg, 1.200 mmol, 39.9% yield) as a brown solid. MS ESI calculated for C₁₉H₂₅NO [M + H]⁺ 284.19, found 284.20. ¹H NMR (300 MHz, Chloroform-*d*): δ 7.32-7.24 (m, 2H), 6.70 (t, *J =* 7.2 Hz, 1H), 6.65-6.54 (m, 2H), 3.51-3.18 (m, 4H), 2.64-2.40 (m, 2H), 2.29-1.31 (m, 14H).

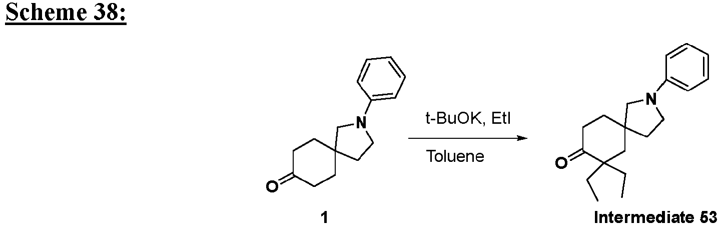

### Intermediate 53: 7,7-diethyl-2-phenyl-2-azaspiro[4.5]decan-8-one

**Step 1:** To a solution of 2-phenyl-2-azaspiro[4.5]decan-8-one (1000 mg, 4.36 mmol) and potassium *tert*-butoxide (489 mg, 4.36 mmol) in toluene (10 mL) was added dropwise iodoethane (1428 mg, 9.16 mmol) at 25 °C. The resulting mixture was stirred at 60 °C for 4 h. After completion of the reaction, the reaction mixture was quenched with aqueous NH₄Cl (10 mL) and diluted with ethyl acetate (300 mL), then washed with brine (50 mL), dried over anhydrous sodium sulfate and filtered. The filtrate was concentrated under reduced pressure and the residue was purified by silica gel column chromatography using 0% - 20% gradient of ethyl acetate in petroleum ether as eluent. The fractions containing desired product were combined and concentrated under reduced pressure to afford 7,7-diethyl-2-phenyl-2-azaspiro[4.5]decan-8-one (460 mg, 1.612 mmol, 37.0% yield) as a yellow oil. MS ESI calculated for C₁₉H₂₇NO [M + H]⁺ 286.21 found 286.15. ¹H-NMR (400 MHz, Chloroform-*d*) δ 7.28-7.22 (m, 2H), 6.75-6.67 (m, 1H), 6.63-6.55 (m, 2H), 3.49-3.32 (m, 3H), 3.22 (d, *J =* 9.2 Hz, 1H), 2.55-2.33 (m, 2H), 2.05-1.99 (m, 2H), 1.98-1.89 (m, 1H), 1.87 (d, *J =* 1.8 Hz, 2H), 1.85-1.75 (m, 1H), 1.75-1.64 (m, 2H), 1.58-1.49 (m, 2H), 0.87-0.72 (m, 6H).

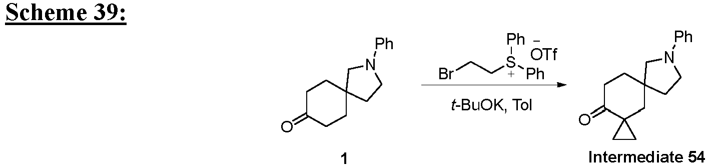

### Intermediate 54: 7-phenyl-7-azadispiro[2.1.45.33]dodecan-12-one

**Step 1:** To a solution of 2-phenyl-2-azaspiro[4.5]decan-8-one (2 g, 8.72 mmol) and potassium 2-methylpropan-2-olate (2.94 g, 26.2 mmol) in toluene (30 mL) was added (2-bromoethyl)diphenylsulfonium trifluoromethanesulfonate (4.64 g, 10.47 mmol) at room temperature. The resulting mixture was stirred at 110 °C for 16 h. To the reaction was added water (200 mL) and extracted with ethyl acetate (400 mL). The organic layer was washed with water (500 mL), brine (500 mL), dried over anhydrous sodium sulfate, filtered and the filtrate was concentrated under reduced pressure. The residue was purified by silica gel column chromatography, eluted with 0~25% ethyl acetate in petroleum ether to afford a yellow oil which was further purified by RP-flash chromatography, eluted with 40 - 80% acetonitrile in water to afford 7-phenyl-7-azadispiro[2.1.45.33]dodecan-12-one (236 mg, 0.924 mmol, 10.60% yield) as a colorless oil. MS ESI calculated for C₁₇H₂₁NO [M + H]⁺ 256.16, found 256.15. ¹H NMR (300 MHz, Chloroform-*d*) δ 7.26-7.21 (m, 2H), 6.71 (t, *J =* 7.2 Hz, 1H), 6.59 (d, *J =* 7.8 Hz, 2H), 3.49-3.40 (m, 2H), 3.37 (d, *J* = 9.2 Hz, 1H), 3.27 (d, *J =* 9.2 Hz, 1H), 2.63-2.45 (m, 2H), 2.15-1.94 (m, 4H), 1.84 (s, 2H), 1.43-1.15 (m, 2H), 0.71-0.59 (m, 2H).

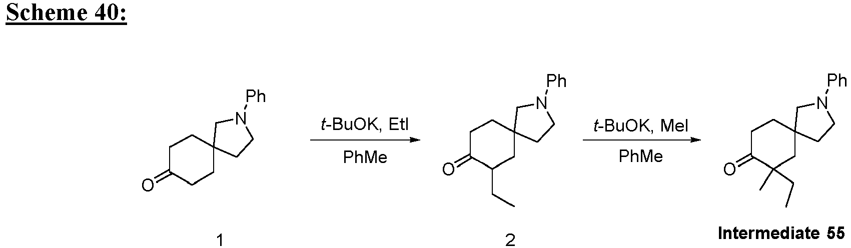

### Intermediate 55: 7-ethyl-7-methyl-2-phenyl-2-azaspiro[4.5]decan-8-one

**Step 1:** To a stirred mixture of 2-phenyl-2-azaspiro[4.5]decan-8-one (2 g, 8.72 mmol) in toluene (20 mL) were added iodoethane (1.224 g, 7.85 mmol) and potassium tert-butoxide (1.174 g, 10.47 mmol) at room temperature under argon atmosphere. The resulting mixture was stirred at 60 °C for 3 h. The reaction was quenched by addition of saturate aqueous NH₄Cl (20 mL), extracted with EtOAc (20 mL × 3). The organic layer was washed with brine (50 mL). The organic layer was dried over anhydrous Na₂SO₄, filtered and the filtrate was concentrated under reduced pressure. The residue was purified by silica gel column chromatography, eluted with 0 - 10% of ethyl acetate in petroleum ether to afford 7-ethyl-2-phenyl-2-azaspiro[4.5]decan-8-one (760 mg, 2.95 mmol, 33.9% yield) as a yellow oil. MS ESI calculated for C₁₇H₂₃NO [M + H]⁺ 258.28 found 258.10. ¹H NMR (300 MHz, Chloroform-*d*) δ 7.39-7.21 (m, 2H), 6.75 (t, *J* = 7.3 Hz, 1H), 6.67 (d, *J* = 8.1 Hz, 2H), 3.55-3.38 (m, 4H), 2.53-2.18 (m, 3H), 2.17-1.99 (m, 1H), 1.99-1.71 (m, 4H), 1.62-1.47 (m, 1H), 1.30-1.14 (m, 1H), 1.04-0.70 (m, 4H).

**Step 2:** To a solution of 7-ethyl-2-phenyl-2-azaspiro[4.5]decan-8-one (760 mg, 2.95 mmol) in toluene (8 mL) were added iodomethane (461 mg, 3.25 mmol) and potassium *tert*-butoxide (398 mg, 3.54 mmol). The resulted mixture was stirred at 60 °C for 2 h, and was then quenched with NH₄Cl (10 mL) and extracted with EtOAc (30 mL × 3). The combined organic layers were dried over Na₂SO₄, and concentrated in vacuo. The crude material was purified by silica gel column chromatography, eluted with 0 - 50% of EtOAc in petroleum ether to afford 7-ethyl-7-methyl-2-phenyl-2-azaspiro[4.5]decan-8-one (320 mg, 1.179 mmol, 39.9% yield) as a colorless oil. MS ESI calculated for C₁₈H₂₅NO [M + H]⁺ 272.19 found 272.15. ¹H NMR (300 MHz, Chloroform-*d*) δ 7.33-7.21 (m, 2H), 6.76 (t, *J =* 7.3 Hz, 1H), 6.70-6.64 (m, 2H), 3.68-3.13 (m, 4H), 2.57-2.32 (m, 1H), 2.15-1.88 (m, 3H), 1.87-1.37 (m, 6H), 1.13-1.07 (m, 3H), 0.91-0.79 (m, 3H).

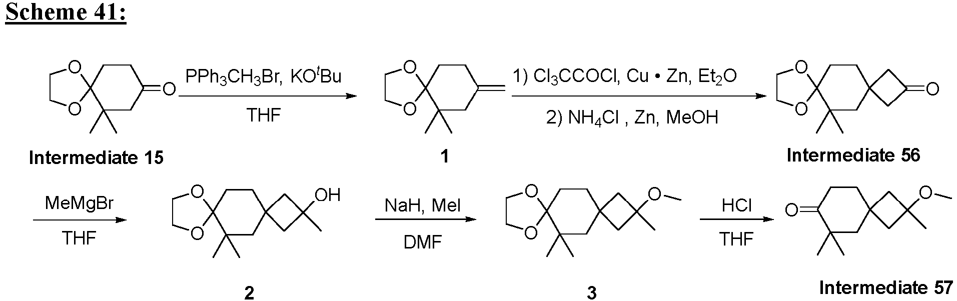

### Intermediates 56 and 57: 6,6-dimethyl-8,11-dioxadispiro[3.2.4⁷.2⁴]tridecan-2-one, 56 and 2-methoxy-2,6,6-trimethylspiro[3.5]nonan-7-one, 57

**Step 1:** To a mixture of methyltriphenylphosphonium bromide (58.2 g, 163 mmol) in THF (100 mL) was added potassium tert-butoxide (18.27 g, 163 mmol) at 0 °C under nitrogen. The mixture was stirred for 30 min, to which was added 6,6-dimethyl-1,4-dioxaspiro[4.5]decan-8-one (10 g, 54.3 mmol) at 0 °C. The resulted mixture was warmed to 25 °C and stirred for 3 h, and was then quenched by water (200 mL) and extracted with ethyl acetate (2 × 300 mL). The combined organic fractions were washed with brine (250 mL), dried over anhydrous sodium sulfate and filtered. The filtrate was concentrated under reduced pressure, and the residue was purified by silica gel column chromatography, eluting with 1 ~ 10% ethyl acetate in petroleum ether, to afford 6,6-dimethyl-8-methylene-1,4-dioxaspiro[4.5]decane (8.6 g, 47.2 mmol, 87 % yield) as a colourless oil. ¹H-NMR (300 MHz, Chloroform-*d*) δ 4.73 (s, 1H), 4.64 (s, 1H), 4.05-3.91 (m, 4H), 2.26 (t, *J =* 6.6 Hz, 2H), 2.13 (s, 2H), 1.68 (t, *J =* 6.6 Hz, 2H), 0.95 (s, 6H).

**Step 2**: To a mixture of 6,6-dimethyl-8-methylene-1,4-dioxaspiro[4.5]decane (7.6 g, 41.7 mmol) and zinc-copper couple (16.13 g, 125 mmol) in Et₂O (65 mL) under nitrogen was slowly added a solution of trichloroacetyl chloride (15.16 g, 83 mmol) in Et₂O (15 mL) at 25 °C. The mixture was stirred for 1.5 h before it was quenched by sodium bicarbonate solution (200 mL) at 0 °C and filtered. The filtrate was extracted with ethyl acetate (2 x 200 mL). The combined organic fractions were washed with brine (200 mL), dried over anhydrous sodium sulfate and filtered. The filtrate was concentrated under reduced pressure to afford brown oil. To a solution of this oil in MeOH (80 ml) were added ammonium chloride (4.46 g, 83 mmol) and zinc (8.18 g, 125 mmol) at 0 °C. The mixture was warmed to 25 °C and stirred for 16 h. The reaction progress was monitored by TLC. The reaction mixture was filtered. The filtrate was concentrated under reduced pressure, and the residue was purified by silica gel column chromatography, eluting with 1 ~ 14% ethyl acetate in petroleum ether, to afford 6,6-dimethyl-8,11-dioxadispiro[3.2.4⁷.2⁴]tridecan-2-one (4.1 g, 18.28 mmol, 43.8 % yield) as an off-white solid. ¹H-NMR (400 MHz, Chloroform-*d*) δ 4.02-7.52 (m, 4H), 2.97-2.72 (m, 4H), 1.83-1.60 (m, 6H), 1.03-1.00 (m, 6H).

**Step 3:** To a solution of 6,6-dimethyl-8,11-dioxadispiro[3.2.4⁷.2⁴]tridecan-2-one (250 mg, 1.115 mmol) in THF (2 mL) was added methylmagnesium bromide (1M in THF, 2.229 mL, 2.229 mmol) at 0 °C and the mixture was stirred for 10 min. Then the mixture was stirred for 3 h at room temperature and was monitored by LCMS and TLC. The reaction mixture was quenched by saturated aqueous NH₄Cl (10 mL) and extracted with ethyl acetate (20 mL × 3). The combined organic layers were washed with brine (40 mL), dried over anhydrous sodium sulfate and filtered. The filtrate was concentrated under reduced pressure to give 2,6,6-trimethyl-8,11-dioxadispiro[3.2.4⁷.2⁴]tridecan-2-ol (250 mg, 1.040 mmol, 93% yield) as a white solid. MS ESI calculated for C₁₄H₂₄O₃ [M + H - H₂O]⁺ 223.17 found 223.25. ¹H NMR (300 MHz, DMSO-*d*₆) δ 4.67 (s, 0.3H), 4.57 (s, 0.7H), 3.94-3.73 (m, 4H), 1.87-1.69 (m, 5H), 1.60-1.40 (m, 5H), 1.22 (s, 0.5H), 1.20 (s, 2.5H), 0.85 (s, 6H).

**Step 4:** To a solution of sodium hydride (60% in miner oil, 80 mg, 1.997 mmol) in DMF (1.0 mL) were added a solution of 2,6,6-trimethyl-8,11-dioxadispiro[3.2.4⁷.2⁴]tridecan-2-ol (192 mg, 0.799 mmol) in DMF (0.5 mL) and methyl iodide (283 mg, 1.997 mmol) in DMF (0.5 mL) at 0 °C and the mixture was stirred for 10 min. The reaction was stirred at 25 °C for 5 h and was monitored by LCMS and TLC. The reaction was quenched by saturated aqueous NH₄Cl (5 mL) and extracted with ethyl acetate (10 mL × 3). The combined organic layers were washed with brine (20 mL), dried over anhydrous sodium sulfate and filtered. The filtrate was concentrated under reduced pressure. The residue was purified by silica gel column chromatography eluting with 1 ~ 60% ethyl acetate in petroleum ether to afford 2-methoxy-2,6,6-trimethyl-8,11-dioxadispiro[3.2.4⁷.2⁴]tridecane (140 mg, 0.550 mmol, 68.9% yield) as a yellow oil .¹H NMR (400 MHz, Chloroform-*d*) δ 3.97-3.86 (m, 4H), 3.13 (s, 3H), 1.97 (m, 2H), 1.83-1.65 (m, 5H), 1.58 (m, 3H), 1.27 (s, 3H), 0.92 (s, 6H).

**Step 5:** To hydrochloric acid (2 M in water, 2.00 mL, 4.00 mmol) was added a solution of 2-methoxy-2,6,6-trimethyl-8,11-dioxadispiro[3.2.4⁷.2⁴]tridecane (140 mg, 0.550 mmol) in THF (2.00 mL) at 0 °C and the mixture was stirred for 10 min. The reaction was stirred at 25 °C for 3 h and was monitored by LCMS and TLC. The reaction mixture was quenched by saturated aqueous NaHCO₃ (10 mL) and extracted with ethyl acetate (10 mL × 3). The combined organic layers were washed with brine (20 mL), dried over anhydrous sodium sulfate and filtered. The filtrate was concentrated under reduced pressure. The residue was purified by silica gel column chromatography eluting with 1 ~ 60% ethyl acetate in petroleum ether to afford 2-methoxy-2,6,6-trimethylspiro[3.5]nonan-7-one (85 mg, 0.404 mmol, 73.4% yield) as a yellow oil. MS ESI calculated for C₁₃H₂₂O₂ [M + H]⁺ 211.16 found 211.10. ¹H NMR (300 MHz, Chloroform-*d*) δ 3.17 (s, 1H), 3.16 (s, 2H), 2.47-2.37 (m, 2H), 2.20-2.06 (m, 2H), 2.04- 1.70 (m, 6H), 1.36 (s, 1H), 1.33 (s, 2H), 1.09 (s, 6H).

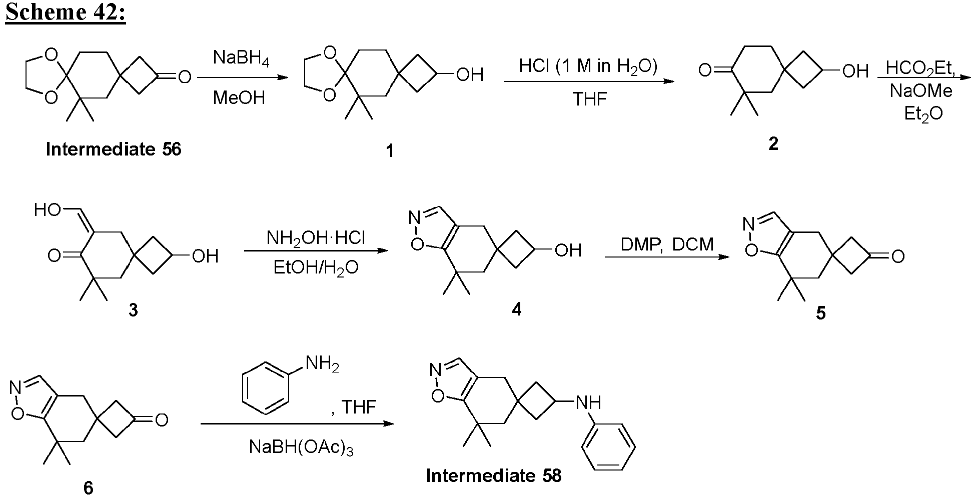

### Intermediate 58: 7,7-dimethyl-N-phenyl-6,7-dihydro-4H-spiro[benzo[d]isoxazole-5,1'-cyclobutan]-3'-amine

**Step 1:** To a mixture of 6,6-dimethyl-8,11-dioxadispiro[3.2.4⁷.2⁴]tridecan-2-one (500 mg, 2.229 mmol) in MeOH (15 mL) under nitrogen was added NaBH₄ (169 mg, 4.46 mmol) at 0 °C. The mixture was stirred for 10 min. The resulted mixture was warmed to 25 °C and stirred for 1.5 h. The reaction progress was monitored by LCMS. The reaction mixture was quenched by saturated ammonium chloride solution (30 mL) and extracted with ethyl acetate (2 x 40 mL). The combined organic fractions were washed with brine (40 mL), dried over anhydrous sodium sulfate and filtered. The filtrate was concentrated under reduced pressure, and the residue was purified by silica gel column chromatography, eluting with 1~23% ethyl acetate in petroleum ether, to afford 6,6-dimethyl-8,11-dioxadispiro[3.2.4⁷.2⁴]tridecan-2-ol (408 mg, 1.803 mmol, 81 % yield) as a colourless oil. MS ESI calculated for C₁₃H₂₂O₃ [M + H]⁺ 227.16 found 227.25. ¹H-NMR (400 MHz, Chloroform-d) δ 4.46-4.18 (m, 1H), 4.02-3.84 (m, 4H), 2.41-2.20 (m, 2H), 1.81-1.68 (m, 3H), 1.68-1.58 (m, 5H), 0.96 (s, 3H), 0.93 (s, 3H).

**Step 2:** To a mixture of 6,6-dimethyl-8,11-dioxadispiro[3.2.4⁷.2⁴]tridecan-2-ol (446 mg, 1.971 mmol) in THF (5 mL) was added HCl (1M in H₂O, 5 mL, 5.00 mmol) at rt. The resulted mixture was warmed to 35 °C and stirred for 1 h. The reaction progress was monitored by LCMS. The reaction mixture was quenched by saturated sodium bicarbonate solution (25 mL) and extracted with ethyl acetate (2 x 30 mL). The combined organic fractions were washed with brine (25 mL), dried over anhydrous sodium sulfate and filtered. The filtrate was concentrated under reduced pressure, and the residue was purified by silica gel column chromatography, eluting with 1~43% ethyl acetate in petroleum ether, to afford 2-hydroxy-6,6-dimethylspiro[3.5]nonan-7-one (312 mg, 1.712 mmol, 87 % yield) as a light yellow oil. MS ESI calculated for C₁₁H₁₈O₂ [M + H]⁺ 183.14 found 183.25. ¹H-NMR (300 MHz, Chloroform-d) δ 4.50-4.30 (m, 1H), 2.52-2.36 (m, 4H), 2.04-1.97 (m, 1H), 1.95-1.83 (m, 3H), 1.78 (s, 1H), 1.76 (s, 2H), 1.12 (s, 3H), 1.09 (s, 3H). **Step 3:** To a mixture of 2-hydroxy-6,6-dimethylspiro[3.5]nonan-7-one (312 mg, 1.712 mmol) in toluene (6 mL) under nitrogen were added ethyl formate (1268 mg, 17.12 mmol) and sodium methylate (30% in MeOH, 3083 mg, 17.12 mmol) at room temperature. The resulted mixture was stirred for 3 h at 35 °C. The reaction progress was monitored by LCMS. The reaction mixture was quenched by saturated aqueous solution of NH₄Cl (20 mL) and extracted with ethyl acetate (2 × 30 mL). The combined organic fractions were washed with brine (20 mL), dried over anhydrous sodium sulfate and filtered. The filtrate was concentrated under reduced pressure to afford (*Z*)-2-hydroxy-8-(hydroxymethylene)-6,6-dimethylspiro[3.5]nonan-7-one (355 mg, 1.688 mmol, 99 % yield) (crude) as a light yellow oil. MS ESI calculated for C₁₂H₁₈O₃ [M + H]⁺ 211.13 found 211.15.

**Step 4:** To a mixture of (*Z*)-2-hydroxy-8-(hydroxymethylene)-6,6-dimethylspiro[3.5]nonan-7-one (355 mg, 1.688 mmol) in EtOH (12 mL) was added a solution of hydroxylamine hydrochloride (1173 mg, 16.88 mmol) in water (1.2 mL) at room temperature. The resulted mixture was stirred at 80 °C for 2 h. The reaction mixture was quenched by saturated aqueous solution of NaHCO₃ (40 mL) and extracted with ethyl acetate (2 x 50 mL). The combined organic fractions were washed with brine (45 mL), dried over anhydrous sodium sulfate and filtered. The filtrate was concentrated under reduced pressure, and the residue was purified by silica gel column chromatography, eluting with 0 ~ 39% ethyl acetate in petroleum ether, to afford 7,7-dimethyl-6,7-dihydro-4*H*-spiro[benzo[d]isoxazole-5,1'-cyclobutan]-3'-ol (189 mg, 0.912 mmol, 54.0 % yield) as a light yellow oil. MS ESI calculated for C₁₂H₁₇NO₂ [M + H]⁺ 208.13 found 208.25. ¹H-NMR (300 MHz, Chloroform-d) δ 8.03 (d, *J* = 1.7 Hz, 1H), 4.67-4.25 (m, 1H), 2.65 (s, 1H), 2.52 (s, 1H), 2.51-2.40 (m, 1H), 2.34-2.23 (m, 1H), 2.01-1.88 (m, 1H), 1.89-1.76 (m, 3H), 1.37 (s, 3H), 1.34 (s, 3H).

**Step 5:** To a mixture of 7,7-dimethyl-6,7-dihydro-4*H*-spiro[benzo[*d*]isoxazole-5,1'-cyclobutan]-3'-ol (350 mg, 1.689 mmol) in DCM (4.5 mL) under nitrogen was added Dess-Martin periodinane (1432 mg, 3.38 mmol) at 0 °C. The resulted mixture was warmed to room temperature and stirred for 1.5 h. The reaction progress was monitored by LCMS. The reaction mixture was quenched by saturated sodium bicarbonate (40 mL) and extracted with ethyl acetate (130 mL × 3). The combined organic fractions were dried over anhydrous sodium sulfate and filtered. The filtrate was concentrated under reduced pressure. The residue was purified by silica gel column chromatography eluting with 1 ~ 40% ethyl acetate in petroleum ether to afford 7,7-dimethyl-6,7-dihydro-4H-spiro[benzo[*d*]isoxazole-5,1'-cyclobutan]-3'-one (312 mg, 1.520 mmol, 90% yield) as a white solid. MS ESI calculated for C₁₂H₁₅NO₂ [M + H]⁺ 206.11 found 206.10. ¹H NMR (400 MHz, Chloroform-*d*) δ 8.06 (s, 1H), 3.17-3.03 (m, 2H), 2.84-2.74 (m, 2H), 2.66 (s, 2H), 2.06 (s, 2H), 1.39 (s, 6H).

**Step 6:** To a solution of 7,7-dimethyl-6,7-dihydro-4*H*-spiro[benzo[d]isoxazole-5,1'-cyclobutan]-3'-one (100 mg, 0.487 mmol) in THF (1 ml) was added aniline (52.2 mg, 0.56 mmol). The resulted mixture was stirred for 2 h at room temperature, to which was added sodium triacetoxyborohydride (147.8 mg, 0.69 mmol). The resulted mixture was stirred for 16 h at room temperature and then concentrated under reduced pressure. The residue was purified by silica gel column chromatography eluting with 0 - 50% of ethyl acetate in petroleum ether to afford 7,7-dimethyl-*N*-phenyl-6,7-dihydro-4*H*-spiro[benzo[d]isoxazole-5,1'-cyclobutan]-3'-amine (90 mg, 0.319 mmol, 65.4% yield) as a colorless oil. MS ESI calculated for C₁₈H₂₂N₂O [M + H]⁺ 283.17 found 283.20. ¹H NMR (400 MHz, Chloroform-*d*) δ 8.06-7.98 (m, 1H), 7.27-7.20 (m, 2H), 6.98-6.80 (m, 3H), 4.10-3.82 (m, 1H), 2.68-2.59 (m, 2H), 2.52-2.25 (m, 2H), 2.12-2.09 (m, 2H), 1.84 (d, *J* = 5.9 Hz, 2H), 1.35 (s, 4H), 1.28 (s, 2H).

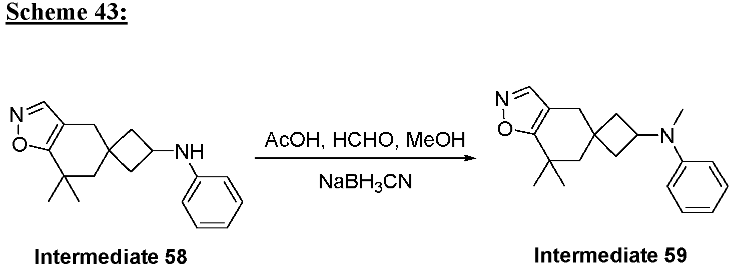

### Intermediate 59: N,7,7-trimethyl-N-phenyl-6,7-dihydro-4H-spiro[benzo[d]isoxazole-5,1'-cyclobutan]-3'-amine

**Step 1:** To a solution of 7,7-dimethyl-*N*-phenyl-6,7-dihydro-4*H*-spiro[benzo[*d*]isoxazole-5,1'-cyclobutan]-3'-amine (75 mg, 0.266 mmol) in MeOH (0.5 mL) was added acetic acid (159 mg, 2.66 mmol), formaldehyde (80 mg, 2.66 mmol) and the solution of Sodium cyanoborohydride (134 mg, 2.125 mmol) in MeOH (0.5 mL). The resulted mixture was stirred at room temperature for 1 h, and then was quenched by saturated aqueous sodium bicarbonate (10 mL). The reaction mixture was extracted with ethyl acetate (30 mL × 3), dried over anhydrous sodium sulfate and filtered. The filtrate was concentrated under reduced pressure. The residue was purified by silica gel column chromatography eluting with 0 - 50% of ethyl acetate in petroleum ether to afford *N*,7,7-trimethyl-*N*-phenyl-6,7-dihydro-4*H*-spiro[benzo[*d*]isoxazole-5,1'-cyclobutan]-3'-amine (70 mg, 0.224 mmol, 84% yield) as a colorless oil. MS ESI calculated for C₁₉H₂₄N₂O [M + H]⁺ 297.19 found 297.25.

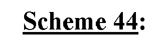

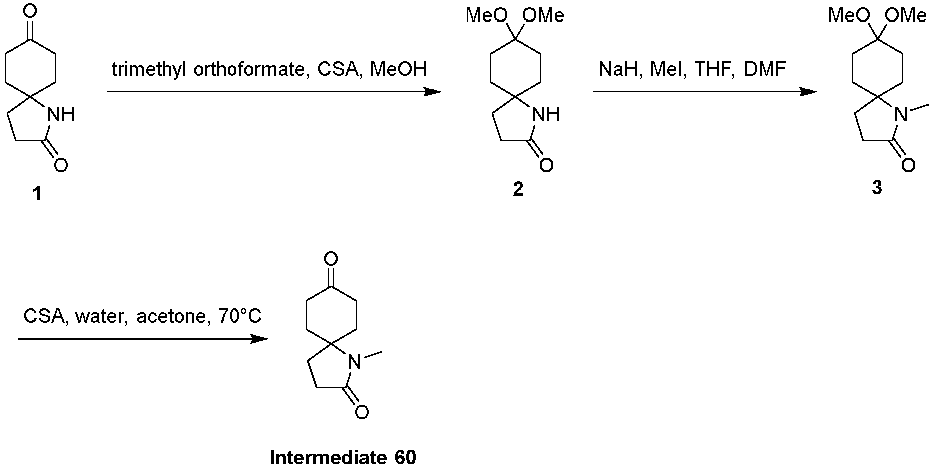

### Intermediate 60: 9,9-dimethyl-1,8-dioxo-2-phenyl-2-azaspiro[4.5]decane-7-carbonitrile

**Step 1:** To a solution of 1-azaspiro[4.5]decane-2,8-dione (2 g, 12 mmol) in MeOH (24 mL) was added trimethyl orthoformate (1.59 mL, 14.4 mmol) and CSA (0.139 g, 0.598 mmol). The mixture was stirred at room temperature for 1 h. The reaction was quenched with triethylamine and concentrated under reduced pressure to afford 8,8-dimethoxy-1-azaspiro[4.5]decan-2-one as a solid. The material was used as is crude in the next step.

**Step 2:** To a solution of 8,8-dimethoxy-1-azaspiro[4.5]decan-2-one (2.44 g, 11.4 mmol) in THF (17 mL) and DMF (5.7 mL) was added NaH (1.33 g, 33.2 mmol) portionwise. The mixture was stirred at room temperature for 30 minutes. Methyl iodide (1.57 ml, 25.2 mmol) was added and the reaction was stirred at room temperature overnight. The mixture was diluted with water and DCM, and the aqueous layer was extracted with DCM (3x). The combined organic layers were washed with brine, dried over anhydrous sodium sulfate, filtered, and concentrated under reduced pressure. The residue was purified by column chromatography on silica (0-10% MeOH/DCM) to afford 8,8-dimethoxy-1-methyl-1-azaspiro[4.5]decan-2-one as an oil. MS: 228 (M + 1).

**Step 3:** To 8,8-dimethoxy-1-methyl-1-azaspiro[4.5]decan-2-one (1.34 g, 5.89 mmol) in acetone (20 mL) was added water (5.31 ml, 295 mmol) and CSA (0.137 g, 0.589 mmol). The mixture was heated at 70 °C for 30 minutes. The reaction was quenched with saturated aqueous sodium bicarbonate, extracted with DCM (3x), and the combined organic layers were dried over anhydrous sodium sulfate, filtered, and concentrated under reduced pressure. The residue was purified by column chromatography on silica (0-10% MeOH/DCM) to afford 9,9-dimethyl-1,8-dioxo-2-phenyl-2-azaspiro[4.5]decane-7-carbonitrile as a solid. MS: 182 (M + 1).

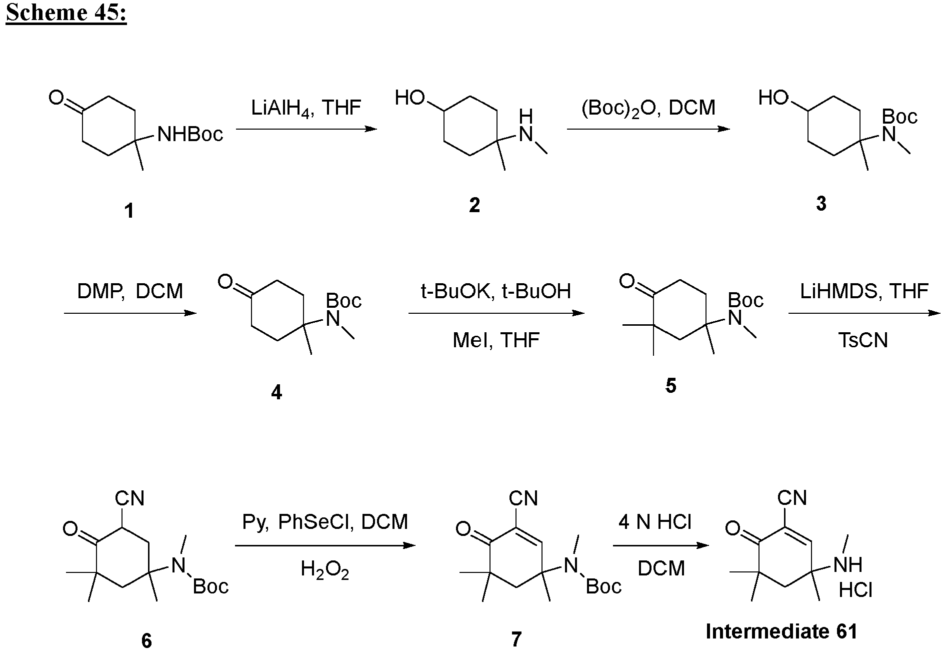

### Intermediate 61: 3,5,5-trimethyl-3-(methylamino)-6-oxocyclohex-1-ene-1-carbonitrile hydrochloride

**Step 1:** To a solution of *tert*-butyl (1-methyl-4-oxocyclohexyl)carbamate (2 g, 8.80 mmol) in THF (70 mL) was added dropwise lithium aluminium hydride (1 M solution in THF, 26.4 mL, 26.4 mmol) at 0 °C. The resulting mixture was heated to 75 °C and stirred for 1 h. Then the mixture was cooled to 0 °C, quenched by addition of water (1 mL), 10% aq. NaOH (1 mL) and water (3 mL). Then the mixture was filtered. The solid was washed with THF (100 mL × 3). The filtrate was concentrated under reduced pressure to give a crude product which was used directly for next step. MS ESI calculated for C₈H₁₇NO [M + H]⁺ 144.13, found 144.25.

**Step 2:** To a solution of 4-methyl-4-(methylamino)cyclohexan-1-ol (1.260 g, 8.80 mmol) in DCM (90 mL) was added di-*tert*-butyl dicarbonate (3.84 g, 17.60 mmol) at 0 °C. The resulting mixture was stirred at room temperature for 16 h. Then the mixture was concentrated under reduced pressure and the residue was purified by column chromatography on silica gel, eluted with 0% - 55% ethyl acetate in petroleum ether to give tert-butyl (4-hydroxy-1-methylcyclohexyl)(methyl)carbamate (1.95 g, 8.01 mmol, 91% yield) as a light yellow oil. MS ESI calculated for C₁₃H₂₅NO₃ [M + H]⁺ 244.18, found 244.25. ¹H NMR (400 MHz, Chloroform-*d*): δ 3.73-3.64 (m, 1H), 2.85 (s, 3H), 1.93-1.69 (m, 4H), 1.47 (s, 9H), 1.44-1.35 (m, 2H), 1.32-1.22 (s, 2H), 1.19 (s, 3H).

**Step 3:** To a solution of *tert*-butyl (4-hydroxy-1-methylcyclohexyl)(methyl)carbamate (1.95 g, 8.01 mmol) in dry DCM (200 mL) was added DMP (6.80 g, 16.03 mmol) at 0 °C under N₂ atmosphere. The resulting mixture was stirred at room temperature for 16 h. Then the mixture was quenched with saturated aq. Na₂S₂O₃ (150 mL) and saturated aq. NaHCO₃ (150 mL), extracted with DCM (300 mL × 3). The combined organic phase was washed with brine (400 mL), dried over anhydrous sodium sulfate. After filtration, the filtrate was concentrated under reduced pressure and the residue was purified by column chromatography on silica gel, eluted with 0% - 40% ethyl acetate in petroleum ether to give *tert*-butyl methyl(1-methyl-4-oxocyclohexyl)carbamate (1.81 g, 7.50 mmol, 94% yield) as a light yellow oil. MS ESI calculated for C₁₃H₂₃NO₃ [M + H]⁺ 242.17, found 242.25. ¹H NMR (400 MHz, Chloroform-d): δ 2.92 (s, 3H), 2.81-2.70 (m, 2H), 2.45-2.23 (m, 4H), 1.78-1.67 (m, 2H), 1.46 (s, 9H), 1.31 (s, 3H). **Step 4:** To a solution of *tert*-butyl methyl(1-methyl-4-oxocyclohexyl)carbamate (2.81 g, 11.64 mmol) in *t*-BuOH (110 mL) was added potassium 2-methylpropan-2-olate (1 M solution in THF, 29.1 mL, 29.1 mmol) at room temperature. The mixture was stirred at 25 °C for 1 h. Then a solution of iodomethane (3.47 g, 24.45 mmol) in THF (5 mL) was added at 0 °C. The resulting mixture was stirred at 25 °C for 3 h. Then the mixture was quenched with saturated aq. NH₄Cl (100 mL), extracted with ethyl acetate (100 mL × 3). The combined organic phase was washed with brine (100 mL), dried over anhydrous sodium sulfate. After filtration, the filtrate was concentrated under reduced pressure and the residue was purified by column chromatography on silica gel, eluted with 0% - 20% ethyl acetate in petroleum ether to give *tert*-butyl methyl(1,3,3-trimethyl-4-oxocyclohexyl)carbamate (1.53 g, 5.68 mmol, 48.8% yield) as a yellow oil. MS ESI calculated for C₁₅H₂₇NO₃ [M + H]⁺ 270.20, found 270.20. ¹H NMR (300 MHz, Chloroform-d): δ 3.16 (dd, *J* = 14.7, 4.2 Hz, 1H), 2.91 (s, 3H), 2.67-2.39 (m, 2H), 2.33-2.21 (m, 1H), 1.87-1.61 (m, 2H), 1.46 (s, 9 H), 1.28 (s, 3H), 1.18 (s, 3H), 1.04 (s, 3H).

**Step 5:** To a solution of *tert*-butyl methyl(1,3,3-trimethyl-4-oxocyclohexyl)carbamate (1.2 g, 4.45 mmol) in THF (25 mL) was added dropwise LiHMDS (1 M solution in THF, 9.80 mL, 9.80 mmol) at -70 °C under N₂ atmosphere. The resulting mixture was stirred at -70 °C for 1 h. Then a solution of 4-methylbenzenesulfonyl cyanide (1.211 g, 6.68 mmol) in THF (5 mL) was added. After stirring at -70 °C for 30 min, the mixture was warmed to room temperature and stirred for 2 h. Major desired product was detected on LCMS and part of SM was left. Then the mixture was quenched with saturated aqueous NH₄Cl (100 mL), extracted with ethyl acetate (100 mL × 3). The combined organic phase was washed with brine (150 mL), dried over anhydrous sodium sulfate. After filtration, the filtrate was concentrated under reduced pressure. The residue was purified by column chromatography on silica gel, eluted with 0% - 30% ethyl acetate in petroleum ether to give tert-butyl (5-cyano-1,3,3-trimethyl-4-oxocyclohexyl)(methyl)carbamate (680 mg, 2.31 mmol, 51.9% yield) as a white solid. MS ESI calculated for C₁₆H₂₆N₂O₃ [M + H]⁺ 295.19, found 295.30. ¹H NMR (400 MHz, Chloroform-*d*): δ 4.00 (dd, *J =* 14.0, 4.0 Hz, 1H), 3.19-3.08 (m, 2H), 3.00 (dd, *J =* 14.0, 3.2 Hz, 1H), 2.92 (s, 3H), 2.85 (s, 3H), 2.57 (dd, *J =* 16.4, 3.2 Hz, 1H), 2.30 (d, *J =* 16.4 Hz, 1H), 2.04-1.94 (m, 1 H), 1.54-1.40 (m, 21H), 1.35 (s, 3H), 1.28 (s, 3H), 1.24 (s, 3H), 1.17 (s, 3H), 1.16 (s, 3H), 1.11 (s, 3H).

**Step 6:** A solution of pyridine (107 mg, 1.359 mmol) in DCM (0.3 mL) was added dropwise to phenyl hypochloroselenoite (260 mg, 1.359 mmol) in DCM (5 mL) at 0 °C. The mixture was stirred at 0 °C for 20 min and then treated dropwise with a solution of *tert*-butyl (5-cyano-1,3,3-trimethyl-4-oxocyclohexyl)(methyl)carbamate (200 mg, 0.679 mmol) in DCM (2 mL). The resulting mixture was stirred at 0 °C for 2 h. Then hydrogen peroxide (30%, 770 mg, 6.79 mmol) was added dropwise at 0 °C and the mixture was vigorously stirred at 0 °C for 40 min. The reaction mixture was quenched by saturated aq. Na₂S₂O₃ (20 mL), extracted with DCM (20 mL × 3), washed with brine (30 mL), dried over anhydrous sodium sulfate. After filtration, the filtrate was concentrated under reduced pressure and the residue was purified by column chromatography on silica gel, eluted with 0% - 35% ethyl acetate in petroleum ether to give *tert-*butyl (3-cyano-1,5,5-trimethyl-4-oxocyclohex-2-en-1-yl)(methyl)carbamate (110 mg, 0.376 mmol, 55.4% yield) as a yellow oil. MS ESI calculated for C₁₆H₂₄N₂O₃ [M - 56 + H]⁺ 237.18, found 237.15. ¹H NMR (300 MHz, Chloroform-d): δ 7.70 (s, 1H), 2.86 (s, 3H), 2.33 (d, *J* = 14.1 Hz, 1H), 1.92 (d, *J* = 14.1 Hz, 1H), 1.54 (s, 3H), 1.43 (s, 9H), 1.23 (s, 3H), 1.21 (s, 3H).

**Step 7:** To a solution of *tert*-butyl (3-cyano-1,5,5-trimethyl-4-oxocyclohex-2-en-1-yl)(methyl)carbamate (110 mg, 0.376 mmol) in DCM (2.1 mL) was added hydrogen chloride (4 M solution in dioxane, 0.941 mL, 3.76 mmol) at 0 °C. The resulting mixture was stirred at room temperature for 2 h. Then the mixture was concentrated under reduced pressure to give crude 3,5,5-trimethyl-3-(methylamino)-6-oxocyclohex-1-ene-1-carbonitrile hydrochloride (86 mg, 0.376 mmol, 100% yield) as a light yellow solid which was used for next step directly. MS ESI calculated for C₁₁H₁₆N₂O [M + H]⁺ 193.14, found 193.20. ¹H NMR (400 MHz, DMSO-*d₆*): δ 9.81 (s, 2H), 7.89 (s, 1H), 2.56 (s, 3H), 2.35 (d, *J =* 14.4 Hz, 1H), 2.13 (dd, *J =* 14.4 Hz, 1H), 1.67 (s, 3H), 1.23 (s, 3H), 1.16 (s, 3H).

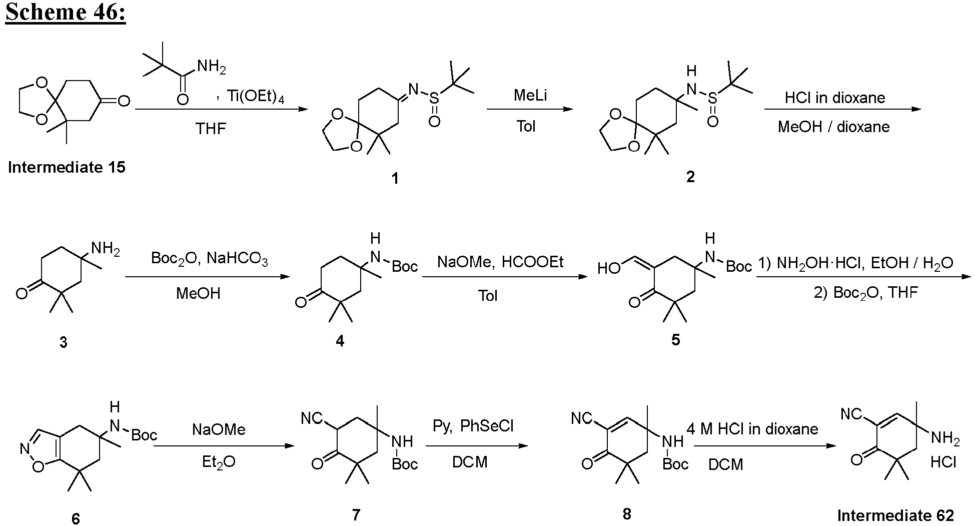

### Intermediate 62: 3-amino-3,5,5-trimethyl-6-oxocyclohex-1-ene-1-carbonitrile hydrochloride

**Step 1:** To a mixture of titanium(*IV*) ethoxide (7.43 g, 32.6 mmol) in THF (80 mL) under nitrogen was added a solution of 6,6-dimethyl-1,4-dioxaspiro[4.5]decan-8-one (2 g, 10.86 mmol) in THF (20 mL) at room temperature. The mixture was stirred for 15 min. This was followed by the slow addition of 2-methylpropane-2-sulfinamide (1.316 g, 10.86 mmol) in THF (80 mL) at room temperature. The resulted mixture was warmed to 65 °C and stirred for 4 h. The reaction progress was monitored by LCMS. The reaction mixture was allowed to cool down to ambient temperature, quenched by brine (80 mL). The resulting slurry solution was filtered through a pad of celite and the filter cake was washed with ethyl acetate (200 mL). The filtrate washed with brine (80 mL). The aqueous layer was extracted with ethyl acetate (200 mL). The combined organic fractions were washed with brine (120 mL), dried over anhydrous sodium sulfate and filtered. The filtrate was concentrated under reduced pressure, the residue was purified by silica gel column chromatography, eluted with 0 ~ 42% ethyl acetate in petroleum ether to afford *N-*(6,6-dimethyl-1,4-dioxaspiro[4.5]decan-8-ylidene)-2-methylpropane-2-sulfinamide (1.96 g, 6.82 mmol, 62.8% yield) as an off-white solid. MS ESI calculated for C₁₄H₂₅NO₃S [M + H]⁺ 288.16 found 288.10. ¹H NMR (400 MHz, CDCl₃) δ 4.08-3.93 (m, 4H), 3.22-2.74 (m, 2H), 2.62-2.44 (m, 2H), 2.05-1.76 (m, 2H), 1.25 (d, *J* = 2.1 Hz, 9H), 1.03 (s, 1 H), 1.02 (s, 2 H), 1.01 (s, 2 H), 1.00 (s, 1 H), .

**Step 2:** To a mixture of methyllithium (1.6 M in Et₂O, 9.79 mL, 15.66 mmol) in toluene (21 mL) under nitrogen was added a solution of *N*-(6,6-dimethyl-1,4-dioxaspiro[4.5]decan-8-ylidene)-2-methylpropane-2-sulfinamide (900 mg, 3.13 mmol) in toluene (6 mL) at 0 °C. The mixture was stirred for 15 min. The resulted mixture was warmed to 25 °C and stirred for 16 h. The reaction progress was monitored by LCMS. The reaction mixture was quenched by saturated aqueous solution of Na₂SO₄ (until gas evolution stopped) and diluted with ethyl acetate (25 mL). Solid Na₂SO₄ was added and the resulting slurry stirred at 0 °C for a further 10 min. The slurry was then filtered and washed with ethyl acetate (50 mL). The filtrate was concentrated under reduced pressure, the residue was purified by silica gel column chromatography, eluted with 0 ~ 56% ethyl acetate in petroleum ether to afford 2-methyl-*N*-(6,6,8-trimethyl-1,4-dioxaspiro[4.5]decan-8-yl)propane-2-sulfinamide (326 mg, 1.074 mmol, 34.3% yield) as a light yellow oil. MS ESI calculated for C₁₅H₂₉NO₃S [M + H]⁺ 304.19 found 304.15. ¹H NMR (300 MHz, CDCl₃) δ 4.03-3.88 (s, 4H), 3.41 (s, 1H), 2.10-1.87 (m, 2H), 1.85-1.63 (m, 3H), 1.51-1.40 (m, 1H), 1.35 (s, 3H), 1.23 (s, 9H), 1.14 (s, 3H), 0.98 (s, 3H).

**Step 3:** To a mixture of 2-methyl-*N*-(6,6,8-trimethyl-1,4-dioxaspiro[4.5]decan-8-yl)propane-2-sulfinamide (3 g, 9.89 mmol) in MeOH (30 mL) and 1,4-dioxane (30 mL) were added water (12 mL) and HCl (4 M in dioxane, 60 mL, 240 mmol) at room temperature. The resulted mixture was warmed to 55 °C and stirred for 16 h. The reaction progress was monitored by LCMS. The reaction mixture was allowed to cool down to ambient temperature, the pH value of the solution was adjusted to 8 with saturated sodium bicarbonate solution. The mixture was concentrated under reduced pressure to afford 4-amino-2,2,4-trimethylcyclohexan-1-one (4.6 g, 9.78 mmol, 99% yield) (crude) as a light yellow solid. MS ESI calculated for C₉H₁₇NO [M + H]⁺ 156.13 found 156.05.

**Step 4:** To a mixture of 4-amino-2,2,4-trimethylcyclohexan-1-one (5.37 g, 11.42 mmol) in MeOH (50 mL) were added sodium bicarbonate (2.88 g, 34.2 mmol) and Boc₂O (3.98 mL, 17.12 mmol) at room temperature. The resulted mixture was stirred for 16 h at 25 °C. The reaction progress was monitored by LCMS. The reaction mixture was quenched by water (150 mL) and extracted with ethyl acetate (200 mL × 2). The combined organic fractions were washed with brine (120 mL), dried over anhydrous sodium sulfate and filtered. The filtrate was concentrated under reduced pressure. The residue was purified by silica gel column chromatography, eluted with 0 ~ 15% ethyl acetate in petroleum ether to afford *tert*-butyl (1,3,3-trimethyl-4-oxocyclohexyl)carbamate (1.54 g, 6.03 mmol, 52.8% yield) as an off-white solid. MS ESI calculated for C₁₄H₂₅NO₃ [M + H - C₄H₈]⁺ 200.13 found 200.15. ¹H NMR (400 MHz, CDCl₃) δ 4.56 (s, 1H), 2.82-2.66 (m, 1H), 2.57-2.45 (m, 1H), 2.36-2.17 (m, 2H), 1.89-1.73 (m, 1H), 1.58 (d, *J* = 14.7 Hz, 1H), 1.46 (s, 9H), 1.38 (s, 3H), 1.27 (s, 3H), 1.06 (s, 3H).

**Step 5:** To a mixture of *tert*-butyl (1,3,3-trimethyl-4-oxocyclohexyl)carbamate (2.2 g, 8.62 mmol) in toluene (66 mL) under nitrogen were added ethyl formate (6.38 g, 86 mmol) and sodium methylate (30% in MeOH, 15.51 g, 86 mmol) at room temperature. The resulted mixture was stirred for 1 h at 25 °C. The reaction progress was monitored by LCMS. The reaction mixture was quenched by saturated aqueous solution of NH₄Cl (100 mL) and extracted with DCM (150 mL × 2). The combined organic fractions were washed with brine (120 mL), dried over anhydrous sodium sulfate and filtered. The filtrate was concentrated under reduced pressure to afford *tert*-butyl (5-(hydroxymethylene)-1,3,3-trimethyl-4-oxocyclohexyl)carbamate (2.441 g, 8.62 mmol, 100% yield) (crude) as an off-white solid. MS ESI calculated for C₁₅H₂₅NO₄ [M + H - C₄H₈]⁺ 228.12 found 228.00.

**Step 6:** To a mixture of *tert*-butyl (5-(hydroxymethylene)-1,3,3-trimethyl-4-oxocyclohexyl)carbamate (2.441 g, 8.61 mmol) in EtOH (120 mL) was added a solution of hydroxylamine hydrochloride (5.99 g, 86 mmol) in water (12 mL) at room temperature. The resulted mixture was stirred for 1 h at 80 °C. The reaction mixture was quenched by saturated aqueous solution of NaHCO₃ (120 mL) and extracted with DCM (120 mL × 2). The combined organic fractions was washed with brine (100 mL), dried over anhydrous sodium sulfate and filtered. The filtrate is dissolved in THF (17 mL) followed by the addition of Boc₂O (5.00 mL, 21.54 mmol) at room temperature. The resulted mixture was stirred for 1 h at room temperature. The reaction progress was monitored by LCMS. The reaction mixture concentrated under reduced pressure. The residue was purified by silica gel column chromatography, eluted with 0 ~ 19% ethyl acetate in petroleum ether to afford *tert*-butyl (5,7,7-trimethyl-4,5,6,7-tetrahydrobenzo[d]isoxazol-5-yl)carbamate (1.84 g, 6.56 mmol, 76% yield) as an off-white solid. MS ESI calculated for C₁₅H₂₄N₂O₃ [M + H]⁺ 281.19 found 281.25. ¹H NMR (300 MHz, CDCl₃) δ 8.05 (s, 1H), 4.37 (s, 1H), 2.69 - 2.52 (m, 3H), 1.62 (d, *J =* 14.2 Hz, 1H), 1.51 (s, 3H), 1.42 (s, 9H), 1.38 (s, 3H), 1.35 (s, 3H).

**Step 7:** To a mixture of *tert*-butyl (5,7,7-trimethyl-4,5,6,7-tetrahydrobenzo[d]isoxazol-5-yl)carbamate (0.9 g, 3.21 mmol) in Et₂O (60 mL) under nitrogen was added sodium methylate (30% in MeOH, 17.34 g, 96 mmol) at 0 °C. The resulted mixture was stirred for 3 h at 25 °C. The reaction progress was monitored by LCMS. The reaction mixture was quenched by saturated aqueous solution of NH₄Cl (20 mL) and extracted with ethyl acetate (30 mL × 2). The combined organic fractions were washed with brine (25 mL), dried over anhydrous sodium sulfate and filtered. The filtrate was concentrated under reduced pressure. The residue was purified by silica gel column chromatography, eluted with 0 ~ 18% ethyl acetate in petroleum ether to afford *tert-*butyl (5-cyano-1,3,3-trimethyl-4-oxocyclohexyl)carbamate (833 mg, 2.97 mmol, 93 % yield) as an off-white solid. MS ESI calculated for C₁₅H₂₄N₂O₃ [M + H]⁺ 281.19 found 281.10. ¹H NMR (300 MHz, CDCl₃) δ 4.47 (s, 1H), 4.12 (dd, *J =* 13.8, 4.8 Hz, 1H), 3.11-2.90 (m, 1H), 2.58-2.26 (m, 1H), 2.16-1.96 (m, 1H), 1.66 (d, *J =* 15.0 Hz, 1H), 1.48 (s, 9H), 1.39 (s, 3H), 1.36 (s, 3H), 1.15 (s, 3H).

**Step 8:** To a mixture of pyridine (0.854 mL, 10.56 mmol) in DCM (15 mL) was added a solution of phenylselenenyl chloride (2.022 g, 10.56 mmol) in DCM (15 mL) at 0 °C and the mixture was stirred for 15 min. To the reaction mixture was added a solution of *tert*-butyl (5-cyano-1,3,3-trimethyl-4-oxocyclohexyl)carbamate (1.48 g, 5.28 mmol) in DCM (15 mL) at 0 °C. The resulted mixture was warmed to 25 °C and stirred for 16 h. The reaction progress was monitored by LCMS. The reaction mixture was concentrated under reduced pressure. The residue was purified by silica gel column chromatography, eluted with 0 ~ 24% ethyl acetate in petroleum ether to afford *tert*-butyl (3-cyano-1,5,5-trimethyl-4-oxocyclohex-2-en-1-yl)carbamate (1.42 g, 5.10 mmol, 97% yield) as a colorless oil. MS ESI calculated for C₁₅H₂₂N₂O₃ [M + H - C₄H₈]⁺ 223.11 found 223.05. ¹H NMR (300 MHz, CDCl₃) δ 7.67 (s, 1H), 4.72 (s, 1H), 2.44 (d, *J =* 14.4 Hz, 1H), 1.91 (d, *J* = 14.4, 1H), 1.56 (s, 3H), 1.45 (s, 9H), 1.28 (s, 3H), 1.27 (s, 3H).

Step 9: To a mixture of *tert*-butyl (3-cyano-1,5,5-trimethyl-4-oxocyclohex-2-en-1-yl)carbamate (1.6 g, 5.75 mmol) in DCM (16 mL) was added HCl (4 M in dioxane, 16 mL, 64.0 mmol) at 0 °C. The resulted mixture was stirred for 1 h at 25 °C. The reaction progress was monitored by LCMS. The reaction mixture was concentrated under reduced pressure. The residue was affording 3-amino-3,5,5-trimethyl-6-oxocyclohex-1-ene-1-carbonitrile hydrochloride (1.2 g, 5.59 mmol, 97% yield) (crude) as an off-white solid. MS ESI calculated for C₁₀H₁₅ClN₂O [M + H + H₂O]⁺ 197.13 found 197.05.

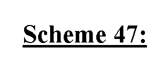

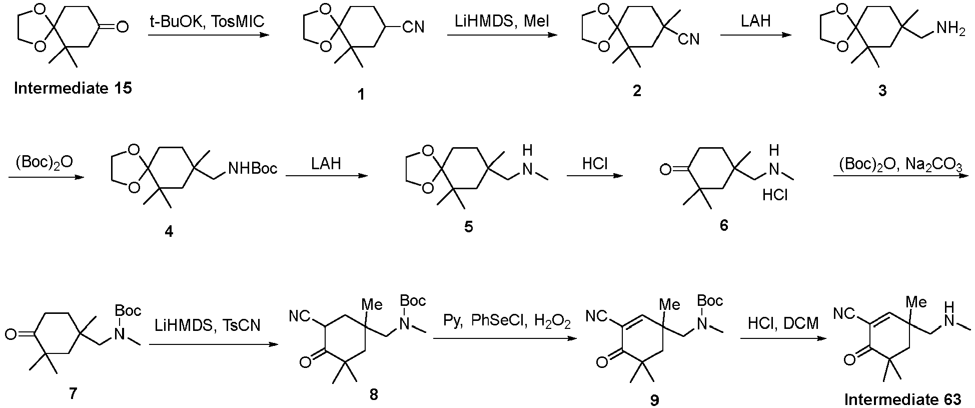

### Intermediate 63: N-((3-cyano-1,5,5-trimethyl-4-oxocyclohex-2-en-1-yl)methyl)-N-methylbenzamide

**Step 1:** To a solution of 6,6-dimethyl-1,4-dioxaspiro[4.5]decan-8-one (3 g, 16.28 mmol) in tetrahydrofuran (150 mL) was added potassium 2-methylpropan-2-olate (2.193 g, 19.54 mmol) at 0 °C and the mixture was stirred for 20 min at 30 °C. Then 1-((isocyanomethyl)sulfonyl)-4-methylbenzene (3.81 g, 19.54 mmol) was added into the reaction at the same temperature. The resulting mixture was stirred for another 4 h. The reaction mixture was quenched by saturated NH₄Cl (150 mL) and extracted with ethyl acetate (300 mL × 3). The combined organic layer was washed with brine (100 mL). The organic layer was dried over anhydrous sodium sulfate, filtered and the filtrate was concentrated under reduced pressure. The residue was purified by column chromatography on silica gel, eluting with 0 ~ 50% ethyl acetate in petroleum ether to give 6,6-dimethyl-1,4-dioxaspiro[4.5]decane-8-carbonitrile (2 g, 9.22 mmol, 56.6% yield) as a yellow oil. MS ESI calculated for C₁₁H₁₇NO₂ [M + H] ⁺ 196.13 found 196.20. ¹H NMR (400 MHz, Chloroform-*d*) δ 4.07-3.80 (m, 4H), 2.73-2.53 (m, 1H), 2.08-1.96 (m, 1H), 1.96-1.80 (m, 2H), 1.70-1.62 (m, 3H), 1.03 (s, 3H), 0.95 (s, 3H).

**Step 2:** To a solution of 6,6-dimethyl-1,4-dioxaspiro[4.5]decane-8-carbonitrile (6 g, 30.7 mmol) in tetrahydrofuran (60 mL) was added lithium bis(trimethylsilyl) amide (1.3 M in THF, 27.2 mL, 35.3 mmol) at 0 °C and the mixture was stirred for 1 h. Then iodomethane (4.80 g, 33.8 mmol) was added into the mixture and the mixture was stirred for 2 h at the same temperature. The reaction mixture was quenched by saturated NH₄Cl and extracted with ethyl acetate (300 mL × 3). The combined organic layers was washed with brine (100 mL), dried over anhydrous sodium sulfate, filtered and the filtrate was concentrated under reduced pressure. The residue was purified by column chromatography on silica gel, eluting with 0~30% ethyl acetate in petroleum ether to give 6,6,8-trimethyl-1,4-dioxaspiro[4.5]decane-8-carbonitrile (5.5 g, 23.65 mmol, 77% yield) as a yellow oil. MS ESI calculated for C₁₂H₁₉NO₂ [M + H] ⁺ 210.14 found 210.15. ¹H NMR (400 MHz, Chloroform-*d*) δ 4.00-3.89 (m, 4H), 2.08-1.97 (m, 2H), 1.77-1.53 (m, 4H), 1.42-1.29 (m, 6H), 0.96-0.85 (m, 3H).

**Step 3:** To a solution of 6,6,8-trimethyl-1,4-dioxaspiro[4.5]decane-8-carbonitrile (1 g, 4.78 mmol) in tetrahydrofuran (10 mL) was added aluminum lithium hydride (2.4 M in THF, 3.98 mL, 9.56 mmol) at 25 °C and the mixture was stirred at 70 °C for 3 h. The reaction mixture was cooled to 0 °C, diluted with diethyl ether (10 mL) and quenched by addition of water (0.3 mL), 15% aqueous NaOH (0.3 mL) and water (0.9 mL). Then the mixture was filtered. The solid was washed with THF (20 mL × 3). The filtrate was concentrated under reduced pressure to give (6,6,8-trimethyl-1,4-dioxaspiro[4.5]decan-8-yl)methanamine (1 g, 4.22 mmol, 88% yield) as a colorless oil. MS ESI calculated for C₁₂H₂₃NO₂ [M + H] ⁺ 214.17 found 214.25. ¹H NMR (300 MHz, Chloroform-*d*) δ 4.04-3.84 (m, 4H), 2.52 (d, *J =* 12.9 Hz, 1H), 2.43 (d, *J* = 13.0 Hz, 1H), 1.75 (ddd, *J* = 13.6, 10.5, 4.3 Hz, 1H), 1.65-1.50 (m, 1H), 1.55-1.44 (m, 1H), 1.44 (d, *J* = 3.8 Hz, 1H), 1.36 (dddd, *J =* 13.0, 8.0, 5.2, 3.0 Hz, 1H), 1.24 (dd, *J =* 13.9, 1.8 Hz, 1H), 1.07 (s, 3H), 0.98 (s, 3H), 0.94 (s, 3H).

**Step 4:** To a solution of (6,6,8-trimethyl-1,4-dioxaspiro[4.5]decan-8-yl)methanamine (5.7 g, 26.7 mmol) in tetrahydrofuran (57 mL) was added di-*tert*-butyl dicarbonate (5.83 g, 26.7 mmol) at 30 °C and the mixture was stirred for 16 h. The reaction mixture was concentrated under reduced pressure. The residue was purified by column chromatography on silica gel, eluting with 0~50 % ethyl acetate in petroleum ether to give *tert*-butyl ((6,6,8-trimethyl-1,4-dioxaspiro[4.5]decan-8-yl)methyl)carbamate (7 g, 20.10 mmol, 75% yield) as a yellow oil. MS ESI calculated for C₁₇H₃₁NO₄ [M + H] ⁺ 314.23 found 314.30. ¹H NMR (300 MHz, Chloroform-*d*) δ 3.96-3.90 (m, 4H), 3.05 (dd, *J =* 13.5, 6.6 Hz, 1H), 2.92 (dd, *J =* 13.5, 6.0 Hz, 1H), 1.79-1.32 (m, 5H), 1.45 (s, 9H), 1.26 (dd, *J* = 13.9, 1.6 Hz, 1H), 1.05 (s, 3H), 0.98 (s, 3H), 0.95 (s, 3H).

**Step 5:** To a solution of *tert*-butyl ((6,6,8-trimethyl-1,4-dioxaspiro[4.5]decan-8-yl)methyl)carbamate (7 g, 22.33 mmol) in tetrahydrofuran (70 mL) was added aluminum lithium hydride (2.4 M in THF, 9.31 mL, 22.33 mmol) at 25 °C and the mixture was stirred at 70 °C for 1 h. Then the mixture was cooled to 0 °C, diluted with diethyl ether (70 mL) and quenched by addition of water (0.8 mL), 15% aqueous NaOH (0.8 mL) and water (2.4 mL). Then the mixture was filtered. The filtrate was washed with THF (70 mL × 3). The filtrate was concentrated under reduced pressure to give *N*-methyl-1-(6,6,8-trimethyl-1,4-dioxaspiro[4.5]decan-8-yl)methanamine (5 g, 19.79 mmol, 89% yield) as a yellow oil. MS ESI calculated for C₁₃H₂₅NO₂ [M + H]⁺ 228.19 found 228.30. ¹H NMR (300 MHz, Chloroform-*d*) δ 4.07-3.85 (m, 4H), 2.44 (s, 3H), 2.41 (d, *J* = 11.4 Hz, 1H), 2.33 (d, *J* = 11.4 Hz, 1H), 1.76 (ddd, *J* = 13.8, 10.7, 4.2 Hz, 1H), 1.60 (dq, *J* = 7.4, 3.9 Hz, 1H), 1.53 (dd, *J =* 13.8, 3.8 Hz, 2H), 1.48-1.35 (m, 1H), 1.31 (dd, *J =* 13.9, 1.7 Hz, 1H), 1.07 (s, 3H), 1.04 (s, 3H), 0.94 (s, 3H).

**Step 6:** To a solution of *N*-methyl-1-(6,6,8-trimethyl-1,4-dioxaspiro[4.5]decan-8-yl)methanamine (5 g, 21.99 mmol) in tetrahydrofuran (50 mL) was added hydrogen chloride (2 M in water, 50.00 mL, 100 mmol) at 25 °C and the mixture was stirred for 2 h. The reaction was neutralized with saturated aqueous sodium carbonate. The mixture was concentrated under reduced pressure to give 2,2,4-trimethyl-4-((methylamino)methyl)cyclohexan-1-one hydrochloride (4 g, 18.2 mmol, 83%) as an off-white solid, which was used for next step without further purification. MS ESI calculated for C₁₁H₂₁NO [M + H] ⁺ 184.16 found 184.30. ¹H NMR (300 MHz, Methanol-*d*₄) δ 3.09 (d, *J* = 12.3 Hz, 1H), 2.89 (d, *J =* 12.3 Hz, 1H), 2.72 (s, 3H), 2.51-2.26 (m, 2H), 1.92-1.73 (m, 2H), 1.77-1.57 (m, 2H), 1.18 (s, 3H), 1.15 (s, 3H), 1.14 (s, 3H).

**Step 7:** To a solution of 2,2,4-trimethyl-4-((methylamino)methyl)cyclohexan-1-one hydrochloride (4 g, 18.20 mmol) in MeOH (40 ml) were added sodium carbonate (5.79 g, 54.6 mmol) and di-*tert*-butyl dicarbonate (5.96 g, 27.3 mmol) at 0 °C and the mixture was stirred at 30 °C for 1 h. The mixture was extracted with ethyl acetate (100 mL × 3), washed with brine (50 mL × 3), dried with Na₂SO₄, filtered and the filtrate was evaporated under reduced pressure. The residue was purified by column chromatography on silica gel , eluting with 0 ~ 30 % ethyl acetate in petroleum ether to give *tert*-butyl methyl((1,3,3-trimethyl-4-oxocyclohexyl)methyl)carbamate (5.2 g, 16.51 mmol, 91% yield) as a yellow oil. MS ESI calculated for C₁₆H₂₉NO₃ [M + H] ⁺ 284.21 found 284.30. ¹H NMR (400 MHz, Chloroform-*d*) δ 3.44- 3.15 (m, 1H), 3.04 (d, *J =* 14.6 Hz, 1H), 2.94 (s, 3H), 2.65-2.48 (m, 1H), 2.50-2.32 (m, 1H), 1.92-1.76 (m, 1H), 1.74-1.62 (m, 2H), 1.56 (dd, *J* = 14.3, 2.3 Hz, 1H), 1.47 (s, 9H), 1.20 (s, 3H), 1.17 (s, 3H), 1.12 (s, 3H).

**Step 8:** To a solution of *tert*-butyl methyl((1,3,3-trimethyl-4-oxocyclohexyl)methyl)carbamate (300 mg, 1.059 mmol) in tetrahydrofuran (3 mL) was added dropwise lithium bis(trimethylsilyl)amide (1.3 M in THF, 1.791 mL, 2.329 mmol) at - 70 °C under N₂ atmosphere. The resulting mixture was stirred at - 70 °C for 1 h. Then a solution of 4-methylbenzenesulfonyl cyanide (288 mg, 1.588 mmol) in tetrahydrofuran (1 mL) was added. After stirring at -70 °C for 30 min, the mixture was warmed to 25 °C and stirred for 2 h. Then the mixture was quenched with saturated aqueous NH₄Cl (5 mL), extracted with ethyl acetate (20 mL × 3). The combined organic layer was washed with brine (10 mL), dried over anhydrous sodium sulfate. After filtration, the filtrate was concentrated under reduced pressure. The residue was purified by column chromatography on silica gel, eluted with 0% ~ 30% ethyl acetate in petroleum ether to give *tert*-butyl ((5-cyano-1,3,3-trimethyl-4-oxocyclohexyl)methyl)(methyl)carbamate (300 mg, 0.875 mmol, 83% yield) as a yellow solid. MS ESI calculated for C₁₇H₂₈N₂O₃ [M + H] ⁺ 309.21 found 309.25. ¹H NMR (300 MHz, Chloroform-*d*) δ 4.76-4.63 (m, 0.25H), 4.42-4.29 (m, 0.25H), 3.99-3.85 (m, 0.5H), 3.23-3.04 (m, 1H), 3.04-2.88 (m, 3H), 2.63-2.50 (m, 0.5H), 2.35-2.21 (m, 0.5H), 2.23-2.08 (m, 1H), 1.97-1.56 (m, 3H), 1.56-1.41 (m, 9H), 1.41-0.94 (m, 9H).

**Step 9:** To a solution of phenyl hypochloroselenoite (1118 mg, 5.84 mmol) in DCM (10 mL) was added dropwise a solution of pyridine (462 mg, 5.84 mmol) in DCM (10 mL) at 0 °C. The mixture was stirred at 0 °C for 20 min and then treated dropwise with a solution of *tert*-butyl ((5-cyano-1,3,3-trimethyl-4-oxocyclohexyl)methyl)(methyl)carbamate (900 mg, 2.92 mmol) in DCM (2 mL). The resulting mixture was stirred at 30 °C for 2 h. Then hydrogen peroxide (30%, 662 mg, 5.84 mmol) was added dropwise at 0 °C and the mixture was vigorously stirred at 0 °C for 1 h. The reaction mixture was quenched by saturated aqueous Na₂S₂O₃ (20 mL), extracted with DCM (50 mL × 3), washed with brine (10 mL), dried over anhydrous sodium sulfate. After filtration, the filtrate was concentrated under reduced pressure and the residue was purified by column chromatography on silica gel, eluted with 0% ~ 35% ethyl acetate in petroleum ether to give *tert*-butyl ((3-cyano-1,5,5-trimethyl-4-oxocyclohex-2-en-1-yl)methyl)(methyl)carbamate (800 mg, 2.350 mmol, 81% yield) as a yellow solid. MS ESI calculated for C₁₇H₂₆N₂O₃ [M + H] ⁺ 307.19 found 307.20. ¹H NMR (300 MHz, Chloroform-*d*) δ 7.46-7.43 (m, 1H), 3.65-3.46 (m, 1H), 3.23-3.08 (m, 1H), 3.00-2.90 (m, 3H), 1.95-1.91 (m, 1H), 1.75-1.70 (m, 1H), 1.49 (s, 9H), 1.32 (s, 3H), 1.25 (s, 3H), 1.24 (s, 3H).

**Step 10:** To a solution of *tert*-butyl ((3-cyano-1,5,5-trimethyl-4-oxocyclohex-2-en-1-yl)methyl)(methyl)carbamate (800 mg, 2.61 mmol) in DCM (8 mL) was added hydrogen chloride (4 M in 1,4 - dioxane, 8 mL, 32.0 mmol) at 0 °C and the mixture was stirred at 25°C for 1 h. The reaction was concentrated under reduced pressure to give 3,5,5-trimethyl-3-((methylamino)methyl)-6-oxocyclohex-1-ene-1-carbonitrile hydrochloride (650 mg, 2.410 mmol, 92% yield) as an off-white solid, which was used for next step without further purification. MS ESI calculated for C₁₂H₁₈N₂O [M + H]⁺ 207.14 found 207.15. ¹H NMR (400 MHz, Methanol-*d*₄) δ 7.60 (s, 1H), 3.18 (s, 2H), 2.77 (s, 3H), 2.12 (d, *J =* 14.7 Hz, 1H), 1.94 (dd, *J* = 14.7, 1.6 Hz, 1H), 1.44 (s, 3H), 1.28 (s, 3H), 1.23 (s, 3H).

**Step 11:** To a solution of 3,5,5-trimethyl-3-((methylamino)methyl)-6-oxocyclohex-1-ene-1-carbonitrile (50 mg, 0.242 mmol) in DCM (2.3 mL) were added TEA (0.068 mL, 0.485 mmol) and benzoyl chloride (40.9 mg, 0.291 mmol) at room temperature. The resulted mixture was stirred at 30 °C for 3 h. To the reaction was added water (20 mL), and the mixture was extracted with ethyl acetate (100 mL). The organic layer was washed with water (100 mL), brine (100 mL), dried over anhydrous sodium sulfate, filtered and the filtrate was concentrated under reduced pressure. The residue was purified by Prep-TLC eluted with petroleum ether : ethyl acetate = 1 : 1 to give *N*-((3-cyano-1,5,5-trimethyl-4-oxocyclohex-2-en-1-yl)methyl)-*N-*methylbenzamide (63 mg, 0.203 mmol, 84% yield) as a white oil. MS ESI calculated for C₁₉H₂₂N₂O₂ [M + H]⁺ 311.17 found 311.10.

**Step 12:** The product was separated by Prep-Chiral-HPLC with the following conditions: Column: CHIRALPAK IH, 2 × 25 cm, 5 µm; Mobile Phase A: Hexanes, Mobile Phase B: EtOH; Flow rate: 20 mL/min; Gradient: 30% B to 30% B in 13.6 min; Wave Length: 254 / 220 nm; RT1: 7.87 min; RT2: 10.55 min. The first product containing (RT1: 7.87 min) peak was combined and concentrated under reduced pressure, then freeze-dried to give *N*-((3-cyano-1,5,5-trimethyl-4-oxocyclohex-2-en-1-yl)methyl)-*N*-methylbenzamide (24.7 mg, 0.080 mmol, 27.4% yield) as a white solid. MS ESI calculated for C₁₉H₂₂N₂O₂ [M + H]⁺ 311.17 found 311.10. ¹H NMR (300 MHz, Chloroform-*d*) δ 7.53-7.22 (m, 6H), 3.97 (d, *J =* 13.5 Hz, 1H), 3.43 (d, *J =* 14.1 Hz, 1H), 3.07 (s, 3H), 2.10 (d, *J =* 13.8 Hz, 1H), 1.86 (d, *J =* 14.7 Hz, 1H), 1.44 (s, 3H), 1.29 (s, 6H). The second product containing (RT2: 10.55 min) peak was combined and concentrated under reduced pressure, then freeze-dried to give *N*-((3-cyano-1,5,5-trimethyl-4-oxocyclohex-2-en-1-yl)methyl)-*N*-methylbenzamide (21.8 mg, 0.070 mmol, 24.22% yield) as a white solid. MS ESI calculated for C₁₉H₂₂N₂O₂ [M + H]⁺ 311.17 found 311.10. ¹H NMR (300 MHz, Chloroform-*d*) δ 7.61-7.14 (m, 6H), 3.97 (d, *J =* 13.5 Hz, 1H), 3.43 (d, *J =* 13.5 Hz, 1H), 3.05 (s, 3H), 2.10 (d, *J* = 13.8 Hz, 1H), 1.86 (d, *J* = 14.7 Hz, 1H), 1.41 (s, 3H), 1.27 (s, 6H).

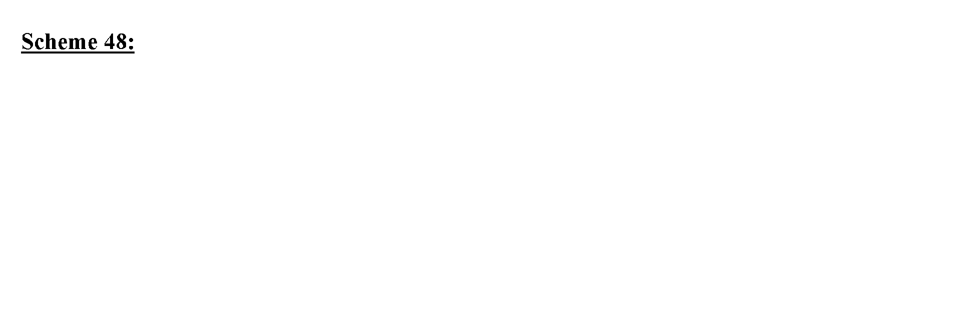

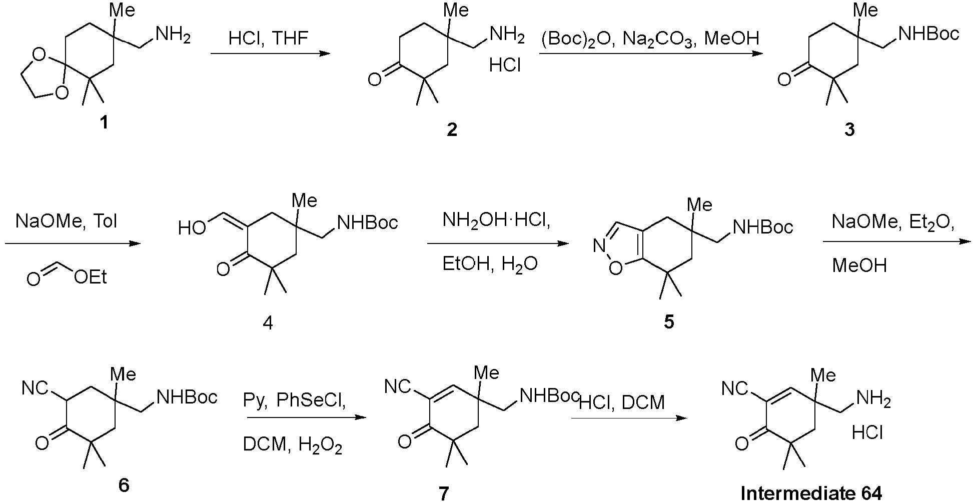

### Intermediate 64: tert-butyl ((3-cyano-1,5,5-trimethyl-4-oxocyclohex-2-en-1-yl)methyl)carbamate

**Step 1:** To a solution of (6,6,8-trimethyl-1,4-dioxaspiro[4.5]decan-8-yl)methanamine (300 mg, 1.406 mmol) in tetrahydrofuran (3 mL) was added hydrogen chloride (2 M in water, 3 mL, 6.00 mmol) at 25 °C and the mixture was stirred for 2 h. The reaction was concentrated under reduced pressure to give 4-(aminomethyl)-2,2,4-trimethylcyclohexan-1-one hydrochloride (289 mg, 1.406 mmol) as a white solid, which was used directly for next step without further purification. MS ESI calculated for C₁₀H₁₉NO [M + H] ⁺ 170.15 found 170.15.

**Step 2:** To a solution of 4-(aminomethyl)-2,2,4-trimethylcyclohexan-1-one hydrochloride (289 mg, 1.406 mmol) in MeOH (2.5 mL) were added sodium carbonate (464 mg, 4.37 mmol) and di-*tert*-butyl dicarbonate (477 mg, 2.187 mmol) at 0 °C and the mixture was stirred at 25 °C for 1 h. The mixture was diluted with ethyl acetate (50 mL), washed with brine (10 mL × 3), dried over anhydrous sodium sulfate, filtered and the filtrate was evaporated under reduced pressure. The residue was purified by column chromatography on silica gel, eluting with 0 ~ 30% ethyl acetate in petroleum ether to give *tert*-butyl ((1,3,3-trimethyl-4-oxocyclohexyl)methyl)carbamate (280 mg, 0.935 mmol, 70.0% yield) as a yellow solid. MS ESI calculated for C₁₅H₂₇NO₃ [M + H] ⁺ 270.20 found 270.20. ¹H NMR (400 MHz, Chloroform-*d*) δ 4.66 (brs, 1H), 3.18 (dd, *J =* 13.9, 7.0 Hz, 1H), 3.00 (dd, *J* = 13.8, 6.4 Hz, 1H), 2.55-2.40 (m, 2H), 1.80-1.73 (m, 1H), 1.68-1.61 (m, 2H), 1.54 (d, *J =* 14.4 Hz, 1H), 1.46 (s, 9H), 1.18 (s, 3H), 1.13 (s, 3H), 1.10 (s, 3H).

**Step 3:** To a solution of *tert*-butyl ((1,3,3-trimethyl-4-oxocyclohexyl)methyl)carbamate (400 mg, 1.485 mmol) in toluene (4 mL) were added ethyl formate (550 mg, 7.42 mmol) and sodium methanolate (401 mg, 7.42 mmol) at 25 °C and the mixture was stirred for 1 h. Then the mixture was quenched with saturated aqueous NH₄Cl (10 mL), extracted with ethyl acetate (30 mL × 3). The combined organic layer was washed with brine (30 mL), dried over anhydrous sodium sulfate. After filtration, the filtrate was concentrated under reduced pressure to give *tert*-butyl ((5-(hydroxymethylene)-1,3,3-trimethyl-4-oxocyclohexyl)methyl)carbamate (442 mg, 1.485 mmol) as a brown solid, which was used directly for next step without further purification. MS ESI calculated for C₁₆H₂₇NO₄ [M + H] ⁺ 298.19 found 298.20.

**Step 4:** To a solution of *tert*-butyl ((5-(hydroxymethylene)-1,3,3-trimethyl-4-oxocyclohexyl)methyl)carbamate (442 mg, 1.485 mmol) in EtOH (20 mL) was added hydroxylamine hydrochloride (93 mg, 1.345 mmol) at 25 °C and the mixture was stirred at 80 °C for 1 h. The reaction mixture was concentrated under reduced pressure. The residue was purified by Column: XBridge Prep C¹⁸ OBD Column, 19 × 150 mm, 5 um; Mobile Phase A: Water, Mobile Phase B: MeCN; Flow rate: 20 mL/min; Gradient: 0% B to 50% B in 30 min, 50 % B; detector: UV 220 / 254 nm to afford *tert*-butyl ((5,7,7-trimethyl-4,5,6,7-tetrahydrobenzo[*d*]isoxazol-5-yl)methyl)carbamate (140 mg, 0.476 mmol, 30.4% yield) as a white solid. MS ESI calculated for C₁₆H₂₆N₂O₃ [M + H] ⁺ 295.19 found 295.25. ¹H NMR (400 MHz, Chloroform-*d*) δ 8.02 (s, 1H), 4.66 (brs, 1H), 3.14-3.03 (m, 2H), 2.35 (d, *J =* 15.3 Hz, 1H), 2.20 (d, *J* = 15.4 Hz, 1H), 1.68 (d, *J* = 14.0 Hz, 1H), 1.58 (d, *J =* 14.1 Hz, 1H), 1.47 (s, 9H), 1.38 (s, 3H), 1.34 (s, 3H), 1.01 (s, 3H).

**Step 5:** To a solution of *tert*-butyl ((5,7,7-trimethyl-4,5,6,7-tetrahydrobenzo[*d*]isoxazol-5-yl)methyl)carbamate (140 mg, 0.476 mmol) in diethyl ether (2.5 mL) was added sodium methanolate (30% in MeOH, 1.285 g, 7.13 mmol) in MeOH (1 mL) at 0 °C and the mixture was stirred at 25 °C for 2 h. The reaction was neutralized with acetic acid. The resulting mixture was diluted with ethyl acetate (20 mL), washed with brine (5 mL × 3), dried over sodium sulfate, filtered and the filtrate was evaporated under reduced pressure. The residue was purified by column chromatography on silica gel, eluted with 0% ~ 30% ethyl acetate in petroleum ether to give *tert*-butyl ((5-cyano-1,3,3-trimethyl-4-oxocyclohexyl)methyl)carbamate (120 mg, 0.408 mmol, 86% yield) as a yellow solid. MS ESI calculated for C₁₆H₂₆N₂O₃ [M + H] ⁺ 295.19 found 295.10

**Step 6:** To a solution of phenyl hypochloroselenoite (156 mg, 0.815 mmol) in DCM (1 mL) was added pyridine (64.5 mg, 0.815 mmol) at 0 °C. The mixture was stirred at 0 °C for 20 min and then treated dropwise with a solution of *tert*-butyl ((5-cyano-1,3,3-trimethyl-4-oxocyclohexyl)methyl)carbamate (120 mg, 0.408 mmol) in DCM (0.5 mL). The resulting mixture was stirred at 25 °C for 2 h. Then hydrogen peroxide (30%, 92 mg, 0.815 mmol) was added dropwise at 0 °C and the mixture was vigorously stirred at 0 °C for 1 h. The reaction mixture was quenched by saturated aqueous Na₂S₂O₃ (5 mL), extracted with DCM (10 mL × 3), washed with brine (10 mL), dried over anhydrous sodium sulfate. After filtration, the filtrate was concentrated under reduced pressure and the residue was purified by column chromatography on silica gel, eluted with 0% ~ 35% ethyl acetate in petroleum ether to give *tert*-butyl ((3-cyano-1,5,5-trimethyl-4-oxocyclohex-2-en-1-yl)methyl)carbamate (90 mg, 0.277 mmol, 68.0% yield) as a yellow solid. MS ESI calculated for C₁₆H₂₄N₂O₃ [M + H] ⁺ 293.18 found 293.20. ¹H NMR (400 MHz, Chloroform-*d*) δ 7.37 (s, 1H), 4.72 (brs, 1H), 3.27-3.16 (m, 2H), 1.93 (d, *J =* 14.6 Hz, 1H), 1.70 (d, *J =* 14.6 Hz, 1H), 1.47 (s, 9H), 1.31 (s, 3H),1.27 (s, 3H), 1.25 (s, 3H).

**Step 7:** To a solution of *tert*-butyl ((3-cyano-1,5,5-trimethyl-4-oxocyclohex-2-en-1-yl)methyl)carbamate (100 mg, 0.342 mmol) in DCM (1 mL) was added hydrogen chloride (4 M in dioxane, 1 mL, 4.00 mmol) at 25 °C and the mixture was stirred for 2 h. The reaction mixture was concentrated under reduced pressure to give 3-(aminomethyl)-3,5,5-trimethyl-6-oxocyclohex-1-ene-1-carbonitrile hydrochloride (78 mg, 0.341 mmol) as a brown solid, which was used directly for next step without further purification. MS ESI calculated for C₁₁H₁₆N₂O [M + H] ⁺ 193.13 found 193.15. ¹H NMR (400 MHz, Methanol-*d*₄) δ 7.62-7.59 (m, 1H), 3.11 (s, 2H), 2.08 (d, *J* = 14.3 Hz, 1H), 1.92 (dd, *J* = 14.7, 1.6 Hz, 1H), 1.44 (s, 3H), 1.30 (s, 3H), 1.24 (s, 3H).

### Examples

The following experimental procedures detail the preparation of specific examples of the instant disclosure.

Note: Many of the compounds claimed exist as a mixture of rotamers in solution at room temperature, which complicates their analyses by ¹H-NMR spectroscopy. In these cases, the peak shifts are listed as ranges of multiplets that encompass the signals from both rotamers, rather than describing individual rotamer peaks.

Note: Racemic compounds purified by reverse phase or normal phase chromatography were further purified by chiral SFC or chiral HPLC. Where mentioned, enantiomer 1 refers to the first eluting peak and enantiomer 2 refers to the second eluting peak. Where more than one chiral centre is present, compounds are reported as active isomers in order of elution or preparation where absolute stereochemistry has not been determined. Otherwise, compounds are achiral or racemic.

### Example 1 - enantiomer 1: 9,9-dimethyl-8-oxo-1-oxaspiro[4.5]dec-6-ene-7-carbonitrile

### Example 2 - enantiomer 2: 9,9-dimethyl-8-oxo-1-oxaspiro[4.5]dec-6-ene-7-carbonitrile

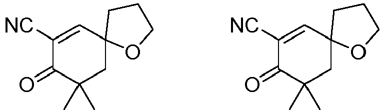

**Step 1:** 1-Oxaspiro[4.5]decan-8-one (1 g, 6.5 mmol) was dissolved in tBuOH (32 mL). Potassium tert-butoxide (1.82 g, 16.2 mmol) was added slowly under a nitrogen atmosphere, and vthe mixture was stirred at room temperature for 1 h. Methyl iodide (0.852 mL, 13.6 mmol) was added dropwise, and the mixture was stirred at room temperature overnight. Water (60 mL) was added and the aqueous phase was extracted with EtOAc (3x). The combined organic layers were washed with brine, dried over anhydrous sodium sulfate, filtered, and concentrated under reduced pressure. The residue was purified by column chromatography on silica (0-100% Et₂O/Hexanes) to give 7,7-dimethyl-1-oxaspiro[4.5]decan-8-one as a liquid. MS: 183 (M + H). Examples can be made using a similar procedure to that described in Steps 2-5, where Step 1 is not required.

**Step 2:** To a suspension of sodium hydride (239 mg, 5.97 mmol) in THF (8.5 mL) at 0 °C was added a solution of 7,7-dimethyl-1-oxaspiro[4.5]decan-8-one (946 mg, 5.19 mmol) in THF (8.5 mL). The reaction mixture was stirred for 20 minutes at 0 °C. Ethyl formate (1.26 mL, 15.6 mmol) was added and the reaction mixture was allowed to warm to room temperature and stirred overnight. The mixture was quenched with sat. NH₄Cl (aq) and extracted with EtOAc (2x). The combined organic layers were dried over anhydrous sodium sulfate, filtered, and concentrated under reduced pressure to afford (Z)-9-(hydroxymethylene)-7,7-dimethyl-1-oxaspiro[4.5]decan-8-one as a liquid, which was used without further purification. MS: 211 (M + H).

**Step 3:** To a solution of (Z)-9-(hydroxymethylene)-7,7-dimethyl-1-oxaspiro[4.5]decan-8-one (984 mg, 4.68 mmol) in 10:1 EtOH:H₂O (12 mL) was added a solution of hydroxylamine hydrochloride (358 mg, 5.15 mmol) in 10:1 EtOH: H₂O (12 mL). The reaction mixture was stirred at 40 °C overnight. The mixture was cooled to room temperature and concentrated under reduced pressure. The residue was basified with sat. NaHCO₃ (aq) and extracted with EtOAc (3x). The combined organic layers were washed with brine, dried over anhydrous sodium sulfate, filtered, and concentrated under reduced pressure. The residue was purified by column chromatography on silica (0-50% Et₂O/hexanes) to give 7,7-dimethyl-4',5',6,7-tetrahydro-3'H,4H-spiro[benzo[d]isoxazole-5,2'-furan] as a solid. MS: 208 (M + H). ¹H NMR (600 MHz, Chloroform-*d*) δ 8.03 (s, 1H), 3.88 (t, *J =* 6.9 Hz, 2H), 2.63 - 2.54 (m, 2H), 2.05 - 1.99 (m, 2H), 1.96 (d, *J* = 13.9 Hz, 1H), 1.84 (t, *J* = 7.5 Hz, 2H), 1.74 (d, *J* = 13.9 Hz, 1H), 1.45 (s, 3H), 1.36 (s, 3H).

Examples can be made using a similar procedure to that described in Steps 3-5, where Step 1-2 is not required.

**Step 4:** To a solution of 7,7-dimethyl-4',5',6,7-tetrahydro-3'H,4H-spiro[benzo[d]isoxazole-5,2'-furan] (473 mg, 2.28 mmol) in MeOH (11 mL) was added sodium methoxide (30% in MeOH, 1.31 mL, 6.85 mmol). The reaction mixture was stirred at room temperature overnight. The mixture was concentrated under reduced pressure, diluted with EtOAc, and neutralized with 1 M aq HCl (1.6 mL). The layers were separated, and the aqueous layer was extracted with EtOAc. The combined organic layers were washed with brine, dried over anhydrous sodium sulfate, filtered, and concentrated under reduced pressure to give 9,9-dimethyl-8-oxo-1-oxaspiro[4.5]decane-7-carbonitrile as solid, which was used without further purification. MS: 208 (M + H).

**Step 5:** To a solution of 9,9-dimethyl-8-oxo-1-oxaspiro[4.5]decane-7-carbonitrile (70 mg, 0.34 mmol) in THF (675 µL) at room temperature was added palladium(II) acetate (15.2 mg, 0.068 mmol). The mixture was stirred at room temperature overnight. Added palladium(II) acetate up to 1 full equivalent and the reaction was stirred for 48 h at room temperature. The mixture was concentrated under reduced pressure, and then diluted with DCM (5 mL) and 1:1 water:brine (5 mL). The organic layer was collected via a phase separator and concentrated under reduced pressure. The residue was purified by column chromatography on silica (0-70% Et₂O/Hexanes) to give 9,9-dimethyl-8-oxo-1-oxaspiro[4.5]dec-6-ene-7-carbonitrile. The racemic mixture was purified by chiral SFC (Lux-2 column, 20%/80% MeOH/CO₂ with 0.1% NH₄OH modifier) to afford two products as oils: 9,9-dimethyl-8-oxo-1-oxaspiro[4.5]dec-6-ene-7-carbonitrile (Example 1 - enantiomer 1). MS: 206 (M + H). ¹H NMR (600 MHz, DMSO-*d*₆) δ 7.81 (s, 1H), 3.88 - 3.80 (m, 2H), 2.13 - 2.04 (m, 4H), 1.98 - 1.91 (m, 2H), 1.15 (s, 3H), 1.11 (s, 3H). 9,9-dimethyl-8-oxo-1-oxaspiro[4.5]dec-6-ene-7-carbonitrile (Example 2 - enantiomer 2). MS: 206 (M + H). ¹H NMR (600 MHz, DMSO-*d*₆) δ 7.81 (s, 1H), 3.88 - 3.80 (m, 2H), 2.12 - 2.04 (m, 4H), 1.98 - 1.91 (m, 2H), 1.15 (s, 3H), 1.11 (s, 3H).

**Alternate Step 5:** To a solution of pyridine (0.18 mL, 2.2 mmol) in DCM (2 mL) at 0 °C was added a solution of phenylselenyl chloride (420 mg, 2.2 mmol) in DCM (3 mL). The reaction mixture was stirred for 20 minutes at 0 °C before the addition of a solution of 3,3-dimethyl-4-oxo-3'H-spiro[cyclohexane-1,1'-isobenzofuran]-5-carbonitrile (280 mg, 1.1 mmol) in DCM (3 mL). The reaction mixture was stirred for 1 h at 0 °C, then washed with aqueous HCl (1 M, 5 mL). The phases were separated, and the organic phase was treated with hydrogen peroxide (50 wt. % in H₂O, 1.3 mL, 22 mmol) at 0 °C. The reaction mixture was stirred for 15 minutes, then the phases were separated and the organic phase was concentrated under reduced pressure. The residue was purified by mass triggered reverse phase HPLC (ACN/H₂O with 0.1% TFA modifier) to afford 5,5-dimethyl-4-oxo-3'H-spiro[cyclohexane-1,1'-isobenzofuran]-2-ene-3-carbonitrile. The racemic mixture was purified by chiral SFC (OJ-H column, 15%/85% MeOH/CO₂ with 0.1% NH₄OH modifier) to afford two products as solids: 5,5-dimethyl-4-oxo-3'H-spiro[cyclohexane-1,1'-isobenzofuran]-2-ene-3-carbonitrile (Example 8 - enantiomer 1). MS: 254 (M + H). ¹H NMR (600 MHz, CDCl₃) δ 7.42-7.33 (m, 2H), 7.30 (d, *J* = 7.4 Hz, 1H), 7.17 (d, *J =* 2.0 Hz, 1H), 7.09 (d, *J =* 7.5 Hz, 1H), 5.21 (s, 2H), 2.29 (d, *J =* 15.0 Hz, 1H), 2.20 (dd, *J =* 15.0, 2.0 Hz, 1H), 1.43 (s, 3H), 1.23 (s, 3H) 5,5-dimethyl-4-oxo-3'H-spiro[cyclohexane-1,1'-isobenzofuran]-2-ene-3-carbonitrile (Example 9 - enantiomer 2). MS: 254 (M + H). ¹H NMR (600 MHz, CDCl₃) δ 7.42-7.33 (m, 2H), 7.30 (d, *J* = 7.4 Hz, 1H), 7.17 (d, *J* = 2.1 Hz, 1H), 7.09 (d, *J* = 7.5 Hz, 1H), 5.21 (s, 2H), 2.29 (d, *J =* 15.0 Hz, 1H), 2.20 (dd, *J* = 15.0, 2.1 Hz, 1H), 1.43 (s, 3H), 1.23 (s, 3H).

**Table 7: The following examples were prepared using a similar procedure as described above for examples 1, 2, 8 and 9. For the oxidation conditions, 1 = Palladium(II) acetate and 2 = PhSeCl, Pyridine, H₂O₂**

| **Ex** | **Structure** | **Compound Name** | **Oxidation Conditions** | **Exact Mass [M+H]⁺** |
|---|---|---|---|---|
| 3 | 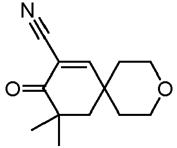 | 10,10-dimethyl-9-oxo-3-oxaspiro[5.5]undec-7-ene-8-carbonitrile | 1 | 220 |
| 4 | 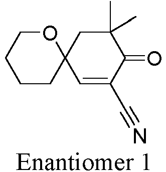 | 10,10-dimethyl-9-oxo-1-oxaspiro[5.5]undec-7-ene-8-carbonitrile | 1 | 220 |
| 5 | 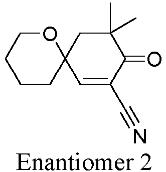 | 10,10-dimethyl-9-oxo-1-oxaspiro[5.5]undec-7-ene-8-carbonitrile | 1 | 220 |
| 6 | 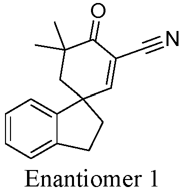 | 5,5-dimethyl-4-oxo-2',3'-dihydrospiro[cycloh ex-2-ene-1,1'-indene]-3-carbonitrile | 1 | 252 |
| 7 | 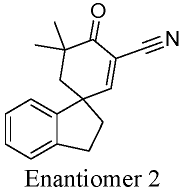 | 5,5-dimethyl-4-oxo-2',3'-dihydrospiro[cycloh ex-2-ene-1,1'-indene]-3-carbonitrile | 1 | 252 |
| 8 | 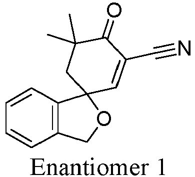 | 5',5'-dimethyl-4'-oxo-3H-spiro[2-benzofuran-1,1'-cyclohex[2]ene]-3'-carbonitrile | 2 | 254 |
| 9 | 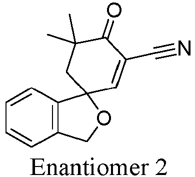 | 5',5'-dimethyl-4'-oxo-3H-spiro[2-benzofuran-1,1'-cyclohex[2]ene]-3'-carbonitrile | 2 | 254 |
| 10 | 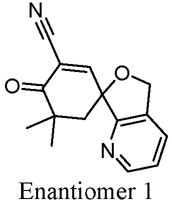 | 5,5-dimethyl-4-oxo-5'H-spiro[cyclohex-2-ene-1,7'-furo[3,4-b]pyridine]-3-carbonitrile | 2 | 255 |
| 11 | 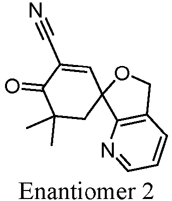 | 5,5-dimethyl-4-oxo-5'H-spiro[cyclohex-2-ene-1,7'-furo[3,4-b]pyridine]-3-carbonitrile | 2 | 255 |
| 12 | 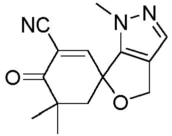 | 1',5,5-1',5,5-trimethyl-4-oxo-1',4'-dihydrospiro[cycloh exane-1,6'-furo[3,4-c]pyrazol]-2-ene-3-carbonitrile | 2 | 258 |
| 13 | 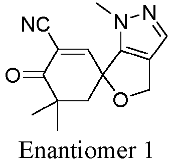 | 1',5,5-1',5,5-trimethyl-4-oxo-1',4'-dihydrospiro[cycloh exane-1,6'-furo[3,4-c]pyrazol]-2-ene-3-carbonitrile | 2 | 258 |
| 14 | 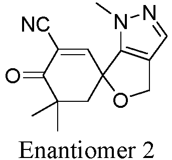 | 1',5,5-1',5,5-trimethyl-4-oxo-1',4'-dihydrospiro[cycloh exane-1,6'-furo[3,4-c]pyrazol]-2-ene-3-carbonitrile | 2 | 258 |
| 15 | 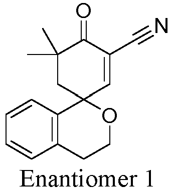 | 5',5'-dimethyl-4'-oxo-3,4-dihydrospiro[2-benzopyran-1,1'-cyclohex[2]ene]-3'-carbonitrile | 2 | 268 |
| 16 | 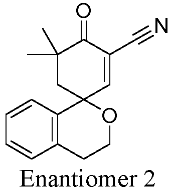 | 5',5'-dimethyl-4'-oxo-3,4-dihydrospiro[2-benzopyran-1,1'-cyclohex[2]ene]-3'-carbonitrile | 2 | 268 |
| 17 | 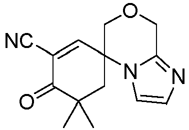 | 5,5-dimethyl-4-oxo-6'*H*,8'*H-*spiro[cyclohexane-1,5'-imidazo[2,1-*c*][1,4]oxazin]-2-ene-3-carbonitrile | 1 | 258 |
| 18 | 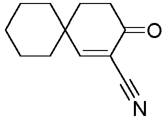 | 3-oxospiro[5.5]undec-1-ene-2-carbonitrile | 2 | 190 |
| 19 | 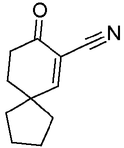 | 8-oxospiro[4.5]dec-6-ene-7-carbonitrile | 2 | 176 |
| 20 | 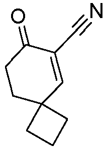 | 7-oxospiro[3.5]non-5-ene-6-carbonitrile | 2 | 162 |
| 21 | 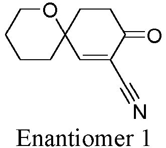 | 9-oxo-1-oxaspiro[5.5]undec-7-ene-8-carbonitrile | 2 | 192 |
| 22 | 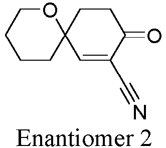 | 9-oxo-1-oxaspiro[5.5]undec-7-ene-8-carbonitrile | 2 | 192 |
| 23* | 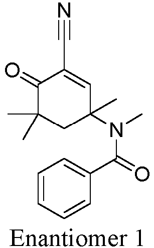 | N-(3-cyano-1,5,5-trimethyl-4-oxocyclohex-2-en-1-yl)-N-methylbenzamide | 1 | 297 |
| 24* | 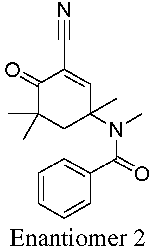 | N-(3-cyano-1,5,5-trimethyl-4-oxocyclohex-2-en-1-yl)-N-methylbenzamide | 1 | 297 |
| 25* | 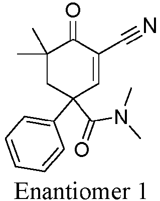 | 5-cyano-N,N,3,3-tetramethyl-4-oxo-3,4-dihydro[1, 1'-biphenyl]-1(2H)-carboxamide | 1 | 297 |
| 26* | 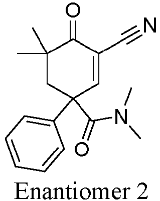 | 5-cyano-N,N,3,3-tetramethyl-4-oxo-3,4-dihydro[1, 1'-biphenyl]-1(2H)-carboxamide | 1 | 297 |
| 27 | 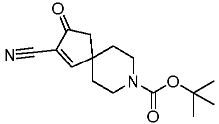 | *tert*-butyl 2-cyano-3-oxo-8-azaspiro[4.5]dec-1-ene-8-carboxylate | 2 | 221 (- *t*Bu) |
| 28 | 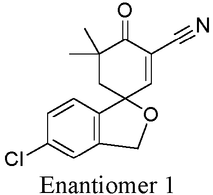 | 5-chloro-5',5'-dimethyl-4'-oxo-3H-spiro[2-benzofuran-1,1'-cyclohex[2]ene]-3'-carbonitrile | 2 | 288 |
| 29 | 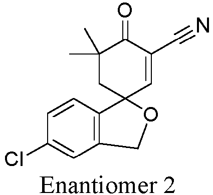 | 5-chloro-5',5'-dimethyl-4'-oxo-3H-spiro[2-benzofuran-1,1'-cyclohex[2]ene]-3'-carbonitrile | 2 | 288 |
| 30 | 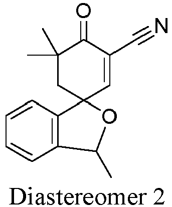 | 3,5',5'-trimethyl-4'-oxo-3H-spiro[2-benzofuran-1,1'-cyclohex[2]ene]-3'-carbonitrile | 2 | 268 |
| 31 | 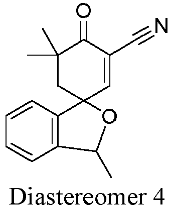 | 3,5',5'-trimethyl-4'-oxo-3H-spiro[2-benzofuran-1,1'-cyclohex[2]ene]-3'-carbonitrile | 2 | 268 |
| 32 | 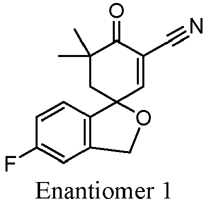 | 5-fluoro-5',5'-dimethyl-4'-oxo-3H-spiro[2-benzofuran-1,1'-cyclohex[2]ene]-3'-carbonitrile | 2 | 272 |
| 33 | 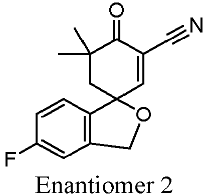 | 5-fluoro-5',5'-dimethyl-4'-oxo-3H-spiro[2-benzofuran-1,1'-cyclohex[2]ene]-3'-carbonitrile | 2 | 272 |
| 34 | 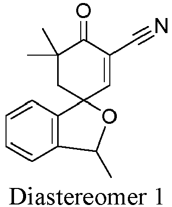 | 3,5', 5'-trimethyl-4'-oxo-3H-spiro[2-benzofuran-1,1'-cyclohex[2]ene]-3'-carbonitrile | 2 | 268 |
| 35 | 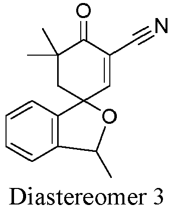 | 3,5',5'-trimethyl-4'-oxo-3H-spiro[2-benzofuran-1,1'-cyclohex[2]ene]-3'-carbonitrile | 2 | 268 |
| 36 | 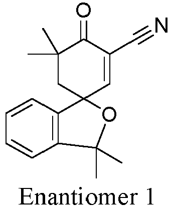 | 3,3,5',5'-tetramethyl-4'-oxo-3H-spiro[2-benzofuran-1,1'-cyclohex[2]ene]-3'-carbonitrile | 2 | 282 |
| 37 | 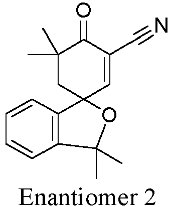 | 3,3,5',5'-tetramethyl-4'-oxo-3H-spiro[2-benzofuran-1,1'-cyclohex[2]ene]-3'-carbonitrile | 2 | 282 |
| 38 | 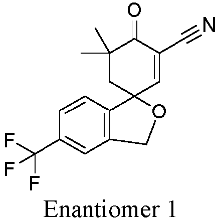 | 5',5'-dimethyl-4'-oxo-5-(trifluoromethyl)-3H-spiro[2-benzofuran-1,1'-cyclohex[2]ene]-3'-carbonitrile | 2 | 322 |
| 39 | 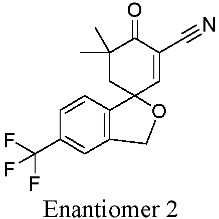 | 5',5'-dimethyl-4'-oxo-5-(trifluoromethyl)-3H-spiro[2-benzofuran-1,1'-cyclohex[2]ene]-3'-carbonitrile | 2 | 322 |
| 40 | 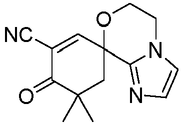 | 5,5-dimethyl- 4-oxo-5',6'-dihydrospiro[cycloh exane-1,8'-imidazo[2,1-c][1,4]oxazin]-2-ene-3-carbonitrile | 2 | 258 |
| 41 | 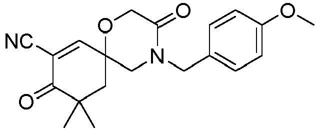 | 4-(4-methoxybenzyl)-10,10-dimethyl-3,9-dioxo-1-oxa-4-azaspiro[5.5]undec-7-ene-8-carbonitrile | 1 | 355 |
| 42 | 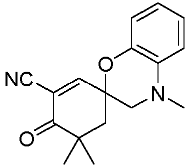 | 4,5', 5'-trimethyl-4'-oxo-3,4-dihydrospiro[benzo[ b][1,4]oxazine-2,1'-cyclohexan]-2'-ene-3'-carbonitrile | 1 | 283 |
| 43 | 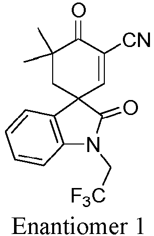 | 5,5-dimethyl-2',4-dioxo-1'-(2,2,2-trifluoroethyl)spiro[c yclohexane-1,3'-indolin]-2-ene-3-carbonitrile | 2 | 349 |
| 44 | 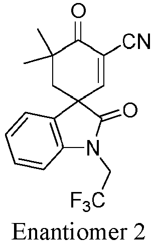 | 5,5-dimethyl-2',4-dioxo-1'-(2,2,2-trifluoroethyl)spiro[c yclohexane-1,3'-indolin]-2-ene-3-carbonitrile | 2 | 349 |
| 45 | 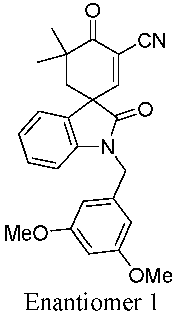 | 1'-(3,5-dimethoxybenzyl)-5,5-dimethyl-2',4-dioxospiro[cyclohex ane-1,3'-indolin]-2-ene-3-carbonitrile | 2 | 417 |
| 46 | 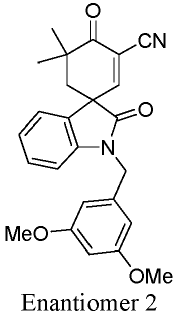 | 1'-(3,5-dimethoxybenzyl)-5,5-dimethyl-2',4-dioxospiro[cyclohex ane-1,3'-indolin]-2-ene-3-carbonitrile | 2 | 417 |
| 47 | 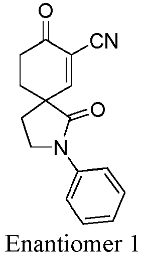 | 1,8-dioxo-2-phenyl-2-azaspiro[4.5]dec-6-ene-7-carbonitrile | 2 | 267 |
| 48 | 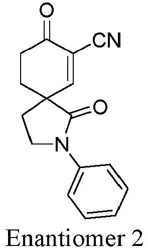 | 1,8-dioxo-2-phenyl-2-azaspiro[4.5]dec-6-ene-7-carbonitrile | 2 | 267 |
| 49 | 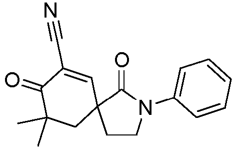 | 9,9-dimethyl-1,8-dioxo-2-phenyl-2-azaspiro[4.5]dec-6-ene-7-carbonitrile | 2 | 295 |
| 50 | 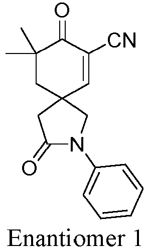 | 9,9-dimethyl-3,8-dioxo-2-phenyl-2-azaspiro[4.5]dec-6-ene-7-carbonitrile | 2 | 295 |
| 51 | 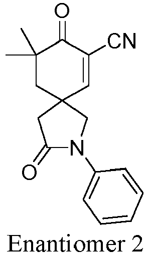 | 9,9-dimethyl-3,8-dioxo-2-phenyl-2-azaspiro[4.5]dec-6-ene-7-carbonitrile | 2 | 295 |
| 52 | 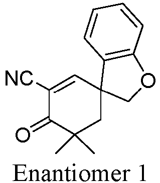 | 5',5'-dimethyl-4'-oxo-2H-spiro[benzofuran-3,1'-cyclohexan]-2'-ene-3'-carbonitrile | 1 | 254 |
| 53 | 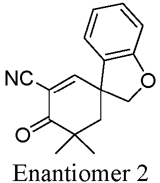 | 5',5'-dimethyl-4'-oxo-2H-spiro[benzofuran-3,1'-cyclohexan]-2'-ene-3'-carbonitrile | 1 | 276 [M + Na] |

| | | | | |
|---|---|---|---|---|
| * reference compound | | | | |

### Example 54: 5,5-dimethyl-4-oxo-1'H-spiro[cyclohexane-1,3'-furo[3,4-c]pyridin]-2-ene-3-carbonitrile

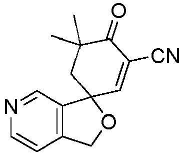

**Step 1:** To a stirred mixture of 1'*H*-spiro[cyclohexane-1,3'-furo[3,4-*c*]pyridin]-4-one (350 mg, 1.722 mmol) in toluene (7 mL) were added potassium *tert*-butoxide (406 mg, 3.62 mmol) and iodomethane (464 mg, 3.27 mmol) at ambient temperature. The mixture was stirred at 110 °C for 3 h. Then the reaction mixture was quenched by saturated aq. NH₄Cl (30 mL), extracted with ethyl acetate (3 × 50 mL). The combined organic phase was washed with brine (50 mL), dried over anhydrous sodium sulfate. After filtration, the filtrate was concentrated under reduced pressure. The residue was purified by RP-Combi-Flash with the following conditions: Column: 18 gel column (80 g), 20-35 µm; Mobile Phase A: 5 mM aq. NH₄HCO₃; Mobile Phase B: MeCN; Gradient: 30% hold 2 min, up to 35% within 20 min; Flow rate: 70 mL/min; Detector: UV 254 & 210 nm; RT: 15 min to get 3,3-dimethyl-1'*H*-spiro[cyclohexane-1,3'-furo[3,4-c]pyridin]-4-one (110 mg, 0.476 mmol, 27% yield) as a white solid. MS: *m*/*z* = 232.20 [M + H]⁺. ¹H NMR (400 MHz, Chloroform-*d*) δ 8.57 - 8.56 (m, 1H), 8.42 (s, 1H), 7.35 - 7.34 (m, 1H), 5.19 (s, 2H), 3.20 - 3.11 (m, 1H), 2.37 - 2.32 (m, 1H), 2.20 - 2.13 (m, 2H), 2.05 (s, 2H), 1.42 (s, 3H), 1.11 (s, 3H).

**Step 2:** To a stirred mixture of 3,3-dimethyl-1'*H*-spiro[cyclohexane-1,3'-furo[3,4-*c*]pyridin]-4-one (88 mg, 0.380 mmol) in THF (2 mL) was added 1 M lithium bis(trimethylsilyl)amide in THF (0.761 mL, 0.761 mmol) at -78 °C. The mixture was stirred at -78 °C for 1 h. Then to the mixture was added 4-methylbenzenesulfonyl cyanide (103 mg, 0.571 mmol) at -78 °C. The mixture was stirred at -78 °C for 30 min and at ambient temperature for 1.5 h. The reaction mixture was quenched by saturated aq. NH₄Cl (10 mL), extracted with ethyl acetate (3 × 30 mL). The combined organic phase was washed with brine (30 mL), dried over anhydrous sodium sulfate. After filtration, the filtrate was concentrated under reduced pressure. The residue was purified RP-Combi-Flash with the following conditions: Column: C 18 gel column (40 g), 20-35 µm, 19 x 150 mm; Mobile Phase A: 5 mM aq. NH₄HCO₃; Mobile Phase B: MeCN; Gradient: 25% hold 3 min, up to 35% within 20 min; Flow rate: 40 mL/min; Detector: UV 254 & 210 nm; RT: 15 min to get 3,3-dimethyl-4-oxo-1'*H*-spiro[cyclohexane-1,3'-furo[3,4-*c*]pyridine]-5-carbonitrile (86 mg, 0.336 mmol, 88% yield) as a yellow solid. MS: *m*/*z* = 257.20 [M + H]⁺. ¹H NMR (400 MHz, Chloroform-*d*) δ 8.63 - 8.59 (m, 1H), 8.43 - 8.42 (m, 1H), 7.30 - 7.29 (m, 1H), 5.21 (s, 2H), 4.52 - 4.47 (m, 1H), 2.60 - 2.51 (m, 2H), 2.15 - 2.09 (m, 2H), 1.48 (s, 3H), 1.21 (s, 3H).

**Step 3:** To a mixture of phenyl hypochloroselenoite (74.7 mg, 0.390 mmol) in DCM (5 mL) was added pyridine (30.9 mg, 0.390 mmol) at 0 °C. The mixture was stirred at 0 °C for 20 min. Then to the mixture was added 3,3-dimethyl-4-oxo-1'*H*-spiro[cyclohexane-1,3'-furo[3,4-*c*]pyridine]-5-carbonitrile (50 mg, 0.195 mmol) at 0 °C and the mixture was stirred at 0 °C for 2 h. The reaction progress was monitored by LCMS and TLC. After completion the reaction mixture was diluted with water (20 mL), extracted with ethyl acetate (3 × 80 mL). The combined organic phase was washed with brine (30 mL), dried over anhydrous sodium sulfate. After filtration, the filtrate was concentrated under reduced pressure and the obtained residue was purified by *prep*-TLC (PE : EA = 1 : 1) to afford the crude product as a little yellow solid. The combined crude product (38 mg) was purified by achiral-SFC with the following conditions: Column: Triart Diol-NP, 20 x 250 mm, 5 um; Mobile Phase A: CO₂, Mobile Phase B: isopropanol; Flow rate:60 mL/min; Gradient:25% B; Detector: UV 254 nm; RT: 6.50 min. The product-containing fractions were collected and *roto*-evaporated *in vacuo* to give 5,5-dimethyl-4-oxo-1'*H*-spiro[cyclohexane-1,3'-furo[3,4-*c*]pyridin]-2-ene-3-carbonitrile (3.4 mg, 0.013 mmol, 8.95% yield) as a light yellow solid. MS: *m*/*z* = 255.20 [M + H]⁺.

Where necessary **Step 4:** 1'-(3,4-dimethylbenzyl)-5,5-dimethyl-2',4-dioxospiro[cyclohexane-1,3'-indolin]-2-ene-3-carbonitrile (150 mg, 0.390 mmol) was dissolved in TFA (3 mL, 0.390 mmol). Then the reaction solution was stirred at 90 °C for 3 h. The reaction progress was monitored by LCMS and TLC. After completion the reaction mixture was concentrated under vacuum and co-evaporated with toluene (three times, 3 mL for each time) to removed excessed TFA. The residue was purified by RP-Combi-Flash with the following conditions: Column: AQ 18 gel column (80 g), 20-35 µm; Mobile Phase A: 5 mM aq. NH₄HCO₃; Mobile Phase B: MeCN (Gradient: 0% B hold 5 min, up to 49.2% B within 31 min, 49.2% B hold 1.2 min; up to 95% B within 1 min, 95% B hold 5 min); Flow rate: 60 mL/min; Detector: UV 254 & 210 nm; RT: 31.3 min. The product-containing fractions were collected and *roto*-evaporated *in vacuo* to give 5,5-dimethyl-2',4-dioxospiro[cyclohexane-1,3'-indolin]-2-ene-3-carbonitrile (49.2 mg, 0.185 mmol, 47.4% yield) as a white solid. MS: *m*/*z* = 265.05 [M - H]⁻. ¹H-NMR (400 MHz, Chloroform-*d*) δ 7.89 (s, 1H), 7.38 - 7.30 (m, 1H), 7.24 - 7.12 (m, 2H), 7.09 (s, 1H), 7.02 - 6.87 (m, 1H), 2.49 - 2.45 (m, 1H), 2.30 - 2.26 (m, 1H), 1.62 (s, 3H), 1.36 (s, 3H).

**Table 8: The following examples were prepared using a similar procedure as described above for example 54.**

| **Ex** | **Structure** | **Compound Name** | **Exact Mass** |
|---|---|---|---|
| 55 | 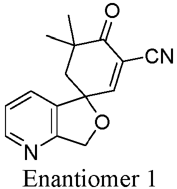 | 5,5-dimethyl-4-oxo-3'*H-*spiro[cyclohexane-1,1'-furo[3,4-*c*]pyridin]-2-ene-3-carbonitrile | 255 [M+H]⁺ |
| 56 | 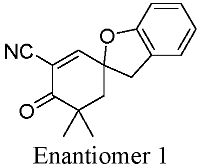 | 5',5'-dimethyl-4'-oxo-3*H-*spiro[benzofuran-2,1'-cyclohexan]-2'-ene-3'-carbonitrile | 252 [M-H] |
| 57 | 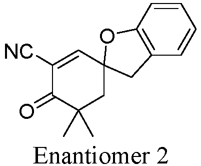 | 5',5'-dimethyl-4'-oxo-3*H-*spiro[benzofuran-2,1'-cyclohexan]-2'-ene-3'-carbonitrile | 252 [M-H] |
| 58 | 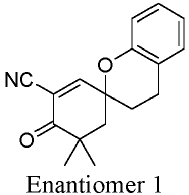 | 5',5'-dimethyl-4'-oxospiro[chromane-2,1'-cyclohexan]-2'-ene-3'-carbonitrile | 266 [M-H] |
| 59 | 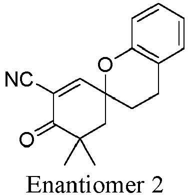 | 5',5'-dimethyl-4'-oxospiro[chromane-2,1'-cyclohexan]-2'-ene-3'-carbonitrile | 266 [M-H] |
| 60 | 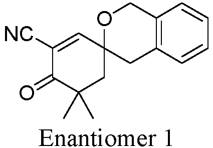 | 5,5-dimethyl-4-oxospiro[cyclohexane-1,3'-isochroman]-2-ene-3-carbonitrile | 266 [M-H] |
| 61 | 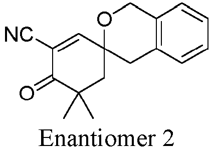 | 5,5-dimethyl-4-oxospiro[cyclohexane-1,3'-isochroman]-2-ene-3-carbonitrile | 266 [M-H] |
| 62 | 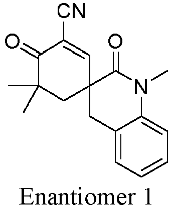 | 1',5,5-trimethyl-2',4-dioxo-1',4'-dihydro-2'H-spiro[cyclohexane-1,3'-quinolin]-2-ene-3-carbonitrile | 295 |
| 63 | 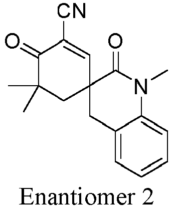 | 1',5,5-trimethyl-2',4-dioxo-1',4'-dihydro-2'H-spiro[cyclohexane-1,3'-quinolin]-2-ene-3-carbonitrile | 295 |

### Example 64, enantiomer 1: 3'-Methoxy-5,5-dimethyl-4-oxo-5'H-spiro[cyclohexane-1,7'-furo[3,4-b]pyridin]-2-ene-3-carbonitrile

### Example 65, enantiomer 2: 3'-Methoxy-5,5-dimethyl-4-oxo-5'H-spiro[cyclohexane-1,7'-furo[3,4-b]pyridin]-2-ene-3-carbonitrile

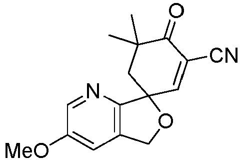

**Step 1:** Sodium hydride (60% in mineral oil, 1.83 g, 45.7 mmol) was added to a mixture of 6-bromo-5-methylpyridin-3-ol (4.3 g, 22.87 mmol) in dimethylformamide (60 mL) at 0 °C. The mixture was stirred at 0 °C for 1 h. Then iodomethane (2.15 mL, 34.3 mmol) was added dropwise. The resulting solution was stirred at ambient temperature for 2 h. The resulting solution was diluted with water (50 mL) and the aqueous layer was extracted with ethyl acetate (400 mL × 3). The combined organic layer was washed with brine (300 mL), dried over anhydrous sodium sulfate and filtered. The filtrate was concentrated under reduced pressure. The residue was purified by silica gel chromatography, eluting with a gradient of ethyl acetate : petroleum ether = 0 : 1 to 1 : 1. The fractions containing desired product were combined and concentrated under reduced pressure to afford 2-bromo-5-methoxy-3-methylpyridine (2.38 g, 11.78 mmol, 52% yield) as a light yellow solid. MS ESI calculated for C₇H₈BrNO [M + H]⁺ 201.98, 203.98, found 201.95, 203.95. ¹H NMR (300 MHz, Chloroform-*d*): δ 7.91 (d, *J* = 3.0 Hz, 1H), 7.09 (d, *J =* 2.9 Hz, 1H), 3.84 (s, 3H), 2.37 (s, 3H).

**Step 2:** To a stirred solution of 2-bromo-5-methoxy-3-methylpyridine (2.38 g, 11.78 mmol) in tetrachloromethane (15 mL) were added 1-bromopyrrolidine-2,5-dione (2.20 g, 12.37 mmol) and benzoyl peroxide (0.38 g, 1.18 mmol) at room temperature. The reaction mixture was stirred at 80 °C for 16 h. The resulting solution was diluted with water (100 mL) and the aqueous layer was extracted with ethyl acetate (200 mL × 3). The combined organic layer was washed with brine (200 mL), dried over anhydrous sodium sulfate and filtered. The filtrate was concentrated under reduced pressure. The residue was purified by silica gel chromatography, eluting with a gradient of ethyl acetate : petroleum ether = 0 : 1 to 1 : 4. The fractions containing desired product were combined and concentrated under reduced pressure to afford 2-bromo-3-(bromomethyl)-5-methoxypyridine (1.5 g, 5.34 mmol, 45% yield) as a light yellow solid. MS ESI calculated for C₇H₇Br₂NO [M + H]⁺ 279.89, 281.89, 283.89, found 279.85, 281.95, 283.95. ¹H NMR (400 MHz, Chloroform-*d*): δ 8.04 (d, *J =* 3.0 Hz, 1H), 7.33 (d, *J* = 3.0 Hz, 1H), 4.54 (s, 2H), 3.90 (s, 3H).

**Step 3:** To a stirred solution of 2-bromo-3-(bromomethyl)-5-methoxypyridine (1.49 g, 5.30 mmol) in 1,4-dioxane (25 mL) was added *N*-methylmorpholine *N*-oxide (2.18 g, 18.56 mmol) at room temperature. The reaction mixture was stirred at 80 °C for 30 min. The resulting solution was diluted with water (100 mL) and the aqueous layer was extracted with ethyl acetate (150 mL × 3). The combined organic layer was washed with brine (200 mL), dried over anhydrous sodium sulfate and filtered. The filtrate was concentrated under reduced pressure. The residue was purified by silica gel chromatography, eluting with a gradient of ethyl acetate : petroleum ether = 0 : 1 to 1 : 1. The fractions containing desired product were combined and concentrated under reduced pressure to afford 2-bromo-5-methoxynicotinaldehyde (1 g, 4.40 mmol, 83% yield) as a light yellow solid. MS ESI calculated for C₇H₆BrNO₂ [M + H]⁺ 215.96, 217.96, found 216.05, 218.05. ¹H NMR (400 MHz, Chloroform-*d*): δ 10.33 (d, *J* = 0.6 Hz, 1H), 8.32 (d, *J* = 3.0 Hz, 1H), 7.68 (d, *J* = 3.2 Hz, 1H), 3.93 (s, 3H).

**Step 4:** Sodium borohydride (0.35 g, 9.26 mmol) was added to a mixture of 2-bromo-5-methoxynicotinaldehyde (1.0 g, 4.63 mmol) in methanol (25 mL) at 0 °C. The mixture was stirred at ambient temperature for 2 h. The resulting solution was diluted with saturated aqueous ammonium chloride (100 mL) and the aqueous layer was extracted with ethyl acetate (100 mL × 3). The combined organic layer was washed with brine (150 mL), dried over anhydrous sodium sulfate and filtered. The residue was purified by silica gel chromatography, eluting with a gradient of ethyl acetate : petroleum ether = 0 : 1 to 1 : 1. The fractions containing desired product were combined and concentrated under reduced pressure to afford (2-bromo-5-methoxypyridin-3-yl)methanol (850 mg, 3.86 mmol, 83% yield) as a light yellow solid. MS ESI calculated for C₇H₈BrNO₂ [M + H]⁺ 217.97, 219.97, found 218.05, 220.05. ¹H NMR (300 MHz, Chloroform-*d*): δ 7.99 (d, *J =* 3.1 Hz, 1H), 7.43 (d, *J =* 3.0 Hz, 1H), 4.72 (s, 2H), 3.88 (s, 3H).

**Step 5:** *n*-Butyllithium (2.5 M in n-hexane, 4.22 mL, 10.55 mmol) was added dropwise to a solution of (2-bromo-5-methoxypyridin-3-yl)methanol (1.15 g, 5.27 mmol) in tetrahydrofuran (8.0 mL) at -78 °C. Then the resulting solution was stirred at -78 °C for 0.5 h under nitrogen atmosphere. 6,6-Dimethyl-1,4-dioxaspiro[4.5]decan-8-one (1.17 g, 6.33 mmol) was added into mixture at -78 °C and the resulting solution was stirred at ambient temperature for 2 h. The reaction mixture was quenched by saturated ammonium chloride solution (100 mL) and extracted with ethyl acetate (200 mL × 3). The combined organic layers was washed with brine (200 mL), dried over anhydrous sodium sulfate and filtered. The filtrate was concentrated under reduced pressure and the residue was purified by reverse phase chromatography with the following conditions: Column, C¹⁸, 120 g, 19 × 150 mm; Mobile phase: acetonitrile in water (5 mM aqueous formic acid), 10 % - 60 % in 20 min; Detector, UV 210 nm. RT: 13 min. The collected fractions were combined and concentrated under reduced pressure to afford 8-(3-(hydroxymethyl)-5-methoxypyridin-2-yl)-6,6-dimethyl-1,4-dioxaspiro[4.5]decan-8-ol (700 mg, 2.17 mmol, 41% yield) as a light yellow oil. MS ESI calculated for C₁₇H₂₅NO₅ [M + H]⁺ 324.17, found 324.20. ¹H NMR (300 MHz, Chloroform-d): 8.17 (d, *J* = 3.0 Hz, 1H), 7.52 (d, *J* = 2.7 Hz, 1H), 5.04-4.87 (m, 2H), 4.07-4.02 (m, 4H), 3.91 (s, 3H), 2.53-2.15 (m, 3H), 1.81-1.49 (m, 3H), 1.37 (s, 3H), 0.90 (s, 3H).

**Step 6:** To a solution of 8-(3-(hydroxymethyl)-5-methoxypyridin-2-yl)-6,6-dimethyl-1,4-dioxaspiro[4.5]decan-8-ol (600 mg, 1.86 mmol) in dichloromethane (6.0 mL) were added*p-*toluenesulfonyl chloride (531 mg, 2.78 mmol), triethylamine (0.78 mL, 5.57 mmol) and 4-dimethylaminopyridine (22.67 mg, 0.19 mmol) at ambient temperature. The reaction mixture was stirred at ambient temperature for 16 h. The reaction mixture was quenched by water (50 mL) and extracted with ethyl acetate (100 mL × 3). The combined organic layers was washed with brine (100 mL), dried over anhydrous sodium sulfate and filtered. The filtrate was concentrated under reduced pressure and the residue was purified by reverse phase chromatography with the following conditions: Column, C¹⁸, 120 g, 19 × 150 mm; Mobile phase: acetonitrile in water (5 mM aqueous formic acid), 20% - 70% in 30 min; Detector, UV 254 nm. RT: 22 min. The collected fractions were combined and concentrated under reduced pressure to afford 3-methoxy-2',2'-dimethyl-5*H*-dispiro[furo[3,4-*b*]pyridine-7,4'-cyclohexane-1',2"-[1,3]dioxolane] (300 mg, 0.98 mmol, 53% yield) as a light yellow oil. MS ESI calculated for C₁₇H₂₃NO₄ [M + H]⁺ 306.16, found 306.20. ¹H NMR (300 MHz, Chloroform-*d*): δ 8.19-8.12 (m, 1H), 7.07 (d, *J =* 2.4 Hz, 1H), 5.02 (t, *J =* 0.9 Hz, 2H), 4.05-3.95 (m, 4H), 3.87 (s, 3H), 2.28-2.08 (m, 3H), 1.79-1.48 (m, 3H), 1.31 (s, 3H), 0.92 (s, 3H).

**Step 7**: To a solution of 3-methoxy-2',2'-dimethyl-5*H*-dispiro[furo[3,4-*b*]pyridine-7,4'-cyclohexane-1',2"-[1,3]dioxolane] (300 mg, 0.98 mmol) in tetrahydrofuran (2 mL) was added dropwise HCl (1.5 M in H₂O, 2.0 mL, 4.00 mmol) at 0 °C under argon atmosphere. The resulting mixture was warmed to ambient temperature and stirred for 3 h. The reaction mixture was quenched by water (20 mL) and the pH value of the solution was adjusted to 6 ~ 7 with saturated aqueous sodium bicarbonate. The reaction mixture was extracted with ethyl acetate (50 mL × 3). The combined organic layers was washed with brine (50 mL), dried over anhydrous sodium sulfate and filtered. The filtrate was concentrated under reduced pressure and the residue was purified by silica gel chromatography, eluting with a gradient of ethyl acetate : petroleum ether - 1 : 9 to 3 : 2 to afford 3'-methoxy-3,3-dimethyl-5'*H*-spiro[cyclohexane-1,7'-furo[3,4-*b*]pyridin]-4-one (200 mg, 0.77 mmol, 78% yield) as a light yellow oil. MS ESI calculated for C₁₅H₁₉NO₃ [M + H]⁺ 262.14, found 262.10. ¹H NMR (300 MHz, Chloroform-*d*): δ 8.17 (s, 1H), 7.13 (d, *J* = 2.4 Hz, 1H), 5.12 (d, *J* = 1.6 Hz, 2H), 3.89 (s, 3H), 3.18 - 3.06 (m, 1H), 2.40-2.25 (m, 3H), 2.15-2.01 (m, 1H), 1.92 (dd, *J =* 14.5, 3.5 Hz, 1H), 1.43 (s, 3H), 1.12 (s, 3H).

**Step 8:** Lithium bis(trimethylsilyl)amide (1 M in THF, 2.14 mL, 2.14 mmol) was added to a solution of 3'-methoxy-3,3-dimethyl-5'*H*-spiro[cyclohexane-1,7'-furo[3,4-*b*]pyridin]-4-one (280 mg, 1.071 mmol) in tetrahydrofuran (3.0 mL) (degassed with nitrogen for 3 times) at -78 °C under the atmosphere of nitrogen. The mixture was stirred at -78 °C for 45 min. Then a solution of 4-methylbenzenesulfonyl cyanide (291 mg, 1.607 mmol) in tetrahydrofuran (1.0 mL) (nitrogen atmosphere) was added at -78 °C. The mixture was stirred at -78 °C for 0.5 h. Then the reaction mixture was stirred at ambient temperature for 16 h. The mixture was quenched by saturated aqueous NH₄Cl (30 mL), diluted with ethyl acetate (50 mL × 2), washed with brine (50 mL), dried over anhydrous sodium sulfate and filtered. The filtrate was concentrated under reduced pressure. The residue was purified by Prep-TLC with ethyl acetate : petroleum ether = 1 : 2 and washed with ethyl acetate to afford 3'-methoxy-3,3-dimethyl-4-oxo-5'*H-*spiro[cyclohexane-1,7'-furo[3,4-*b*]pyridine]-5-carbonitrile (100 mg, 0.35 mmol, 33% yield) as a light yellow solid. MS ESI calculated for C₁₆H₁₈N₂O₃ [M + H]⁺ 287.13, found 287.10. ¹H NMR (300 MHz, Chloroform-*d*): δ 8.16-8.15 (m, 1H), 7.12-7.11 (m, 1H), 5.13 (s, 2H), 4.43 (dd, *J* = 13.8, 5.1 Hz, 1H), 3.87 (s, 3H), 2.62-2.55 (m, 1H), 2.45-2.34 (m, 1H), 2.31 (d, *J =* 14.7 Hz, 1H), 1.92 (dd, *J* = 14.7, 3.7 Hz, 1H), 1.46 (s, 3H), 1.18 (s, 3H).

**Step 9:** A solution of pyridine (55.3 mg, 0.70 mmol) in dichloromethane (0.5 mL) was added dropwise to phenyl hypochloroselenoite (134 mg, 0.70 mmol) in dichloromethane (0.5 mL) at 0 °C. The mixture was stirred at 0 °C for 20 min and then treated dropwise with a solution of 3'-methoxy-3,3-dimethyl-4-oxo-5'*H*-spiro[cyclohexane-1,7'-furo[3,4-*b*]pyridine]-5-carbonitrile (100 mg, 0.35 mmol) in dichloromethane (1.5 mL). The resulting mixture was stirred at 0 °C for 3 h. The mixture was quenched by water (30 mL), diluted with dichloromethane (50 mL × 2), washed with brine (50 mL), dried over anhydrous sodium sulfate and filtered. The filtrate was concentrated under reduced pressure. The residue was purified by Prep-TLC with ethyl acetate : petroleum ether = 1 : 2 and washed with ethyl acetate to afford 3'-methoxy-5,5-dimethyl-4-oxo-5'*H*-spiro[cyclohexane-1,7'-furo[3,4-*b*]pyridin]-2-ene-3-carbonitrile (80 mg, 0.28 mmol, 61% yield) as a light yellow oil. MS ESI calculated for C₁₆H₁₆N₂O₃ [M + H]⁺ 285.12, found 285.10. **Step 10:** 3'-Methoxy-5,5-dimethyl-4-oxo-5'*H*-spiro[cyclohexane-1,7'-furo[3,4-*b*]pyridin]-2-ene-3-carbonitrile (80 mg, 0.28 mmol) was purified by Prep-Chiral-HPLC with the following conditions: CHIRALPAK IA - 3, 4.6 × 50 mm (3 um); Mobile phase : Hexanes : *iso*-propanol = 80 : 20; Detector, UV 254 nm. 3'-Methoxy-5,5-dimethyl-4-oxo-5'*H*-spiro[cyclohexane-1,7'-furo[3,4-*b*]pyridin]-2-ene-3-carbonitrile (26.0 mg, 0.091 mmol, 33% yield) was obtained at 1.78 min as a light yellow solid; MS ESI calculated for C₁₆H₁₆N₂O₃ [M + H]⁺ 285.12, found 285.05. ¹H NMR (300 MHz, Chloroform-*d*): δ 8.22 (d, *J* = 2.4 Hz, 1H), 7.19 (d, *J =* 2.0 Hz, 1H), 7.16 (d, *J =* 2.6 Hz, 1H), 5.22 (d, *J =* 13.0 Hz, 1H), 5.16 (d, *J =* 13.1 Hz, 1H), 3.90 (s, 3H), 2.60 (d, *J =* 15.0 Hz, 1H), 2.10 (dd, *J =* 14.9, 2.1 Hz, 1H), 1.43 (s, 3H), 1.27 (s, 3H). 3'-Methoxy-5,5-dimethyl-4-oxo-5'*H*-spiro[cyclohexane-1,7'-furo[3,4-*b*]pyridin]-2-ene-3-carbonitrile (27.1 mg, 0.095 mmol, 34% yield) was obtained at 3.16 min as a white solid. MS ESI calculated for C₁₆H₁₆N₂O₃ [M + H]⁺ 285.12, found 285.05. ¹H NMR (300 MHz, Chloroform-*d*): δ 8.23 (d, *J* = 2.4 Hz, 1H), 7.19 (d, *J =* 2.0 Hz, 1H), 7.17 (d, *J =* 2.6 Hz, 1H), 5.22 (d, *J* = 13.2 Hz, 1H), 5.16 (d, *J =* 13.1 Hz, 1H), 3.92 (s, 3H), 2.61 (d, *J =* 15.0 Hz, 1H), 2.10 (dd, *J* = 14.9, 2.1 Hz, 1H), 1.46 (s, 3H), 1.27 (s, 3H).

**Table 8: The following examples were prepared using a similar procedure as described above for example 65, starting from Step 5 when the requisite aldehyde is available.**

| **Ex** | **Structure** | **Compound Name** | **Exact Mass [M+H]⁺** |
|---|---|---|---|
| 66 | 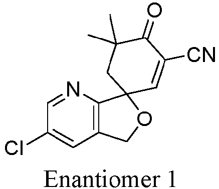 | 3'-chloro-5,5-dimethyl-4-oxo-5'*H-*spiro[cyclohexane-1,7'-furo[3,4-*b*]pyridin]-2-ene-3-carbonitrile | 289, 291 |
| 67 | 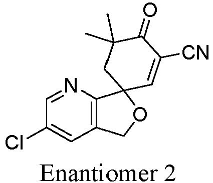 | 3'-chloro-5,5-dimethyl-4-oxo-5'*H-*spiro[cyclohexane-1,7'-furo[3,4-*b*]pyridin]-2-ene-3-carbonitrile | 289, 291 |
| 68 | 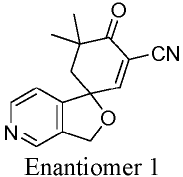 | 5,5-Dimethyl-4-oxo-3'*H-*spiro[cyclohexane-1,1'-furo[3,4-*c*]pyridin]-2-ene-3-carbonitrile | 255 |
| 69 | 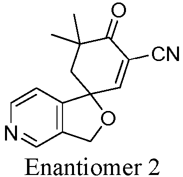 | 5,5-Dimethyl-4-oxo-3'*H-*spiro[cyclohexane-1,1'-furo[3,4-*c*]pyridin]-2-ene-3-carbonitrile | 255 |
| 70 | 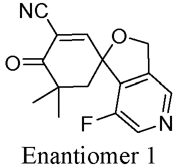 | 7'-fluoro-5,5-dimethyl-4-oxo-3'*H-*spiro[cyclohexane-1,1'-furo[3,4-*c*]pyridin]-2-ene-3-carbonitrile | 273 |
| 71 | 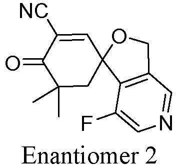 | 7'-fluoro-5,5-dimethyl-4-oxo-3'*H-*spiro[cyclohexane-1,1'-furo[3,4-*c*]pyridin]-2-ene-3-carbonitrile | 273 |
| 72 | 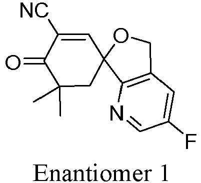 | 3'-fluoro-5,5-dimethyl-4-oxo-5'*H-*spiro[cyclohexane-1,7'-furo[3,4-*b*]pyridin]-2-ene-3-carbonitrile | 273 |
| 73 | 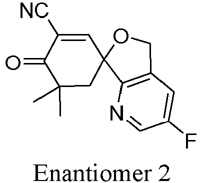 | 3'-fluoro-5,5-dimethyl-4-oxo-5'*H-*spiro[cyclohexane-1,7'-furo[3,4-*b*]pyridin]-2-ene-3-carbonitrile | 273 |
| 74 | 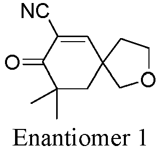 | 1',5,5-trimethyl-4-oxospiro[cyclohexane-1,3'-indolin]-2-ene-3-carbonitrile | 206 |
| 75 | 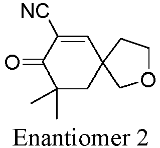 | 1',5,5-trimethyl-4-oxospiro[cyclohexane-1,3'-indolin]-2-ene-3-carbonitrile | 206 |

### Example 76, Isomer 1: 9,9-dimethyl-8-oxo-3-(pyrazin-2-yl)-1-oxaspiro[4.5]dec-6-ene-7-carbonitrile 2,2,2-trifluoroacetic acid

### Example 77, Isomer 2: 9,9-dimethyl-8-oxo-3-(pyrazin-2-yl)-1-oxaspiro[4.5]dec-6-ene-7-carbonitrile 2,2,2-trifluoroacetic acid

### Example 78, Isomer 3: 9,9-dimethyl-8-oxo-3-(pyrazin-2-yl)-1-oxaspiro[4.5]dec-6-ene-7-carbonitrile 2,2,2-trifluoroacetic acid

### Example 79, Isomer 4: 9,9-dimethyl-8-oxo-3-(pyrazin-2-yl)-1-oxaspiro[4.5]dec-6-ene-7-carbonitrile 2,2,2-trifluoroacetic acid

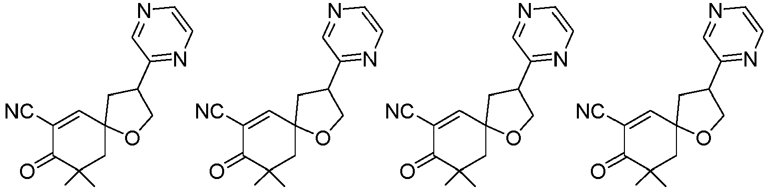

**Step 1:** To a solution of 2-(6,6-dimethyl-1,4,9-trioxadispiro[4.2.4⁸.2⁵]tetradec-11-en-11-yl)pyrazine (330 mg, 1.091 mmol) in MeOH (6 mL) was added Pd / C (99mg, 0.930 mmol) at room temperature. Replaced the system three times with hydrogen and stirred for 2 h at room temperature under hydrogen atmosphere. The reaction progress was monitored by LCMS and TLC. After completion of the reaction, the mixture was filtered and the solvent was removed under reduced pressure to afford 2-(6,6-dimethyl-1,4,9-trioxadispiro[4.2.4⁸.2⁵]tetradecan-11-yl)pyrazine (330 mg, 1.084 mmol, 99% yield) as a light yellow oil. MS ESI calculated for C₁₇H₂₄N₂O₃ [M + H]⁺ 305.19 found 305.15.

**Alternative Step 1:** To a solution of 6,6-dimethyl-1,4,9-trioxadispiro[4.2.4⁸.2⁵]tetradecan-12-one (220 mg, 0.916 mmol) in THF (0.5 mL) was added methylmagnesium bromide (1 M in THF, 1.831 mL, 1.831 mmol) at 0 °C. The resulted mixture was stirred for 16 hours at 60 °C. The reaction progress was monitored by LCMS and TLC. After completion of the reaction, the reaction mixture was quenched by NH₄Cl aq. (1 mL), and diluted with ethyl acetate (40 mL), then washed with brine (10 mL × 3), dried over anhydrous sodium sulfate and filtered. The filtrate was concentrated in vacuo. The residue was purified by silica gel column chromatography using 10% - 40% gradient of ethyl acetate in petroleum ether as eluent. The fractions containing desired product were combined and concentrated under reduced pressure to afford 6,6,12-trimethyl-1,4,9-trioxadispiro[4.2.4⁸.2⁵]tetradecan-12-ol (80 mg, 0.312 mmol, 34.1% yield). MS ESI calculated for C₁₄H₂₄O₄ [M + H]⁺ 257.17 found 257.20. ¹H-NMR (400 MHz, Chloroform-*d*) δ 4.07-3.69 (m, 6H), 2.14-1.88 (m, 3H), 1.72-1.38 (m, 5H), 1.28 (s, 3H), 1.22 (s, 3H), 0.91 (s, 3H) and 6,6,12-trimethyl-1,4,9-trioxadispiro[4.2.4⁸.2⁵]tetradecan-12-ol (80 mg, 0.312 mmol, 34.1% yield) as a yellow oil. MS ESI calculated for C₁₄H₂₄O₄ [M + H]⁺ 257.17 found 257.20. ¹H-NMR (400 MHz, Chloroform-*d*) δ 4.07-3.69 (m, 6H), 2.14-1.88 (m, 3H), 1.72-1.38 (m, 5H), 1.27 (s, 3H), 1.25 (s, 3H), 0.91 (s, 3H).

Alternatively, isomers can be separated at Step 8.

**Alternative Step 1:** To a solution of sodium borohydride (47.2 mg, 1.248 mmol) in MeOH (3 mL) was added solution of 6,6-dimethyl-1,4,9-trioxadispiro[4.2.4⁸.2⁵]tetradecan-11-one (200 mg, 0.832 mmol) in MeOH (3 mL) and the mixture was stirred for 10 min prtected with nitrogen at 0 °C. Then the mixture was stirred for 2 h at room temperature. The reaction mixture was quenched by saturated aqueous NH₄Cl (2 mL) and extracted with ethyl acetate (5 mL × 3). The combined organic layers was washed with brine (4 mL), dried over anhydrous sodium sulfate and filtered. The filtrate was concentrated under reduced pressure to give crude 6,6-dimethyl-1,4,9-trioxadispiro[4.2.4⁸.2⁵]tetradecan-11-ol (195 mg, 0.805 mmol, 97% yield) as a yellow oil. MS ESI calculated for C₁₃H₂₂O₄ [M + H]⁺ 243.15 found 243.25. ¹H NMR (300 MHz, Chloroform-*d*) δ 4.82-4.39 (m, 1H), 4.04-3.67 (m, 6H), 2.03-1.50 (m, 8H), 1.23-1.10 (m, 3H), 0.97-0.77 (m, 3H).

Step 1 and Alternative step 1, may also combined and performed in a singe step.

**Step 2:** To a solution of 2-(6,6-dimethyl-1,4,9-trioxadispiro[4.2.4⁸.2⁵]tetradecan-11-yl)pyrazine (330 mg, 1.084 mmol) in THF (4.5mL) were added 1 N aqueous HCl (4.34 mL, 4.34 mmol) at room temperature. The resulted mixture was stirred for 6 hours at 40 °C. The reaction progress was monitored by LCMS and TLC. After completion of the reaction, the reaction mixture quenched with *sat.* NaHCO₃ (30 mL), and extracted with ethyl acetate (50 mL × 3). The organic layer was washed with brine (60 mL × 3), dried over anhydrous sodium sulfate and filtered. The filtrate was concentrated in vacuo. The residue was purified by silica gel column chromatography using 10% - 60% gradient of ethyl acetate in petroleum ether as eluent. The fractions containing desired product were combined and concentrated under reduced pressure to afford 7,7-dimethyl-3-(pyrazin-2-yl)-1-oxaspiro[4.5]decan-8-one (250 mg, 0.960 mmol, 89% yield) as a light yellow oil. MS ESI calculated for C₁₅H₂₀N₂O₂ [M + H]⁺ 261.16 found 261.10.

**Step 3:** To a solution of 7,7-dimethyl-3-(pyrazin-2-yl)-1-oxaspiro[4.5]decan-8-one (250 mg, 0.960 mmol) was added sodium methylate (657 mg, 3.65 mmol) and ethyl formate (285 mg, 3.84 mmol) at room temperature. The resulted mixture was stirred for 3 h at 30 °C. The reaction progress was monitored by LCMS. After completion of the reaction, the reaction mixture was added with 5% aqueous HCl solution (50 mL), and extracted with ethyl acetate (50 mL × 3). The organic layer was washed with brine (60 mL × 3), dried over anhydrous sodium sulfate and filtered. The filtrate was concentrated in vacuo to afford (*Z*)-9-(hydroxymethylene)-7,7-dimethyl-3-(pyrazin-2-yl)-1-oxaspiro[4.5]decan-8-one (250 mg, 0.867 mmol, 90% yield) as a light yellow solid that was used without further purification. MS ESI calculated for C₁₆H₂₀N₂O₃ [M + H]⁺ 289.16 found 289.10.

**Step 4:** To a solution of (*Z*)-9-(hydroxymethylene)-7,7-dimethyl-3-(pyrazin-2-yl)-1-oxaspiro[4.5]decan-8-one (250 mg, 0.867 mmol) in EtOH (2mL) was added a mixture of hydroxylamine hydrochloride (602 mg, 8.67 mmol) in water (1 mL) at room temperature. Then the mixture stirred for 3 h at 85 °C, the reaction progress was monitored by LCMS. After completion of the reaction, the reaction mixture quenched with water (30 mL), and extracted with ethyl acetate (40 mL × 3). The organic layer was washed with brine (50 mL × 3), dried over anhydrous sodium sulfate and filtered. The filtrate was concentrated in vacuo. The residue was purified by silica gel column chromatography using 10% - 60% gradient of ethyl acetate in petroleum ether as eluent. The fractions containing desired product were combined and concentrated under reduced pressure to afford 7,7-dimethyl-4'-(pyrazin-2-yl)-4',5',6,7-tetrahydro-3'*H*,4*H*-spiro[benzo[*d*]isoxazole-5,2'-furan] (60 mg, 0.200 mmol, 23.04% yield) as a light yellow oil and 7,7-dimethyl-4'-(pyrazin-2-yl)-4',5',6,7-tetrahydro-3'*H*,4*H*-spiro[benzo[*d*]isoxazole-5,2'-furan] (90 mg, 0.300 mmol, 34.6% yield) as a light yellow oil. MS ESI calculated for C₁₆H₁₉N₃O₂ [M + H]⁺ 286.16 found 286.10. ¹H NMR (300 MHz, Chloroform-*d*) δ 8.62-8.56 (m, 1H), 8.54 (d, *J* = 1.6 Hz, 1H), 8.48 (d, *J* = 2.6 Hz, 1H), 8.06 (s, 1H), 4.31 (t, *J* = 8.2 Hz, 1H), 4.02 (t, *J =* 8.5 Hz, 1H), 3.88-3.72 (m, 1H), 2.93-2.67 (m, 2H), 2.40-2.27 (m, 2H), 2.09 (dd, *J =* 14.0, 1.8 Hz, 1H), 1.82 (d, *J =* 14.0 Hz, 1H), 1.50 (s, 3H), 1.40 (s, 3H). MS ESI calculated for C₁₆H₁₉N₃O₂ [M + H]⁺ 286.16 found 286.10. ¹H NMR (300 MHz, Chloroform-*d*) δ 8.61-8.57 (m, 1H), 8.54 (d, *J* = 1.5 Hz, 1H), 8.49 (d, *J =* 2.6 Hz, 1H), 8.06 (s, 1H), 4.31 (t, *J =* 8.2 Hz, 1H), 4.05 (t, *J* = 8.7 Hz, 1H), 3.86 - 3.75 (m, 1H), 2.82 - 2.62 (m, 2H), 2.32 (t, *J =* 9.2 Hz, 2H), 2.20 (d, *J* = 1.3 Hz, 1H), 1.92 (d, *J* = 14.0 Hz, 1H), 1.48 (s, 3H), 1.39 (s, 3H).

Alternatively, isomers of subsequent examples may be separated in **Step 7.**

**Step 5Q:** To a solution of 7,7-dimethyl-4'-(pyrazin-2-yl)-4',5',6,7-tetrahydro-3'*H*,4*H-*spiro[benzo[*d*]isoxazole-5,2'-furan] (60 mg, 0.210 mmol) in Et₂O (2 mL) and MeOH (1 mL) was added sodium methylate (30% in methanol, 1325 mg, 7.36 mmol) at room temperature. Then the mixture stirred for 2 h at room temperature. The reaction was quenched with 1 N aqueous HCl (20 mL), and extracted with ethyl acetate (30 mL × 3). The organic layer was washed with brine (30 mL × 3), dried over anhydrous sodium sulfate and filtered. The filtrate was concentrated in vacuo to afford 9,9-dimethyl-8-oxo-3-(pyrazin-2-yl)-1-oxaspiro[4.5]decane-7-carbonitrile (PH-EBR2320-270-5Q) (33 mg, 0.116 mmol, 55.0 % yield) as a light yellow oil. MS ESI calculated for C₁₆H₁₉N₃O₂ [M + H]⁺ 286.16 found 286.10.

**Step 5H:** To a solution of 7,7-dimethyl-4'-(pyrazin-2-yl)-4',5',6,7-tetrahydro-3'*H*,4*H-*spiro[benzo[*d*]isoxazole-5,2'-furan] (90 mg, 0.315 mmol) in Et₂O (1 mL) and MeOH (0.5 mL) was added sodium methylate (30% in methanol, 1988 mg, 11.04 mmol) at room temperature. Then the mixture stirred for 2 h at room temperature. The reaction was quenched with 1 N aqueous HCl (30 mL), and extracted with ethyl acetate (40 mL × 3). The organic layer was washed with brine (50 mL × 3), dried over anhydrous sodium sulfate and filtered. The filtrate was concentrated in vacuo to afford 9,9-dimethyl-8-oxo-3-(pyrazin-2-yl)-1-oxaspiro[4.5]decane-7-carbonitrile (70 mg, 0.245 mmol, 78% yield) as a light yellow oil. MS ESI calculated for C₁₆H₁₉N₃O₂ [M + H]⁺ 286.16 found 286.10.

**Step 6Q:** Phenylselenenyl chloride (44.3 mg, 0.231 mmol) was added dropwise to a solution of pyridine (0.019 mL, 0.231 mmol) in DCM (0.3 mL) at 0 °C. The resulting solution was stirring at 0 °C for 30 min under nitrogen atmosphere. A solution of 9,9-dimethyl-8-oxo-3-(pyrazin-2-yl)-1-oxaspiro[4.5]decane-7-carbonitrile (33 mg, 0.116 mmol) in DCM (0.2 mL) was added into the above mixture and the whole was stirred at 0 °C under nitrogen atmosphere for 16 h. The reaction progress was monitored by LCMS and TLC. After completion of reaction, the result solution was concentrated under reduced pressure. The residue was purified by *RP*-Flash (Column: C18 20g Column; Mobile Phase A: water (0.1% TFA), Mobile Phase B: MeCN; Flow rate: 35 mL / min; Gradient: 2% B to 30% B in 30 min, Detector: UV 210 nm; RT = 28 min). The fractions containing desired product were combined and concentrated to afford 9,9-dimethyl-8-oxo-3-(pyrazin-2-yl)-1-oxaspiro[4.5]dec-6-ene-7-carbonitrile 2,2,2-trifluoroacetic acid (PH-EBR2320-270-0Q) (30 mg, 0.075 mmol, 65.3 % yield) as a light yellow oil. MS ESI calculated for C₁₆H₁₇N₃O₂ [M + H]⁺ 284.13 found 284.10. ¹H NMR (300 MHz, Chloroform-d) δ 8.74 (s, 1H), 8.64-8.50 (m, 2H), 7.73 (s, 1H), 4.47-4.30 (m, 1H), 4.05 (t, *J =* 8.7 Hz, 1H), 3.87 (t, *J* = 8.0 Hz, 1H), 2.55 (d, *J =* 8.1 Hz, 2H), 2.33-2.06 (m, 2H), 1.32 (s, 3H), 1.24 (s, 3H). ¹⁹F NMR (282 MHz, Chloroform - *d*) δ -75.64 (s, 3F).

**Step 6H:** Phenylselenenyl chloride (94 mg, 0.491 mmol) was added dropwise to a solution of pyridine (0.040 mL, 0.491 mmol) in DCM (0.6 mL) at 0 °C. The resulting solution was stirring at 0 °C for 30 min under nitrogen atmosphere. A solution of 9,9-dimethyl-8-oxo-3-(pyrazin-2-yl)-1-oxaspiro[4.5]decane-7-carbonitrile (70 mg, 0.245 mmol) in DCM (0.40 mL) was added into the above mixture and the whole was stirred at 0 °C under nitrogen atmosphere for 16 h. The reaction progress was monitored by LCMS. After completion of reaction, the result solution was concentrated under reduced pressure. The residue was purified by *RP*-Flash (Column: C18 20g Column; Mobile Phase A: water (0.1% TFA), Mobile Phase B: MeCN; Flow rate: 35 mL / min; Gradient: 2% B to 30% B in 30 min, Detector: UV 210 nm; RT = 28 min). The fractions containing desired product were combined and concentrated to afford 9,9-dimethyl-8-oxo-3-(pyrazin-2-yl)-1-oxaspiro[4.5]dec-6-ene-7-carbonitrile 2,2,2-trifluoroacetic acid (PH-EBR2320-270-0H) (60 mg, 0.151 mmol, 61.6 % yield) as a light yellow oil. MS ESI calculated for C₁₆H₁₇N₃O₂ [M + H]⁺ 284.13 found 284.10. ¹H NMR (300 MHz, Chloroform-*d*) δ 8.85 (s, 1H), 8.70-8.58 (m, 2H), 7.42 (s, 1H), 4.42 (t, *J =* 8.0 Hz, 1H), 4.12 (t, *J =* 8.6 Hz, 1H), 3.93 (t, *J =* 8.3 Hz, 1H), 2.66-2.47 (m, 2H), 2.39-2.17 (m, 2H), 1.34 (s, 3H), 1.24 (s, 3H). ¹⁹F NMR (282 MHz, Chloroform - *d*) δ -75.59 (s, 3F).

**Alternative Step 6:** To a solution of 3-hydroxy-9,9-dimethyl-8-oxo-3-(pyridin-3-yl)-1-oxaspiro[4.5]decane-7-carbonitrile (50 mg, 0.166 mmol) in toluene (0.5 mL) and was added DDQ (76 mg, 0.333 mmol) at room temperature. The resulted mixture was stirred for 2 hours at 115 °C. After completion of the reaction, the reaction mixture was concentrated in vacuo, the residue was purified by *Prep*-TLC with DCM / IPA (10:1) and washed with DCM / IPA (10:1) to afford 3-hydroxy-9,9-dimethyl-8-oxo-3-(pyridin-3-yl)-1-oxaspiro[4.5]dec-6-ene-7-carbonitrile (15 mg, 0.050 mmol, 30.2 % yield) as a yellow solid. MS ESI calculated for C₁₇H₁₈N₂O₃ [M + H]⁺ 299.14 found 299.15. ¹H NMR (300 MHz, Chloroform - *d*) δ 8.72 (s, 1H), 8.55 (s, 1H), 7.98 (s, 1H), 7.87 (d, *J* = 8.1 Hz, 1H), 7.41 (s, 1H), 4.24 (s, 2H), 2.67 (d, *J* = 13.8 Hz, 1H), 2.42 (d, *J* = 13.8 Hz, 1H), 2.30 (d, *J* = 14.4 Hz, 1H), 2.10 (d, *J* = 14.4 Hz, 1H), 1.31 (s, 3H), 1.20 (s, 3H).

**Step 7Q:** PH-EBR2320-270-0Q was separated by *Prep*-Chiral-HPLC with the following conditions: Column: CHIRALPAK IG, 2 × 25 cm, 5 µm; Mobile Phase A: MtBE--HPLC, Mobile Phase B: EtOH--HPLC; Flow rate: 20 mL / min; Gradient: 5% B to 5% B in 14 min; Wave Length: 254 / 220 nm; RT1(min): 5.74; RT2(min): 11.79. The first product containing (RT1: 5.74 min) peak was combined and concentrated under reduced pressure, then freeze-dried to give 9,9-dimethyl-8-oxo-3-(pyrazin-2-yl)-1-oxaspiro[4.5]dec-6-ene-7-carbonitrile 2,2,2-trifluoroacetic acid (PH-EBR2320-270-0QQ) (5.8 mg, 0.014 mmol, 17.98 % yield) as a light yellow solid. MS ESI calculated for C₁₆H₁₇N₃O₂ [M + H]⁺ 284.14 found 284.15. ¹H NMR (300 MHz, Chloroform-*d*) δ 8.60 (s, 1H), 8.51 (s, 2H), 7.73 (s, 1H), 4.37 (dd, *J* = 9.0, 7.4 Hz, 1H), 4.02 (t, *J* = 8.6 Hz, 1H), 3.88-3.75 (m, 1H), 2.51 (dd, *J =* 8.2, 3.0 Hz, 2H), 2.30-2.05 (m, 2H), 1.29 (s, 3H), 1.22 (s, 3H). ¹⁹F NMR (282 MHz, Chloroform-*d*) δ -75.83 (s, 3F). The second product containing (RT2: 11.79 min) peak was combined and concentrated under reduced pressure, then freeze-dried to give 9,9-dimethyl-8-oxo-3-(pyrazin-2-yl)-1-oxaspiro[4.5]dec-6-ene-7-carbonitrile 2,2,2-trifluoroacetic acid (PH-EBR2320-270-0QH) (16.1 mg, 0.037 mmol, 48.8 % yield) as a light yellow solid. MS ESI calculated for C₁₆H₁₇N₃O₂ [M + H]⁺ 284.14 found 284.20. ¹H NMR (300 MHz, Chloroform - *d*) δ 8.68-8.39 (m, 3H), 7.72 (s, 1H), 4.42-4.32 (m, 1H), 4.02 (t, *J* = 8.6 Hz, 1H), 3.88-3.75 (m, 1H), 2.51 (dd, *J* = 8.1, 2.5 Hz, 2H), 2.30-2.05 (m, 2H), 1.29 (s, 3H), 1.22 (s, 3H). ¹⁹F NMR (282 MHz, Chloroform-*d*) δ -75.81 (s, 3F).

**Step 7H:** PH-EBR2320-270-0H was separated by *Prep*-Chiral-HPLC with the following conditions: Column: Column: CHIRALPAK IG, 2 × 25 cm, 5 µm; Mobile Phase A: MtBE-HPLC, Mobile Phase B: EtOH--HPLC; Flow rate: 20 *mL* / min; Gradient: 5% B to 5% B in 10.5 min; Wave Length: 254 / 220 nm; RT1(min): 5.47; RT2(min): 8.25. The first product containing (RT1: 5.47 min) peak was combined and concentrated under reduced pressure, then freeze-dried to give 9,9-dimethyl-8-oxo-3-(pyrazin-2-yl)-1-oxaspiro[4.5]dec-6-ene-7-carbonitrile 2,2,2-trifluoroacetic acid (PH-EBR2320-270-0HQ) (19.5 mg, 0.047 mmol, 26.7 % yield) as a light yellow solid. MS ESI calculated for C₁₆H₁₇N₃O₂ [M + H]⁺ 284.14 found 284.15. ¹H NMR (300 MHz, Chloroform - *d*) δ 8.62-8.45 (m, 3H), 7.40 (s, 1H), 4.42-4.28 (m, 1H), 4.08 (t, *J* = 8.7 Hz, 1H), 3.88-3.74 (m, 1H), 2.53 (d, *J* = 8.9 Hz, 2H), 2.35-2.15 (m, 2H), 1.31 (s, 3H), 1.21 (s, 3H). ¹⁹F NMR (282 MHz, Chloroform - *d*) δ -75.85 (s, 3F). The second product containing (RT2: 8.25 min) peak was combined and concentrated under reduced pressure, then freeze-dried to give 9,9-dimethyl-8-oxo-3-(pyrazin-2-yl)-1-oxaspiro[4.5]dec-6-ene-7-carbonitrile 2,2,2-trifluoroacetic acid (PH-EBR2320-270-0HH) (15.7mg, 0.038 mmol, 21.53 % yield) as a light yellow solid. MS ESI calculated for C₁₆H₁₇N₃O₂ [M + H]⁺ 284.14 found 284.20. ¹H NMR (300 MHz, Chloroform *- d*) δ 8.60-8.47 (m, 3H), 7.39 (s, 1H), 4.36 (dd, *J* = 8.6, 7.6 Hz, 1H), 4.08 (t, *J* = 8.7 Hz, 1H), 3.88-3.74 (m, 1H), 2.53 (d, *J* = 8.9 Hz, 2H), 2.35-2.14 (m, 2H), 1.31 (s, 3H), 1.21 (s, 3H). ¹⁹F NMR (282 MHz, Chloroform - *d*) δ -75.82 (s, 3F).

**Table 9: The following examples were prepared using a similar procedure as described above. For the oxidation conditions, 1 = Palladium(II) acetate and 2 = PhSeCl, Pyridine, H₂O₂ 3 = DDQ**

| **Ex.** | **Structure** | **Compound Name** | **Oxidation Conditions** | **Exact Mass [M+H]⁺** |
|---|---|---|---|---|
| 80 | 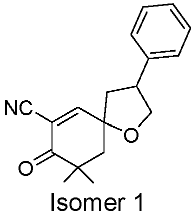 | 9,9-dimethyl-8-oxo-3-(pyrazin-2-yl)-1-oxaspiro[4.5]dec-6-ene-7-carbonitrile 2,2,2-trifluoroacetic acid | 2 | [M + Na + MeCN]⁺ 345.16 |
| 81 | 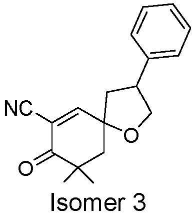 | 9,9-dimethyl-8-oxo-3-(pyrazin-2-yl)-1-oxaspiro[4.5]dec-6-ene-7-carbonitrile 2,2,2-trifluoroacetic acid | 2 | [M + Na + MeCN]⁺ 345.16 |
| 82 | 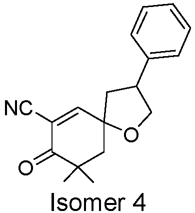 | 9,9-dimethyl-8-oxo-3-(pyrazin-2-yl)-1-oxaspiro[4.5]dec-6-ene-7-carbonitrile 2,2,2-trifluoroacetic acid | 2 | [M + Na + MeCN]⁺ 345.16 |
| 83 | 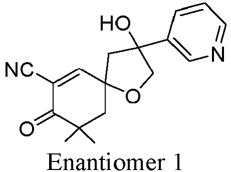 | 3-hydroxy-9,9-dimethyl-8-oxo-3-(pyridin-3-yl)-1-oxaspiro[4.5]dec-6-ene-7-carbonitrile | 3 | 299 |
| 84 | 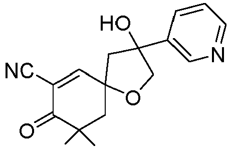 | 3-hydroxy-9,9-dimethyl-8-oxo-3-(pyridin-3-yl)-1-oxaspiro[4.5]dec-6-ene-7-carbonitrile | 3 | 299 |
| | 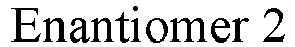 | | | |
| 85 | 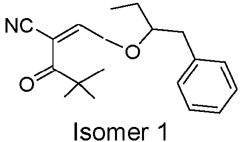 | 2-benzyl-9,9-dimethyl-8-oxo-1-oxaspiro[4.5]dec-6-ene-7-carbonitrile | 3 | 296 |
| 86 | 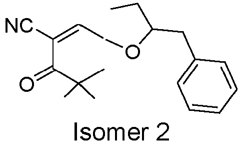 | 2-benzyl-9,9-dimethyl-8-oxo-1-oxaspiro[4.5]dec-6-ene-7-carbonitrile | 3 | 296 |
| 87 | 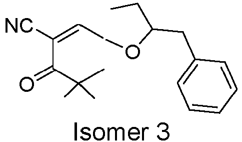 | 2-benzyl-9,9-dimethyl-8-oxo-1-oxaspiro[4.5]dec-6-ene-7-carbonitrile | 3 | 296 |
| 88 | 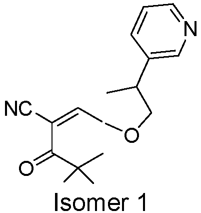 | 9,9-dimethyl-8-oxo-3-(pyridin-3-yl)-1-oxaspiro[4.5]dec-6-ene-7-carbonitrile | 2 | 283 |
| 89 | 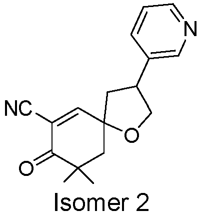 | 9,9-dimethyl-8-oxo-3-(pyridin-3-yl)-1-oxaspiro[4.5]dec-6-ene-7-carbonitrile | 2 | 283 |
| 90 | 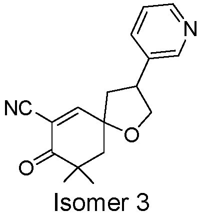 | 9,9-dimethyl-8-oxo-3-(pyridin-3-yl)-1-oxaspiro[4.5]dec-6-ene-7-carbonitrile | 2 | 283 |
| 91 | 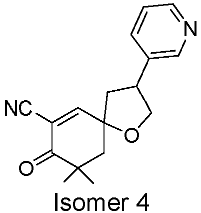 | 9,9-dimethyl-8-oxo-3-(pyridin-3-yl)-1-oxaspiro[4.5]dec-6-ene-7-carbonitrile | 2 | 283 |
| 92 | 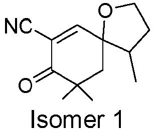 | 4,9,9-trimethyl-8-oxo-1-oxaspiro[4.5]dec-6-ene-7-carbonitrile | 3 | 220 |
| 93 | 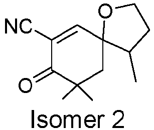 | 4,9,9-trimethyl-8-oxo-1-oxaspiro[4.5]dec-6-ene-7-carbonitrile | 3 | 220 |
| 94 | 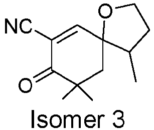 | 4,9,9-trimethyl-8-oxo-1-oxaspiro[4.5]dec-6-ene-7-carbonitrile | 3 | 220 |
| 95 | 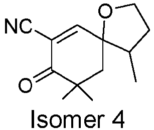 | 4,9,9-trimethyl-8-oxo-1-oxaspiro[4.5]dec-6-ene-7-carbonitrile | 3 | 220 |
| 96 | 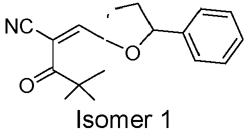 | 9,9-dimethyl-8-oxo-2-phenyl-1-oxaspiro[4.5]dec-6-ene-7-carbonitrile | 1 | 299 [M + NH₄]⁺ |
| 97 | 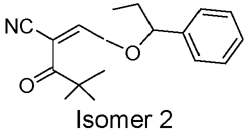 | 9,9-dimethyl-8-oxo-2-phenyl-1-oxaspiro[4.5]dec-6-ene-7-carbonitrile | 1 | 299 [M + NH₄]⁺ |
| 98 | 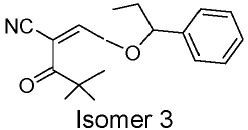 | 9,9-dimethyl-8-oxo-2-phenyl-1-oxaspiro[4.5]dec-6-ene-7-carbonitrile | 1 | 299 [M + NH₄]⁺ |
| 99 | 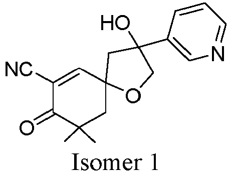 | 3-hydroxy-9,9-dimethyl-8-oxo-3-(pyridin-3-yl)-1-oxaspiro[4.5]dec-6-ene-7-carbonitrile | 3 | 299 |
| 100 | 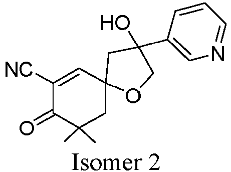 | 3-hydroxy-9,9-dimethyl-8-oxo-3-(pyridin-3-yl)-1-oxaspiro[4.5]dec-6-ene-7-carbonitrile | 3 | 299 |
| 101 | 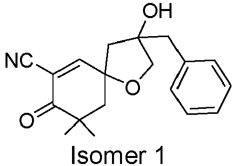 | 3-benzyl-3-hydroxy-9,9-dimethyl-8-oxo-1-oxaspiro[4.5]dec-6-ene-7-carbonitrile | 1 | 344 [M + Na]⁺ |
| 102 | 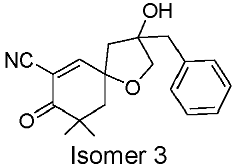 | 3-benzyl-3-hydroxy-9,9-dimethyl-8-oxo-1-oxaspiro[4.5]dec-6-ene-7-carbonitrile | 1 | 375 [M + Na + MeCN]⁺ |
| 103 | 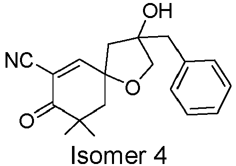 | 3-benzyl-3-hydroxy-9,9-dimethyl-8-oxo-1-oxaspiro[4.5]dec-6-ene-7-carbonitrile | 1 | 375 [M + Na + MeCN]⁺ |
| 104 | 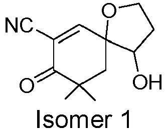 | 4-hydroxy-9,9-dimethyl-8-oxo-1-oxaspiro[4.5]dec-6-ene-7-carbonitrile | 3 | 220 [M - H]⁻ |
| 105 | 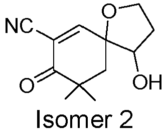 | 4-hydroxy-9,9-dimethyl-8-oxo-1-oxaspiro[4.5]dec-6-ene-7-carbonitrile | 3 | 220 [M - H]⁻ |
| 106 | 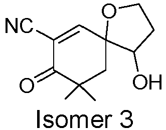 | 4-hydroxy-9,9-dimethyl-8-oxo-1-oxaspiro[4.5]dec-6-ene-7-carbonitrile | 3 | 220 [M - H]⁻ |
| 107 | 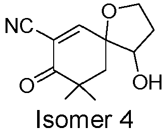 | 4-hydroxy-9,9-dimethyl-8-oxo-1-oxaspiro[4.5]dec-6-ene-7-carbonitrile | 3 | 220 [M - H]⁻ |
| 108 | 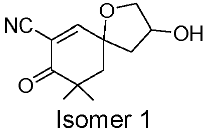 | 3-Hydroxy-9,9-dimethyl-8-oxo-1-oxaspiro[4.5]dec-6-ene-7-carbonitrile | 1 | 220 [M - H]⁻ |
| 109 | 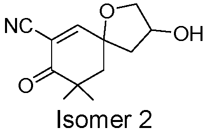 | 3-Hydroxy-9,9-dimethyl-8-oxo-1-oxaspiro[4.5]dec-6-ene-7-carbonitrile | 1 | 220 [M - H]⁻ |
| 110 | 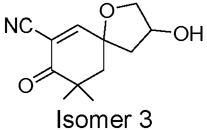 | 3-Hydroxy-9,9-dimethyl-8-oxo-1-oxaspiro[4.5]dec-6-ene-7-carbonitrile | 1 | 220 [M - H]⁻ |
| 111 | 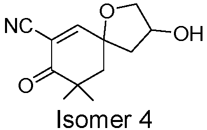 | 3-Hydroxy-9,9-dimethyl-8-oxo-1-oxaspiro[4.5]dec-6-ene-7-carbonitrile | 1 | 220 [M - H]⁻ |
| 112 | 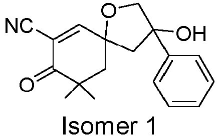 | 3-hydroxy-9,9-dimethyl-8-oxo-3-phenyl-1-oxaspiro[4.5]dec-6-ene-7-carbonitrile | 1 | 315 [M + NH₄]⁺ |
| 113 | 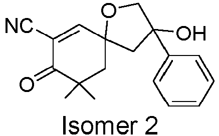 | 3-hydroxy-9,9-dimethyl-8-oxo-3-phenyl-1-oxaspiro[4.5]dec-6-ene-7-carbonitrile | 1 | 315 [M + NH₄]⁺ |
| 114 | 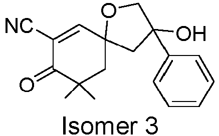 | 3-hydroxy-9,9-dimethyl-8-oxo-3-phenyl-1-oxaspiro[4.5]dec-6-ene-7-carbonitrile | 1 | 315 [M + NH₄]⁺ |
| 115 | 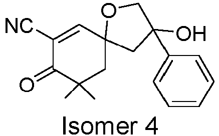 | 3-hydroxy-9,9-dimethyl-8-oxo-3-phenyl-1-oxaspiro[4.5]dec-6-ene-7-carbonitrile | 1 | 315 [M + NH₄]⁺ |
| 116 | 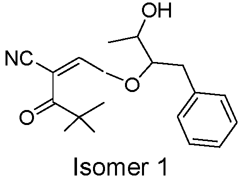 | 2-benzyl-3-hydroxy-9,9-dimethyl-8-oxo-1-oxaspiro[4.5]dec-6-ene-7-carbonitrile | 1 | 310 [M - H]⁻ |
| 117 | 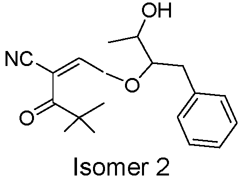 | 2-benzyl-3-hydroxy-9,9-dimethyl-8-oxo-1-oxaspiro[4.5]dec-6-ene-7-carbonitrile | 1 | 310 [M - H]⁻ |
| 118 | 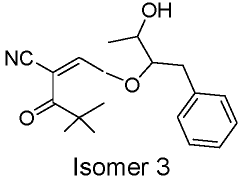 | 2-benzyl-3-hydroxy-9,9-dimethyl-8-oxo-1-oxaspiro[4.5]dec-6-ene-7-carbonitrile | 1 | 334 [M + Na]⁺ |
| 119 | 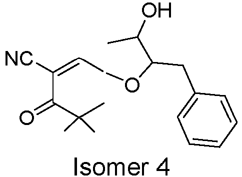 | 2-benzyl-3-hydroxy-9,9-dimethyl-8-oxo-1-oxaspiro[4.5]dec-6-ene-7-carbonitrile | 1 | 334 [M + Na]⁺ |
| 120 | 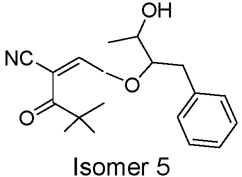 | 2-benzyl-3-hydroxy-9,9-dimethyl-8-oxo-1-oxaspiro[4.5]dec-6-ene-7-carbonitrile | 1 | 310 [M - H]⁻ |
| 121 | 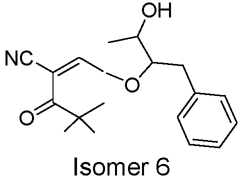 | 2-benzyl-3-hydroxy-9,9-dimethyl-8-oxo-1-oxaspiro[4.5]dec-6-ene-7-carbonitrile | 1 | 310 [M - H]⁻ |
| 122 | 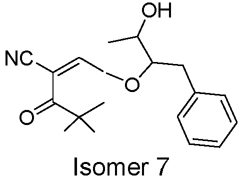 | 2-benzyl-3-hydroxy-9,9-dimethyl-8-oxo-1-oxaspiro[4.5]dec-6-ene-7-carbonitrile | 1 | 310 [M - H]⁻ |
| 123 | 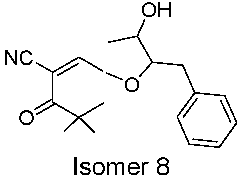 | 2-benzyl-3-hydroxy-9,9-dimethyl-8-oxo-1-oxaspiro[4.5]dec-6-ene-7-carbonitrile | 1 | 334 [M + Na]⁺ |

### Example 124, isomer 1: 3'-(hydroxymethyl)-5,5-dimethyl-4-oxo-3'H-spiro[cyclohexane-1,1'-isobenzofuran]-2-ene-3-carbonitrile

### Example 125, isomer 2: 3'-(hydroxymethyl)-5,5-dimethyl-4-oxo-3'H-spiro[cyclohexane-1,1'-isobenzofuran]-2-ene-3-carbonitrile

### Example 126, isomer 3: 3'-(hydroxymethyl)-5,5-dimethyl-4-oxo-3'H-spiro[cyclohexane-1,1'-isobenzofuran]-2-ene-3-carbonitrile

### Example 127, isomer 4: 3'-(hydroxymethyl)-5,5-dimethyl-4-oxo-3'H-spiro[cyclohexane-1,1'-isobenzofuran]-2-ene-3-carbonitrile

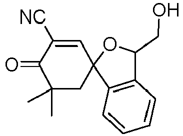

**Step 1:** To a solution of 2-(2-bromophenyl)oxirane (2 g, 10.05 mmol) in THF (35 mL) at -78 °C was added N-butyllithium (4.82 ml, 12.06 mmol). The resulting suspension was stirred for 30 mins. A solution of 6,6-dimethyl-1,4-dioxaspiro[4.5]decan-8-one (1.851 g, 10.05 mmol) in THF (4 mL) was added and the reaction mixture was stirred for 1.5 h at -78 °C, then warmed to 0 °C. The reaction mixture was quenched with NH4Cl(aq), extracted with EtOAc (x3), washed with brine, dried over Na2SO4, concentrated, and purified by silica gel column chromatography (0 to 60% Et₂O/hexanes) to afford (2',2'-dimethyl-3H dispiro[isobenzofuran-1,4'-cyclohexane-1',2"-[1,3]dioxolan]-3-yl)methanol as an oil. MS: 285 (M - 17)

**Step 2:** To a solution of (2',2'-dimethyl-3H-dispiro[isobenzofuran-1,4'-cyclohexane-1',2"-[1,3]dioxolan]-3-yl)methanol (2.9 g, 9.53 mmol) in DCM (23.82 ml) was added imidazole (1.297 g, 19.05 mmol) and tert-butyldimethylsilyl chloride (1.436 g, 9.53 mmol). The reaction mixture was left to stir at rt overnight.

The reaction mixture was concentrated to afford tert-butyl((2',2'-dimethyl-3H-dispiro[isobenzofuran-1,4'-cyclohexane-1',2"-[1,3]dioxolan]-3-yl)methoxy)dimethylsilane as an oil. MS: 418 (M + 1)

**Step 3:** A solution of tert-butyl((2',2'-dimethyl-3H-dispiro[isobenzofuran-1,4'-cyclohexane-1',2"-[1,3]dioxolan]-3-yl)methoxy)dimethylsilane (3.67 g, 8.77 mmol) in THF (17.53 ml) and 1 M HCl(aq) (17.53 ml) was stirred overnight at rt.

The reaction mixture was quenched with 1 M HCl(aq), extracted with DCM, concentrated, and purified by silica gel column chromatography (0 to 40% Et₂O/hexanes) to afford 3'-(((tert-butyldimethylsilyl)oxy)methyl)-3,3-dimethyl-3'H-spiro[cyclohexane-1,1'-isobenzofuran]-4-one as an oil. MS: 375 (M + 1)

**Step 4:** To a solution of 2,2,6,6-tetramethylpiperidine (1.352 ml, 8.01 mmol) in THF (25 mL) at 0 °C was added N-butyllithium (3.20 ml, 8.01 mmol). The reaction mixture was stirred for 20 mins, then cooled down to -78 °C before the addition of a solution of 3'-(((tert-butyldimethylsilyl)oxy)methyl)-3,3-dimethyl-3'H-spiro[cyclohexane-1,1'-isobenzofuran]-4-one (1 g, 2.67 mmol) in THF (5 mL), The reaction mixture was stirred for a further 20 mins. Then, a solution of tosyl chloride (0.580 g, 3.20 mmol) in THF (7.5 mL) was added. The reaction mixture was allowed to warm to rt and stirred overnight.

The reaction mixture was quenched with 1 M HCl(aq), extracted with EtOAc, washed with brine, dried over Na2SO4, concentrated, and purified by silica gel column chromatography (0 to 50% Et₂O/hexanes) to afford 3'-(((tert-butyldimethylsilyl)oxy)methyl)-3,3-dimethyl-4-oxo-3'H-spiro[cyclohexane-1,1'-isobenzofuran]-5-carbonitrile as an oil. MS: 400 (M + 1)

**Step 5:** To a solution of pyridine (0.335 mL, 4.15 mmol) in DCM (2.87 mL) at 0 °C was added a solution of phenylselenenyl chloride (796 mg, 4.15 mmol) in DCM (2 mL). The reaction mixture was stirred for 20 mins at 0 °C before the addition of a solution of 3'-(((tert-butyldimethylsilyl)oxy)methyl)-3,3-dimethyl-4-oxo-3'H-spiro[cyclohexane-1,1'-isobenzofuran]-5-carbonitrile (830 mg, 2.077 mmol) in DCM (2 mL). The reaction mixture was allowed to stir for 1 h, then washed with 1 M HCl(aq) and treated with hydrogen peroxide (2.55 mL, 41.5 mmol) at 0 °C. The reaction mixture was stirred for 15 mins, then quenched with sat. NaHCO3(aq), extracted with DCM, washed with water, dried over Na2SO4 and concentrated to afford 3'-(((tert-butyldimethylsilyl)oxy)methyl)-5,5-dimethyl-4-oxo-3'H-spiro[cyclohexane-1,1'-isobenzofuran]-2-ene-3-carbonitrile as an oil. MS: 398 (M + 1)

**Step 6:** To a solution of 3'-(((tert-butyldimethylsilyl)oxy)methyl)-5,5-dimethyl-4-oxo-3'H-spiro[cyclohexane-1,1'-isobenzofuran]-2-ene-3-carbonitrile (826 mg, 2.078 mmol) in DCM (9.216 mL) was added hydrochloride acid (4 M in 1,4-dioxane, 3.64 mL, 14.54 mmol). The reaction mixture was left to stir overnight at rt.

The reaction mixture was concentrated and purified by silica gel column chromatography (0 to 70% Et2O/hexanes) and by SFC (MeOH) to afford 3'-(hydroxymethyl)-5,5-dimethyl-4-oxo-3'H-spiro[cyclohexane-1,1'-isobenzofuran]-2-ene-3-carbonitrile (isomer 1, 1^{st} eluting): ¹H NMR (600 MHz, Chloroform-d) δ 7.45 - 7.37 (m, 2H), 7.28 (d, *J* = 7.6 Hz, 1H), 7.24 (d, *J* = 1.6 Hz, 1H), 7.11 - 7.07 (m, 1H), 5.46 - 5.41 (m, 1H), 4.05 - 3.95 (m, 1H), 3.85 (dd, *J* = 12.0, 4.6 Hz, 1H), 2.27 (d, *J =* 15.0 Hz, 1H), 2.20 (dd, *J =* 15.0, 1.8 Hz, 1H), 1.45 (s, 3H), 1.23 (s, 3H), 3'-(hydroxymethyl)-5,5-dimethyl-4-oxo-3'H-spiro[cyclohexane-1,1'-isobenzofuran]-2-ene-3-carbonitrile (isomer 2, 2^{nd} eluting): ¹H NMR (600 MHz, Chloroform-d) δ 7.46 - 7.35 (m, 2H), 7.28 (d, *J* = 7.3 Hz, 1H), 7.24 (d, *J =* 2.0 Hz, 1H), 7.12 - 7.07 (m, 1H), 5.52 - 5.35 (m, 1H), 4.01 (dd, *J =* 12.0, 3.2 Hz, 1H), 3.93 - 3.78 (m, 1H), 2.28 (d, *J* = 15.0 Hz, 1H), 2.20 (dd, *J* = 15.0, 2.0 Hz, 1H), 1.45 (s, 3H), 1.23 (s, 3H), 3'-(hydroxymethyl)-5,5-dimethyl-4-oxo-3'H-spiro[cyclohexane-1,1'-isobenzofuran]-2-ene-3-carbonitrile (isomer 3, 3^{rd} eluting): ¹H NMR (600 MHz, Chloroform-d) δ 7.47 - 7.38 (m, 2H), 7.30 (d, *J* = 7.3 Hz, 1H), 7.15 (d, *J* = 2.1 Hz, 1H), 7.12 (d, *J* = 7.3 Hz, 1H), 5.54 - 5.38 (m, 1H), 3.99 (dd, *J =* 11.9, 3.4 Hz, 1H), 3.87 (dd, *J* = 12.0, 5.2 Hz, 1H), 2.53 - 2.31 (m, 1H), 2.31 - 2.13 (m, 1H), 1.44 (s, 3H), 1.23 (s, 3H), and 3'-(hydroxymethyl)-5,5-dimethyl-4-oxo-3'H-spiro[cyclohexane-1,1'-isobenzofuran]-2-ene-3-carbonitrile (isomer 4, 4^{th} eluting): ¹H NMR (600 MHz, Chloroform-*d*) δ 7.47 - 7.37 (m, 2H), 7.30 (d, *J* = 7.3 Hz, 1H), 7.15 (d, *J* = 2.1 Hz, 1H), 7.12 (d, *J* = 7.2 Hz, 1H), 5.44 (dt, *J =* 20.8, 4.2 Hz, 1H), 3.99 (dd, *J =* 12.0, 3.4 Hz, 1H), 3.86 (td, *J =* 11.7, 5.1 Hz, 1H), 2.41 (d, *J* = 15.1 Hz, 1H), 2.24 (dd, *J =* 15.1, 2.1 Hz, 1H), 1.46 (s, 3H), 1.24 (s, 3H).

### Example 128: 1'-(4-methoxybenzyl)-5,5-dimethyl-4-oxo-6',7'-dihydro-1'H-spiro[cyclohexane-1,4'-pyrano[3,4-d]imidazol]-2-ene-3-carbonitrile

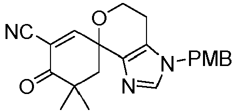

**Step 1:** To a mixture of 4,5-diiodo-1*H*-imidazole (25 g, 78 mmol) and K₂CO₃ (16.20 g, 117 mmol) in acetonitrile (250 mL) was added 4-methoxybenzyl chloride (15.90 mL, 117 mmol) at room temperature. The reaction mixture was stirred for 10 min at room temperature and continued to stir for 16 h at 80 °C. The reaction mixture was filtered. The filtrate was concentrated under reduced pressure and the residue was purified by silica gel column chromatography, eluted with 1 ~ 70 % ethyl acetate in petroleum ether to afford 4,5-diiodo-1-(4-methoxybenzyl)-1*H*-imidazole (17 g, 38.6 mmol, 49.4% yield) as a yellow solid. MS ESI calculated for C₁₁H₁₀I₂N₂O [M + H]⁺ 440.89 found 440.95. ¹H NMR (300 MHz, Chloroform-d) δ 7.57 (s, 1H), 7.11 (d, *J =* 8.7 Hz, 2H), 6.89 (d, *J =* 8.7 Hz, 2H), 5.08 (s, 2H), 3.81 (s, 3H). **Step 2:** To a solution of 4,5-diiodo-1-(4-methoxybenzyl)-1H-imidazole (17 g, 38.6 mmol) in THF (350 mL) was added ethylmagnesium bromide (3 M in THF, 14.17 mL, 42.5 mmol) at 0 °C and the solution was stirred for 1 h at room temperature. Then a solution of DMF (8.97 mL, 116 mmol) in THF (30 mL) was added to the mixture at 0 °C and the mixture was stirred for 16 h at room temperature. The reaction mixture was quenched by water (50 mL), extracted with ethyl acetate (200 mL × 3). The combined organic layers was washed with brine (200 mL), dried over anhydrous sodium sulfate and filtered. The filtrate was concentrated under reduced pressure. The residue was purified by silica gel column chromatography, eluted with 1 ~ 70 % ethyl acetate in petroleum ether to afford 4-iodo-1-(4-methoxybenzyl)-1*H*-imidazole-5-carbaldehyde (9.8 g, 28.6 mmol, 74.1% yield) as a yellow solid. MS ESI calculated for C₁₂H₁₁IN₂O₂ [M + H]⁺ 342.99 found 343.00. ¹H NMR (300 MHz, Chloroform-*d*) δ 9.64 (d, *J =* 1.0 Hz, 1H), 7.57 (d, *J* = 1.0 Hz, 1H), 7.19 (d, *J =* 8.7 Hz, 2H), 6.88 (d, *J =* 8.7 Hz, 2H), 5.42 (s, 2H), 3.80 (s, 3H).

**Step 3:** To a solution of (methoxymethyl)triphenylphosphonium chloride (6.51 g, 19.00 mmol) in THF (200 mL) was added lithium bis(trimethylsilyl)amide (1 M in THF, 19 mL, 19.00 mmol) at 0 °C and the mixture was stirred for 2 h at room temperature. Then a solution of 4-iodo-1-(4-methoxybenzyl)-1*H*-imidazole-5-carbaldehyde (5 g, 14.61 mmol) in THF (20 mL) was added to the mixture at 0 °C and the resulting mixture was stirred for 16 h at room temperature. The reaction mixture was quenched by NH₄Cl (200 mL), diluted with ethyl acetate (500 mL), washed with brine (200 mL × 2), dried over anhydrous sodium sulfate and filtered. The filtrate was concentrated under reduced pressure. The reaction mixture was purified by silica gel column chromatography, eluted with 0 ~ 50 % ethyl acetate in petroleum ether to afford 4-iodo-1-(4-methoxybenzyl)-5-(2-methoxyvinyl)-1*H*-imidazole (3.8 g, 10.06 mmol, 68.8% yield) as a light yellow solid. MS ESI calculated for C₁₄H₁₅IN₂O₂ [M + H]⁺ 371.02 found 371.10. ¹H NMR (400 MHz, Chloroform-*d*) δ 7.42 (s, 0.5H), 7.35 (s, 0.5H), 7.08-6.99 (m, 2H), 6.95 (d, *J* = 12.9 Hz, 0.5H), 6.90-6.81 (m, 2H), 6.18 (d, *J* = 6.7 Hz, 0.5H), 5.28 (d, *J* = 12.8 Hz, 0.5H), 5.02 (s, 1H), 5.00 (s, 1H), 4.97 (d, *J* = 6.7 Hz, 0.5H), 3.800 (s, 1.5H), 3.796 (s, 1.5H), 3.72 (s, 1.5H), 3.64 (s, 1.5H).

**Step 4:** To a solution of 4-iodo-1-(4-methoxybenzyl)-5-(2-methoxyvinyl)-1*H*-imidazole (3.8 g, 10.26 mmol) in acetone (20 mL) was added HBr (48%, 20 mL, 147 mmol) at room temperature. The resulted mixture was allowed to warm to 40 °C and stir for 16 h at this temperature. The reaction mixture was concentrated under reduced pressure. The residue was diluted with ethyl acetate (300 mL), washed with brine (100 mL × 2), dried over anhydrous sodium sulfate and filtered. The filtrate was concentrated under reduced pressure. The residue was purified by silica gel column chromatography, eluted with 0 ~ 60 % ethyl acetate in petroleum ether to give 2-(4-iodo-1-(4-methoxybenzyl)-1*H*-imidazol-5-yl)acetaldehyde (2.3 g, 6.46 mmol, 62.9% yield) as a yellow solid. MS ESI calculated for C₁₃H₁₃IN₂O₂ [M + H]⁺ 357.00 found 357.00. ¹H NMR (400 MHz, Chloroform-*d*) δ 9.45 (t, *J =* 1.7 Hz, 1H), 7.54 (s, 1H), 7.01 (d, *J* = 8.7 Hz, 2H), 6.88 (d, *J* = 8.6 Hz, 2H), 5.00 (s, 2H), 3.80 (s, 3H), 3.59 (d, *J =* 1.6 Hz, 2H).

**Step 5:** To a solution of 2-(4-iodo-1-(4-methoxybenzyl)-1H-imidazol-5-yl)acetaldehyde (2.3 g, 6.46 mmol) in MeOH (25 mL) was added NaBH₄ (0.366 g, 9.69 mmol) at 0 °C. The resulted mixture was stirred for 2 h at room temperature. The reaction solution was quenched by water (20 mL), diluted with ethyl acetate (200 mL), washed with brine (50 mL × 2), dried over anhydrous sodium sulfate and filtered. The filtrate was concentrated under reduced pressure. The residue was purified by silica gel column chromatography, eluted with 0 ~ 10 % MeOH in DCM to give 2-(4-iodo-1-(4-methoxybenzyl)-1*H*-imidazol-5-yl)ethan-1-ol (1.9 g, 4.99 mmol, 77% yield) as a light yellow solid. MS ESI calculated for C₁₃H₁₅IN₂O₂ [M + H]⁺ 359.02 found 359.10. ¹H NMR (400 MHz, Chloroform-*d*) δ 7.42 (s, 1H), 7.04 (d, *J* = 8.5 Hz, 2H), 6.87 (d, *J* = 8.6 Hz, 2H), 5.15 (s, 2H), 3.80 (s, 3H), 3.71 (t, *J* = 6.3 Hz, 2H), 2.76 (t, *J* = 6.3 Hz, 2H).

**Step 6:** To a solution of 2-(4-iodo-1-(4-methoxybenzyl)-1*H*-imidazol-5-yl)ethan-1-ol (330 mg, 0.921 mmol) in DCM (4 mL) was added ethylmagnesium bromide (3 M in THF, 270 mg, 2.027 mmol) at 0 °C. The mixture was stirred for 1 h at room temperature. Then 6,6-dimethyl-1,4-dioxaspiro[4.5]decan-8-one (170 mg, 0.921 mmol) was added at 0 °C. The resulted mixture was stirred for 2 h at room temperature. The reaction solution was quenched by water (4 mL), diluted with ethyl acetate (50 mL), washed with brine (20 mL × 2), dried over anhydrous sodium sulfate and filtered. The filtrate was concentrated under reduced pressure. The residue was purified by RP-Flash with the following conditions: Column: Flash C¹⁸ 40 g; Mobile Phase A: water, Mobile Phase B: MeCN; Flow rate: 40 mL / min; Gradient: 2% B to 40% B in 25 min, Detector: UV 220 nm; RT = 20 min. The collected fractions were combined and concentrated under reduced pressure to afford 8-(5-(2-hydroxyethyl)-1-(4-methoxybenzyl)-1*H*-imidazol-4-yl)-6,6-dimethyl-1,4-dioxaspiro[4.5]decan-8-ol (100 mg, 0.240 mmol, 26.1% yield) as a light yellow solid. MS ESI calculated for C₂₃H₃₂N₂O₅ [M + H]⁺ 417.23 found 417.30. ¹H NMR (300 MHz, DMSO-*d*₆) δ 7.46 (s, 1H), 7.06 (d, *J* = 8.6 Hz, 2H), 6.95-6.86 (m, 2H), 5.04 (s, 2H), 3.90-3.83 (m, 4H), 3.73 (s, 3H), 3.52-3.32 (m, 2H), 2.93-2.75 (m, 2H), 2.37-2.13 (m, 1H), 2.10-1.89 (m, 2H), 1.66-1.39 (m, 3H), 1.17 (s, 3H), 0.73 (s, 3H).

**Step 7:** To a solution of 8-(5-(2-hydroxyethyl)-1-(4-methoxybenzyl)-1*H*-imidazol-4-yl) -6,6-dimethyl-1,4-dioxaspiro[4.5]decan-8-ol (1 g, 2.401 mmol) in DCM (10 mL) were added TEA (1.00 mL, 7.20 mmol), DMAP (0.029 g, 0.240 mmol) and TsCl (0.687 g, 3.60 mmol) at 0 °C. The resulted mixture was stirred for 2 h at room temperature. The reaction solution was quenched by water (20 mL), diluted with DCM (60 mL), washed with brine (30 mL), dried over anhydrous sodium sulfate and filtered. The filtrate was concentrated under reduced pressure. The residue was purified by RP-Flash with the following conditions: Column: Flash C¹⁸ 120 g; Mobile Phase A: water, Mobile Phase B: MeCN; Flow rate: 30 mL / min; Gradient: 2% B to 50% B in 25 min, Detector: UV 220 nm; RT = 20 min. The collected fractions were combined and concentrated under reduced pressure to afford 2-(4-(8-hydroxy-6,6-dimethyl-1,4-dioxaspiro[4.5]decan-8-yl)-1-(4-methoxybenzyl)-1*H*-imidazol-5-yl)ethyl 4-methylbenzenesulfonate (1 g, 1.609 mmol, 67.0% yield) as a light yellow solid. MS ESI calculated for C₃₀H₃₈N₂O₇S [M + H]⁺ 571.24 found 571.25. ¹H NMR (300 MHz, Chloroform-d) δ 7.74-7.64 (m, 2H), 7.45 (d, *J* = 2.7 Hz, 1H), 7.36 (dd, *J* = 8.7, 1.0 Hz, 2H), 7.09-7.00 (m, 2H), 6.97-6.86 (m, 2H), 5.04 (s, 2H), 4.14-3.90 (m, 6H), 3.86 (d, *J =* 1.6 Hz, 3H), 3.13 (t, *J =* 6.9 Hz, 2H), 2.50 (s, 3H), 2.46-2.08 (m, 3H), 1.83-1.66 (m, 1H), 1.57 (ddd, *J* = 14.4, 8.5, 3.0 Hz, 2H), 1.31 (s, 3H), 0.89 (s, 3H).

**Step 8:** To a solution of 2-(4-(8-hydroxy-6,6-dimethyl-1,4-dioxaspiro[4.5]decan-8-yl)-1- (4-methoxybenzyl)-1*H*-imidazol-5-yl)ethyl 4-methylbenzenesulfonate (1.2 g, 2.103 mmol) in DMF (13 mL) was added NaH (60% in mineral oil, 0.126 g, 3.15 mmol) at 0 °C and the mixture was stirred for 30 min. Then the mixture stirred for 4 h at room temperature. The reaction mixture was quenched by NH₄Cl (30 mL) and extracted with ethyl acetate (100 mL × 2). The combined organic layer was washed with brine (30 mL × 2), dried over anhydrous sodium sulfate and filtered. The residue was purified by RP-Flash with the following conditions: Column: Flash C¹⁸ 120 g; Mobile Phase A: water, Mobile Phase B: MeCN; Flow rate: 60 mL / min; Gradient: 2% B to 50% B in 20 min, Detector: UV 220 nm; RT = 20 min. The collected fractions were combined and concentrated under reduced pressure to afford 1-(4-methoxybenzyl)-2',2'-dimethyl-6,7-dihydro-1*H*-dispiro[pyrano[3,4-*d*]imidazole-4,4'-cyclohexane-1',2"-[1,3]dioxolane] (440 mg, 1.068 mmol, 50.8% yield) as a light yellow solid. MS ESI calculated for C₂₃H₃₀N₂O₄ [M + H]⁺ 399.22 found 399.15. ¹H NMR (300 MHz, Chloroform-d) δ 7.36 (s, 1H), 7.03 (d, *J* = 8.6 Hz, 2H), 6.86 (d, *J* = 8.6 Hz, 2H), 4.93 (s, 2H), 4.03-3.82 (m, 6H), 3.80 (s, 3H), 2.63-2.22 (m, 3H), 2.20-1.98 (m, 2H), 1.81-1.63 (m, 2H), 1.57-1.45 (m, 1H), 1.25 (s, 3H), 0.87 (s, 3H).

**Step 9:** To a solution of 1-(4-methoxybenzyl)-2',2'-dimethyl-6,7-dihydro-1*H*-dispiro [pyrano[3,4-*d*]imidazole-4,4'-cyclohexane-1',2"-[1,3]dioxolane] (440 mg, 1.104 mmol) in THF (5 mL) was added HCl (2 M in water, 4 mL, 8.00 mmol) at 0 °C. The resulted mixture was allowed to warm to room temperature and stir for 2 h. The reaction mixture was concentrated under reduced pressure. The residue was purified by RP-Flash with the following conditions: Column: Flash C¹⁸ 120 g; Mobile Phase A: water, Mobile Phase B: MeCN; Flow rate: 60 mL / min; Gradient: 2 % B to 50 % B in 20 min, Detector: UV 220 nm; RT = 20 min. The collected fractions were combined and concentrated under reduced pressure to afford 1'-(4-methoxybenzyl)-3,3-dimethyl-6',7'-dihydro-1'*H*-spiro[cyclohexane-1,4'-pyrano[3,4-*d*]imidazol]-4-one (370 mg, 1.044 mmol, 95% yield) as a light yellow oil. MS ESI calculated for C₂₁H₂₆N₂O₃ [M + H]⁺ 355.19 found 355.25. ¹H NMR (300 MHz, Chloroform-*d*) δ 7.42 (s, 1H), 7.06 (d, *J* = 8.6 Hz, 2H), 6.88 (d, *J* = 8.7 Hz, 2H), 4.96 (s, 2H), 3.97 (dd, *J* = 5.9, 5.0 Hz, 2H), 3.81 (s, 3H), 3.02 (td, *J* = 14.2, 5.7 Hz, 1H), 2.64-2.33 (m, 3H), 2.33-2.20 (m, 2H), 2.20-2.02 (m, 2H), 1.35 (s, 3H), 1.07 (s, 3H).

**Step 10:** To a solution of 1'-(4-methoxybenzyl)-3,3-dimethyl-6',7'-dihydro-1'*H*-spiro [cyclohexane-1,4'-pyrano[3,4-*d*]imidazol]-4-one (340 mg, 0.959 mmol) in toluene (5 mL) were added ethyl formate (0.859 mL, 10.55 mmol) and sodium methoxide (30% in MeOH, 1.90 g, 10.55 mmol). The resulted mixture was stirred for 2 h at room temperature. The reaction solution was quenched by NH₄Cl (10 mL), diluted with ethyl acetate (50 mL), washed with brine (20 mL × 2), dried over anhydrous sodium sulfate, filtered. The filtrate was concentrated in vacuo to give crude 5-(hydroxymethylene)-1'-(4-methoxybenzyl)-3,3-dimethyl-6',7'-dihydro-1'*H-*spiro[cyclohexane-1,4'-pyrano[3,4-*d*]imidazol]-4-one (366.78 mg, 0.959 mmol) as a yellow solid, which was used for the next step without further purification. MS ESI calculated for C₂₂H₂₆N₂O₄ [M + H]⁺ 383.19 found 383.25.

**Step 11:** To a solution of 5-(hydroxymethylene)-1'-(4-methoxybenzyl)-3,3-dimethyl-6',7'-dihydro-1'*H*-spiro[cyclohexane-1,4'-pyrano[3,4-*d*]imidazol]-4-one (367 mg, 0.960 mmol) in EtOH (4 mL) and water (4 mL) were added hydroxylamine hydrochloride (80 mg, 1.151 mmol) and sodium acetate (157 mg, 1.919 mmol). The resulted mixture was stirred for 2 h at room temperature. The reaction solution was quenched by water (5 mL), diluted with ethyl acetate (30 mL), washed with brine (10 mL × 2), dried over anhydrous sodium sulfate, filtered, and concentrated in vacuo. The resulting solution was purified by RP-Flash with the following conditions: Column: Flash C¹⁸ 40 g; Mobile Phase A: water, Mobile Phase B: MeCN; Flow rate: 35 mL / min; Gradient: 2% B to 45% B in 20 min, Detector: UV 220 nm; RT = 20 min. The collected fractions were combined and concentrated under reduced pressure to afford 1'-(4-methoxybenzyl)-3,3-dimethyl-4-oxo-6',7'-dihydro-1'*H*-spiro[cyclohexane-1,4'-pyrano[3,4-*d*]imidazole]-5-carbaldehyde oxime (320 mg, 0.805 mmol, 84% yield) as an off-white solid. MS ESI calculated for C₂₂H₂₇N₃O₄ [M + H]⁺ 398.20 found 398.25. ¹H NMR (300 MHz, Methanol-*d*₄) δ 7.61 (s, 1H), 7.50 (d, *J =* 6.7 Hz, 1H), 7.15 (d, *J* = 8.5 Hz, 2H), 6.93 (d, *J* = 8.5 Hz, 2H), 5.10 (s, 2H), 4.02 (t, *J* = 5.5 Hz, 2H), 3.95-3.83 (m, 1H), 3.80 (d, *J* = 1.2 Hz, 3H), 2.64-2.09 (m, 6H), 1.42 (s, 3H), 1.07 (s, 3H).

**Step 12:** To a solution of 1'-(4-methoxybenzyl)-3,3-dimethyl-4-oxo-6',7'-dihydro-1'*H*-spiro [cyclohexane-1,4'-pyrano[3,4-*d*]imidazole]-5-carbaldehyde oxime (244 mg, 0.614 mmol) in acetonitrile (1 mL) was added bismuth(III) trifluoromethanesulfonate (403 mg, 0.614 mmol). The resulted mixture was stirred for 4 h at 80 °C. The reaction was concentrated under reduced pressure. The residue was purified by Prep-TLC with CH₂Cl₂ / MeOH (15 : 1) to afford 1'-(4-methoxybenzyl)-7,7-dimethyl-6,6',7,7'-tetrahydro-1'*H*,4*H*-spiro[benzo[*d*]isoxazole-5,4'-pyrano[3,4-*d*]imidazole] (200 mg, 0.527 mmol, 86% yield) as a yellow solid. MS ESI calculated for C₂₂H₂₅N₃O₃ [M + H]⁺ 380.19 found 380.20. ¹H NMR (300 MHz, Chloroform-*d*) δ 8.04 (s, 1H), 7.54 (s, 1H), 7.09 (d, *J* = 8.5 Hz, 2H), 6.90 (d, *J* = 8.6 Hz, 2H), 4.99 (s, 2H), 3.92-3.78 (m, 2H), 3.82 (s, 3H), 3.16 (d, *J* = 16.3 Hz, 1H), 2.70 (d, *J* = 16.5 Hz, 1H), 2.63-2.38 (m, 2H), 2.28 (d, *J =* 14.6 Hz, 1H), 2.10 (d, *J* = 15.2 Hz, 1H), 1.45 (s, 3H), 1.37 (s, 3H).

**Step 13:** To a solution of sodium methanolate (1424 mg, 7.91 mmol) in MeOH (0.5 mL) was added a solution of 1'-(4-methoxybenzyl)-7,7-dimethyl-6,6',7,1'-tetrahydro-1'*H*,4*H-*spiro[benzo[*d*] isoxazole-5,4'-pyrano[3,4-*d*]imidazole] (200 mg, 0.527 mmol) in Et₂O (2 mL). The resulted mixture was stirred for 2 h at room temperature. The reaction solution was quenched by water (5 mL), diluted with ethyl acetate (20 mL), washed with brine (6 mL × 2), dried over anhydrous sodium sulfate and filtered. The filtrate was concentrated under reduced pressure. The residue was purified by Prep-TLC with CH₂Cl₂ / MeOH (20 : 1) to afford 1'-(4-methoxybenzyl)-3,3-dimethyl-4-oxo-6',7'-dihydro-1'*H*-spiro[cyclohexane-1,4'-pyrano[3,4-*d*]imidazole]-5-carbonitrile (140 mg, 0.343 mmol, 65.1% yield) as a yellow solid. MS ESI calculated for C₂₂H₂₅N₃O₃ [M + H]⁺ 380.19 found 380.25. ¹H NMR (300 MHz, Chloroform-d) δ 7.55 (s, 1H), 7.07 (d, *J* = 8.3 Hz, 2H), 6.90 (d, *J* = 8.2 Hz, 2H), 4.99 (s, 2H), 4.36 (d, *J* = 13.7 Hz, 1H), 3.98 (s, 2H), 3.81 (d, *J* = 1.7 Hz, 3H), 2.86-1.92 (m, 6H), 1.38 (s, 3H), 1.14 (s, 3H).

**Step 14:** To a solution of pyridine (58.4 mg, 0.738 mmol) in DCM (0.5 mL) was added phenylselenyl chloride (141 mg, 0.738 mmol) in DCM (0.5 mL) at 0 °C. The reaction was degassed with oxygen. Then a solution of 1'-(4-methoxybenzyl)-3,3-dimethyl-4-oxo-6',7'-dihydro-1'*H*-spiro[cyclohexane-1,4'-pyrano[3,4-*d*]imidazole]-5-carbonitrile (140 mg, 0.369 mmol) in DCM (0.5 mL) was added. The resulted mixture was stirred for 20 h at room temperature. The reaction mixture was quenched by saturated NH₄Cl (2 mL), extracted with ethyl acetate (10 mL × 3). The combined organic layers was washed with brine (6 mL), dried over anhydrous sodium sulfate and filtered. The filtrate was concentrated under reduced pressure. The residue was purified by Prep-TLC with CH₂Cl₂ / MeOH (15 : 1) to afford 1'-(4-methoxybenzyl)-5,5-dimethyl-4-oxo-6',1'-dihydro-1'*H*-spiro[cyclohexane-1,4'-pyrano[3,4-*d*]imidazol]-2-ene-3-carbonitrile (40 mg, 0.106 mmol, 28.7% yield) as a yellow solid which was further purified by Prep-HPLC with the following conditions: Column: XBridge Prep C¹⁸ OBD Column, 19 × 150 mm, 5µm; Mobile Phase A: Water (10 mM ammonium acetate), Mobile Phase B: MeCN; Flow rate: 20 mL / min; Gradient: 40% B to 70% B in 4.5 min, 70 % B; Wave Length: 254 / 210 nm; RT₁ (min): 4.35; Number Of Runs: 0. The fraction combined and concentrated under reduced pressure, lyophilized to give 1'-(4-methoxybenzyl)-5,5-dimethyl-4-oxo-6',7'-dihydro-1'*H*-spiro[cyclohexane-1,4'-pyrano[3,4-*d*]imidazol]-2-ene-3-carbonitrile (5.7 mg, 0.015 mmol, 37.2% yield) as an off-white solid. MS ESI calculated for C₂₂H₂₃N₃O₃ [M + H]⁺ 378.17 found 378.20. ¹H NMR (300 MHz, Chloroform-*d*) δ 7.51 (s, 1H), 7.33 (s, 1H), 7.10 (d, *J =* 8.2 Hz, 2H), 6.93 (d, *J* = 8.2 Hz, 2H), 5.02 (s, 2H), 4.11-3.90 (m, 2H), 3.84 (s, 3H), 2.65-2.54 (m, 3H), 2.17 (d, *J =* 15.1 Hz, 1H), 1.39 (s, 3H), 1.24 (s, 3H).

### Example 129, enantiomer 1: 12-oxo-2-phenyl-2-azadispiro[4.1.4⁷.3⁵]tetradec-13-ene-13-carbonitrile

### Example 130, enantiomer 2: 12-oxo-2-phenyl-2-azadispiro[4.1.4⁷.3⁵]tetradec-13-ene-13-carbonitrile

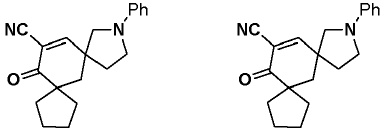

**Step 1:** To a solution of 2-phenyl-2-azadispiro[4.1.4⁷.3⁵]tetradecan-12-one (230 mg, 0.812 mmol) in THF (4 mL) was added dropwise LiHMDS (1 M solution in THF, 1.623 mL, 1.623 mmol) at -70 °C under N₂ atmosphere. The resulting mixture was stirred at -70 °C for 40 min. Then a solution of 4-methylbenzenesulfonyl cyanide (221 mg, 1.217 mmol) in THF (1.5 mL) was added. After stirring at -70 °C for 30 min, the mixture was warmed to room temperature and stirred for 2 h. Then the mixture was quenched with saturated aqueous NH₄Cl (15 mL), extracted with ethyl acetate (15 mL × 3). The combined organic phase was washed with brine (20 mL), dried over anhydrous sodium sulfate. After filtration, the filtrate was concentrated under reduced pressure and the residue was purified by preparative TLC (Petroleum ehter : EtOAc = 8 : 1) to give 12-oxo-2-phenyl-2-azadispiro[4.1.4⁷.3⁵]tetradecane-13-carbonitrile (130 mg, 0.421 mmol, 51.9% yield) as a yellow solid. MS ESI calculated for C₂₀H₂₄N₂O [M + H]⁺ 309.19, found 309.15. ¹H NMR (300 MHz, Chloroform-*d*): δ 7.41-7.16 (m, 2H), 6.83-6.66 (m, 1H), 6.64-6.51 (m, 2H), 4.05-3.84 (m, 3H), 3.67-3.27 (m, 2H), 2.57-2.36 (m, 2H), 2.31-1.50 (m, 12H).

**Step 2:** To a solution of 12-oxo-2-phenyl-2-azadispiro[4.1.4⁷.3⁵]tetradecane-13-carbonitrile (100 mg, 0.324 mmol) in THF (3.5 mL) was added diacetoxypalladium (58.2 mg, 0.259 mmol) at room temperature. The mixture was stirred at room temperature for 16 h. The mixture (three batches combined, ~ 0.486 mmol starting material) was purified by reverse phase chromatography with the following conditions: Column: C¹⁸ gel column, 40 g, 20-45 um, 100 A; Mobile Phase A: water, Mobile Phase B: MeCN; Gradient: 0% B hold 5 min, up to 95% B within 30 min, 95%B hold 5 min; Flow rate: 40 mL/min; RT = 25.5 min; Detector: UV 254 & 210 nm. The product-containing fractions were collected and evaporated to afford 12-oxo-2-phenyl-2-azadispiro[4.1.47.35]tetradec-13-ene-13-carbonitrile (62 mg, 0.202 mmol, 41.6% yield) as a yellow solid. MS ESI calculated for C₂₀H₂₂N₂O [M + H]⁺ 307.17, found 307.20. ¹H NMR (400 MHz, Chloroform-*d*): δ 7.52 (s, 1H), 7.35-7.23 (m, 2H), 6.80 (m, 1H), 6.66-6.58 (m, 2H), 3.73-3.29 (m, 4H), 2.40-1.38 (m, 12H).

Racemic 12-Oxo-2-phenyl-2-azadispiro[4.1.47.35]tetradec-13-ene-13-carbonitrile (62 mg, 0.202 mmol) was separated by Prep-Chiral-HPLC with the following conditions: Column: CHIRAL ART Cellulose-SB, 2 × 25 cm, 5 um; Mobile Phase A:
Hexanes , Mobile Phase B: EtOH; Flow rate: 20 mL/min; Gradient: 50% B to 50% B in 20 min; Detector: UV 220/254 nm; RT₁:13.879 min; RT₂:18.324 min;
The fractions of the first peak (RT₁: 13.879 min) were combined and concentrated under reduced pressure, lyophilized to give the faster enantiomer (12 mg, 0.039 mmol, 19.35 % yield) as a yellow solid which was further purified by Prep-HPLC with the following conditions: Column: SunFire Prep C¹⁸ OBD Column, 19 × 150 mm, 5 um, 10 nm; Mobile Phase A: Water (0.05% TFA), Mobile Phase B: MeCN; Flow rate: 20 mL/min; Gradient: 20% B to 50% B in 6 min; Detector: UV 210/254 nm. The product-containing fractions were combined and concentrated under reduced pressure, lyophilized to give 12-oxo-2-phenyl-2-azadispiro[4.1.47.35]tetradec-13-ene-13-carbonitrile - **enantiomer 1:** 12-oxo-2-phenyl-2-azadispiro[4.1.47.35]tetradec-13-ene-13-carbonitrile (3.4 mg, 0.011 mmol, 28.3% yield) as a yellow solid. MS ESI calculated for C₂₀H₂₂N₂O [M + MeCN + H]⁺ 348.17, found 348.30. ¹H NMR (300 MHz, DMSO-*d₆*): δ 7.91 (s, 1H), 7.24-7.11 (m, 2H), 6.69-6.48 (m, 3H), 3.63-3.43 (m, 2H), 3.42-3.20 (m, 2H), 2.29-2.18 (m, 1H), 2.17-2.09 (m, 3H), 2.06-1.95 (m, 2H), 1.71-1.55 (m, 4H), 1.53-1.42 (m, 2H).

The fractions of the second peak (RT₂: 18.324 min) were combined and concentrated under reduced pressure, lyophilized to give the slower enantiomer (15 mg, 0.049 mmol, 24.19% yield) as a yellow solid which was further purified by Prep-HPLC with the following conditions: Column: SunFire Prep C¹⁸ OBD Column, 19 × 150 mm, 5 um, 10 nm; Mobile Phase A: Water (0.05% TFA), Mobile Phase B: MeCN; Flow rate: 20 mL/min; Gradient: 40% B to 65% B in 6 min; Detector: UV 210/254 nm. The product-containing fractions were combined and concentrated under reduced pressure, lyophilized to give 12-oxo-2-phenyl-2-azadispiro[4.1.47.35]tetradec-13-ene-13-carbonitrile - **enantiomer 2:** 12-oxo-2-phenyl-2-azadispiro[4.1.47.35]tetradec-13-ene-13-carbonitrile (4.8 mg, 0.016 mmol, 32.0% yield) as a yellow solid. MS ESI calculated for C₂₀H₂₂N₂O [M + MeCN + H]⁺ 348.17, found 348.30. ¹H NMR (300 MHz, DMSO-*d₆*): δ 7.91 (s, 1H), 7.24-7.11 (m, 2H), 6.66-6.51 (m, 3H), 3.57-3.20 (m, 4H), 2.29-2.18 (m, 1H), 2.17-2.09 (m, 3H), 2.08-1.97 (m, 2H), 1.74-1.55 (m, 4H), 1.53-1.37 (m, 2H).

**Table 10: The following examples were prepared using a similar procedure as described above for examples 129 and 130**

| **Ex** | **Structure** | **Compound Name** | **Exact Mass** |
|---|---|---|---|
| 131 | 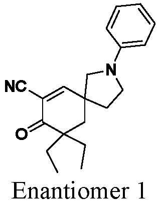 | 9,9-diethyl-8-oxo-2-phenyl-2-azaspiro[4.5]dec-6-ene-7-carbonitrile | 309 **[M+H]⁺** |
| 132 | 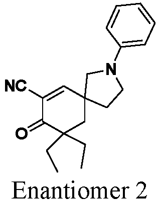 | 9,9-diethyl-8-oxo-2-phenyl-2-azaspiro[4.5]dec-6-ene-7-carbonitrile | 309 **[M+H]⁺** |
| 133 | 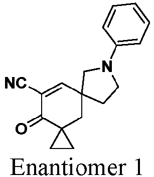 | 12-oxo-7-phenyl-7-azadispiro[2.1.4⁵.3³]dodec-10-ene-11-carbonitrile | 279 **[M+H]⁺** |
| 134 | 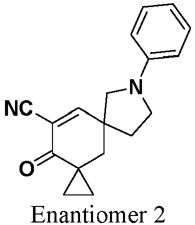 | 12-oxo-7-phenyl-7-azadispiro[2.1.4⁵.3³]dodec-10-ene-11-carbonitrile | 279 **[M+H]⁺** |
| 135 | 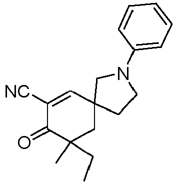 | 9-ethyl-9-methyl-8-oxo-2-phenyl-2-azaspiro[4.5]dec-6-ene-7-carbonitrile | 336 **[M+MeCN+H]⁺** |
| 136 | 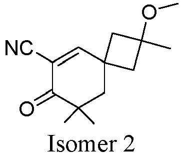 | 2-Methoxy-2,8,8-trimethyl-7-oxospiro[3.5]non-5-ene-6-carbonitrile | 234 **[M+H]⁺** |

### Example 137, isomer 1: 8,8-dimethyl-2-(methyl(phenyl)amino)-7-oxospiro[3.5]non-5-ene-6-carbonitrile

### Example 138, isomer 2: 8,8-dimethyl-2-(methyl(phenyl)amino)-7-oxospiro[3.5]non-5-ene-6-carbonitrile

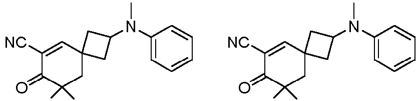

**Step 1:** To a solution of *N*,7,7-trimethyl-*N*-phenyl-6,7-dihydro-4*H*-spiro[benzo[*d*]isoxazole-5,1'-cyclobutan]-3'-amine (70 mg, 0.236 mmol) in Et₂O (1 mL) was added Sodium methylate (30% in Methy alcohol, 638 mg, 3.54 mmol). The resulted mixture was stirred at 30 °C for 2 h. The reaction was quenched by addition of aq. NH4Cl (10 mL), diluted with ethyl acetate (3 × 30 mL), and the organic layer was washed with water (60 mL) and brine (60 mL). The organic layer was dried over anhydrous Na₂SO₄, filtered and the filtrate was concentrated under reduced pressure. The crude material was purified by silica gel column chromatography eluting with 0 - 30% of Ethyl acetate in Petroleum ether to afford 8,8-dimethyl-2-(methyl(phenyl)amino)-7-oxospiro[3.5]nonane-6-carbonitrile (60 mg, 0.192 mmol, 81% yield) as a white solid. MS ESI calculated for C₁₉H₂₄N₂O [M + H]⁺ 297.19 found 297.30. ¹H NMR (300 MHz, Methanol-*d*₄) δ 7.27-7.14 (m, 2H), 6.92-6.74 (m, 3H), 3.88 (tq, *J* = 15.0, 7.9 Hz, 1H), 2.82-2.72 (m, 3H), 2.72-2.59 (m, 1H), 2.54-1.68 (m, 8H), 1.32-1.01 (m, 6H).

**Step 2:** To a solution of 8,8-dimethyl-2-(methyl(phenyl)amino)-7-oxospiro[3.5]nonane-6-carbonitrile (60 mg, 0.202 mmol) in THF (0.6 mL) was added palladium (II) acetate (36.32 mg, 0.162 mmol). The resulted mixture was stirred at room temperature for 3 h. The reaction progress was monitored by LCMS. After completion of the reaction, the reaction mixture was diluted by ethyl acetate (30 mL), filtered and the filtrate was concentrated under reduced pressure. The residue was purified by RP-flash chromatography eluting with 0 - 50% acetonitrile in water to afford 8,8-dimethyl-2-(methyl(phenyl)amino)-7-oxospiro[3.5]non-5-ene-6-carbonitrile (30 mg, 0.102 mmol, 50.3% yield) as a colorless oil. MS ESI calculated for C₁₉H₂₂N₂O [M + H]⁺ 295.17 found 295.25.

The cis/trans product mixture was separated by Prep-Chiral-HPLC with the following conditions: Column: CHIRAL ART Cellulose-SB, 2 × 25 cm, 5 µm; Mobile Phase A: Hex-HPLC, Mobile Phase B: EtOH--HPLC; Flow rate: 20 mL/min; Gradient: 30% B to 30% B in 19.5 min; Wave Length: 254/220 nm; RT1: 11.37 min; RT2: 15.43 min; Sample Solvent: EtOH- -HPLC; Injection Volume: 1 mL; Number Of Runs: 2.

The fractions containing the faster isomer (RT = 11.37 min) were combined and concentrated under reduced pressure, then freeze-dried to give **isomer 1:** 8,8-dimethyl-2-(methyl(phenyl)amino)-7-oxospiro[3.5]non-5-ene-6-carbonitrile (4 mg, 0.014 mmol, 6.71% yield) as an off-white solid. MS ESI calculated for C₁₉H₂₂N₂O [M + H]⁺ 295.17 found 295.15. ¹H NMR (300 MHz, Chloroform-*d*) δ 7.79 (s, 1H), 7.39-7.18 (m, 2H), 6.99-6.72 (m, 3H), 4.09 (t, *J* = 7.8 Hz, 1H), 2.82 (s, 3H), 2.64 (dd, *J* = 5.7 Hz, 2H), 2.42-2.26 (m, 2H), 2.00 (s, 2H), 1.13 (s, 6H).

The fractions containing the slower isomer (RT = 15.43 min) were combined and concentrated under reduced pressure, then freeze-dried to give **isomer 2:** 8,8-dimethyl-2-(methyl(phenyl)amino)-7-oxospiro[3.5]non-5-ene-6-carbonitrile (14.5 mg, 0.049 mmol, 24.3% yield) as an off-white solid. MS ESI calculated for C₁₉H₂₂N₂O [M + H]⁺ 295.17 found 295.20. ¹H NMR (400 MHz, Chloroform-*d*) δ 7.53 (d, *J* = 12 Hz, 1H), 7.37-7.15 (m, 2H), 6.91-6.74 (m, 3H), 4.00-4.07 (m, 1H), 2.83 (s, 3H), 2.51 (t, *J* = 10.8 Hz, 2H), 2.57-2.47 (m, 2H), 2.43-2.30 (m, 2H), 1.18 (s, 6H).

**Table 11: The following example was prepared using a similar procedure as described above for examples 137 and 138.**

| **Ex** | **Structure** | **Compound Name** | **Exact Mass [M+H]⁺** |
|---|---|---|---|
| 139 | 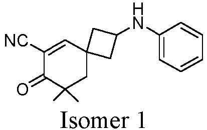 | 8,8-dimethyl-7-oxo-2-(phenylamino)spiro[3.5]non-5-ene-6-carbonitrile | 281 |

### Example 140 - enantiomer 1: 4-acetyl-10,10-dimethyl-9-oxo-1-oxa-4-azaspiro[5.5]undec-7-ene-8-carbonitrile

### Example 141 - enantiomer 2: 4-acetyl-10,10-dimethyl-9-oxo-1-oxa-4-azaspiro[5.5]undec-7-ene-8-carbonitrile

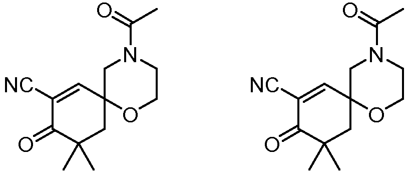

**Step 1:** Triethylamine (0.78 mL, 5.6 mmol) and acetyl chloride (0.20 mL, 2.8 mmol) were added to a solution of 10,10-dimethyl-9-oxo-1-oxa-4-azaspiro[5.5]undec-7-ene-8-carbonitrile, HCl (310 mg, 1.41 mmol) in DCM (10 mL). The reaction mixture was stirred overnight at room temperature. The reaction mixture was quenched with aqueous HCl (1 M, 5 mL) and extracted with DCM (3x). The combined organic layers were dried over anhydrous sodium sulfate, filtered, and concentrated under reduced pressure. The residue was purified by mass triggered reverse phase HPLC (ACN/H₂O with 0.1% TFA modifier) to afford 4-acetyl-10,10-dimethyl-9-oxo-1-oxa-4-azaspiro[5.5]undec-7-ene-8-carbonitrile. The racemic mixture was purified by chiral SFC (IA column, 20%/80% MeOH/CO₂ with 0.1% NH₄OH modifier) to afford two products as solids:
4-acetyl-10,10-dimethyl-9-oxo-1-oxa-4-azaspiro[5.5]undec-7-ene-8-carbonitrile (Example 36 - enantiomer 1). MS: 263 (M + H). ¹H NMR (600 MHz, CDCl₃) δ 7.59-7.50 (m, 1H), 4.15-4.01 (m, 1H), 3.81-3.66 (m, 2H), 3.50-3.36 (m, 2H), 3.29-3.18 (m, 1H), 2.19-2.10 (m, 3H), 2.07-2.01 (m, 1H), 1.91-1.79 (m, 1H), 1.33-1.20 (m, 6H).

4-acetyl-10,10-dimethyl-9-oxo-1-oxa-4-azaspiro[5.5]undec-7-ene-8-carbonitrile (Example 37 - enantiomer 2). MS: 263 (M + H). ¹H NMR (600 MHz, CDCl₃) δ 7.59-7.49 (m, 1H), 4.15-4.01 (m, 1H), 3.81-3.66 (m, 2H), 3.50-3.35 (m, 2H), 3.27-3.18 (m, 1H), 2.19-2.10 (m, 3H), 2.07-2.04 (m, 1H), 1.91-1.79 (m, 1H), 1.33-1.20 (m, 6H).

**Table 12: The following examples were prepared using a similar procedure as described above for examples 140 and 141.**

| **Ex** | **Structure** | **Compound Name** | **Exact Mass [M+H]⁺** |
|---|---|---|---|
| 142 | 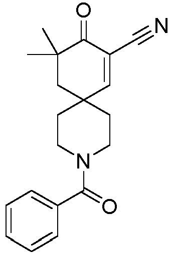 | 3-(benzenecarbonyl)-10,10-dimethyl-9-oxo-3-azaspiro[5.5]undec-7-ene-8-carbonitrile | 323 |
| 143 | 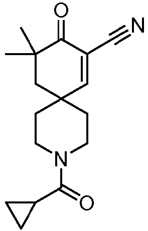 | 3-(cyclopropanecarbonyl)-10,10-dimethyl-9-oxo-3-azaspiro[5.5]undec-7-ene-8-carbonitrile | 287 |
| 144 | 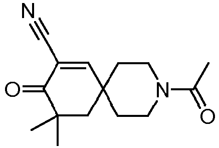 | 3-acetyl-10,10-dimethyl-9-oxo-3-azaspiro[5.5]undec-7-ene-8-carbonitrile | 261 |
| 145 | 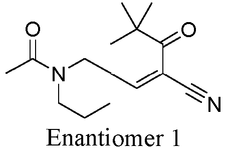 | 2-acetyl-10,10-dimethyl-9-oxo-2-azaspiro[5.5]undec-7-ene-8-carbonitrile | 261 |
| 146 | 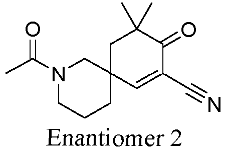 | 2-acetyl-10,10-dimethyl-9-oxo-2-azaspiro[5.5]undec-7-ene-8-carbonitrile | 261 |
| 147 | 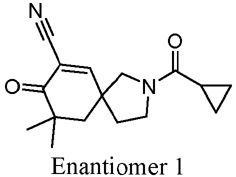 | 2-(cyclopropanecarbonyl)-9,9-dimethyl-8-oxo-2-azaspiro[4.5]dec-6-ene-7-carbonitrile | 273 |
| 148 | 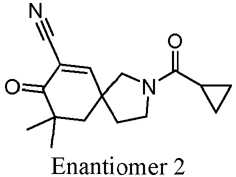 | 2-(cyclopropanecarbonyl)-9,9-dimethyl-8-oxo-2-azaspiro[4.5]dec-6-ene-7-carbonitrile | 273 |
| 149 | 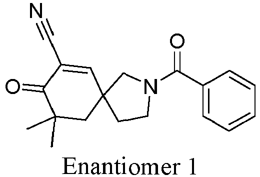 | 2-(benzenecarbonyl)-9,9-dimethyl-8-oxo-2-azaspiro[4.5]dec-6-ene-7-carbonitrile | 309 |
| 150 | 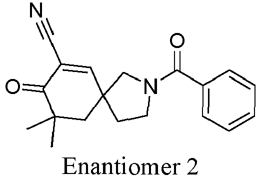 | 2-(benzenecarbonyl)-9,9-dimethyl-8-oxo-2-azaspiro[4.5]dec-6-ene-7-carbonitrile | 309 |
| 151 | 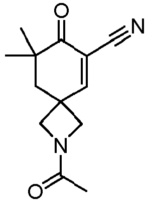 | 2-acetyl-8,8-dimethyl-7-oxo-2-azaspiro[3.5]non-5-ene-6-carbonitrile | 233 |
| 152 | 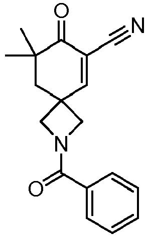 | 2-(benzenecarbonyl)-8,8-dimethyl-7-oxo-2-azaspiro[3.5]non-5-ene-6-carbonitrile | 295 |
| 153 | 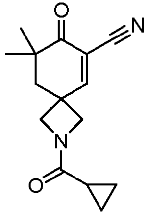 | 2-(cyclopropanecarbonyl)-8,8-dimethyl-7-oxo-2-azaspiro[3.5]non-5-ene-6-carbonitrile | 259 |
| 154 | 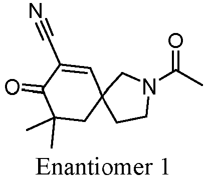 | 2-acetyl-9,9-dimethyl-8-oxo-2-azaspiro[4.5]dec-6-ene-7-carbonitrile | 247 |
| 155 | 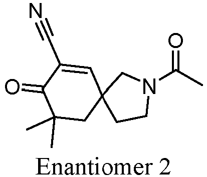 | 2-acetyl-9,9-dimethyl-8-oxo-2-azaspiro[4.5]dec-6-ene-7-carbonitrile | 247 |
| 156 | 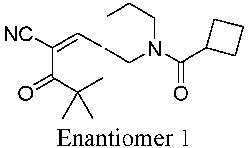 | 2-(cyclobutanecarbonyl)-10,10-dimethyl-9-oxo-2-azaspiro[5.5]undec-7-ene-8-carbonitrile | 301 |
| 157 | 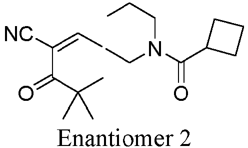 | 2-(cyclobutanecarbonyl)-10,10-dimethyl-9-oxo-2-azaspiro[5.5]undec-7-ene-8-carbonitrile | 301 |
| 158 | 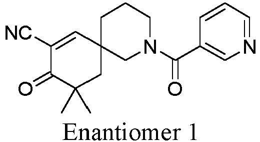 | 10,10-dimethyl-2-nicotinoyl-9-oxo-2-azaspiro[5.5]undec-7-ene-8-carbonitrile | 324 |
| 159 | 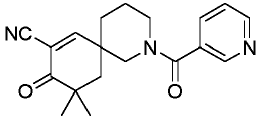 | 10,10-dimethyl-2-nicotinoyl-9-oxo-2-azaspiro[5.5]undec-7-ene-8-carbonitrile | 324 |
| | 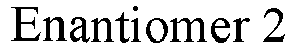 | | |
| 160* | 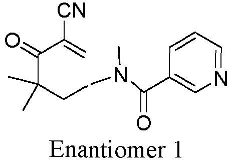 | *N*-(3-cyano-1,5,5-trimethyl-4-oxocyclohex-2-en-1-yl)-*N-*methylnicotinamide | 298 |
| 161* | 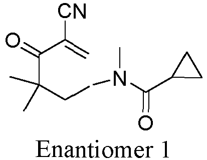 | *N*-(3-cyano-1,5,5-trimethyl-4-oxocyclohex-2-en-1-yl)-*N-*methylcyclopropanecarboxamide | 261 |
| 162* | 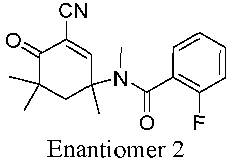 | *N*-(3-cyano-1,5,5-trimethyl-4-oxocyclohex-2-en-1-yl)-2-fluoro-*N*-methylbenzamide | 315 |
| 163* | 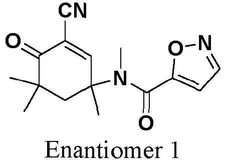 | *N*-(3-cyano-1,5,5-trimethyl-4-oxocyclohex-2-en-1-yl)-*N-*methylisoxazole-5-carboxamide | 288 |
| 164* | 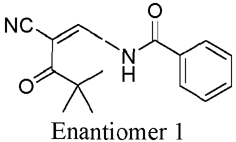 | *N*-(3-cyano-1,5,5-trimethyl-4-oxocyclohex-2-en-1-yl)benzamide | 283 |
| 165* | 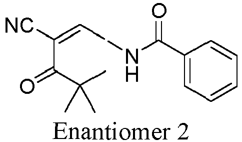 | *N*-(3-cyano-1,5,5-trimethyl-4-oxocyclohex-2-en-1-yl)benzamide | 283 |
| 166* | 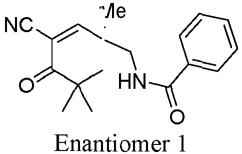 | *N*-((3-cyano-1,5,5-trimethyl-4-oxocyclohex-2-en-1-yl)methyl)benzamide | 297 |

| | | | |
|---|---|---|---|
| * reference compound | | | |

### General Procedures for Table 13:

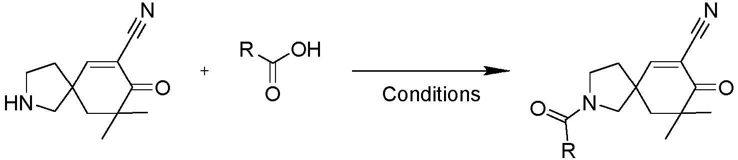

### Method A: EDCI, Pyridine, 50 °C, 16 h

An 8 mL vial was charged with 9,9-dimethyl-8-oxo-2-azaspiro[4.5]dec-6-ene-7-carbonitrile (10.2 mg, 50 µmol), carboxylic acid (75 µmol), EDCI (14.4 mg, 75 µmol) and pyridine (500 µL). The vial was capped and shaken at 50 °C for 16 h. The reaction was monitored by LCMS. After the reaction was completed, the reaction mixture was concentrated by Speedvac. The resulting residue was dissolved in MeCN and H₂O and purified by preparative HPLC to afford pure final product.

### Method B: T₃P, Dioxane, TEA, 80 °C, 16 h

To an 8 mL vial charged with 9,9-dimethyl-8-oxo-2-azaspiro[4.5]dec-6-ene-7-carbonitrile (10.2 mg, 50 µmol) and carboxylic acid (75.0 µmol) was added Dioxane (500 µL), triethylamine (20.2 mg, 200 µmol) and 1-propanephosphonic anhydride (23.9 mg, 75 µmol, 50% wt in EtOAc). The vial was capped and shaken at 80 °C for 16 h. The reaction was monitored by LCMS. The reaction mixture was concentrated and extracted with DCM (1 mL*3) and water (1 mL). The organic layers were dried by anhydrous Na₂SO₄ and concentrated by Speedvac to afford crude intermediates. The resulting residue was dissolved in MeCN and H₂O and purified by preparative HPLC to afford pure final product.

**Table 13: Compounds were prepared in a similar manner to the general procedure**

| **Ex.** | **Enant iomer** | **Structure** | **IUPAC Name** | **Exact Mass [M+H]** + | **Meth od** |
|---|---|---|---|---|---|
| 167 | 1 | 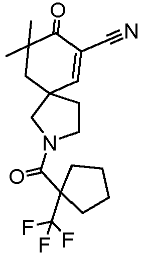 | 9,9-dimethyl-8-oxo-2-[1-(trifluoromethyl)cyclope ntane-1-carbonyl]-2-azaspiro[4.5]dec-6-ene-7-carbonitrile | 369.0 | A |
| 168 | 1 | 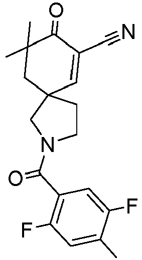 | 2-(2,5-difluoro-4-methylbenzene-1-carbonyl)-9,9-dimethyl-8-oxo-2-azaspiro[4.5]dec-6-ene-7-carbonitrile | 359.0 | A |
| 169 | 1 | 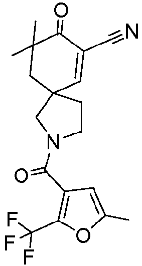 | 9,9-dimethyl-2-[5-methyl-2-(trifluoromethyl)furan-3-carbonyl]-8-oxo-2-azaspiro[4.5]dec-6-ene-7-carbonitrile | 381.0 | A |
| 170 | 1 | 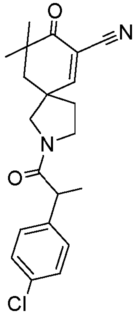 | 2-[2-(4-chlorophenyl)propanoyl] -9,9-dimethyl-8-oxo-2-azaspiro[4.5]dec-6-ene-7-carbonitrile | 371.0 | A |
| 171 | 1 | 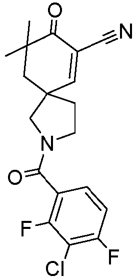 | 2-(3-chloro-2,4-difluorobenzene-1-carbonyl)-9,9-dimethyl-8-oxo-2-azaspiro[4.5]dec-6-ene-7-carbonitrile | 379.0 | A |
| 172 | 1 | 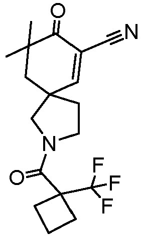 | 9,9-dimethyl-8-oxo-2-[1-(trifluoromethyl)cyclobut ane-1-carbonyl]-2-azaspiro[4.5]dec-6-ene-7-carbonitrile | 355.1 | A |
| 173 | 1 | 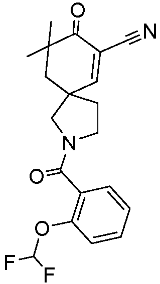 | 2-[2-(difluoromethoxy)benzen e-1-carbonyl]-9,9-dimethyl-8-oxo-2-azaspiro[4.5]dec-6-ene-7-carbonitrile | 375.0 | A |
| 174 | 1 | 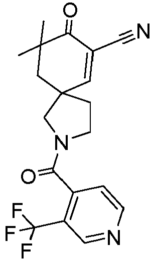 | 9,9-dimethyl-8-oxo-2-[3-(trifluoromethyl)pyridine -4-carbonyl]-2-azaspiro[4.5]dec-6-ene-7-carbonitrile | 378.0 | B |
| 175 | 1 | 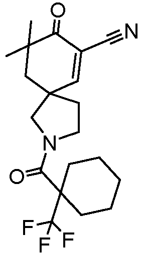 | 9,9-dimethyl-8-oxo-2-[1-(trifluoromethyl)cyclohe xane-1-carbonyl]-2-azaspiro[4.5]dec-6-ene-7-carbonitrile | 383.1 | A |
| 176 | 1 | 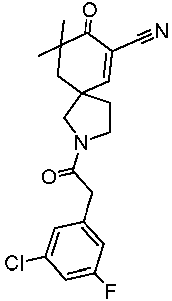 | 2-[(3-chloro-5-fluorophenyl)acetyl]-9,9-dimethyl-8-oxo-2-azaspiro[4.5]dec-6-ene-7-carbonitrile | 375.0 | A |
| 177 | 1 | 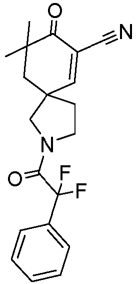 | 2-[difluoro(phenyl)acetyl]-9,9-dimethyl-8-oxo-2-azaspiro[4.5]dec-6-ene-7-carbonitrile | 359.0 | A |
| 178 | 1 | 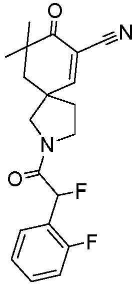 | 2-[fluoro(2-fluorophenyl)acetyl]-9,9-dimethyl-8-oxo-2-azaspiro[4.5]dec-6-ene-7-carbonitrile | 359.0 | A |
| 179 | 1 | 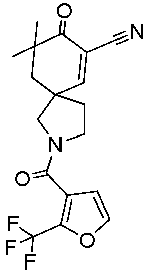 | 9,9-dimethyl-8-oxo-2-[2-(trifluoromethyl)furan-3-carbonyl]-2-azaspiro[4.5]dec-6-ene-7-carbonitrile | 367.0 | A |
| 180 | 1 | 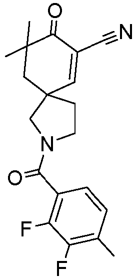 | 2-(2,3-difluoro-4-methylbenzene-1-carbonyl)-9,9-dimethyl-8-oxo-2-azaspiro[4.5]dec-6-ene-7-carbonitrile | 359.0 | A |
| 181 | 1 | 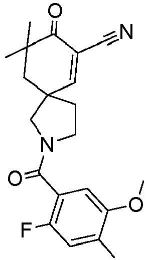 | 2-(2-fluoro-5-methoxy-4-methylbenzene-1-carbonyl)-9,9-dimethyl-8-oxo-2-azaspiro[4.5]dec-6-ene-7-carbonitrile | 371.0 | A |
| 182 | 1 | 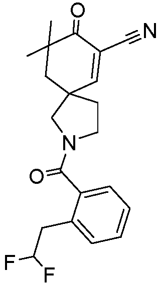 | 2-[2-(2,2-difluoroethyl)benzene-1-carbonyl]-9,9-dimethyl-8-oxo-2-azaspiro[4.5]dec-6-ene-7-carbonitrile | 373.1 | A |
| 183 | 2 | 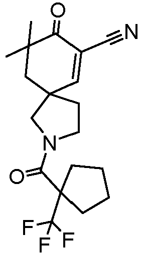 | 9,9-dimethyl-8-oxo-2-[1-(trifluoromethyl)cyclope ntane-1-carbonyl]-2-azaspiro[4.5]dec-6-ene-7-carbonitrile | 369.0 | A |
| 184 | 2 | 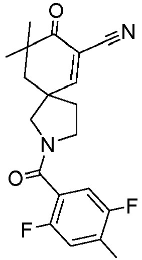 | 2-(2,5-difluoro-4-methylbenzene-1-carbonyl)-9,9-dimethyl-8-oxo-2-azaspiro[4.5]dec-6-ene-7-carbonitrile | 359.0 | A |
| 185 | 2 | 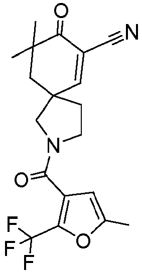 | 9,9-dimethyl-2-[5-methyl-2-(trifluoromethyl)furan-3-carbonyl]-8-oxo-2-azaspiro[4.5]dec-6-ene-7-carbonitrile | 381.0 | A |
| 186 | 2 | 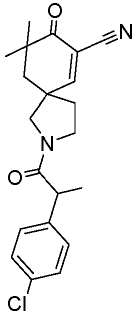 | 2-[2-(4-chlorophenyl)propanoyl] -9,9-dimethyl-8-oxo-2-azaspiro[4.5]dec-6-ene-7-carbonitrile | 371.0 | A |
| 187 | 2 | 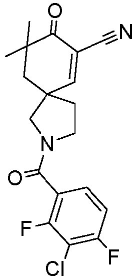 | 2-(3-chloro-2,4-difluorobenzene-1-carbonyl)-9,9-dimethyl-8-oxo-2-azaspiro[4.5]dec-6-ene-7-carbonitrile | 379.0 | A |
| 188 | 2 | 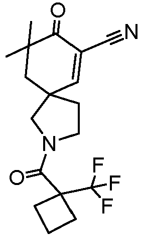 | 9,9-dimethyl-8-oxo-2-[1-(trifluoromethyl)cyclobut ane-1-carbonyl]-2-azaspiro[4.5]dec-6-ene-7-carbonitrile | 355.0 | A |
| 189 | 2 | 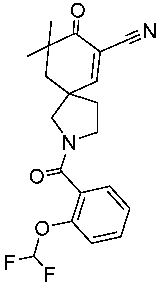 | 2-[2-(difluoromethoxy)benzen e-1-carbonyl]-9,9-dimethyl-8-oxo-2-azaspiro[4.5]dec-6-ene-7-carbonitrile | 375.1 | A |
| 190 | 2 | 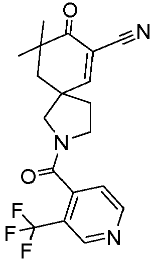 | 9,9-dimethyl-8-oxo-2-[3-(trifluoromethyl)pyridine -4-carbonyl]-2-azaspiro[4.5]dec-6-ene-7-carbonitrile | 378.0 | B |
| 191 | 2 | 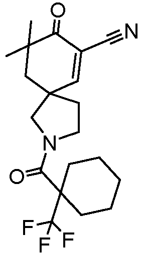 | 9,9-dimethyl-8-oxo-2-[1-(trifluoromethyl)cyclohe xane-1-carbonyl]-2-azaspiro[4.5]dec-6-ene-7-carbonitrile | 383.1 | A |
| 192 | 2 | 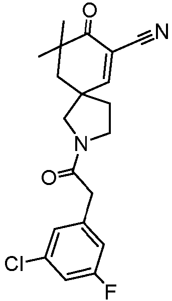 | 2-[(3-chloro-5-fluorophenyl)acetyl]-9,9-dimethyl-8-oxo-2-azaspiro[4.5]dec-6-ene-7-carbonitrile | 375.0 | A |
| 193 | 2 | 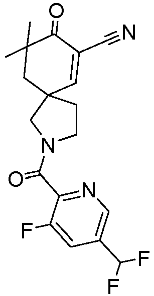 | 2-[5-(difluoromethyl)-3-fluoropyridine-2-carbonyl]-9,9-dimethyl-8-oxo-2-azaspiro[4.5]dec-6-ene-7-carbonitrile | 378.0 | A |
| 194 | 2 | 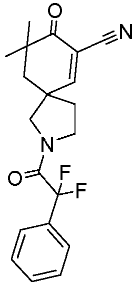 | 2-[difluoro(phenyl)acetyl]-9,9-dimethyl-8-oxo-2-azaspiro[4.5]dec-6-ene-7-carbonitrile | 359.0 | A |
| 195 | 2 | 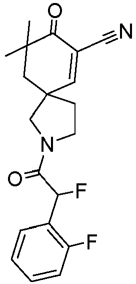 | 2-[fluoro(2-fluorophenyl)acetyl]-9,9-dimethyl-8-oxo-2-azaspiro[4.5]dec-6-ene-7-carbonitrile | 359.1 | A |
| 196 | 2 | 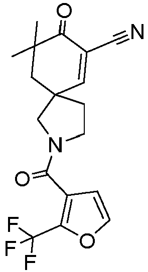 | 9,9-dimethyl-8-oxo-2-[2-(trifluoromethyl)furan-3-carbonyl]-2-azaspiro[4.5]dec-6-ene-7-carbonitrile | 367.0 | A |
| 197 | 2 | 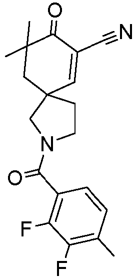 | 2-(2,3-difluoro-4-methylbenzene-1-carbonyl)-9,9-dimethyl-8-oxo-2-azaspiro[4.5]dec-6-ene-7-carbonitrile | 359.0 | A |
| 198 | 2 | 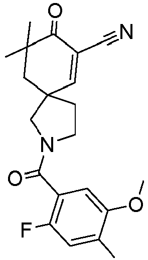 | 2-(2-fluoro-5-methoxy-4-methylbenzene-1-carbonyl)-9,9-dimethyl-8-oxo-2-azaspiro[4.5]dec-6-ene-7-carbonitrile | 371.1 | A |
| 199 | 2 | 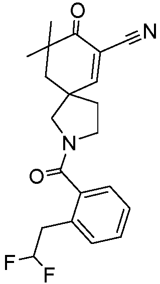 | 2-[2-(2,2-difluoroethyl)benzene-1-carbonyl]-9,9-dimethyl-8-oxo-2-azaspiro[4.5]dec-6-ene-7-carbonitrile | 373.0 | A |
| 200 | 1 | 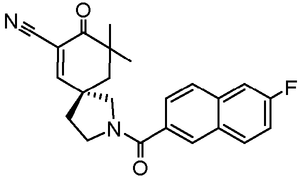 | (5R)-2-(6-fluoronaphthalene-2-carbonyl)-9,9-dimethyl-8-oxo-2-azaspiro[4.5]dec-6-ene-7-carbonitrile | 377.0 | A |
| 201 | 1 | 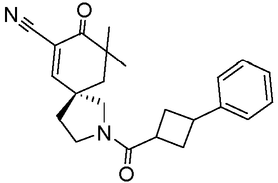 | (5R)-9,9-dimethyl-8-oxo-2-(3-phenylcyclobutane-1-carbonyl)-2-azaspiro[4.5]dec-6-ene-7-carbonitrile | 363.0 | A |
| 202 | 1 | 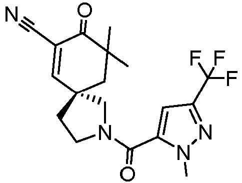 | (5R)-9,9-dimethyl-2-[1-methyl-3-(trifluoromethyl)-1H-pyrazole-5-carbonyl]-8-oxo-2-azaspiro[4.5]dec-6-ene-7-carbonitrile | 381.0 | A |
| 203 | 1 | 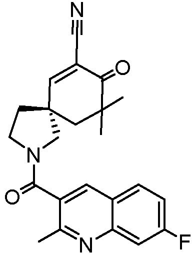 | (5R)-2-(7-fluoro-2-methylquinoline-3-carbonyl)-9,9-dimethyl-8-oxo-2-azaspiro[4.5]dec-6-ene-7-carbonitrile | 392.0 | A |
| 204 | 1 | 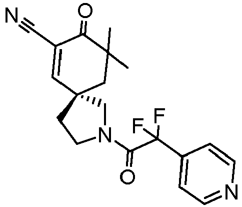 | (SR)-2-[difluoro(pyridin-4-yl)acetyl]-9,9-dimethyl-8-oxo-2-azaspiro[4.5]dec-6-ene-7-carbonitrile | 360.0 | A |
| 205 | 1 | 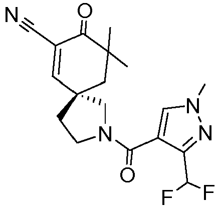 | (5R)-2-[3-(difluoromethyl)-1-methyl-1H-pyrazole-4-carbonyl]-9,9-dimethyl-8-oxo-2-azaspiro[4.5]dec-6-ene-7-carbonitrile | 363.0 | A |
| 206 | 1 | 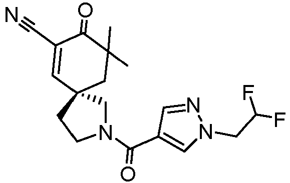 | (5R)-2-[1-(2,2-difluoroethyl)-1H-pyrazole-4-carbonyl]-9,9-dimethyl-8-oxo-2-azaspiro[4.5]dec-6-ene-7-carbonitrile | 363.0 | A |
| 207 | 1 | 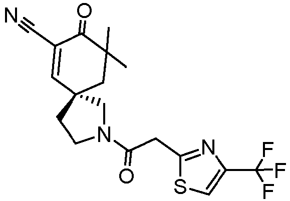 | (5R)-9,9-dimethyl-8-oxo-2-{ [4-(trifluoromethyl)-1,3-thiazol-2-yl]acetyl}-2-azaspiro[4.5]dec-6-ene-7-carbonitrile | 398.0 | A |
| 208 | 1 | 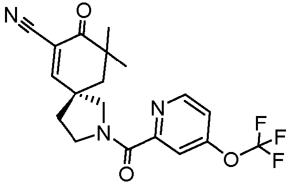 | (5R)-9,9-dimethyl-8-oxo-2-[4-(trifluoromethoxy)pyridi ne-2-carbonyl]-2-azaspiro[4.5]dec-6-ene-7-carbonitrile | 394.0 | A |
| 209 | 1 | 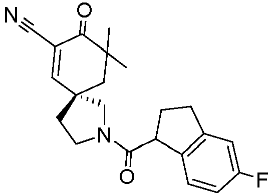 | (5R)-2-(5-fluoro-2,3-dihydro-1H-indene-1-carbonyl)-9,9-dimethyl-8-oxo-2-azaspiro[4.5]dec-6-ene-7-carbonitrile | 367.0 | A |
| 210 | 1 | 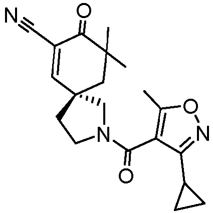 | (SR)-2-(3-cyclopropyl-5-methyl-1,2-oxazole-4-carbonyl)-9,9-dimethyl-8-oxo-2-azaspiro[4.5]dec-6-ene-7-carbonitrile | 354.0 | A |
| 211 | 1 | 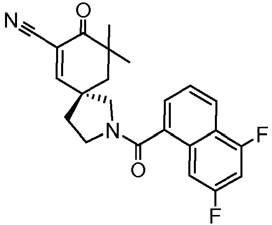 | (5R)-2-(5,7-difluoronaphthalene-1-carbonyl)-9,9-dimethyl-8-oxo-2-azaspiro[4.5]dec-6-ene-7-carbonitrile | 395.0 | A |
| 212 | 1 | 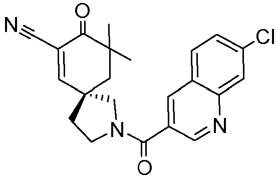 | (5R)-2-(7-chloroquinoline-3-carbonyl)-9,9-dimethyl-8-oxo-2-azaspiro[4.5]dec-6-ene-7-carbonitrile | 394.0 | A |
| 213 | 1 | 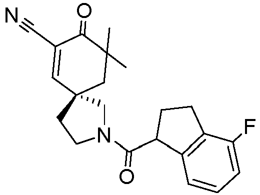 | (5R)-2-(4-fluoro-2,3-dihydro-1H-indene-1-carbonyl)-9,9-dimethyl-8-oxo-2-azaspiro[4.5]dec-6-ene-7-carbonitrile | 367.0 | A |
| 214 | 1 | 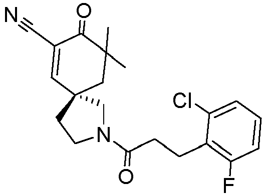 | (5R)-2-[3-(2-chloro-6-fluorophenyl)propanoyl]-9,9-dimethyl-8-oxo-2-azaspiro[4.5]dec-6-ene-7-carbonitrile | 389.0 | A |
| 215 | 1 | 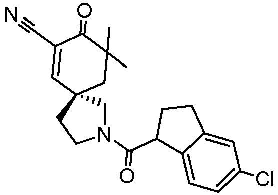 | (5R)-2-(5-chloro-2,3-dihydro-1H-indene-1-carbonyl)-9,9-dimethyl-8-oxo-2-azaspiro[4.5]dec-6-ene-7-carbonitrile | 383.0 | A |
| 216 | 1 | 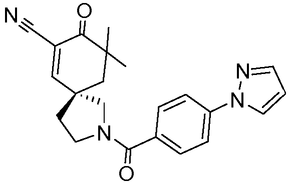 | (5R)-9,9-dimethyl-8-oxo-2-[4-(1H-pyrazol-1-yl)benzene-1-carbonyl]-2-azaspiro[4.5]dec-6-ene-7-carbonitrile | 375.0 | A |
| 217 | 1 | 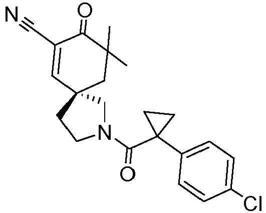 | (5R)-2-[1-(4-chlorophenyl)cyclopropa ne-1-carbonyl]-9,9-dimethyl-8-oxo-2-azaspiro[4.5]dec-6-ene-7-carbonitrile | 383.0 | A |
| 218 | 1 | 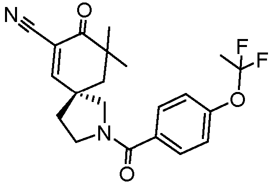 | (5R)-2-[4-(1,1-difluoroethoxy)benzene-1-carbonyl]-9,9-dimethyl-8-oxo-2-azaspiro[4.5]dec-6-ene-7-carbonitrile | 389.0 | A |
| 219 | 1 | 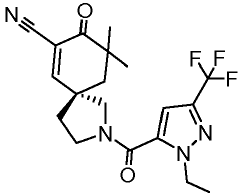 | (5R)-2-[1-ethyl-3-(trifluoromethyl)-1H-pyrazole-5-carbonyl]-9,9-dimethyl-8-oxo-2-azaspiro[4.5]dec-6-ene-7-carbonitrile | 395.0 | A |
| 220 | 1 | 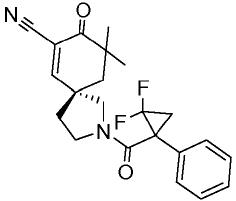 | (5R)-2-(2,2-difluoro-1-phenylcyclopropane-1-carbonyl)-9,9-dimethyl-8-oxo-2-azaspiro[4.5]dec-6-ene-7-carbonitrile | 385.0 | A |
| 221 | 1 | 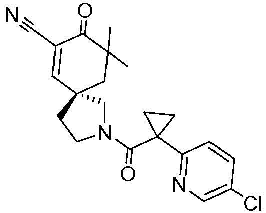 | (5R)-2-[1-(5-chloropyridin-2-yl)cyclopropane-1-carbonyl]-9,9-dimethyl-8-oxo-2-azaspiro[4.5]dec-6-ene-7-carbonitrile | 384.0 | A |
| 222 | 1 | 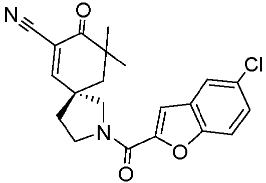 | (5R)-2-(5-chloro-1-benzofuran-2-carbonyl)-9,9-dimethyl-8-oxo-2-azaspiro[4.5]dec-6-ene-7-carbonitrile | 383.0 | A |
| 223 | 1 | 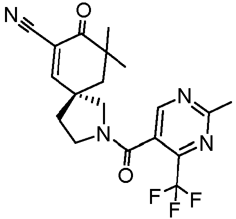 | (5R)-9,9-dimethyl-2-[2-methyl-4-(trifluoromethyl)pyrimidi ne-5-carbonyl]-8-oxo-2-azaspiro[4.5]dec-6-ene-7-carbonitrile | 393.0 | A |
| 224 | 1 | 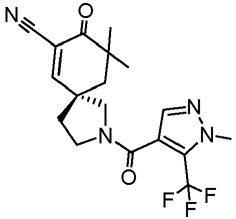 | (5R)-9,9-dimethyl-2-[1-methyl-5-(trifluoromethyl)-1H-pyrazole-4-carbonyl]-8-oxo-2-azaspiro[4.5]dec-6-ene-7-carbonitrile | 381.0 | A |
| 225 | 1 | 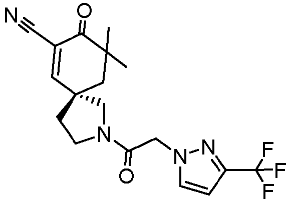 | (5R)-9,9-dimethyl-8-oxo-2-{[3-(trifluoromethyl)-1H-pyrazol-1-yl]acetyl}-2-azaspiro[4.5]dec-6-ene-7-carbonitrile | 381.0 | A |
| 226 | 1 | 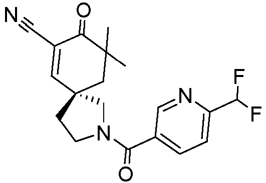 | (5R)-2-[6-(difluoromethyl)pyridine -3-carbonyl]-9,9-dimethyl-8-oxo-2-azaspiro[4.5]dec-6-ene-7-carbonitrile | 360.0 | A |
| 227 | 1 | 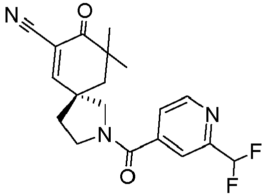 | (5R)-2-[2-(difluoromethyl)pyridine -4-carbonyl]-9,9-dimethyl-8-oxo-2-azaspiro[4.5]dec-6-ene-7-carbonitrile | 360.0 | A |
| 228 | 1 | 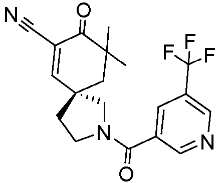 | (SR)-9,9-dimethyl-8-oxo-2-[5-(trifluoromethyl)pyridine -3-carbonyl]-2-azaspiro[4.5]dec-6-ene-7-carbonitrile | 378.0 | A |
| 229 | 1 | 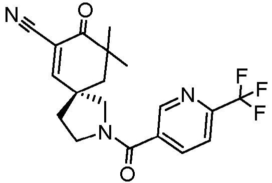 | (SR)-9,9-dimethyl-8-oxo-2-[6-(trifluoromethyl)pyridine -3-carbonyl]-2-azaspiro[4.5]dec-6-ene-7-carbonitrile | 378.0 | A |
| 230 | 1 | 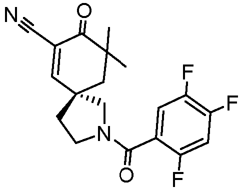 | (5R)-9,9-dimethyl-8-oxo-2-(2,4,5-trifluorobenzene-1-carbonyl)-2-azaspiro[4.5]dec-6-ene-7-carbonitrile | 363.0 | A |
| 231 | 1 | 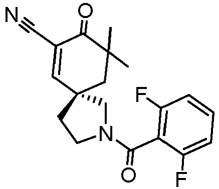 | (5R)-2-(2,6-difluorobenzene-1-carbonyl)-9,9-dimethyl-8-oxo-2-azaspiro[4.5]dec-6-ene-7-carbonitrile | 345.0 | A |
| 232 | 1 | 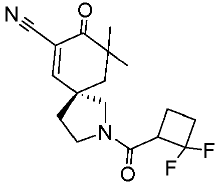 | (5R)-2-(2,2-difluorocyclobutane-1-carbonyl)-9,9-dimethyl-8-oxo-2-azaspiro[4.5]dec-6-ene-7-carbonitrile | 323.0 | A |
| 233 | 1 | 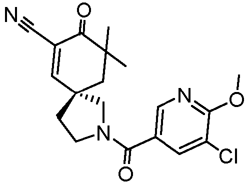 | (5R)-2-(5-chloro-6-methoxypyridine-3-carbonyl)-9,9-dimethyl-8-oxo-2-azaspiro[4.5]dec-6-ene-7-carbonitrile | 374.0 | A |
| 234 | 1 | 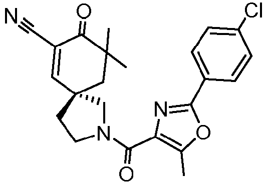 | (5R)-2-[2-(4-chlorophenyl)-5-methyl-1,3-oxazole-4-carbonyl]-9,9-dimethyl-8-oxo-2-azaspiro[4.5]dec-6-ene-7-carbonitrile | 424.0 | A |
| 235 | 1 | 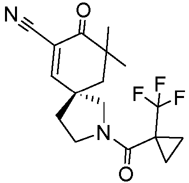 | (5R)-9,9-dimethyl-8-oxo-2-[1-(trifluoromethyl)cyclopro pane-1-carbonyl]-2-azaspiro[4.5]dec-6-ene-7-carbonitrile | 341.0 | A |
| 236 | 1 | 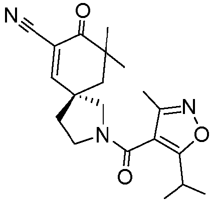 | (5R)-9,9-dimethyl-2-[3-methyl-5-(propan-2-yl)-1,2-oxazole-4-carbonyl]-8-oxo-2-azaspiro[4.5]dec-6-ene-7-carbonitrile | 356.0 | A |
| 237 | 1 | 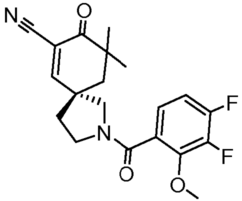 | (SR)-2-(3,4-difluoro-2-methoxybenzene-1-carbonyl)-9,9-dimethyl-8-oxo-2-azaspiro[4.5]dec-6-ene-7-carbonitrile | 375.0 | A |
| 238 | 1 | 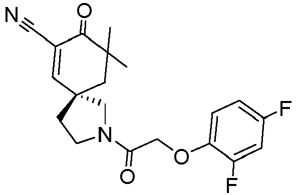 | (SR)-2-[(2,4-difluorophenoxy)acetyl]-9,9-dimethyl-8-oxo-2-azaspiro[4.5]dec-6-ene-7-carbonitrile | 375.0 | A |
| 239 | 1 | 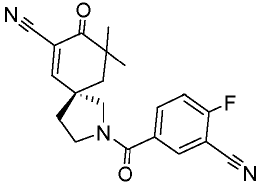 | (5R)-2-(3-cyano-4-fluorobenzene-1-carbonyl)-9,9-dimethyl-8-oxo-2-azaspiro[4.5]dec-6-ene-7-carbonitrile | 352.0 | A |
| 240 | 1 | 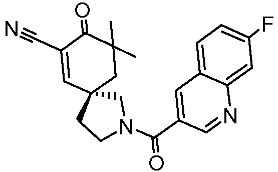 | (5R)-2-(7-fluoroquinoline-3-carbonyl)-9,9-dimethyl-8-oxo-2-azaspiro[4.5]dec-6-ene-7-carbonitrile | 378.0 | A |
| 241 | 1 | 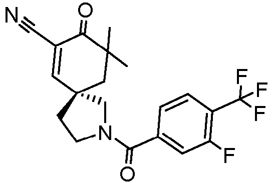 | (5R)-2-[3-fluoro-4-(trifluoromethyl)benzene -1-carbonyl]-9,9-dimethyl-8-oxo-2-azaspiro[4.5]dec-6-ene-7-carbonitrile | 395.0 | A |
| 242 | 1 | 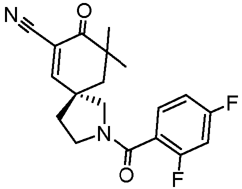 | (5R)-2-(2,4-difluorobenzene-1-carbonyl)-9,9-dimethyl-8-oxo-2-azaspiro[4.5]dec-6-ene-7-carbonitrile | 345.0 | A |
| 243 | 1 | 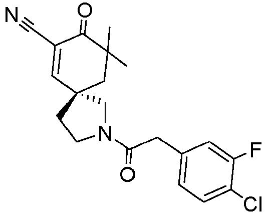 | (5R)-2-[(4-chloro-3-fluorophenyl)acetyl]-9,9-dimethyl-8-oxo-2-azaspiro[4.5]dec-6-ene-7-carbonitrile | 375.0 | A |
| 244 | 1 | 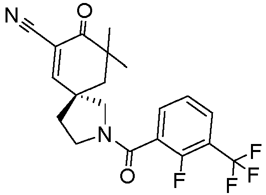 | (5R)-2-[2-fluoro-3-(trifluoromethyl)benzene -1-carbonyl]-9,9-dimethyl-8-oxo-2-azaspiro[4.5]dec-6-ene-7-carbonitrile | 395.0 | A |
| 245 | 1 | 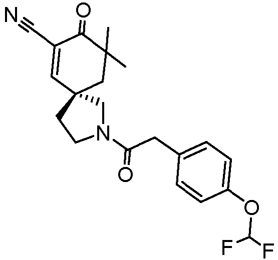 | (5R)-2-{[4-(difluoromethoxy)phenyl ]acetyl}-9,9-dimethyl-8-oxo-2-azaspiro[4.5]dec-6-ene-7-carbonitrile | 389.0 | A |
| 246 | 1 | 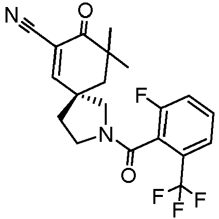 | (5R)-2-[2-fluoro-6-(trifluoromethyl)benzene -1-carbonyl]-9,9-dimethyl-8-oxo-2-azaspiro[4.5]dec-6-ene-7-carbonitrile | 395.0 | A |
| 247 | 1 | 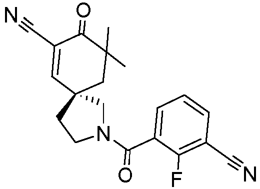 | (5R)-2-(3-cyano-2-fluorobenzene-1-carbonyl)-9,9-dimethyl-8-oxo-2-azaspiro[4.5]dec-6-ene-7-carbonitrile | 352.0 | A |
| 248 | 1 | 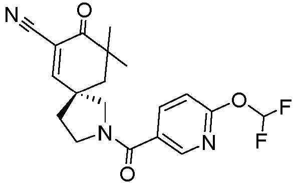 | (5R)-2-[6-(difluoromethoxy)pyridin e-3-carbonyl]-9,9-dimethyl-8-oxo-2-azaspiro[4.5]dec-6-ene-7-carbonitrile | 376.0 | A |
| 249 | 1 | 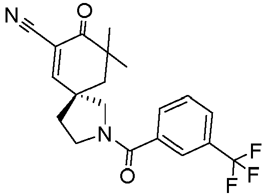 | (5R)-9,9-dimethyl-8-oxo-2-[3-(trifluoromethyl)benzene -1-carbonyl]-2-azaspiro[4.5]dec-6-ene-7-carbonitrile | 377.0 | A |
| 250 | 1 | 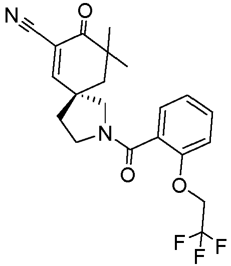 | (5R)-9,9-dimethyl-8-oxo-2-[2-(2,2,2-trifluoroethoxy)benzene-1-carbonyl]-2-azaspiro[4.5]dec-6-ene-7-carbonitrile | 407.0 | A |
| 251 | 1 | 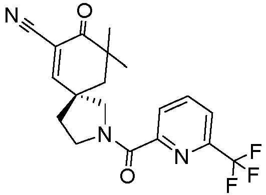 | (5R)-9,9-dimethyl-8-oxo-2-[6-(trifluoromethyl)pyridine -2-carbonyl]-2-azaspiro[4.5]dec-6-ene-7-carbonitrile | 378.0 | A |
| 252 | 1 | 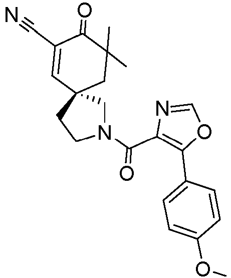 | (5R)-2-[5-(4-methoxyphenyl)-1,3-oxazole-4-carbonyl]-9,9-dimethyl-8-oxo-2-azaspiro[4.5]dec-6-ene-7-carbonitrile | 406.1 | A |
| 253 | 1 | 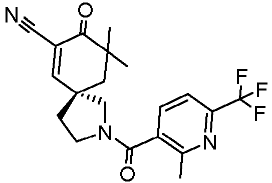 | (5R)-9,9-dimethyl-2-[2-methyl-6-(trifluoromethyl)pyridine -3-carbonyl]-8-oxo-2-azaspiro[4.5]dec-6-ene-7-carbonitrile | 392.0 | A |
| 254 | 1 | 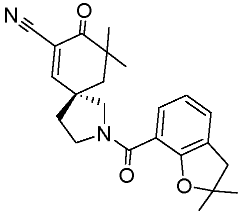 | (5R)-2-(2,2-dimethyl-2,3-dihydro-1-benzofuran-7-carbonyl)-9,9-dimethyl-8-oxo-2-azaspiro[4.5]dec-6-ene-7-carbonitrile | 379.1 | A |
| 255 | 1 | 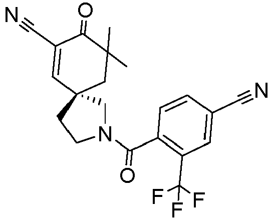 | (5R)-2-[4-cyano-2-(trifluoromethyl)benzene -1-carbonyl]-9,9-dimethyl-8-oxo-2-azaspiro[4.5]dec-6-ene-7-carbonitrile | 402.0 | A |
| 256 | 1 | 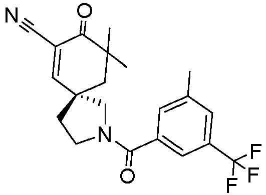 | (5R)-9,9-dimethyl-2-[3-methyl-5-(trifluoromethyl)benzene -1-carbonyl]-8-oxo-2-azaspiro[4.5]dec-6-ene-7-carbonitrile | 391.0 | A |
| 257 | 1 | 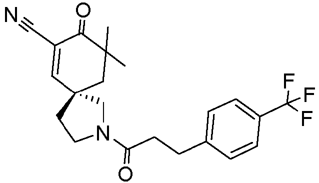 | (5R)-9,9-dimethyl-8-oxo-2-{3-[4-(trifluoromethyl)phenyl] propanoyl }-2-azaspiro[4.5]dec-6-ene-7-carbonitrile | 405.0 | A |
| 258 | 1 | 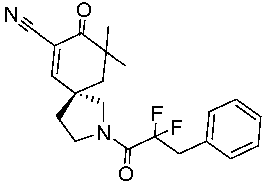 | (5R)-2-(2,2-difluoro-3-phenylpropanoyl)-9,9-dimethyl-8-oxo-2-azaspiro[4.5]dec-6-ene-7-carbonitrile | 373.0 | A |
| 259 | 1 | 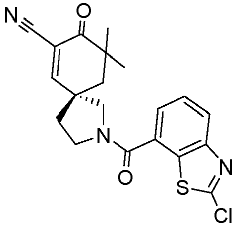 | (5R)-2-(2-chloro-1,3-benzothiazole-7-carbonyl)-9,9-dimethyl-8-oxo-2-azaspiro[4.5]dec-6-ene-7-carbonitrile | 400.0 | A |
| 260 | 1 | 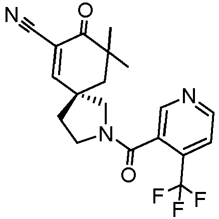 | (5R)-9,9-dimethyl-8-oxo-2-[4-(trifluoromethyl)pyridine -3-carbonyl]-2-azaspiro[4.5]dec-6-ene-7-carbonitrile | 378.0 | A |
| 261 | 1 | 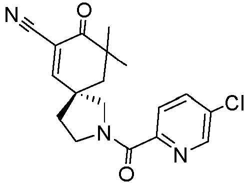 | (5R)-2-(5-chloropyridine-2-carbonyl)-9,9-dimethyl-8-oxo-2-azaspiro[4.5]dec-6-ene-7-carbonitrile | 344.0 | A |
| 262 | 1 | 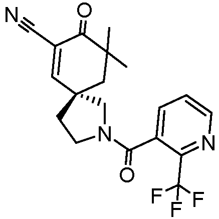 | (5R)-9,9-dimethyl-8-oxo-2-[2-(trifluoromethyl)pyridine -3-carbonyl]-2-azaspiro[4.5]dec-6-ene-7-carbonitrile | 378.0 | A |
| 263 | 1 | 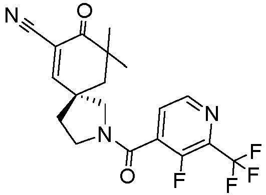 | (5R)-2-[3-fluoro-2-(trifluoromethyl)pyridine -4-carbonyl]-9,9-dimethyl-8-oxo-2-azaspiro[4.5]dec-6-ene-7-carbonitrile | 396.0 | A |
| 264 | 1 | 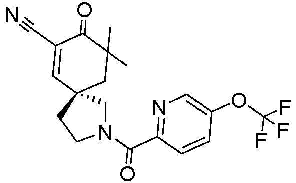 | (5R)-9,9-dimethyl-8-oxo-2-[5-(trifluoromethoxy)pyridi ne-2-carbonyl]-2-azaspiro[4.5]dec-6-ene-7-carbonitrile | 394.0 | A |
| 265 | 1 | 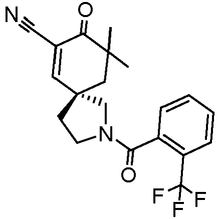 | (5R)-9,9-dimethyl-8-oxo-2-[2-(trifluoromethyl)benzene -1-carbonyl]-2-azaspiro[4.5]dec-6-ene-7-carbonitrile | 377.0 | A |
| 266 | 1 | 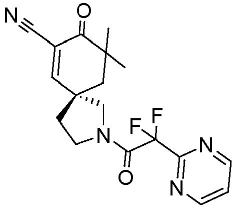 | (5R)-2-[difluoro(pyrimidin-2-yl)acetyl]-9,9-dimethyl-8-oxo-2-azaspiro[4.5]dec-6-ene-7-carbonitrile | 361.0 | A |
| 267 | 1 | 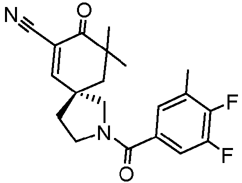 | (5R)-2-(3,4-difluoro-5-methylbenzene-1-carbonyl)-9,9-dimethyl-8-oxo-2-azaspiro[4.5]dec-6-ene-7-carbonitrile | 359.0 | A |
| 268 | 1 | 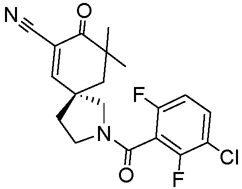 | (5R)-2-(3-chloro-2,6-difluorobenzene-1-carbonyl)-9,9-dimethyl-8-oxo-2-azaspiro[4.5]dec-6-ene-7-carbonitrile | 379.0 | A |
| 269 | 1 | 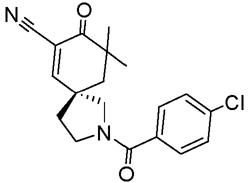 | (5R)-2-(4-chlorobenzene-1-carbonyl)-9,9-dimethyl-8-oxo-2-azaspiro[4.5]dec-6-ene-7-carbonitrile | 343.0 | A |
| 270 | 1 | 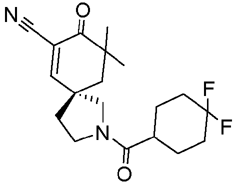 | (5R)-2-(4,4-difluorocyclohexane-1-carbonyl)-9,9-dimethyl-8-oxo-2-azaspiro[4.5]dec-6-ene-7-carbonitrile | 351.1 | A |
| 271 | 1 | 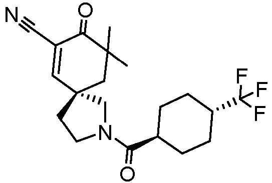 | (5R)-9,9-dimethyl-8-oxo-2-[trans-4-(trifluoromethyl)cyclohe xane-1-carbonyl]-2-azaspiro[4.5]dec-6-ene-7-carbonitrile | 383.0 | A |
| 272 | 1 | 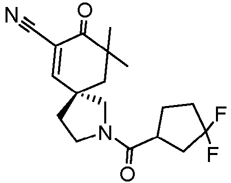 | (5R)-2-(3,3-difluorocyclopentane-1-carbonyl)-9,9-dimethyl-8-oxo-2-azaspiro[4.5]dec-6-ene-7-carbonitrile | 337.0 | A |
| 273 | 1 | 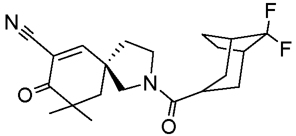 | (5R)-2-(8,8-difluorobicyclo[3.2.1]oct ane-3-carbonyl)-9,9-dimethyl-8-oxo-2-azaspiro[4.5]dec-6-ene-7-carbonitrile | 377.1 | A |
| 274 | 1 | 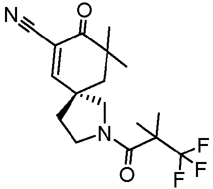 | (5R)-9,9-dimethyl-8-oxo-2-(3,3,3-trifluoro-2,2-dimethylpropanoyl)-2-azaspiro[4.5]dec-6-ene-7-carbonitrile | 343.0 | A |
| 275 | 1 | 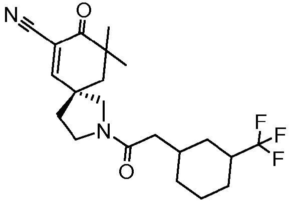 | (SR)-9,9-dimethyl-8-oxo-2-{[3-(trifluoromethyl)cyclohe xyl]acetyl}-2-azaspiro[4.5]dec-6-ene-7-carbonitrile | 397.1 | A |
| 276 | 1 | 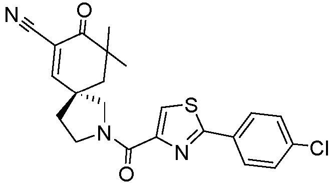 | (5R)-2-[2-(4-chlorophenyl)-1,3-thiazole-4-carbonyl]-9,9-dimethyl-8-oxo-2-azaspiro[4.5]dec-6-ene-7-carbonitrile | 426.0 | A |
| 277 | 1 | 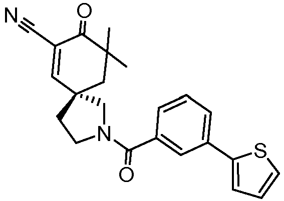 | (5R)-9,9-dimethyl-8-oxo-2-[3-(thiophen-2-yl)benzene-1-carbonyl]-2-azaspiro[4.5]dec-6-ene-7-carbonitrile | 391.0 | A |
| 278 | 1 | 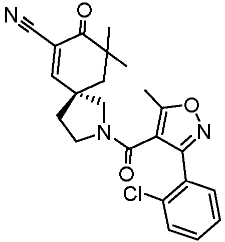 | (5R)-2-[3-(2-chlorophenyl)-5-methyl-1,2-oxazole-4-carbonyl]-9,9-dimethyl-8-oxo-2-azaspiro[4.5]dec-6-ene-7-carbonitrile | 424.0 | A |
| 279 | 1 | 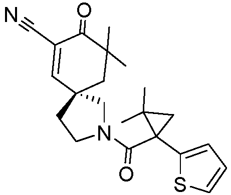 | (5R)-2-[2,2-dimethyl-1-(thiophen-2-yl)cyclopropane-1-carbonyl]-9,9-dimethyl-8-oxo-2-azaspiro[4.5]dec-6-ene-7-carbonitrile | 383.0 | A |
| 280 | 1 | 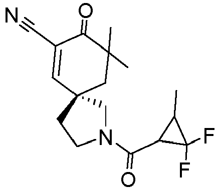 | (5R)-2-(2,2-difluoro-3-methylcyclopropane-1-carbonyl)-9,9-dimethyl-8-oxo-2-azaspiro[4.5]dec-6-ene-7-carbonitrile | 323.0 | A |
| 281 | 1 | 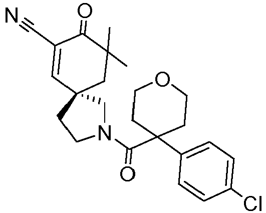 | (5R)-2-[4-(4-chlorophenyl)oxane-4-carbonyl]-9,9-dimethyl-8-oxo-2-azaspiro[4.5]dec-6-ene-7-carbonitrile | 427.0 | A |
| 282 | 1 | 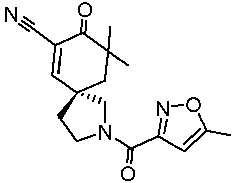 | (5R)-9,9-dimethyl-2-(5-methyl-1,2-oxazole-3-carbonyl)-8-oxo-2-azaspiro[4.5]dec-6-ene-7-carbonitrile | 314.0 | A |
| 283 | 1 | 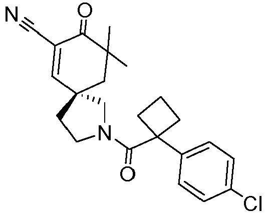 | (5R)-2-[1-(4-chlorophenyl)cyclobutan e-1-carbonyl]-9,9-dimethyl-8-oxo-2-azaspiro[4.5]dec-6-ene-7-carbonitrile | 397.0 | A |
| 284 | 1 | 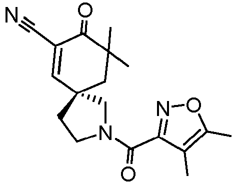 | (5R)-2-(4,5-dimethyl-1,2-oxazole-3-carbonyl)-9,9-dimethyl-8-oxo-2-azaspiro[4.5]dec-6-ene-7-carbonitrile | 328.0 | A |
| 285 | 1 | 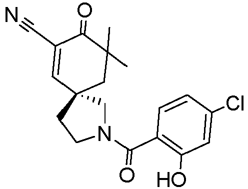 | (5R)-2-(4-chloro-2-hydroxybenzene-1-carbonyl)-9,9-dimethyl-8-oxo-2-azaspiro[4.5]dec-6-ene-7-carbonitrile | 359.0 | A |
| 286 | 1 | 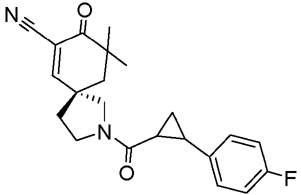 | (5R)-2-[2-(4-fluorophenyl)cyclopropa ne-1-carbonyl]-9,9-dimethyl-8-oxo-2-azaspiro[4.5]dec-6-ene-7-carbonitrile | 367.1 | A |
| 287 | 1 | 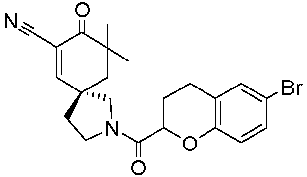 | (5R)-2-(6-bromo-3,4-dihydro-2H-1-benzopyran-2-carbonyl)-9,9-dimethyl-8-oxo-2-azaspiro[4.5]dec-6-ene-7-carbonitrile | 442.9, 445.0 | A |
| 288 | 1 | 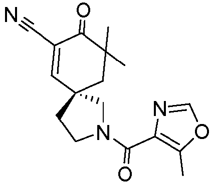 | (5R)-9,9-dimethyl-2-(5-methyl-1,3-oxazole-4-carbonyl)-8-oxo-2-azaspiro[4.5]dec-6-ene-7-carbonitrile | 314.1 | A |
| 289 | 1 | 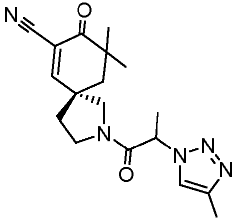 | (5R)-9,9-dimethyl-2-[2-(4-methyl-1H-1,2,3-triazol-1-yl)propanoyl]-8-oxo-2-azaspiro[4.5]dec-6-ene-7-carbonitrile | 342.1 | A |
| 290 | 1 | 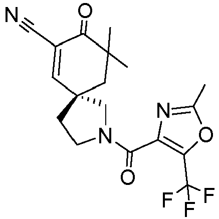 | (5R)-9,9-dimethyl-2-[2-methyl-5-(trifluoromethyl)-1,3-oxazole-4-carbonyl]-8-oxo-2-azaspiro[4.5]dec-6-ene-7-carbonitrile | 382.0 | A |
| 291 | 1 | 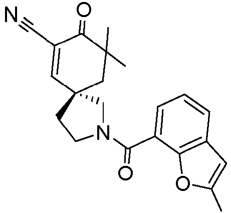 | (5R)-9,9-dimethyl-2-(2-methyl-1-benzofuran-7-carbonyl)-8-oxo-2-azaspiro[4.5]dec-6-ene-7-carbonitrile | 363.1 | A |
| 292 | 1 | 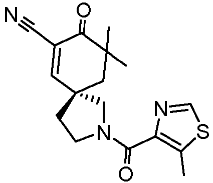 | (5R)-9,9-dimethyl-2-(5-methyl-1,3-thiazole-4-carbonyl)-8-oxo-2-azaspiro[4.5]dec-6-ene-7-carbonitrile | 330.0 | A |
| 293 | 1 | 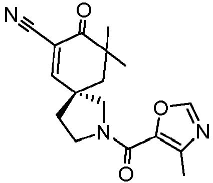 | (5R)-9,9-dimethyl-2-(4-methyl-1,3-oxazole-5-carbonyl)-8-oxo-2-azaspiro[4.5]dec-6-ene-7-carbonitrile | 314.0 | A |
| 294 | 1 | 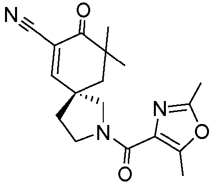 | (5R)-2-(2,5-dimethyl-1,3-oxazole-4-carbonyl)-9,9-dimethyl-8-oxo-2-azaspiro[4.5]dec-6-ene-7-carbonitrile | 328.0 | A |
| 295 | 1 | 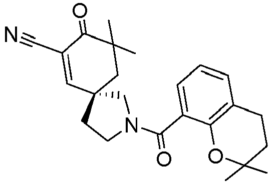 | (5R)-2-(2,2-dimethyl-3,4-dihydro-2H-1-benzopyran-8-carbonyl)-9,9-dimethyl-8-oxo-2-azaspiro[4.5]dec-6-ene-7-carbonitrile | 393.1 | A |
| 296 | 1 | 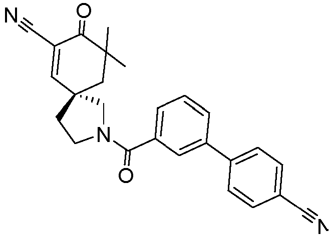 | (5R)-2-(4'-cyano[1,1'-biphenyl]-3-carbonyl)-9,9-dimethyl-8-oxo-2-azaspiro[4.5]dec-6-ene-7-carbonitrile | 410.0 | A |
| 297 | 1 | 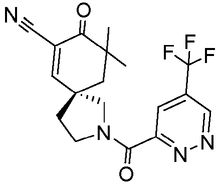 | (5R)-9,9-dimethyl-8-oxo-2-[5-(trifluoromethyl)pyridazi ne-3-carbonyl]-2-azaspiro[4.5]dec-6-ene-7-carbonitrile | 379.0 | A |
| 298 | 1 | 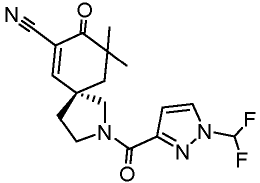 | (5R)-2-[1-(difluoromethyl)-1H-pyrazole-3-carbonyl]-9,9-dimethyl-8-oxo-2-azaspiro[4.5]dec-6-ene-7-carbonitrile | 349.0 | A |
| 299 | 1 | 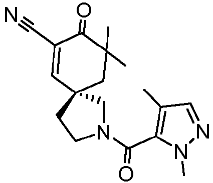 | (5R)-2-(1,4-dimethyl-1H-pyrazole-5-carbonyl)-9,9-dimethyl-8-oxo-2-azaspiro[4.5]dec-6-ene-7-carbonitrile | 327.0 | A |
| 300 | 1 | 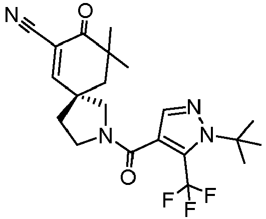 | (5R)-2-[1-tert-butyl-5-(trifluoromethyl)-1H-pyrazole-4-carbonyl]-9,9-dimethyl-8-oxo-2-azaspiro[4.5]dec-6-ene-7-carbonitrile | 423.0 | A |
| 301 | 1 | 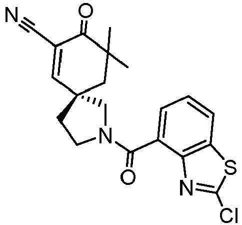 | (5R)-2-(2-chloro-1,3-benzothiazole-4-carbonyl)-9,9-dimethyl-8-oxo-2-azaspiro[4.5]dec-6-ene-7-carbonitrile | 400.0 | A |
| 302 | 1 | 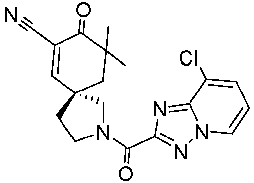 | (5R)-2-(8-chloro[1,2,4]triazolo[1,5-a]pyridine-2-carbonyl)-9,9-dimethyl-8-oxo-2-azaspiro[4.5]dec-6-ene-7-carbonitrile | 384.0 | A |
| 303 | 1 | 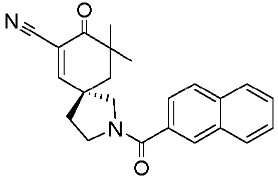 | (5R)-9,9-dimethyl-2-(naphthalene-2-carbonyl)-8-oxo-2-azaspiro[4.5]dec-6-ene-7-carbonitrile | 359.0 | A |
| 304 | 1 | 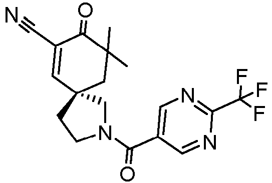 | (5R)-9,9-dimethyl-8-oxo-2-[2-(trifluoromethyl)pyrimidi ne-5-carbonyl]-2-azaspiro[4.5]dec-6-ene-7-carbonitrile | 379.0 | A |
| 305 | 1 | 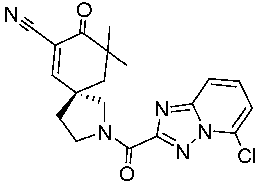 | (5R)-2-(5-chloro[1,2,4]triazolo[1,5-a]pyridine-2-carbonyl)-9,9-dimethyl-8-oxo-2-azaspiro[4.5]dec-6-ene-7-carbonitrile | 384.0 | A |
| 306 | 1 | 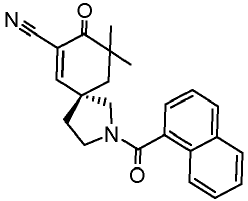 | (5R)-9,9-dimethyl-2-(naphthalene-1-carbonyl)-8-oxo-2-azaspiro[4.5]dec-6-ene-7-carbonitrile | 359.1 | A |
| 307 | 1 | 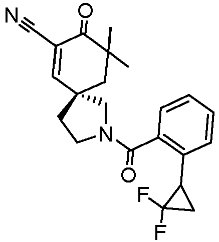 | (5R)-2-[2-(2,2-difluorocyclopropyl)benz ene-1-carbonyl]-9,9-dimethyl-8-oxo-2-azaspiro[4.5]dec-6-ene-7-carbonitrile | 385.0 | A |
| 308 | 1 | 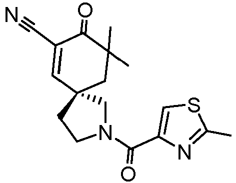 | (5R)-9,9-dimethyl-2-(2-methyl-1,3-thiazole-4-carbonyl)-8-oxo-2-azaspiro[4.5]dec-6-ene-7-carbonitrile | 330.0 | A |
| 309 | 2 | 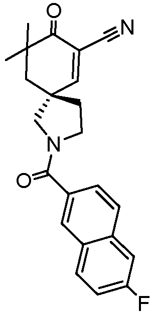 | (5S)-2-(6-fluoronaphthalene-2-carbonyl)-9,9-dimethyl-8-oxo-2-azaspiro[4.5]dec-6-ene-7-carbonitrile | 377.0 | A |
| 310 | 2 | 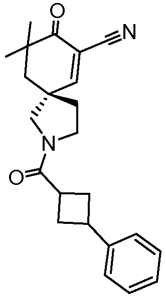 | (5S)-9,9-dimethyl-8-oxo-2-(3-phenylcyclobutane-1-carbonyl)-2-azaspiro[4.5]dec-6-ene-7-carbonitrile | 363.0 | A |
| 311 | 2 | 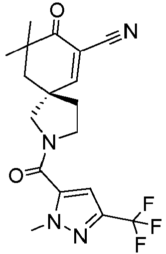 | (5S)-9,9-dimethyl-2-[1-methyl-3-(trifluoromethyl)-1H-pyrazole-5-carbonyl]-8-oxo-2-azaspiro[4.5]dec-6-ene-7-carbonitrile | 381.0 | A |
| 312 | 2 | 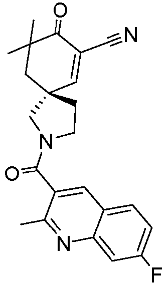 | (5S)-2-(7-fluoro-2-methylquinoline-3-carbonyl)-9,9-dimethyl-8-oxo-2-azaspiro[4.5]dec-6-ene-7-carbonitrile | 392.0 | A |
| 313 | 2 | 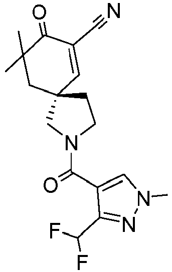 | (5S)-2-[3-(difluoromethyl)-1-methyl-1H-pyrazole-4-carbonyl]-9,9-dimethyl-8-oxo-2-azaspiro[4.5]dec-6-ene-7-carbonitrile | 363.0 | A |
| 314 | 2 | 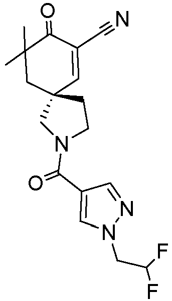 | (5S)-2-[1-(2,2-difluoroethyl)-1H-pyrazole-4-carbonyl]-9,9-dimethyl-8-oxo-2-azaspiro[4.5]dec-6-ene-7-carbonitrile | 363.0 | A |
| 315 | 2 | 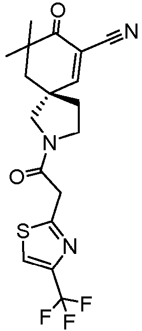 | (5S)-9,9-dimethyl-8-oxo-2-{ [4-(trifluoromethyl)-1,3-thiazol-2-yl]acetyl}-2-azaspiro[4.5]dec-6-ene-7-carbonitrile | 398.0 | A |
| 316 | 2 | 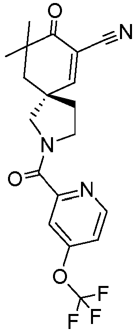 | (5S)-9,9-dimethyl-8-oxo-2-[4-(trifluoromethoxy)pyridi ne-2-carbonyl]-2-azaspiro[4.5]dec-6-ene-7-carbonitrile | 394.0 | A |
| 317 | 2 | 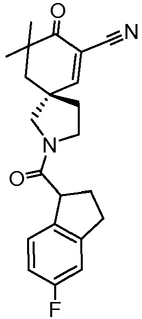 | (5S)-2-(5-fluoro-2,3-dihydro-1H-indene-1-carbonyl)-9,9-dimethyl-8-oxo-2-azaspiro[4.5]dec-6-ene-7-carbonitrile | 367.0 | A |
| 318 | 2 | 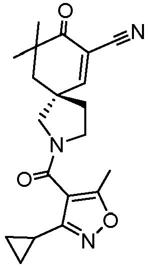 | (5S)-2-(3-cyclopropyl-5-methyl-1,2-oxazole-4-carbonyl)-9,9-dimethyl-8-oxo-2-azaspiro[4.5]dec-6-ene-7-carbonitrile | 354.0 | A |
| 319 | 2 | 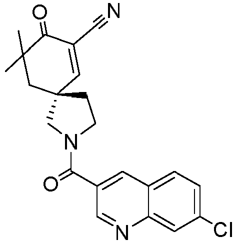 | (5S)-2-(7-chloroquinoline-3-carbonyl)-9,9-dimethyl-8-oxo-2-azaspiro[4.5]dec-6-ene-7-carbonitrile | 394.0 | A |
| 320 | 2 | 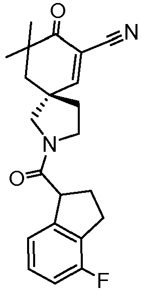 | (5S)-2-(4-fluoro-2,3-dihydro-1H-indene-1-carbonyl)-9,9-dimethyl-8-oxo-2-azaspiro[4.5]dec-6-ene-7-carbonitrile | 367.1 | A |
| 321 | 2 | 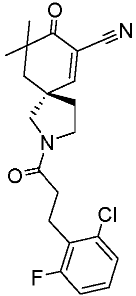 | (5S)-2-[3-(2-chloro-6-fluorophenyl)propanoyl]-9,9-dimethyl-8-oxo-2-azaspiro[4.5]dec-6-ene-7-carbonitrile | 389.0 | A |
| 322 | 2 | 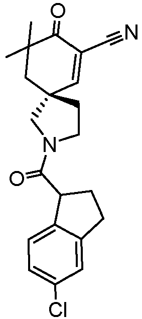 | (5S)-2-(5-chloro-2,3-dihydro-1H-indene-1-carbonyl)-9,9-dimethyl-8-oxo-2-azaspiro[4.5]dec-6-ene-7-carbonitrile | 383.0 | A |
| 323 | 2 | 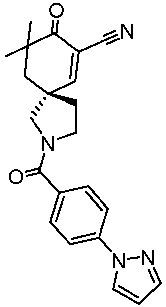 | (5S)-9,9-dimethyl-8-oxo-2-[4-(1H-pyrazol-1-yl)benzene-1-carbonyl]-2-azaspiro[4.5]dec-6-ene-7-carbonitrile | 375.1 | A |
| 324 | 2 | 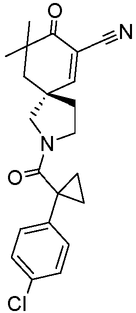 | (5S)-2-[1-(4-chlorophenyl)cyclopropa ne-1-carbonyl]-9,9-dimethyl-8-oxo-2-azaspiro[4.5]dec-6-ene-7-carbonitrile | 383.0 | A |
| 325 | 2 | 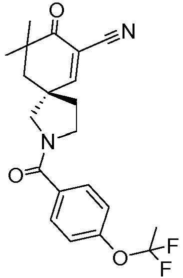 | (5S)-2-[4-(1,1-difluoroethoxy)benzene-1-carbonyl]-9,9-dimethyl-8-oxo-2-azaspiro[4.5]dec-6-ene-7-carbonitrile | 389.0 | A |
| 326 | 2 | 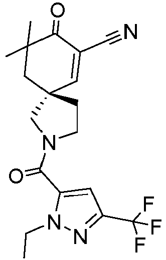 | (5S)-2-[1-ethyl-3-(trifluoromethyl)-1H-pyrazole-5-carbonyl]-9,9-dimethyl-8-oxo-2-azaspiro[4.5]dec-6-ene-7-carbonitrile | 395.0 | A |
| 327 | 2 | 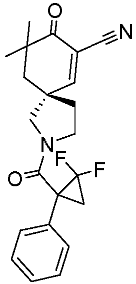 | (5S)-2-(2,2-difluoro-1-phenylcyclopropane-1-carbonyl)-9,9-dimethyl-8-oxo-2-azaspiro[4.5]dec-6-ene-7-carbonitrile | 385.0 | A |
| 328 | 2 | 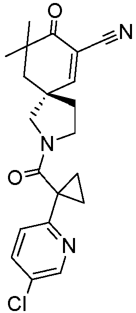 | (5S)-2-[1-(5-chloropyridin-2-yl)cyclopropane-1-carbonyl]-9,9-dimethyl-8-oxo-2-azaspiro[4.5]dec-6-ene-7-carbonitrile | 384.0 | A |
| 329 | 2 | 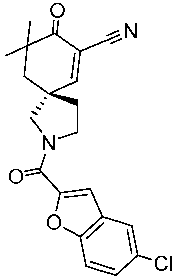 | (5S)-2-(5-chloro-1-benzofuran-2-carbonyl)-9,9-dimethyl-8-oxo-2-azaspiro[4.5]dec-6-ene-7-carbonitrile | 383.0 | A |
| 330 | 2 | 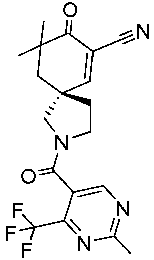 | (5S)-9,9-dimethyl-2-[2-methyl-4-(trifluoromethyl)pyrimidi ne-5-carbonyl]-8-oxo-2-azaspiro[4.5]dec-6-ene-7-carbonitrile | 393.0 | A |
| 331 | 2 | 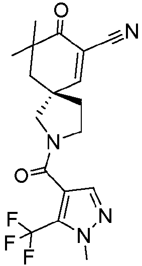 | (5S)-9,9-dimethyl-2-[1-methyl-5-(trifluoromethyl)-1H-pyrazole-4-carbonyl]-8-oxo-2-azaspiro[4.5]dec-6-ene-7-carbonitrile | 381.0 | A |
| 332 | 2 | 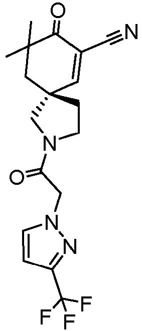 | (5S)-9,9-dimethyl-8-oxo-2-{[3-(trifluoromethyl)-1H-pyrazol-1-yl]acetyl}-2-azaspiro[4.5]dec-6-ene-7-carbonitrile | 381.0 | A |
| 333 | 2 | 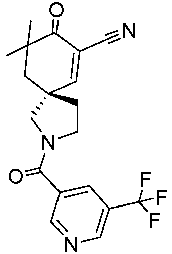 | (5S)-9,9-dimethyl-8-oxo-2-[5-(trifluoromethyl)pyridine -3-carbonyl]-2-azaspiro[4.5]dec-6-ene-7-carbonitrile | 378.0 | A |
| 334 | 2 | 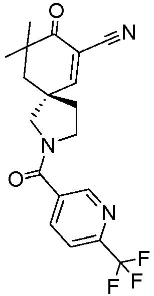 | (5S)-9,9-dimethyl-8-oxo-2-[6-(trifluoromethyl)pyridine -3-carbonyl]-2-azaspiro[4.5]dec-6-ene-7-carbonitrile | 378.0 | A |
| 335 | 2 | 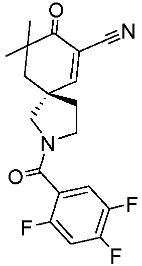 | (5S)-9,9-dimethyl-8-oxo-2-(2,4,5-trifluorobenzene-1-carbonyl)-2-azaspiro[4.5]dec-6-ene-7-carbonitrile | 363.0 | A |
| 336 | 2 | 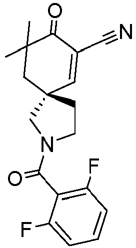 | (5S)-2-(2,6-difluorobenzene-1-carbonyl)-9,9-dimethyl-8-oxo-2-azaspiro[4.5]dec-6-ene-7-carbonitrile | 345.0 | A |
| 337 | 2 | 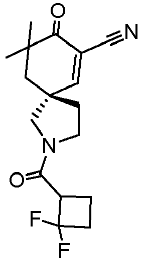 | (5S)-2-(2,2-difluorocyclobutane-1-carbonyl)-9,9-dimethyl-8-oxo-2-azaspiro[4.5]dec-6-ene-7-carbonitrile | 323.0 | A |
| 338 | 2 | 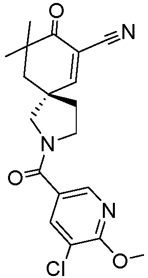 | (5S)-2-(5-chloro-6-methoxypyridine-3-carbonyl)-9,9-dimethyl-8-oxo-2-azaspiro[4.5]dec-6-ene-7-carbonitrile | 374.0 | A |
| 339 | 2 | 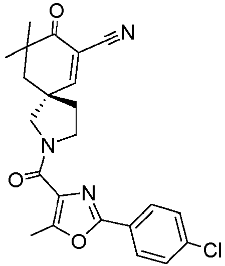 | (5S)-2-[2-(4-chlorophenyl)-5-methyl-1,3-oxazole-4-carbonyl]-9,9-dimethyl-8-oxo-2-azaspiro[4.5]dec-6-ene-7-carbonitrile | 424.0 | A |
| 340 | 2 | 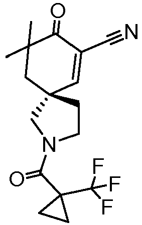 | (5S)-9,9-dimethyl-8-oxo-2-[1-(trifluoromethyl)cyclopro pane-1-carbonyl]-2-azaspiro[4.5]dec-6-ene-7-carbonitrile | 341.0 | A |
| 341 | 2 | 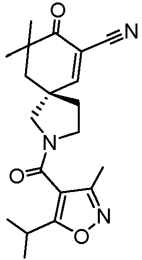 | (5S)-9,9-dimethyl-2-[3-methyl-5-(propan-2-yl)-1,2-oxazole-4-carbonyl]-8-oxo-2-azaspiro[4.5]dec-6-ene-7-carbonitrile | 356.1 | A |
| 342 | 2 | 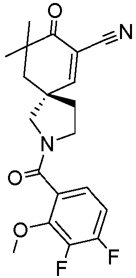 | (5S)-2-(3,4-difluoro-2-methoxybenzene-1-carbonyl)-9,9-dimethyl-8-oxo-2-azaspiro[4.5]dec-6-ene-7-carbonitrile | 375.0 | A |
| 343 | 2 | 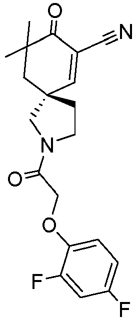 | (5S)-2-[(2,4-difluorophenoxy)acetyl]-9,9-dimethyl-8-oxo-2-azaspiro[4.5]dec-6-ene-7-carbonitrile | 375.0 | A |
| 344 | 2 | 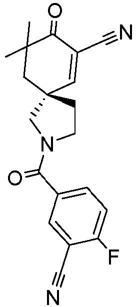 | (5S)-2-(3-cyano-4-fluorobenzene-1-carbonyl)-9,9-dimethyl-8-oxo-2-azaspiro[4.5]dec-6-ene-7-carbonitrile | 352.0 | A |
| 345 | 2 | 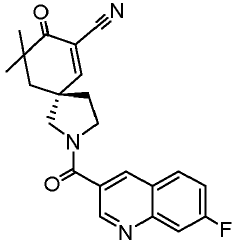 | (5S)-2-(7-fluoroquinoline-3-carbonyl)-9,9-dimethyl-8-oxo-2-azaspiro[4.5]dec-6-ene-7-carbonitrile | 378.0 | A |
| 346 | 2 | 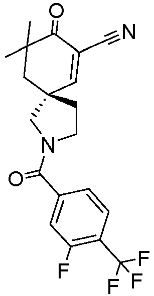 | (5S)-2-[3-fluoro-4-(trifluoromethyl)benzene -1-carbonyl]-9,9-dimethyl-8-oxo-2-azaspiro[4.5]dec-6-ene-7-carbonitrile | 395.0 | A |
| 347 | 2 | 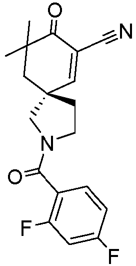 | (5S)-2-(2,4-difluorobenzene-1-carbonyl)-9,9-dimethyl-8-oxo-2-azaspiro[4.5]dec-6-ene-7-carbonitrile | 345.0 | A |
| 348 | 2 | 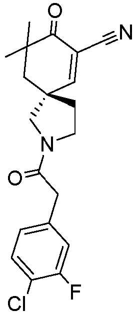 | (5S)-2-[(4-chloro-3-fluorophenyl)acetyl]-9,9-dimethyl-8-oxo-2-azaspiro[4.5]dec-6-ene-7-carbonitrile | 375.0 | A |
| 349 | 2 | 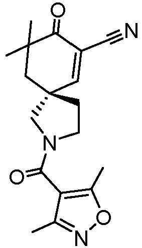 | (5S)-2-(3, 5-dimethyl-1,2-oxazole-4-carbonyl)-9,9-dimethyl-8-oxo-2-azaspiro[4.5]dec-6-ene-7-carbonitrile | 328.0 | A |
| 350 | 2 | 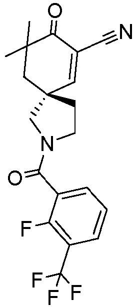 | (5S)-2-[2-fluoro-3-(trifluoromethyl)benzene -1-carbonyl]-9,9-dimethyl-8-oxo-2-azaspiro[4.5]dec-6-ene-7-carbonitrile | 395.0 | A |
| 351 | 2 | 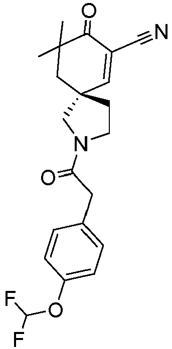 | (5S)-2-{ [4-(difluoromethoxy)phenyl ]acetyl}-9,9-dimethyl-8-oxo-2-azaspiro[4.5]dec-6-ene-7-carbonitrile | 389.0 | A |
| 352 | 2 | 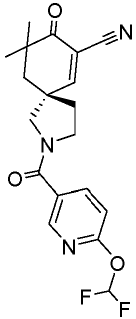 | (5S)-2-[6-(difluoromethoxy)pyridin e-3-carbonyl]-9,9-dimethyl-8-oxo-2-azaspiro[4.5]dec-6-ene-7-carbonitrile | 376.0 | A |
| 353 | 2 | 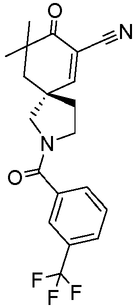 | (5S)-9,9-dimethyl-8-oxo-2-[3-(trifluoromethyl)benzene -1-carbonyl]-2-azaspiro[4.5]dec-6-ene-7-carbonitrile | 377.0 | A |
| 354 | 2 | 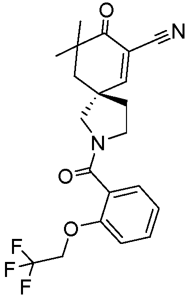 | (5S)-9,9-dimethyl-8-oxo-2-[2-(2,2,2-trifluoroethoxy)benzene-1-carbonyl]-2-azaspiro[4.5]dec-6-ene-7-carbonitrile | 407.1 | A |
| 355 | 2 | 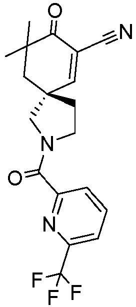 | (5S)-9,9-dimethyl-8-oxo-2-[6-(trifluoromethyl)pyridine -2-carbonyl]-2-azaspiro[4.5]dec-6-ene-7-carbonitrile | 378.0 | A |
| 356 | 2 | 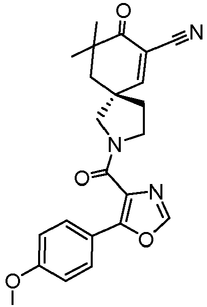 | (5S)-2-[5-(4-methoxyphenyl)-1,3-oxazole-4-carbonyl]-9,9-dimethyl-8-oxo-2-azaspiro[4.5]dec-6-ene-7-carbonitrile | 406.0 | A |
| 357 | 2 | 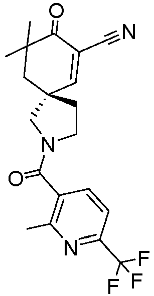 | (5S)-9,9-dimethyl-2-[2-methyl-6-(trifluoromethyl)pyridine -3-carbonyl]-8-oxo-2-azaspiro[4.5]dec-6-ene-7-carbonitrile | 392.0 | A |
| 358 | 2 | 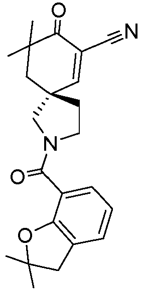 | (5S)-2-(2,2-dimethyl-2,3-dihydro-1-benzofuran-7-carbonyl)-9,9-dimethyl-8-oxo-2-azaspiro[4.5]dec-6-ene-7-carbonitrile | 379.0 | A |
| 359 | 2 | 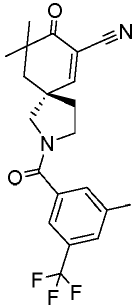 | (5S)-9,9-dimethyl-2-[3-methyl-5-(trifluoromethyl)benzene -1-carbonyl]-8-oxo-2-azaspiro[4.5]dec-6-ene-7-carbonitrile | 391.0 | A |
| 360 | 2 | 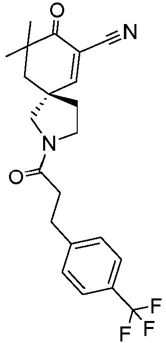 | (5S)-9,9-dimethyl-8-oxo-2-{3-[4-(trifluoromethyl)phenyl] propanoyl }-2-azaspiro[4.5]dec-6-ene-7-carbonitrile | 405.1 | A |
| 361 | 2 | 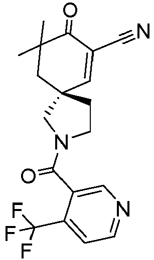 | (5S)-9,9-dimethyl-8-oxo-2-[4-(trifluoromethyl)pyridine -3-carbonyl]-2-azaspiro[4.5]dec-6-ene-7-carbonitrile | 378.0 | A |
| 362 | 2 | 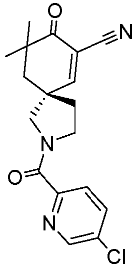 | (5S)-2-(5-chloropyridine-2-carbonyl)-9,9-dimethyl-8-oxo-2-azaspiro[4.5]dec-6-ene-7-carbonitrile | 344.0 | A |
| 363 | 2 | 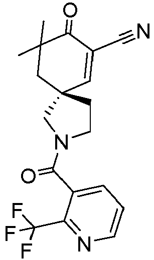 | (5S)-9,9-dimethyl-8-oxo-2-[2-(trifluoromethyl)pyridine -3-carbonyl]-2-azaspiro[4.5]dec-6-ene-7-carbonitrile | 378.0 | A |
| 364 | 2 | 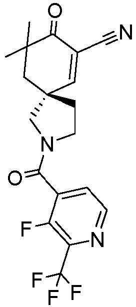 | (5S)-2-[3-fluoro-2-(trifluoromethyl)pyridine -4-carbonyl]-9,9-dimethyl-8-oxo-2-azaspiro[4.5]dec-6-ene-7-carbonitrile | 395.9 | A |
| 365 | 2 | 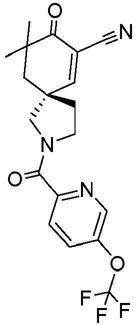 | (5S)-9,9-dimethyl-8-oxo-2-[5-(trifluoromethoxy)pyridi ne-2-carbonyl]-2-azaspiro[4.5]dec-6-ene-7-carbonitrile | 394.0 | A |
| 366 | 2 | 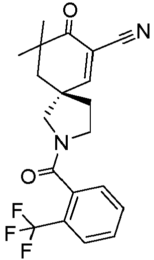 | (5S)-9,9-dimethyl-8-oxo-2-[2-(trifluoromethyl)benzene -1-carbonyl]-2-azaspiro[4.5]dec-6-ene-7-carbonitrile | 377.0 | A |
| 367 | 2 | 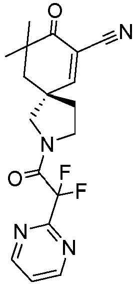 | (5S)-2-[difluoro(pyrimidin-2-yl)acetyl]-9,9-dimethyl-8-oxo-2-azaspiro[4.5]dec-6-ene-7-carbonitrile | 361.0 | A |
| 368 | 2 | 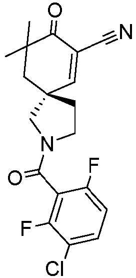 | (5S)-2-(3-chloro-2,6-difluorobenzene-1-carbonyl)-9,9-dimethyl-8-oxo-2-azaspiro[4.5]dec-6-ene-7-carbonitrile | 379.0 | A |
| 369 | 2 | 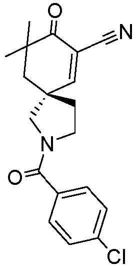 | (5S)-2-(4-chlorobenzene-1-carbonyl)-9,9-dimethyl-8-oxo-2-azaspiro[4.5]dec-6-ene-7-carbonitrile | 343.0 | A |
| 370 | 2 | 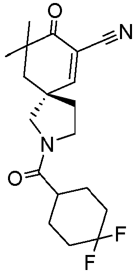 | (5S)-2-(4,4-difluorocyclohexane-1-carbonyl)-9,9-dimethyl-8-oxo-2-azaspiro[4.5]dec-6-ene-7-carbonitrile | 351.1 | A |
| 371 | 2 | 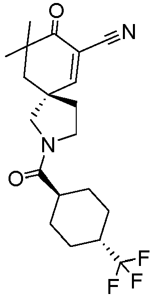 | (5S)-9,9-dimethyl-8-oxo-2-[trans-4-(trifluoromethyl)cyclohe xane-1-carbonyl]-2-azaspiro[4.5]dec-6-ene-7-carbonitrile | 383.0 | A |
| 372 | 2 | 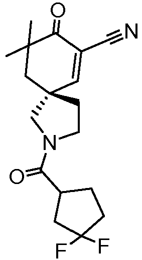 | (5S)-2-(3,3-difluorocyclopentane-1-carbonyl)-9,9-dimethyl-8-oxo-2-azaspiro[4.5]dec-6-ene-7-carbonitrile | 337.0 | A |
| 373 | 2 | 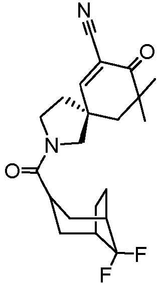 | (5S)-2-(8,8-difluorobicyclo[3.2.1]oct ane-3-carbonyl)-9,9-dimethyl-8-oxo-2-azaspiro[4.5]dec-6-ene-7-carbonitrile | 377.1 | A |
| 374 | 2 | 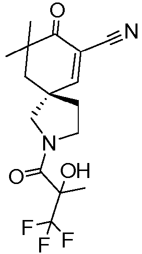 | (5S)-9,9-dimethyl-8-oxo-2-(3,3,3-trifluoro-2-hydroxy-2-methylpropanoyl)-2-azaspiro[4.5]dec-6-ene-7-carbonitrile | 345.0 | A |
| 375 | 2 | 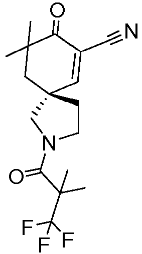 | (5S)-9,9-dimethyl-8-oxo-2-(3,3,3-trifluoro-2,2-dimethylpropanoyl)-2-azaspiro[4.5]dec-6-ene-7-carbonitrile | 343.0 | A |
| 376 | 2 | 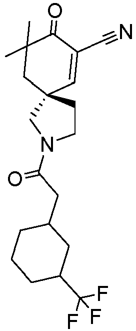 | (5S)-9,9-dimethyl-8-oxo-2-{[3-(trifluoromethyl)cyclohe xyl]acetyl}-2-azaspiro[4.5]dec-6-ene-7-carbonitrile | 397.1 | A |
| 377 | 2 | 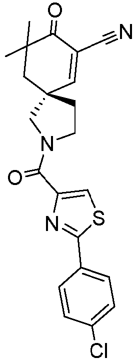 | (5S)-2-[2-(4-chlorophenyl)-1,3-thiazole-4-carbonyl]-9,9-dimethyl-8-oxo-2-azaspiro[4.5]dec-6-ene-7-carbonitrile | 426.0 | A |
| 378 | 2 | 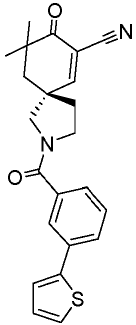 | (5S)-9,9-dimethyl-8-oxo-2-[3-(thiophen-2-yl)benzene-1-carbonyl]-2-azaspiro[4.5]dec-6-ene-7-carbonitrile | 391.0 | A |
| 379 | 2 | 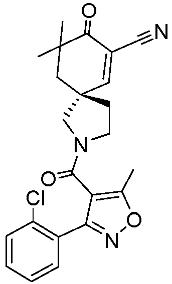 | (5S)-2-[3-(2-chlorophenyl)-5-methyl-1,2-oxazole-4-carbonyl]-9,9-dimethyl-8-oxo-2-azaspiro[4.5]dec-6-ene-7-carbonitrile | 424.0 | A |
| 380 | 2 | 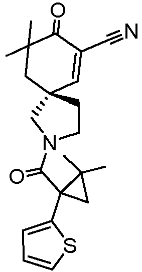 | (5S)-2-[2,2-dimethyl-1-(thiophen-2-yl)cyclopropane-1-carbonyl]-9,9-dimethyl-8-oxo-2-azaspiro[4.5]dec-6-ene-7-carbonitrile | 383.0 | A |
| 381 | 2 | 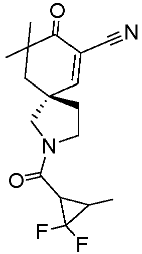 | (5S)-2-(2,2-difluoro-3-methylcyclopropane-1-carbonyl)-9,9-dimethyl-8-oxo-2-azaspiro[4.5]dec-6-ene-7-carbonitrile | 323.1 | A |
| 382 | 2 | 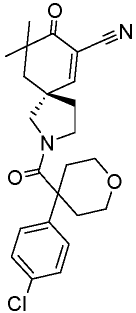 | (5S)-2-[4-(4-chlorophenyl)oxane-4-carbonyl]-9,9-dimethyl-8-oxo-2-azaspiro[4.5]dec-6-ene-7-carbonitrile | 427.0 | A |
| 383 | 2 | 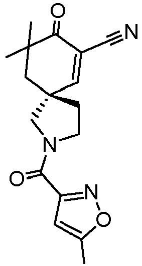 | (5S)-9,9-dimethyl-2-(5-methyl-1,2-oxazole-3-carbonyl)-8-oxo-2-azaspiro[4.5]dec-6-ene-7-carbonitrile | 314.0 | A |
| 384 | 2 | 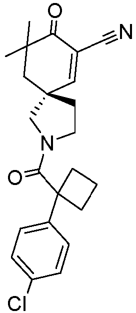 | (5S)-2-[1-(4-chlorophenyl)cyclobutan e-1-carbonyl]-9,9-dimethyl-8-oxo-2-azaspiro[4.5]dec-6-ene-7-carbonitrile | 397.0 | A |
| 385 | 2 | 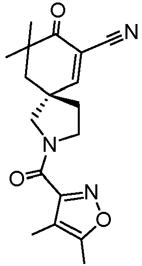 | (5S)-2-(4,5-dimethyl-1,2-oxazole-3-carbonyl)-9,9-dimethyl-8-oxo-2-azaspiro[4.5]dec-6-ene-7-carbonitrile | 328.1 | A |
| 386 | 2 | 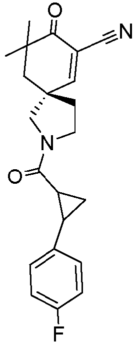 | (5S)-2-[2-(4-fluorophenyl)cyclopropa ne-1-carbonyl]-9,9-dimethyl-8-oxo-2-azaspiro[4.5]dec-6-ene-7-carbonitrile | 367.0 | A |
| 387 | 2 | 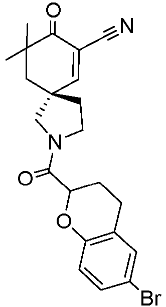 | (5S)-2-(6-bromo-3,4-dihydro-2H-1-benzopyran-2-carbonyl)-9,9-dimethyl-8-oxo-2-azaspiro[4.5]dec-6-ene-7-carbonitrile | 442.9, 445.0 | A |
| 388 | 2 | 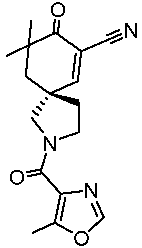 | (5S)-9,9-dimethyl-2-(5-methyl-1,3-oxazole-4-carbonyl)-8-oxo-2-azaspiro[4.5]dec-6-ene-7-carbonitrile | 314.0 | A |
| 389 | 2 | 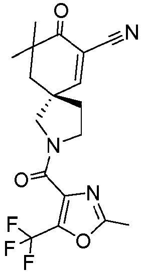 | (5S)-9,9-dimethyl-2-[2-methyl-5-(trifluoromethyl)-1,3-oxazole-4-carbonyl]-8-oxo-2-azaspiro[4.5]dec-6-ene-7-carbonitrile | 382.0 | A |
| 390 | 2 | 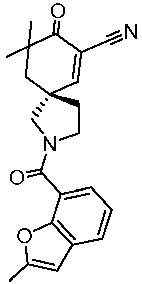 | (5S)-9,9-dimethyl-2-(2-methyl-1-benzofuran-7-carbonyl)-8-oxo-2-azaspiro[4.5]dec-6-ene-7-carbonitrile | 363.0 | A |
| 391 | 2 | 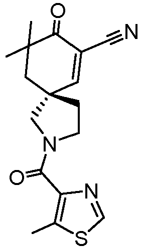 | (5S)-9,9-dimethyl-2-(5-methyl-1,3-thiazole-4-carbonyl)-8-oxo-2-azaspiro[4.5]dec-6-ene-7-carbonitrile | 330.0 | A |
| 392 | 2 | 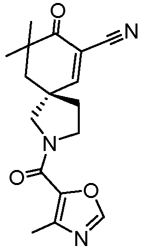 | (5S)-9,9-dimethyl-2-(4-methyl-1,3-oxazole-5-carbonyl)-8-oxo-2-azaspiro[4.5]dec-6-ene-7-carbonitrile | 314.0 | A |
| 393 | 2 | 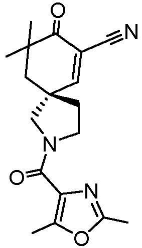 | (5S)-2-(2,5-dimethyl-1,3-oxazole-4-carbonyl)-9,9-dimethyl-8-oxo-2-azaspiro[4.5]dec-6-ene-7-carbonitrile | 328.0 | A |
| 394 | 2 | 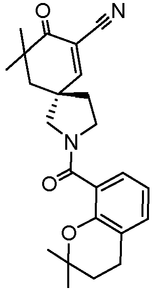 | (5S)-2-(2,2-dimethyl-3,4-dihydro-2H-1-benzopyran-8-carbonyl)-9,9-dimethyl-8-oxo-2-azaspiro[4.5]dec-6-ene-7-carbonitrile | 393.1 | A |
| 395 | 2 | 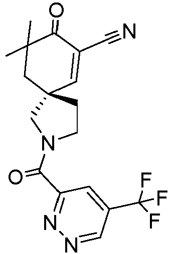 | (5S)-9,9-dimethyl-8-oxo-2-[5-(trifluoromethyl)pyridazi ne-3-carbonyl]-2-azaspiro[4.5]dec-6-ene-7-carbonitrile | 379.0 | A |
| 396 | 2 | 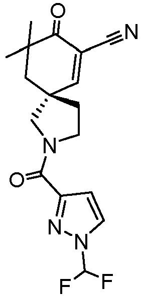 | (5S)-2-[1-(difluoromethyl)-1H-pyrazole-3-carbonyl]-9,9-dimethyl-8-oxo-2-azaspiro[4.5]dec-6-ene-7-carbonitrile | 349.0 | A |
| 397 | 2 | 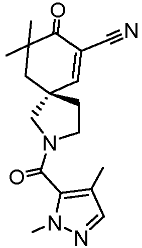 | (5S)-2-(1,4-dimethyl-1H-pyrazole-5-carbonyl)-9,9-dimethyl-8-oxo-2-azaspiro[4.5]dec-6-ene-7-carbonitrile | 327.0 | A |
| 398 | 2 | 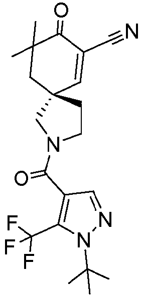 | (5S)-2-[1-tert-butyl-5-(trifluoromethyl)-1H-pyrazole-4-carbonyl]-9,9-dimethyl-8-oxo-2-azaspiro[4.5]dec-6-ene-7-carbonitrile | 423.1 | A |
| 399 | 2 | 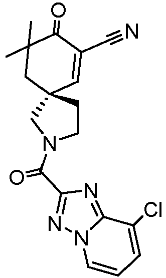 | (5S)-2-(8-chloro[1,2,4]triazolo[1,5-a]pyridine-2-carbonyl)-9,9-dimethyl-8-oxo-2-azaspiro[4.5]dec-6-ene-7-carbonitrile | 384.0 | A |
| 400 | 2 | 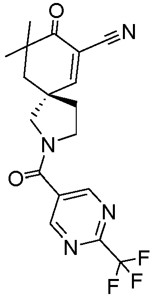 | (5S)-9,9-dimethyl-8-oxo-2-[2-(trifluoromethyl)pyrimidi ne-5-carbonyl]-2-azaspiro[4.5]dec-6-ene-7-carbonitrile | 379.0 | A |
| 401 | 2 | 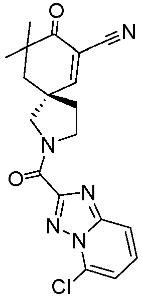 | (5S)-2-(5-chloro[1,2,4]triazolo[1,5-a]pyridine-2-carbonyl)-9,9-dimethyl-8-oxo-2-azaspiro[4.5]dec-6-ene-7-carbonitrile | 384.0 | A |
| 402 | 2 | 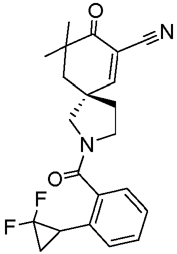 | (5S)-2-[2-(2,2-difluorocyclopropyl)benz ene-1-carbonyl]-9,9-dimethyl-8-oxo-2-azaspiro[4.5]dec-6-ene-7-carbonitrile | 385.0 | A |
| 403 | 2 | 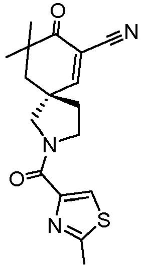 | (5S)-9,9-dimethyl-2-(2-methyl-1,3-thiazole-4-carbonyl)-8-oxo-2-azaspiro[4.5]dec-6-ene-7-carbonitrile | 330.0 | A |

### General procedure for Table 14

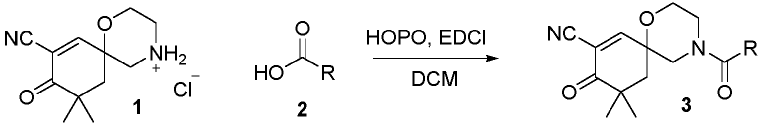

In 8 mL reaction vials were added 10,10-dimethyl-9-oxo-1-oxa-4-azaspiro[5.5]undec-7-ene-8-carbonitrile (15.4 mg, 70 µmol), Monomers (105 µmol), 2-hydroxypyridine n-oxide (11.6 mg, 105 µmol), N,N-Diisopropylethylamine (27.1 mg, 210 µmol) and EDC hydrochloride (20.1 mg, 105 µmol) in DCM (700 µL) under N₂ atmosphere to give a solution. The vials were capped and shaken at 65 °C for 16 hrs. After the reactions were completed, the reaction mixtures were concentrated by Speedvac. The residue was dissolved in ACN / H₂O and purified by preparative HPLC to give pure final product.

**Table 14: Compounds were prepared in a similar manner to the general procedure**

| **Ex.** | **Ena ntio mer** | **Structure** | **IUPAC Name** | **Exact Mass [M+H]+** |
|---|---|---|---|---|
| 404 | 1 | 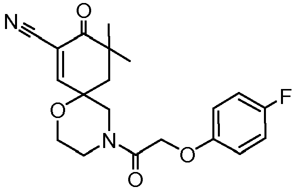 | 4-[(4-fluorophenoxy)acetyl]-10,10-dimethyl-9-oxo-1-oxa-4-azaspiro[5.5]undec-7-ene-8-carbonitrile | Calc'd 373.0, found 373.0 |
| 405 | 1 | 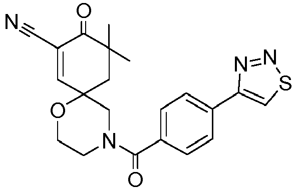 | 10,10-dimethyl-9-oxo-4-[4-(1,2,3-thiadiazol-4-yl)benzene-1-carbonyl]-1-oxa-4-azaspiro[5.5]undec-7-ene-8-carbonitrile | Calc'd 409.0, found 409.0 |
| 406 | 1 | 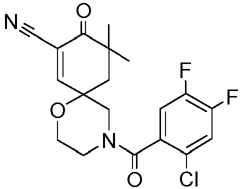 | 4-(2-chloro-4,5-difluorobenzene-1-carbonyl)-10,10-dimethyl-9-oxo-1-oxa-4-azaspiro[5.5]undec-7-ene-8-carbonitrile | Calc'd 395.0, found 395.0 |
| 407 | 1 | 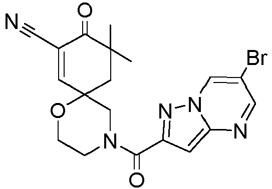 | 4-(6-bromopyrazolo[1,5-a]pyrimidine-2-carbonyl)-10, 10-dimethyl-9-oxo-1-oxa-4-azaspiro[5.5]undec-7-ene-8-carbonitrile | Calc'd 444.0, found 443.9, 446.0 |
| 408 | 1 | 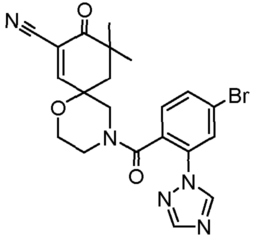 | 4-[4-bromo-2-(1H-1,2,4-triazol-1-yl)benzene-1-carbonyl]-10,10-dimethyl-9-oxo-1-oxa-4-azaspiro[5.5]undec-7-ene-8-carbonitrile | Calc'd 470.0, found 470.0, 472.0 |
| 409 | 1 | 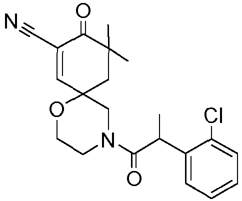 | 4-[2-(2-chlorophenyl)propanoyl]-10,10-dimethyl-9-oxo-1-oxa-4-azaspiro[5.5]undec-7-ene-8-carbonitrile | Calc'd 387.0, found 387.0 |
| 410 | 1 | 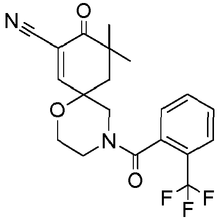 | 10,10-dimethyl-9-oxo-4-[2-(trifluoromethyl)benzene-1-carbonyl]-1-oxa-4-azaspiro[5.5]undec-7-ene-8-carbonitrile | Calc'd 393.0, found 393.0 |
| 411 | 1 | 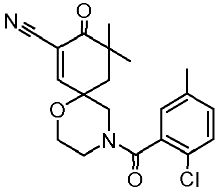 | 4-(2-chloro-5-methylbenzene-1-carbonyl)-10,10-dimethyl-9-oxo-1-oxa-4-azaspiro[5.5]undec-7-ene-8-carbonitrile | Calc'd 373.0, found 373.0 |
| 412 | 1 | 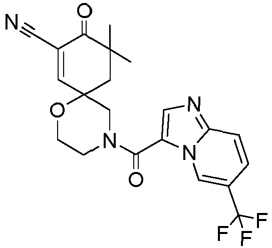 | 10,10-dimethyl-9-oxo-4-[6-(trifluoromethyl)imidazo[1,2-a]pyridine-3-carbonyl]-1-oxa-4-azaspiro[5.5]undec-7-ene-8-carbonitrile | Calc'd 433.0, found 433.0 |
| 413 | 1 | 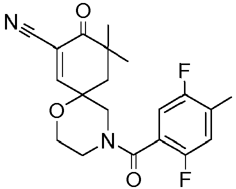 | 4-(2,5-difluoro-4-methylbenzene-1-carbonyl)-10,10-dimethyl-9-oxo-1-oxa-4-azaspiro[5.5]undec-7-ene-8-carbonitrile | Calc'd 375.0, found 375.0 |
| 414 | 1 | 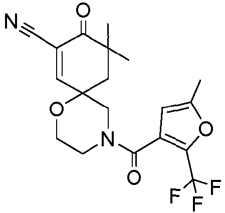 | 10,10-dimethyl-4-[5-methyl-2-(trifluoromethyl)furan-3-carbonyl]-9-oxo-1-oxa-4-azaspiro[5.5]undec-7-ene-8-carbonitrile | Calc'd 397.0, found 397.0 |
| 415 | 1 | 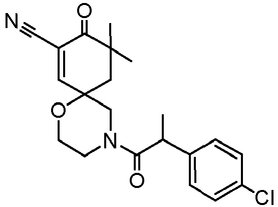 | 4-[2-(4-chlorophenyl)propanoyl]-10,10-dimethyl-9-oxo-1-oxa-4-azaspiro[5.5]undec-7-ene-8-carbonitrile | Calc'd 387.0, found 387.0 |
| 416 | 1 | 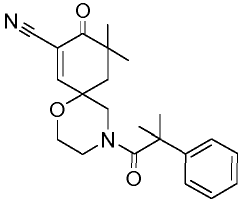 | 10,10-dimethyl-4-(2-methyl-2-phenylpropanoyl)-9-oxo-1-oxa-4-azaspiro[5.5]undec-7-ene-8-carbonitrile | Calc'd 367.0, found 367.1 |
| 417 | 1 | 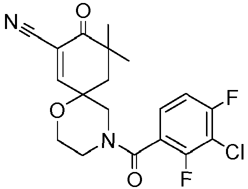 | 4-(3-chloro-2,4-difluorobenzene-1-carbonyl)-10,10-dimethyl-9-oxo-1-oxa-4-azaspiro[5.5]undec-7-ene-8-carbonitrile | Calc'd 395.0, found 395.0 |
| 418 | 1 | 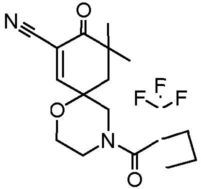 | 10,10-dimethyl-9-oxo-4-[1-(trifluoromethyl)cyclobutane-1-carbonyl]-1-oxa-4-azaspiro[5.5]undec-7-ene-8-carbonitrile | Calc'd 371.0, found 371.0 |
| 419 | 1 | 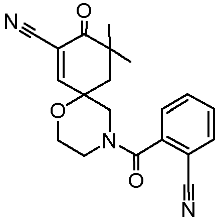 | 4-(2-cyanobenzene-1-carbonyl)-10,10-dimethyl-9-oxo-1-oxa-4-azaspiro[5.5]undec-7-ene-8-carbonitrile | Calc'd 350.0, found 350.0 |
| 420 | 1 | 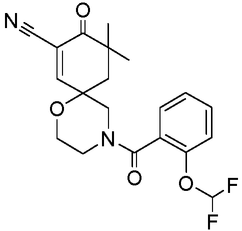 | 4-[2-(difluoromethoxy)benzene-1-carbonyl]-10,10-dimethyl-9-oxo-1-oxa-4-azaspiro[5.5]undec-7-ene-8-carbonitrile | Calc'd 391.0, found 391.0 |
| 421 | 1 | 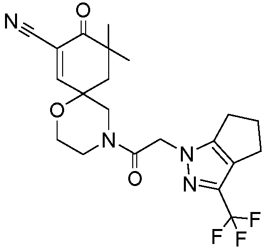 | 10,10-dimethyl-9-oxo-4-{[3-(trifluoromethyl)-5,6-dihydrocyclopenta[c]pyrazol-1 (4H)-yl] acetyl }-1-oxa-4-azaspiro[5.5]undec-7-ene-8-carbonitrile | Calc'd 437.0, found 437.0 |
| 422 | 1 | 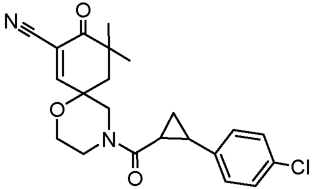 | 4-[2-(4-chlorophenyl)cyclopropane-1-carbonyl]-10,10-dimethyl-9-oxo-1-oxa-4-azaspiro[5.5]undec-7-ene-8-carbonitrile | Calc'd 399.0, found 399.0 |
| 423 | 1 | 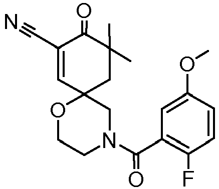 | 4-(2-fluoro-5-methoxybenzene-1-carbonyl)-10,10-dimethyl-9-oxo-1-oxa-4-azaspiro[5.5]undec-7-ene-8-carbonitrile | Calc'd 373.0, found 373.0 |
| 424 | 1 | 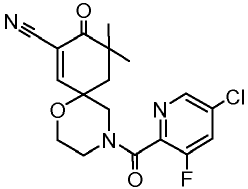 | 4-(5-chloro-3-fluoropyridine-2-carbonyl)-10, 10-dimethyl-9-oxo-1-oxa-4-azaspiro[5.5]undec-7-ene-8-carbonitrile | Calc'd 378.0, found 378.0 |
| 425 | 1 | 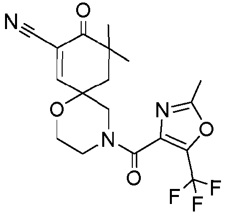 | 10,10-dimethyl-4-[2-methyl-5-(trifluoromethyl)-1,3-oxazole-4-carbonyl]-9-oxo-1-oxa-4-azaspiro[5.5]undec-7-ene-8-carbonitrile | Calc'd 398.0, found 398.0 |
| 426 | 1 | 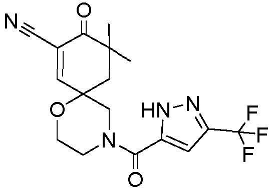 | 10,10-dimethyl-9-oxo-4-[3-(trifluoromethyl)-1H-pyrazole-5-carbonyl]-1-oxa-4-azaspiro[5.5]undec-7-ene-8-carbonitrile | Calc'd 383.0, found 383.0 |
| 427 | 1 | 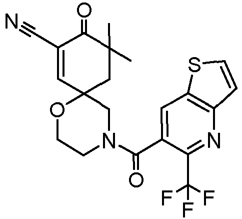 | 10,10-dimethyl-9-oxo-4-[5-(trifluoromethyl)thieno[3,2-b]pyridine-6-carbonyl]-1-oxa-4-azaspiro[5.5]undec-7-ene-8-carbonitrile | Calc'd 450.0, found 450.0 |
| 428 | 1 | 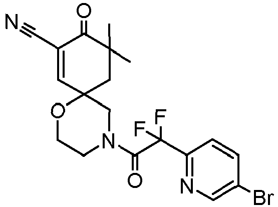 | 4-[(5-bromopyridin-2-yl)(difluoro)acetyl]-10, 10-dimethyl-9-oxo-1-oxa-4-azaspiro[5.5]undec-7-ene-8-carbonitrile | Calc'd 454.0, found 453.9, 455.9 |
| 429 | 1 | 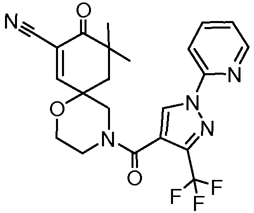 | 10,10-dimethyl-9-oxo-4-[1-(pyridin-2-yl)-3-(trifluoromethyl)-1H-pyrazole-4-carbonyl]-1-oxa-4-azaspiro[5.5]undec-7-ene-8-carbonitrile | Calc'd 460.0, found 460.0 |
| 430 | 1 | 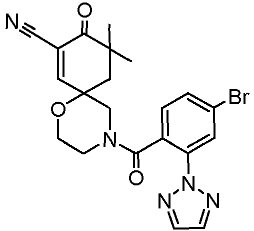 | 4-[4-bromo-2-(2H-1,2,3-triazol-2-yl)benzene-1-carbonyl]-10,10-dimethyl-9-oxo-1-oxa-4-azaspiro[5.5]undec-7-ene-8-carbonitrile | Calc'd 470.0, found 470.0, 471.9 |
| 431 | 1 | 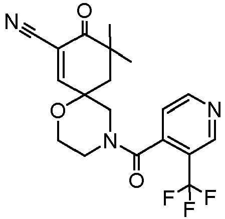 | 10,10-dimethyl-9-oxo-4-[3-(trifluoromethyl)pyridine-4-carbonyl]-1-oxa-4-azaspiro[5.5]undec-7-ene-8-carbonitrile | Calc'd 394.0, found 394.0 |
| 432 | 1 | 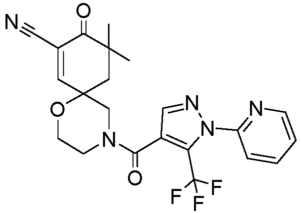 | 10,10-dimethyl-9-oxo-4-[1-(pyridin-2-yl)-5-(trifluoromethyl)-1H-pyrazole-4-carbonyl]-1-oxa-4-azaspiro[5.5]undec-7-ene-8-carbonitrile | Calc'd 460.0, found 460.0 |
| 433 | 1 | 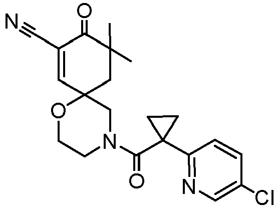 | 4-[1-(5-chloropyridin-2-yl)cyclopropane-1-carbonyl]-10,10-dimethyl-9-oxo-1-oxa-4-azaspiro[5.5]undec-7-ene-8-carbonitrile | Calc'd 400.0, found 400.0 |
| 434 | 1 | 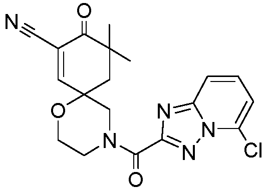 | 4-(5-chloro[1,2,4]triazolo[1,5-a]pyridine-2-carbonyl)-10,10-dimethyl-9-oxo-1-oxa-4-azaspiro[5.5]undec-7-ene-8-carbonitrile | Calc'd 400.0, found 400.0 |
| 435 | 1 | 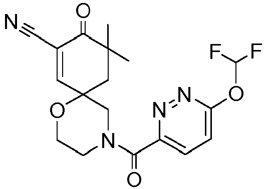 | 4-[6-(difluoromethoxy)pyridazine-3-carbonyl]-10,10-dimethyl-9-oxo-1-oxa-4-azaspiro[5.5]undec-7-ene-8-carbonitrile | Calc'd 393.0, found 393.0 |
| 436 | 1 | 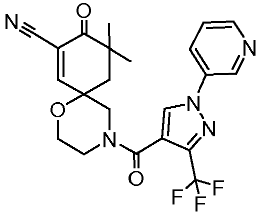 | 10,10-dimethyl-9-oxo-4-[1-(pyridin-3-yl)-3-(trifluoromethyl)-1H-pyrazole-4-carbonyl]-1-oxa-4-azaspiro[5.5]undec-7-ene-8-carbonitrile | Calc'd 460.0, found 460.0 |
| 437 | 1 | 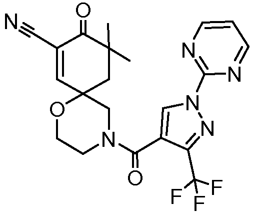 | 10,10-dimethyl-9-oxo-4-[1-(pyrimidin-2-yl)-3-(trifluoromethyl)-1H-pyrazole-4-carbonyl]-1-oxa-4-azaspiro[5.5]undec-7-ene-8-carbonitrile | Calc'd 461.0, found 461.0 |
| 438 | 1 | 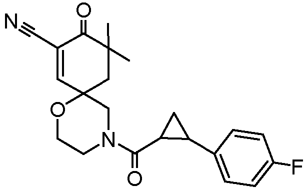 | 4-[2-(4-fluorophenyl)cyclopropane-1-carbonyl]-10,10-dimethyl-9-oxo-1-oxa-4-azaspiro[5.5]undec-7-ene-8-carbonitrile | Calc'd 383.0, found 383.0 |
| 439 | 1 | 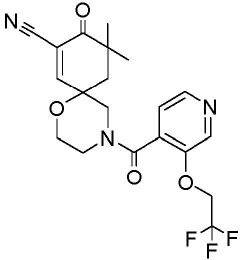 | 10,10-dimethyl-9-oxo-4-[3-(2,2,2-trifluoroethoxy)pyridine-4-carbonyl]-1-oxa-4-azaspiro[5.5]undec-7-ene-8-carbonitrile | Calc'd 424.0, found 424.0 |
| 440 | 1 | 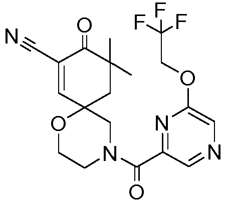 | 10,10-dimethyl-9-oxo-4-[6-(2,2,2-trifluoroethoxy)pyrazine-2-carbonyl]-1-oxa-4-azaspiro[5.5]undec-7-ene-8-carbonitrile | Calc'd 425.0, found 425.0 |
| 441 | 1 | 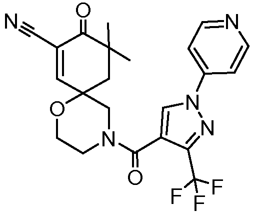 | 10,10-dimethyl-9-oxo-4-[1-(pyridin-4-yl)-3-(trifluoromethyl)-1H-pyrazole-4-carbonyl]-1-oxa-4-azaspiro[5.5]undec-7-ene-8-carbonitrile | Calc'd 460.0, found 460.0 |
| 442 | 1 | 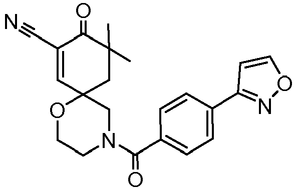 | 10,10-dimethyl-4-[4-(1,2-oxazol-3-yl)benzene-1-carbonyl]-9-oxo-1-oxa-4-azaspiro[5.5]undec-7-ene-8-carbonitrile | Calc'd 392.0, found 392.0 |
| 443 | 1 | 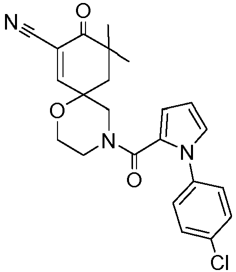 | 4-[1-(4-chlorophenyl)-1H-pyrrole-2-carbonyl]-10,10-dimethyl-9-oxo-1-oxa-4-azaspiro[5.5]undec-7-ene-8-carbonitrile | Calc'd 424.0, found 424.0 |
| 444 | 1 | 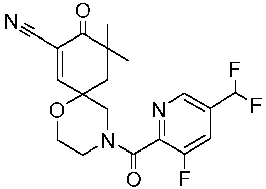 | 4-[5-(difluoromethyl)-3-fluoropyridine-2-carbonyl]-10,10-dimethyl-9-oxo-1-oxa-4-azaspiro[5.5]undec-7-ene-8-carbonitrile | Calc'd 394.0, found 394.0 |
| 445 | 1 | 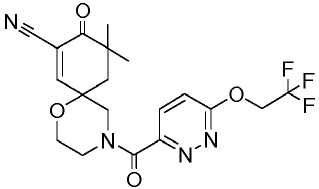 | 10,10-dimethyl-9-oxo-4-[6-(2,2,2-trifluoroethoxy)pyridazine-3-carbonyl]-1-oxa-4-azaspiro[5.5]undec-7-ene-8-carbonitrile | Calc'd 425.0, found 425.0 |
| 446 | 1 | 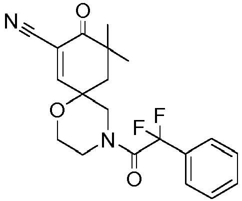 | 4-[difluoro(phenyl)acetyl]-10,10-dimethyl-9-oxo-1-oxa-4-azaspiro[5.5]undec-7-ene-8-carbonitrile | Calc'd 375.0, found 375.0 |
| 447 | 1 | 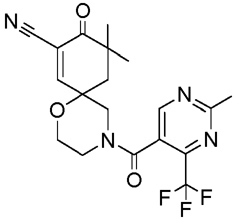 | 10,10-dimethyl-4-[2-methyl-4-(trifluoromethyl)pyrimidine-5-carbonyl]-9-oxo-1-oxa-4-azaspiro[5.5]undec-7-ene-8-carbonitrile | Calc'd 409.0, found 409.0 |
| 448 | 1 | 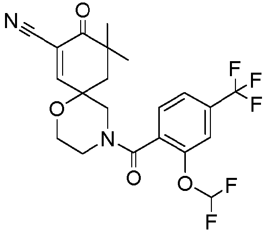 | 4-[2-(difluoromethoxy)-4-(trifluoromethyl)benzene-1-carbonyl]-10,10-dimethyl-9-oxo-1-oxa-4-azaspiro[5.5]undec-7-ene-8-carbonitrile | Calc'd 459.0, found 459.0 |
| 449 | 1 | 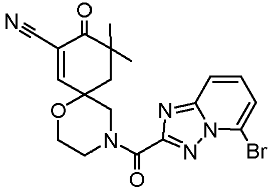 | 4-(5-bromo[1,2,4]triazolo[1,5-a]pyridine-2-carbonyl)-10,10-dimethyl-9-oxo-1-oxa-4-azaspiro[5.5]undec-7-ene-8-carbonitrile | Calc'd 444.0, found 444.0, 445.9 |
| 450 | 1 | 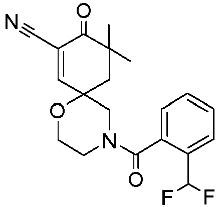 | 4-[2-(difluoromethyl)benzene-1-carbonyl]-10,10-dimethyl-9-oxo-1-oxa-4-azaspiro[5.5]undec-7-ene-8-carbonitrile | Calc'd 375.0, found 375.0 |
| 451 | 1 | 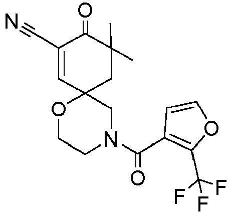 | 10,10-dimethyl-9-oxo-4-[2-(trifluoromethyl)furan-3-carbonyl]-1-oxa-4-azaspiro[5.5]undec-7-ene-8-carbonitrile | Calc'd 383.0, found 383.0 |
| 452 | 1 | 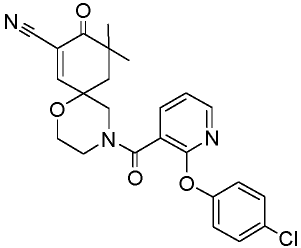 | 4-[2-(4-chlorophenoxy)pyridine-3-carbonyl]-10,10-dimethyl-9-oxo-1-oxa-4-azaspiro[5.5]undec-7-ene-8-carbonitrile | Calc'd 452.0, found 452.0 |
| 453 | 1 | 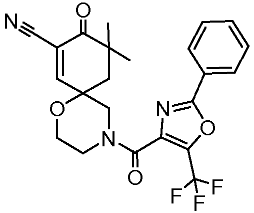 | 10,10-dimethyl-9-oxo-4-[2-phenyl-5-(trifluoromethyl)-1,3-oxazole-4-carbonyl]-1-oxa-4-azaspiro[5.5]undec-7-ene-8-carbonitrile | Calc'd 460.0, found 460.0 |
| 454 | 1 | 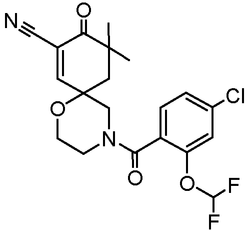 | 4-[4-chloro-2-(difluoromethoxy)benzene-1-carbonyl]-10,10-dimethyl-9-oxo-1-oxa-4-azaspiro[5.5]undec-7-ene-8-carbonitrile | Calc'd 425.0, found 425.0 |
| 455 | 1 | 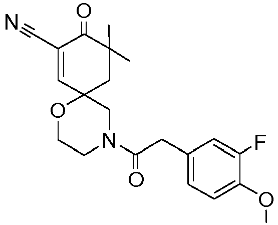 | 4-[(3-fluoro-4-methoxyphenyl)acetyl]-10,10-dimethyl-9-oxo-1-oxa-4-azaspiro[5.5]undec-7-ene-8-carbonitrile | Calc'd 387.0, found 387.0 |
| 456 | 1 | 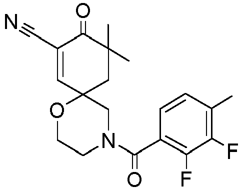 | 4-(2,3-difluoro-4-methylbenzene-1-carbonyl)-10,10-dimethyl-9-oxo-1-oxa-4-azaspiro[5.5]undec-7-ene-8-carbonitrile | Calc'd 375.0, found 375.0 |
| 457 | 1 | 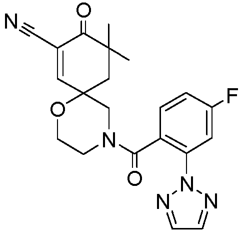 | 4-[4-fluoro-2-(2H-1,2,3-triazol-2-yl)benzene-1-carbonyl]-10,10-dimethyl-9-oxo-1-oxa-4-azaspiro[5.5]undec-7-ene-8-carbonitrile | Calc'd 410.0, found 410.0 |
| 458 | 1 | 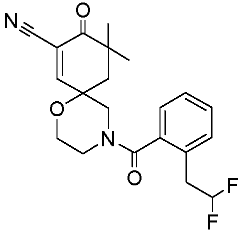 | 4-[2-(2,2-difluoroethyl)benzene-1-carbonyl]-10,10-dimethyl-9-oxo-1-oxa-4-azaspiro[5.5]undec-7-ene-8-carbonitrile | Calc'd 389.0, found 389.0 |
| 459 | 1 | 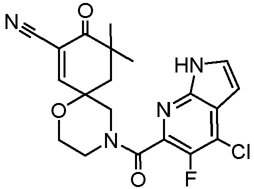 | 4-(4-chloro-5-fluoro-1H-pyrrolo[2,3-b]pyridine-6-carbonyl)-10, 10-dimethyl-9-oxo-1-oxa-4-azaspiro[5.5]undec-7-ene-8-carbonitrile | Calc'd 417.0, found 416.9 |
| 460 | 1 | 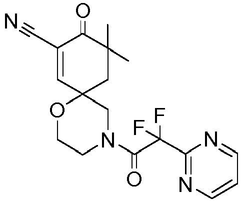 | 4-[difluoro(pyrimidin-2-yl)acetyl]-10,10-dimethyl-9-oxo-1-oxa-4-azaspiro[5.5]undec-7-ene-8-carbonitrile | Calc'd 377.0, found 377.0 |
| 461 | 1 | 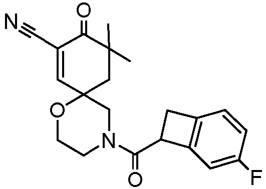 | 4-(4-fluorobicyclo[4.2.0]octa-1,3,5-triene-7-carbonyl)-10,10-dimethyl-9-oxo-1-oxa-4-azaspiro[5.5]undec-7-ene-8-carbonitrile | Calc'd 369.0, found 369.0 |
| 462 | 2 | 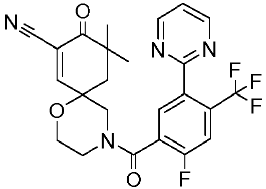 | 4-[2-fluoro-5-(pyrimidin-2-yl)-4-(trifluoromethyl)benzene-1-carbonyl]-10,10-dimethyl-9-oxo-1-oxa-4-azaspiro[5.5]undec-7-ene-8-carbonitrile | Calc'd 489.0, found 489.0 |
| 463 | 2 | 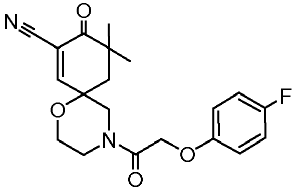 | 4-[(4-fluorophenoxy)acetyl]-10,10-dimethyl-9-oxo-1-oxa-4-azaspiro[5.5]undec-7-ene-8-carbonitrile | Calc'd 373.0, found 373.0 |
| 464 | 2 | 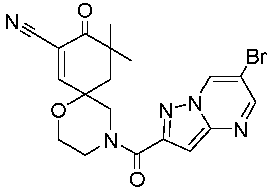 | 4-(6-bromopyrazolo[1,5-a]pyrimidine-2-carbonyl)-10,10-dimethyl-9-oxo-1-oxa-4-azaspiro[5.5]undec-7-ene-8-carbonitrile | Calc'd 444.0, found 443.9, 445.9 |
| 465 | 2 | 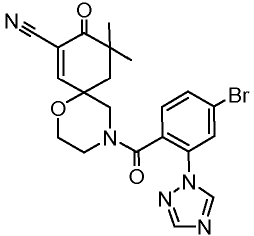 | 4-[4-bromo-2-(1H-1,2,4-triazol-1-yl)benzene-1-carbonyl]-10,10-dimethyl-9-oxo-1-oxa-4-azaspiro[5.5]undec-7-ene-8-carbonitrile | Calc'd 470.0, found 470.0 |
| 466 | 2 | 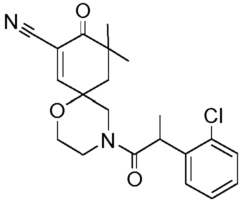 | 4-[2-(2-chlorophenyl)propanoyl]-10,10-dimethyl-9-oxo-1-oxa-4-azaspiro[5.5]undec-7-ene-8-carbonitrile | Calc'd 387.0, found 387.0 |
| 467 | 2 | 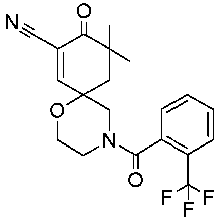 | 10,10-dimethyl-9-oxo-4-[2-(trifluoromethyl)benzene-1-carbonyl]-1-oxa-4-azaspiro[5.5]undec-7-ene-8-carbonitrile | Calc'd 393.0, found 393.0 |
| 468 | 2 | 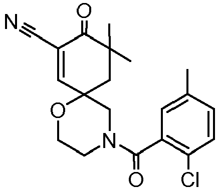 | 4-(2-chloro-5-methylbenzene-1-carbonyl)-10,10-dimethyl-9-oxo-1-oxa-4-azaspiro[5.5]undec-7-ene-8-carbonitrile | Calc'd 373.0, found 373.0 |
| 469 | 2 | 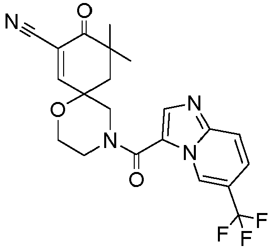 | 10,10-dimethyl-9-oxo-4-[6-(trifluoromethyl)imidazo[1,2-a]pyridine-3-carbonyl]-1-oxa-4-azaspiro[5.5]undec-7-ene-8-carbonitrile | Calc'd 433.0, found 433.0 |
| 470 | 2 | 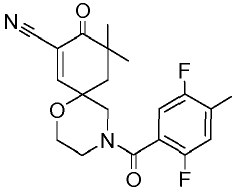 | 4-(2,5-difluoro-4-methylbenzene-1-carbonyl)-10,10-dimethyl-9-oxo-1-oxa-4-azaspiro[5.5]undec-7-ene-8-carbonitrile | Calc'd 375.0, found 375.0 |
| 471 | 2 | 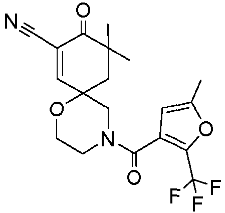 | 10,10-dimethyl-4-[5-methyl-2-(trifluoromethyl)furan-3-carbonyl]-9-oxo-1-oxa-4-azaspiro[5.5]undec-7-ene-8-carbonitrile | Calc'd 397.0, found 397.0 |
| 472 | 2 | 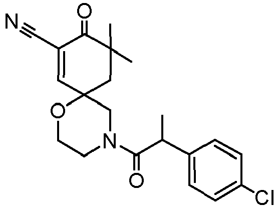 | 4-[2-(4-chlorophenyl)propanoyl]-10,10-dimethyl-9-oxo-1-oxa-4-azaspiro[5.5]undec-7-ene-8-carbonitrile | Calc'd 387.0, found 387.0 |
| 473 | 2 | 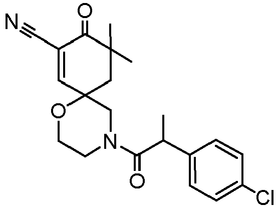 | 4-[2-(4-chlorophenyl)propanoyl]-10,10-dimethyl-9-oxo-1-oxa-4-azaspiro[5.5]undec-7-ene-8-carbonitrile | Calc'd 387.0, found 387.0 |
| 474 | 2 | 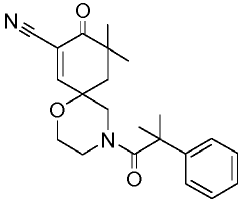 | 10,10-dimethyl-4-(2-methyl-2-phenylpropanoyl)-9-oxo-1-oxa-4-azaspiro[5.5]undec-7-ene-8-carbonitrile | Calc'd 367.0, found 367.1 |
| 475 | 2 | 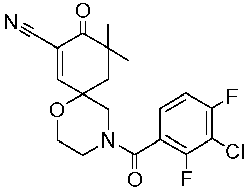 | 4-(3-chloro-2,4-difluorobenzene-1-carbonyl)-10,10-dimethyl-9-oxo-1-oxa-4-azaspiro[5.5]undec-7-ene-8-carbonitrile | Calc'd 395.0, found 395.0 |
| 476 | 2 | 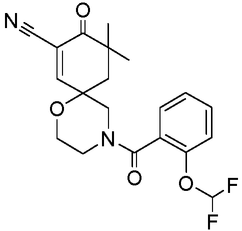 | 4-[2-(difluoromethoxy)benzene-1-carbonyl]-10,10-dimethyl-9-oxo-1-oxa-4-azaspiro[5.5]undec-7-ene-8-carbonitrile | Calc'd 391.0, found 391.0 |
| 477 | 2 | 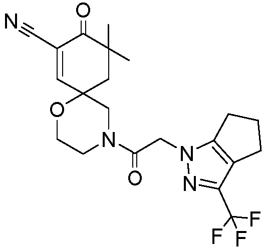 | 10,10-dimethyl-9-oxo-4-{[3-(trifluoromethyl)-5,6-dihydrocyclopenta[c]pyrazol-1 (4H)-yl] acetyl }-1-oxa-4-azaspiro[5.5]undec-7-ene-8-carbonitrile | Calc'd 437.0, found 437.0 |
| 478 | 2 | 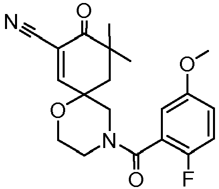 | 4-(2-fluoro-5-methoxybenzene-1-carbonyl)-10,10-dimethyl-9-oxo-1-oxa-4-azaspiro[5.5]undec-7-ene-8-carbonitrile | Calc'd 373.0, found 373.0 |
| 479 | 2 | 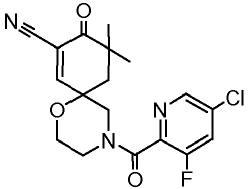 | 4-(5-chloro-3-fluoropyridine-2-carbonyl)-10,10-dimethyl-9-oxo-1-oxa-4-azaspiro[5.5]undec-7-ene-8-carbonitrile | Calc'd 378.0, found 378.0 |
| 480 | 2 | 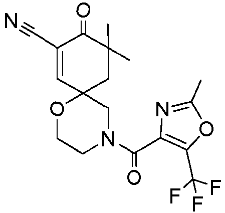 | 10,10-dimethyl-4-[2-methyl-5-(trifluoromethyl)-1,3-oxazole-4-carbonyl]-9-oxo-1-oxa-4-azaspiro[5.5]undec-7-ene-8-carbonitrile | Calc'd 398.0, found 398.0 |
| 481 | 2 | 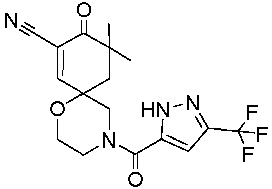 | 10,10-dimethyl-9-oxo-4-[3-(trifluoromethyl)-1H-pyrazole-5-carbonyl]-1-oxa-4-azaspiro[5.5]undec-7-ene-8-carbonitrile | Calc'd 383.0, found 383.0 |
| 482 | 2 | 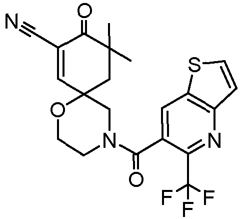 | 10,10-dimethyl-9-oxo-4-[5-(trifluoromethyl)thieno[3,2-b]pyridine-6-carbonyl]-1-oxa-4-azaspiro[5.5]undec-7-ene-8-carbonitrile | Calc'd 450.0, found 450.0 |
| 483 | 2 | 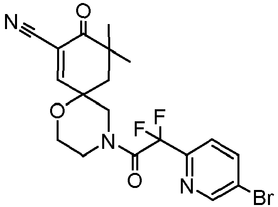 | 4-[(5-bromopyridin-2-yl)(difluoro)acetyl]-10,10-dimethyl-9-oxo-1-oxa-4-azaspiro[5.5]undec-7-ene-8-carbonitrile | Calc'd 454.0, found 453.9, 455.9 |
| 484 | 2 | 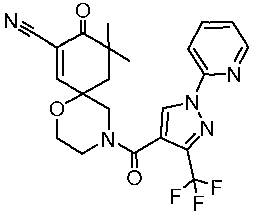 | 10,10-dimethyl-9-oxo-4-[1-(pyridin-2-yl)-3-(trifluoromethyl)-1H-pyrazole-4-carbonyl]-1-oxa-4-azaspiro[5.5]undec-7-ene-8-carbonitrile | Calc'd 460.0, found 460.0 |
| 485 | 1 | 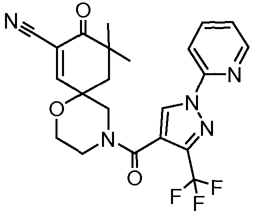 | 10,10-dimethyl-9-oxo-4-[1-(pyridin-2-yl)-3-(trifluoromethyl)-1H-pyrazole-4-carbonyl]-1-oxa-4-azaspiro[5.5]undec-7-ene-8-carbonitrile | Calc'd 460.0, found 460.0 |
| 486 | 2 | 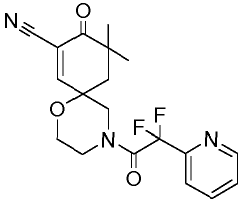 | 4-[difluoro(pyridin-2-yl)acetyl]-10,10-dimethyl-9-oxo-1-oxa-4-azaspiro[5.5]undec-7-ene-8-carbonitrile | Calc'd 376.0, found 376.0 |
| 487 | 2 | 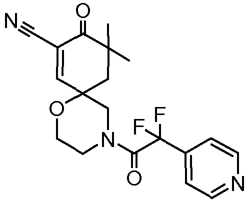 | 4-[difluoro(pyridin-4-yl)acetyl]-10,10-dimethyl-9-oxo-1-oxa-4-azaspiro[5.5]undec-7-ene-8-carbonitrile | Calc'd 376.0, found 376.0 |
| 488 | 2 | 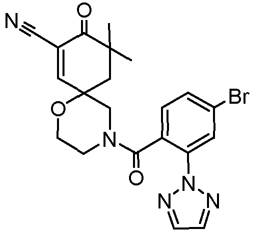 | 4-[4-bromo-2-(2H-1,2,3-triazol-2-yl)benzene-1-carbonyl]-10,10-dimethyl-9-oxo-1-oxa-4-azaspiro[5.5]undec-7-ene-8-carbonitrile | Calc'd 470.0, found 470.0, 472.0 |
| 489 | 2 | 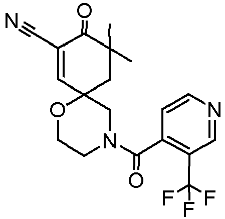 | 10,10-dimethyl-9-oxo-4-[3-(trifluoromethyl)pyridine-4-carbonyl]-1-oxa-4-azaspiro[5.5]undec-7-ene-8-carbonitrile | Calc'd 394.0, found 394.0 |
| 490 | 2 | 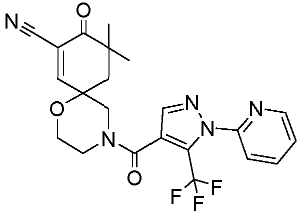 | 10,10-dimethyl-9-oxo-4-[1-(pyridin-2-yl)-5-(trifluoromethyl)-1H-pyrazole-4-carbonyl]-1-oxa-4-azaspiro[5.5]undec-7-ene-8-carbonitrile | Calc'd 460.0, found 460.0 |
| 491 | 2 | 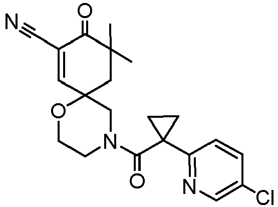 | 4-[1-(5-chloropyridin-2-yl)cyclopropane-1-carbonyl]-10,10-dimethyl-9-oxo-1-oxa-4-azaspiro[5.5]undec-7-ene-8-carbonitrile | Calc'd 400.0, found 400.0 |
| 492 | 2 | 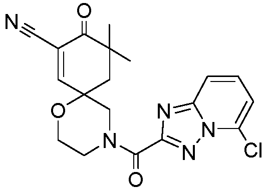 | 4-(5-chloro[1,2,4]triazolo[1,5-a]pyridine-2-carbonyl)-10,10-dimethyl-9-oxo-1-oxa-4-azaspiro[5.5]undec-7-ene-8-carbonitrile | Calc'd 400.0, found 400.0 |
| 493 | 2 | 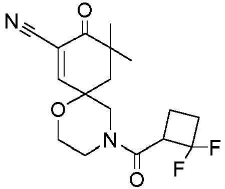 | 4-(2,2-difluorocyclobutane-1-carbonyl)-10,10-dimethyl-9-oxo-1-oxa-4-azaspiro[5.5]undec-7-ene-8-carbonitrile | Calc'd 339.0, found 339.0 |
| 494 | 2 | 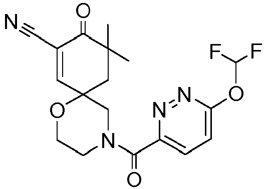 | 4-[6-(difluoromethoxy)pyridazine-3-carbonyl]-10,10-dimethyl-9-oxo-1-oxa-4-azaspiro[5.5]undec-7-ene-8-carbonitrile | Calc'd 393.0, found 393.0 |
| 495 | 2 | 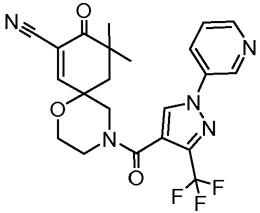 | 10,10-dimethyl-9-oxo-4-[1-(pyridin-3-yl)-3-(trifluoromethyl)-1H-pyrazole-4-carbonyl]-1-oxa-4-azaspiro[5.5]undec-7-ene-8-carbonitrile | Calc'd 460.0, found 460.0 |
| 496 | 2 | 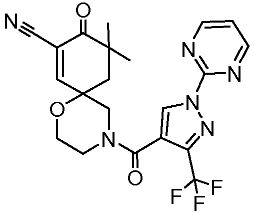 | 10,10-dimethyl-9-oxo-4-[1-(pyrimidin-2-yl)-3-(trifluoromethyl)-1H-pyrazole-4-carbonyl]-1-oxa-4-azaspiro[5.5]undec-7-ene-8-carbonitrile | Calc'd 461.0, found 461.0 |
| 497 | 2 | 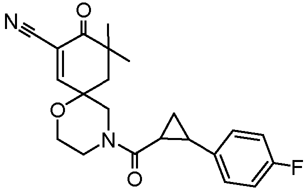 | 4-[2-(4-fluorophenyl)cyclopropane-1-carbonyl]-10,10-dimethyl-9-oxo-1-oxa-4-azaspiro[5.5]undec-7-ene-8-carbonitrile | Calc'd 383.0, found 383.0 |
| 498 | 2 | 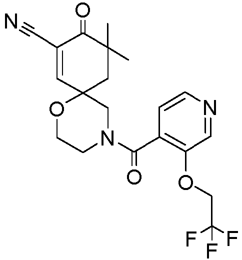 | 10,10-dimethyl-9-oxo-4-[3-(2,2,2-trifluoroethoxy)pyridine-4-carbonyl]-1-oxa-4-azaspiro[5.5]undec-7-ene-8-carbonitrile | Calc'd 424.0, found 424.0 |
| 499 | 2 | 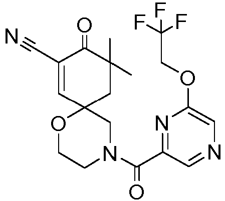 | 10,10-dimethyl-9-oxo-4-[6-(2,2,2-trifluoroethoxy)pyrazine-2-carbonyl]-1-oxa-4-azaspiro[5.5]undec-7-ene-8-carbonitrile | Calc'd 425.0, found 425.0 |
| 500 | 2 | 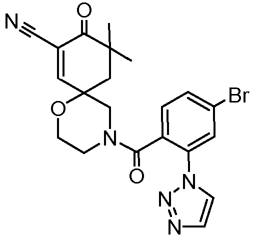 | 4-[4-bromo-2-(1H-1,2,3-triazol-1-yl)benzene-1-carbonyl]-10,10-dimethyl-9-oxo-1-oxa-4-azaspiro[5.5]undec-7-ene-8-carbonitrile | Calc'd 470.0, found 470.0, 472.0 |
| 501 | 2 | 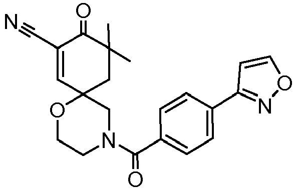 | 10,10-dimethyl-4-[4-(1,2-oxazol-3-yl)benzene-1-carbonyl]-9-oxo-1-oxa-4-azaspiro[5.5]undec-7-ene-8-carbonitrile | Calc'd 392.0, found 392.0 |
| 502 | 2 | 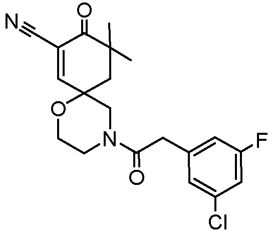 | 4-[(3-chloro-5-fluorophenyl)acetyl]-10,10-dimethyl-9-oxo-1-oxa-4-azaspiro[5.5]undec-7-ene-8-carbonitrile | Calc'd 391.0, found 391.0 |
| 503 | 2 | 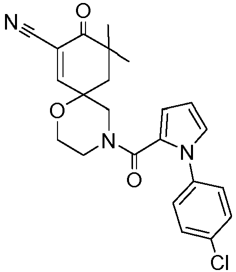 | 4-[1-(4-chlorophenyl)-1H-pyrrole-2-carbonyl]-10,10-dimethyl-9-oxo-1-oxa-4-azaspiro[5.5]undec-7-ene-8-carbonitrile | Calc'd 424.0, found 424.0 |
| 504 | 2 | 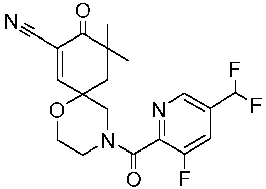 | 4-[5-(difluoromethyl)-3-fluoropyridine-2-carbonyl]-10,10-dimethyl-9-oxo-1-oxa-4-azaspiro[5.5]undec-7-ene-8-carbonitrile | Calc'd 394.0, found 394.0 |
| 505 | 2 | 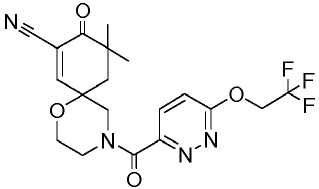 | 10,10-dimethyl-9-oxo-4-[6-(2,2,2-trifluoroethoxy)pyridazine-3-carbonyl]-1-oxa-4-azaspiro[5.5]undec-7-ene-8-carbonitrile | Calc'd 425.0, found 425.0 |
| 506 | 2 | 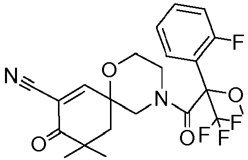 | 10,10-dimethyl-9-oxo-4-[3,3,3-trifluoro-2-(2-fluorophenyl)-2-methoxypropanoyl]-1-oxa-4-azaspiro[5.5]undec-7-ene-8-carbonitrile | Calc'd 455.0, found 455.1 |
| 507 | 2 | 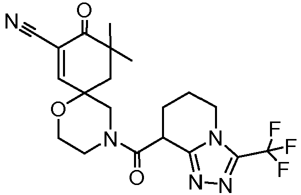 | 10,10-dimethyl-9-oxo-4-[3-(trifluoromethyl)-5,6,7,8-tetrahydro[1,2,4]triazolo[4,3-a]pyridine-8-carbonyl]-1-oxa-4-azaspiro[5.5]undec-7-ene-8-carbonitrile | Calc'd 438.0, found 438.0 |
| 508 | 2 | 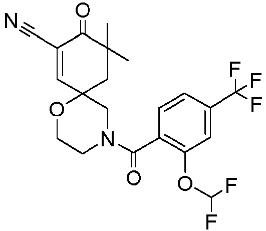 | 4-[2-(difluoromethoxy)-4-(trifluoromethyl)benzene-1-carbonyl]-10,10-dimethyl-9-oxo-1-oxa-4-azaspiro[5.5]undec-7-ene-8-carbonitrile | Calc'd 459.0, found 459.0 |
| 509 | 2 | 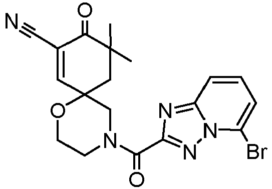 | 4-(5-bromo[1,2,4]triazolo[1,5-a]pyridine-2-carbonyl)-10,10-dimethyl-9-oxo-1-oxa-4-azaspiro[5.5]undec-7-ene-8-carbonitrile | Calc'd 444.0, found 443.9, 445.9 |
| 510 | 2 | 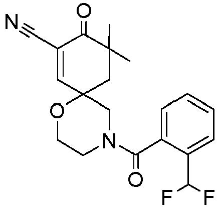 | 4-[2-(difluoromethyl)benzene-1-carbonyl]-10,10-dimethyl-9-oxo-1-oxa-4-azaspiro[5.5]undec-7-ene-8-carbonitrile | Calc'd 375.0, found 375.0 |
| 511 | 2 | 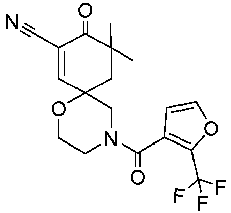 | 10,10-dimethyl-9-oxo-4-[2-(trifluoromethyl)furan-3-carbonyl]-1-oxa-4-azaspiro[5.5]undec-7-ene-8-carbonitrile | Calc'd 383.0, found 383.0 |
| 512 | 2 | 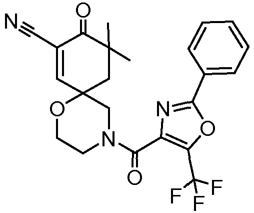 | 10,10-dimethyl-9-oxo-4-[2-phenyl-5-(trifluoromethyl)-1,3-oxazole-4-carbonyl]-1-oxa-4-azaspiro[5.5]undec-7-ene-8-carbonitrile | Calc'd 460.0, found 460.0 |
| 513 | 2 | 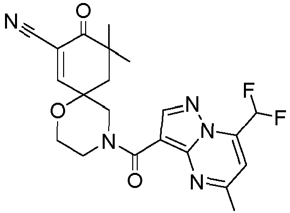 | 4-[7-(difluoromethyl)-5-methylpyrazolo[1,5-a]pyrimidine-3-carbonyl]-10, 10-dimethyl-9-oxo-1-oxa-4-azaspiro[5.5]undec-7-ene-8-carbonitrile | Calc'd 430.0, found 430.0 |
| 514 | 2 | 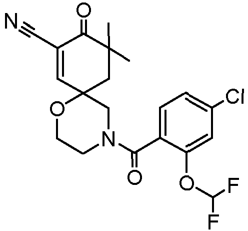 | 4-[4-chloro-2-(difluoromethoxy)benzene-1-carbonyl]-10,10-dimethyl-9-oxo-1-oxa-4-azaspiro[5.5]undec-7-ene-8-carbonitrile | Calc'd 425.0, found 425.0 |
| 515 | 2 | 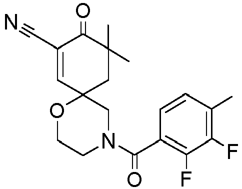 | 4-(2,3-difluoro-4-methylbenzene-1-carbonyl)-10,10-dimethyl-9-oxo-1-oxa-4-azaspiro[5.5]undec-7-ene-8-carbonitrile | Calc'd 375.0, found 375.0 |
| 516 | 2 | 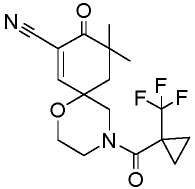 | 10,10-dimethyl-9-oxo-4-[1-(trifluoromethyl)cyclopropane-1-carbonyl]-1-oxa-4-azaspiro[5.5]undec-7-ene-8-carbonitrile | Calc'd 357.0, found 357.0 |
| 517 | 2 | 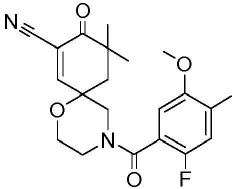 | 4-(2-fluoro-5-methoxy-4-methylbenzene-1-carbonyl)-10,10-dimethyl-9-oxo-1-oxa-4-azaspiro[5.5]undec-7-ene-8-carbonitrile | Calc'd 387.0, found 387.0 |
| 518 | 2 | 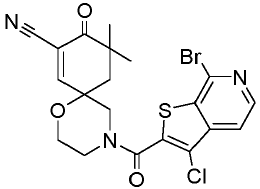 | 4-(7-bromo-3-chlorothieno[2,3-c]pyridine-2-carbonyl)-10,10-dimethyl-9-oxo-1-oxa-4-azaspiro[5.5]undec-7-ene-8-carbonitrile | Calc'd 494.0, found 493.9, 495.8 |
| 519 | 2 | 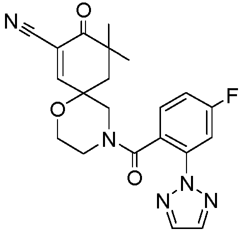 | 4-[4-fluoro-2-(2H-1,2,3-triazol-2-yl)benzene-1-carbonyl]-10,10-dimethyl-9-oxo-1-oxa-4-azaspiro[5.5]undec-7-ene-8-carbonitrile | Calc'd 410.0, found 410 |
| 520 | 2 | 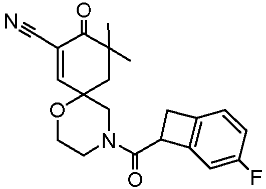 | 4-(4-fluorobicyclo[4.2.0]octa-1,3,5-triene-7-carbonyl)-10,10-dimethyl-9-oxo-1-oxa-4-azaspiro[5.5]undec-7-ene-8-carbonitrile | Calc'd 369.0, found 369.1 |
| 521 | 2 | 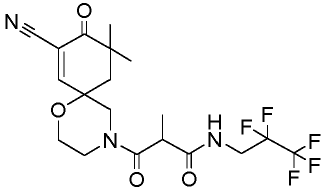 | 3-(8-cyano-10,10-dimethyl-9-oxo-1-oxa-4-azaspiro[5.5]undec-7-en-4-yl)-2-methyl-3-oxo-N-(2,2,3,3,3-pentafluoropropyl)propanamide | Calc'd 452.0, found 452.1 |
| | | | | |
| 522 | 2 | 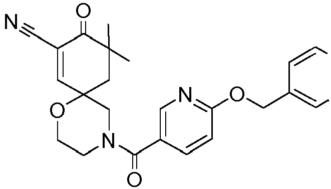 | 4-{6-[(4-fluorophenyl)methoxy]pyridine-3-carbonyl}-10,10-dimethyl-9-oxo-1-oxa-4-azaspiro[5.5]undec-7-ene-8-carbonitrile | Calc'd 450.0, found 450.1 |

### General procedure for Table 15

To a stirring solution of 8-cyano-10,10-dimethyl-9-oxo-1-oxa-4-azaspiro[5.5]undec-7-en-4-ium chloride (15 mg, 1.0 equ., 0.059 mmol) and triethylamine (3 equ.) in DCM, the carboxylic acid (2.0 equ., 0.117 mmol) and a 50% 1-propanephosphonic anhydride solution in EtOAc (5 equ.) were added. The resulting mixture was stirred at *r.t.* for 3h. When LCMS control showed residual starting material, an additional aliquot of triethylamine (3 equ.) was added and the reaction stirred for further 1h. After completion, the reaction mixture was diluted in DCM, and the organic phase was washed with NaHCO₃, water and brine. The organic phase was dried over anhydrous MgSO₄, filtrated and concentrated under reduced pressure.

The crude material was purified by column chromatography on gradient elution MTBE/Heptane 0% to 100% or MeOH/DCM 0% to 5% to provide the desired product in 37%-50% yield.

**Table 15: Compounds were prepared in a similar manner to the general procedure**

| **Ex.** | **Enanti omer** | **Structure** | **IUPAC Name** | **Exact Mass [M+H]+** |
|---|---|---|---|---|
| 523 | 2 | 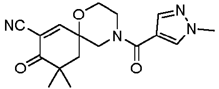 | 10,10-dimethyl-4-(1-methyl-1H-pyrazole-4-carbonyl)-9-oxo-1-oxa-4-azaspiro[5.5]undec-7-ene-8-carbonitrile | 329 |
| 524 | 2 | 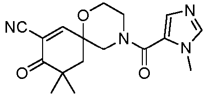 | 10,10-dimethyl-4-(1-methyl-1H-imidazole-5-carbonyl)-9-oxo-1-oxa-4-azaspiro[5.5]undec-7-ene-8-carbonitrile | 329 |
| 525 | 2 | 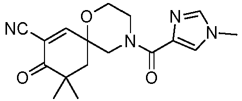 | 10,10-dimethyl-4-(1-methyl-1H-imidazole-4-carbonyl)-9-oxo-1-oxa-4-azaspiro[5.5]undec-7-ene-8-carbonitrile | 329 |
| 526 | 2 | 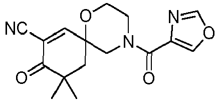 | 10,10-dimethyl-4-(oxazole-4-carbonyl)-9-oxo-1-oxa-4-azaspiro[5.5]undec-7-ene-8-carbonitrile | 316 |
| 527 | 2 | 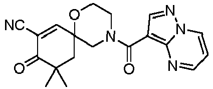 | 10,10-dimethyl-9-oxo-4-(pyrazolo[1,5-a]pyrimidine-3-carbonyl)-1-oxa-4-azaspiro[5.5]undec-7-ene-8-carbonitrile | 366 |
| 528 | 2 | 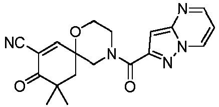 | 10,10-dimethyl-9-oxo-4-(pyrazolo[1,5-a]pyrimidine-2-carbonyl)-1-oxa-4-azaspiro[5.5]undec-7-ene-8-carbonitrile | 366 |
| 529 | 2 | 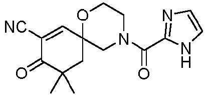 | 4-(1H-imidazole-2-carbonyl)-10,10-dimethyl-9-oxo-1-oxa-4-azaspiro[5.5]undec-7-ene-8-carbonitrile (enantiomer 2) | 315 |
| 530 | 2 | 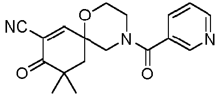 | 10,10-dimethyl-4-nicotinoyl-9-oxo-1-oxa-4-azaspiro[5.5]undec-7-ene-8-carbonitrile | 326 |
| 531 | 1 | 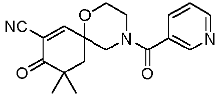 | 10,10-dimethyl-4-nicotinoyl-9-oxo-1-oxa-4-azaspiro[5.5]undec-7-ene-8-carbonitrile | 326 |
| 532 | rac | 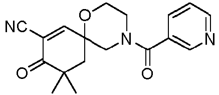 | 10,10-dimethyl-4-nicotinoyl-9-oxo-1-oxa-4-azaspiro[5.5]undec-7-ene-8-carbonitrile | 326 |
| 533 | rac | 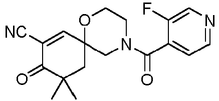 | 4-(3-fluoroisonicotinoyl)-10,10-dimethyl-9-oxo-1-oxa-4-azaspiro[5.5]undec-7-ene-8-carbonitrile | 344 |
| 534 | rac | 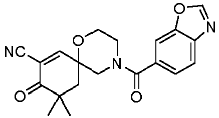 | 4-(benzo[d]oxazole-6-carbonyl)-10,10-dimethyl-9-oxo-1-oxa-4-azaspiro[5.5]undec-7-ene-8-carbonitrile | 366 |
| 535 | rac | 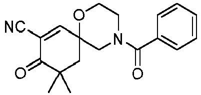 | 4-benzoyl-10,10-dimethyl-9-oxo-1-oxa-4-azaspiro[5.5]undec-7-ene-8-carbonitrile | 325 |

### Example 536: 8,8-dimethyl-7-oxo-2-(pyrimidine-4-carbonyl)-2-azaspiro[3.5]non-5-ene-6-carbonitrile

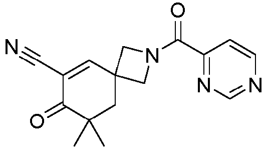

To a solution of pyrimidine-4-carboxylic acid (9.8 mg, 0.079 mmol), HATU (32 mg, 0.083 mmol), and DIEA (34.5 µL, 0.198 mmol) in DMF (0.2 mL) at room temperature was added a solution of 8,8-dimethyl-7-oxo-2-azaspiro[3.5]non-5-ene-6-carbonitrile, HCl (15.0 mg, 0.066 mmol) in DMF (0.13 mL). The reaction mixture was stirred at room temperature overnight. The mixture was filtered and purified by mass triggered reverse phase HPLC (ACN/water with 0.1% TFA modifier) to afford 8,8-dimethyl-7-oxo-2-(pyrimidine-4-carbonyl)-2-azaspiro[3.5]non-5-ene-6-carbonitrile as a solid. MS: 297 (M + H). ¹H NMR (500 MHz, DMSO-*d*₆) δ 9.27 (s, 1H), 9.03 (d, *J =* 5.0 Hz, 1H), 8.29 (s, 1H), 7.95 (d, *J* = 5.0 Hz, 1H), 4.81 (d, *J =* 10.3 Hz, 1H), 4.68 (d, *J* = 10.3 Hz, 1H), 4.30 (d, *J =* 10.6 Hz, 1H), 4.14 (d, *J =* 10.6 Hz, 1H), 2.26 (s, 2H), 1.05 (s, 3H), 1.04 (s, 3H).

**Table 16: The following examples were prepared using a similar procedure as described above for example 536.**

| **Ex** | **Structure** | **Compound Name** | **Exact Mass [M+H]⁺** |
|---|---|---|---|
| 537 | 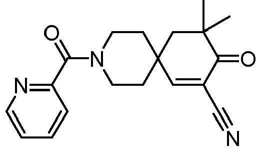 | 10,10-dimethyl-9-oxo-3-(pyridine-2-carbonyl)-3-azaspiro[5.5]undec-7-ene-8-carbonitrile | 324 |
| 538 | 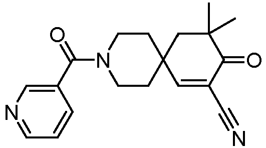 | 10,10-dimethyl-9-oxo-3-(pyridine-3-carbonyl)-3-azaspiro[5.5]undec-7-ene-8-carbonitrile | 324 |
| 539 | 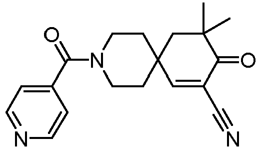 | 10,10-dimethyl-9-oxo-3-(pyridine-4-carbonyl)-3-azaspiro[5.5]undec-7-ene-8-carbonitrile | 324 |
| 540 | 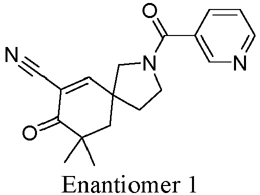 | 9,9-dimethyl-8-oxo-2-(pyridine-3-carbonyl)-2-azaspiro[4.5]dec-6-ene-7-carbonitrile | 310 |
| 541 | 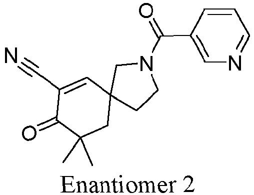 | 9,9-dimethyl-8-oxo-2-(pyridine-3-carbonyl)-2-azaspiro[4.5]dec-6-ene-7-carbonitrile | 310 |
| 542 | 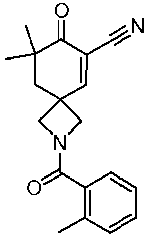 | 8,8-dimethyl-2-(2-methylbenzene-1-carbonyl)-7-oxo-2-azaspiro[3.5]non-5-ene-6-carbonitrile | 309 |
| 543 | 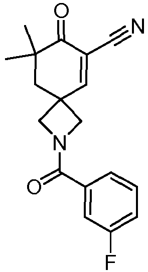 | 2-(3-fluorobenzene-1-carbonyl)-8,8-dimethyl-7-oxo-2-azaspiro[3.5]non-5-ene-6-carbonitrile | 313 |
| 544 | 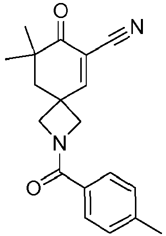 | 8,8-dimethyl-2-(4-methylbenzene-1-carbonyl)-7-oxo-2-azaspiro[3.5]non-5-ene-6-carbonitrile | 309 |
| 545 | 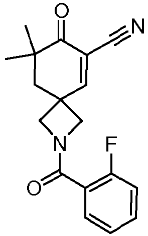 | 2-(2-fluorobenzene-1-carbonyl)-8,8-dimethyl-7-oxo-2-azaspiro[3.5]non-5-ene-6-carbonitrile | 313 |
| 546 | 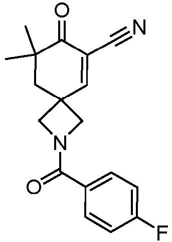 | 2-(4-fluorobenzene-1-carbonyl)-8,8-dimethyl-7-oxo-2-azaspiro[3.5]non-5-ene-6-carbonitrile | 313 |
| 547 | 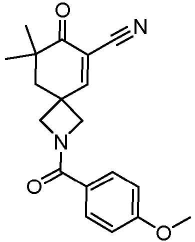 | 2-(4-methoxybenzene-1-carbonyl)-8,8-dimethyl-7-oxo-2-azaspiro[3.5]non-5-ene-6-carbonitrile | 325 |
| 548 | 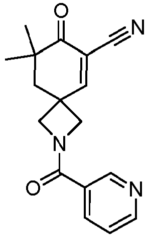 | 8,8-dimethyl-7-oxo-2-(pyridine-3-carbonyl)-2-azaspiro[3.5]non-5-ene-6-carbonitrile | 296 |
| 549 | 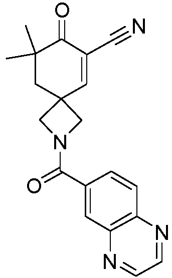 | 8,8-dimethyl-7-oxo-2-(quinoxaline-6-carbonyl)-2-azaspiro[3.5]non-5-ene-6-carbonitrile | 347 |
| 550 | 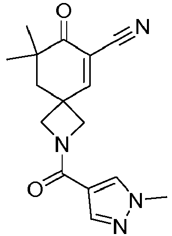 | 8,8-dimethyl-2-(1-methyl-1H-pyrazole-4-carbonyl)-7-oxo-2-azaspiro[3.5]non-5-ene-6-carbonitrile | 299 |
| 551 | 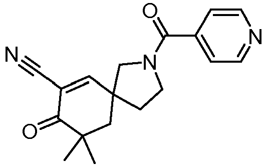 Made from Intermediate 5 | 9,9-dimethyl-8-oxo-2-(pyridine-4-carbonyl)-2-azaspiro[4.5]dec-6-ene-7-carbonitrile | 310 |
| 552 | 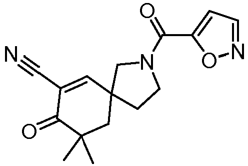 Made from Intermediate 5 | 9,9-dimethyl-2-(1,2-oxazole-5-carbonyl)-8-oxo-2-azaspiro[4.5]dec-6-ene-7-carbonitrile | 300 |
| 553 | 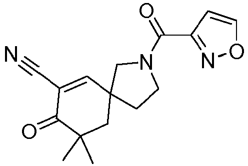 Made from Intermediate 5 | 9,9-dimethyl-2-(1,2-oxazole-3-carbonyl)-8-oxo-2-azaspiro[4.5]dec-6-ene-7-carbonitrile | 300 |
| 554 | 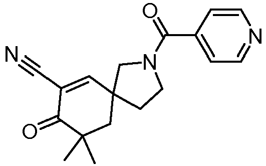 Made from Intermediate 6 | 9,9-dimethyl-8-oxo-2-(pyridine-4-carbonyl)-2-azaspiro[4.5]dec-6-ene-7-carbonitrile | 310 |
| 555 | 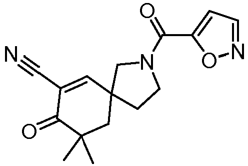 Made from Intermediate 6 | 9,9-dimethyl-2-(1,2-oxazole-5-carbonyl)-8-oxo-2-azaspiro[4.5]dec-6-ene-7-carbonitrile | 300 |
| 556 | 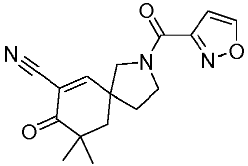 Made from Intermediate 6 | 9,9-dimethyl-2-(1,2-oxazole-3-carbonyl)-8-oxo-2-azaspiro[4.5]dec-6-ene-7-carbonitrile | 300 |
| 557 * | 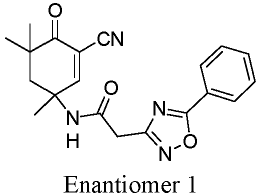 | N-(3-cyano-1,5,5-trimethyl-4-oxocyclohex-2-en-1-yl)-2-(5-phenyl-1,2,4-oxadiazol-3-yl)acetamide | 365 |
| 558 * | 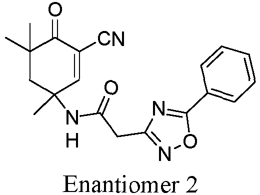 | N-(3-cyano-1,5,5-trimethyl-4-oxocyclohex-2-en-1-yl)-2-(5-phenyl-1,2,4-oxadiazol-3-yl)acetamide | 365 |
| 559 * | 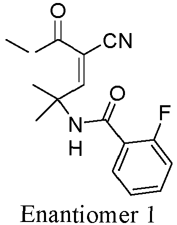 | *N*-(3-cyano-1,5,5-trimethyl-4-oxocyclohex-2-en-1-yl)-2-fluorobenzamide | 301 |
| 560 * | 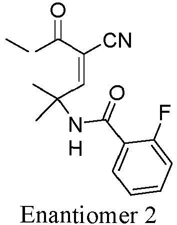 | *N*-(3-cyano-1,5,5-trimethyl-4-oxocyclohex-2-en-1-yl)-2-fluorobenzamide | 301 |
| 561 * | 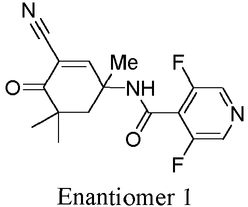 | *N*-(3-cyano-1,5,5-trimethyl-4-oxocyclohex-2-en-1-yl)-3,5-difluoroisonicotinamide | 320 |
| 562 * | 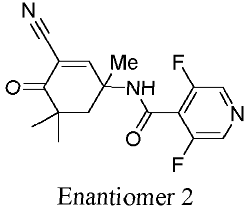 | *N*-(3-cyano-1,5,5-trimethyl-4-oxocyclohex-2-en-1-yl)-3,5-difluoroisonicotinamide | 320 |
| 563 * | 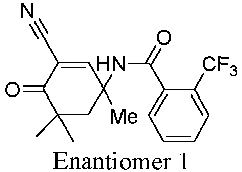 | *N*-(3-cyano-1,5,5-trimethyl-4-oxocyclohex-2-en-1-yl)-2-(trifluoromethyl)benzamide | 351 |
| 564 * | 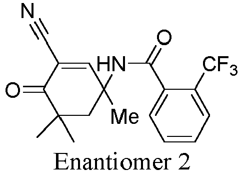 | *N*-(3-cyano-1,5,5-trimethyl-4-oxocyclohex-2-en-1-yl)-2-(trifluoromethyl)benzamide | 351 |

| | | | |
|---|---|---|---|
| * reference compound | | | |

### Example 565: 8,8-dimethyl-2-(3-methylbenzoyl)-7-oxo-2-azaspiro[3.5]non-5-ene-6-carbonitrile

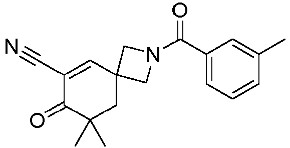

To a solution of 8,8-dimethyl-7-oxo-2-azaspiro[3.5]non-5-ene-6-carbonitrile, HCl (15 mg, 0.066 mmol) and 3-methylbenzoic acid (11 mg, 0.079 mmol) in DCM (0.66 mL) at room temperature was added triethylamine (37 µL, 0.26 mmol) followed by 1-propanephosphonic anhydride (79 µL, 0.13 mmol). The reaction mixture was stirred at room temperature for 30 minutes, then quenched with saturated aqueous sodium bicarbonate (1 mL). The mixture was then extracted with DCM (3 x 1 mL). The combined organic layers were dried over anhydrous sodium sulfate, filtered, and concentrated under reduced pressure. The residue was purified by mass triggered reverse phase HPLC (ACN/water with 0.1% TFA modifier) to afford 8,8-dimethyl-2-(3-methylbenzoyl)-7-oxo-2-azaspiro[3.5]non-5-ene-6-carbonitrile as a solid. MS: 309 (M + H). ¹H NMR (499 MHz, DMSO-*d*₆) δ 8.35 (s, 1H), 7.48 (s, 1H), 7.43 (d, *J =* 6.1 Hz, 1H), 7.39 - 7.29 (m, 2H), 4.51 (d, *J* = 8.5 Hz, 1H), 4.35 (d, *J* = 8.2 Hz, 1H), 4.23 (d, *J* = 9.9 Hz, 1H), 4.09 (d, *J* = 9.7 Hz, 1H), 2.35 (s, 3H), 2.24 (s, 2H), 1.07 (s, 3H), 1.03 (s, 3H).

### General Procedures for Table 17:

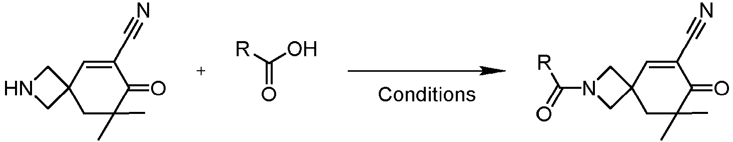

### Method A: EDCI, Pyridine, 50 °C, 16 h

An 8 mL vial was charged with 8,8-dimethyl-7-oxo-2-azaspiro[3.5]non-5-ene-6-carbonitrile (11.4 mg, 60 µmol), carboxylic acid (90 µmol), EDCI (17.3 mg, 90 µmol) and pyridine (600 µL). The vial was capped and shaken at 50 °C for 16 h. The reaction was monitored by LCMS. After the reaction was completed, the reaction mixture was concentrated by Speedvac. The resulting residue was dissolved in MeCN and H₂O and purified by preparative HPLC to afford pure final product.

### Method B: T₃P, DCM, TEA,100 °C, 16 h

To an 8 mL vial charged with 8,8-dimethyl-7-oxo-2-azaspiro[3.5]non-5-ene-6-carbonitrile (11.4 mg, 60 µmol) and carboxylic acid (90.0 µmol) was added DCM (500 µL), triethylamine (20.2 mg, 200 µmol) and 1-propanephosphonic anhydride (60 µL, 50.0 µmol). The vial was capped and shaken at 100 °C for 16 h. The reaction was monitored by LCMS. The reaction mixture was concentrated and extracted with DCM (1 mL*3) and water (1 mL). The organic layers were dried by anhydrous Na₂SO₄ and concentrated by Speedvac to afford crude intermediates. The resulting residue was dissolved in MeCN and H₂O and purified by preparative HPLC to afford pure final product.

**Table 17: Compounds were prepared in a similar manner to the general procedures**

| **Ex.** | **Structure** | **Name** | **Exact Mass [M+H]+** | **Synthesis Method** |
|---|---|---|---|---|
| 566 | 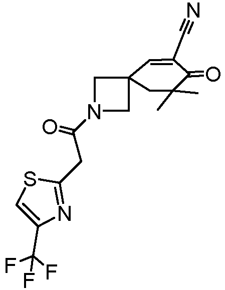 | 8,8-dimethyl-7-oxo-2-{[4-(trifluoromethyl)-1,3-thiazol-2-yl]acetyl}-2-azaspiro[3.5]non-5-ene-6-carbonitrile | Calc'd 384.0, found 383.9 | A |
| 567 | 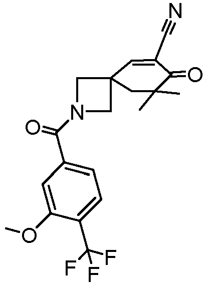 | 2-[3-methoxy-4-(trifluoromethyl)benzene -1-carbonyl]-8,8-dimethyl-7-oxo-2-azaspiro[3.5]non-5-ene-6-carbonitrile | Calc'd 393.0, found 393.0 | A |
| 568 | 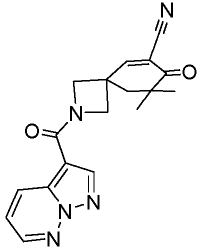 | 8,8-dimethyl-7-oxo-2-(pyrazolo[1,5-b]pyridazine-3-carbonyl)-2-azaspiro[3.5]non-5-ene-6-carbonitrile | Calc'd 336.0, found 336.0 | A |
| 569 | 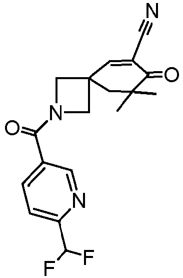 | 2-[6-(difluoromethyl)pyridine -3-carbonyl]-8,8-dimethyl-7-oxo-2-azaspiro[3.5]non-5-ene-6-carbonitrile | Calc'd 346.0, found 346.0 | B |
| 570 | 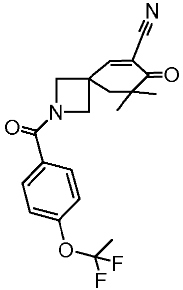 | 2-[4-(1,1-difluoroethoxy)benzene-1-carbonyl]-8,8-dimethyl-7-oxo-2-azaspiro[3.5]non-5-ene-6-carbonitrile | Calc'd 375.0, found 375.0 | B |
| 571 | 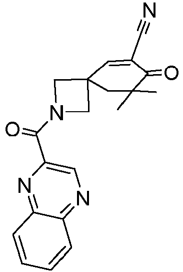 | 8,8-dimethyl-7-oxo-2-(quinoxaline-2-carbonyl)-2-azaspiro[3.5]non-5-ene-6-carbonitrile | Calc'd 347.0, found 347.0 | B |
| 572 | 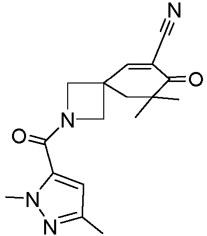 | 2-(1,3-dimethyl-1H-pyrazole-5-carbonyl)-8,8-dimethyl-7-oxo-2-azaspiro[3.5]non-5-ene-6-carbonitrile | Calc'd 313.0, found 313.0 | B |
| 573 | 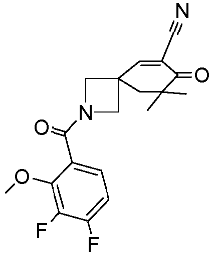 | 2-(3,4-difluoro-2-methoxybenzene-1-carbonyl)-8,8-dimethyl-7-oxo-2-azaspiro[3.5]non-5-ene-6-carbonitrile | Calc'd 361.0, found 361.0 | B |
| 574 | 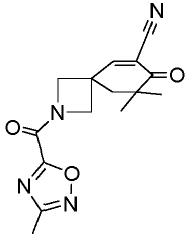 | 8,8-dimethyl-2-(3-methyl-1,2,4-oxadiazole-5-carbonyl)-7-oxo-2-azaspiro[3.5]non-5-ene-6-carbonitrile | Calc'd 301.0, found 301.0 | A |
| 575 | 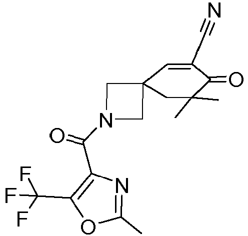 | 8,8-dimethyl-2-[2-methyl-5-(trifluoromethyl)-1,3-oxazole-4-carbonyl]-7-oxo-2-azaspiro[3.5]non-5-ene-6-carbonitrile | Calc'd 368.0, found 368.0 | A |
| 576 | 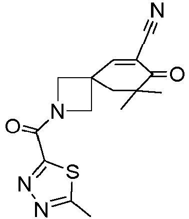 | 8,8-dimethyl-2-(5-methyl-1,3,4-thiadiazole-2-carbonyl)-7-oxo-2-azaspiro[3.5]non-5-ene-6-carbonitrile | Calc'd 317.0, found 317.0 | A |
| 577 | 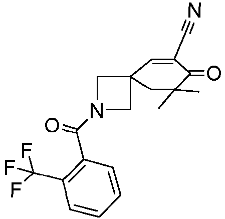 | 8,8-dimethyl-7-oxo-2-[2-(trifluoromethyl)benzene -1-carbonyl]-2-azaspiro[3.5]non-5-ene-6-carbonitrile | Calc'd 363.0, found 363.0 | A |
| 578 | 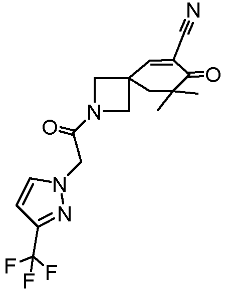 | 8,8-dimethyl-7-oxo-2-{[3-(trifluoromethyl)-1H-pyrazol-1-yl]acetyl}-2-azaspiro[3.5]non-5-ene-6-carbonitrile | Calc'd 367.0, found 367.0 | A |
| 579 | 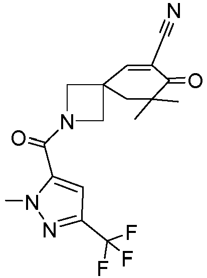 | 8,8-dimethyl-2-[1-methyl-3-(trifluoromethyl)-1H-pyrazole-5-carbonyl]-7-oxo-2-azaspiro[3.5]non-5-ene-6-carbonitrile | Calc'd 367.0, found 367.0 | A |
| 580 | 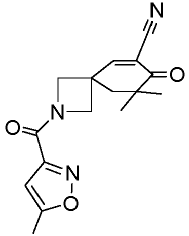 | 8,8-dimethyl-2-(5-methyl-1,2-oxazole-3-carbonyl)-7-oxo-2-azaspiro[3.5]non-5-ene-6-carbonitrile | Calc'd 300.0, found 300.0 | A |
| 581 | 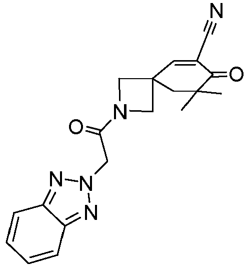 | 2-[(2H-benzotriazol-2-yl)acetyl]-8,8-dimethyl-7-oxo-2-azaspiro[3.5]non-5-ene-6-carbonitrile | Calc'd 350.0, found 350.0 | A |
| 582 | 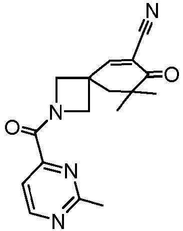 | 8,8-dimethyl-2-(2-methylpyrimidine-4-carbonyl)-7-oxo-2-azaspiro[3.5]non-5-ene-6-carbonitrile | Calc'd 311.0, found 311.0 | A |
| 583 | 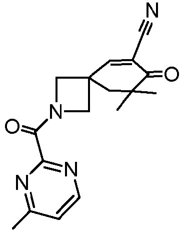 | 8,8-dimethyl-2-(4-methylpyrimidine-2-carbonyl)-7-oxo-2-azaspiro[3.5]non-5-ene-6-carbonitrile | Calc'd 311.0, found 311.0 | A |
| 584 | 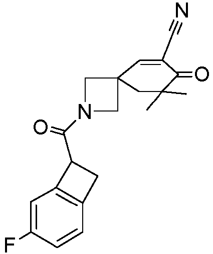 | 2-(4-fluorobicyclo[4.2.0]octa-1,3,5-triene-7-carbonyl)-8,8-dimethyl-7-oxo-2-azaspiro[3.5]non-5-ene-6-carbonitrile | Calc'd 339.0, found 339.0 | A |
| 585 | 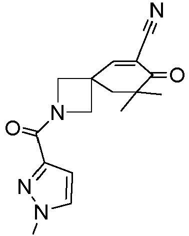 | 8,8-dimethyl-2-(1-methyl-1H-pyrazole-3-carbonyl)-7-oxo-2-azaspiro[3.5]non-5-ene-6-carbonitrile | Calc'd 299.0, found 299.0 | A |
| 586 | 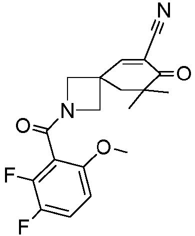 | 2-(2,3-difluoro-6-methoxybenzene-1-carbonyl)-8,8-dimethyl-7-oxo-2-azaspiro[3.5]non-5-ene-6-carbonitrile | Calc'd 361.0, found 361.0 | A |
| 587 | 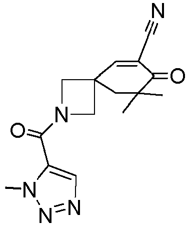 | 8,8-dimethyl-2-(1-methyl-1H-1,2,3-triazole-5-carbonyl)-7-oxo-2-azaspiro[3.5]non-5-ene-6-carbonitrile | Calc'd 300.0, found 300.1 | A |
| 588 | 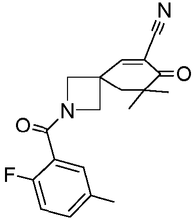 | 2-(2-fluoro-5-methylbenzene-1-carbonyl)-8,8-dimethyl-7-oxo-2-azaspiro[3.5]non-5-ene-6-carbonitrile | Calc'd 327.0, found 327.0 | A |
| 589 | 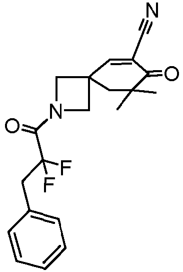 | 2-(2,2-difluoro-3-phenylpropanoyl)-8,8-dimethyl-7-oxo-2-azaspiro[3.5]non-5-ene-6-carbonitrile | Calc'd 359.0, found 359.0 | A |
| 590 | 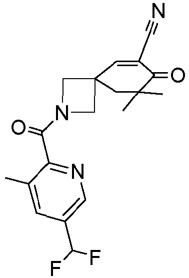 | 2-[5-(difluoromethyl)-3-methylpyridine-2-carbonyl]-8,8-dimethyl-7-oxo-2-azaspiro[3.5]non-5-ene-6-carbonitrile | Calc'd 360.0, found 360.0 | A |
| 591 | 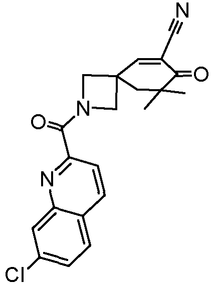 | 2-(7-chloroquinoline-2-carbonyl)-8,8-dimethyl-7-oxo-2-azaspiro[3.5]non-5-ene-6-carbonitrile | Calc'd 380.0, found 380.0 | A |
| 592 | 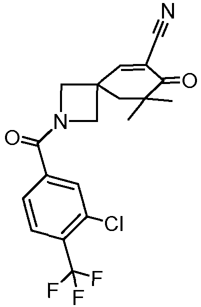 | 2-[3-chloro-4-(trifluoromethyl)benzene -1-carbonyl]-8,8-dimethyl-7-oxo-2-azaspiro[3.5]non-5-ene-6-carbonitrile | Calc'd 397.0, found 397.0 | A |
| 593 | 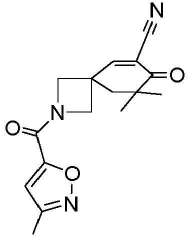 | 8,8-dimethyl-2-(3-methyl-1,2-oxazole-5-carbonyl)-7-oxo-2-azaspiro[3.5]non-5-ene-6-carbonitrile | Calc'd 300.0, found 300.0 | A |
| 594 | 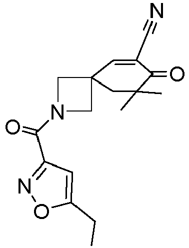 | 2-(5-ethyl-1,2-oxazole-3-carbonyl)-8,8-dimethyl-7-oxo-2-azaspiro[3.5]non-5-ene-6-carbonitrile | Calc'd 314.0, found 314.0 | A |
| 595 | 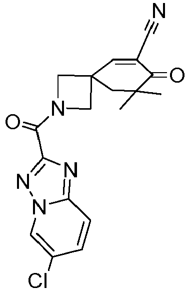 | 2-(6-chloro[1,2,4]triazolo[1,5-a]pyridine-2-carbonyl)-8,8-dimethyl-7-oxo-2-azaspiro[3.5]non-5-ene-6-carbonitrile | Calc'd 370.0, found 370.0 | A |
| 596 | 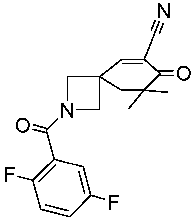 | 2-(2,5-difluorobenzene-1-carbonyl)-8,8-dimethyl-7-oxo-2-azaspiro[3.5]non-5-ene-6-carbonitrile | Calc'd 331.0, found 331.0 | A |
| 597 | 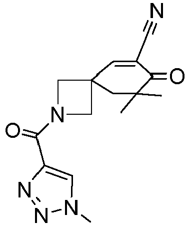 | 8,8-dimethyl-2-(1-methyl-1H-1,2,3-triazole-4-carbonyl)-7-oxo-2-azaspiro[3.5]non-5-ene-6-carbonitrile | Calc'd 300.0, found 300.0 | A |
| 598 | 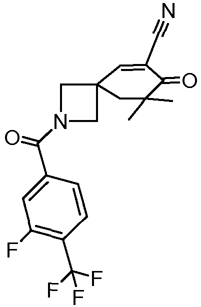 | 2-[3-fluoro-4-(trifluoromethyl)benzene -1-carbonyl]-8,8-dimethyl-7-oxo-2-azaspiro[3.5]non-5-ene-6-carbonitrile | Calc'd 381.0, found 381.0 | A |
| 599 | 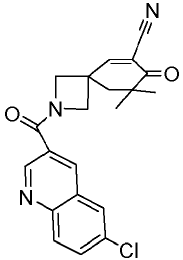 | 2-(6-chloroquinoline-3-carbonyl)-8,8-dimethyl-7-oxo-2-azaspiro[3.5]non-5-ene-6-carbonitrile | Calc'd 380.0, found 380.0 | A |
| 600 | 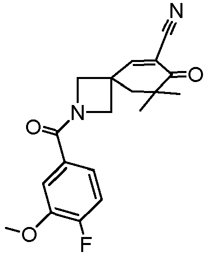 | 2-(4-fluoro-3-methoxybenzene-1-carbonyl)-8,8-dimethyl-7-oxo-2-azaspiro[3.5]non-5-ene-6-carbonitrile | Calc'd 343.0, found 343.1 | A |
| 601 | 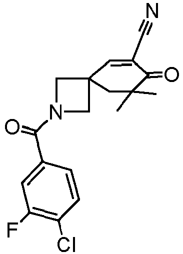 | 2-(4-chloro-3-fluorobenzene-1-carbonyl)-8,8-dimethyl-7-oxo-2-azaspiro[3.5]non-5-ene-6-carbonitrile | Calc'd 347.0, found 347.0 | A |
| 602 | 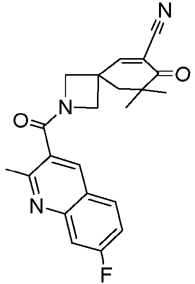 | 2-(7-fluoro-2-methylquinoline-3-carbonyl)-8,8-dimethyl-7-oxo-2-azaspiro[3.5]non-5-ene-6-carbonitrile | Calc'd 378.0, found 378.0 | A |
| 603 | 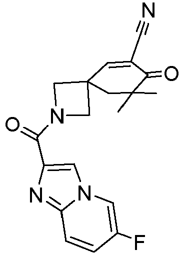 | 2-(6-fluoroimidazo[1,2-a]pyridine-2-carbonyl)-8,8-dimethyl-7-oxo-2-azaspiro[3.5]non-5-ene-6-carbonitrile | Calc'd 353.0, found 353.0 | A |
| 604 | 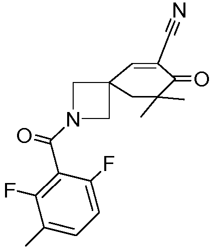 | 2-(2,6-difluoro-3-methylbenzene-1-carbonyl)-8,8-dimethyl-7-oxo-2-azaspiro[3.5]non-5-ene-6-carbonitrile | Calc'd 345.0, found 345.1 | A |
| 605 | 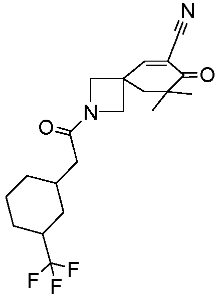 | 8,8-dimethyl-7-oxo-2-{[3-(trifluoromethyl)cyclohe xyl]acetyl}-2-azaspiro[3.5]non-5-ene-6-carbonitrile | Calc'd 383.0, found 383.1 | A |
| 606 | 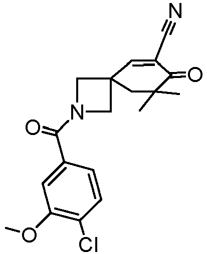 | 2-(4-chloro-3-methoxybenzene-1-carbonyl)-8,8-dimethyl-7-oxo-2-azaspiro[3.5]non-5-ene-6-carbonitrile | Calc'd 359.0, found 359.0 | A |
| 607 | 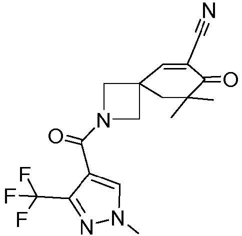 | 8,8-dimethyl-2-[1-methyl-3-(trifluoromethyl)-1H-pyrazole-4-carbonyl]-7-oxo-2-azaspiro[3.5]non-5-ene-6-carbonitrile | Calc'd 367.0, found 367.0 | A |
| 608 | 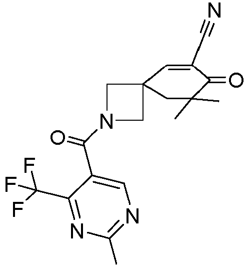 | 8,8-dimethyl-2-[2-methyl-4-(trifluoromethyl)pyrimidi ne-5-carbonyl]-7-oxo-2-azaspiro[3.5]non-5-ene-6-carbonitrile | Calc'd 379.0, found 379.0 | A |
| 609 | 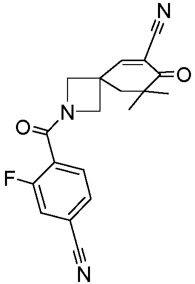 | 2-(4-cyano-2-fluorobenzene-1-carbonyl)-8,8-dimethyl-7-oxo-2-azaspiro[3.5]non-5-ene-6-carbonitrile | Calc'd 338.0, found 338.0 | A |
| 610 | 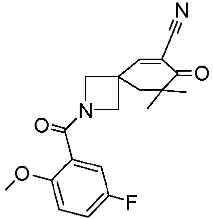 | 2-(5-fluoro-2-methoxybenzene-1-carbonyl)-8,8-dimethyl-7-oxo-2-azaspiro[3.5]non-5-ene-6-carbonitrile | Calc'd 343.0, found 343.1 | A |
| 611 | 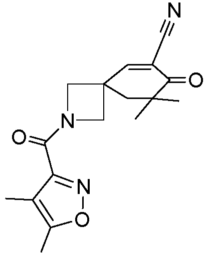 | 2-(4,5-dimethyl-1,2-oxazole-3-carbonyl)-8,8-dimethyl-7-oxo-2-azaspiro[3.5]non-5-ene-6-carbonitrile | Calc'd 314.0, found 314.0 | A |
| 612 | 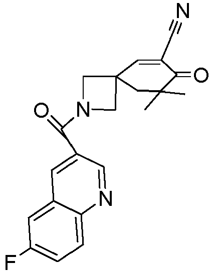 | 2-(6-fluoroquinoline-3-carbonyl)-8,8-dimethyl-7-oxo-2-azaspiro[3.5]non-5-ene-6-carbonitrile | Calc'd 364.0, found 364.0 | A |
| 613 | 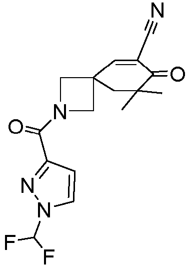 | 2-[1-(difluoromethyl)-1H-pyrazole-3-carbonyl]-8,8-dimethyl-7-oxo-2-azaspiro[3.5]non-5-ene-6-carbonitrile | Calc'd 335.0, found 335.0 | A |
| 614 | 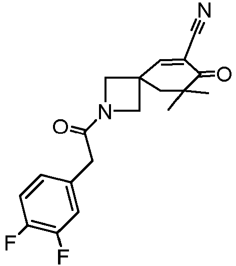 | 2-[(3,4-difluorophenyl)acetyl]-8,8-dimethyl-7-oxo-2-azaspiro[3.5]non-5-ene-6-carbonitrile | Calc'd 345.0, found 345.0 | A |
| 615 | 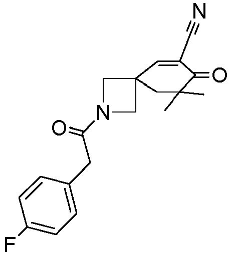 | 2-[(4-fluorophenyl)acetyl]-8,8-dimethyl-7-oxo-2-azaspiro[3.5]non-5-ene-6-carbonitrile | Calc'd 327.0, found 327.0 | A |
| 616 | 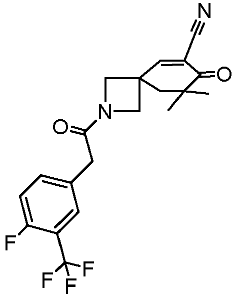 | 2-{[4-fluoro-3-(trifluoromethyl)phenyl]a cetyl }-8, 8-dimethyl-7-oxo-2-azaspiro[3.5]non-5-ene-6-carbonitrile | Calc'd 395.0, found 395.0 | A |
| 617 | 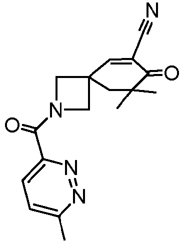 | 8,8-dimethyl-2-(6-methylpyridazine-3-carbonyl)-7-oxo-2-azaspiro[3.5]non-5-ene-6-carbonitrile | Calc'd 311.0, found 311.1 | A |
| 618 | 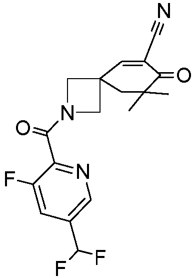 | 2-[5-(difluoromethyl)-3-fluoropyridine-2-carbonyl]-8,8-dimethyl-7-oxo-2-azaspiro[3.5]non-5-ene-6-carbonitrile | Calc'd 364.0, found 364.0 | A |
| 619 | 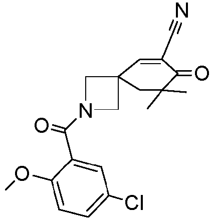 | 2-(5-chloro-2-methoxybenzene-1-carbonyl)-8,8-dimethyl-7-oxo-2-azaspiro[3.5]non-5-ene-6-carbonitrile | Calc'd 359.0, found 359.0 | A |
| 620 | 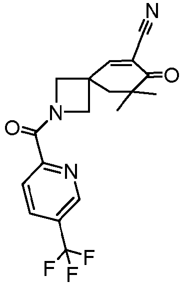 | 8,8-dimethyl-7-oxo-2-[5-(trifluoromethyl)pyridine -2-carbonyl]-2-azaspiro[3.5]non-5-ene-6-carbonitrile | Calc'd 364.0, found 364.0 | A |
| 621 | 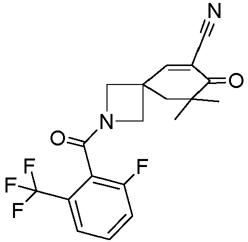 | 2-[2-fluoro-6-(trifluoromethyl)benzene -1-carbonyl]-8,8-dimethyl-7-oxo-2-azaspiro[3.5]non-5-ene-6-carbonitrile | Calc'd 381.0, found 381.0 | A |
| 622 | 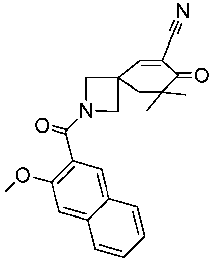 | 2-(3-methoxynaphthalene-2-carbonyl)-8,8-dimethyl-7-oxo-2-azaspiro[3.5]non-5-ene-6-carbonitrile | Calc'd 375.0, found 375.1 | A |
| 623 | 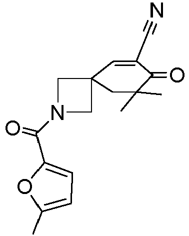 | 8,8-dimethyl-2-(5-methylfuran-2-carbonyl)-7-oxo-2-azaspiro[3.5]non-5-ene-6-carbonitrile | Calc'd 299.0, found 299.0 | A |
| 624 | 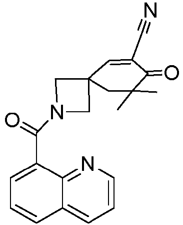 | 8,8-dimethyl-7-oxo-2-(quinoline-8-carbonyl)-2-azaspiro[3.5]non-5-ene-6-carbonitrile | Calc'd 346.0, found 346.0 | A |
| 625 | 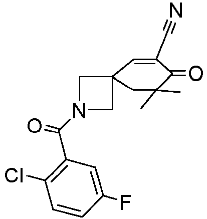 | 2-(2-chloro-5-fluorobenzene-1-carbonyl)-8,8-dimethyl-7-oxo-2-azaspiro[3.5]non-5-ene-6-carbonitrile | Calc'd 347.0, found 347.0 | A |
| 626 | 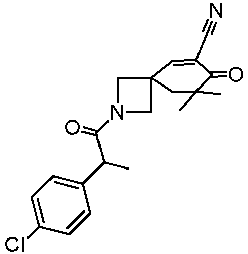 | 2-[2-(4-chlorophenyl)propanoyl] -8,8-dimethyl-7-oxo-2-azaspiro[3.5]non-5-ene-6-carbonitrile | Calc'd 357.0, found 357.0 | A |
| 627 | 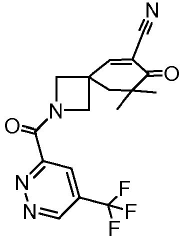 | 8,8-dimethyl-7-oxo-2-[5-(trifluoromethyl)pyridazi ne-3-carbonyl]-2-azaspiro[3.5]non-5-ene-6-carbonitrile | Calc'd 365.0, found 365.0 | A |
| 628 | 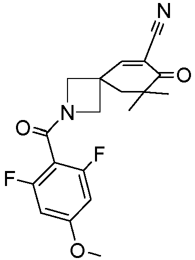 | 2-(2,6-difluoro-4-methoxybenzene-1-carbonyl)-8,8-dimethyl-7-oxo-2-azaspiro[3.5]non-5-ene-6-carbonitrile | Calc'd 361.0, found 361.0 | A |
| 629 | 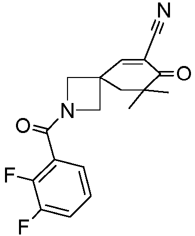 | 2-(2,3-difluorobenzene-1-carbonyl)-8,8-dimethyl-7-oxo-2-azaspiro[3.5]non-5-ene-6-carbonitrile | Calc'd 331.0, found 331.0 | A |
| 630 | 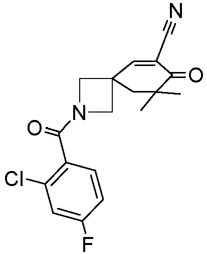 | 2-(2-chloro-4-fluorobenzene-1-carbonyl)-8,8-dimethyl-7-oxo-2-azaspiro[3.5]non-5-ene-6-carbonitrile | Calc'd 347.0, found 347.0 | A |
| 631 | 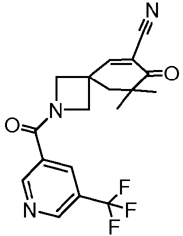 | 8,8-dimethyl-7-oxo-2-[5-(trifluoromethyl)pyridine -3-carbonyl]-2-azaspiro[3.5]non-5-ene-6-carbonitrile | Calc'd 364.0, found 364.0 | A |
| 632 | 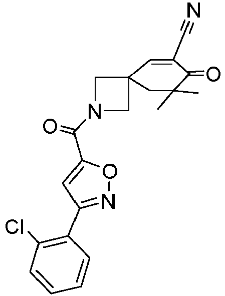 | 2-[3-(2-chlorophenyl)-1,2-oxazole-5-carbonyl]-8,8-dimethyl-7-oxo-2-azaspiro[3.5]non-5-ene-6-carbonitrile | Calc'd 396.0, found 396.0 | A |
| 633 | 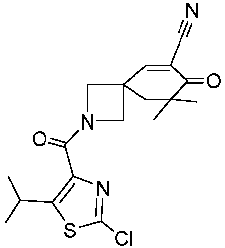 | 2-[2-chloro-5-(propan-2-yl)-1,3-thiazole-4-carbonyl]-8,8-dimethyl-7-oxo-2-azaspiro[3.5]non-5-ene-6-carbonitrile | Calc'd 378.0, found 378.0 | A |
| 634 | 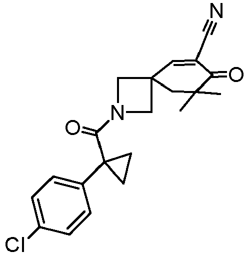 | 2-[1-(4-chlorophenyl)cyclopropa ne-1-carbonyl]-8,8-dimethyl-7-oxo-2-azaspiro[3.5]non-5-ene-6-carbonitrile | Calc'd 369.0, found 369.0 | A |
| 635 | 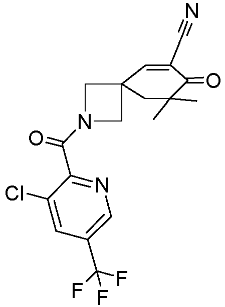 | 2-[3-chloro-5-(trifluoromethyl)pyridine -2-carbonyl]-8,8-dimethyl-7-oxo-2-azaspiro[3.5]non-5-ene-6-carbonitrile | Calc'd 398.0, found 398.0 | A |
| 636 | 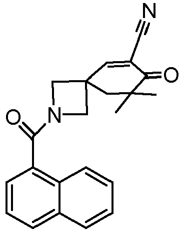 | 8,8-dimethyl-2-(naphthalene-1-carbonyl)-7-oxo-2-azaspiro[3.5]non-5-ene-6-carbonitrile | Calc'd 345.0, found 345.0 | A |
| 637 | 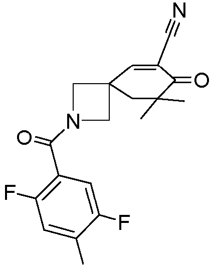 | 2-(2,5-difluoro-4-methylbenzene-1-carbonyl)-8,8-dimethyl-7-oxo-2-azaspiro[3.5]non-5-ene-6-carbonitrile | Calc'd 345.0, found 345.0 | A |
| 638 | 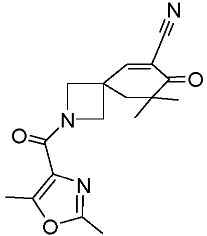 | 2-(2,5-dimethyl-1,3-oxazole-4-carbonyl)-8,8-dimethyl-7-oxo-2-azaspiro[3.5]non-5-ene-6-carbonitrile | Calc'd 314.0, found 314.0 | A |
| 639 | 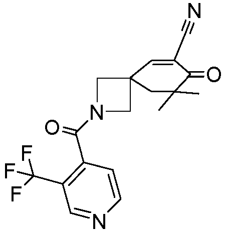 | 8,8-dimethyl-7-oxo-2-[3-(trifluoromethyl)pyridine -4-carbonyl]-2-azaspiro[3.5]non-5-ene-6-carbonitrile | Calc'd 364.0, found 364.0 | A |
| 640 | 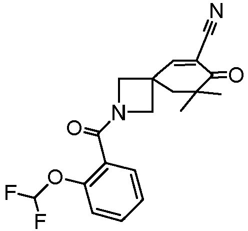 | 2-[2-(difluoromethoxy)benzen e-1-carbonyl]-8,8-dimethyl-7-oxo-2-azaspiro[3.5]non-5-ene-6-carbonitrile | Calc'd 361.0, found 361.0 | A |
| 641 | 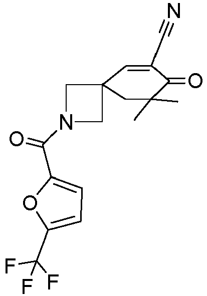 | 8,8-dimethyl-7-oxo-2-[5-(trifluoromethyl)furan-2-carbonyl]-2-azaspiro[3.5]non-5-ene-6-carbonitrile | Calc'd 353.0, found 353.0 | A |
| 642 | 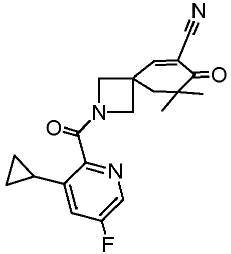 | 2-(3-cyclopropyl-5-fluoropyridine-2-carbonyl)-8,8-dimethyl-7-oxo-2-azaspiro[3.5]non-5-ene-6-carbonitrile | Calc'd 354.0, found 354.1 | A |
| 643 | 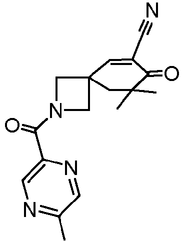 | 8,8-dimethyl-2-(5-methylpyrazine-2-carbonyl)-7-oxo-2-azaspiro[3.5]non-5-ene-6-carbonitrile | Calc'd 311.0, found 311.1 | A |
| 644 | 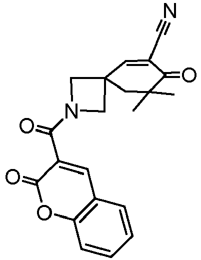 | 8,8-dimethyl-7-oxo-2-(2-oxo-2H-1-benzopyran-3-carbonyl)-2-azaspiro[3.5]non-5-ene-6-carbonitrile | Calc'd 363.0, found 363.0 | A |
| 645 | 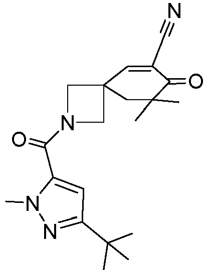 | 2-(3-tert-butyl-1-methyl-1H-pyrazole-5-carbonyl)-8,8-dimethyl-7-oxo-2-azaspiro[3.5]non-5-ene-6-carbonitrile | Calc'd 355.0, found 355.1 | A |
| 646 | 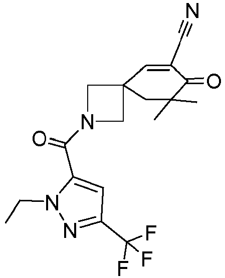 | 2-[1-ethyl-3-(trifluoromethyl)-1H-pyrazole-5-carbonyl]-8,8-dimethyl-7-oxo-2-azaspiro[3.5]non-5-ene-6-carbonitrile | Calc'd 381.0, found 381.0 | A |
| 647 | 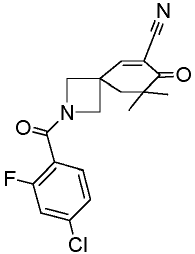 | 2-(4-chloro-2-fluorobenzene-1-carbonyl)-8,8-dimethyl-7-oxo-2-azaspiro[3.5]non-5-ene-6-carbonitrile | Calc'd 347.0, found 347.0 | A |
| 648 | 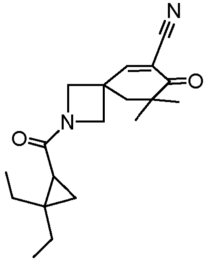 | 2-(2,2-diethylcyclopropane-1-carbonyl)-8,8-dimethyl-7-oxo-2-azaspiro[3.5]non-5-ene-6-carbonitrile | Calc'd 315.0, found 315.1 | A |
| 649 | 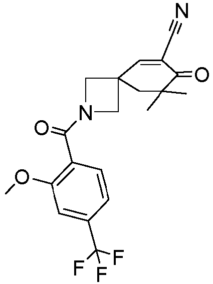 | 2-[2-methoxy-4-(trifluoromethyl)benzene -1-carbonyl]-8,8-dimethyl-7-oxo-2-azaspiro[3.5]non-5-ene-6-carbonitrile | Calc'd 393.0, found 393.0 | A |
| 650 | 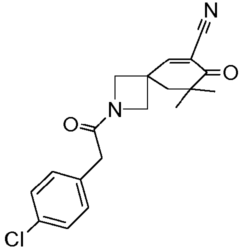 | 2-[(4-chlorophenyl)acetyl]-8,8-dimethyl-7-oxo-2-azaspiro[3.5]non-5-ene-6-carbonitrile | Calc'd 343.0, found 343.0 | A |
| 651 | 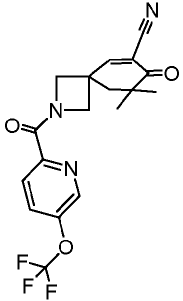 | 8,8-dimethyl-7-oxo-2-[5-(trifluoromethoxy)pyridi ne-2-carbonyl]-2-azaspiro[3.5]non-5-ene-6-carbonitrile | Calc'd 380.0, found 380.0 | A |
| 652 | 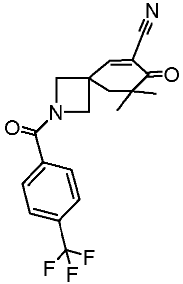 | 8,8-dimethyl-7-oxo-2-[4-(trifluoromethyl)benzene -1-carbonyl]-2-azaspiro[3.5]non-5-ene-6-carbonitrile | Calc'd 363.0, found 363.0 | A |
| 653 | 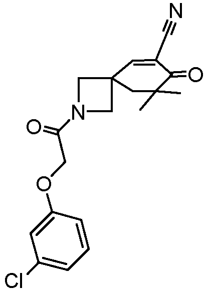 | 2-[(3-chlorophenoxy)acetyl]-8,8-dimethyl-7-oxo-2-azaspiro[3.5]non-5-ene-6-carbonitrile | Calc'd 359.0, found 359.0 | A |
| 654 | 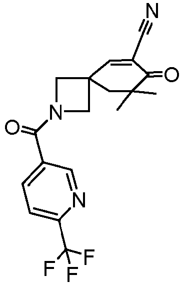 | 8,8-dimethyl-7-oxo-2-[6-(trifluoromethyl)pyridine -3-carbonyl]-2-azaspiro[3.5]non-5-ene-6-carbonitrile | Calc'd 364.0, found 364.0 | A |
| 655 | 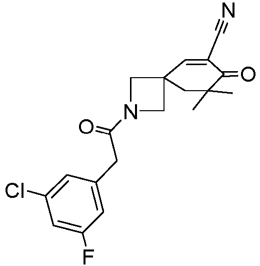 | 2-[(3-chloro-5-fluorophenyl)acetyl]-8,8-dimethyl-7-oxo-2-azaspiro[3.5]non-5-ene-6-carbonitrile | Calc'd 361.0, found 361.0 | A |
| 656 | 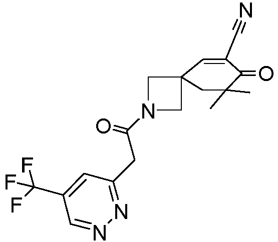 | 8,8-dimethyl-7-oxo-2-{[5-(trifluoromethyl)pyridazi n-3-yl]acetyl}-2-azaspiro[3.5]non-5-ene-6-carbonitrile | Calc'd 379.0, found 379.0 | A |
| 657 | 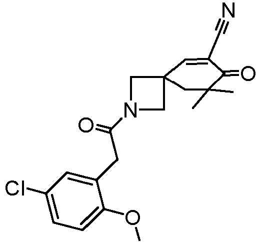 | 2-[(5-chloro-2-methoxyphenyl)acetyl]-8,8-dimethyl-7-oxo-2-azaspiro[3.5]non-5-ene-6-carbonitrile | Calc'd 373.0, found 373.0 | A |
| 658 | 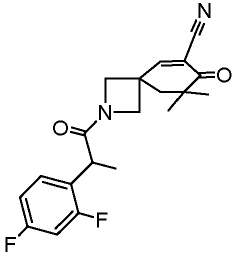 | 2-[2-(2,4-difluorophenyl)propanoy l]-8,8-dimethyl-7-oxo-2-azaspiro[3.5]non-5-ene-6-carbonitrile | Calc'd 359.0, found 359.0 | A |
| 659 | 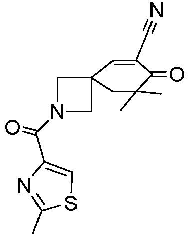 | 8,8-dimethyl-2-(2-methyl-1,3-thiazole-4-carbonyl)-7-oxo-2-azaspiro[3.5]non-5-ene-6-carbonitrile | Calc'd 316.0, found 316.0 | A |
| 660 | 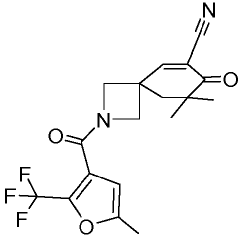 | 8,8-dimethyl-2-[5-methyl-2-(trifluoromethyl)furan-3-carbonyl]-7-oxo-2-azaspiro[3.5]non-5-ene-6-carbonitrile | Calc'd 367.0, found 367.0 | A |
| 661 | 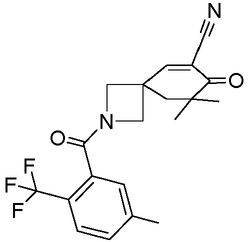 | 8,8-dimethyl-2-[5-methyl-2-(trifluoromethyl)benzene -1-carbonyl]-7-oxo-2-azaspiro[3.5]non-5-ene-6-carbonitrile | Calc'd 377.0, found 377.0 | A |
| 662 | 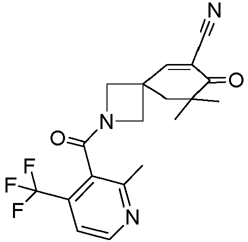 | 8,8-dimethyl-2-[2-methyl-4-(trifluoromethyl)pyridine -3-carbonyl]-7-oxo-2-azaspiro[3.5]non-5-ene-6-carbonitrile | Calc'd 378.0, found 378.0 | A |
| 663 | 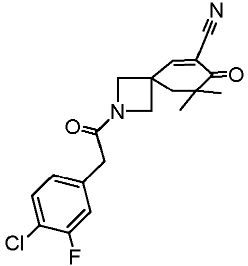 | 2-[(4-chloro-3-fluorophenyl)acetyl]-8,8-dimethyl-7-oxo-2-azaspiro[3.5]non-5-ene-6-carbonitrile | Calc'd 361.0, found 360.9 | A |
| 664 | 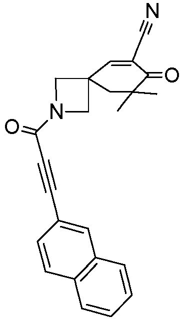 | 8,8-dimethyl-2-[3-(naphthalen-2-yl)prop-2-ynoyl]-7-oxo-2-azaspiro[3.5]non-5-ene-6-carbonitrile | Calc'd 369.0, found 369.0 | A |
| 665 | 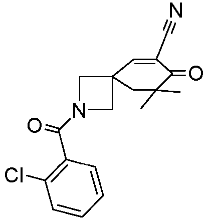 | 2-(2-chlorobenzene-1-carbonyl)-8,8-dimethyl-7-oxo-2-azaspiro[3.5]non-5-ene-6-carbonitrile | Calc'd 329.0, found 329.0 | A |
| 666 | 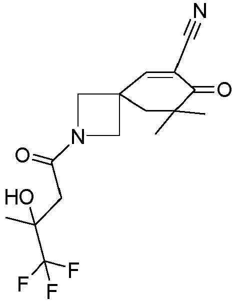 | 8,8-dimethyl-7-oxo-2-(4,4,4-trifluoro-3-hydroxy-3-methylbutanoyl)-2-azaspiro[3.5]non-5-ene-6-carbonitrile | Calc'd 345.0, found 345.0 | A |
| 667 | 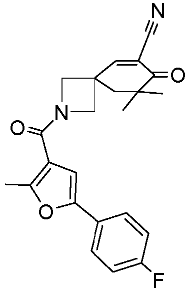 | 2-[5-(4-fluorophenyl)-2-methylfuran-3-carbonyl]-8,8-dimethyl-7-oxo-2-azaspiro[3.5]non-5-ene-6-carbonitrile | Calc'd 393.0, found 393.1 | A |
| 668 | 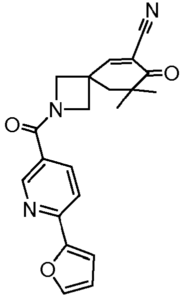 | 2-[6-(furan-2-yl)pyridine-3-carbonyl]-8,8-dimethyl-7-oxo-2-azaspiro[3.5]non-5-ene-6-carbonitrile | Calc'd 362.0, found 362.0 | A |
| 669 | 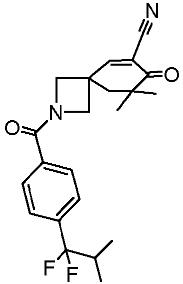 | 2-[4-(1,1-difluoro-2-methylpropyl)benzene-1-carbonyl]-8,8-dimethyl-7-oxo-2-azaspiro[3.5]non-5-ene-6-carbonitrile | Calc'd 387.0, found 387.1 | A |
| 670 | 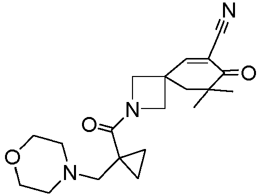 | 8,8-dimethyl-2-{1-[(morpholin-4-yl)methyl]cyclopropane-1-carbonyl}-7-oxo-2-azaspiro[3.5]non-5-ene-6-carbonitrile | Calc'd 358.0, found 358.1 | A |
| 671 | 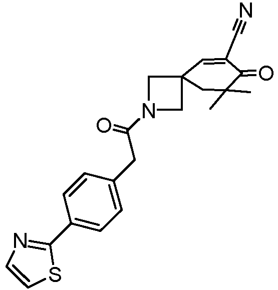 | 8,8-dimethyl-7-oxo-2-{ [4-(1,3-thiazol-2-yl)phenyl]acetyl }-2-azaspiro[3.5]non-5-ene-6-carbonitrile | Calc'd 392.0, found 392.0 | A |
| 672 | 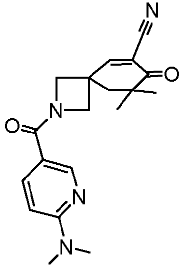 | 2-[6-(dimethylamino)pyridine -3-carbonyl]-8,8-dimethyl-7-oxo-2-azaspiro[3.5]non-5-ene-6-carbonitrile | Calc'd 339.0, found 339.1 | A |
| 673 | 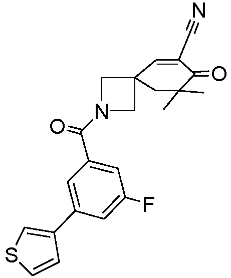 | 2-[3-fluoro-5-(thiophen-3-yl)benzene-1-carbonyl]-8,8-dimethyl-7-oxo-2-azaspiro[3.5]non-5-ene-6-carbonitrile | Calc'd 395.0, found 395.0 | A |
| 674 | 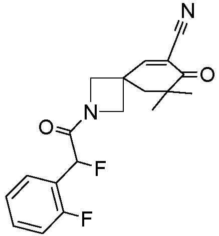 | 2-[fluoro(2-fluorophenyl)acetyl]-8,8-dimethyl-7-oxo-2-azaspiro[3.5]non-5-ene-6-carbonitrile | Calc'd 345.0, found 345.0 | A |
| 675 | 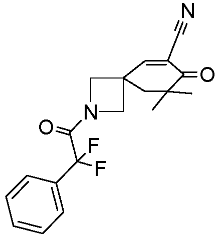 | 2-[difluoro(phenyl)acetyl]-8,8-dimethyl-7-oxo-2-azaspiro[3.5]non-5-ene-6-carbonitrile | Calc'd 345.0, found 345.0 | A |
| 676 | 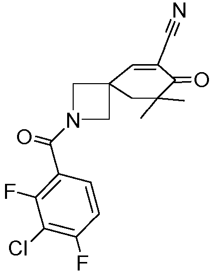 | 2-(3-chloro-2,4-difluorobenzene-1-carbonyl)-8,8-dimethyl-7-oxo-2-azaspiro[3.5]non-5-ene-6-carbonitrile | Calc'd 365.0, found 365.0 | A |
| 677 | 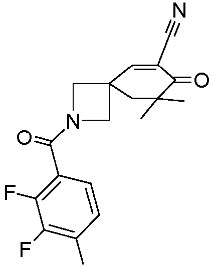 | 2-(2,3-difluoro-4-methylbenzene-1-carbonyl)-8,8-dimethyl-7-oxo-2-azaspiro[3.5]non-5-ene-6-carbonitrile | Calc'd 345.0, found 345.0 | A |
| 678 | 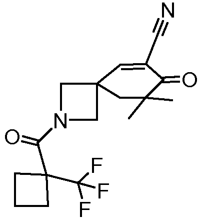 | 8,8-dimethyl-7-oxo-2-[1-(trifluoromethyl)cyclobut ane-1-carbonyl]-2-azaspiro[3.5]non-5-ene-6-carbonitrile | Calc'd 341.0, found 341.0 | A |
| 679 | 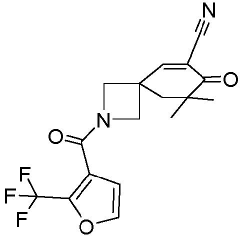 | 8,8-dimethyl-7-oxo-2-[2-(trifluoromethyl)furan-3-carbonyl]-2-azaspiro[3.5]non-5-ene-6-carbonitrile | Calc'd 353.0, found 353.0 | A |
| 680 | 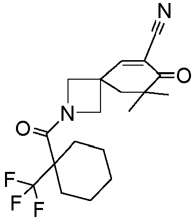 | 8,8-dimethyl-7-oxo-2-[1-(trifluoromethyl)cyclohe xane-1-carbonyl]-2-azaspiro[3.5]non-5-ene-6-carbonitrile | Calc'd 369.0, found 369.0 | A |
| 681 | 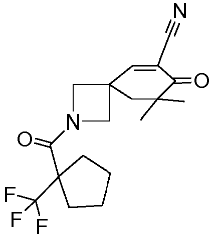 | 8,8-dimethyl-7-oxo-2-[1-(trifluoromethyl)cyclope ntane-1-carbonyl]-2-azaspiro[3.5]non-5-ene-6-carbonitrile | Calc'd 355.0, found 355.0 | A |
| 682 | 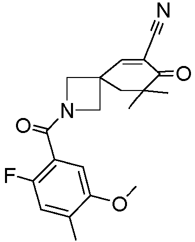 | 2-(2-fluoro-5-methoxy-4-methylbenzene-1-carbonyl)-8,8-dimethyl-7-oxo-2-azaspiro[3.5]non-5-ene-6-carbonitrile | Calc'd 357.0, found 357.0 | A |
| 683 | 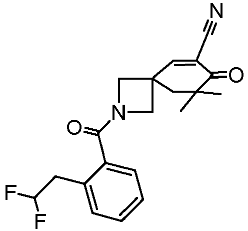 | 2-[2-(2,2-difluoroethyl)benzene-1-carbonyl]-8,8-dimethyl-7-oxo-2-azaspiro[3.5]non-5-ene-6-carbonitrile | Calc'd 359.0, found 359.0 | A |
| 684 | 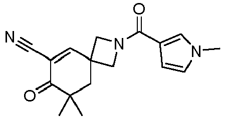 | 8,8-dimethyl-2-(1-methyl-1H-pyrrole-3-carbonyl)-7-oxo-2-azaspiro[3.5]non-5-ene-6-carbonitrile | 298 | B |
| 685 | 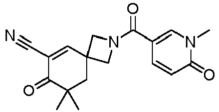 | 8,8-dimethyl-2-(1-methyl-6-oxo-1,6-dihydropyridine-3-carbonyl)-7-oxo-2-azaspiro[3.5]non-5-ene-6-carbonitrile | 326 | B |
| 686 | 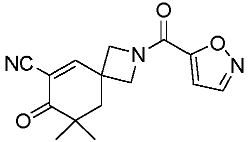 | 2-(isoxazole-5-carbonyl)-8,8-dimethyl-7-oxo-2-azaspiro[3.5]non-5-ene-6-carbonitrile | 286 | B |
| 687 | 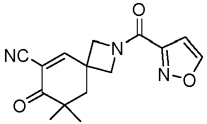 | 2-(isoxazole-3-carbonyl)-8,8-dimethyl-7-oxo-2-azaspiro[3.5]non-5-ene-6-carbonitrile | 286 | B |
| 688 | 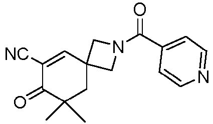 | 2-isonicotinoyl-8,8-dimethyl-7-oxo-2-azaspiro[3.5]non-5-ene-6-carbonitrile | 296 | B |
| 689 | 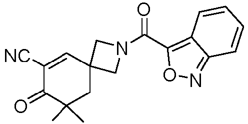 | 2-(benzo[c]isoxazole-3-carbonyl)-8,8-dimethyl-7-oxo-2-azaspiro[3.5]non-5-ene-6-carbonitrile | 358 [M + Na] | B |
| 690 | 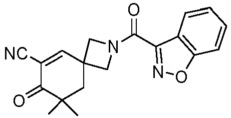 | 2-(benzo[d]isoxazole-3-carbonyl)-8,8-dimethyl-7-oxo-2-azaspiro[3.5]non-5-ene-6-carbonitrile | 336 | B |
| 691 | 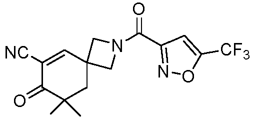 | 8,8-dimethyl-7-oxo-2-(5-(trifluoromethyl)isoxazol e-3-carbonyl)-2-azaspiro[3.5]non-5-ene-6-carbonitrile | 354 | B |
| 692 | 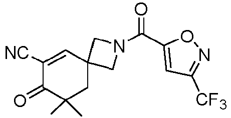 | 8,8-dimethyl-7-oxo-2-(3-(trifluoromethyl)isoxazol e-5-carbonyl)-2-azaspiro[3.5]non-5-ene-6-carbonitrile | 354 | B |
| 693 | 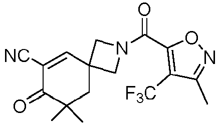 | 8,8-dimethyl-2-(3-methyl-4-(trifluoromethyl)isoxazol e-5-carbonyl)-7-oxo-2-azaspiro[3.5]non-5-ene-6-carbonitrile | 368 | B |
| 694 | 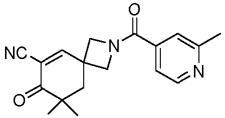 | 8,8-dimethyl-2-(2-methylisonicotinoyl)-7-oxo-2-azaspiro[3.5]non-5-ene-6-carbonitrile | 310 | B |
| 695 | 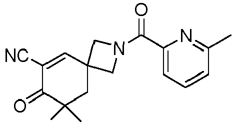 | 8,8-dimethyl-2-(6-methylpicolinoyl)-7-oxo-2-azaspiro[3.5]non-5-ene-6-carbonitrile | 310 | B |
| 696 | 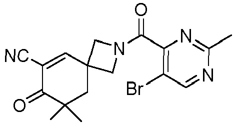 | 2-(5-bromo-2-methylpyrimidine-4-carbonyl)-8,8-dimethyl-7-oxo-2-azaspiro[3.5]non-5-ene-6-carbonitrile | 389 | B |
| 697 | 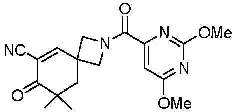 | 2-(2,6-dimethoxypyrimidine-4-carbonyl)-8,8-dimethyl-7-oxo-2-azaspiro[3.5]non-5-ene-6-carbonitrile | 357 | B |
| 698 | 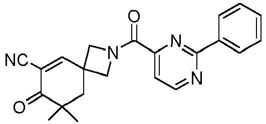 | 8,8-dimethyl-7-oxo-2-(2-phenylpyrimidine-4-carbonyl)-2-azaspiro[3.5]non-5-ene-6-carbonitrile | 373 | B |
| 699 | 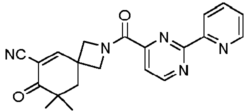 | 8,8-dimethyl-7-oxo-2-(2-(pyridin-2-yl)pyrimidine-4-carbonyl)-2-azaspiro[3.5]non-5-ene-6-carbonitrile | 374 | B |
| 700 | 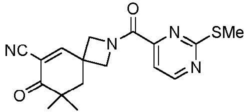 | 8,8-dimethyl-2-(2-(methylthio)pyrimidine-4-carbonyl)-7-oxo-2-azaspiro[3.5]non-5-ene-6-carbonitrile | 343 | B |
| 701 | 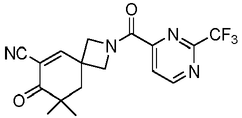 | 8,8-dimethyl-7-oxo-2-(2-(trifluoromethyl)pyrimidi ne-4-carbonyl)-2-azaspiro[3.5]non-5-ene-6-carbonitrile | 365 | B |
| 702 | 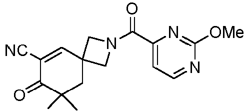 | 2-(2-methoxypyrimidine-4-carbonyl)-8,8-dimethyl-7-oxo-2-azaspiro[3.5]non-5-ene-6-carbonitrile | 327 | B |
| 703 | 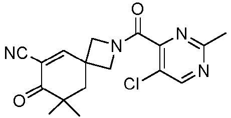 | 2-(5-chloro-2-methylpyrimidine-4-carbonyl)-8,8-dimethyl-7-oxo-2-azaspiro[3.5]non-5-ene-6-carbonitrile | 345 | B |
| 704 | 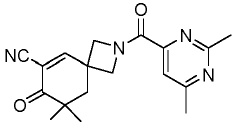 | 2-(2,6-dimethylpyrimidine-4-carbonyl)-8,8-dimethyl-7-oxo-2-azaspiro[3.5]non-5-ene-6-carbonitrile | 325 | B |
| 705 | 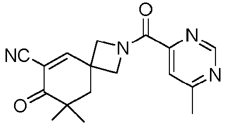 | 8,8-dimethyl-2-(6-methylpyrimidine-4-carbonyl)-7-oxo-2-azaspiro[3.5]non-5-ene-6-carbonitrile | 311 | B |
| 706 | 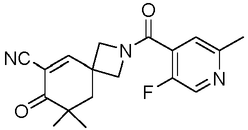 | 2-(5-fluoro-2-methylisonicotinoyl)-8,8-dimethyl-7-oxo-2-azaspiro[3.5]non-5-ene-6-carbonitrile | 328 | B |
| 707 | 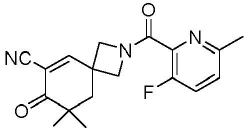 | 2-(3-fluoro-6-methylpicolinoyl)-8,8-dimethyl-7-oxo-2-azaspiro[3.5]non-5-ene-6-carbonitrile | 328 | B |
| 708 | 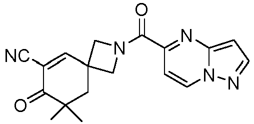 | 8,8-dimethyl-7-oxo-2-(pyrazolo[1,5-a]pyrimidine-5-carbonyl)-2-azaspiro[3.5]non-5-ene-6-carbonitrile | 336 | B |
| 709 | 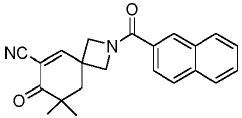 | 2-(2-naphthoyl)-8,8-dimethyl-7-oxo-2-azaspiro[3.5]non-5-ene-6-carbonitrile | 345 | B |
| 710 | 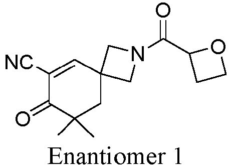 | 8,8-dimethyl-2-(oxetane-2-carbonyl)-7-oxo-2-azaspiro[3.5]non-5-ene-6-carbonitrile | 297 [M + Na] | B |
| 711 | 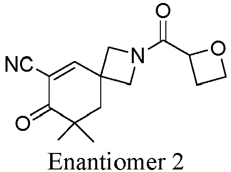 | 8,8-dimethyl-2-(oxetane-2-carbonyl)-7-oxo-2-azaspiro[3.5]non-5-ene-6-carbonitrile | 297 [M + Na] | B |
| 712 | 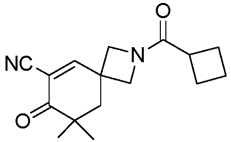 | 2-(cyclobutanecarbonyl)-8,8-dimethyl-7-oxo-2-azaspiro[3.5]non-5-ene-6-carbonitrile | 273 | B |
| 713 | 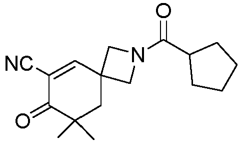 | 2-(cyclopentanecarbonyl)-8,8-dimethyl-7-oxo-2-azaspiro[3.5]non-5-ene-6-carbonitrile | 287 | B |
| 714 | 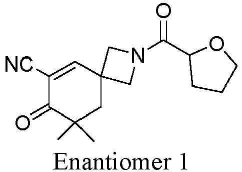 | 8,8-dimethyl-7-oxo-2-(tetrahydrofuran-2-carbonyl)-2-azaspiro[3.5]non-5-ene-6-carbonitrile | 311 [M+ Na] | B |
| 715 | 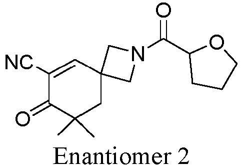 | 8,8-dimethyl-7-oxo-2-(tetrahydrofuran-2-carbonyl)-2-azaspiro[3.5]non-5-ene-6-carbonitrile | 311 [M+ Na] | B |
| 716 | 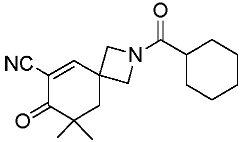 | 2-(cyclohexanecarbonyl)-8,8-dimethyl-7-oxo-2-azaspiro[3.5]non-5-ene-6-carbonitrile | 301 | B |
| 717 | 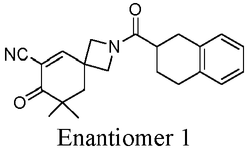 | 8,8-dimethyl-7-oxo-2-(1,2,3,4-tetrahydronaphthalene-2-carbonyl)-2-azaspiro[3.5]non-5-ene-6-carbonitrile | 349 | B |
| 718 | 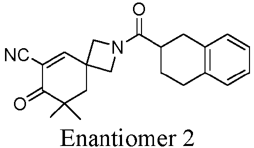 | 8,8-dimethyl-7-oxo-2-(1,2,3,4-tetrahydronaphthalene-2-carbonyl)-2-azaspiro[3.5]non-5-ene-6-carbonitrile | 349 | B |
| 719 | 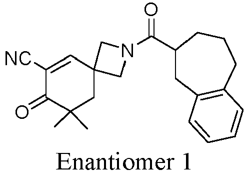 | 8,8-dimethyl-7-oxo-2-(6,7,8,9-tetrahydro-5H-benzo[7]annulene-6-carbonyl)-2-azaspiro[3.5]non-5-ene-6-carbonitrile | 385 [M + Na] | B |
| 720 | 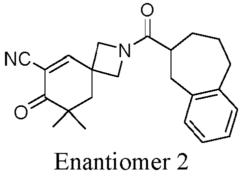 | 8,8-dimethyl-7-oxo-2-(6,7,8,9-tetrahydro-5H-benzo[7]annulene-6-carbonyl)-2-azaspiro[3.5]non-5-ene-6-carbonitrile | 385 [M + Na] | B |
| 721 | 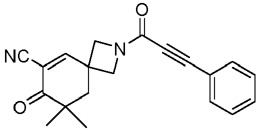 | 8,8-dimethyl-7-oxo-2-(3-phenylpropioloyl)-2-azaspiro[3.5]non-5-ene-6-carbonitrile | 319 | B |
| 722 | 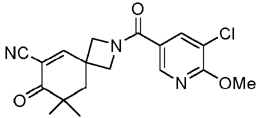 | 2-(5-chloro-6-methoxynicotinoyl)-8,8-dimethyl-7-oxo-2-azaspiro[3.5]non-5-ene-6-carbonitrile | 360 | B |
| 723 | 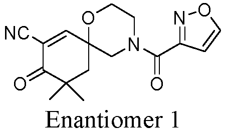 | 4-(isoxazole-3-carbonyl)-10,10-dimethyl-9-oxo-1-oxa-4-azaspiro[5.5]undec-7-ene-8-carbonitrile | 338 [M + Na] | B |
| 724 | 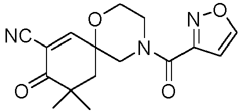 | 4-(isoxazole-3-carbonyl)-10,10-dimethyl-9-oxo-1-oxa-4-azaspiro[5.5]undec-7- | 338 [M + Na] | B |
| | 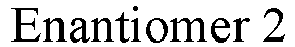 | ene-8-carbonitrile | | |
| 725 | 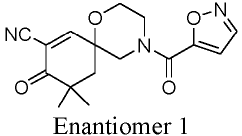 | 4-(isoxazole-5-carbonyl)-10,10-dimethyl-9-oxo-1-oxa-4-azaspiro[5.5]undec-7-ene-8-carbonitrile | 338 [M + Na] | B |
| 726 | 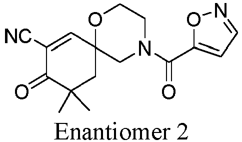 | 4-(isoxazole-5-carbonyl)-10,10-dimethyl-9-oxo-1-oxa-4-azaspiro[5.5]undec-7-ene-8-carbonitrile | 338 [M + Na] | B |
| 727 | 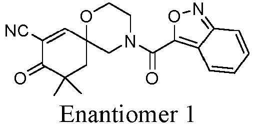 | 4-(benzo[c]isoxazole-3-carbonyl)-10,10-dimethyl-9-oxo-1-oxa-4-azaspiro[5.5]undec-7-ene-8-carbonitrile | 388 [M + Na] | B |
| 728 | 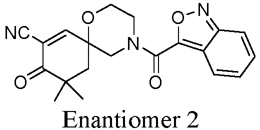 | 4-(benzo[c]isoxazole-3-carbonyl)-10,10-dimethyl-9-oxo-1-oxa-4-azaspiro[5.5]undec-7-ene-8-carbonitrile | 388 [M + Na] | B |
| 729 | 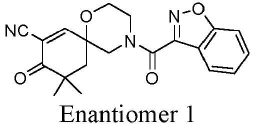 | 4-(benzo[d]isoxazole-3-carbonyl)-10,10-dimethyl-9-oxo-1-oxa-4-azaspiro[5.5]undec-7-ene-8-carbonitrile | 366 | B |
| 730 | 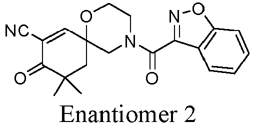 | 4-(benzo[d]isoxazole-3-carbonyl)-10,10-dimethyl-9-oxo-1-oxa-4-azaspiro[5.5]undec-7-ene-8-carbonitrile | 366 | B |
| 731 | 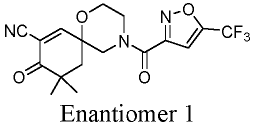 | 10,10-dimethyl-9-oxo-4-(5-(trifluoromethyl)isoxazol e-3-carbonyl)-1-oxa-4-azaspiro[5.5]undec-7-ene-8-carbonitrile | 384 | B |
| 732 | 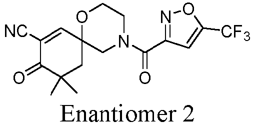 | 10,10-dimethyl-9-oxo-4-(5-(trifluoromethyl)isoxazol e-3-carbonyl)-1-oxa-4-azaspiro[5.5]undec-7-ene-8-carbonitrile | 384 | B |
| 733 | 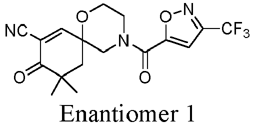 | 10,10-dimethyl-9-oxo-4-(3-(trifluoromethyl)isoxazol e-5-carbonyl)-1-oxa-4-azaspiro[5.5]undec-7- | 384 | B |
| | | ene-8-carbonitrile | | |
| 734 | 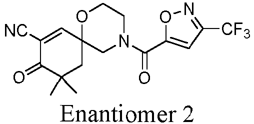 | 10,10-dimethyl-9-oxo-4-(3-(trifluoromethyl)isoxazol e-5-carbonyl)-1-oxa-4-azaspiro[5.5]undec-7-ene-8-carbonitrile | 384 | B |
| 735 | 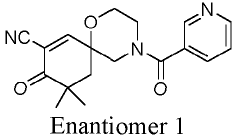 | 10,10-dimethyl-4-nicotinoyl-9-oxo-1-oxa-4-azaspiro[5.5]undec-7-ene-8-carbonitrile | 326 | B |
| 736 | 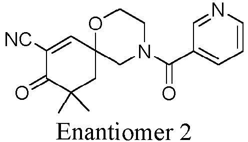 | 10,10-dimethyl-4-nicotinoyl-9-oxo-1-oxa-4-azaspiro[5.5]undec-7-ene-8-carbonitrile | 326 | B |
| 737 | 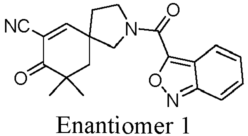 | 2-(benzo[c]isoxazole-3-carbonyl)-9,9-dimethyl-8-oxo-2-azaspiro[4.5]dec-6-ene-7-carbonitrile | 372 [M + Na] | B |
| 738 | 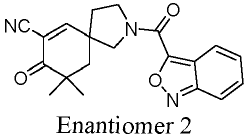 | 2-(benzo[c]isoxazole-3-carbonyl)-9,9-dimethyl-8-oxo-2-azaspiro[4.5]dec-6-ene-7-carbonitrile | 372 [M + Na] | B |
| 739 | 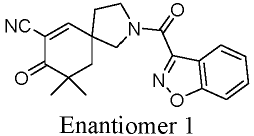 | 2-(benzo[d]isoxazole-3-carbonyl)-9,9-dimethyl-8-oxo-2-azaspiro[4.5]dec-6-ene-7-carbonitrile | 350 | B |
| 740 | 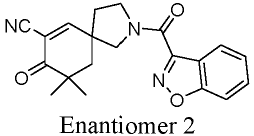 | 2-(benzo[d]isoxazole-3-carbonyl)-9,9-dimethyl-8-oxo-2-azaspiro[4.5]dec-6-ene-7-carbonitrile | 350 | B |
| 741 | 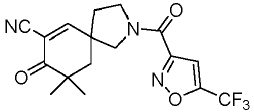 Made from Intermediate 6 | 9,9-dimethyl-8-oxo-2-(5-(trifluoromethyl)isoxazol e-3-carbonyl)-2-azaspiro[4.5]dec-6-ene-7-carbonitrile | 368 | B |
| 742 | 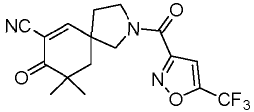 Made from Intermediate 6 | 9,9-dimethyl-8-oxo-2-(5-(trifluoromethyl)isoxazol e-3-carbonyl)-2-azaspiro[4.5]dec-6-ene-7-carbonitrile | 368 | B |
| 743 | 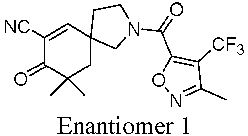 | 9,9-dimethyl-2-(3-methyl-4-(trifluoromethyl)isoxazol e-5-carbonyl)-8-oxo-2-azaspiro[4.5]dec-6-ene-7-carbonitrile | 382 | B |
| 744 | 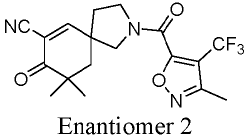 | 9,9-dimethyl-2-(3-methyl-4-(trifluoromethyl)isoxazol e-5-carbonyl)-8-oxo-2-azaspiro[4.5]dec-6-ene-7-carbonitrile | 382 | B |
| 745 | 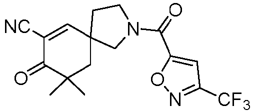 Made from Intermediate 6 | 9,9-dimethyl-8-oxo-2-(3-(trifluoromethyl)isoxazol e-5-carbonyl)-2-azaspiro[4.5]dec-6-ene-7-carbonitrile | 368 | B |
| 746 | 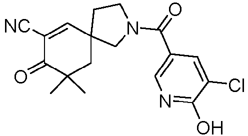 Made from Intermediate 6 | 2-(5-chloro-6-hydroxynicotinoyl)-9,9-dimethyl-8-oxo-2-azaspiro[4.5]dec-6-ene-7-carbonitrile | 360 | B |
| 747 | 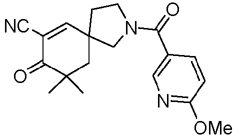 Made from Intermediate 6 | 2-(6-methoxynicotinoyl)-9,9-dimethyl-8-oxo-2-azaspiro[4.5]dec-6-ene-7-carbonitrile | 340 | B |
| 748 | 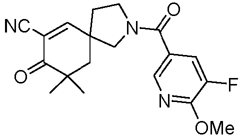 Made from Intermediate 6 | 2-(5-fluoro-6-methoxynicotinoyl)-9,9-dimethyl-8-oxo-2-azaspiro[4.5]dec-6-ene-7-carbonitrile | 358 | B |
| 749 | 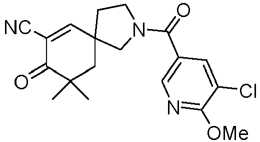 Made from Intermediate 6 | 2-(5-chloro-6-methoxynicotinoyl)-9,9-dimethyl-8-oxo-2-azaspiro[4.5]dec-6-ene-7-carbonitrile | 374 | B |
| 750 | 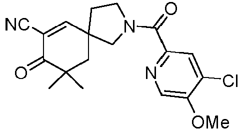 Made from Intermediate 6 | 2-(4-chloro-5-methoxypicolinoyl)-9,9-dimethyl-8-oxo-2-azaspiro[4.5]dec-6-ene-7-carbonitrile | 374 | B |
| 751 | 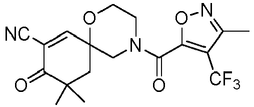 Made from Intermediate 8 | 10,10-dimethyl-4-(3-methyl-4-(trifluoromethyl)isoxazol e-5-carbonyl)-9-oxo-1-oxa-4-azaspiro[5.5]undec-7-ene-8-carbonitrile | 398 | B |
| 752 | 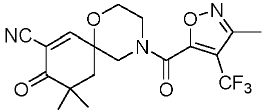 Made from Intermediate 8 | 10,10-dimethyl-4-(3-methyl-4-(trifluoromethyl)isoxazol e-5-carbonyl)-9-oxo-1-oxa-4-azaspiro[5.5]undec-7-ene-8-carbonitrile | 398 | B |
| 753 | 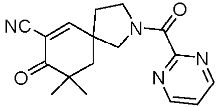 Made from Intermediate 6 | 9,9-dimethyl-8-oxo-2-(pyrimidine-2-carbonyl)-2-azaspiro[4.5]dec-6-ene-7-carbonitrile | 311 | B |
| 754 | 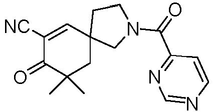 Made from Intermediate 6 | 9,9-dimethyl-8-oxo-2-(pyrimidine-4-carbonyl)-2-azaspiro[4.5]dec-6-ene-7-carbonitrile | 311 | B |
| 755 | 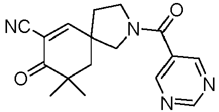 Made from Intermediate 6 | 9,9-dimethyl-8-oxo-2-(pyrimidine-5-carbonyl)-2-azaspiro[4.5]dec-6-ene-7-carbonitrile | 311 | B |
| 756 | 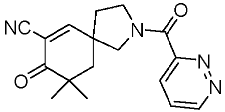 Made from Intermediate 6 | 9,9-dimethyl-8-oxo-2-(pyridazine-3-carbonyl)-2-azaspiro[4.5]dec-6-ene-7-carbonitrile | 311 | B |
| 757 | 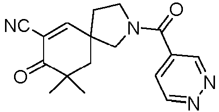 Made from Intermediate 6 | 9,9-dimethyl-8-oxo-2-(pyridazine-4-carbonyl)-2-azaspiro[4.5]dec-6-ene-7-carbonitrile | 311 | B |
| 758 | 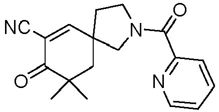 Made from Intermediate 6 | 9,9-dimethyl-8-oxo-2-picolinoyl-2-azaspiro[4.5]dec-6-ene-7-carbonitrile | 310 | B |
| 759 | 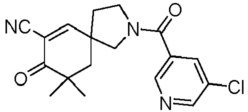 | 2-(5-chloronicotinoyl)-9,9-dimethyl-8-oxo-2-azaspiro[4.5]dec-6-ene-7-carbonitrile | 344 | B |
| | Made from Intermediate 6 | | | |
| 760 | 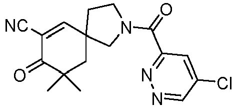 | 2-(5-chloropyridazine-3-carbonyl)-9,9-dimethyl-8-oxo-2-azaspiro[4.5]dec-6-ene-7-carbonitrile | 345 | B |
| 761 | 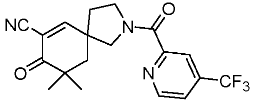 Made from Intermediate 6 | 9,9-dimethyl-8-oxo-2-(4-(trifluoromethyl)picolino yl)-2-azaspiro[4.5]dec-6-ene-7-carbonitrile | 378 | B |
| 762 | 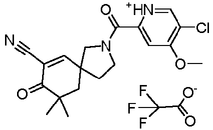 Made from Intermediate 6 | 5-chloro-2-(7-cyano-9,9-dimethyl-8-oxo-2-azaspiro[4.5]dec-6-ene-2-carbonyl)-4-methoxypyridin-1-ium 2,2,2-trifluoroacetate | 374 | B |
| 763 | 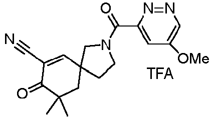 Made from Intermediate 6 | 2-(5-methoxypyridazine-3-carbonyl)-9,9-dimethyl-8-oxo-2-azaspiro[4.5]dec-6-ene-7-carbonitrile | 341 | B |
| 764 | 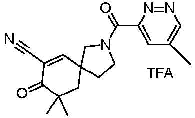 Made from Intermediate 6 | 9,9-dimethyl-2-(5-methylpyridazine-3-carbonyl)-8-oxo-2-azaspiro[4.5]dec-6-ene-7-carbonitrile | 325 | B |
| 765 | 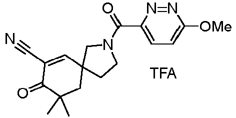 Made from Intermediate 6 | 2-(6-methoxypyridazine-3-carbonyl)-9,9-dimethyl-8-oxo-2-azaspiro[4.5]dec-6-ene-7-carbonitrile compound with 2,2,2-trifluoro-113-ethan-1-one (1:1) | 341 | B |

### General Procedures for Table 18:

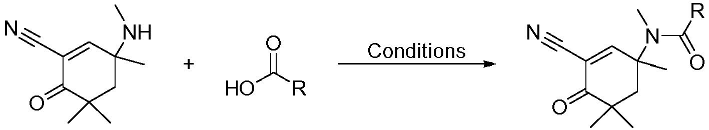

### Method A: BopCl, NMM, DMA, MeCN, 60 °C, 16 h

An 8 mL vial was charged with (R)-3,5,5-trimethyl-3-(methylamino)-6-oxocyclohex-1-ene-1-carbonitrile (9.61 mg, 50.0 µmol), carboxylic acid (50 µmol), DMA (250 µL) and MeCN (250 µL). Bis(2-oxo-3-oxazolidinyl)phosphinic chloride (19.1 mg, 75.0 µmol) and NMM (15.2 mg, 150 µmol) were then added to the reaction mixture. The vial was capped and shaken at 60 °C for 16 h. The reaction was monitored by LCMS. After the reaction was completed, the reaction mixture was concentrated by Speedvac. The resulting residue was dissolved in MeCN and H₂O and purified by preparative HPLC to afford pure final product.

### Method B: Mukaiyama, DIEA, THF, 80 °C, 16 h

An 8 mL vial was charged with (R)-3,5,5-trimethyl-3-(methylamino)-6-oxocyclohex-1-ene-1-carbonitrile (9.61 mg, 50.0 µmol), carboxylic acid (50 µmol) and THF (500 µL). 2-Chloro-1-methylpyridinium iodide (12.8 mg, 50.0 µmol) and NN-diisopropylethylamine (6.46 mg, 50.0 µmol) were then added to the reaction mixture. The vial was capped and shaken at 80°C for 16 h. The reaction was monitored by LCMS. After the reaction was completed, the reaction mixture was concentrated by Speedvac. The resulting residue was dissolved in MeCN and H₂O and purified by preparative HPLC to afford pure final product.

**Table 18: Compounds were prepared in a similar manner to the general procedures**

| **Ex.** | **Enant iomer** | **Structure** | **IUPAC Name** | **Exact Mass [M+H]+** | **Svnthes is Method** |
|---|---|---|---|---|---|
| 766* | 1 | 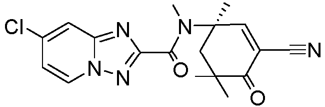 | 7-chloro-N-[(1R)-3-cyano-1,5,5-trimethyl-4-oxocyclohex-2-en-1-yl]-N-methyl[1,2,4]triazolo[ 1,5-a]pyridine-2-carboxamide | Calc'd 372.0, found 372.1 | B |
| 767* | 2 | 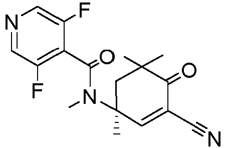 | N-[(1S)-3-cyano-1,5,5-trimethyl-4-oxocyclohex-2-en-1-yl]-3,5-difluoro-N-methylpyridine-4-carboxamide | Calc'd 334.0, found 334.1 | A |
| 768* | 2 | 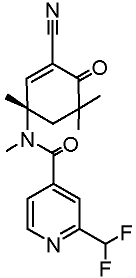 | N-[(1S)-3-cyano-1,5,5-trimethyl-4-oxocyclohex-2-en-1-yl]-2-(difluoromethyl)-N-methylpyridine-4-carboxamide | Calc'd 348.0, found 348.1 | A |
| 769* | 2 | 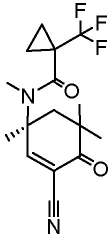 | N-[(1S)-3-cyano-1,5,5-trimethyl-4-oxocyclohex-2-en-1-yl]-N-methyl-1-(trifluoromethyl)cyclo propane-1-carboxamide | Calc'd 329.0, found 329.1 | B |
| 770* | 2 | 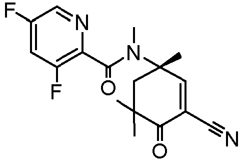 | N-[(1S)-3-cyano-1,5,5-trimethyl-4-oxocyclohex-2-en-1-yl]-3,5-difluoro-N-methylpyridine-2-carboxamide | Calc'd 334.0, found 334.1 | A |
| 771* | 2 | 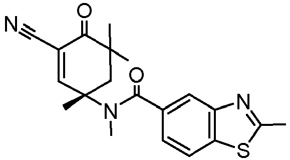 | (S)-N-(3-cyano-1,5,5-trimethyl-4-oxocyclohex-2-en-1-yl)-N,2-dimethylbenzo[d]thiaz ole-5-carboxamide | 368 | A |
| 772* | 2 | 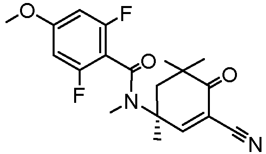 | N-[(1S)-3-cyano-1,5,5-trimethyl-4-oxocyclohex-2-en-1-yl]-2,6-difluoro-4-methoxy-N-methylbenzamide | Calc'd 363.0, found 363.1 | A |
| 773* | 2 | 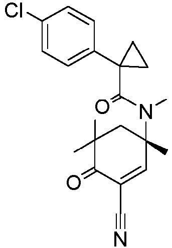 | 1-(4-chlorophenyl)-N-[(1S)-3-cyano-1,5,5-trimethyl-4-oxocyclohex-2-en-1-yl]-N-methylcyclopropane-1-carboxamide | Calc'd 371.0, found 371.1 | A |
| 774* | 2 | 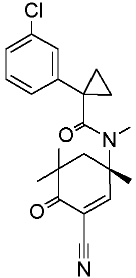 | 1-(3-chlorophenyl)-N-[(1S)-3-cyano-1,5,5-trimethyl-4-oxocyclohex-2-en-1-yl]-N-methylcyclopropane-1-carboxamide | Calc'd 371.0, found 371.1 | B |
| 775* | 2 | 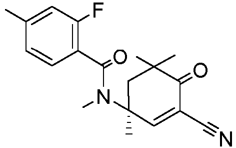 | N-[(1S)-3-cyano-1,5,5-trimethyl-4-oxocyclohex-2-en-1-yl]-2-fluoro-N,4-dimethylbenzamide | Calc'd 329.0, found 329.1 | A |
| 776* | 2 | 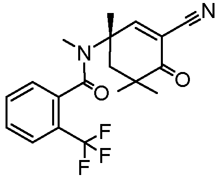 | N-[(1S)-3-cyano-1,5,5-trimethyl-4-oxocyclohex-2-en-1-yl]-N-methyl-2-(trifluoromethyl)benza mide | Calc'd 365.0, found 365.1 | A |
| 777* | 2 | 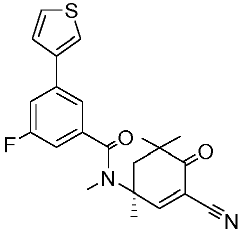 | N-[(1S)-3-cyano-1,5,5-trimethyl-4-oxocyclohex-2-en-1-yl]-3-fluoro-N-methyl-5-(thiophen-3-yl)benzamide | Calc'd 397.0, found 397.1 | A |
| 778* | 2 | 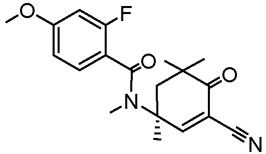 | N-[(1S)-3-cyano-1,5,5-trimethyl-4-oxocyclohex-2-en-1-yl]-2-fluoro-4-methoxy-N-methylbenzamide | Calc'd 345.0, found 345.1 | A |
| 779* | 2 | 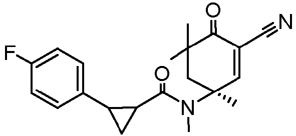 | N-[(1S)-3-cyano-1,5,5-trimethyl-4-oxocyclohex-2-en-1-yl]-2-(4-fluorophenyl)-N-methylcyclopropane-1-carboxamide | Calc'd 355.0, found 355.1 | B |
| 780* | 2 | 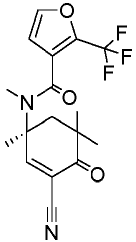 | N-[(1S)-3-cyano-1,5,5-trimethyl-4-oxocyclohex-2-en-1-yl]-N-methyl-2-(trifluoromethyl)furan -3-carboxamide | Calc'd 355.0, found 355.1 | B |
| 781* | 2 | 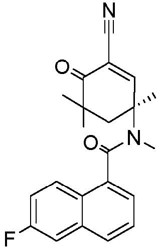 | N-[(1S)-3-cyano-1,5,5-trimethyl-4-oxocyclohex-2-en-1-yl]-6-fluoro-N-methylnaphthalene-1-carboxamide | Calc'd 365.0, found 365.1 | A |
| 782* | 2 | 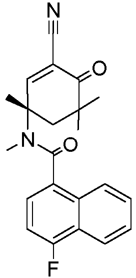 | N-[(1S)-3-cyano-1,5,5-trimethyl-4-oxocyclohex-2-en-1-yl]-4-fluoro-N-methylnaphthalene-1-carboxamide | Calc'd 365.0, found 365.1 | A |
| 783* | 2 | 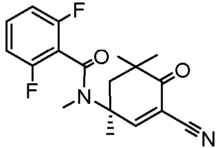 | N-[(1S)-3-cyano-1,5,5-trimethyl-4-oxocyclohex-2-en-1-yl]-2,6-difluoro-N-methylbenzamide | Calc'd 333.0, found 333.1 | A |
| 784* | 2 | 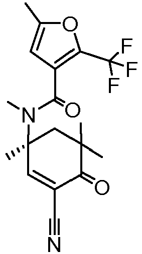 | N-[(1S)-3-cyano-1,5,5-trimethyl-4-oxocyclohex-2-en-1-yl]-N,5-dimethyl-2-(trifluoromethyl)furan -3-carboxamide | Calc'd 369.0, found 369.1 | B |
| 785* | 2 | 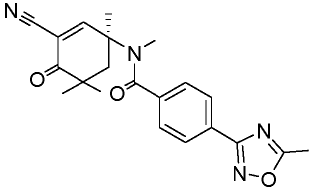 | N-[(1S)-3-cyano-1,5,5-trimethyl-4-oxocyclohex-2-en-1-yl]-N-methyl-4-(5-methyl-1,2,4-oxadiazol-3 - yl)benzamide | Calc'd 379.0, found 379.1 | A |
| 786* | 2 | 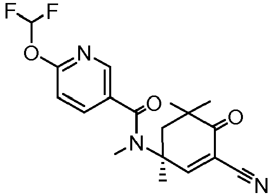 | N-[(1S)-3-cyano-1,5,5-trimethyl-4-oxocyclohex-2-en-1-yl]-6-(difluoromethoxy)-N-methylpyridine-3-carboxamide | Calc'd 364.0, found 364.1 | A |
| 787* | 2 | 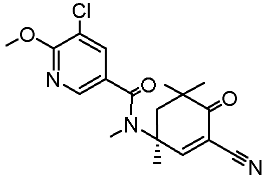 | 5-chloro-N-[(1S)-3-cyano-1,5,5-trimethyl-4-oxocyclohex-2-en-1-yl]-6-methoxy-N-methylpyridine-3-carboxamide | Calc'd 362.0, found 362.0 | A |
| 788* | 2 | 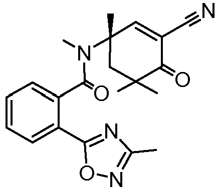 | N-[(1S)-3-cyano-1,5,5-trimethyl-4-oxocyclohex-2-en-1-yl]-N-methyl-2-(3-methyl-1,2,4-oxadiazol-5-yl)benzamide | Calc'd 379.0, found 379.1 | A |

| | | | | | |
|---|---|---|---|---|---|
| * reference compound | | | | | |

### Example 789: 7-oxo-1-oxaspiro[3.5]non-5-ene-6-carbonitrile

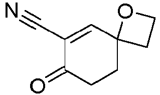

**Step 1:** To LDA (2 M in THF, 1.2 mL, 2.4 mmol) was added THF (1.5 mL) and the mixture was cooled to -78 °C under an N₂ atmosphere. To this mixture was added a solution of 1-oxaspiro[3.5]nonan-7-one (280 mg, 2.0 mmol) in THF (1.5 mL). The resulting mixture was stirred at -78 °C for 20 minutes, then taken up in a syringe and added to a mixture of 4-methylbenzenesulfonyl cyanide (724 mg, 3.99 mmol) in THF (1.5 mL) at -78 °C under an atmosphere of N₂. The reaction mixture was allowed to stir at -78 °C for 45 minutes. The mixture was quenched with NH₃.H₂O (5 mL) and extracted with EtOAc (2 x 10 mL). The combined organic layers were washed with 1 N aq. HCl and then brine, dried over anhydrous sodium sulfate, filtered, and concentrated under reduced pressure to afford 7-oxo-1-oxaspiro[3.5]nonane-6-carbonitrile as an oil. MS: 166 (M + H). ¹H NMR (400 MHz, Chloroform-*d*): δ 4.76 - 4.48 (m, 2H), 4.30 - 4.29 (m, 1H), 2.83 - 2.59 (m, 1H), 2.57 - 2.40 (m, 4H), 2.30 - 2.02 (m, 2H), 1.97 - 1.84 (m, 1H).

**Step 2:** A solution of 7-oxo-1-oxaspiro[3.5]nonane-6-carbonitrile (80 mg, 0.48 mmol) in DMF (2 mL) was added 1,3-dibromo-5,5-dimethylhydantoin (49.1 mg, 0.172 mmol) at 0 °C. The reaction was stirred at 0 °C for 1 h, and then pyridine (80 mg, 1.0 mmol) was added. The reaction was heated at 55 °C for 2 h, and then cooled to room temperature. EtOAc (10 mL) was added, and the mixture was washed with 1 N aq. HCl (5 mL) and water (5 mL). The organic layer was dried over anhydrous sodium sulfate, filtered, and concentrated under reduced pressure. The residue was purified by column chromatography on silica (0-70% EtOAc/hexane) and then further purified by mass triggered reverse phase HPLC (5-40% ACN/water with 5 mM formic acid modifier) to give 7-oxo-1-oxaspiro[3.5]non-5-ene-6-carbonitrile as a solid. MS: 164 (M + H). ¹H NMR (300 MHz, Chloroform-*d*): δ 7.86 (s, 1H), 4.75 - 4.59 (m, 2H), 2.88 - 2.82 (m, 2H), 2.80 - 2.69 (m, 1H), 2.67 - 2.38 (m, 3H).

**Table 19: The following example was prepared using a similar procedure as described above for example 789.**

| **Ex** | **Structure** | **IUPAC Name** | **Exact Mass [M+H]⁺** |
|---|---|---|---|
| 790 | 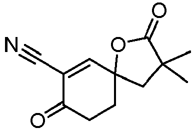 | 3,3-dimethyl-2,8-dioxo-1-oxaspiro[4.5]dec-6-ene-7-carbonitrile | 220 |

### Example 791 - enantiomer 1: 9,9-dimethyl-8-oxo-2-phenyl-2-azaspiro[4.5]dec-6-ene-7-carbonitrile

### Example 792 - enantiomer 2: 9,9-dimethyl-8-oxo-2-phenyl-2-azaspiro[4.5]dec-6-ene-7-carbonitrile

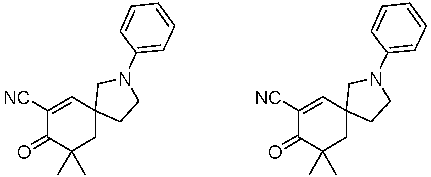

To a vial containing 9,9-dimethyl-8-oxo-2-azaspiro[4.5]dec-6-ene-7-carbonitrile, HCl (131 mg, 0.544 mmol), RuPhos Pd G2 (42 mg, 0.054 mmol), and 2-dicyclohexylphosphino-2',6'-diisopropoxybiphenyl (25 mg, 0.054 mmol) under a nitrogen atmosphere was added dioxane (2.7 mL). Bromobenzene (69 µL, 0.65 mmol) and sodium *tert*-butoxide (2.0 M in THF, 544 µL, 1.09 mmol) were added and the mixture was stirred at 60 °C overnight. The mixture was cooled to room temperature, diluted with DCM, filtered through Celite^{®}, and concentrated under reduced pressure. The residue was purified by column chromatography on silica (0-70% Et₂O/Hexanes) to give 9,9-dimethyl-8-oxo-2-phenyl-2-azaspiro[4.5]dec-6-ene-7-csarbonitrile. The racemic mixture was purified by chiral SFC (OJ-H column, 40%/60% MeOH/CO₂ with 0.1% NH₄OH modifier) to afford two products as solids:
9,9-dimethyl-8-oxo-2-phenyl-2-azaspiro[4.5]dec-6-ene-7-carbonitrile (Example 78 - enantiomer 1). MS: 281 (M + H). ¹H NMR (600 MHz, DMSO-*d*₆) δ 7.94 (s, 1H), 7.18 (t, *J =* 7.9 Hz, 2H), 6.64 (t, *J =* 7.3 Hz, 1H), 6.57 (d, *J* = 7.9 Hz, 2H), 3.56 - 3.50 (m, 2H), 3.40 - 3.35 (m, 1H), 3.31 - 3.28 (m, 1H), 2.31 - 2.26 (m, 1H), 2.17 - 2.11 (m, 1H), 2.06 (s, 2H), 1.16 (s, 6H). 9,9-dimethyl-8-oxo-2-phenyl-2-azaspiro[4.5]dec-6-ene-7-carbonitrile (Example 79 - enantiomer 2). MS: 281 (M + H). ¹H NMR (600 MHz, DMSO-*d*₆) δ 7.94 (s, 1H), 7.18 (t, *J =* 7.9 Hz, 2H), 6.64 (t, *J =* 7.2 Hz, 1H), 6.57 (d, *J =* 8.0 Hz, 2H), 3.56 - 3.49 (m, 2H), 3.40 - 3.36 (m, 1H), 3.31 - 3.29 (m, 1H), 2.31 - 2.26 (m, 1H), 2.18 - 2.11 (m, 1H), 2.06 (s, 2H), 1.16 (s, 6H).

### Example 793, enantiomer 1: 9,9-dimethyl-8-oxo-2-(pyridin-3-yl)-2-azaspiro[4.5]dec-6-ene-7-carbonitrile

### Example 794, enantiomer 2: 9,9-dimethyl-8-oxo-2-(pyridin-3-yl)-2-azaspiro[4.5]dec-6-ene-7-carbonitrile

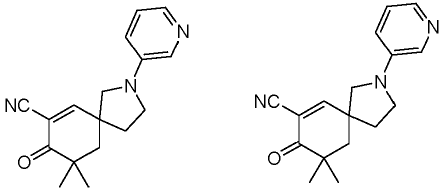

**Step 1:** To vial containing 9,9-dimethyl-8-oxo-2-azaspiro[4.5]dec-6-ene-7-carbonitrile, HCl (Intermediate $, 20 mg, 0.083 mmol) and racemic-BINAP-Pd-G3 (8.2 mg, 8.3 µmol) was added THF (665 µL), 3-bromopyridine (9.6 µL, 0.10 mmol), and sodium *tert*-butoxide (2.0 M in THF, 166 µl, 0.332 mmol). The mixture was stirred overnight at 80°C. The reaction was cooled to room temperature, quenched with water, and extracted with EtOAc (3x). The combined organic layers were dried over anhydrous sodium sulfate, filtered, and concentrated under reduced pressure. The residue was purified by mass triggered reverse phase HPLC (ACN/H₂O with 0.1% TFA modifier) to afford 9,9-dimethyl-8-oxo-2-(pyridin-3-yl)-2-azaspiro[4.5]dec-6-ene-7-carbonitrile as a solid TFA salt. MS: 282 (M + H). ¹H NMR (600 MHz, DMSO-*d*₆) δ 8.11 (d, *J* = 2.7 Hz, 1H), 8.08 (d, *J =* 5.2 Hz, 1H), 7.96 (s, 1H), 7.72 (dd, *J =* 8.7, 5.3 Hz, 1H), 7.56 (d, *J* = 6.9 Hz, 1H), 3.72 (d, *J =* 10.6 Hz, 1H), 3.65 - 3.60 (m, 1H), 3.56 - 3.51 (m, 1H), 3.43 (d, *J =* 10.6 Hz, 1H), 2.38 - 2.33 (m, 1H), 2.23 - 2.16 (m, 1H), 2.08 (q, *J =* 14.2 Hz, 2H), 1.17 (d, *J =* 2.6 Hz, 6H). Synthesized as described in the example above from intermediate 5 to afford 9,9-dimethyl-8-oxo-2-(pyridin-3-yl)-2-azaspiro[4.5]dec-6-ene-7-carbonitrile as a solid TFA salt. MS: 282 (M + H). ¹H NMR (600 MHz, DMSO-*d*₆) δ 8.12 (d, *J =* 2.7 Hz, 1H), 8.09 (d, *J =* 5.2 Hz, 1H), 7.96 (s, 1H), 7.74 (dd, *J =* 8.7, 5.3 Hz, 1H), 7.59 (dd, *J =* 8.7, 2.2 Hz, 1H), 3.72 (d, *J =* 10.6 Hz, 1H), 3.66 - 3.60 (m, 1H), 3.56 - 3.52 (m, 1H), 3.43 (d, *J=* 10.6 Hz, 1H), 2.38 - 2.32 (m, 1H), 2.23 - 2.15 (m, 1H), 2.08 (q, *J =* 14.2 Hz, 2H), 1.17 (d, *J =* 2.7 Hz, 6H).

### Example 795: 2-(6-(1,1-difluoroethyl)pyridin-2-yl)-9,9-dimethyl-8-oxo-2-azaspiro[4.5]dec-6-ene-7-carbonitrile

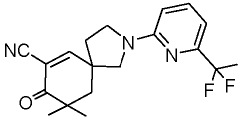

**Step 1:** In 8 mL vials, 9,9-dimethyl-8-oxo-2-azaspiro[4.5]dec-6-ene-7-carbonitrile (10.2 mg, 50 µmol) and 2-bromo-6-(1,1-difluoroethyl)pyridine (11.5 mg, 50 µmol) were added, then XPhos Pd G3 (2.11 mg, 2.5 µmol), t-BuONa (14.7 mg, 150 µmol) were added in dioxane (1000 µl). The vials were capped and shaken at 120 °C for 16 h. Reaction was monitored by LCMS. After the the reaction mixture was concentrated by Speedvac. The residue was dissolved in ACN/ H₂O and purified by preparative HPLC (Instrument EJ; Method: TFA; Column: Phenomenex XbridgeC18 100*30mm*5um; Condition water (0.1%TFA)-CAN; Begin B 51; End B 81 Gradient Time (min): 8.8; 100%B Hold Time (min): 2; Flow Rate(ml/min): 20; Injections 1) to provide 2-(6-(1,1-difluoroethyl)pyridin-2-yl)-9,9-dimethyl-8-oxo-2-azaspiro[4.5]dec-6-ene-7-carbonitrile (MS (ESI) [M+H]+ *m*/*z* 346.0, 2.92 mg.

### Example 796: 8,8-dimethyl-7-oxo-2-(pyridin-3-yl)-2-azaspiro[3.5]non-5-ene-6-carbonitrile

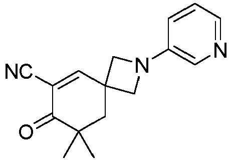

A vial was charged with 8,8-dimethyl-7-oxo-2-azaspiro[3.5]non-5-ene-6-carbonitrile, HCl (22.7 mg, 0.10 mmol) and SPhos Pd G4 (7.9 mg, 0.01 mmol) and brought into the glovebox. THF (0.8 mL), 3-bromopyridine (12 µL, 0.12 mmol) and sodium *tert*-butoxide (2 M in THF, 0.2 mL, 0.4 mmol) were added. The vial was sealed, removed from the glovebox, and placed in a preheated pie-block at 80 °C. The reaction mixture was stirred at 80 °C overnight, then cooled to room temperature and quenched with water (1.0 mL). The mixture was then extracted with EtOAc (3 x 1 mL), the combined organic layers were dried over anhydrous sodium sulfate, filtered, and concentrated under reduced pressure. The residue was purified by mass triggered reverse phase HPLC (ACN/water with 0.1% TFA modifier) to afford 8,8-dimethyl-7-oxo-2-(pyridin-3-yl)-2-azaspiro[3.5]non-5-ene-6-carbonitrile as a solid. MS: 268 (M + H). ¹H NMR (600 MHz, DMSO-*d*₆) δ 8.36 (s, 1H), 8.00 - 7.95 (m, 1H), 7.87 (d, *J =* 2.8 Hz, 1H), 7.20 (dd, *J =* 8.2, 4.6 Hz, 1H), 6.89 (ddd, *J* = 8.3, 2.8, 1.2 Hz, 1H), 4.12 (d, *J =* 7.4 Hz, 2H), 3.93 (d, *J =* 7.4 Hz, 2H), 2.25 (s, 2H), 1.08 (s, 6H).

**Table 20: The following example was prepared using a similar procedure as described above for example 796.**

| **Ex** | **Structure** | **IUPAC Name** | **Exact Mass [M+H]⁺** |
|---|---|---|---|
| 797 | 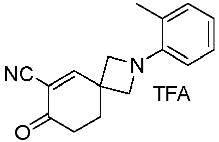 | 7-oxo-2-(o-tolyl)-2-azaspiro[3.5]non-5-ene-6-carbonitrile trifluoroacetate | 281 |
| 798 | 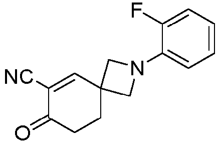 | 2-(2-fluorophenyl)-7-oxo-2-azaspiro[3.5]non-5-ene-6-carbonitrile trifluoroacetate | 285 |
| 799 | 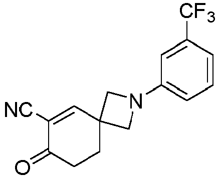 | 7-oxo-2-(3-(trifluoromethyl)phenyl)-2-azaspiro[3.5]non-5-ene-6-carbonitrile trifluoroacetate | 335 |
| 800 | 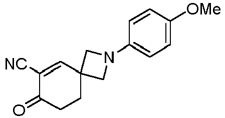 | 2-(4-methoxyphenyl)-7-oxo-2-azaspiro[3.5]non-5-ene-6-carbonitrile trifluoroacetate | 297 |
| 801 | 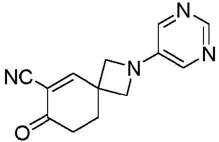 | 7-oxo-2-(pyrimidin-5-yl)-2-azaspiro[3.5]non-5-ene-6-carbonitrile trifluoroacetate | 269 |
| 802 | 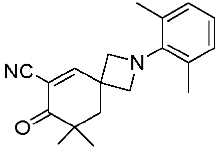 | 2-(2,6-dimethylphenyl)-8,8-dimethyl-7-oxo-2-azaspiro[3.5]non-5-ene-6-carbonitrile | 295 |

### Example 803: 4-(2-methoxyphenyl)-10,10-dimethyl-9-oxo-1-oxa-4-azaspiro[5.5]undec-7-ene-8-carbonitrile

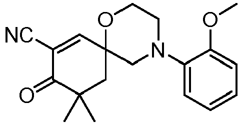

In a microwave vial, equipped with a magnetic stirrer and inert atmosphere, to a solution of 8-cyano-10,10-dimethyl-9-oxo-1-oxa-4-azaspiro[5.5]undec-7-en-4-ium chloride, **Intermediate 7** (80 mg, 0.312 mmols) in dry dioxane (3.0 mL) potassium carbonate (43 mg, 0.312 mmols) was added and the resulting mixture was stirred at *r.t.* for 1h. Then, an additional aliquot of potassium carbonate (60 mg, 0.44 mmols), 1-bromo-2-methoxybenzene (46.6 µL, 0.374 mmols) and XPhos Pd G3 (26.4 mg, 0.031 mmols) were added. The resulting reaction mixture was sealed and stirred at 80 °C overnight under inert atmosphere. The reaction mixture was allowed to cool down to *r.t.,* filtrated through celite and concentrated under reduced pressure. The residue was purified by column chromatography on silica (0-50% MTBE/Heptane) to afford 4-(2-methoxyphenyl)-10,10-dimethyl-9-oxo-1-oxa-4-azaspiro[5.5]undec-7-ene-8-carbonitrile (3% IY) as a dark solid. MS: 327 [M + H]. ¹H NMR (400 MHz, Chloroform-d) δ 8.50 (d, J = 1.3 Hz, 1H), 7.09 - 7.03 (m, 1H), 6.94 (d, J = 4.3 Hz, 2H), 6.89 (d, J = 8.0 Hz, 1H), 4.05 - 3.99 (m, 1H), 3.88 - 3.85 (partially overlapped signals, 4H), 3.24 - 3.18 (m, 2H), 3.13 - 3.07 (m, 1H), 2.75 (d, J = 12.5 Hz, 1H), 2.05 (dd, J = 14.7, J' = 1.6 Hz, 1H), 1.80 (d, J = 14.7 Hz, 1H), 1.35 (s, 3H), 1.19 (s, 3H).

**Table 21: The following example was prepared using a similar procedure as described above for example 803.**

| **Ex** | **Structure** | **IUPAC Name** | **Exact Mass [M+H]⁺** |
|---|---|---|---|
| 804 | 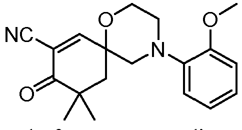 Made from Intermediate 8 | 4-(2-methoxyphenyl)-10,10-dimethyl-9-oxo-1-oxa-4-azaspiro[5.5]undec-7-ene-8-carbonitrile | 327 |
| 805 | 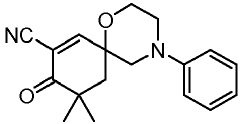 Made from Intermediate 8 | 10,10-dimethyl-9-oxo-4-phenyl-1-oxa-4-azaspiro[5.5]undec-7-ene-8-carbonitrile | 297 |
| 806 | 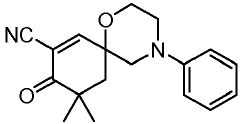 Made from Intermediate 7 | 10,10-dimethyl-9-oxo-4-phenyl-1-oxa-4-azaspiro[5.5]undec-7-ene-8-carbonitrile | 297 |

### Example 807, enantiomer 1: 2-(2-methoxypyridin-3-yl)-9,9-dimethyl-8-oxo-2-azaspiro[4.5]dec-6-ene-7-carbonitrile

### Example 808, enantiomer 2: 2-(2-methoxypyridin-3-yl)-9,9-dimethyl-8-oxo-2-azaspiro[4.5]dec-6-ene-7-carbonitrile

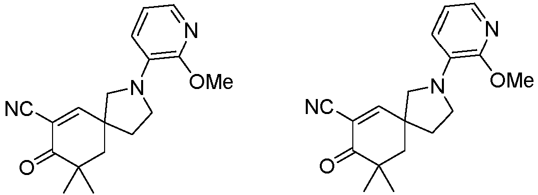

**Step 1:** To a solution of 7,7-dimethyl-6,7-dihydro-4*H*-spiro[benzo[d]isoxazole-5,3'-pyrrolidine] hydrochloride (300 mg, 1.236 mmol) and 3-bromo-2-methoxypyridine (349 mg, 1.854 mmol) and sodium *tert*-butoxide (386 mg, 4.02 mmol) in 2-MeTHF (6 mL) was added XantPhos Pd G4 (71.4 mg, 0.074 mmol) at 25 °C. The resulting mixture was stirred at 60 °C for 16 h. After completion of the reaction, the mixture was concentrated under reduced pressure. The residue was purified by silica gel column chromatography, eluted with 1~10% MeOH in DCM to afford 2-(2-methoxypyridin-3-yl)-9,9-dimethyl-8-oxo-2-azaspiro[4.5]decane-7-carbonitrile (164 mg, 0.523 mmol, 42.3% yield) as a yellow solid. MS ESI calculated for C₁₈H₂₃N₃O₂ [M + H]⁺ 314.18 found 314.20. ¹H-NMR (400 MHz, Chloroform-*d*) δ 7.70 (s, 1H), 7.05-6.70 (m, 2H), 4.11-4.82 (m, 4H), 3.74-3.16 (m, 4H), 2.71-2.52 (m, 1H), 2.29-1.78 (m, 5H), 1.35-1.18 (m, 6H).

**Step 2:** To a solution of phenylselenyl chloride (113 mg, 0.589 mmol) in DCM (1 mL) was added pyridine (46.6 mg, 0.589 mmol) in DCM (1 mL) at 0 °C and the solution was stirred for 20 min. Then a solution of 2-(2-methoxypyridin-3-yl)-9,9-dimethyl-8-oxo-2-azaspiro[4.5]decane-7-carbonitrile (123 mg, 0.392 mmol) in DCM (1 mL) was added and the mixture was stirred for 3 h at 0 °C. Then the hydrogen peroxide (30% in water, 89 mg, 0.785 mmol) was added and the mixture was stirred for 1 h at 0 °C. The reaction mixture was quenched by saturated Na₂S₂O₃ (5 mL) and extracted with ethyl acetate (10 mL × 3). The combined organic layers was washed with brine (20 mL), dried over anhydrous sodium sulfate and filtered. The filtrate was concentrated under reduced pressure. The residue was purified by reverse phase with the following conditions: column C¹⁸ spherical 20-35 um; mobile phase: 0%-50% acetonitrile in water, UV 254 nm. The collected fractions were combined and concentrated under reduced pressure to give 2-(2-methoxypyridin-3-yl)-9,9-dimethyl-8-oxo-2-azaspiro[4.5]dec-6-ene-7-carbonitrile (45 mg, 0.145 mmol, 36.8% yield) as a yellow oil. MS ESI calculated for C₁₈H₂₁N₃O₂ [M + H]⁺ 312.16 found 312.20. ¹H-NMR (400 MHz, Chloroform-*d*) δ 7.72 (d, *J =* 4.8 Hz, 1H), 7.62 (s, 1H), 6.92-6.86 (m, 1H), 6.86-6.79 (m, 1H), 3.99 (s, 3H), 3.76-3.65 (m, 1H), 3.62 (d, *J =* 10.3 Hz, 1H), 3.39-3.31 (m, 1H), 3.27 (d, *J =* 10.3 Hz, 1H), 2.28-2.19 (m, 1H), 2.19-2.02 (m, 2H), 2.02-1.94 (m, 1H), 1.25 (s, 3H), 1.23 (s, 3H).

**Alternative Step 2:** To a solution of 4-(6-(2-hydroxyethyl)pyridin-3-yl)-10,10-dimethyl-9-oxo-1-oxa-4-azaspiro[5.5]undecane-8-carbonitrile (300 mg, 0.874 mmol) in toluene (3 mL) was added DDQ (297 mg, 1.310 mmol). The resulted mixture was stirred for 2 hours at 105 °C. After completion of the reaction, the reaction mixture was concentrated in vacuo. The residue was purified by RP-Combi-Flash with the following conditions: Column: AQ-C¹⁸ OBD Column, 40 g; Mobile Phase A: water (0.1% TFA), Mobile Phase B: MeCN; Flow rate:40 mL/min; Gradient: 5%-60% in 35 min. Detector: UV 254 & 210 nm; The fractions containing desired product were combined and concentrated to afford 4-(6-(2-hydroxyethyl)pyridin-3-yl)-10,10-dimethyl-9-oxo-1-oxa-4-azaspiro[5.5]undec-7-ene-8-carbonitrile (30 mg, 0.088 mmol, 10.06% yield) as a yellow solid. MS ESI calculated for C₁₉H₂₃N₃O₃ [M + H]⁺ 342.17 found 342.30

**Step 3:** The mixture was resolved by Prep-Chiral-HPLC with the following conditions: Column: CHIRAL ART Cellulose-SB, 2 × 25 cm, 5 µm; Mobile Phase A: Hexanes, Mobile Phase B: IPA; Flow rate: 20 mL/min; Gradient: 30% B to 30% B in 17.5 min; Wave Length: 220/254 nm; RT1(min): 12.73; RT2(min): 15.64; Sample Solvent: EtOH; Injection Volume: 1.5 mL; Number Of Runs: 5 2-(2-methoxypyridin-3-yl)-9,9-dimethyl-8-oxo-2-azaspiro[4.5]dec-6-ene-7-carbonitrile (13.9 mg, 0.045 mmol, 30.9% yield) was obtained at 12.73 min as a yellow solid. MS ESI calculated for C₁₈H₂₁N₃O₂ [M + H]⁺ 312.16 found 312.10. ¹H-NMR (300 MHz, Chloroform-*d*) δ 7.76 (dd, *J =* 5.0, 1.6 Hz, 1H), 7.64 (s, 1H), 7.00-6.91 (m, 1H), 6.91-6.81 (m, 1H), 4.04 (s, 3H), 3.81-3.68 (m, 1H), 3.65 (d, *J =* 10.4 Hz, 1H), 3.45-3.31 (m, 1H), 3.32 (d, *J =* 10.4 Hz, 1H), 2.35-2.06 (m, 3H), 2.06-1.95 (m, 1H), 1.27 (s, 3H), 1.25 (s, 3H).

2-(2-methoxypyridin-3-yl)-9,9-dimethyl-8-oxo-2-azaspiro[4.5]dec-6-ene-7-carbonitrile (14.1 mg, 0.045 mmol, 31.3% yield) was obtained at 15.64 min as a yellow solid. MS ESI calculated for C₁₈H₂₁N₃O₂ [M + H]⁺ 312.16 found 312.15. ¹H-NMR (300 MHz, Chloroform-*d*) δ 7.75 (dd, *J =* 4.9, 1.7 Hz, 1H), 7.64 (s, 1H), 6.98-6.90 (m, 1H), 6.89-6.81 (m, 1H), 4.02 (s, 3H), 3.80-3.68 (m, 1H), 3.64 (d, *J =* 10.3 Hz, 1H), 3.43-3.33 (m, 1H), 3.30 (d, *J =* 10.3 Hz, 1H), 2.31-2.08 (m, 3H), 2.00 (dd, *J =* 14.6, 1.0 Hz, 1H), 1.27 (s, 3H), 1.25 (s, 3H).

**Table 22: The following examples were prepared using a similar procedure as described above for examples 807 and 808.**

| **Ex** | **Structure** | **IUPAC Name** | **Exact Mass [M+H]⁺** |
|---|---|---|---|
| 809 | 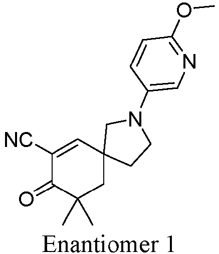 | 2-(6-methoxypyridin-3 -yl)-9,9-dimethyl-8-oxo-2-azaspiro[4.5]dec-6-ene-7-carbonitrile | 312 |
| 810 | 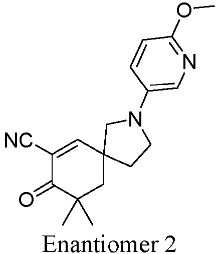 | 2-(6-methoxypyridin-3 -yl)-9,9-dimethyl-8-oxo-2-azaspiro[4.5]dec-6-ene-7-carbonitrile | 312 |
| 811 | 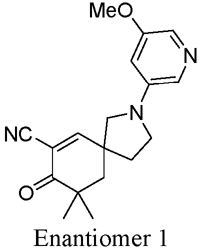 | 2-(5-methoxypyridin-3-yl)-9,9-dimethyl-8-oxo-2-azaspiro[4.5]dec-6-ene-7-carbonitrile | 312 |
| 812 | 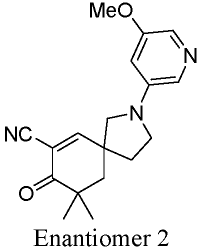 | 2-(5-methoxypyridin-3-yl)-9,9-dimethyl-8-oxo-2-azaspiro[4.5]dec-6-ene-7-carbonitrile | 312 |
| 813 | 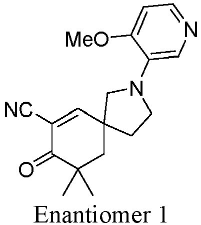 | 2-(4-methoxypyridin-3 -yl)-9,9-dimethyl-8-oxo-2-azaspiro[4.5]dec-6-ene-7-carbonitrile | 312 |
| 814 | 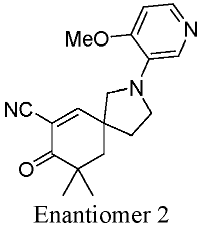 | 2-(4-methoxypyridin-3 -yl)-9,9-dimethyl-8-oxo-2-azaspiro[4.5]dec-6-ene-7-carbonitrile | 312 |
| 815 | 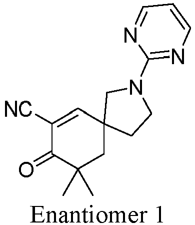 | 9,9-dimethyl-8-oxo-2-(pyrimidin-2-yl)-2-azaspiro[4.5]dec-6-ene-7-carbonitrile | 283 |
| 816 | 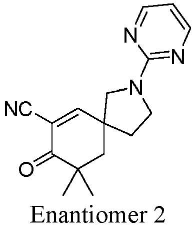 | 9,9-dimethyl-8-oxo-2-(pyrimidin-2-yl)-2-azaspiro[4.5]dec-6-ene-7-carbonitrile | 283 |
| 817 | 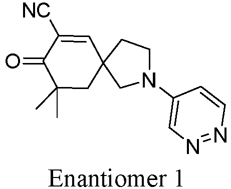 | 9,9-dimethyl-8-oxo-2-(pyridazin-4-yl)-2-azaspiro[4.5]dec-6-ene-7-carbonitrile | 283 |
| 818 | 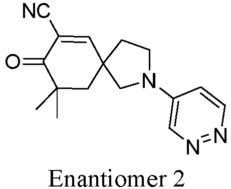 | 9,9-dimethyl-8-oxo-2-(pyridazin-4-yl)-2-azaspiro[4.5]dec-6-ene-7-carbonitrile | 283 |
| 819 | 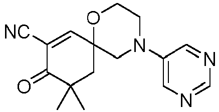 | 10,10-dimethyl-9-oxo-4-(pyrimidin-5-yl)-1-oxa-4-azaspiro[5.5]undec-7-ene-8- | 299 |
| | Enantiomer 1 | carbonitrile | |
| 820 | 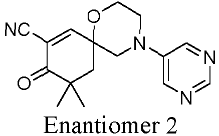 | 10,10-dimethyl-9-oxo-4-(pyrimidin-5-yl)-1-oxa-4-azaspiro[5.5]undec-7-ene-8-carbonitrile | 299 |
| 821 | 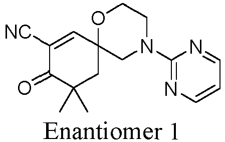 | 10,10-dimethyl-9-oxo-4-(pyrimidin-2-yl)-1-oxa-4-azaspiro[5.5]undec-7-ene-8-carbonitrile | 299 |
| 822 | 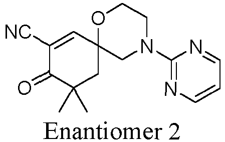 | 10,10-dimethyl-9-oxo-4-(pyrimidin-2-yl)-1-oxa-4-azaspiro[5.5]undec-7-ene-8-carbonitrile | 299 |
| 823 | 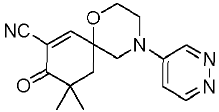 | 10,10-dimethyl-9-oxo-4-(pyridazin-4-yl)-1-oxa-4-azaspiro[5.5]undec-7-ene-8-carbonitrile | 299 |
| 824 | 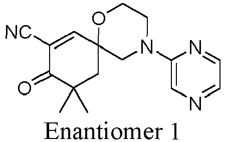 | 10,10-dimethyl-9-oxo-4-(pyrazin-2-yl)-1-oxa-4-azaspiro[5.5]undec-7-ene-8-carbonitrile 2,2,2-trifluoroacetate | 299 |
| 825 | 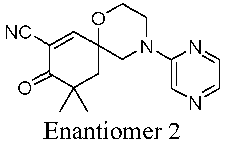 | 10,10-dimethyl-9-oxo-4-(pyrazin-2-yl)-1-oxa-4-azaspiro[5.5]undec-7-ene-8-carbonitrile 2,2,2-trifluoroacetate | 299 |
| 826 | 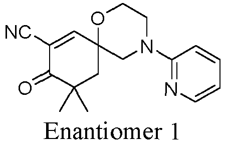 | 10,10-dimethyl-9-oxo-4-(pyridin-2-yl)-1-oxa-4-azaspiro[5.5]undec-7-ene-8-carbonitrile | 298 |
| 827 | 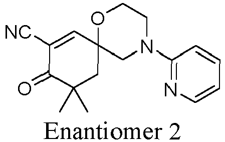 | 10,10-dimethyl-9-oxo-4-(pyridin-2-yl)-1-oxa-4-azaspiro[5.5]undec-7-ene-8-carbonitrile | 298 |
| 828 | 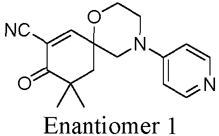 | 10,10-dimethyl-9-oxo-4-(pyridin-4-yl)-1-oxa-4-azaspiro[5.5]undec-7-ene-8-carbonitrile | 298 |
| 829 | 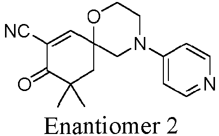 | 10,10-dimethyl-9-oxo-4-(pyridin-4-yl)-1-oxa-4-azaspiro[5.5]undec-7-ene-8-carbonitrile | 298 |
| 830 | 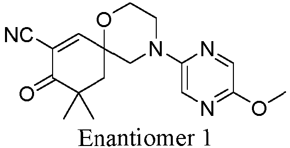 | 4-(5-methoxypyrazin-2-yl)-10,10-dimethyl-9-oxo-1-oxa-4-azaspiro[5.5]undec-7-ene-8-carbonitrile | 329 |
| 831 | 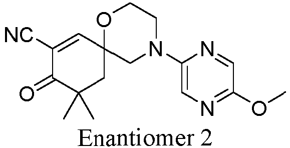 | 4-(5-methoxypyrazin-2-yl)-10,10-dimethyl-9-oxo-1-oxa-4-azaspiro[5.5]undec-7-ene-8-carbonitrile | 329 |
| 832 | 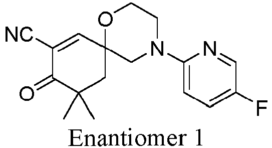 | 4-(5-fluoropyridin-2-yl)-10,10-dimethyl-9-oxo-1-oxa-4-azaspiro[5.5]undec-7-ene-8-carbonitrile | 316 |
| 833 | 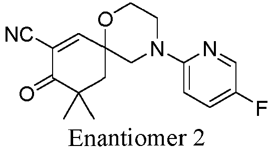 | 4-(5-fluoropyridin-2-yl)-10,10-dimethyl-9-oxo-1-oxa-4-azaspiro[5.5]undec-7-ene-8-carbonitrile | 316 |
| 834 | 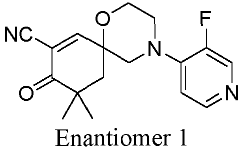 | 4-(3-fluoropyridin-4-yl)-10,10-dimethyl-9-oxo-1-oxa-4-azaspiro[5.5]undec-7-ene-8-carbonitrile | 316 |
| 835 | 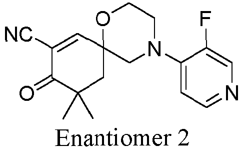 | 4-(3-fluoropyridin-4-yl)-10,10-dimethyl-9-oxo-1-oxa-4-azaspiro[5.5]undec-7-ene-8-carbonitrile | 316 |
| 836 | 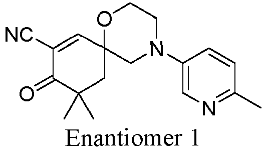 | 10,10-dimethyl-4-(6-methylpyridin-3-yl)-9-oxo-1-oxa-4-azaspiro[5.5]undec-7-ene-8-carbonitrile 2,2,2-trifluoroacetate | 312 |
| 837 | 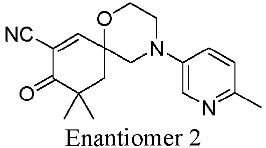 | 10,10-dimethyl-4-(6-methylpyridin-3-yl)-9-oxo-1-oxa-4-azaspiro[5.5]undec-7-ene-8-carbonitrile 2,2,2-trifluoroacetate | 312 |
| 838 | 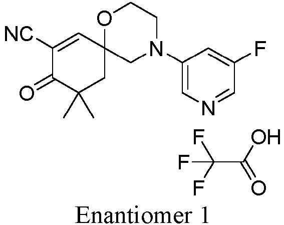 | 4-(5-fluoropyridin-3-yl)-10,10-dimethyl-9-oxo-1-oxa-4-azaspiro[5.5]undec-7-ene-8-carbonitrile 2,2,2-trifluoroacetate | 316 |
| 839 | 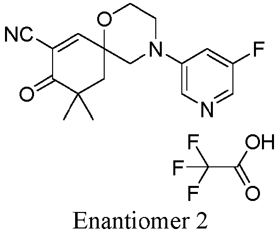 | 4-(5-fluoropyridin-3-yl)-10,10-dimethyl-9-oxo-1-oxa-4-azaspiro[5.5]undec-7-ene-8-carbonitrile 2,2,2-trifluoroacetate | 316 |
| 840 | 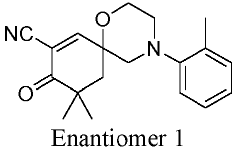 | 10,10-dimethyl-9-oxo-4-(o-tolyl)-1-oxa-4-azaspiro[5.5]undec-7-ene-8-carbonitrile | 311 |
| 841 | 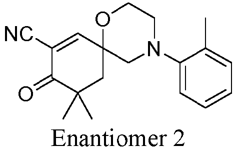 | 10,10-dimethyl-9-oxo-4-(o-tolyl)-1-oxa-4-azaspiro[5.5]undec-7-ene-8-carbonitrile | 311 |
| 842 | 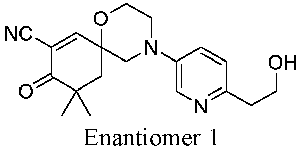 | 4-(6-(2-hydroxyethyl)pyridin-3-yl)-10,10-dimethyl-9-oxo-1-oxa-4-azaspiro[5.5]undec-7-ene-8-carbonitrile | 342 |
| 843 | 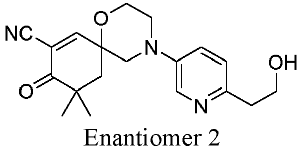 | 4-(6-(2-hydroxyethyl)pyridin-3-yl)-10,10-dimethyl-9-oxo-1-oxa-4-azaspiro[5.5]undec-7-ene-8-carbonitrile | 342 |

### Example 844: 10,10-dimethyl-9-oxo-4-(pyrimidin-4-yl)-1-oxa-4-azaspiro[5.5]undec-7-ene-8-carbonitrile

### Example 845, enantiomer 1: 10,10-dimethyl-9-oxo-4-(pyrimidin-4-yl)-1-oxa-4-azaspiro[5.5]undec-7-ene-8-carbonitrile

### Example 846, enantiomer 2: 10,10-dimethyl-9-oxo-4-(pyrimidin-4-yl)-1-oxa-4-azaspiro[5.5]undec-7-ene-8-carbonitrile

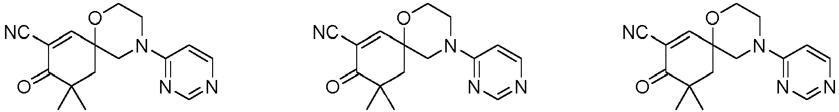

**Step 1:** DIPEA (0.506 mL, 2.90 mmol) was added to a solution of 7,7-dimethyl-6,7-dihydro-4*H-*spiro[benzo[*d*]isoxazole-5,2'-morpholine]hydrochloride (250 mg, 0.966 mmol) and 4-chloropyrimidine hydrochloride (219 mg, 1.449 mmol) in MeCN (8 mL) at 25 °C under the atmosphere N₂. The resulting solution was stirred at 82 °C for 18 h. The reaction solution was concentrated under reduced pressure. The residue was purified by silica gel column chromatography, eluted with MeOH / DCM (0 - 10 %) to afford 7,7-dimethyl-4'-(pyrimidin-4-yl)-6,7-dihydro-4*H*-spiro[benzo [*d*]isoxazole-5,2'-morpholine] (220 mg, 0.732 mmol, 76% yield) as a yellow solid. MS ESI calculated for: C₁₆H₂₀N₄O₂ [M + H]⁺ 301.16 found 301.30. ¹H NMR (300 MHz, Chloroform-*d*) δ 8.63 (s, 1H), 8.26 (d, *J* = 6.3 Hz, 1H), 8.05 (s, 1H), 6.51 (d, *J =* 6.3 Hz, 1H), 3.87-3.70 (m, 4H), 3.66-3.52 (m, 2H), 2.79 (dd, *J =* 16.0, 1.8 Hz, 1H), 2.54 (d, *J =* 16.0 Hz, 1H), 2.26 (dd, *J =* 14.5, 1.8 Hz, 1H), 1.68 (d, *J =* 14.5 Hz, 1H), 1.44 (s, 3H), 1.38 (s, 3H).

**Step 2,** where necessary: A solution of sodium methanolate in MeOH (30%, 1.8 g, 9.99 mmol) in MeOH (1 mL) was added to a mixture of 7,7-dimethyl-4'-(pyrimidin-4-yl)-6,7-dihydro-4*H* - spiro[benzo[*d*]isoxazole-5,2'-morpholine] (200 mg, 0.666 mmol) in Et₂O (2 mL) at 0 °C under the atmosphere of N₂. The solution was stirred at 25 °C for 2 h. The pH value of the solution was adjusted to 6 - 7 with AcOH. The mixture was concentrated under reduced pressure. The residue was purified by silica gel column, eluted with MeOH / DCM (0 - 10 %) to afford 10,10-dimethyl-9-oxo-4-(pyrimidin-4-yl)-1-oxa -4-azaspiro[5.5]undecane-8-carbonitrile (180 mg, 0.599 mmol, 90% yield) as a white solid. MS ESI calculated for: C₁₆H₂₀N₄O₂ [M + H]⁺ 301.16 found 301.30. ¹H NMR (400 MHz, Chloroform-*d*) δ 8.63 (s, 1H), 8.29 (d, *J =* 6.1 Hz, 1H), 6.54 (d, *J* = 6.2 Hz, 1H), 4.22 (dd, *J =* 13.7, 4.7 Hz, 1H), 4.04-3.84 (m, 2H), 3.82-3.74 (m, 1H), 3.71 (d, *J* = 13.3 Hz, 1H), 3.68-3.60 (m, 1H), 3.57 (d, *J =* 13.4 Hz, 1H), 2.69 (dt, *J =* 13.7, 4.3 Hz, 1H), 2.27 (dd, *J =* 14.9, 3.8 Hz, 1H), 2.07 (d, *J =* 13.7 Hz, 1H), 1.60 (d, *J =* 14.9 Hz, 1H), 1.36 (s, 3H), 1.15 (s, 3H).

**Step 3:** A solution of phenylselenyl chloride (230 mg, 1.199 mmol) in DCM (1 mL) was added to a solution of pyridine (0.097 mL, 1.199 mmol) in DCM (1 mL) at 0 °C under the atmosphere of N₂. The solution was stirred at 0 °C for 0.5 h. Then a solution of 10,10-dimethyl-9-oxo-4-(pyrimidin-4-yl)-1-oxa-4-azaspiro[5.5]undecane-8-carbonitrile (180 mg, 0.599 mmol) in DCM (0.5 mL) was added. The reaction solution was stirred at 0 °C for 6 h. The reaction solution was concentrated reduced pressure. The residue was purified by RP - column with the following conditions: C¹⁸ Column, 40 g; Mobile Phase A: Water, Mobile Phase B: MeCN; Flow rate: 30 mL/min; Gradient: 28% B to 35% B in 4.3 min; 254 nm; RT1: 6.8 min. The combined fractions were concentrated under reduced pressure to afford product (80 mg). The product was purified by Prep-HPLC with the following conditions: Column: X Bridge Prep C¹⁸ OBD Column, 19 × 150 mm, 5 µm; Mobile Phase A: Water (10 mmo /L NH₄HCO₃), Mobile Phase B: MeCN; Flow rate: 20 mL/min; Gradient: 30 % B to 50 % B in 5 min; Wave Length: 254 / 210 nm; RT1: 4.5 min. The combined fractions were concentrated under reduced pressure, lyophilized to afford 10,10-dimethyl-9-oxo-4-(pyrimidin-4-yl)-1-oxa-4-azaspiro[5.5]undec-7-ene-8-carbonitrile (20 mg, 0.067 mmol, 11% yield) as a white solid. MS ESI calculated for: C₁₆H₁₈N₄O₂ [M + H]⁺ 299.14 found 299.05. ¹H NMR (400 MHz, Chloroform-d) δ 8.67-8.66 (m, 1H), 8.35 (d, *J* = 6.2 Hz, 1H), 7.60 (d, *J=* 1.8 Hz, 1H), 6.57 (dd, *J=* 6.2, 1.3 Hz, 1H), 4.21 (d, *J* = 13.7 Hz, 1H), 3.98-3.80 (m, 3H), 3.47-3.34 (m, 2H), 2.15 (dd, *J* = 14.7, 1.9 Hz, 1H), 1.95 (d, *J=* 14.7 Hz, 1H), 1.36 (s, 3H), 1.25 (s, 3H).

**Step 4:** The mixture was separated by Prep-CHIRAL-HPLC with the following conditions: Column: CHIRALPAK IE, 2 × 25 cm, 5 µm; Mobile Phase A: Hexanes (0.5% 2M NH₃-MeOH), Mobile Phase B: IPA; Flow rate: 20 mL/min; Gradient: 45% B to 45% B in 15 min; Wave Length: 220/254 nm; RT₁ (min): 9.579; RT₂ (min): 12.098; Sample Solvent: EtOH; Injection Volume: 0.5 mL; Number of Runs: 13.

The fractions of the first peak (RT₁: 9.579 min) were combined and concentrated under reduced pressure, lyophilized to afford 32 mg product which was further purified by Prep-HPLC with the following conditions: Column: X Bridge Prep C¹⁸ OBD Column, 19 × 150 mm, 5 µm; Mobile Phase A: Water (10 mmol/L NH₄HCO₃), Mobile Phase B: MeCN; Flow rate: 20 mL/min; Gradient: 20 % B to 60 % B in 4.5 min; Wave Length: 254/210 nm; RT1(min): 4.35. The fractions were combined, concentrated under reduced pressure, lyophilized to afford 10,10-dimethyl-9-oxo-4-(pyrimidin-4-yl)-1-oxa-4-azaspiro[5.5]undec-7-ene-8-carbonitrile (16.1 mg, 0.054 mmol, 16% yield) as a white solid. MS ESI calculated for: C₁₆H₁₈N₄O₂ [M + H]⁺ 299.14 found 299.05. ¹H NMR (300 MHz, Chloroform-d) δ 8.66 (s, 1H), 8.34 (d, *J* = 6.3 Hz, 1H), 7.60 (t, *J=* 8.7 Hz, 1H), 6.57 (dd, *J=* 6.3, 1.2 Hz, 1H), 4.22 (d, *J=* 13.8 Hz, 1H), 3.97-3.82 (m, 3H), 3.45-3.36 (m, 2H), 2.15 (dd, *J* = 14.7, 1.8 Hz, 1H), 1.95 (d, *J* = 14.7 Hz, 1H), 1.59 (s, 3H), 1.35 (s, 3H). The fractions of the second peak (RT₂: 12.098 min) were combined and concentrated under reduced pressure, lyophilized to afford 30.8 mg product which was further purified by Prep-HPLC with the following conditions: Column: XBridge Prep C¹⁸ OBD Column, 19 × 150 mm, 5 µm; Mobile Phase A: Water (10 mmol/L NH₄HCO₃), Mobile Phase B: MeCN; Flow rate: 20 mL/min; Gradient: 25 % B to 45 % B in 4.5 min, Wave Length: 254/210 nm; RT1(min): 4.35. The fractions were combined concentrated under reduced pressure, lyophilized to afford 10,10-dimethyl-9-oxo-4-(pyrimidin-4-yl)-1-oxa-4-azaspiro[5.5]undec-7-ene-8-carbonitrile (18.3 mg, 0.061 mmol, 18% yield) as a white solid. MS ESI calculated for: C₁₆H₁₈N₄O₂ [M + H]⁺ 299.14 found 299.05. ¹H NMR (400 MHz, Chloroform-d) δ 8.64 (d, *J=* 21.6 Hz, 1H), 8.34 (d, *J=* 6.0 Hz, 1H), 7.60 (d, *J=* 1.6 Hz, 1H), 6.56 (d, *J* = 6.0 Hz, 1H), 4.19 (d, *J* = 13.8 Hz, 1H), 3.96-3.84 (m, 3H), 3.44-3.36 (m, 2H), 2.14 (dd, *J* = 14.8, 1.2 Hz, 1H), 1.95 (d, *J* = 14.8 Hz, 1H),1.58 (s, 3H), 1.33 (s, 3H).

**Table 23: The following examples were prepared using a similar procedure as described above for example 845, and 846.**

| | | | |
|---|---|---|---|
| 847 | 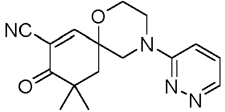 | 10,10-dimethyl-9-oxo-4-(pyridazin-3-yl)-1-oxa-4-azaspiro[5.5]undec-7-ene-8-carbonitrile | 299 |
| 848 | 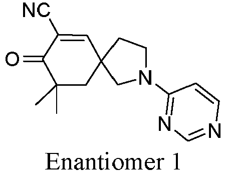 | 9,9-dimethyl-8-oxo-2-(pyrimidin-4-yl)-2-azaspiro[4.5]dec-6-ene-7-carbonitrile | 283 |
| 849 | 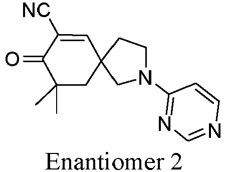 | 9,9-dimethyl-8-oxo-2-(pyrimidin-4-yl)-2-azaspiro[4.5]dec-6-ene-7-carbonitrile | 283 |
| 850 | 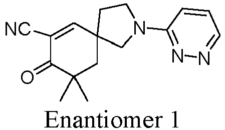 | 9,9-dimethyl-8-oxo-2-(pyridazin-3-yl)-2-azaspiro[4.5]dec-6-ene-7-carbonitrile | 283 |
| 851 | 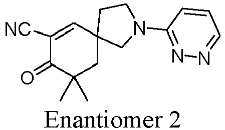 | 9,9-dimethyl-8-oxo-2-(pyridazin-3-yl)-2-azaspiro[4.5]dec-6-ene-7-carbonitrile | 283 |

### Example 852, enantiomer 1: 10,10-dimethyl-4-(1-methyl-1H-pyrazol-4-yl)-9-oxo-1-oxa-4-azaspiro[5.5]undec-7-ene-8-carbonitrile

### Example 853, enantiomer 2: 10,10-dimethyl-4-(1-methyl-1H-pyrazol-4-yl)-9-oxo-1-oxa-4-azaspiro[5.5]undec-7-ene-8-carbonitrile

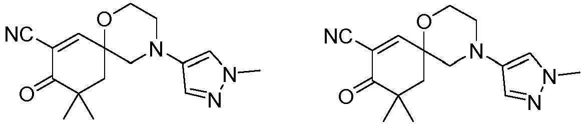

**Step 1:** Argon was bubbled through a mixture of 7,7-dimethyl-6,7-dihydro-4H-spiro[benzo[d]isoxazole-5,2'-morpholine] hydrochloride (100 mg, 0.386 mmol), 4-iodo-1-methyl-1H-pyrazole (161 mg, 0.773 mmol) and sodium tert-butoxide (111 mg, 1.159 mmol) in 1,4-dioxane (1 mL) for 10 min. Then tBuXphos Pd G₃ (61.4 mg, 0.077 mmol) was added. The resulted mixture was stirred at 30 °C for 16 hours. The reaction progress was monitored by LCMS and TLC. After completion of the reaction, the reaction mixture was filtered. The filtrate was concentrated in vacuo. The residue was purified by preparative TLC (developed by ethyl acetate / ethyl acetate 1:1) to afford 10,10-dimethyl-4-(1-methyl-1H-pyrazol-4-yl)-9-oxo-1-oxa-4-azaspiro[5.5]undecane-8-carbonitrile (60 mg, 0.198 mmol, 51.3% yield) as a yellow solid. MS ESI calculated for C₁₆H₂₂N₄O₂ [M + H]⁺ 303.17 found 303.20. ¹H-NMR (400 MHz, Chloroform-*d)* δ 7.17 (d, *J=* 0.9 Hz, 1H), 6.95 (s, 1H), 4.22 (dd, *J* = 13.7, 4.7 Hz, 1H), 3.97 (ddd, *J* = 11.9, 6.2, 4.0 Hz, 1H), 3.88 (ddd, *J=* 11.9, 5.7, 4.0 Hz, 1H), 3.83 (s, 3H), 2.97-2.84 (m, 3H), 2.72 (d, *J* = 11.5 Hz, 1H), 2.65 (d, *J=* 11.5 Hz, 1H), 2.47 (dd, *J* = 15.0, 3.8 Hz, 1H), 1.98 (t, *J* = 13.8 Hz, 1H), 1.51 (d, *J* = 14.9 Hz, 1H), 1.36 (s, 3H), 1.14 (s, 3H).

**Step 2:** To a solution of 10,10-dimethyl-4-(1-methyl-1H-pyrazol-4-yl)-9-oxo-1-oxa-4-azaspiro[5.5]undecane-8-carbonitrile (60 mg, 0.198 mmol) in THF (0.5 mL) was added palladium(II) acetate (44.5 mg, 0.198 mmol). The resulted mixture was stirred for 16 hours at 30 °C. The reaction progress was monitored by LCMS and TLC. After completion of the reaction, the reaction mixture was filtered. The filtrate was concentrated in vacuo. The residue was purified by RP-Combi-Flash with the following conditions: Column: AQ-C¹⁸ 40 g, 25 µm, Mobile Phase A: water (0.1% TFA), Mobile Phase B: MeCN; Flow rate: 40 mL/min; Gradient: 0 - 50% in 30min Detector: UV 254 & 210 nm; The fractions containing desired product were combined and concentrated under reduced pressure to afford 10,10-dimethyl-4-(1-methyl-1H-pyrazol-4-yl)-9-oxo-1-oxa-4-azaspiro[5.5]undec-7-ene-8-carbonitrile (25 mg, 0.083 mmol, 41.9% yield) as yellow solid. MS ESI calculated for C₁₆H₂₀N₄O₂ [M + H]⁺ 301.16 found 301.10.

**Step 3:** The mixture was resolved by Prep-Chiral-HPLC with the following conditions: Column: CHIRAL ART Cellulose-SB, 2 × 25 cm, 5 µm; Mobile Phase A: Hexanes, Mobile Phase B: EtOH; Flow rate: 20 mL/min; Gradient: 30% B to 30% B in 20 min; Wave Length: 220 / 254 nm; RT1(min): 15.62; RT2(min): 18.54; Sample Solvent: EtOH; Injection Volume: 0.3 mL; Number Of Runs: 10,10-dimethyl-4-(1-methyl-1H-pyrazol-4-yl)-9-oxo-1-oxa-4-azaspiro[5.5]undec-7-ene-8-carbonitrile (3 mg, 9.99 µmol, 12.0% yield) was obtained at 15.62 min as a yellow solid. MS ESI calculated for C₁₆H₂₀N₄O₂ [M + H]⁺ 301.16 found 301.10. ¹H-NMR (400 MHz, Chloroform-d) δ 8.13 (d, *J=* 1.8 Hz, 1H), 7.22 (s, 1H), 7.01 (s, 1H), 3.96 (td, *J* = 11.3, 10.4, 3.0 Hz, 1H), 3.91-3.80 (m, 1H), 3.86 (s, 3H), 3.13 (d, *J=* 11.7 Hz, 1H), 2.99-2.71 (m, 3H), 2.09 (dd, *J* = 14.5, 1.9 Hz, 1H), 1.85 (d, *J* = 14.7 Hz, 1H), 1.34 (s, 3H), 1.20 (s, 3H). 10,10-dimethyl-4-(1-methyl-1*H*-pyrazol-4-yl)-9-oxo-1-oxa-4-azaspiro[5.5]undec-7-ene-8-carbonitrile (4 mg, 0.013 mmol, 16.0% yield) was obtained at 18.54 min as a yellow solid. MS ESI calculated for C₁₆H₂₀N₄O₂ [M + H]⁺ 301.16 found 301.10. ¹H-NMR (400 MHz, Chloroform-*d)* δ 8.15 (d, *J=* 1.8 Hz, 1H), 7.25 (s, 1H), 7.04 (s, 1H), 3.99 (td, *J=* 11.3, 10.4, 2.9 Hz, 1H), 3.93-3.81 (m, 1H), 3.89 (s, 3H), 3.15 (d, *J* = 11.7 Hz, 1H), 3.00-2.72 (m, 3H), 2.11 (dd, *J* = 14.7, 1.9 Hz, 1H), 1.87 (d, *J=* 14.7 Hz, 1H), 1.37 (s, 3H), 1.22 (s, 3H).

### Example 854, enantiomer 1: 4-(2-methoxypyrimidin-5-yl)-10,10-dimethyl-9-oxo-1-oxa-4-azaspiro[5.5]undec-7-ene-8-carbonitrile 2,2,2-trifluoroacetate

### Example 855, enantiomer 2: 4-(2-methoxypyrimidin-5-yl)-10,10-dimethyl-9-oxo-1-oxa-4-azaspiro[5.5]undec-7-ene-8-carbonitrile 2,2,2-trifluoroacetate

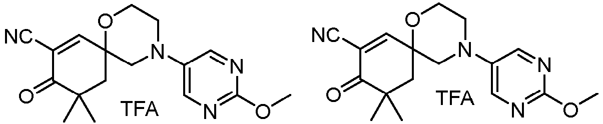

**Step 1:** To a solution of 7,7-dimethyl-6,7-dihydro-4H-spiro[benzo[d]isoxazole-5,2'-morpholine] hydrochloride (100 mg, 0.386 mmol) in THF (0.5 mL) were added 5-iodo-2-methoxypyrimidine (137 mg, 0.580 mmol), NaO*^{t}*Bu (130 mg, 1.353 mmol) and Pd-PEPPSI-IHeptCl (18.82 mg, 0.019 mmol) at room temperature under nitrogen atmosphere. The mixture stirred for 3 h at 40 °C. The reaction progress was monitored by LCMS. After completion of the reaction, the reaction mixture was concentrated in vacuo and the residue was purified by silica gel column chromatography using 20% - 65% gradient of ethyl acetate in petroleum ether as eluent. The fractions containing desired product were combined and concentrated under reduced pressure to afford 4-(2-methoxypyrimidin-5-yl)-10,10-dimethyl-9-oxo-1-oxa-4-azaspiro[5.5]undecane-8-carbonitrile (46 mg, 0.128 mmol, 33.1% yield) as a light yellow oil. MS ESI calculated for C₁₇H₂₂N₄O₃ [M + H]⁺ 331.18 found 331.10.

**Step 2:** PhSeCl (53.3 mg, 0.278 mmol) was added dropwise to a solution of pyridine (0.023 mL, 0.278 mmol) in DCM (1mL). The resulting solution was stirred at 0 °C for 30 min under nitrogen atmosphere. A solution of 4-(2-methoxypyrimidin-5-yl)-10,10-dimethyl-9-oxo-1-oxa-4-azaspiro[5.5]undecane-8-carbonitrile (46 mg, 0.139 mmol) in DCM (1.000 mL) was added into the above mixture and the whole was stirred at 0 °C under nitrogen atmosphere for 16 h. The reaction progress was monitored by LCMS and TLC. After completion of the reaction, the reaction mixture was concentrated in vacuo. The residue was purified by RP-Flash (Column: C¹⁸ 40g Column; Mobile Phase A: water (0.1% TFA), Mobile Phase B: MeCN; Flow rate: 50 mL / min; Gradient: 2% B to 20% B in 5 min, Gradient: 20% B to 50% B in 25 min, Detector: UV 210 nm; RT = 30 min. The fractions containing desired product were combined and concentrated to afford 4-(2-methoxypyrimidin-5-yl)-10,10-dimethyl-9-oxo-1-oxa-4-azaspiro[5.5]undec-7-ene-8-carbonitrile 2,2,2-trifluoroacetate (30 mg, 0.064 mmol, 46.3 % yield) as a light yellow solid. MS ESI calculated for C₁₇H₂₀N₄O₃ [M + H]⁺ 329.16 found 329.15. ¹H NMR (300 MHz, Chloroform-d) δ 8.35 (s, 2H), 8.08 (d, *J* = 1.8 Hz, 1H), 4.06 (s, 3H), 4.04-3.93 (m, 2H), 3.36 (d, *J= 11.7* Hz, 1H), 3.19-2.94 (m, 3H), 2.23 - 2.11 (m, 1H), 1.98-1.84(m, 1H), 1.39 (s, 3H), 1.24 (s, 3H). ¹⁹F NMR (282 MHz, Chloroform-d) δ-75.84 (s, 3F).

**Step 3:** The product was separated by Prep-Chiral-HPLC with the following conditions: Column: Chiral ART Cellulose-SA, 2 × 25 cm, 5 µm; Mobile Phase A: Hexanes, Mobile Phase B: EtOH; Flow rate: 20 mL / min; Gradient: 50% B to 50% B in 21 min; Wave Length: 254 / 220 nm; RT1(min): 8.32; RT2(min): 15.4. The first product containing (RT1: 8.32 min) peak was combined and concentrated under reduced pressure, then freeze-dried to give 4-(2-methoxypyrimidin-5-yl)-10,10-dimethyl-9-oxo-1-oxa-4-azaspiro[5.5]undec-7-ene-8-carbonitrile 2,2,2-trifluoroacetate (7.9 mg, 0.017 mmol, 24.49% yield) as an orange solid. MS ESI calculated for C₁₇H₂₀N₄O₃ [M + H]⁺ 329.16 found 329.10. ¹H NMR (300 MHz, Chloroform-d) δ 8.26 (s, 2H), 8.12 (d, *J* = 1.8 Hz, 1H), 4.09 - 3.90 (m, 5H), 3.38-3.24 (m, 1H), 3.16-2.96 (m, 3H), 2.22-2.07 (m, 1H), 1.96-1.85 (m, 1H), 1.39 (s, 3H), 1.24 (s, 3H). ¹⁹F NMR (282 MHz, Chloroform-d) δ-75.84 (s, 3F). The second product containing (RT2: 15.4 min) peak was combined and concentrated under reduced pressure, then freeze-dried to give 4-(2-methoxypyrimidin-5-yl)-10,10-dimethyl-9-oxo-1-oxa-4-azaspiro[5.5]undec-7-ene-8-carbonitrile 2,2,2-trifluoroacetate (7.7 mg, 0.016 mmol, 23.87% yield) as a light yellow solid. MS ESI calculated for C₁₇H₂₀N₄O₃ [M + H]⁺ 329.16 found 329.15. ¹H NMR (300 MHz, Chloroform-d) δ 8.26 (s, 2H), 8.12 (d, *J* = 1.8 Hz, 1H), 4.07-3.91 (m, 5H), 3.36-3.26 (m, 1H), 3.18-2.87 (m, 3H), 2.21-2.10 (m, 1H), 1.96-1.85 (m, 1H), 1.39 (s, 3H), 1.24 (s, 3H). ¹⁹F NMR (282 MHz, Chloroform-d) δ-75.83 (s, 3F).

### Example 856, enantiomer 2: 10,10-dimethyl-4-(5-methylpyridin-3-yl)-9-oxo-1-oxa-4-azaspiro[5.5]undec-7-ene-8-carbonitrile 2,2,2-trifluoroacetate

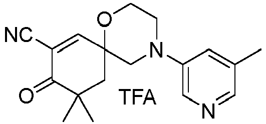

**Step 1:** To a solution of 7,7-dimethyl-6,7-dihydro-4H-spiro[benzo[d]isoxazole-5,2'-morpholine] hydrochloride (200 mg, 0.773 mmol) in toluene (5 mL) were added 3-bromo-5-methylpyridine (199 mg, 1.159 mmol), palladium(II) acetate (17.35 mg, 0.077 mmol), tri-tert-butylphosphonium tetrafluoroborate (44.9 mg, 0.155 mmol) and NaO*^{t}*Bu (353 mg, 3.67 mmol) at room temperature under nitrogen atmosphere. The mixture was stirred for 2 h at 100 °C. The reaction progress was monitored by LCMS and TLC. After completion of the reaction, the reaction mixture was concentrated in vacuo. The residue was purified by silica gel column chromatography using 0% - 2% gradient of DCM in MeOH as eluent. The fractions containing desired product were combined and concentrated under reduced pressure to afford 10,10-dimethyl-4-(5-methylpyridin-3-yl)-9-oxo-1-oxa-4-azaspiro[5.5]undecane-8-carbonitrile (150 mg, 0.455 mmol, 58.8% yield) as a light-yellow solid. MS ESI calculated for C₁₈H₂₃N₃O₂ [M + H]⁺ 314.19 found 314.10.

**Step 2:** Phenylselenenyl chloride (183 mg, 0.957 mmol) was added dropwise to a solution pyridine (0.077 ml, 0.957 mmol) in DCM (4 mL). The resulting solution was stirring at 0 °C for 30 min under the nitrogen atmosphere. A solution of 10,10-dimethyl-4-(5-methylpyridin-3-yl)-9-oxo-1-oxa-4-azaspiro[5.5]undecane-8-carbonitrile (150 mg, 0.479 mmol) in DCM (4 mL) was added into the above mixture and the mixture was stirred under 0 °C for 16 h. The reaction progress was monitored by LCMS and TLC. After completion of the reaction, the reaction mixture was concentrated in vacuo. The residue was purified RP-Flash (Column: C¹⁸ 40g Column; Mobile Phase A: water (0.01% TFA), Mobile Phase B: ACN; Flow rate: 50 mL / min; Gradient: 2% B to 20% B in 20 min, Detector: UV 210 nm; RT = 19 min. The fractions containing desired product were combined and concentrated to afford 10,10-dimethyl-4-(5-methylpyridin-3-yl)-9-oxo-1-oxa-4-azaspiro[5.5]undec-7-ene-8-carbonitrile 2,2,2-trifluoroacetate (80 mg, 0.173 mmol, 36.1% yield) as a light yellow oil. MS ESI calculated for C₁₈H₂₁N₃O₂ [M + H]⁺ 312.17 found 312.20. ¹H NMR (400 MHz, Chloroform-d) δ 8.52 (s, 1H), 8.09 (s, 1H), 7.85 (s, 1H), 7.62 (s, 1H), 4.07 - 3.99 (m, 2H), 3.68 (d, *J=* 11.8 Hz, 1H), 3.58 (d, *J* = 12.4 Hz, 1H), 3.31-3.25 (m, 1H), 3.18 (d, *J* = 12.0 Hz, 1H), 2.55 (s, 3H), 2.16 (d, *J* = 14.4 Hz, 1H), 2.06-1.95 (m, 1H), 1.38 (s, 3H), 1.24 (s, 3H). ¹⁹F NMR (376 MHz, Chloroform-d) δ -75.82 (s, 3F).

**Step 3:** The product was separated by Prep-Chiral-HPLC with the following conditions: mn: CHIRAL ART Cellulose-SC, 2 × 25 cm, 5 µm; Mobile Phase A: Hexanes (0.1% FA), Mobile Phase B: EtOH; Flow rate: 20 mL / min; Gradient: 30% B to 30% B in 27 min; Wave Length: 220 / 254 nm; RT1 (min): 19.09; RT2 (min): 23.17. The second product containing (RT2: 23.17 min) peak was combined and concentrated under reduced pressure, then freeze-dried to give 10,10-dimethyl-4-(5-methylpyridin-3-yl)-9-oxo-1-oxa-4-azaspiro[5.5]undec-7-ene-8-carbonitrile 2,2,2-trifluoroacetate (4.7 mg, 5.37 µmol, 2.86% yield) as a dark yellow solid. MS ESI calculated for C₁₈H₂₁N₃O₂ [M + H]⁺ 312.17 found 312.15. ¹H NMR (300 MHz, Chloroform-d) δ 8.20 (s, 1H), 8.08 (s, 1H), 8.02 (s, 1H), 7.18 (s, 1H), 4.08-3.90 (m, 2H), 3.51 (d, *J=* 12.2 Hz, 1H), 3.34 (d, *J* = 12.6 Hz, 1H), 3.20-2.97 (m, 2H), 2.40 (s, 3H), 2.17 (d, *J* = 14.6 Hz, 1H), 1.93 (d, *J* = 14.7 Hz, 1H), 1.39 (s, 3H), 1.25 (s, 3H). ¹⁹F NMR (282 MHz, Chloroform-d) δ -75.67 (s, 3F).

**Table 24: The following examples were prepared using a similar procedure as described above for example 856.**

| **Ex** | **Structure** | **IUPAC Name** | **Exact Mass [M+H]⁺** |
|---|---|---|---|
| 857 | 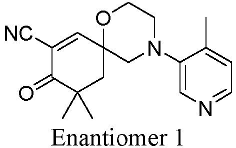 | 10,10-dimethyl-4-(4-methylpyridin-3-yl)-9-oxo-1-oxa-4-azaspiro[5.5]undec-7-ene-8-carbonitrile 2,2,2-trifluoroacetate | 312 |
| 858 | 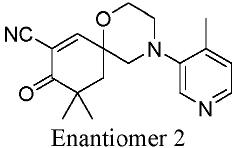 | 10,10-dimethyl-4-(4-methylpyridin-3-yl)-9-oxo-1-oxa-4-azaspiro[5.5]undec-7-ene-8-carbonitrile 2,2,2-trifluoroacetate | 312 |

### Example 859, enantiomer 1: (4-(2,6-dimethylphenyl)-10,10-dimethyl-9-oxo-1-oxa-4-azaspiro[5.5]undec-7-ene-8-carbonitrile 2,2,2-trifluoroacetic acid

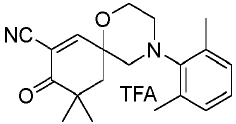

**Step 1:** To a solution of 7,7-dimethyl-6,7-dihydro-4H-spiro[benzo[d]isoxazole-5,2'-morpholine] hydrochloride (800 mg, 3.09 mmol) in THF (15 mL) and water (15 mL) was added K₂CO₃ (427 mg, 3.09 mmol). The solution was stirred at room temperature for 1 h. The reaction mixture was quenched with H₂O (30 mL), extracted with ethyl acetate (3 x 50 mL). The combined organic layers were washed with brine (*sat.*, 2 x 50 mL), dried over sodium sulfate, filtered and concentrated in vacuo to give 7,7-dimethyl-6,7-dihydro-4H-spiro[benzo[d]isoxazole-5,2'-morpholine] (640 mg, 2.88 mmol, 93 % yield) (crude) as a white solid. To a mixture of 7,7-dimethyl-6,7-dihydro-4H-spiro[benzo[d]isoxazole-5,2'-morpholine] (640 mg, 2.88 mmol) in acetonitrile (12 mL) at rt were added (2,6-dimethylphenyl)boronic acid (648 mg, 4.32 mmol), pyridine (1139 mg, 14.40 mmol) and diacetoxycopper (1046 mg, 5.76 mmol), the resulting mixture was stirred for 3 h at 80 °C. The mixture reaction was quenched with H₂O (*aq.* 50 mL), extracted with Ethyl acetate (3 × 80 mL). The combined organic layers were washed with brine (*sat.*, 2 × 80 mL), dried over Sodium sulfate, filtered and the solvent was removed under reduced pressure. The crude was purified by preparative TLC (developed by EA/ PE 1/5) to afford 4'-(2,6-dimethylphenyl)-7,7-dimethyl-6,7-dihydro-4H-spiro[benzo[d]isoxazole-5,2'-morpholine] (20 mg, 0.058 mmol, 2.022 % yield) as a yellow solid. MS ESI calculated for C₂₀H₂₆N₂O₂ [M + H]⁺ 327.21 found 327.15.

**Step 2:** To a solution of 4'-(2,6-dimethylphenyl)-7,7-dimethyl-6,7-dihydro-4*H-*spiro[benzo[d]isoxazole-5,2'-morpholine] (20 mg, 0.061 mmol) in Et₂O (0.6 mL) and MeOH (0.2 mL) was added sodium methanolate (30% in MeOH, 66.2 mg, 1.225 mmol). The solution was stirred at room temperature for 1 h. This organic solution was washed with 5% aqueous HCl solution (3 mL × 3). The combined aqueous layers were extracted with ethyl acetate (5 mL × 2). The combined organic layers were then washed with saturated aqueous NaHCO₃ solution (10 mL) and brine (10 mL), dried with MgSO₄, filtered and concentrated in vacuo to afford 4-(2,6-dimethylphenyl)-10,10-dimethyl-9-oxo-1-oxa-4-azaspiro[5.5]undecane-8-carbonitrile (20.00 mg, 0.061 mmol, 100 % yield) as a crystalline solid which was used without further purification: MS ESI calculated for C₂₀H₂₆N₂O₂ [M + H]⁺ 327.21 found 327.25.

**Step 3:** To a mixture of 4-(2,6-dimethylphenyl)-10,10-dimethyl-9-oxo-1-oxa-4-azaspiro[5.5]undecane-8-carbonitrile (14mg, 0.043 mmol) in THF (0.7 mL) at rt was added diacetoxypalladium (9.63 mg, 0.043 mmol), the resulting mixture was stirred for 3h at rt. The solid was filtered out. The filtrate was concentrated under vacuum. The residue was purified by Prep-HPLC with the following conditions: Column: SunFire Prep C18 OBD Column, 19 x 150 mm, 5µm; Mobile Phase A: Water (0.05% TFA), Mobile Phase B: ACN; Flow rate: 20 mL / min; Gradient: 70% B to 90% B in 5 min, 90% B; Wave Length: 254 nm; RT1(min): 4.98 to afford 4-(2,6-dimethylphenyl)-10,10-dimethyl-9-oxo-1-oxa-4-azaspiro[5.5]undec-7-ene-8-carbonitrile (4-(2,6-dimethylphenyl)-10,10-dimethyl-9-oxo-1-oxa-4-azaspiro[5.5]undec-7-ene-8-carbonitrile 2,2,2-trifluoroacetic acid (2.2 mg, 4.92 µmol, 11.47 % yield) as an light yellow solid. MS ESI calculated for C₂₀H₂₄N₂O₂ [M + H]⁺ 325.19 found 325.30. ¹H NMR (300 MHz, Chloroform - *d*) δ 8.11 (d, *J=* 1.6 Hz, 1H), 6.63 (s, 1H), 6.55 (s, 2H), 3.99-3.78 (m, 2H), 3.44-3.38 (m, 1H), 3.25 (d, *J=* 12.5 Hz, 1H), 3.06-2.86 (m, 2H), 2.30 (s, 6H), 2.19-2.04 (m, 1H), 1.88 (d, *J* = 14.6 Hz, 1H), 1.36 (s, 3H), 1.21 (s, 3H). ¹⁹F NMR (376 MHz, Chloroform - *d)* δ -75.70 (s, 3F).

### Example 860, enantiomer 1: 4-(3-methoxyphenyl)-10,10-dimethyl-9-oxo-1-oxa-4-azaspiro[5.5]undec-7-ene-8-carbonitrile

### Example 861, enantiomer 2: 4-(3-methoxyphenyl)-10,10-dimethyl-9-oxo-1-oxa-4-azaspiro[5.5]undec-7-ene-8-carbonitrile

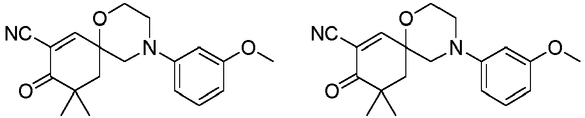

**Step 1:** To a solution of 7,7-dimethyl-6,7-dihydro-4H-spiro[benzo[d]isoxazole-5,2'-morpholine] hydrochloride (150 mg, 0.580 mmol) in 2-MeTHF (1.5 mL) were added 1-iodo-3-methoxybenzene (204 mg, 0.870 mmol) and sodium tert-butoxide (265 mg, 2.75 mmol) and stirred for 10 min degassed with nitrogen. Then the XantPhos Pd G4 (55.8 mg, 0.058 mmol) was added to mixture stirred for 16 h at 60 °C. The reaction solution was concentrated under reduced pressure, the residue was purified by silica gel column chromatography, eluted with 0% - 40% ethyl acetate in petroleum ether to afford 4-(3-methoxyphenyl)-10,10-dimethyl-9-oxo-1-oxa-4-azaspiro[5.5]undecane-8-carbonitrile (110 mg, 0.335 mmol, 57.8% yield) as a yellowish solid. MS ESI calculated for C₁₉H₂₄N₂O₃ [M + H]⁺ 329.19 found 329.20. ¹H NMR (400 MHz, Chloroform-d) δ 7.25 (d, *J* = 8.0 Hz, 1H), 6.67 - 6.56 (m, 3H), 4.27 (dd, *J* = 13.7, 4.8 Hz, 1H), 4.16-4.08 (m, 1H), 4.03-3.95 (m, 1H), 3.83 (s, 3H), 3.32-3.22 (m, 2H), 3.05 (d, *J* = 12.1 Hz, 1H), 3.00-2.90 (m, 2H), 2.67 (d, *J* = 15.3 Hz, 1H), 2.05 (t, *J* = 13.7 Hz, 1H), 1.58 - 1.55 (m, 1H), 1.40 (s, 3H), 1.18 (s, 3H).

**Step 2:** A solution of 4-(3-methoxyphenyl)-10,10-dimethyl-9-oxo-1-oxa-4-azaspiro[5.5]undecane-8-carbonitrile (110 mg, 0.335 mmol) in dry THF (1.5 mL) were added palladium(II) acetate (60.2 mg, 0.268 mmol) at room temperature under the atmosphere of nitrogen, the solution was stirred at room temperature for 2 h. The reaction mixture was concentrated under reduced pressure and purified by reverse phase with the following conditions: column C18 spherical 20-35um; mobile phase: acetonitrile in water 0 - 55%, UV 254 nm. The collected fractions was combined and concentrated under reduced pressure to afford 4-(3-methoxyphenyl)-10,10-dimethyl-9-oxo-1-oxa-4-azaspiro[5.5]undec-7-ene-8-carbonitrile (16 mg, 0.049 mmol, 14.64% yield) as a yellow solid. MS ESI calculated for C₁₉H₂₂N₂O₃ [M + H] ⁺ 327.17 found 327.20.

**Optional Step3:** To a solution of 4-(6-(benzyloxy)pyridin-3-yl)-10,10-dimethyl-9-oxo-1-oxa-4-azaspiro[5.5]undec-7-ene-8-carbonitrile (140 mg, 0.347 mmol) in DCM (2 mL) was added trichloroborane (1 M in DCM, 0.520 ml, 0.520 mmol) at 0 °C under N₂ atmosphere. The resulting mixture was stirred at 40 °C for 3 h. The mixture was quenched with MeOH (0.5 mL). The reaction mixture was concentrated under reduced pressure. The mixture was diluted with ethyl acetate (10 mL). The combined organic layer was washed with aqueous sodium bicarbonate (5 mL × 3), dried over anhydrous sodium sulfate, filtered and the solvent was evaporated under reduced pressure. The crude was purified by column chromatography on silica gel, eluting with 0% ~ 10% MeOH in DCM to give 10,10-dimethyl-9-oxo-4-(6-oxo-1,6-dihydropyridin-3-yl)-1-oxa-4-azaspiro[5.5]undec-7-ene-8-carbonitrile (40 mg, 0.115 mmol, 33.1% yield) as a yellow solid. MS ESI calculated for C₁₇H₁₉N₃O₃ [M + H] ⁺ 314.14 found 314.15.

**Step 4:** The mixture was resolution by Prep-Chiral-HPLC with the following conditions: Column: CHIRALPAK IG, 2 x 25 cm, 5 µm; Mobile Phase A: Hexanees (0.1% FA), Mobile Phase B: EtOH; Flow rate: 20 mL / min; Gradient: 20% B to 20% B in 18.5 min; Wave Length: 254 / 220 nm; RT1 (min): 12.39; RT2 (min): 15.60; Sample Solvent: EtOH; Injection Volume: 0.8 mL; Number Of Runs: 5. 4-(3-methoxyphenyl)-10,10-dimethyl-9-oxo-1-oxa-4-azaspiro[5.5]undec-7-ene-8-carbonitrile (9.0 mg, 0.028 mmol, 31.0 % yield) was obtained at 12.39 min as a light yellow solid. MS ESI calculated for C₁₉H₂₂N₂O₃ [M + H] ⁺ 327.17 found 327.10. ¹H NMR (400 MHz, Chloroform-d) δ 8.09 (d, *J* = 1.8 Hz, 1H), 7.24 (t, *J* = 8.2 Hz, 1H), 6.54 (dd, *J* = 8.2, 2.3 Hz, 2H), 6.46 (t, *J* = 2.3 Hz, 1H), 4.02-3.87 (m, 2H), 3.83 (s, 3H), 3.45 (d, *J* = 12.0 Hz, 1H), 3.28 (d, *J=* 12.7, 1H), 3.09 - 3.00 (m, 1H), 2.96 (d, *J* = 12.7 Hz, 1H), 2.14 (dd, *J* = 14.7, 1.9 Hz, 1H), 1.90 (d, *J* = 14.7 Hz, 1H), 1.38 (s, 3H), 1.24 (s, 3H). 4-(3-methoxyphenyl)-10,10-dimethyl-9-oxo-1-oxa-4-azaspiro[5.5]undec-7-ene-8-carbonitrile (9.1 mg, 0.028 mmol, 31.4% yield) was obtained at 15.60 min as a white solid. MS ESI calculated for C₁₉H₂₂N₂O₃ [M + H] ⁺ 327.17 found 327.15. ¹H NMR (400 MHz, Chloroform-d) δ 8.09 (d, *J* = 1.9 Hz, 1H), 7.24 (t, *J=* 8.1 Hz, 1H), 6.54 (dd, *J* = 8.2, 2.4 Hz, 2H), 6.46 (t, *J* = 2.4 Hz, 1H), 4.01-3.86 (m, 2H), 3.83 (s, 3H), 3.45 (d, *J* = 12.1 Hz, 1H), 3.27 (d, *J* = 12.7 Hz, 1H), 3.08 - 3.00 (m, 1H), 2.96 (d, *J=* 12.7 Hz, 1H), 2.14 (dd, *J* = 14.8, 1.9 Hz, 1H), 1.89 (d, *J* = 14.7 Hz, 1H), 1.38 (s, 3H), 1.24 (s, 3H).

**Table 25: The following examples were prepared using a similar procedure as described above for example 860 and 861.**

| **Ex** | **Structure** | **IUPAC Name** | **Exact Mass [M+H]⁺** |
|---|---|---|---|
| 862 | 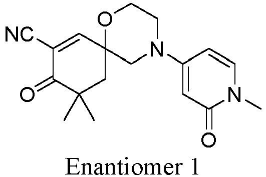 | 10,10-dimethyl-4-(1-methyl-2-oxo-1,2-dihydropyridin-4-yl)-9-oxo-1-oxa-4-azaspiro[5.5]undec-7-ene-8-carbonitrile | 328 |
| 863 | 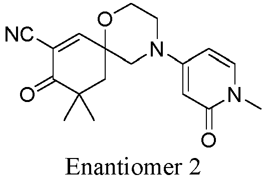 | 10,10-dimethyl-4-(1-methyl-2-oxo-1,2-dihydropyridin-4-yl)-9-oxo-1-oxa-4-azaspiro[5.5]undec-7-ene-8-carbonitrile | 328 |
| 864 | 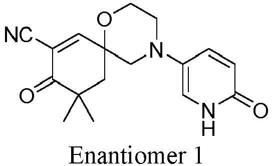 | 10,10-dimethyl-9-oxo-4-(6-oxo-1,6-dihydropyridin-3-yl)-1-oxa-4-azaspiro[5.5]undec-7-ene-8-carbonitrile | 314 |
| 865 | 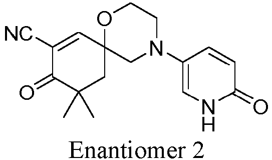 | 10,10-dimethyl-9-oxo-4-(6-oxo-1,6-dihydropyridin-3-yl)-1-oxa-4-azaspiro[5.5]undec-7-ene-8-carbonitrile | 314 |
| 866 | 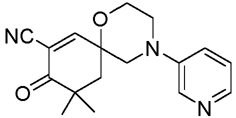 | 10,10-dimethyl-9-oxo-4-(pyridin-3-yl)-1-oxa-4-azaspiro[5.5]undec-7-ene-8-carbonitrile | 298 |
| | 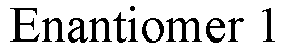 | | |
| 867 | 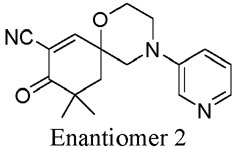 | 10,10-dimethyl-9-oxo-4-(pyridin-3-yl)-1-oxa-4-azaspiro[5.5]undec-7-ene-8-carbonitrile | 298 |
| 868 | 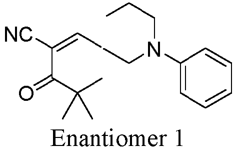 | 10,10-dimethyl-9-oxo-2-phenyl-2-azaspiro[5.5]undec-7-ene-8-carbonitrile | 295 |
| 869 | 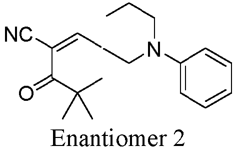 | 10,10-dimethyl-9-oxo-2-phenyl-2-azaspiro[5.5]undec-7-ene-8-carbonitrile | 295 |
| 870 | 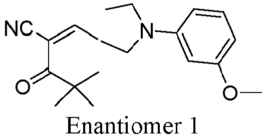 | 2-(3-methoxyphenyl)-9,9-dimethyl-8-oxo-2-azaspiro[4.5]dec-6-ene-7-carbonitrile | 311 |
| 871 | 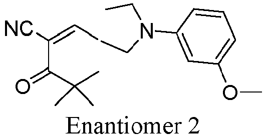 | 2-(3-methoxyphenyl)-9,9-dimethyl-8-oxo-2-azaspiro[4.5]dec-6-ene-7-carbonitrile | 311 |
| 872 | 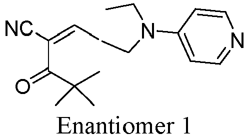 | 9,9-dimethyl-8-oxo-2-(pyridin-4-yl)-2-azaspiro[4.5]dec-6-ene-7-carbonitrile | 282 |
| 873 | 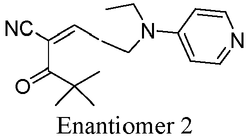 | 9,9-dimethyl-8-oxo-2-(pyridin-4-yl)-2-azaspiro[4.5]dec-6-ene-7-carbonitrile | 282 |
| 874 | 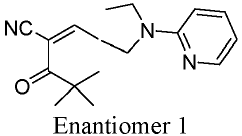 | 9,9-dimethyl-8-oxo-2-(pyridin-2-yl)-2-azaspiro[4.5]dec-6-ene-7-carbonitrile | 282 |
| 875 | 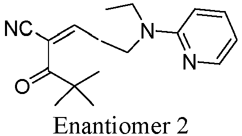 | 9,9-dimethyl-8-oxo-2-(pyridin-2-yl)-2-azaspiro[4.5]dec-6-ene-7-carbonitrile | 282 |
| 876 | 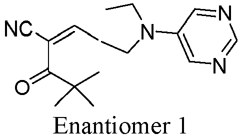 | 9,9-dimethyl-8-oxo-2-(pyrimidin-5-yl)-2-azaspiro[4.5]dec-6-ene-7-carbonitrile | 283 |
| 877 | 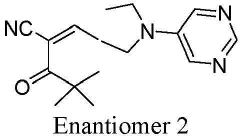 | 9,9-dimethyl-8-oxo-2-(pyrimidin-5-yl)-2-azaspiro[4.5]dec-6-ene-7-carbonitrile | 283 |
| 878 | 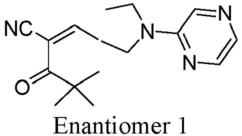 | 9,9-dimethyl-8-oxo-2-(pyrazin-2-yl)-2-azaspiro[4.5]dec-6-ene-7-carbonitrile | 283 |
| 879 | 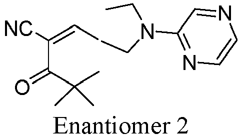 | 9,9-dimethyl-8-oxo-2-(pyrazin-2-yl)-2-azaspiro[4.5]dec-6-ene-7-carbonitrile | 283 |
| 880 | 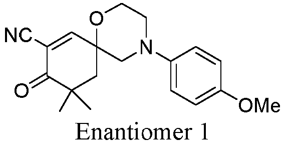 | 4-(4-methoxyphenyl)-10,10-dimethyl-9-oxo-1-oxa-4-azaspiro[5.5]undec-7-ene-8-carbonitrile | 327 |
| 881 | 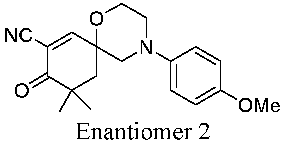 | 4-(4-methoxyphenyl)-10,10-dimethyl-9-oxo-1-oxa-4-azaspiro[5.5]undec-7-ene-8-carbonitrile | 327 |

### Example 882 - enantiomer 1: 1',5,5-trimethyl-2',4-dioxospiro[cyclohexane-1,3'-indolin]-2-ene-3-carbonitrile

### Example 883 - enantiomer 2: 1',5,5-trimethyl-2',4-dioxospiro[cyclohexane-1,3'-indolin]-2-ene-3-carbonitrile

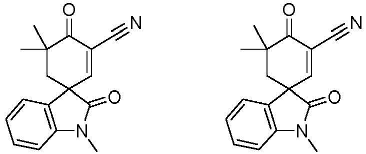

**Step 1:** To a solution of spiro[cyclohexane-1,3'-indoline]-2',4-dione **(0.50 g, 2.3 mmol)** in tBuOH (12 mL) was added potassium tert-butoxide (0.91 g, 8.1 mmol) at room temperature. The mixture was stirred at room temperature for 1 h. Methyl iodide (0.45 mL, 7.2 mmol) was added to the mixture, and the reaction was stirred overnight at room temperature. The reaction was quenched with water (25 mL) and the mixture was extracted with EtOAc (3 x 20 mL). The combined organic layers were washed with brine (30 mL), dried over anhydrous sodium sulfate, filtered, and concentrated under reduced pressure. The residue was purified by column chromatography on silica (0-30% EtOAc/Hexanes) to afford 1',3,3-trimethylspiro[cyclohexane-1,3'-indoline]-2',4-dione as a solid. MS: 258 (M + H).

**Step 2:** To a solution of 1',3,3-trimethylspiro[cyclohexane-1,3'-indoline]-2',4-dione (277 mg, 1.08 mmol) in THF (1.2 mL) **at -78** °C was added a solution of LDA **(2 M THF, 1.2 mL, 2.4 mmol)** dropwise. The mixture was stirred at -78 °C for 30 minutes, then a solution of 4-methylbenzenesulfonyl cyanide (195 mg, 1.08 mmol) in THF (2.4 mL) was added dropwise. The mixture was stirred for an additional 45 minutes at -78 °C. The reaction was quenched with 2 M NH₄OH (0.5 mL) and then warmed to room temperature. The mixture was then neutralized with 1 M HCl to a pH of ~7, and extracted with EtOAc (3 x 5 mL). The combined organic layers were dried over anhydrous sodium sulfate, filtered, and concentrated under reduced pressure. The residue was purified by column chromatography on silica (0-30% EtOAc/Hexanes) to afford 1',3,3-trimethyl-2',4-dioxospiro[cyclohexane-1,3'-indoline]-5-carbonitrile as a solid. MS: 283 (M + H).

**Step 3:** To a solution of 1',3,3-trimethyl-2',4-dioxospiro[cyclohexane-1,3'-indoline]-5-carbonitrile (210 mg, 0.744 mmol) in THF (2.5 mL) at room temperature was added palladium(II) acetate **(167 mg, 0.744 mmol).** The mixture was stirred at room temperature for 2 h, then the reaction was quenched with water (2 mL) and extracted with EtOAc (3 x 5 mL). The combined organic layers were dried over anhydrous sodium sulfate, filtered, and concentrated under reduced pressure. The residue was purified by column chromatography on silica (0-40% EtOAc/Hexanes) and then repurified by mass triggered reverse phase HPLC (ACN/water with 0.1% TFA modifier) to afford 1',5,5-trimethyl-2',4-dioxospiro[cyclohexane-1,3'-indolin]-2-ene-3-carbonitrile. The racemic mixture was purified by chiral SFC (OJ-H column, 10%/90% MeOH/CO₂ with 0.1% NH₄OH modifier) to afford two products as solids: 1',5,5-trimethyl-2',4-dioxospiro[cyclohexane-1,3'-indolin]-2-ene-3-carbonitrile (Example 81 - enantiomer 1): MS: 281 (M + H). ¹H NMR (499 MHz, DMSO-*d*₆) δ 7.65 (d, *J* = 1.1 Hz, 1H), 7.45 (d, *J=* 6.8 Hz, 1H), 7.43 - 7.36 (m, 1H), 7.16 - 7.04 (m, 2H), 3.18 (s, 3H), 2.37 (d, *J* = 14.8 Hz, 1H), 2.21 (dd, *J=* 14.8, 1.1 Hz, 1H), 1.39 (s, 3H), 1.21 (s, 3H). 1',5,5-trimethyl-2',4-dioxospiro[cyclohexane-1,3'-indolin]-2-ene-3-carbonitrile (Example 82 - enantiomer 2): as a solid. MS: 281 (M + H). ¹H NMR (499 MHz, DMSO-*d*₆) δ 7.65 (d, *J* = 1.1 Hz, 1H), 7.45 (d, *J* = 6*.*8 Hz, 1H), 7.42 - 7.36 (m, 1H), 7.16 - 7.04 (m, 2H), 3.18 (s, 3H), 2.37 (d, *J=* 14.8 Hz, 1H), 2.21 (dd, *J=* 14.8, 1.1 Hz, 1H), 1.39 (s, 3H), 1.21 (s, 3H).

**Alternative Step 3:** To a solution of pyridine (30 µL, 0.38 mmol) in DCM (0.3 mL) at 0 °C was added a solution of phenylselenyl chloride (72 mg, 0.38 mmol) in DCM (0.5 mL). The reaction mixture was stirred for 20 minutes at 0 °C before the addition of a solution of 1,9,9-trimethyl-2,8-dioxo-1-azaspiro[4.5]decane-7-carbonitrile (44 mg, 0.188 mmol) in DCM (0.9 mL). The reaction mixture was stirred for 1 h at 0 °C, and was subsequently washed with 1 M aqueous HCl (0.8 mL) then water (1.6 mL). The phases were separated, and the organic phase was treated with hydrogen peroxide (30% in water, 384 µL, 3.8 mmol) at 0 °C. The reaction mixture was stirred vigorously for 15 mins, then the phases were separated and the organic phase was concentrated under reduced pressure. The residue was purified by column chromatography on silica (0-100% Et₂O/Hexanes) to afford 1,9,9-trimethyl-2,8-dioxo-1-azaspiro[4.5]dec-6-ene-7-carbonitrile. The racemic mixture was purified by chiral SFC (AS-H column, 20%/80% MeOH/CO₂) to afford two products as solids:
1,9,9-trimethyl-2,8-dioxo-1-azaspiro[4.5]dec-6-ene-7-carbonitrile (enantiomer 1): MS: 233 (M +1). ¹H NMR (499 MHz, DMSO-*d*₆) δ 7.88 (d, *J=* 1.9 Hz, 1H), 2.62 (s, 3H), 2.41 - 2.34 (m, 3H), 2.28 - 2.20 (m, 2H), 1.88 (dd, *J* = 13.9, 2.0 Hz, 1H), 1.21 (s, 3H), 1.13 (s, 3H).

**Table 26: The following examples were prepared using a similar procedure as described above for examples 882 and 883. For the oxidation conditions, 1 = Palladium(II) acetate and 2 = PhSeCl, Pyridine, H₂O₂**

| **Ex** | **Structure** | **Compound Name** | **Oxidation Conditions** | **Exact Mass [M+H]⁺** |
|---|---|---|---|---|
| 884 | 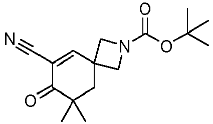 | tert-butyl 6-cyano-8,8-dimethyl-7-oxo-2-azaspiro[3.5]non-5-ene-2-carboxylate | 1 | 313 |
| 885 | 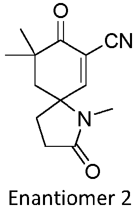 | 1,9,9-trimethyl-2,8-dioxo-1-azaspiro[4.5]dec-6-ene-7-carbonitrile | 2 | 233 |
| 886 | 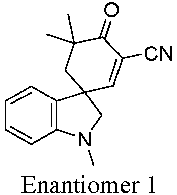 | 1',5,5-trimethyl-4-oxospiro[cyclohexane-1,3'-indolin]-2-ene-3-carbonitrile | 1 | 267 |
| 887 | 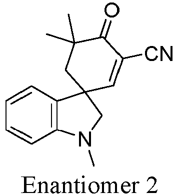 | 1',5,5-trimethyl-4-oxospiro[cyclohexane-1,3'-indolin]-2-ene-3-carbonitrile | 1 | 267 |

### Example 888: 4-(cyclopropylmethyl)-10,10-dimethyl-9-oxo-1-oxa-4-azaspiro[5.5]undec-7-ene-8-carbonitrile (enantiomer 2)

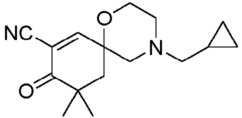

To a solution of (chloromethyl)cyclopropane (16.20 µl, 0.175 mmol) in DMF (584 µl) sodium iodide (31.5 mg, 0.210 mmol) was added and the resulting mixture was stirred at 80 °C for 2h. Then, the reaction mixture was allowed to cool down to *r.t.* and 8-cyano-10,10-dimethyl-9-oxo-1-oxa-4-azaspiro[5.5]undec-7-en-4-ium chloride, **Intermediate 8** (30 mg, 0.117 mmols) and N,N-diisopropylethylamine (50.9 µl, 0.292 mmol) were added. The resulting reaction mixture was stirred at *r.t.* for 72h. After LCMS control, additional aliquots of sodium iodide (31.5 mg, 0.210 mmol), N,N-diisopropylethylamine (50.9 µl, 0.292 mmol) and (chloromethyl)cyclopropane (16.20 µl, 0.175 mmol) were added and the reaction mixture was stirred at 45 °C overnight. The reaction mixture was allowed to cool down to *r.t.,* diluted with H₂O (5 mL) and the organic phase was extracted with DCM (3 x 10 mL). The combined organic layers were washed with H₂O (2 x 10 mL), dried over anhydrous MgSO4, filtrated and concentrated under reduced pressure. The residue was purified by column chromatography on silica (0-5% MeOH/DCM) to afford 4-(cyclopropylmethyl)-10,10-dimethyl-9-oxo-1-oxa-4-azaspiro[5.5]undec-7-ene-8-carbonitrile (21% IY) as an orange solid. MS = 275 [M + H]. ¹H NMR (400 MHz, Chloroform-d) δ 8.12 (s, 1H), 3.86 - 3.81 (m, 1H), 3.75 - 3.70 (m, 1H), 2.82 (d, J = 11.0 Hz, 1H), 2.66 (d, J = 11.8 Hz, 1H), 2.35 - 2.23 (partially overlapped signals, 5H), 2.01 (d, J = 14.7 Hz, 1H), 1.80 (d, J = 14.7 Hz, 1H), 1.31 (s, 3H), 1.17 (s, 3H), 0.53 (m, 2H), 0.11 (m, 2H).

**Table 27: The following examples were prepared using a similar procedure as described above for examples 888.**

| **Ex** | **Structure** | **Compound Name** | **Exact Mass [M+H]⁺** |
|---|---|---|---|
| 889 | 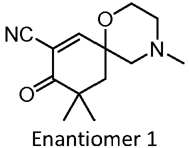 | 4,10,10-trimethyl-9-oxo-1-oxa-4-azaspiro[5.5]undec-7-ene-8-carbonitrile | 235 |
| 890 | 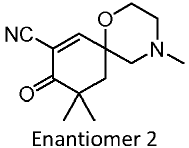 | 4,10,10-trimethyl-9-oxo-1-oxa-4-azaspiro[5.5]undec-7-ene-8-carbonitrile | 235 |
| 891 | 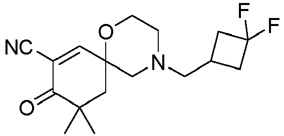 From Intermediate 8 | 4-((3,3-difluorocyclobutyl)methyl)-10,10-dimethyl-9-oxo-1-oxa-4-azaspiro[5.5]undec-7-ene-8-carbonitrile | 325 |
| 892 | 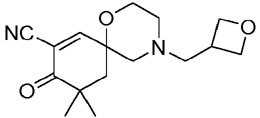 From Intermediate 8 | 10,10-dimethyl-4-(oxetan-3-ylmethyl)-9-oxo-1-oxa-4-azaspiro[5.5]undec-7-ene-8-carbonitrile | 291 |

### Example 893: 8,8-dimethyl-7-oxo-2-(pyridin-3-ylmethyl)-2-azaspiro[3.5]non-5-ene-6-carbonitrile

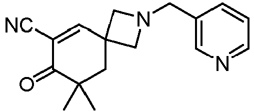

To a solution of 8,8-dimethyl-7-oxo-2-azaspiro[3.5]non-5-ene-6-carbonitrile, HCl (15 mg, 0.066 mmol) in DCM (0.22 mL) was added DIEA (40 µL, 0.23 mmol) followed by 3-(bromomethyl)pyridine (13 mg, 0.079 mmol). The reaction mixture was stirred at room temperature overnight, then quenched with water (1.0 mL). The mixture was extracted with DCM (3 x 1 mL) and the combined organic layers were dried over anhydrous sodium sulfate, filtered, and concentrated under reduced pressure. The residue was purified by mass triggered reverse phase HPLC (ACN/water with 0.1% TFA modifier) to afford 8,8-dimethyl-7-oxo-2-(pyridin-3-ylmethyl)-2-azaspiro[3.5]non-5-ene-6-carbonitrile as the TFA salt. MS: 282 (M + H). ¹H NMR (499 MHz, CDCl₃) δ 8.58 - 8.46 (m, 2H), 8.00 (s, 1H), 7.66 (d, *J* = 7.8 Hz, 1H), 7.30 (dd, *J=* 7.7, 4.9 Hz, 1H), 3.66 (s, 2H), 3.49 (d, *J* = 7.3 Hz, 2H), 3.21 (d, *J* = 6.9 Hz, 2H), 2.06 (s, 2H), 1.10 (s, 6H).

**Table 28: The following examples were prepared using a similar procedure as described above for example 893.**

| **Ex** | **Structure** | **Compound Name** | **Exact Mass [M+H]⁺** |
|---|---|---|---|
| 894 | 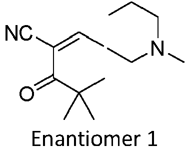 | 2,10,10-trimethyl-9-oxo-2-azaspiro[5.5]undec-7-ene-8-carbonitrile | 233 |
| 895 | 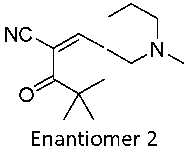 | 2,10,10-trimethyl-9-oxo-2-azaspiro[5.5]undec-7-ene-8-carbonitrile | 233 |
| 896 | 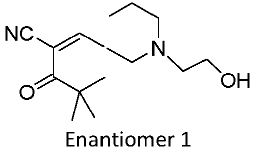 | 2-(2-hydroxyethyl)-10,10-dimethyl-9-oxo-2-azaspiro[5.5]undec-7-ene-8-carbonitrile | 263 |
| 897 | 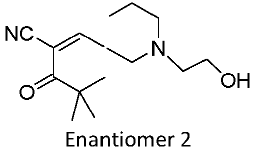 | 2-(2-hydroxyethyl)-10,10-dimethyl-9-oxo-2-azaspiro[5.5]undec-7-ene-8-carbonitrile | 263 |
| 898 | 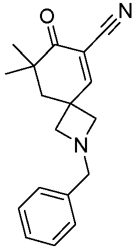 | 2-benzyl-8,8-dimethyl-7-oxo-2-azaspiro[3.5]non-5-ene-6-carbonitrile | 281 |
| 899 | 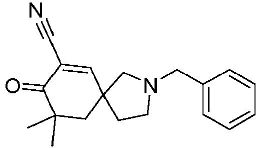 Made from Intermediate 5 | 2-benzyl-9,9-dimethyl-8-oxo-2-azaspiro[4.5]dec-6-ene-7-carbonitrile | 295 |
| 900 | 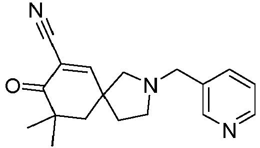 Made from Intermediate 5 | 9,9-dimethyl-8-oxo-2-[(pyridin-3-yl)methyl]-2-azaspiro[4.5]dec-6-ene-7-carbonitrile | 296 |
| 901 | 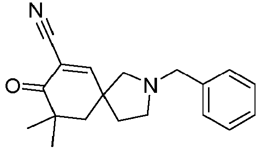 Made from Intermediate 6 | 2-benzyl-9,9-dimethyl-8-oxo-2-azaspiro[4.5]dec-6-ene-7-carbonitrile | 295 |
| 902 | 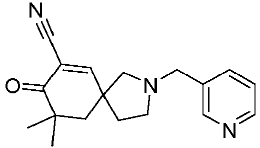 Made from Intermediate 6 | 9,9-dimethyl-8-oxo-2-[(pyridin-3-yl)methyl]-2-azaspiro[4.5]dec-6-ene-7-carbonitrile | 296 |

### Example 903: 8,8-dimethyl-7-oxo-2-((3-(trifluoromethyl)pyrazin-2-yl)methyl)-2-azaspiro[3.5]non-5-ene-6-carbonitrile

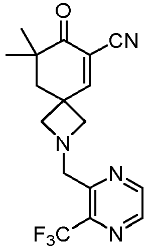

To 8,8-dimethyl-7-oxo-2-azaspiro[3.5]non-5-ene-6-carbonitrile, HCl (22.7 mg, 0.1 mmol) and 2-(bromomethyl)-3-(trifluoromethyl)pyrazine (36.2 mg, 0.150 mmol) dissolved in DMF (500 µL) was added Et₃N (28 µL, 0.2 mmol). The reaction was stirred at room temperature for 3 hours. The reaction was filtered, and purified by mass triggered reverse phase HPLC (ACN/H₂O with 0.1% TFA modifier) to afford 8,8-dimethyl-7-oxo-2-((3-(trifluoromethyl)pyrazin-2-yl)methyl)-2-azaspiro[3.5]non-5-ene-6-carbonitrile, TFA as a solid. MS: 351 (M + H). ¹H NMR (499 MHz, DMSO-*d*₆) δ 9.00 (d, *J* = 1.9 Hz, 1H), 8.91 (s, 1H), 8.30 (s, 1H), 5.09 (m, 2H), 4.56 (m, 2H), 4.42 (m, 2H), 2.34 (m, 2H).

**Table 29: The following examples were prepared using a similar procedure as described above for example 903.**

| **Ex** | **Structure** | **Compound Name** | **Exact Mass [M+H]⁺** |
|---|---|---|---|
| 904 | 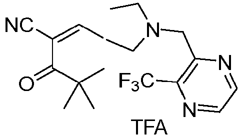 | 9,9-dimethyl-8-oxo-2-((3-(trifluoromethyl)pyrazin-2-yl)methyl)-2-azaspiro[4.5]dec-6-ene-7-carbonitrile compound with 2,2,2-trifluoroacetaldehyde | 365 |
| 905 | 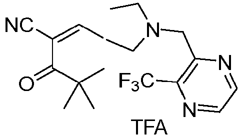 | 9,9-dimethyl-8-oxo-2-((3-(trifluoromethyl)pyrazin-2-yl)methyl)-2-azaspiro[4.5]dec-6-ene-7-carbonitrile compound with 2,2,2-trifluoroacetaldehyde | 365 |

### Example 906: 4-benzyl-10,10-dimethyl-9-oxo-1-oxa-4-azaspiro[5.5]undec-7-ene-8-carbonitrile

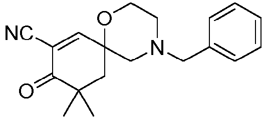

To a suspension of 8-cyano-10,10-dimethyl-9-oxo-1-oxa-4-azaspiro[5.5]undec-7-en-4-ium chloride (80 mg, 0.312 mmols) and potassium carbonate (129 mg, 0.935 mmols) in acetonitrile (328 uL), benzyl bromide (40.7 uL, 58.6 mmols) was added and the resulting reaction mixture was stirred at *r.t.* overnight. The reaction mixture was diluted with H₂O (5 mL) and the organic phase was extracted with EtOAc (3 x 10 mL). The combined organic layers were dried over anhydrous MgSO4, filtered and concentrated under reduced pressure. The residue was purified by column chromatography on silica (0-5% MeOH/DCM) to afford 4-benzyl-10,10-dimethyl-9-oxo-1-oxa-4-azaspiro[5.5]undec-7-ene-8-carbonitrile (52% IY) as a redsolid. MS: 311 [M + H⁺]. VT ¹H NMR (400 MHz, DMSO-d₆) δ 8.19 (broad s,1H), 7.36 - 7.28 (m, 5H), 3.84 (broad signal, 1H), 3.71 (broad signal, 1H), 3.52 (broad signal, 2H), 2.77 - 2.52 (broad signal, 2H), 2.43 - 2.10 (broad signal, 2H), 2.01 - 1.92 (m, 2H), 1.20 (s, 3H), 1.05 (s, 3H).

**Table 30: The following examples were prepared using a similar procedure as described above for example 906.**

| **Ex** | **Structure** | **Compound Name** | **Exact Mass [M+H]⁺** |
|---|---|---|---|
| 907 | 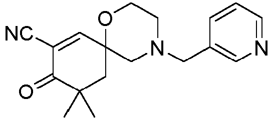 Made from Intermediate 4 | 10,10-dimethyl-9-oxo-4-(pyridin-3-ylmethyl)-1-oxa-4-azaspiro[5.5]undec-7-ene-8-carbonitrile | 312 |
| 908 | 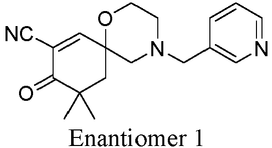 | 10,10-dimethyl-9-oxo-4-(pyridin-3-ylmethyl)-1-oxa-4-azaspiro[5.5]undec-7-ene-8-carbonitrile | 312 |
| 909 | 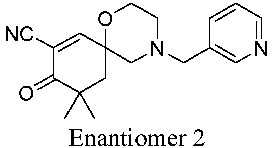 | 10,10-dimethyl-9-oxo-4-(pyridin-3-ylmethyl)-1-oxa-4-azaspiro[5.5]undec-7-ene-8-carbonitrile | 312 |
| 910 | 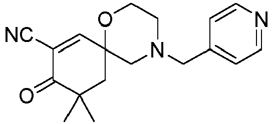 Made from Intermediate 8 | 10,10-dimethyl-9-oxo-4-(pyridin-4-ylmethyl)-1-oxa-4-azaspiro[5.5]undec-7-ene-8-carbonitrile | 312 |
| 911 | 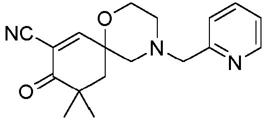 Made from Intermediate 8 | 10,10-dimethyl-9-oxo-4-(pyridin-2-ylmethyl)-1-oxa-4-azaspiro[5.5]undec-7-ene-8-carbonitrile | 312 |
| 912 | 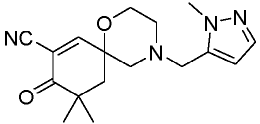 Made from Intermediate 8 | 10,10-dimethyl-4-((1-methyl-1H-pyrazol-5-yl)methyl)-9-oxo-1-oxa-4-azaspiro[5.5]undec-7-ene-8-carbonitrile | 315 |

### Example 913: 8,8-dimethyl-7-oxo-2-(phenylsulfonyl)-2-azaspiro[3.5]non-5-ene-6-carbonitrile

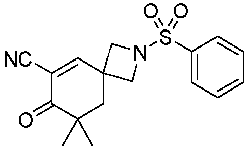

To a solution of 8,8-dimethyl-7-oxo-2-azaspiro[3.5]non-5-ene-6-carbonitrile, HCl (15 mg, 0.066 mmol) in DCM (0.22 mL) at room temperature was added triethylamine (28 µL, 0.20 mmol) followed by benzenesulfonyl chloride (10 µL, 0.079 mmol). The reaction mixture was stirred at room temperature overnight, then quenched with water (1.0 mL). The mixture was then extracted with DCM (3 x 1 mL), and the combined organic layers were dried over anhydrous sodium sulfate, filtered, and concentrated under reduced pressure. The residue was purified by mass triggered reverse phase HPLC (ACN/water with 0.1% TFA modifier) to afford 8,8-dimethyl-7-oxo-2-(phenylsulfonyl)-2-azaspiro[3.5]non-5-ene-6-carbonitrile as a solid. MS: 331 (M + H). ¹H NMR (499 MHz, DMSO-*d*₆) δ 7.95 (s, 1H), 7.91 - 7.86 (m, 2H), 7.83 (t, *J* = 7.4 Hz, 1H), 7.74 (t, *J=* 7.7 Hz, 2H), 4.03 (d, *J* = 8.6 Hz, 2H), 3.70 (d, *J* = 8.6 Hz, 2H), 1.71 (s, 2H), 0.91 (s, 6H).

**Table 31: The following examples were prepared using a similar procedure as described above for example 913.**

| **Ex** | **Structure** | **Compound Name** | **Exact Mass [M+H]⁺** |
|---|---|---|---|
| 914 | 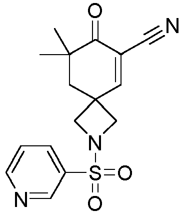 | 8,8-dimethyl-7-oxo-2-[(pyridin-3-yl)sulfonyl]-2-azaspiro[3.5]non-5-ene-6-carbonitrile | 332 |
| 915 | 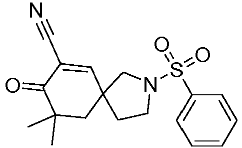 Made from Intermediate $ | 9,9-dimethyl-8-oxo-2-(phenylsulfonyl)-2-azaspiro[4.5]dec-6-ene-7-carbonitrile | 345 |
| 916 | 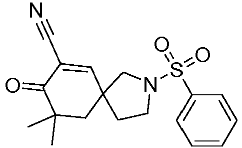 Made from Intermediate $ | 9,9-dimethyl-8-oxo-2-(phenylsulfonyl)-2-azaspiro[4.5]dec-6-ene-7-carbonitrile | 345 |
| 917 | 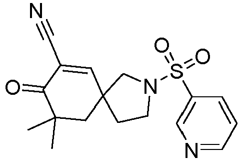 Made from Intermediate $ | 9,9-dimethyl-8-oxo-2-[(pyridin-3-yl)sulfonyl]-2-azaspiro[4.5]dec-6-ene-7-carbonitrile | 346 |
| 918 | 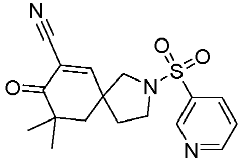 Made from Intermediate $ | 9,9-dimethyl-8-oxo-2-[(pyridin-3-yl)sulfonyl]-2-azaspiro[4.5]dec-6-ene-7-carbonitrile | 346 |
| 919 | 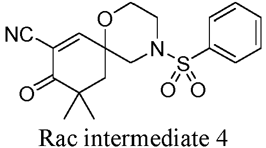 | 10,10-dimethyl-9-oxo-4-(phenylsulfonyl)-1-oxa-4-azaspiro[5.5]undec-7-ene-8- | 361 |
| 920 | 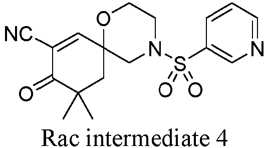 | 10,10-dimethyl-9-oxo-4-(pyridin-3-ylsulfonyl)-1-oxa-4-azaspiro[5.5]undec-7-ene-8-carbonitrile | 362 |
| 921 | 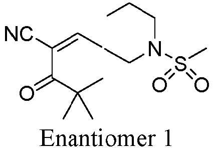 | 10,10-dimethyl-2-(methylsulfonyl)-9-oxo-2-azaspiro[5.5]undec-7-ene-8-carbonitrile | 297 |
| 922 | 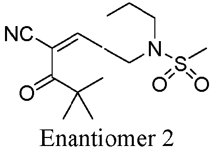 | 10,10-dimethyl-2-(methylsulfonyl)-9-oxo-2-azaspiro[5.5]undec-7-ene-8-carbonitrile | 297 |
| 923 * | 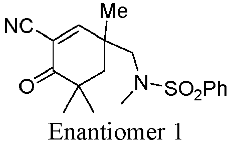 | N-((3-cyano-1,5,5-trimethyl-4-oxocyclohex-2-en-1-yl)methyl)-N-methylbenzenesulfonamide | 369 |
| 924 * | 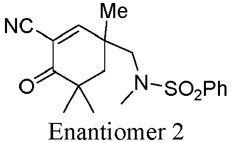 | N-((3-cyano-1,5,5-trimethyl-4-oxocyclohex-2-en-1-yl)methyl)-N-methylbenzenesulfonamide | 369 |

| | | | |
|---|---|---|---|
| * reference compound | | | |

### Example 925: 3-acetyl-9-oxo-3-azaspiro[5.5]undec-7-ene-8-carbonitrile

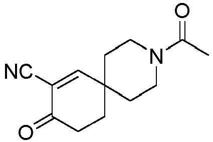

**Step 1:** LDA (2.0 M in THF/heptane/ethylbenzene, 524 µL, 1.05 mmol) was combined with THF (1 mL) and cooled to -78 °C under a N₂ atmosphere. To this mixture was added a solution of tert-butyl 9-oxo-3-azaspiro[5.5]undecane-3-carboxylate (250 mg, 0.935 mmol) in THF (1 mL). The resulting mixture was stirred at -78 °C for 20 minutes, then taken up in a syringe and added to a mixture of tosyl cyanide (339 mg, 1.87 mmol) in THF (1 mL) at -78 °C under an atmosphere of N₂. The reaction mixture was stirred at -78 °C for 45 minutes. The mixture was quenched with NaOH (1 M in water, 2 mL) and extracted with EtOAc (2x). The combined organic layers were washed with HCl (1 N in water) and brine, the dried over anhydrous sodium sulfate, filtered, and concentrated under reduced pressure. The residue was purified by column chromatography on silica (0-70% EtOAc/Hexanes) to give tert-butyl 8-cyano-9-oxo-3-azaspiro[5.5]undecane-3-carboxylate as an oil. MS: 315 (M + Na).

**Step 2:** *Tert-butyl* 8-cyano-9-oxo-3-azaspiro[5.5]undecane-3-carboxylate (128 mg, 0.438 mmol) was dissolved in toluene (4.4 mL). DDQ (132 mg, 0.582 mmol) was added, and the mixture was heated at 110 °C for 2 h. The mixture was cooled to room temperature, filtered, and the filtrate was concentrated under reduced pressure. The residue was purified by column chromatography on silica (0-100% EtOAc/Hexanes) to give tert-butyl 8-cyano-9-oxo-3-azaspiro[5.5]undec-7-ene-3-carboxylate as an oil. MS: 313 (M + Na). ¹H NMR (600 MHz, Chloroform-d) δ 7.55 (s, 1H), 3.65 - 3.60 (m, 2H), 3.48 - 3.42 (m, 2H), 2.63 - 2.59 (m, 2H), 1.78 - 1.72 (m, 2H), 1.69 - 1.62 (m, 4H), 1.50 (s, 9H).

**Step 3:** To a solution of *tert-*butyl 8-cyano-9-oxo-3-azaspiro[5.5]undec-7-ene-3-carboxylate (30 mg, 0.103 mmol) dissolved in DCM (1 mL) was added HCl (4.0 M in dioxane, 258 µL, 1.03 mmol). The mixture was stirred at room temperature overnight, and then concentrated under reduced pressure to give 9-oxo-3-azaspiro[5.5]undec-7-ene-8-carbonitrile, HCl as a solid, which was used without further purification. MS: 191 (M + H).

**Step 4:** To a mixture of 9-oxo-3-azaspiro[5.5]undec-7-ene-8-carbonitrile, HCl (23.4 mg, 0.123 mmol) in DCM (1.2 mL) was added TEA (69 µL, 0.49 mmol). Acetyl chloride (13 µL, 0.18 mmol) was added, and the mixture was stirred at room temperature overnight. The mixture was concentrated under reduced pressure and then purified by column chromatography on silica 0-10% MeOH/DCM) to give 3-acetyl-9-oxo-3-azaspiro[5.5]undec-7-ene-8-carbonitrile as a solid. MS: 233 (M + H). ¹H NMR (600 MHz, DMSO-*d*₆) δ 8.07 (s, 1H), 3.67 - 3.62 (m, 1H), 3.59 - 3.53 (m, 1H), 3.48 - 3.39 (m, 2H), 2.57 - 2.53 (m, 2H), 2.01 (s, 3H), 2.00 - 1.94 (m, 2H), 1.77 - 1.71 (m, 1H), 1.69 - 1.58 (m, 2H), 1.55 - 1.49 (m, 1H).

### Example 926: 10,10-dimethyl-9-oxo-3-phenyl-3-azaspiro[5.5]undec-7-ene-8-carbonitrile

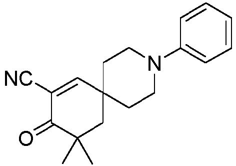

**Step 1:** Potassium tert-butoxide (1.08 g, 9.59 mmol) was added slowly to tert-butyl 9-oxo-3-azaspiro[5.5]undecane-3-carboxylate (1.03 g, 3.84 mmol) in tBuOH (19 mL) under a nitrogen atmosphere. The mixture was stirred at room temperature for 1 h, and methyl iodide (0.504 mL, 8.06 mmol) was added dropwise. The mixture was stirred at room temperature overnight. Water (10 mL) was added and the aqueous phase was extracted with EtOAc (3x). The combined organic layers were washed with brine, dried over anhydrous sodium sulfate, filtered, and concentrated under reduced pressure. The residue was purified by column chromatography on silica (0-50% Et₂O/Hexanes) to give tert-butyl 8,8-dimethyl-9-oxo-3-azaspiro[5.5]undecane-3-carboxylate as a liquid. MS: 240 (M + H - tBu).

**Step 2:** To tert-butyl 8,8-dimethyl-9-oxo-3-azaspiro[5.5]undecane-3-carboxylate (780 mg, 2.64 mmol) dissolved in DCM (20 mL) was added HCl (4.0 M in dioxane, 6.60 mL, 26.4 mmol). The mixture was stirred at room temperature for 4 h. The mixture was concentrated under reduced pressure to give 8,8-dimethyl-3-azaspiro[5.5]undecan-9-one, HCl as a solid which was used without further purification. MS: 196 (M + H).

**Step 3:** To vial containing 8,8-dimethyl-3-azaspiro[5.5]undecan-9-one, HCl (200 mg, 0.863 mmol), RuPhos Pd G2 (67.0 mg, 0.086 mmol), and 2-dicyclohexylphosphino-2',6'-diisopropoxybiphenyl (40.3 mg, 0.086 mmol) under a nitrogen atmosphere was added dioxane (4.3 mL). Bromobenzene (109 µL, 1.04 mmol) and sodium tert-butoxide (863 µL, 1.73 mmol) were added and the mixture was stirred at 60°C overnight. The reaction was cooled to room temperature, diluted with DCM, filtered through Celite^{®}, and concentrated under reduced pressure. The residue was purified by column chromatography on silica (0-70% Et₂O/Hexanes) to give 8,8-dimethyl-3-phenyl-3-azaspiro[5.5]undecan-9-one as a solid. MS: 272 (M + H).

**Step 4:** To a degassed vial containing LDA (2.0 M in THF/heptane/ethylbenzene, 304 µL, 0.608 mmol) at -78 °C was added a solution of 8,8-dimethyl-3-phenyl-3-azaspiro[5.5]undecan-9-one (83 mg, 0.30 mmol) in THF (400 µL) dropwise. The mixture was stirred at -78 °C for 30 minutes. Added a solution of 4-methylbenzenesulfonyl cyanide (55 mg, 0.30 mmol) in THF (800 µL) dropwise, and the reaction was stirred for an additional 30 minutes at -78 °C. The reaction was quenched with 2M NH₄OH (0.6 mL) and then warmed to room temperature. The mixture was neutralized with 1M HCl to a pH of ~7, and the mixture was extracted three times with EtOAc. The combined organic layers were dried over anhydrous sodium sulfate, filtered, and concentrated under reduced pressure. The residue was purified by column chromatography on silica (0-70% Et₂O/Hexanes and then 0-10% MeOH/DCM) to give 10,10-dimethyl-9-oxo-3-phenyl-3-azaspiro[5.5]undecane-8-carbonitrile as an oil. MS: 297 (M + H).

**Step 5:** To a solution of 10,10-dimethyl-9-oxo-3-phenyl-3-azaspiro[5.5]undecane-8-carbonitrile (83 mg, 0.28 mmol) in THF (1 mL) at room temperature was added palladium(II) acetate (63 mg, 0.28 mmol). The mixture was stirred at room temperature overnight. The mixture was concentrated under reduced pressure, diluted with DCM (5 mL), and then 1:1 water:brine was added. The organic layer was collected and concentrated under reduced pressure. The residue was purified by column chromatography on silica (0-100% Et₂O/Hexanes) to give 10,10-dimethyl-9-oxo-3-phenyl-3-azaspiro[5.5]undec-7-ene-8-carbonitrile as a solid. MS: 295 (M + H). ¹H NMR (600 MHz, DMSO-*d*₆) δ 8.07 (s, 1H), 7.25 - 7.19 (m, 2H), 6.97 (d, *J=* 8.0 Hz, 2H), 6.77 (t, *J= 7.2* Hz, 1H), 3.42 - 3.37 (m, 2H), 3.18 - 3.12 (m, 2H), 1.95 (s, 2H), 1.91 - 1.85 (m, 2H), 1.77 - 1.71 (m, 2H), 1.15 (s, 6H).

### Example 927: 2-benzyl-9,9-dimethyl-3,8-dioxo-2-azaspiro[4.5]dec-6-ene-7-carbonitrile

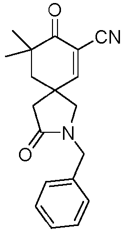

**Step 1:** To a solution of tert-butyl 7,7-dimethyl-6,7-dihydro-4H-spiro[benzo[d]isoxazole-5,3'-pyrrolidine]-1'-carboxylate (800 mg, 2.61 mmol) in 2-MeTHF (8.7 mL) was added HCl (4M in dioxane, 6.5 mL, 26.1 mmol) and the resulting solution stirred at RT for 20 h. The reaction mixture was quenched by slow addition of aqueous saturated sodium bicarbonate and extracted with DCM (3x). The combined organic layers were filtered through a phase separator and concentrated under reduced pressure to yield 7,7-dimethyl-6,7-dihydro-4H-spiro[benzo[d]isoxazole-5,3'-pyrrolidine] which was used in the next step without further purification. MS: 207 (M + H- CO₂*t*Bu).

**Step 2:** To a solution of 7,7-dimethyl-6,7-dihydro-4H-spiro[benzo[d]isoxazole-5,3'-pyrrolidine] (92 mg, 0.45 mmol) and benzaldehyde (47 mg, 0.45 mmol) in MeOH:AcOH (10:1, 0.7 mL) was added 2-picoline borane (49 mg, 0.45mmol). The reaction was stirred at RT for 4 h and quenched with a solution of 10% aqueous HCl. The resulting solution was stirred for a further 30 min and then made alkaline (pH= 9) by addition of aqueous saturated sodium bicarbonate. EtOAc (10 mL) was added and the phases separated. The aqueous layer was extracted with EtOAc (2x), the combined organic layers were extracted with brine (1x) and filtered through a phase separator prior concentration under reduced pressure. The residue was purified by column chromatography on silica (0-80% MTBE/Heptanes) to afford 1'-benzyl-7,7-dimethyl-6,7-dihydro-4H-spiro[benzo[d]isoxazole-5,3'-pyrrolidine]. MS: 297 (M + H).

**Step 3:** To a solution of 1'-benzyl-7,7-dimethyl-6,7-dihydro-4H-spiro[benzo[d]isoxazole-5,3'-pyrrolidine] (62 mg, 0.21 mmol) and sodium bicarbonate (176 mg, 2.09 mmol) in THF:water (2.5:1, 8.3 mL) was added iodine (398 mg, 1.57 mmol). The reaction was stirred at RT for 5.5 h and then transferred to a 1:1 solution of aqueous saturated sodium bicarbonate and sodium thiosulfate. The resulting solution was extracted with DCM (2x). The combined organic layers were extracted with aqueous saturated sodium bicarbonate (1x), filtered through a phase separator and concentrated under reduced pressure. The resulting regioisomers (1.5:1 mixture) were purified and separated by column chromatography on silica (0-100% MTBE/Heptanes) to afford 1'-benzyl-7,7-dimethyl-6,7-dihydro-4H-spiro[benzo[d]isoxazole-5,3'-pyrrolidin]-5'-one as the major product. MS: 311 (M + H).

**Step 4:** To a solution of 1'-benzyl-7,7-dimethyl-6,7-dihydro-4H-spiro[benzo[d]isoxazole-5,3'-pyrrolidin]-5'-one (29 mg, 0.09 mmol) in MeOH (0.5 mL) was added sodium methoxide (0.5 M solution, 0.56 mL) and the resulting solution was stirred at RT for 16 h. The reaction mixture was quenched by slow addition of aqueous 1M HCl and extracted with EtOAc (3x). The combined organic layers were filtered through a phase separator and concentrated under reduced pressure to yield 2-benzyl-9,9-dimethyl-3,8-dioxo-2-azaspiro[4.5]decane-7-carbonitrile which was used in the next step without further purification. MS: 311 (M + H).

**Step 5:** A solution of pyridine (6 µL, 0.07 mmol) and phenylselenyl chloride (14 mg, 0.07 mmol) in DCM (150 µL) pre-stirred at 0°C for 20 min was added dropwise to a solution of 2-benzyl-9,9-dimethyl-3,8-dioxo-2-azaspiro[4.5]decane-7-carbonitrile (11 mg, 0.03 mmol) in DCM (150 µL). The resulting solution was stirred at 0°C for a further 1 hour, and hydrogen peroxide (18 µL, 0.18 mmol) was added. The reaction was stirred for 1 hour and quenched with aqueous 1M HCl. The aqueous layer was extracted with DCM (2x) and filtered through a phase separator prior concentration under reduced pressure. The residue was purified by mass triggered reverse phase HPLC (ACN/H2O with 0.1% TFA modifier) to afford 2-benzyl-9,9-dimethyl-3,8-dioxo-2-azaspiro[4.5]dec-6-ene-7-carbonitrile. MS: 309 (M + H). 1H NMR (400 MHz, CDCl₃) δ 7.40 - 7.34 (m, 3H), 7.30 (s, 1H), 7.25 - 7.24 (m, 2H), 4.53 - 4.45 (m, 2H), 3.28 (q, J = 10.6 Hz, 2H), 2.68 (s, 2H), 1.98 - 1.97 (m, 2H), 1.18 (s, 3H), 1.12 (s, 3H).

### Example 928: 7'-fluoro-1',5,5-trimethyl-2',4-dioxospiro[cyclohexane-1,3'-indolin]-2-ene-3-carbonitrile

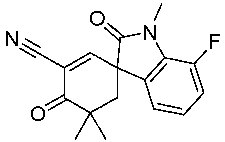

**Step 1:** Potassium tert-butoxide (1 M in THF, 0.662 mL, 0.662 mmol) was added to a solution of 7-fluoroindolin-2-one (2.0 g, 13 mmol) in DMSO (10 mL) at room temperature under an atmosphere of N₂. The mixture was heated to 45 °C, and methyl acrylate (3.53 g, 41.0 mmol) was added. The solution was stirred at 45 °C for 1 h. The reaction was cooled to room temperature and quenched with aq. NH₄Cl (20 mL). The mixture was diluted with EtOAc (400 mL), and the organic phase was washed with brine (3 x 100 mL), dried over anhydrous sodium sulfate, filtered, and concentrated under reduced pressure. The residue was purified by column chromatography on silica (0-45% EtOAc/Pet Ether) to afford trimethyl 3,3',3"-(7-fluoro-2-oxoindoline-1,3,3-triyl)tripropanoate as an oil. MS: 410 (M + H).

**Step 2:** Potassium *tert*-butoxide (1 M in THF, 25 mL, 25 mmol) was added portionwise over 40 minutes to trimethyl 3,3',3"-(7-fluoro-2-oxoindoline-1,3,3-triyl)tripropanoate (5.0 g, 12 mmol) in DMSO (5 mL) at room temperature under an atmosphere of N₂. The reaction mixture was stirred at 60 °C for 2 h. Then water (20 mL) was added, and the mixture was stirred at 80 °C for 4 h.

The reaction mixture was cooled to room temperature and diluted with EtOAc (50 mL). The organic layer was washed with brine (2 x 100 mL), dried over anhydrous sodium sulfate, filtered, and concentrated under reduced pressure. The residue was purified by column chromatography on silica (0-35% EtOAc/Pet Ether) to give 7'-fluorospiro[cyclohexane-1,3'-indoline]-2',4-dione as a solid. ¹H NMR (300 MHz, CDCl₃): δ 8.31 (s, 1H), 7.28 - 7.05 (m, 3H), 3.18 - 3.03 (m, 2H), 2.56 - 2.41 (m, 2H), 2.29 - 2.16 (m, 4H).

**Step 3:** Potassium tert-butoxide (1 M in THF, 7.50 mL, 7.50 mmol) was added to a mixture of 7'-fluorospiro[cyclohexane-1,3'-indoline]-2',4-dione (500 mg, 2.14 mmol) in tBuOH (20 mL) at 0 °C under an atmosphere of N₂. The mixture was stirred at 25 °C for 1 h, and then a solution of iodomethane (882 mg, 6.22 mmol) in tBuOH (1 mL) was added dropwise. The reaction was stirred at room temperature for 2.5 h. The solution was quenched with water (50 mL), diluted with EtOAc (200 mL), and the organic phase was washed with brine (2 x 100 mL), dried over anhydrous sodium sulfate, filtered, and concentrated under reduced pressure. The residue was purified by column chromatography on silica (0-30% EtOAc/Pet Ether) to afford 7'-fluoro-1',3,3-trimethylspiro[cyclohexane-1,3'-indoline]-2',4-dione as a solid. MS: 276 (M + H). ¹H NMR (400 MHz, CDCl₃): δ 7.30 - 7.28 (m, 1H), 7.19 - 7.13 (m, 1H), 7.11 - 7.03 (m, 1H), 2.51 (s, 3H), 2.38 - 2.19 (m, 2H), 2.13 - 1.93 (m, 4H), 1.22 (s, 3H), 1.12 (s, 3H).

**Step 4:** Lithium bis(trimethylsilyl)amide (1 M in THF, 0.686 mL, 0.686 mmol) was added to a solution of 7'-fluoro-1',3,3-trimethylspiro[cyclohexane-1,3'-indoline]-2',4-dione (126 mg, 0.458 mmol) in degassed THF (1.0 mL) at -78 °C, and the solution was stirred at -78 °C for 1 h. A solution of 4-methylbenzenesulfonyl cyanide (100 mg, 0.549 mmol) in degassed THF was added, and the reaction mixture was stirred at -78 °C for 4 h. The reaction was quenched with saturated aq. NH₄Cl (10 mL), diluted with EtOAc (100 mL), and the organic phase was washed with brine (2 x 30 mL), dried over anhydrous sodium sulfate, filtered, and concentrated under reduced pressure. The residue was purified by *prep-TLC* (1:3 EtOAc:Pet Ether and then 1:1 EtOAc:Pet Ether) to afford 7'-fluoro-1',3,3-trimethyl-2',4-dioxospiro[cyclohexane-1,3'-indoline]-5-carbonitrile as a solid. MS: 301 (M + H).

**Step 5:** A solution of phenylselenyl chloride (140 mg, 0.733 mmol) in DCM (0.5 mL) was added to a solution of pyridine (57.9 mg, 0.733 mmol) in DCM (0.5 mL) at 0 °C under an atmosphere of N₂, and the solution was stirred at room temperature for 20 minutes. Then a solution of 7'-fluoro-1',3,3-trimethyl-2',4-dioxospiro[cyclohexane-1,3'-indoline]-5-carbonitrile (110 mg, 0.366 mmol) in DCM (0.5 mL) was added, and the reaction mixture was stirred at 0 °C for 2 h. Hydrogen peroxide (831 mg, 7.33 mmol) was added, and the solution was stirred at 0 °C for 40 minutes. The reaction mixture was quenched with aq. Na₂S₂O₃ (5 mL), diluted with EtOAc (50 mL), and the organic phase was washed with brine (2 x 20 mL), dried over anhydrous sodium sulfate, filtered, and concentrated under reduced pressure. The residue was purified by reverse phase HPLC (60-70% ACN/water with 5 mM formic acid buffer) to afford 7'-fluoro-1',5,5-trimethyl-2',4-dioxospiro[cyclohex[2]ene-1,3'-indoline]-3-carbonitrile as a solid. MS: 299 (M + H). ¹H NMR (400 MHz, CDCl₃): δ 7.15 - 7.04 (m, 2H), 6.99 - 6.94 (m, 2H), 3.47 (d, *J=* 2.0 Hz, 3H), 2.38 (dd, *J =* 1.2, 15.2 Hz, 1H), 2.23 (dd, *J =* 1.2, 15.2 Hz, 1H), 1.47 (s, 3H), 1.31 (s, 3H). **Step 6,** where required: 1'-(3,4-dimethylbenzyl)-5,5-dimethyl-2',4-dioxospiro[cyclohexane-1,3'-indolin]-2-ene-3-carbonitrile (150 mg, 0.390 mmol) was dissolved in TFA (3 mL, 0.390 mmol). Then the reaction solution was stirred at 90 °C for 3 h. The reaction progress was monitored by LCMS and TLC. After completion the reaction mixture was concentrated under vacuum and co-evaporated with toluene (three times, 3 mL for each time) to removed excessed TFA. The residue was purified by RP-Combi-Flash with the following conditions: Column: AQ 18 gel column (80 g), 20-35 µm; Mobile Phase A: 5 mM aq. NH₄HCO₃; Mobile Phase B: MeCN (Gradient: 0% B hold 5 min, up to 49.2% B within 31 min, 49.2% B hold 1.2 min; up to 95% B within 1 min, 95% B hold 5 min); Flow rate: 60 mL/min; Detector: UV 254 & 210 nm; RT: 31.3 min. The product-containing fractions were collected and roto-evaporated *in vacuo* to give 5,5-dimethyl-2',4-dioxospiro[cyclohexane-1,3'-indolin]-2-ene-3-carbonitrile (49.2 mg, 0.185 mmol, 47.4% yield) as a white solid. MS: *m*/*z* = 265.05 [M - H]⁻. ¹H-NMR (400 MHz, Chloroform-d) δ 7.89 (s, 1H), 7.38 - 7.30 (m, 1H), 7.24 - 7.12 (m, 2H), 7.09 (s, 1H), 7.02 - 6.87 (m, 1H), 2.49 - 2.45 (m, 1H), 2.30 - 2.26 (m, 1H), 1.62 (s, 3H), 1.36 (s, 3H).

**Table 32: The following examples were prepared using a similar procedure as described above in example 928.**

| **Ex** | **Structure** | **Compound Name** | **Exact Mass [M+H]⁺** |
|---|---|---|---|
| 929 | 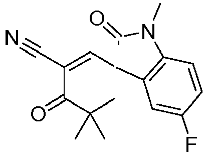 | 5'-fluoro-1',5,5-trimethyl-2',4-dioxospiro[cyclohexane-1,3'-indolin]-2-ene-3-carbonitrile | 299 |
| 930 | 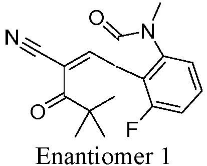 | 4'-fluoro-1',5,5-trimethyl-2',4-dioxospiro[cyclohexane-1,3'-indolin]-2-ene-3-carbonitrile | 299 |
| 931 | 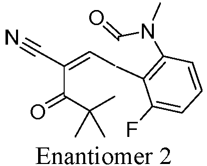 | 4'-fluoro-1',5,5-trimethyl-2',4-dioxospiro[cyclohexane-1,3'-indolin]-2-ene-3-carbonitrile | 299 |
| 932 | 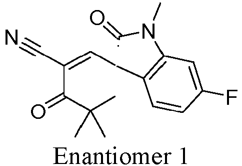 | 6'-fluoro-1',5,5-trimethyl-2',4-dioxospiro[cyclohexane-1,3'-indolin]-2-ene-3-carbonitrile | 299 |
| 933 | 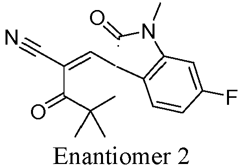 | 6'-fluoro-1',5,5-trimethyl-2',4-dioxospiro[cyclohexane-1,3'-indolin]-2-ene-3-carbonitrile | 299 |
| 934 | 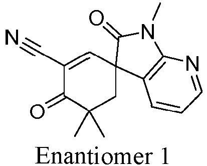 | 1',5,5-trimethyl-2',4-dioxo-1',2'-dihydrospiro[cyclohexane-1,3'-pyrrolo[2,3-b]pyridin]-2-ene-3-carbonitrile | 282 |
| 935 | 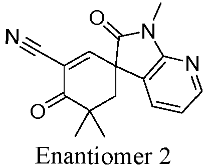 | 1',5,5-trimethyl-2',4-dioxo-1',2'-dihydrospiro[cyclohexane-1,3'-pyrrolo[2,3-b]pyridin]-2-ene-3-carbonitrile | 282 |
| 936 | 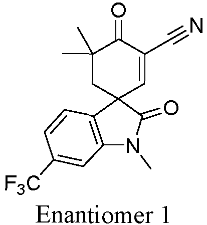 | 1',5,5-trimethyl-2',4-dioxo-6'-(trifluoromethyl)spiro[cyclohex ane-1,3'-indolin]-2-ene-3-carbonitrile | 349 |
| 937 | 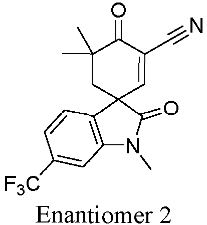 | 1',5,5-trimethyl-2',4-dioxo-6'-(trifluoromethyl)spiro[cyclohex ane-1,3'-indolin]-2-ene-3-carbonitrile | 349 |
| 938 | 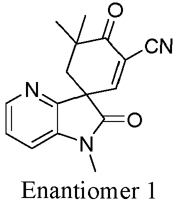 | 1',5,5-trimethyl-2',4-dioxo-1',2'-dihydrospiro[cyclohexane-1,3'-pyrrolo[3,2-b]pyridin]-2-ene-3-carbonitrile | 282 |
| 939 | 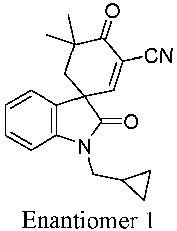 | 1'-(cyclopropylmethyl)-5,5-dimethyl-2',4-dioxospiro[cyclohexane-1,3'-indolin]-2-ene-3-carbonitrile | 298 |
| 940 | 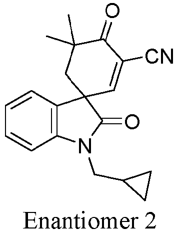 | 1'-(cyclopropylmethyl)-5,5-dimethyl-2',4-dioxospiro[cyclohexane-1,3'-indolin]-2-ene-3-carbonitrile | 298 |
| 941 | 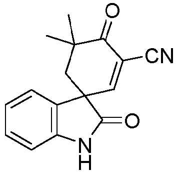 | 5,5-dimethyl-2',4-dioxospiro[cyclohexane-1,3'-indolin]-2-ene-3-carbonitrile | 265 [M-H]⁻ |

### Example 942, enantiomer 1: 1'-cyclopropyl-5,5-dimethyl-2',4-dioxospiro[cyclohexane-1,3'-indolin]-2-ene-3-carbonitrile

### Example 943, enantiomer 2: 1'-cyclopropyl-5,5-dimethyl-2',4-dioxospiro[cyclohexane-1,3'-indolin]-2-ene-3-carbonitrile

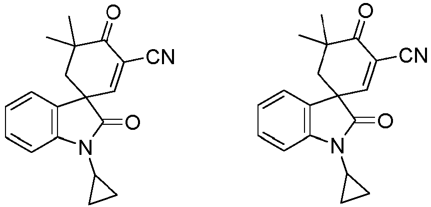

**Step 1:** To a stirred solution of 5,5-dimethyl-2',4-dioxospiro[cyclohexane-1,3'-indolin]-2-ene-3-carbonitrile (38 mg, 0.143 mmol) in MeCN (3 mL) were added cyclopropylboronic acid (18.39 mg, 0.214 mmol), diacetoxycopper (78 mg, 0.428 mmol) and pyridine (56.4 mg, 0.713 mmol) under air. Then the reaction mixture was stirred at 105 °C for 1 h. The reaction progress was monitored by LCMS and TLC. The reaction mixture was filtered and the filtrate was purified by *prep*-TLC (PE : EA = 1 : 1) to afford 1'-cyclopropyl-5,5-dimethyl-2',4-dioxospiro[cyclohexane-1,3'-indolin]-2-ene-3-carbonitrile (20 mg, 0.065 mmol, 45.7% yield) as a light yellow solid, which was used through in resolution. MS: *m*/*z* = 307.30 [M + H]⁺. ¹H-NMR (400 MHz, Chloroform-*d*) 7.41 - 7.39 (m, 1H), 7.21 - 7.15 (m, 3H), 7.01 - 6.98 (m, 1H), 2.73 - 2.71 (m, 1H), 2.40 - 2.36 (m, 1H), 2.28 - 2.20 (m, 1H), 1.52 (s, 3H), 1.34 (s, 3H), 1.20 - 1.14 (m, 2H), 0.94 - 0.86 (m, 2H). 1'-cyclopropyl-5,5-dimethyl-2',4-dioxospiro[cyclohexane-1,3'-indolin]-2-ene-3-carbonitrile (24 mg, 0.078 mmol) was purified by Chiral-HPLC with the following condition: Column: CHIRAL ART Cellulose-SB, 4.6*100mm,3um; Mobile Phase A:Hex : EtOH=70:30, Mobile Phase B:; Flow rate:1 mL/min; Gradient:0 B to 30% B in min; nm; RT1:5.6; RT25.3. The combined fractions was concentrated under reduced pressure, the residue was lyophilized to afford 1'-cyclopropyl-5,5-dimethyl-2',4-dioxospiro[cyclohexane-1,3'-indolin]-2-ene-3-carbonitrile (5.7 mg, 0.015 mmol, 19.48 % yield) as a yellow solid and 1'-cyclopropyl-5,5-dimethyl-2',4-dioxospiro[cyclohexane-1,3'-indolin]-2-ene-3-carbonitrile (5.3 mg, 0.014 mmol, 18.33 % yield) as a light yellow solid.

### Example 944, enantiomer 1: 5,5-dimethyl-4-oxospiro[cyclohexane-1,3'-indolin]-2-ene-3-carbonitrile 2,2,2-trifluoroacetate

### Example 945, enantiomer 2: 5,5-dimethyl-4-oxospiro[cyclohexane-1,3'-indolin]-2-ene-3-carbonitrile 2,2,2-trifluoroacetate

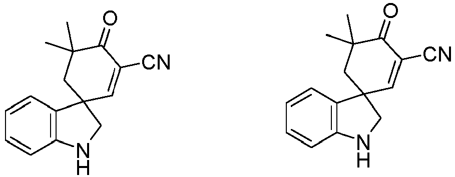

**Step 1:** Ethylene glycol (3.85 g, 62.0 mmol), *p*-toluenesulfonic acid monohydrate (0.410 g, 2.156 mmol) were added to a mixture of spiro[cyclohexane-1,3'-indoline]-2',4-dione (5.8 g, 26.9 mmol) in toluene (60 mL) at 25 °C. The mixture was stirred at 110 °C for 16 h. The resulting solution was diluted with water (60 mL) and the aqueous layer was extracted with ethyl acetate (60 mL × 3). The combined organic layer was washed with brine (100 mL × 3), dried over anhydrous Na₂SO₄ and filtered. The filtrate was concentrated under reduced pressure. The residue was purified by silica gel column chromatography, eluted with 1% - 50% ethyl acetate in petroleum ether. The fractions containing desired product were combined and concentrated under reduced pressure to afford dispiro[indoline-3,1'-cyclohexane-4',2"-[1,3]dioxolan]-2-one (6.5 g, 23.81 mmol, 88% yield) as a light yellow solid. MS ESI calculated for C₁₅H₁₇NO₃ [M + H]⁺ 260.12 found 260.15. ¹H NMR (400 MHz, Chloroform-*d*) δ 8.11 (brs, 1H), 7.39-7.32 (m, 1H), 7.21 (td, *J* = 7.7, 1.2 Hz, 1H), 7.03 (td, *J* = 7.6, 1.1 Hz, 1H), 6.90 (dt, *J* = 7.7, 0.8 Hz, 1H), 4.05-4.00 (m, 4H), 2.33-2.15 (m, 2H), 2.08-1.97 (m, 2H), 1.97-1.81 (m, 4H).

**Step 2:** LAH (1 M in THF, 8.10 mL, 8.10 mmol) was added to a solution of dispiro[indoline-3,1'-cyclohexane-4',2"-[1,3]dioxolan]-2-one (2 g, 7.71 mmol) in THF (20 mL) at 0 °C under the atmosphere of N₂. The mixture was stirred at 60 °C for 16 h . To the reaction mixture were added water (0.3 mL), 15% NaOH (0.3 mL), water (0.9 mL). The mixture was stirred at 0 °C for 40 min. Then the mixture was filtered, the filtrate was concentrated under reduced pressure. The residue was purified by silica gel column chromatography, eluted with 1% - 35% ethyl acetate in petroleum ether. The fractions containing desired product were combined and concentrated under reduced pressure to afford dispiro[indoline-3,1'-cyclohexane-4',2"-[1,3]dioxolane] (1.79 g, 6.20 mmol, 80% yield) as a light yellow solid. MS ESI calculated for C₁₅H₁₉NO₂ [M + H]⁺ 246.14 found 246.05. ¹H NMR (300 MHz, Chloroform-d) δ 7.16-6.98 (m, 2H), 6.80-6.61 (m, 2H), 4.06-3.90 (m, 4H), 3.46 (s, 2H), 2.10-1.88 (m, 2H), 1.88-1.63 (m, 6H).

**Step 3:** To a solution of dispiro[indoline-3,1'-cyclohexane-4',2"-[1,3]dioxolane] (1.7 g, 6.93 mmol) in THF (15 mL) was added aqueous HCl (2 M, 15 mL, 30.0 mmol) at 25 °C. The reaction mixture was stirred at room temperature for 2 h. The resulting solution was diluted with water (20 mL) and the aqueous layer was extracted with ethyl acetate (20 mL × 3). The combined organic layer was washed with brine (20 mL × 3), dried over anhydrous Na₂SO₄ and filtered. The filtrate was concentrated under reduced pressure. The residue was purified by silica gel column chromatography, eluted with 1% - 40% ethyl acetate in petroleum ether. The fractions containing desired product were combined and concentrated under reduced pressure to afford spiro[cyclohexane-1,3'-indolin]-4-one (1.35 g, 6.57 mmol, 95% yield) as a light yellow solid. MS ESI calculated for C₁₃H₁₅NO [M + H]⁺ 202.12 found 202.10. MS: *m*/*z* = 202.10 [M + H]⁺. ¹H NMR (300 MHz, Chloroform-d) δ 7.14-7.02 (m, 2H), 6.82-6.64 (m, 2H), 3.64 (s, 2H), 2.54-2.43 (m, 4H), 2.17-2.07 (m, 4H).

**Step 4:** To a stirred solution of spiro[cyclohexane-1,3'-indolin]-4-one (1.6 g, 7.95 mmol) in DCM (20 mL) at 0 °C under nitrogen atmosphere were added TEA (2.216 mL, 15.90 mmol), DMAP (0.097 g, 0.795 mmol) and Boc₂O (2.77 mL, 11.92 mmol). The resulting mixture was stirred at room temperature for 16 h. The reaction mixture was quenched with saturated solution of ammonium chloride (50 mL) and diluted with ethyl acetate (100 mL). The organic layers were washed with brine, dried over sodium sulfate and concentrated. The crude was purified by silica gel column chromatography, eluted with 0~30% ethyl acetate in petroleum ether to afford *tert-*butyl 4-oxospiro[cyclohexane-1,3'-indoline]-1'-carboxylate (1 g, 3.31 mmol, 41.7% yield) as a light yellow solid. MS ESI calculated for C₁₈H₂₃NO₃ [M -C₄H₈ + H]⁺ 246.11 found 246.20. ¹H NMR (400 MHz, Chloroform-d) δ 7.27-7.17 (m, 2H), 7.11 (d, *J* = 7.5 Hz, 1H), 6.99 (d, *J* = 7.5 Hz, 1H), 4.05 (s, 2H), 2.62-2.43 (m, 4H), 2.19-2.01 (m, 4H), 1.60 (s, 9H).

**Step 5:** To a solution of tert-butyl 4-oxospiro[cyclohexane-1,3'-indoline]-1'-carboxylate (1.1 g, 3.65 mmol) in THF (35 mL) were added NaH (60% in mineral oil, 0.292 g, 7.30 mmol) at 0 °C. Then a solution of MeI (0.685 mL, 10.95 mmol) in THF (1.5 mL) was added. The resulted mixture was stirred for 1 h at 105 °C. The reaction solution was quenched by water (50 mL), diluted with ethyl acetate (100 mL), washed with brine (50 mL × 2), dried over anhydrous Na₂SO₄ and filtered. The filtrate was concentrated under reduced pressure. The reaction was purified by silica gel column chromatography, eluted with 0 - 20% ethyl acetate in petroleum ether to give tert-butyl 3,3-dimethyl-4-oxospiro[cyclohexane-1,3'-indoline]-1'-carboxylate (330 mg, 0.912 mmol, 24.98 % yield) as a light yellow solid. MS ESI calculated for C₂₀H₂₇NO₃ [M - C₄H₈ + MeCN + H]⁺ 315.14 found 315.15. ¹H NMR (300 MHz, Chloroform-d) δ 7.29-7.15 (m, 2H), 7.13-7.04 (m, 1H), 6.98 (d, *J=* 7.5 Hz, 1H), 4.30 (brs, 1H), 4.06-3.96 (m, 1H), 2.87 (td, *J* = 14.6, 5.7 Hz, 1H), 2.40-2.16 (m, 2H), 2.16-1.80 (m, 3H), 1.62 (s, 9H), 1.29 (s, 3H), 1.15 (s, 3H). **Step 6:** To a solution of tert-butyl 3,3-dimethyl-4-oxospiro[cyclohexane-1,3'-indoline]-1'-carboxylate (325 mg, 0.987 mmol) in toluene (3 mL) were added ethyl formate (0.883 mL, 10.85 mmol) and sodium methoxide (1954 mg, 10.85 mmol). The resulted mixture was stirred for 1 h at room temperature. The reaction solution was quenched by water (5 mL), diluted with ethyl acetate (10 mL), washed with brine (10 mL × 2), dried over anhydrous Na₂SO₄, filtered, and concentrated in vacuo to give tert-butyl 5-(hydroxymethylene)-3,3-dimethyl-4-oxospiro[cyclohexane-1,3'-indoline]-1'-carboxylate (350 mg, 0.979 mmol, 99% yield) as an oil, which was used for the next step without further purification. MS ESI calculated for C₂₁H₂₇NO₄ [M -C₄H₈ + H]⁺ 302.13 found 302.10.

**Step 7:** To a solution of *tert*-butyl 5-(hydroxymethylene)-3,3-dimethyl-4-oxospiro[cyclohexane-1,3'-indoline]-1'-carboxylate (350 mg, 0.979 mmol) in EtOH (6 mL) were added hydroxylamine hydrochloride (708 mg, 10.18 mmol) in water (0.5 mL). The resulted mixture was stirred for 1 h at 80 °C. The reaction solution was quenched by water (5 mL), diluted with ethyl acetate (5 mL), washed with brine (5 mL × 2), dried over anhydrous Na₂SO₄, filtered, and concentrated in vacuo to give tert-butyl 7,7-dimethyl-6,7-dihydro-4H-spiro[benzo[d]isoxazole-5,3'-indoline]-1'-carboxylate (325 mg, 0.917 mmol, 94% yield) as an oil, which was used for the next step without further purification. MS ESI calculated for C₂₁H₂₆N₂O₃ [M -C₄H₈ + H]⁺ 299.13 found 299.10.

**Step 8:** To a solution of sodium methanolate (2477 mg, 13.75 mmol) in MeOH (15 mL) were added tert-butyl 7,7-dimethyl-6,7-dihydro-4H-spiro[benzo[d]isoxazole-5,3'-indoline]-1'-carboxylate (325 mg, 0.917 mmol) in Et₂O (30 mL). The resulted mixture was stirred for 2 h at room temperature. The reaction solution was quenched by NH₄Cl (10 mL), diluted with ethyl acetate (20 mL), washed with brine (10 mL × 2), dried over anhydrous Na₂SO₄ and filtered. The filtrate was concentrated under reduced pressure. The reaction was purified by silica gel column chromatography, eluted with 0 - 20% EtOAc in petroleum ether to give tert-butyl 5-cyano-3,3-dimethyl-4-oxospiro[cyclohexane-1,3'-indoline]-1'-carboxylate (190 mg, 0.536 mmol, 58.5% yield) as a yellow solid. MS ESI calculated for C₂₁H₂₆N₂O₃ [M + Na]⁺ 377.19 found 377.15. ¹H NMR (400 MHz, Chloroform-d) δ 7.267-7.18 (m, 2H), 7.15-7.04 (m, 1H), 7.00 (d, *J=* 7.5 Hz, 1H), 4.30 (brs, 1H), 4.17-3.93 (m, 2H), 2.47 (dd, *J=* 13.8, 5.1 Hz, 1H), 2.31 (d, *J* = 14.5 Hz, 1H), 2.27-2.15 (m, 1H), 2.00 (dd, *J* = 14.5, 3.5 Hz, 1H), 1.62 (s, 9H), 1.33 (s, 3H), 1.22 (s, 3H).

**Step 9:** To a solution of *tert*-butyl 5-cyano-3,3-dimethyl-4-oxospiro[cyclohexane-1,3'-indoline]-1'-carboxylate (190 mg, 0.536 mmol) in THF (2 mL) was added Pd(OAc)₂ (96 mg, 0.429 mmol). The resulted mixture was stirred for 4 h at room temperature. The filtrate was concentrated under reduced pressure. The residue was purified by Prep-TLC with ethyl acetate / petroleum ether (1 : 3) to afford tert-butyl 3-cyano-5,5-dimethyl-4-oxospiro[cyclohexane-1,3'-indolin]-2-ene-1'-carboxylate (100 mg, 0.281 mmol, 52.4% yield) as a yellow solid. MS ESI calculated for C₂₁H₂₄N₂O₃ [M + MeCN + Na]⁺ 416.18 found 416.15. ¹H NMR (400 MHz, Chloroform-d) δ 7.34-7.25 (m, 3H), 7.07-6.98 (m, 2H), 4.20-4.07 (m, 2H), 2.35 (d, *J* = 14.7 Hz, 1H), 2.18 (d, *J* = 14.8, 2.1 Hz, 1H), 1.59 (s, 9H), 1.31 (s, 3H), 1.25 (s, 3H).

**Step 10:** To hydrogen chloride (4.0 M in 1,4-dioxane, 2 mL, 0.284 mmol) was added tert-butyl 3-cyano-5,5-dimethyl-4-oxospiro[cyclohexane-1,3'-indolin]-2-ene-1'-carboxylate (100 mg, 0.284 mmol). The resulted mixture was stirred for 1 h at room temperature. The reaction solution was concentrated in vacuo to give 5,5-dimethyl-4-oxospiro[cyclohexane-1,3'-indolin]-2-ene-3-carbonitrile (70 mg, 0.277 mmol, 98 % yield) as a light yellow soild, which was used for the next step without further purification. MS ESI calculated for C₁₆H₁₆N₂O [M + H]⁺ 253.13 found 253.10. ¹H NMR (400 MHz, Chloroform-d) δ 7.75-7.69 (m, 1H), 7.59-7.49 (m, 2H), 7.42 (d, *J* = 1.8 Hz, 1H), 7.31-7.25 (m, 1H), 4.23-2.4.01(m, 2H), 2.43 (d, *J* = 14.4 Hz, 1H), 2.35 (d, *J=* 14.9 Hz, 1H), 1.35 (s, 3H), 1.26 (s, 3H).

**Step 11:** 5,5-dimethyl-4-oxospiro[cyclohexane-1,3'-indolin]-2-ene-3-carbonitrile (20 mg, 0.079 mmol) was separated by Chiral-Prep-HPLC with the following conditions: CHIRALPAK IH-3, 4.6 × 50 mm (3 µm); mobile phase: Hexanes: IPA = 70 : 30; Detector, UV 254 nm. 5,5-dimethyl-4-oxospiro[cyclohexane-1,3'-indolin]-2-ene-3-carbonitrile (5.5 mg, 0.022 mmol) was obtained at 2.77 min as a yellow green solid which was purified by Prep-HPLC with the following conditions: Column: SunFire Prep C¹⁸ OBD Column, 19 × 150 mm, 5 µm, 10 nm; Mobile Phase A: Water (0.05 % TFA), Mobile Phase B: MeCN; Flow rate: 20 mL/min; Gradient: 15% B to 50% B in 6 min; Wave Length: 210/254 nm; RT1 (min): 5.57; 5,5-dimethyl-4-oxospiro[cyclohexane-1,3'-indolin]-2-ene-3-carbonitrile 2,2,2-trifluoroacetate (3.1 mg, 8.18 µmol, 10.32% yield) was obtained as a yellow green solid; MS ESI calculated for C₁₆H₁₆N₂O [M + H]⁺ 253.32 found 253.05. ¹H NMR (300 MHz, Chloroform-d) δ 7.43 (d, *J* = 1.8 Hz, 1H), 7.37-7.27 (m, 1H), 7.14-7.01 (m, 3H), 5.43 (brs, 1H), 3.96 (d, *J* = 10.3 Hz, 1H), 3.89 (d, *J* = 10.4 Hz, 1H), 2.39 (d, *J* = 14.8 Hz, 1H), 2.27 (d, *J* = 14.9 Hz, 1H), 1.31 (s, 3H), 1.28 (s, 3H).

**Step 12:** 5,5-dimethyl-4-oxospiro[cyclohexane-1,3'-indolin]-2-ene-3-carbonitrile (7.2 mg, 0.029 mmol) was obtained at 3.87 min as a light yellow solid which was purified by Prep-HPLC with the following conditions: Column: SunFire Prep C¹⁸ OBD Column, 19 × 150 mm, 5 µm, 10 nm; Mobile Phase A: Water (0.05% TFA), Mobile Phase B: MeCN; Flow rate: 20 mL/min; Gradient: 15% B to 50% B in 6 min; Wave Length: 210/254 nm; RT1 (min): 5.57; 5,5-dimethyl-4-oxospiro[cyclohexane-1,3'-indolin]-2-ene-3-carbonitrile 2,2,2-trifluoroacetate (5.0 mg, 0.013 mmol, 16.81% yield) was obtained as a light yellow solid. MS ESI calculated for C₁₆H₁₆N₂O [M + MeCN + H]⁺ 294.32 found 294.05. ¹H NMR (300 MHz, Chloroform-d) δ 7.43 (s, 1H), 7.33 (s, 1H), 7.20-7.05 (m, 3H), 5.68 (brs, 1H), 3.98 (d, *J=* 10.6 Hz, 1H), 3.90 (d, *J=* 10.5 Hz, 1H), 2.39 (d, *J=* 14.9 Hz, 1H), 2.27 (d, *J=* 14.9 Hz, 1H), 1.31 (s, 3H), 1.28 (s, 3H).

### Example 946, enantiomer 1 1'-acetyl-5,5-dimethyl-4-oxospiro[cyclohexane-1,3'-indolin]-2-ene-3-carbonitrile

### Example 947, enantiomer 2 1'-acetyl-5,5-dimethyl-4-oxospiro[cyclohexane-1,3'-indolin]-2-ene-3-carbonitrile

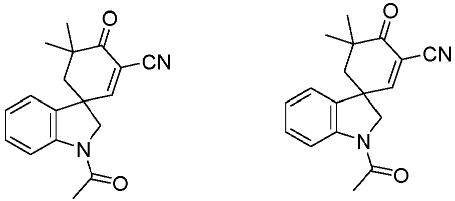

**Step 1:** To a solution of 5,5-dimethyl-4-oxospiro[cyclohexane-1,3'-indolin]-2-ene-3-carbonitrile (70 mg, 0.277 mmol) in DCM (1 mL) were added acetyl chloride (43.6 mg, 0.555 mmol) and DIPEA (0.194 mL, 1.110 mmol) at 0 °C. The resulted mixture was stirred for 2 h at room temperature. The reaction solution was quenched by water (2 mL), diluted with DCM (6 mL), washed with brine (6 mL × 2), dried over anhydrous Na₂SO₄ and filtered. The filtrate was concentrated under reduced pressure. The residue was purified by Prep-TLC with ethyl acetate / petroleum ether (1 : 1) to afford 1'-acetyl-5,5-dimethyl-4-oxospiro[cyclohexane-1,3'-indolin]-2-ene-3-carbonitrile (60 mg, 0.204 mmol, 73.5% yield) as a yellow solid. MS ESI calculated for C₁₈H₁₈N₂O₂ [M + MeCN + H]⁺ 336.14 found 336.10. ¹H NMR (300 MHz, Chloroform-d) δ 8.29-8.21 (m, 1H), 7.33-7.28 (m, 2H), 7.18-7.04 (m, 2H), 4.31- 4.15 (m, 2H), 2.60-2.09 (m, 2H), 2.27 (s, 3H), 1.26 (s, 6H).

**Step 2:** 1'-Acetyl-5,5-dimethyl-4-oxospiro[cyclohexane-1,3'-indolin]-2-ene-3-carbonitrile (90 mg, 0.306 mmol) was purified by Chiral-Prep-HPLC with the following conditions: CHIRALPAK IA-3, 4.6 × 50 mm (3 µm); Mobile phase: Hexanes : EtOH = 70 : 30; Detector, UV 254 nm. RT₁: 1.45min; RT₂: 1.84 min;
The fractions of the first peak (RT₁: 1.45 min) were combined and concentrated under reduced pressure, lyophilized to give 1'-acetyl-5,5-dimethyl-4-oxospiro[cyclohexane-1,3'-indolin]-2-ene-3-carbonitrile (23.7 mg, 0.080 mmol, 26.0% yield) as a light yellow solid. MS ESI calculated for C₁₈H₁₈N₂O₂ [M + H]⁺ 295.14 found 295.25. ¹H NMR (300 MHz, DMSO-*d*₆) δ 8.10 (d, *J* = 8.1 Hz, 1H), 7.76 (s, 1H), 7.33-7.28 (m, 2H), 7.09-7.06 (m, 1H), 4.47 (d, *J=* 11.1 Hz, 1H), 4.34 (d, *J* = 11.0 Hz, 1H), 2.57-2.44 (m, 1H), 2.26-2.16 (m, 1H), 2.20 (s, 3H), 1.24 (s, 3H), 1.14 (s, 3H). The fractions of the second peak (RT₁: 1.84 min) were combined and concentrated under reduced pressure, lyophilized to give 1'-acetyl-5,5-dimethyl-4-oxospiro[cyclohexane-1,3'-indolin]-2-ene-3-carbonitrile (25.0 mg, 0.083 mmol, 27.2 % yield) as a light yellow solid. MS ESI calculated for C₁₈H₁₈N₂O₂ [M + H]⁺ 295.14 found 295.25. ¹H NMR (300 MHz, DMSO-*d*₆) δ 8.10 (d, *J=* 8.0 Hz, 1H), 7.76 (s, 1H), 7.36-7.28 (m, 2H), 7.09-7.06 (m, 1H), 4.47 (d, *J=* 11.1 Hz, 1H), 4.34 (d, *J=* 11.1 Hz, 1H), 2.62-2.39 (m, 1H), 2.32-2.12 (m, 1H), 2.20 (s, 3H), 1.24 (s, 3H), 1.14 (s, 3H).

### Example 948, enantiomer 1: 2-benzoyl-3,3,9,9-tetramethyl-8-oxo-2-azaspiro[4.5]dec-6-ene-7-carbonitrile

### Example 949, enantiomer 2: 2-benzoyl-3,3,9,9-tetramethyl-8-oxo-2-azaspiro[4.5]dec-6-ene-7-carbonitrile

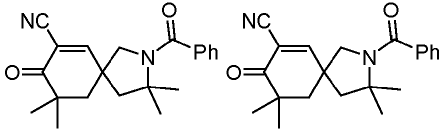

**Step 1:** To a solution of 1,4-dioxa-10-azadispiro[4.2.4⁸.2⁵]tetradecan-11-one (1 g, 4.73 mmol) in THF (16 mL) at 0 °C was added NaH (0.295 g, 6.15 mmol). The mixture was stirred at 0 °C for 15 minutes. Added (bromomethyl)benzene (0.619 ml, 5.21 mmol) and the reaction was stirred for 3 h at room temperature. The reaction was quenched with water and extracted with DCM (3x). The combined organic layers were dried over anhydrous sodium sulfate, filtered, and concentrated under reduced pressure. The residue was purified by column chromatography on silica (0-100% EtOAc/Hexanes) to afford 10-benzyl-1,4-dioxa-10-azadispiro[4.2.4⁸.2⁵]tetradecan-11-one as a solid. MS: 302 (M + 1).

**Step 2:** To a solution of 10-benzyl-1,4-dioxa-10-azadispiro[4.2.4⁸.2⁵]tetradecan-11-one (750 mg, 2.49 mmol) in DCM (10 mL) was added 2,6-di-tert-butyl-4-methylpyridine (613 mg, 2.99 mmol). The mixture was cooled to -78 °C, and trifluoromethanesulfonic anhydride (0.502 ml, 2.99 mmol) was added dropwise. The mixture was stirred at -78 °C for 45 minutes. Methylmagnesium bromide (3 M in Et₂O, 2.49 mL, 7.47 mmol) was added and the reaction was slowly warmed to room temperature and stirred overnight. Added additional methylmagnesium bromide (3 M in Et₂O, 2.49 ml, 7.47 mmol), and the reaction was stirred for 2 h at room temperature. The mixture was quench with saturated aqueous NH₄Cl, and extracted with DCM (3x). The combined organic layers were washed with brine, dried over anhydrous sodium sulfate, filtered, and concentrated under reduced pressure. The residue was purified by column chromatography on silica (0-10% MeOH/DCM) to afford 10-benzyl-11,11-dimethyl-1,4-dioxa-10-azadispiro[4.2.4⁸.2⁵]tetradecane as an oil. MS: 316 (M + 1).

**Step 3:** To a solution of 10-benzyl-11,11-dimethyl-1,4-dioxa-10-azadispiro[4.2.4⁸.2⁵]tetradecane (690 mg, 2.19 mmol) in acetone (11 mL) was added *p*-toluenesulfonic acid monohydrate (0.458 g, 2.41 mmol) and water (0.394 ml, 21.9 mmol). The reaction was stirred at 70 °C overnight. Added additional water (0.394 ml, 21.9 mmol) and *p*-toluenesulfonic acid monohydrate (0.458 g, 2.41 mmol) and stirred at 70 °C for 4 h. The reaction was cooled to room temperature and quenched with saturated aqueous sodium bicarbonate. Extracted with DCM (3x), and the combined organic layers were dried over anhydrous sodium sulfate, filtered, and concentrated under reduced pressure. The residue was purified by column chromatography on silica (0-10% MeOH/DCM) to afford 2-benzyl-3,3-dimethyl-2-azaspiro[4.5]decan-8-one as an oil. MS: 272 (M + 1).

**Step 4:** To a solution of 2-benzyl-3,3-dimethyl-2-azaspiro[4.5]decan-8-one (568 mg, 2.09 mmol) in t-butanol (10 mL) was added potassium tert-butoxide (0.587 g, 5.23 mmol). The mixture was stirred for 30 minutes at room temperature. Methyl iodide (0.275 mL, 4.39 mmol) was added and the reaction was stirred overnight at room temperature. Added an additional 184 µL of MeI and stirred for 36 h. The reaction was quenched with water, extracted with EtOAc (3x), and the combined organic layers were dried over sodium sulfate, filtered, and concentrated under reduced pressure. The residue was purified by column chromatography on silica (0-30% EtOAc/Hexanes) to afford 2-benzyl-3,3,7,7-tetramethyl-2-azaspiro[4.5]decan-8-one as an oil. MS: 300 (M + 1).

**Step 5:** To a parr shaker reaction tube containing palladium hydroxide on carbon (20 wt%, 200 mg, 0.285 mmol) was added a solution of 2-benzyl-3,3,7,7-tetramethyl-2-azaspiro[4.5]decan-8-one (200 mg, 0.668 mmol) in ethanol (10 mL). Concentrated HCl (62 µL, 2.04 mmol) was added, and the reaction was run on a parr shaker pressurized with 50 psi H₂ for 72 h. The reaction was filtered through celite, and rinsed with MeOH. The filtrated was concentrated under reduced pressure, and the residue was purified by column chromatography on silica (0-10% MeOH/DCM) to afford 3,3,7,7-tetramethyl-2-azaspiro[4.5]decan-8-one as an oil. MS: 210 (M + 1).

**Step 6:** To a solution of 3,3,7,7-tetramethyl-2-azaspiro[4.5]decan-8-one (140 mg, 0.67 mmol) in DCM (2.2 mL) was added and TEA (280 µL, 2.01 mmol) add benzoyl chloride (117 µL, 1.01 mmol). The reaction was stirred at room temperature overnight. The mixture was concentrated under reduced pressure and the residue was purified by column chromatography on silica (0-100% EtOAc/Hexanes) to afford 2-benzoyl-3,3,7,7-tetramethyl-2-azaspiro[4.5]decan-8-one as a solid. MS: 314 (M + 1).

**Step 7:** To a solution of 2-benzoyl-3,3,7,7-tetramethyl-2-azaspiro[4.5]decan-8-one (183 mg, 0.584 mmol) in THF (1.9 mL) at -78 °C was added LDA (1.0 M in THF, 1460 µL, 1.460 mmol). The mixture was stirred at -78 °C for 30 minutes then a solution of tosyl cyanide (127 mg, 0.701 mmol) in THF (1 mL) was added. The reaction was stirred for 1 h at -78 °C and then was slowly warmed to room temperature and stirred overnight. The reaction was quenched with 2M NH₄OH (0.35 mL), and neutralize with 1M HCl (2.5 mL). The mixture was extracted with EtOAc (3x), and the combined organic layers were dried over anhydrous sodium sulfate, filtered, and concentrated under reduced pressure. The residue was purified by column chromatography on silica (0-50% EtOAc/Hexanes) to afford 2-benzoyl-3,3,9,9-tetramethyl-8-oxo-2-azaspiro[4.5]decane-7-carbonitrile as an oil. MS: 339.

**Step 8:** Pyridine (38 µL, 0.47 mmol) was added a solution of phenylselenenyl chloride (89 mg, 0.47 mmol) in DCM (1.2 mL) at 0 °C. The reaction mixture was stirred for 20 mins at 0 °C, and then a solution of 2-benzoyl-3,3,9,9-tetramethyl-8-oxo-2-azaspiro[4.5]decane-7-carbonitrile (79 mg, 0.233 mmol) in DCM (1.2 mL) was added The reaction mixture was stirred for 2 h at 0°C. The reaction was washed with 1 M aqueous HCl (1.0 mL) and water (2.0 mL), and to the collected organic layer at 0°C was added hydrogen peroxide (30% in water, 477 µL, 4.67 mmol). The reaction was stirred at 0 °C for 10 minutes, and then was washed with 1 M aqueous HCl (1.0 mL) and water (2.0 mL). The mixture was concentrated under reduced pressure, and the residue was purified by column chromatography on silica (0-60% EtOAc/Hexanes) to afford racemic 2-benzoyl-3,3,9,9-tetramethyl-8-oxo-2-azaspiro[4.5]dec-6-ene-7-carbonitrile. The racemic mixture was purified by chiral SFC (CCU column, 20%/80% MeOH/CO₂) to afford two products as oils: 2-benzoyl-3,3,9,9-tetramethyl-8-oxo-2-azaspiro[4.5]dec-6-ene-7-carbonitrile. MS: 337 (M + 1). ¹H NMR (499 MHz, Chloroform-d) δ 7.57 (s, 1H), 7.44 - 7.37 (m, 5H), 3.58 - 3.49 (m, 2H), 2.33 (d, *J* = 13.6 Hz, 1H), 2.18 (d, *J=* 13.6 Hz, 1H), 1.98 (d, *J* = 14.3 Hz, 1H), 1.85 (d, *J* = 14.3 Hz, 1H), 1.75 (s, 3H), 1.69 (s, 3H), 1.23 (s, 3H), 1.12 (s, 3H). 2-benzoyl-3,3,9,9-tetramethyl-8-oxo-2-azaspiro[4.5]dec-6-ene-7-carbonitrile. MS: 337 (M + 1). ¹H NMR (499 MHz, Chloroform-d) δ 7.57 (s, 1H), 7.44 - 7.37 (m, 5H), 3.58 - 3.49 (m, 2H), 2.33 (d, *J* = 13.6 Hz, 1H), 2.18 (d, *J=* 13.6 Hz, 1H), 1.98 (d, *J* = 14.3 Hz, 1H), 1.85 (d, *J* = 14.3 Hz, 1H), 1.75 (s, 3H), 1.69 (s, 3H), 1.23 (s, 3H), 1.12 (s, 3H).

### Example 950, enantiomer 1: 2-benzoyl-9,9-dimethyl-8-oxo-2-azaspiro[4.4]non-6-ene-7-carbonitrile

### Example 951, enantiomer 2: 2-benzoyl-9,9-dimethyl-8-oxo-2-azaspiro[4.4]non-6-ene-7-carbonitrile

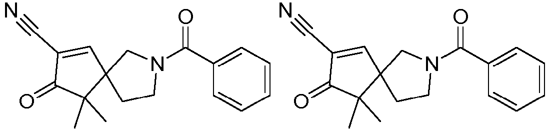

**Step 1:** A solution of tert-butyl 7-oxo-2-azaspiro[4.4]nonane-2-carboxylate (515 mg, 2.15 mmol) dissolved in anhydrous 2-MeTHF (10 mL) was added slowly to a 20 mL microwave vial containing NaH (172 mg, 4.30 mmol) under a nitrogen atmosphere. The resulting solution was stirred at room temperature for 10 min. Methyl iodide (0.404 mL, 6.45 mmol) was added and the reaction was heated at 105°C for 1 h. The reaction mixture was cooled to 0°C and quenched by slow addition of aqueous saturated ammonium chloride. The mixture was extracted with EtOAc (3x) and the combined organic layers were dried over anhydrous sodium sulfate, filtered, and concentrated under reduced pressure. The residue was purified by column chromatography on silica (0-60% Et₂O/Hexanes) to afford *tert*-butyl 6,6-dimethyl-7-oxo-2-azaspiro[4.4]nonane-2-carboxylate as an oil. MS: 212 (M + H - tBu).

**Step 2:** To a suspension of sodium hydride (93 mg, 2.3 mmol) in THF (4 mL) at 0 °C was added a solution of *tert*-butyl 6,6-dimethyl-7-oxo-2-azaspiro[4.4]nonane-2-carboxylate (311 mg, 1.16 mmol) in THF (4 mL). The reaction mixture was stirred for 20 mins. and then ethyl formate (282 µL, 3.49 mmol) was added. The reaction mixture was warmed to room temperature and stirred over the weekend. The mixture was quenched with saturated aqueous ammonium chloride and extracted with EtOAc (2x). The combined organic layers were dried over anhydrous sodium sulfate, filtered, and concentrated under reduced pressure to afford tert-butyl-8-(hydroxymethylene)-6,6-dimethyl-7-oxo-2-azaspiro[4.4]nonane-2-carboxylate as a solid. MS: 240 (M + H - tBu).

**Step 3:** To a solution of tert-butyl-8-(hydroxymethylene)-6,6-dimethyl-7-oxo-2-azaspiro[4.4]nonane-2-carboxylate (344 mg, 1.17 mmol) in water (333 µL) and acetic acid (2 mL) was added hydroxylamine hydrochloride (97 mg, 1.4 mmol) and sodium acetate (191 mg, 2.33 mmol). The mixture was stirred at 60 °C for 3 h. The reaction was cooled to room temperature and extracted with DCM (3x). The combined organic layers were washed with sat. aqueous sodium bicarbonate and brine, dried over anhydrous sodium sulfate, filtered, and concentrated under reduced pressure to afford *tert*-butyl-8-((hydroxyimino)methyl)-6,6-dimethyl-7-oxo-2-azaspiro[4.4]nonane-2-carboxylate as an oil. MS: 255 (M + H - tBu).

**Step 4:** To a vial containing *tert*-butyl-8-((hydroxyimino)methyl)-6,6-dimethyl-7-oxo-2-azaspiro[4.4]nonane-2-carboxylate (361 mg, 1.16 mmol) in THF (11.6 mL) cooled to 0 °C was added triethylamine (1.3 mL, 9.3 mmol) and then thionyl chloride (0.340 mL, 4.65 mmol) dropwise. The mixture was stirred for 1 h at 0 °C. Water (50 mL) was added slowly to the mixture at 0°C, and the mixture was extracted with EtOAc (3x). The combined organic layers were washed with brine, dried over anhydrous sodium sulfate, filtered, and concentrated under reduced pressure. The residue was purified by column chromatography on silica (0-70% Et₂O/hexanes) to afford *tert*-butyl 6,6-dimethyl-4,6-dihydrospiro[cyclopenta[d]isoxazole-5,3'-pyrrolidine]-1'-carboxylate as an oil. MS: 237 (M + H - tBu).

**Step 5:** To a solution of *tert*-butyl 6,6-dimethyl-4,6-dihydrospiro[cyclopenta[d]isoxazole-5,3'-pyrrolidine]-1'-carboxylate (179 mg, 0.611 mmol) in MeOH (3.1 mL) was added sodium methoxide (349 µL, 1.83 mmol). The reaction mixture was stirred at room temperature overnight. The mixture was concentrated under reduced pressure, diluted with EtOAc, and neutralized with 1 M aq HCl (3 mL). The layers were separated, and the aqueous layer was extracted with EtOAc. The combined organic layers were washed with brine, dried over anhydrous sodium sulfate, filtered, and concentrated under reduced pressure to give *tert*-butyl 8-cyano-6,6-dimethyl-7-oxo-2-azaspiro[4.4]nonane-2-carboxylate as an oil. MS: 237 (M + H - *t*Bu).

**Step 6:** To a solution of *tert*-butyl 8-cyano-6,6-dimethyl-7-oxo-2-azaspiro[4.4]nonane-2-carboxylate (158 mg, 0.540 mmol) in THF (1.5 mL) at room temperature was added palladium(II) acetate (121 mg, 0.54 mmol). The mixture was stirred at room temperature overnight. Another equivalent of palladium(II) acetate was added and the mixture was stirred at room temperature overnight. The mixture was concentrated under reduced pressure, diluted with DCM (5 mL), and then 1:1 water:brine was added. The organic layer was collected via a phase separator and concentrated under reduced pressure. The residue was purified by column chromatography on silica (0-100% Et2O/Hexanes) to afford tert-butyl 7-cyano-9,9-dimethyl-8-oxo-2-azaspiro[4.4]non-6-ene-2-carboxylate as an oil. MS: 235 (M + H - tBu).

**Step 7:** To a solution of tert-butyl 7-cyano-9,9-dimethyl-8-oxo-2-azaspiro[4.4]non-6-ene-2-carboxylate (70 mg, 0.24 mmol) dissolved in DCM (2.4 mL) was added HCl (4.0 M in dioxane, 603 µL, 2.41 mmol). The mixture was stirred at room temperature overnight. The mixture was concentrated under reduced pressure to give 9,9-dimethyl-8-oxo-2-azaspiro[4.4]non-6-ene-7-carbonitrile as a solid HCL salt. MS: 191 (M + H).

**Step 8:** To a solution of 9,9-dimethyl-8-oxo-2-azaspiro[4.4]non-6-ene-7-carbonitrile, HCl (50.2 mg, 0.22 mmol) in DCM (2.2 mL) was added TEA (123 µL, 0.886 mmol). Benzoyl chloride (38.6 µL, 0.332 mmol) was added and the mixture was stirred at room temperature overnight. The mixture was diluted with water and extracted with DCM (2x). The combined organic layers were washed with brine, dried over anhydrous sodium sulfate, filtered, and concentrated under reduced pressure. The residue was purified by column chromatography on silica (0-10% MeOH/DCM) to afford racemic 2-benzoyl-9,9-dimethyl-8-oxo-2-azaspiro[4.4]non-6-ene-7-carbonitrile. The racemic mixture was purified by chiral SFC (OJ-H column, 15%/85% MeOH/CO₂) to afford two products as solids: 2-benzoyl-9,9-dimethyl-8-oxo-2-azaspiro[4.4]non-6-ene-7-carbonitrile - enantiomer 1. MS: 295 (M + H). ¹H NMR (600 MHz, DMSO-*d*₆) δ 9.02 - 8.72 (m, 1H), 7.63 - 7.42 (m, 5H), 3.81 - 3.67 (m, 2H), 3.67 - 3.60 (m, 1H), 3.48 - 3.43 (m, 1H), 2.29 - 2.18 (m, 1H), 1.98 - 1.84 (m, 1H), 1.12 - 0.92 (m, 6H).

2-benzoyl-9,9-dimethyl-8-oxo-2-azaspiro[4.4]non-6-ene-7-carbonitrile - enantiomer 2. MS: 295 (M + H). ¹H NMR (600 MHz, DMSO-*d*₆) δ 9.02 - 8.72 (m, 1H), 7.63 - 7.42 (m, 5H), 3.81 - 3.67 (m, 2H), 3.67 - 3.60 (m, 1H), 3.48 - 3.43 (m, 1H), 2.29 - 2.18 (m, 1H), 1.98 - 1.84 (m, 1H), 1.12 - 0.92 (m, 6H).

### PathHunter^{®} U2OS Keap1-Nrf2 Nuclear Translocation Cellular Assay

PathHunter^{®} U2OS Keap1-NRF2 Nuclear Translocation Cell Line (Eurofins-DiscoverX) is engineered to express an Enzyme Donor (ED) tagged to Nrf2 protein and an enzyme acceptor (EA) in the nucleus. ED-Nrf2 retains normal function in this cell line. Under normal conditions, ED-Nrf2 is bound to Keap1 and is marked for degradation through the ubiquitination pathway. When the Nrf2 pathway is activated, ED-Nrf2 is translocated to the nucleus, allowing complementation of ED with EA to form an active enzyme that hydrolyzes a substrate (provided in the assay kit) to generate a chemiluminescent signal. The translocation of ED-Nrf2 to the nucleus is a surrogate readout for measuring the potency of compounds to disrupt the Keap1-Nrf2 interaction that is needed to facilitate Nrf2 degradation.

Assay ready cells are thawed and resuspended in cell plating media (OptiMEM supplemented with 1% FBS). Specifically, 5,000 cells in 20 µL medium are seeded into each well of a 384-well Culture Plate (Perkin Elmer) and allowed to incubate at 37 °C with 5% CO₂ overnight. The following morning, 60 nL of compound (in DMSO at a highest concentration of 10 mM and subsequently diluted, also in DMSO, 1:3 to create a 10-point serial dilution) is added to each well of the prepared cell plate using a Labcyte ECHO. The assay plate is centrifuged (150 x g for 15 seconds) before placing in a 5% CO₂ incubator at 37 °C for 5-6 hours. After compound treatment, the plate is taken out of the incubator and allowed to equilibrate to ambient temperature (15-30 mins). Once equilibrated, 12.5 µL of detection mix is added to each well and luminescence signals are measured after 60 mins of incubation. EC₅₀ values are determined by non-linear regression fit to a model for sigmoidal 4-parameters dose-response curve using a Spotfire assisted template.

**Table 33. PathHunter^{®} U2OS Keap1-Nrf2 nuclear translocation cellular assay data**

| Example No. | PathHunter Assay (EC₅₀, nM) |
|---|---|
| **1** | 575 |
| **2** | 157 |
| **3** | 6895 |
| **4** | 532 |
| **5** | 108 |
| **6** | 1089 |
| **7** | 160 |
| **8** | 251 |
| **9** | 14 |
| **10** | 78 |
| **11** | 98 |
| **12** | 116 |
| **13** | 128 |
| **14** | 531 |
| **15** | 476 |
| **16** | 173 |
| **17** | 1909 |
| **18** | 10230 |
| **19** | 9545 |
| **20** | 952 |
| **21** | 569 |
| **22** | 939 |
| **23** | 20860 |
| **24** | 309 |
| **25** | 2430 |
| **26** | 1597 |
| **27** | 15310 |
| **28** | 139 |
| **29** | 2.9 |
| **30** | 1280 |
| **31** | 21 |
| **32** | 6.9 |
| **33** | 126 |
| **34** | 1302 |
| **35** | 31 |
| **36** | 1788 |
| **37** | 44 |
| **38** | 543 |
| **39** | 29 |
| **40** | 1192 |
| **41** | 311 |
| **42** | 35 |
| **43** | 255 |
| **44** | 231 |
| **45** | 653 |
| **46** | 960 |
| **47** | 3785 |
| **48** | 1799 |
| **49** | 313 |
| **50** | 712 |
| **51** | 51 |
| **52** | 771 |
| **53** | 32 |
| **54** | 73 |
| **55** | 50 |
| **56** | 639 |
| **57** | 1290 |
| **58** | 199 |
| **59** | 60 |
| **60** | 790 |
| **61** | 35 |
| **62** | 319 |
| **63** | 984 |
| **64** | 50 |
| **65** | 215 |
| **66** | 5.9 |
| **67** | 385 |
| **68** | 591 |
| **69** | 31 |
| **70** | 15 |
| **71** | 306 |
| **72** | 28 |
| **73** | 288 |
| **74** | 4028 |
| **75** | 1798 |
| **76** | 775 |
| **77** | 171 |
| **78** | 129 |
| **79** | 50 |
| **80** | 589 |
| **81** | 606 |
| **82** | 2964 |
| **83** | 592 |
| **84** | 319 |
| **85** | 8091 |
| **86** | 2519 |
| **87** | 375 |
| **88** | 708 |
| **89** | 312 |
| **90** | 144 |
| **91** | 183 |
| **92** | 341 |
| **93** | 782 |
| **94** | 11 |
| **95** | 1614 |
| **96** | 526 |
| **97** | 7027 |
| **98** | 570 |
| **99** | 592 |
| **100** | 319 |
| **101** | 342 |
| **102** | 676 |
| **103** | 21 |
| **104** | 495 |
| **105** | 432 |
| **106** | 474 |
| **107** | 435 |
| **108** | 2346 |
| **109** | 339 |
| **110** | 1248 |
| **111** | 1617 |
| **112** | 697 |
| **113** | 571 |
| **114** | 63 |
| **115** | 450 |
| **116** | 9647 |
| **117** | 2349 |
| **118** | 612 |
| **119** | 1700 |
| **120** | 754 |
| **121** | 1197 |
| **122** | 1209 |
| **123** | 3079 |
| **124** | 514 |
| **125** | 25 |
| **126** | 555 |
| **127** | 17 |
| **128** | 891 |
| **129** | 1524 |
| **130** | 1030 |
| **131** | 1052 |
| **132** | 1401 |
| **133** | 390 |
| **134** | 106 |
| **135** | 318 |
| **136** | 6437 |
| **137** | 1726 |
| **138** | 7623 |
| **139** | 4643 |
| **140** | 1256 |
| **141** | 227 |
| **142** | 4120 |
| **143** | 6073 |
| **144** | 5548 |
| **145** | 781 |
| **146** | 747 |
| **147** | 65 |
| **148** | 321 |
| **149** | 572 |
| **150** | 54 |
| **151** | 2366 |
| **152** | 228 |
| **153** | 1967 |
| **154** | 973 |
| **155** | 379 |
| **156** | 693 |
| **157** | 596 |
| **158** | 1688 |
| **159** | 682 |
| **160** | 548 |
| **161** | 227 |
| **162** | 4831 |
| **163** | 4262 |
| **164** | 264 |
| **165** | 3038 |
| **166** | 9282 |
| **167** | 290 |
| **168** | 150 |
| **169** | 199 |
| **170** | 414 |
| **171** | 88 |
| **172** | 211 |
| **173** | 280 |
| **174** | 649 |
| **175** | 1219 |
| **176** | 316 |
| **177** | 198 |
| **178** | 250 |
| **179** | 293 |
| **180** | 230 |
| **181** | 271 |
| **182** | 154 |
| **183** | 706 |
| **184** | 718 |
| **185** | 1345 |
| **186** | 325 |
| **187** | 1019 |
| **188** | 935 |
| **189** | 1359 |
| **190** | 3465 |
| **191** | 940 |
| **192** | 784 |
| **193** | 524 |
| **194** | 531 |
| **195** | 420 |
| **196** | 1434 |
| **197** | 819 |
| **198** | 951 |
| **199** | 3131 |
| **200** | 60 |
| **201** | 90 |
| **202** | 406 |
| **203** | 418 |
| **204** | 45 |
| **205** | 1595 |
| **206** | 677 |
| **207** | 532 |
| **208** | 31 |
| **209** | 169 |
| **210** | 1075 |
| **211** | 299 |
| **212** | 14 |
| **213** | 186 |
| **214** | 204 |
| **215** | 263 |
| **216** | 23 |
| **217** | 1255 |
| **218** | 331 |
| **219** | 273 |
| **220** | 1220 |
| **221** | 591 |
| **222** | 52 |
| **223** | 546 |
| **224** | 644 |
| **225** | 1075 |
| **226** | 167 |
| **227** | 327 |
| **228** | 65 |
| **229** | 321 |
| **230** | 187 |
| **231** | 213 |
| **232** | 197 |
| **233** | 12 |
| **234** | 36 |
| **235** | 501 |
| **236** | 2294 |
| **237** | 263 |
| **238** | 331 |
| **239** | 79 |
| **240** | 23 |
| **241** | 380 |
| **242** | 225 |
| **243** | 363 |
| **244** | 256 |
| **245** | 374 |
| **246** | 285 |
| **247** | 173 |
| **248** | 71 |
| **249** | 168 |
| **250** | 534 |
| **251** | 289 |
| **252** | 168 |
| **253** | 947 |
| **254** | 351 |
| **255** | 263 |
| **256** | 206 |
| **257** | 329 |
| **258** | 345 |
| **259** | 27 |
| **260** | 107 |
| **261** | 63 |
| **262** | 294 |
| **263** | 364 |
| **264** | 340 |
| **265** | 172 |
| **266** | 308 |
| **267** | 51 |
| **268** | 106 |
| **269** | 73 |
| **270** | 420 |
| **271** | 702 |
| **272** | 293 |
| **273** | 412 |
| **274** | 226 |
| **275** | 410 |
| **276** | 67 |
| **277** | 288 |
| **278** | 1729 |
| **279** | 1286 |
| **280** | 323 |
| **281** | 3762 |
| **282** | 97 |
| **283** | 785 |
| **284** | 102 |
| **285** | 396 |
| **286** | 217 |
| **287** | 357 |
| **288** | 83 |
| **289** | 4351 |
| **290** | 260 |
| **291** | 279 |
| **292** | 307 |
| **293** | 34 |
| **294** | 292 |
| **295** | 1150 |
| **296** | 130 |
| **297** | 11 |
| **298** | 181 |
| **299** | 3058 |
| **300** | 2603 |
| **301** | 200 |
| **302** | 40 |
| **303** | 85 |
| **304** | 1157 |
| **305** | 60 |
| **306** | 285 |
| **307** | 310 |
| **308** | 164 |
| **309** | 1651 |
| **310** | 1006 |
| **311** | 2877 |
| **312** | 1701 |
| **313** | 1702 |
| **314** | 1323 |
| **315** | 262 |
| **316** | 1317 |
| **317** | 994 |
| **318** | 1700 |
| **319** | 402 |
| **320** | 1508 |
| **321** | 198 |
| **322** | 1315 |
| **323** | 768 |
| **324** | 2165 |
| **325** | 1251 |
| **326** | 8638 |
| **327** | 1398 |
| **328** | 409 |
| **329** | 674 |
| **330** | 2161 |
| **331** | 3238 |
| **332** | 812 |
| **333** | 1150 |
| **334** | 1280 |
| **335** | 1517 |
| **336** | 632 |
| **337** | 734 |
| **338** | 515 |
| **339** | 859 |
| **340** | 1049 |
| **341** | 1445 |
| **342** | 1396 |
| **343** | 585 |
| **344** | 1850 |
| **345** | 265 |
| **346** | 2262 |
| **347** | 859 |
| **348** | 731 |
| **349** | 2422 |
| **350** | 1775 |
| **351** | 794 |
| **352** | 893 |
| **353** | 1447 |
| **354** | 1944 |
| **355** | 491 |
| **356** | 414 |
| **357** | 1885 |
| **358** | 499 |
| **359** | 3318 |
| **360** | 710 |
| **361** | 597 |
| **362** | 467 |
| **363** | 1902 |
| **364** | 1670 |
| **365** | 501 |
| **366** | 2486 |
| **367** | 204 |
| **368** | 477 |
| **369** | 1163 |
| **370** | 1674 |
| **371** | 1258 |
| **372** | 1242 |
| **373** | 1802 |
| **374** | 754 |
| **375** | 1038 |
| **376** | 886 |
| **377** | 681 |
| **378** | 2016 |
| **379** | 815 |
| **380** | 503 |
| **381** | 707 |
| **382** | 1839 |
| **383** | 385 |
| **384** | 5334 |
| **385** | 574 |
| **386** | 377 |
| **387** | 971 |
| **388** | 362 |
| **389** | 1559 |
| **390** | 575 |
| **391** | 896 |
| **392** | 2181 |
| **393** | 3280 |
| **394** | 2345 |
| **395** | 202 |
| **396** | 384 |
| **397** | 2686 |
| **398** | 2863 |
| **399** | 436 |
| **400** | 1947 |
| **401** | 303 |
| **402** | 2967 |
| **403** | 548 |
| **404** | 1096 |
| **405** | 1005 |
| **406** | 1442 |
| **407** | 806 |
| **408** | 1403 |
| **409** | 740 |
| **410** | 1186 |
| **411** | 1055 |
| **412** | 367 |
| **413** | 1236 |
| **414** | 1607 |
| **415** | 1744 |
| **416** | 1317 |
| **417** | 2386 |
| **418** | 2446 |
| **419** | 1179 |
| **420** | 1057 |
| **421** | 701 |
| **422** | 521 |
| **423** | 1739 |
| **424** | 1606 |
| **425** | 1016 |
| **426** | 6356 |
| **427** | 952 |
| **428** | 913 |
| **429** | 2467 |
| **430** | 1191 |
| **431** | 1889 |
| **432** | 1331 |
| **433** | 894 |
| **434** | 927 |
| **435** | 1257 |
| **436** | 1987 |
| **437** | 2658 |
| **438** | 703 |
| **439** | 1322 |
| **440** | 1410 |
| **441** | 2098 |
| **442** | 1577 |
| **443** | 1179 |
| **444** | 2479 |
| **445** | 937 |
| **446** | 1360 |
| **447** | 2243 |
| **448** | 1164 |
| **449** | 584 |
| **450** | 1353 |
| **451** | 1565 |
| **452** | 2008 |
| **453** | 3068 |
| **454** | 1438 |
| **455** | 1598 |
| **456** | 1508 |
| **457** | 1563 |
| **458** | 1829 |
| **459** | 1356 |
| **460** | 1208 |
| **461** | 889 |
| **462** | 400 |
| **463** | 171 |
| **464** | 49 |
| **465** | 462 |
| **466** | 39 |
| **467** | 215 |
| **468** | 255 |
| **469** | 36 |
| **470** | 372 |
| **471** | 235 |
| **472** | 60 |
| **473** | 1744 |
| **474** | 289 |
| **475** | 142 |
| **476** | 200 |
| **477** | 108 |
| **478** | 172 |
| **479** | 228 |
| **480** | 313 |
| **481** | 1588 |
| **482** | 327 |
| **483** | 191 |
| **484** | 83 |
| **485** | 2467 |
| **486** | 248 |
| **487** | 433 |
| **488** | 254 |
| **489** | 823 |
| **490** | 379 |
| **491** | 281 |
| **492** | 398 |
| **493** | 70 |
| **494** | 521 |
| **495** | 732 |
| **496** | 924 |
| **497** | 84 |
| **498** | 559 |
| **499** | 265 |
| **500** | 396 |
| **501** | 98 |
| **502** | 148 |
| **503** | 70 |
| **504** | 415 |
| **505** | 297 |
| **506** | 1071 |
| **507** | 4089 |
| **508** | 417 |
| **509** | 592 |
| **510** | 322 |
| **511** | 212 |
| **512** | 266 |
| **513** | 158 |
| **514** | 292 |
| **515** | 206 |
| **516** | 430 |
| **517** | 278 |
| **518** | 356 |
| **519** | 253 |
| **520** | 31 |
| **521** | 1309 |
| **522** | 119 |
| **523** | 665 |
| **524** | 1520 |
| **525** | 1205 |
| **526** | 243 |
| **527** | 297 |
| **528** | 67 |
| **529** | 473 |
| **530 2** | 802 |
| **531 1** | 1308 |
| **532** | 990 |
| **533** | 1136 |
| **534** | 217 |
| **535** | 314 |
| **536** | 337 |
| **537** | 4943 |
| **538** | 5741 |
| **539** | 10830 |
| **540** | 1230 |
| **541** | 43 |
| **542** | 979 |
| **543** | 324 |
| **544** | 491 |
| **545** | 184 |
| **546** | 391 |
| **547** | 471 |
| **548** | 750 |
| **549** | 101 |
| **550** | 1916 |
| **551** | 449 |
| **552** | 8.3 |
| **553** | 22 |
| **554** | 1991 |
| **555** | 194 |
| **556** | 214 |
| **557** | 5450 |
| **558** | 3771 |
| **559** | 12 |
| **560** | 221 |
| **561** | 1757 |
| **562** | 125 |
| **563** | 299 |
| **564** | 4293 |
| **565** | 176 |
| **566** | 2209 |
| **567** | 501 |
| **568** | 408 |
| **569** | 462 |
| **570** | 498 |
| **571** | 171 |
| **572** | 348 |
| **573** | 5390 |
| **574** | 586 |
| **575** | 899 |
| **576** | 418 |
| **577** | 4352 |
| **578** | 3513 |
| **579** | 590 |
| **580** | 725 |
| **581** | 1536 |
| **582** | 90 |
| **583** | 1893 |
| **584** | 1517 |
| **585** | 297 |
| **586** | 336 |
| **587** | 1838 |
| **588** | 155 |
| **589** | 6383 |
| **590** | 1850 |
| **591** | 591 |
| **592** | 3060 |
| **593** | 673 |
| **594** | 1602 |
| **595** | 470 |
| **596** | 131 |
| **597** | 1417 |
| **598** | 2942 |
| **599** | 494 |
| **600** | 594 |
| **601** | 497 |
| **602** | 1401 |
| **603** | 380 |
| **604** | 137 |
| **605** | 4231 |
| **606** | 417 |
| **607** | 4557 |
| **608** | 8692 |
| **609** | 484 |
| **610** | 845 |
| **611** | 1386 |
| **612** | 222 |
| **613** | 199 |
| **614** | 1658 |
| **615** | 1249 |
| **616** | 9484 |
| **617** | 552 |
| **618** | 344 |
| **619** | 785 |
| **620** | 595 |
| **621** | 296 |
| **622** | 2019 |
| **623** | 103 |
| **624** | 1272 |
| **625** | 2143 |
| **626** | 4733 |
| **627** | 251 |
| **628** | 293 |
| **629** | 135 |
| **630** | 1194 |
| **631** | 526 |
| **632** | 1410 |
| **633** | 293 |
| **634** | 8612 |
| **635** | 535 |
| **636** | 6264 |
| **637** | 252 |
| **638** | 736 |
| **639** | 1597 |
| **640** | 2592 |
| **641** | 67 |
| **642** | 1145 |
| **643** | 355 |
| **644** | 378 |
| **645** | 436 |
| **646** | 645 |
| **647** | 158 |
| **648** | 286 |
| **649** | 1949 |
| **650** | 2593 |
| **651** | 592 |
| **652** | 1087 |
| **653** | 2306 |
| **654** | 1169 |
| **655** | 1353 |
| **656** | 9173 |
| **657** | 1943 |
| **658** | 2705 |
| **659** | 202 |
| **660** | 3133 |
| **661** | 1824 |
| **662** | 2669 |
| **663** | 2747 |
| **664** | 233 |
| **665** | 863 |
| **666** | 3669 |
| **667** | 442 |
| **668** | 622 |
| **669** | 4884 |
| **670** | 7853 |
| **671** | 1284 |
| **672** | 407 |
| **673** | 1478 |
| **674** | 1366 |
| **675** | 3082 |
| **676** | 174 |
| **677** | 324 |
| **678** | 5196 |
| **679** | 1612 |
| **680** | 5190 |
| **681** | 4642 |
| **682** | 130 |
| **683** | 2548 |
| **684** | 208 |
| **685** | 24080 |
| **686** | 205 |
| **687** | 319 |
| **688** | 908 |
| **689** | 289 |
| **690** | 3811 |
| **691** | 1534 |
| **692** | 954 |
| **693** | 331 |
| **694** | 783 |
| **695** | 108 |
| **696** | 931 |
| **697** | 48 |
| **698** | 1311 |
| **699** | 77 |
| **700** | 17 |
| **701** | 62 |
| **702** | 56 |
| **703** | 937 |
| **704** | 127 |
| **705** | 420 |
| **706** | 335 |
| **707** | 192 |
| **708** | 48 |
| **709** | 368 |
| **710** | 6733 |
| **711** | 8765 |
| **712** | 5196 |
| **713** | 2262 |
| **714** | 2383 |
| **715** | 2719 |
| **716** | 2871 |
| **717** | 1517 |
| **718** | 2149 |
| **719** | 1584 |
| **720** | 1688 |
| **721** | 53 |
| **722** | 824 |
| **723** | 596 |
| **724** | 84 |
| **725** | 728 |
| **726** | 52 |
| **727** | 356 |
| **728** | 9.9 |
| **729** | 17 |
| **730** | 1401 |
| **731** | 1570 |
| **732** | 378 |
| **733** | 902 |
| **734** | 307 |
| **735** | 1308 |
| **736** | 802 |
| **737** | 18 |
| **738** | 160 |
| **739** | 98 |
| **740** | 567 |
| **741** | 135 |
| **742** | 643 |
| **743** | 23 |
| **744** | 1032 |
| **745** | 130 |
| **746** | 4052 |
| **747** | 15 |
| **748** | 12 |
| **749** | 12 |
| **750** | 36 |
| **751** | 1257 |
| **752** | 91 |
| **753** | 360 |
| **754** | 167 |
| **755** | 546 |
| **756** | 9.2 |
| **757** | 6380 |
| **758** | 118 |
| **759** | 15 |
| **760** | 12 |
| **761** | 38 |
| **762** | 92 |
| **763** | 9 |
| **764** | 14 |
| **765** | 25 |
| **766** | 2005 |
| **767** | 375 |
| **768** | 1100 |
| **769** | 1590 |
| **770** | 881 |
| **771** | 600 |
| **772** | 3724 |
| **773** | 2729 |
| **774** | 3264 |
| **775** | 587 |
| **776** | 324 |
| **777** | 463 |
| **778** | 1290 |
| **779** | 3953 |
| **780** | 649 |
| **781** | 2700 |
| **782** | 736 |
| **783** | 370 |
| **784** | 2210 |
| **785** | 470 |
| **786** | 2128 |
| **787** | 1953 |
| **788** | 892 |
| **789** | 13880 |
| **790** | 12060 |
| **791** | 348 |
| **792** | 44 |
| **793** | 44 |
| **794** | 546 |
| **795** | 157 |
| **796** | 3886 |
| **797** | 2093 |
| **798** | 2643 |
| **799** | 688 |
| **800** | 3246 |
| **801** | 1283 |
| **802** | 1160 |
| **803** | 820 |
| **804** | 34 |
| **805** | 9.5 |
| **806** | 446 |
| **807** | 61 |
| **808** | 588 |
| **809** | 289 |
| **810** | 173 |
| **811** | 364 |
| **812** | 127 |
| **813** | 600 |
| **814** | 63 |
| **815** | 204 |
| **816** | 244 |
| **817** | 390 |
| **818** | 1451 |
| **819** | 12 |
| **820** | 1025 |
| **821** | 21 |
| **822** | 383 |
| **823** | 9453 |
| **824** | 13 |
| **825** | 209 |
| **826** | 5.8 |
| **827** | 74 |
| **828** | 285 |
| **829** | 1921 |
| **830** | 11 |
| **831** | 171 |
| **832** | 133 |
| **833** | 8.3 |
| **834** | 39 |
| **835** | 3.2 |
| **836** | 6.5 |
| **837** | 193 |
| **838** | 129 |
| **839** | 6.4 |
| **840** | 123 |
| **841** | 526 |
| **842** | 22 |
| **843** | 409 |
| **844** | 409 |
| **845** | 54 |
| **846** | 580 |
| **847** | 32 |
| **848** | 499 |
| **849** | 81 |
| **850** | 34 |
| **851** | 499 |
| **852** | 10 |
| **853** | 120 |
| **854** | 560 |
| **855** | 8.3 |
| **856** | 4.5 |
| **857** | 784 |
| **858** | 2.5 |
| **859** | 25 |
| **860** | 322 |
| **861** | 5.9 |
| **862** | 690 |
| **863** | 1752 |
| **864** | 4930 |
| **865** | 434 |
| **866** | 17 |
| **867** | 3.1 |
| **868** | 1462 |
| **869** | 1041 |
| **870** | 92 |
| **871** | 805 |
| **872** | 364 |
| **873** | 49 |
| **874** | 14 |
| **875** | 253 |
| **876** | 305 |
| **877** | 44 |
| **878** | 33 |
| **879** | 229 |
| **880** | 608 |
| **881** | 11 |
| **882** | 32 |
| **883** | 32 |
| **884** | 3862 |
| **885** | 3158 |
| **886** | 564 |
| **887** | 35 |
| **888** | 11 |
| **889** | 779 |
| **890** | 37 |
| **891** | 12 |
| **892** | 17 |
| **893** | 894 |
| **894** | 2086 |
| **895** | 6781 |
| **896** | 1741 |
| **897** | 1297 |
| **898** | 1504 |
| **899** | 1938 |
| **900** | 1557 |
| **901** | 2875 |
| **902** | 2200 |
| **903** | 1587 |
| **904** | 1823 |
| **905** | 2176 |
| **906** | 46 |
| **907** | 29 |
| **908** | 371 |
| **909** | 18 |
| **910** | 92 |
| **911** | 58 |
| **912** | 50 |
| **913** | 519 |
| **914** | 1765 |
| **915** | 447 |
| **916** | 147 |
| **917** | 325 |
| **918** | 147 |
| **919** | 267 |
| **920** | 679 |
| **921** | 1290 |
| **922** | 3598 |
| **923** | 1404 |
| **924** | 3179 |
| **925** | 7417 |
| **926** | 6544 |
| **927** | 513 |
| **928** | 110 |
| **929** | 127 |
| **930** | 58 |
| **931** | 63 |
| **932** | 44 |
| **933** | 38 |
| **934** | 190 |
| **935** | 242 |
| **936** | 117 |
| **937** | 121 |
| **938** | 7508 |
| **939** | 147 |
| **940** | 195 |
| **941** | 225 |
| **942** | 339 |
| **943** | 546 |
| **944** | 49 |
| **945** | 365 |
| **946** | 191 |
| **947** | 24 |
| **948** | 45 |
| **949** | 247 |
| **950** | 2423 |
| **951** | 1103 |

## Claims

1. A compound according to Formula (I): 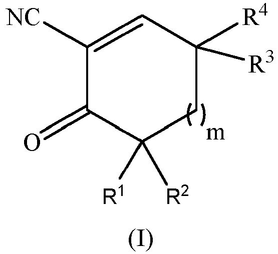
or a pharmaceutically acceptable salt or solvate thereof,
wherein:
R¹ and R² are each independently methyl;
R³ and R⁴ together with the carbon to which they are bonded form a cyclobutyl, cyclopentyl, or
cyclohexyl said cyclobutyl, cyclopentyl and cyclohexyl being optionally substituted with 1-4 substituents R¹²; or
R³ and R⁴ together with the carbon to which they are bonded form an azetidine, a piperidine, a pyrrolidine, a morpholine, a tetrahydrofuran or a tetrahydropyran, said azetidine, piperidine, pyrrolidine, morpholine, tetrahydrofuran and tetrahydropyran being optionally substituted with 1-4 substituents R¹²;
each R¹² is independently selected from:
(1) -C₁₋₆alkyl,
(2) halogen,
(3) oxo,
(4) -(CH₂)ₚC₃₋₆cycloalkyl,
(5) -(CH₂)ₚC₆₋₁₀aryl,
(6) -(CH₂)ₚhet, wherein het denotes a 3- to 9-membered saturated or unsaturated heterocyclic ring comprising 1 to 4 heteroatoms independently selected from O, S and N,
(7) -(CH₂)ₚ-OR¹⁴,
(8) -(CH₂)ₚ-NR¹⁵R¹⁶,
(9) -(CH₂)ₚ-C(O)NR¹⁷R¹⁸,
(10) -(CH₂)ₚ-S(O)ₙNR¹⁷R¹⁸
(11) -(CH₂)ₚ-NR¹⁷C(O)R¹⁸,
(12) -(CH₂)ₚ-NR¹⁷SO₂R¹⁸,
(13) -(CH₂)ₚ-C(O)R¹⁹,
(14) -(CH₂)ₚ-S(O)ₙR¹⁹
(15) -(CH₂)ₚ-C(O)OR²⁰ and
(16) -(CH₂)ₚ-CN;
wherein said C₁₋₆alkyl, C₃₋₆cycloalkyl C₆₋₁₀aryl and 3- to 9-membered heterocylic ring are each independently substituted with one or more substituents selected from halogen, hydroxyl, C₁₋₆alkyl, -OC₁₋₆alkyl and -SC₁₋₆alkyl and wherein said -C₁₋₆alkyl, -C₃₋₆cycloalkyl, -OC₁-₆alkyl and -SC₁₋₆alkyl are optionally substituted with one or more halogens or one or more hydroxyls;
or two R¹² substituents on adjacent atoms of the cyclobutyl, cyclopentyl, cyclohexyl, azetidine, piperidine, pyrrolidine, morpholine, tetrahydrofuran or tetrahydropyran ring together with the ring atoms to which they are bonded form a fused bicyclic ring system which is optionally substituted with one or more substituents R²¹;
R¹⁴ is H, -C₁₋₆alkyl or -C₃₋₈cycloalkyl, said -C₁₋₆alkyl and -C₃₋₈cycloalkyl being optionally substituted with one or more halogens;
R¹⁵ is H or -C₁₋₆alkyl optionally substituted with one or more halogens;
R¹⁶ is H, C₁₋₆alkyl, C₃₋₆cycloalkyl, C6-10aryl or a 5- to 8-membered heteroaryl comprising 1 to 3 heteroatoms independently selected from O, S and N, wherein said C₁₋₆alkyl, C₃₋₆cycloalkyl C6-10aryl and 5- to 8-membered heteroaryl are each independently optionally substituted with one or more substituents selected from halogen, hydroxyl, C₁₋₆alkyl and -OC₁₋₆alkyl;
R¹⁷ is H or C₁₋₆alkyl optionally substituted with one or more halogens;
R¹⁸, R¹⁹ and R²⁰ are each independently H, -C₁₋₆alkyl, -C₂₋₆alkenyl, -C₂₋₆alkynyl, -C₃₋₆cycloalkyl -C₆₋₁₂aryl or a 5- to 10-membered heteroaryl comprising 1 to 3 heteroatoms independently selected from O, S and N, wherein said -C₁₋₆alkyl, -C₂₋₆alkenyl, -C₂₋₆alkynyl, - C₃₋₆cycloalkyl -C₆₋₁₂aryl and 5- to 10-membered heteroaryl are each optionally independently substituted with one or more substituents R²⁷;
each R²¹ is independently selected from H, halogen, hydroxyl, -C₁₋₆alkyl and -OC₁₋₆alkyl, wherein said -C₁₋₆alkyl and -OC₁₋₆alkyl are each optionally independently substituted with one or more substituents selected from halogen, hydroxyl and -OC₁₋₆alkyl;
R²⁷ is halogen, hydroxyl, nitrile, -C₁₋₆alkyl, -O-C₁₋₆alkyl, -S-C₁₋₆alkyl, C(O)NHC₁₋₆alkyl, -C₃₋₆cycloalkyl, -C₆₋₁₀aryl, -O-C₆₋₁₀aryl, -O-C₁₋₂alkylC₆₋₁₀aryl or a 5- to 8-membered heteroaryl comprising 1 to 3 heteroatoms independently selected from O, S and N, wherein each occurrence of said -C₁₋₆alkyl, -C₃₋₆cycloalkyl -C₆₋₁₀aryl and 5- to 8-membered heteroaryl are independently substituted with one or more substituents selected from halogen, hydroxyl, nitrile, -C₁₋₆alkyl, -OC₁₋₆alkyl, and -N(C₁₋₆alkyl)₂ wherein said -C₁₋₆alkyl and -OC₁₋₆alkyl are each independently substituted with one or more halogen;
m is 0, 1 or 2;
each n is independently 0, 1 or 2 and
each p is independently 0, 1 or 2.

2. The compound according to claim 1, or a pharmaceutically acceptable salt or solvate thereof, wherein R³ and R⁴ together with the carbon to which they are bonded form a cyclobutyl, cyclopentyl, or cyclohexyl, said cyclobutyl, cyclopentyl and cyclohexyl being optionally substituted with 1-4 substituents R¹².

3. The compound according to claim 1, or a pharmaceutically acceptable salt or solvate thereof, wherein R³ and R⁴ together with the carbon to which they are bonded form an azetidine, a piperidine, a pyrrolidine, a morpholine, a tetrahydrofuran or a tetrahydropyran, said azetidine, piperidine, pyrrolidine, morpholine, tetrahydrofuran and tetrahydropyran being optionally substituted with 1-4 substituents R¹².

4. The compound according to claim 1, or a pharmaceutically acceptable salt or solvate thereof, wherein R³ and R⁴ together with the carbon to which they are bonded form a spirocyclic carbocyclic or heterocyclic ring selected from: 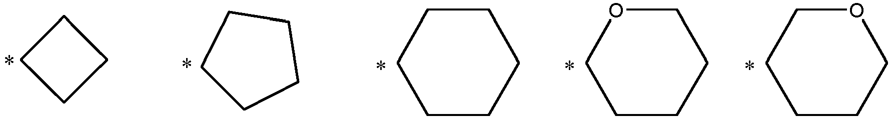 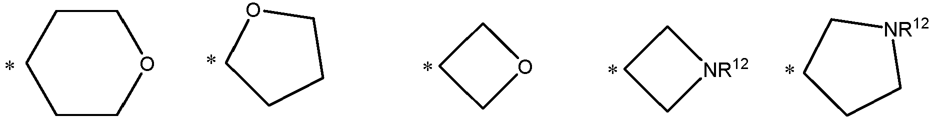 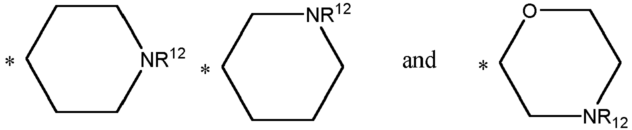 wherein the asterisk denotes the point of attachment of the spirocyclic carbocyclic or heterocyclic ring with the cyanoenone, said spirocyclic carbocyclic or heterocyclic ring being optionally substituted with 1-4 substituents R¹².

5. The compound according to claim 1, or a pharmaceutically acceptable salt or solvate thereof, having formula Ia -Id 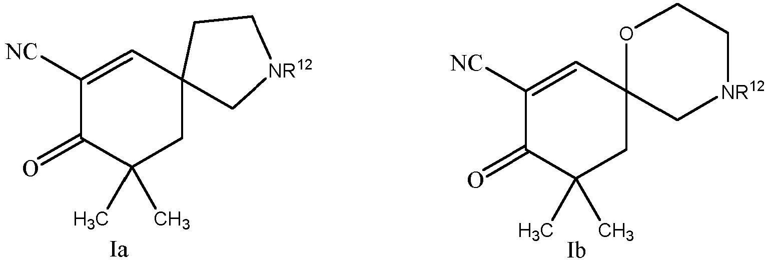 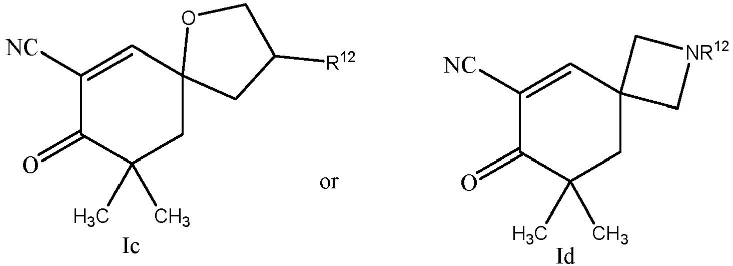 wherein, R¹² is selected from hydroxyl, -C₁₋₆alkyl, -C(O)O-C₁₋₆alkyl, -C(O)-C₁₋₆alkyl, -C(O)-C₃₋₆cycloalkyl, -C(0)-C6-10aryl, -C(O)-het, -S(O)₂-C₆₋₁₀aryl, -C₆₋₁₀aryl, het, -CH₂-C₆₋₁₀aryl, - CH₂-C₃₋₆cycloalkyl and -CH₂-het, wherein het represents a 3- to 9-membered saturated or unsaturated heterocyclic ring comprising 1 to 3 heteroatoms independently selected from O, S and N, wherein each occurrence of said C₁₋₆alkyl, C₃₋₆cycloalkyl, C₆₋₁₀aryl and 3- to 9-membered heterocyclic ring is optionally independently substituted with one or more substituents selected from halogen, hydroxyl, C₁₋₆alkyl, -C1-6haloalkyl, -C1-6hydroxyalkyl, - O-C₁₋₆alkyl and -S-C1 ₋₆alkyl.

6. The compound according to claim 1, or a pharmaceutically acceptable salt or solvate thereof, having formula IIa. 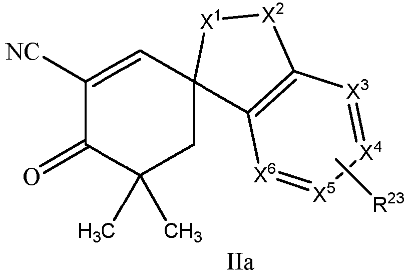 wherein,
X¹ is O or CR^{12a}R^{12a};
X² is CR^{12a}R^{12a}, C(O) or NR^{12a};
X³, X⁴, X⁵ and X⁶ are each independently N or CR²³, with the proviso that no more than 2 of X³-X⁶ can be N;
each R^{12a} is independently H, -C(O)C₁₋₆alkyl, or-C₁₋₆alkyl, optionally substituted with hydroxyl and
each R²³ is independently H, halogen, -C₁₋₆alkyl or -C₁₋₆haloalkyl.

7. The compound according to any of claims 1-6 which is selected from: 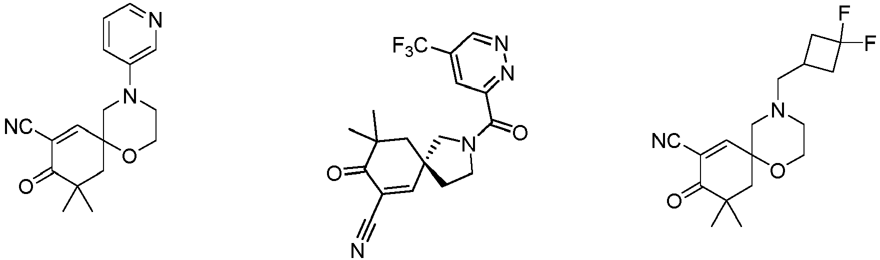 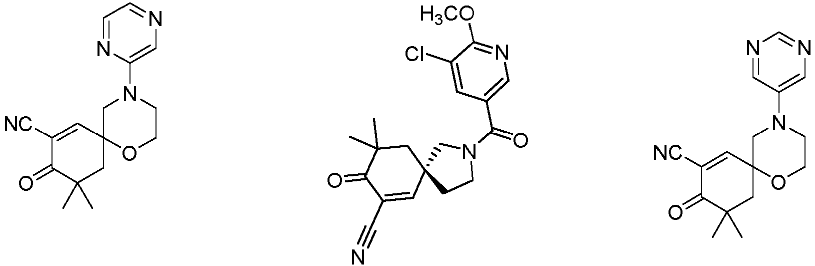 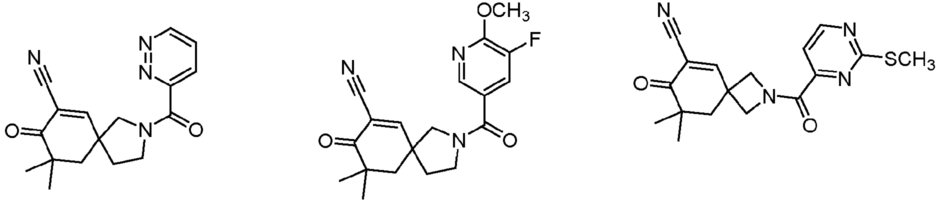 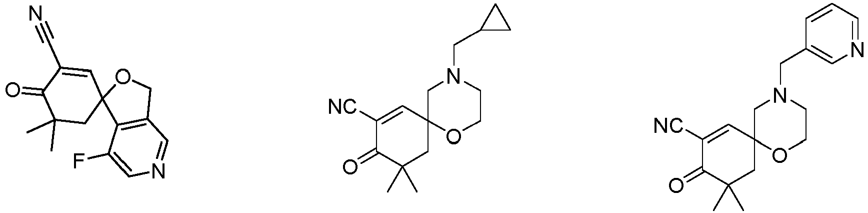 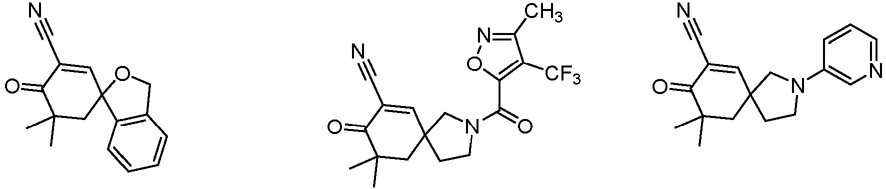 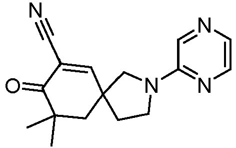 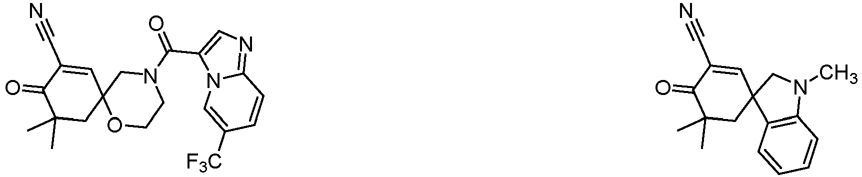 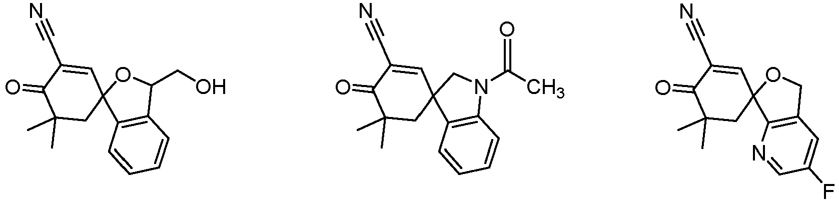 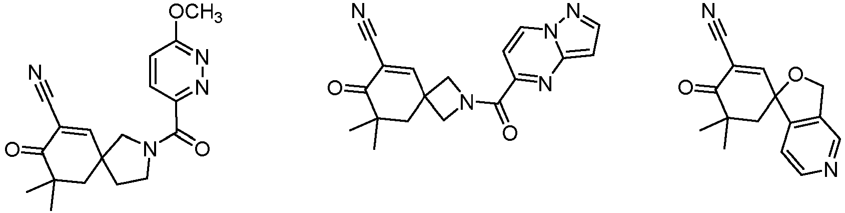 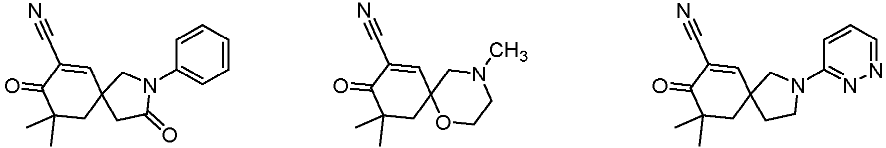 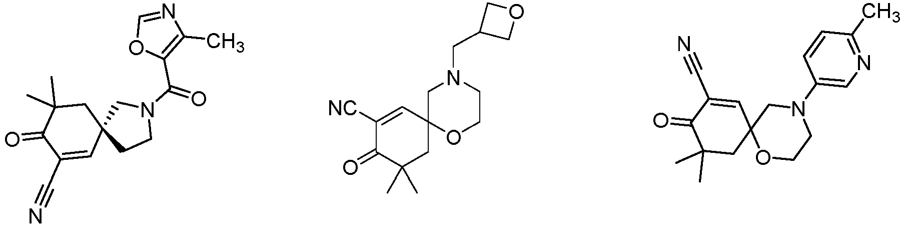 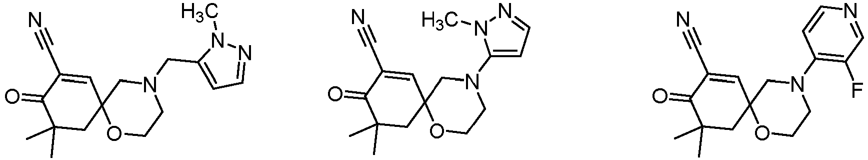 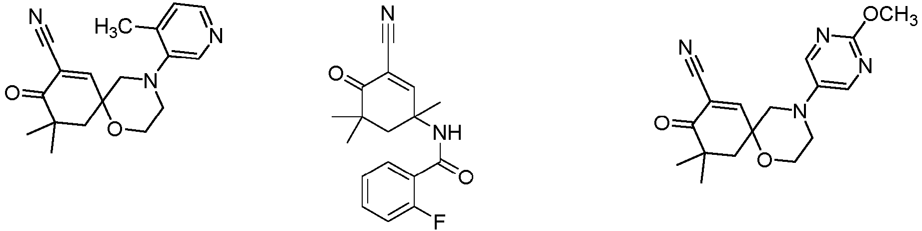 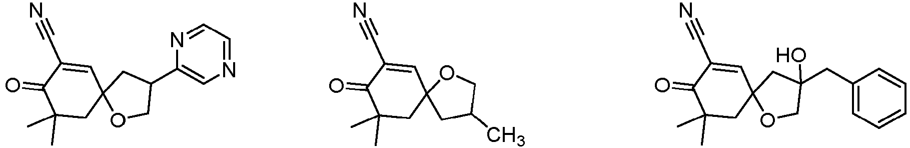 and 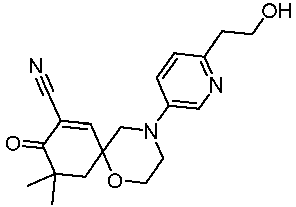 or a pharmaceutically acceptable salt or solvate thereof.

8. A pharmaceutical composition comprising a compound according to any of claims 1-7, or a pharmaceutically acceptable salt or solvate thereof, together with one or more pharmaceutically acceptable excipients.

9. A compound according to any of claims 1-7, or a pharmaceutically acceptable salt or solvate thereof, for use in therapy.

10. A compound according to any of claims 1-7, or a pharmaceutically acceptable salt or solvate thereof, for use in the treatment of a neurodegenerative disease selected from Freidreich's ataxia, amyotrophic lateral sclerosis, stroke, Alzheimer's disease, Parkinson's disease, Huntington's disease, peripheral neuropathy and hearing loss in a patient.

11. A combination comprising a compound according to any of claims 1-7, or a pharmaceutically acceptable salt or solvate thereof, and one or more other therapeutic agents.

## Patentansprüche

1. Eine Verbindung gemäß der Formel (I): 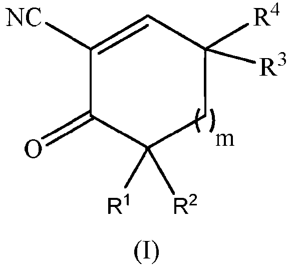
oder ein pharmazeutisch annehmbares Salz oder Solvat davon,
wobei:
R¹ und R² jeweils unabhängig Methyl sind,
R³ und R⁴ zusammen mit dem Kohlenstoff, an den sie gebunden sind, ein Cyclobutyl, Cyclopentyl oder Cyclohexyl bilden, wobei das Cyclobutyl, Cyclopentyl und Cyclohexyl gegebenenfalls substituiert ist mit 1 - 4 Substituenten R¹², oder
R³ und R⁴ zusammen mit dem Kohlenstoff, an den sie gebunden sind, ein Azetidin, ein Piperidin, ein Pyrrolidin, ein Morpholin, ein Tetrahydrofuran oder ein Tetrahydropyran bilden, wobei das Azetidin, Piperidin, Pyrrolidin, Morpholin, Tetrahydrofuran oder Tetrahydropyran gegebenenfalls substituiert ist mit 1 - 4 Substituenten R¹²,
jedes R¹² unabhängig ausgewählt ist aus:
(1) -C₁₋₆-Alkyl,
(2) Halogen,
(3) Oxo,
(4) -(CH₂)ₚC₃₋₆-Cycloalkyl,
(5) -(CH₂)ₚC₆₋₁₀-Aryl,
(6) -(CH₂)ₚHet, wobei Het für einen 3- bis 9-gliedrigen gesättigten oder ungesättigten heterocyclischen Ring steht, der 1 bis 4 Heteroatome, unabhängig ausgewählt aus O, S und N, umfasst,
(7) -(CH₂)ₚ-OR¹⁴,
(8) -(CH₂)ₚ-NR¹⁵R¹⁶,
(9) -(CH₂)ₚ-C(O)NR¹⁷R¹⁸,
(10) -(CH₂)ₚ-S(O)ₙNR¹⁷R¹⁸,
(11) -(CH₂)ₚ-NR¹⁷C(O)R¹⁸,
(12) -(CH₂)ₚ-NR¹⁷SO₂R¹⁸,
(13) -(CH₂)ₚ-C(O)R¹⁹,
(14) -(CH₂)ₚ-S(O)ₙR¹⁹,
(15) -(CH₂)ₚ-C(O)OR²⁰ und
(16) -(CH₂)ₚ-CN,
wobei das C₁₋₆-Alkyl, C₃₋₆-Cycloalkyl, C₆₋₁₀-Aryl und der 3- bis 9-gliedrige heterocyclische Ring jeweils unabhängig substituiert sind mit einem oder mehreren Substituenten, ausgewählt aus Halogen, Hydroxyl, C₁₋₆-Alkyl, -OC₁₋₆-Alkyl und -SC₁₋₆-Alkyl, und wobei das
-C₁₋₆-Alkyl, -C₃₋₆-Cycloalkyl, -OC₁₋₆-Alkyl und -SC₁₋₆-Alkyl gegebenenfalls substituiert sind mit einem oder mehreren Halogenen oder einem oder mehreren Hydroxyl,
oder zwei R¹²-Substituenten an benachbarten Atomen des Cyclobutyl-, Cyclopentyl-, Cyclohexyl-, Azetidin-, Piperidin-, Pyrrolidin-, Morpholin-, Tetrahydrofuran- oder Tetrahydropyranrings zusammen mit den Ringatomen, an die sie gebunden sind, ein kondensiertes bicyclisches Ringsystem bilden, das gegebenenfalls substituiert ist mit einem oder mehreren Substituenten R²¹,
R¹⁴ H, -C₁₋₆-Alkl oder -C₃₋₈-Cycloalkyl ist, wobei das -C₁₋₆-Alkyl und -C₃₋₈-Cycloalkyl gegebenenfalls substituiert ist mit einem oder mehreren Halogenen,
R¹⁵ H oder -C₁₋₆-Alkyl ist, gegebenenfalls substituiert mit einem oder mehreren Halogenen, R¹⁶ H, C₁₋₆-Alkyl, C₃₋₆-Cycloalkyl, C₆₋₁₀-Aryl oder ein 5- bis 8-gliedriges Heteroaryl, das 1 bis 3 Heteroatome, unabhängig ausgewählt aus O, S und N, umfasst, ist, wobei das C₁₋₆-Alkyl, C₃₋₆-Cycloalkyl, C₆₋₁₀-Aryl und das 5- bis 8-gliedrige Heteroaryl jeweils unabhängig gegebenenfalls substituiert sind mit einem oder mehreren Substituenten, ausgewählt aus Halogen, Hydroxyl, C₁₋₆-Alkyl und -OC₁₋₆-Alkyl,
R¹⁷ H oder C₁₋₆-Alkyl ist, gegebenenfalls substituiert mit einem oder mehreren Halogenen, R¹⁸, R¹⁹ und R²⁰ jeweils unabhängig H, -C₁₋₆-Alkyl, -C₂₋₆-Alkenyl, -C₂₋₆-Alkinyl, -C₃₋₆-Cycloalkyl, - C₆₋₁₂-Aryl oder ein 5- bis 10-gliedriges Heteroaryl, das 1 bis 3 Heteroatome, unabhängig ausgewählt aus O, S und N, umfasst, sind, wobei das -C₁₋₆-Alkyl, -C₂₋₆-Alkenyl, -C₂₋₆-Alkinyl, -C₃₋₆-Cycloalkyl, -C₆₋₁₂-Aryl und das 5- bis 10-gliedrige Heteroaryl jeweils gegebenenfalls unabhängig substituiert sind mit einem oder mehreren Substituenten R²⁷, jedes R²¹ unabhängig ausgewählt ist aus H, Halogen, Hydroxyl, -C₁₋₆-Alkyl und -OC₁₋₆-Alkyl, wobei das -C₁₋₆-Alkyl und -OC₁₋₆-Alkyl jeweils gegebenenfalls unabhängig substituiert sind mit einem oder mehreren Substituenten, ausgewählt aus Halogen, Hydroxyl und -OC₁₋₆-Alkyl, R²⁷ Halogen, Hydroxyl, Nitril, -C₁₋₆-Alkyl, -O-C₁₋₆-Alkyl, -S-C₁₋₆-Alkyl, C(O)NHC₁₋₆-Alkyl, -C₃₋₆-Cycloalkyl, -C₆₋₁₀-Aryl, -O-C₆₋₁₀-Aryl, -O-C₁₋₂-Alkyl-C₆₋₁₀-aryl oder ein 5- bis 8-gliedriges Heteroaryl, das 1 bis 3 Heteroatome, unabhängig ausgewählt aus O, S und N, umfasst, ist, wobei das -C₁₋₆-Alkyl, -C₃₋₆-Cycloalkyl, -C₆₋₁₀-Aryl und 5- bis 8-gliedrige Heteroaryl bei jedem Vorkommen unabhängig substituiert sind mit einem oder mehreren Substituenten, ausgewählt aus Halogen, Hydroxyl, Nitril, -C₁₋₆-Alkyl, -OC₁₋₆-Alkyl und -N(C₁₋₆-Alkyl)₂, wobei das -C₁₋₆-Alkyl und -OC₁₋₆-Alkyl jeweils unabhängig substituiert sind mit einem oder mehreren Halogen,
m 0, 1 oder 2 ist,
jedes n unabhängig 0, 1 oder 2 ist, und
jedes p unabhängig 0, 1 oder 2 ist.

2. Die Verbindung gemäß Anspruch 1 oder ein pharmazeutisch annehmbares Salz oder Solvat davon, wobei R³ und R⁴ zusammen mit dem Kohlenstoff, an den sie gebunden sind, ein Cyclobutyl, Cyclopentyl oder Cyclohexyl bilden, wobei das Cyclobutyl, Cyclopentyl und Cyclohexyl gegebenenfalls substituiert sind mit 1 - 4 Substituenten R¹².

3. Die Verbindung gemäß Anspruch 1 oder ein pharmazeutisch annehmbares Salz oder Solvat davon, wobei R³ und R⁴ zusammen mit dem Kohlenstoff, an den sie gebunden sind, ein Azetidin, ein Piperidin, ein Pyrrolidin, ein Morpholin, ein Tetrahydrofuran oder ein Tetrahydropyran bilden, wobei das Azetidin, Piperidin, Pyrrolidin, Morpholin, Tetrahydrofuran und Tetrahydropyran gegebenenfalls substituiert sind mit 1 - 4 Substituenten R¹².

4. Die Verbindung gemäß Anspruch 1 oder ein pharmazeutisch annehmbares Salz oder Solvat davon, wobei R³ und R⁴ zusammen mit dem Kohlenstoff, an den sie gebunden sind, einen spirocyclischen carbocyclischen oder heterocyclischen Ring bilden, ausgewählt aus: 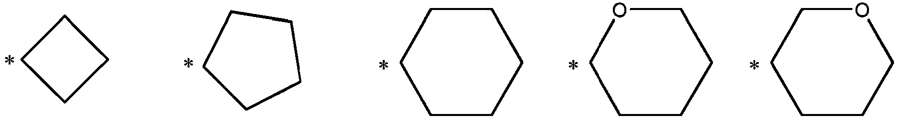 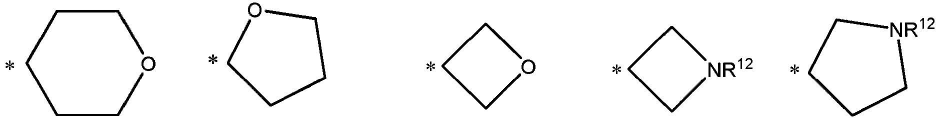 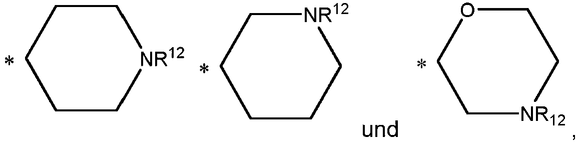 wobei das Sternchen den Verknüpfungspunkt des spirocyclischen carbocyclischen oder heterocyclischen Rings mit dem Cyanoenon bedeutet, wobei der spirocyclische carbocyclische oder heterocyclische Ring gegebenenfalls substituiert ist mit 1 - 4 Substituenten R¹².

5. Die Verbindung gemäß Anspruch 1, oder ein pharmazeutisch annehmbares Salz oder Solvat davon, mit der Formel la - Id 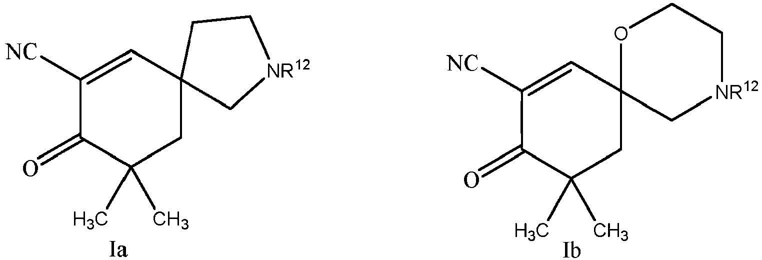 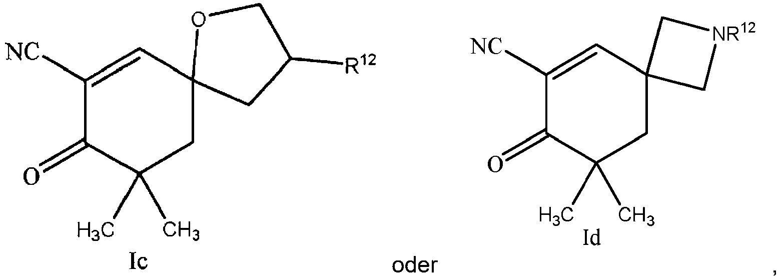 wobei
R¹² ausgewählt ist aus Hydroxyl, -C₁₋₆-Alkyl, -C(O)O-C₁₋₆-Alkyl, -C(O)-C₁₋₆-Alkyl, -C(O)-C₃₋₆-Cycloalky, -C(O)-C₆₋₁₀-Aryl, -(O)-Het, -S(O)₂-C₆₋₁₀-Aryl, -C₆₋₁₀-Aryl, Het, -CH₂-C₆₋₁₀-Aryl, -CH₂-C₃₋₆-Cycloalkyl und -CH₂-Het, wobei Het einen 3- bis 9-gliedrigen gesättigten oder ungesättigten heterocyclischen Ring bedeutet, der 1 bis 3 Heteroatome, unabhängig ausgewählt aus O, S und N, umfasst, wobei das C₁₋₆-Alkyl, C₃₋₆-Cycloalkyl, C₆₋₁₀-Aryl und der 3-bis 9-gliedrige heterocyclische Ring bei jedem Vorkommen gegebenenfalls unabhängig substituiert sind mit einem oder mehreren Substituenten, ausgewählt aus Halogen, Hydroxyl, C₁₋₆-Alkyl, -C₁₋₆-Halogenalkyl, -C₁₋₆-Hydroxyalkyl, -O-C₁₋₆-Alkyl und -S-C₁₋₆-Alkyl.

6. Die Verbindung gemäß Anspruch 1, oder ein pharmazeutisch annehmbares Salz oder Solvat davon, mit der Formel IIa 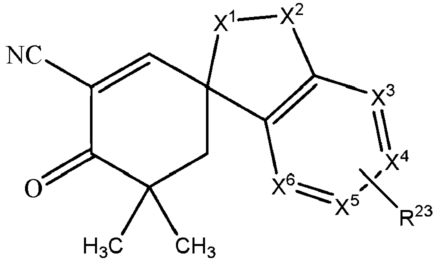 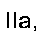 wobei
X¹ O oder CR^{12a}R^{12a} ist,
X² CR^{12a}R^{12a}, C(O) oder NR^{12a} ist,
X³, X⁴, X⁵ und X⁶ jeweils unabhängig N oder CR²³ sind, mit der Maßgabe, dass nicht mehr als 2 von X³ - X⁶ N sein können,
jedes R^{12a} unabhängig H, -C(O)C₁₋₆-Alkyl oder -C₁₋₆-Alkyl ist, gegebenenfalls substituiert mit Hydroxyl, und
jedes R²³ unabhängig H, Halogen, -C₁₋₆-Alkyl oder -C₁₋₆-Halogenalkyl ist.

7. Die Verbindung gemäß einem der Ansprüche 1 - 6, ausgewählt aus: 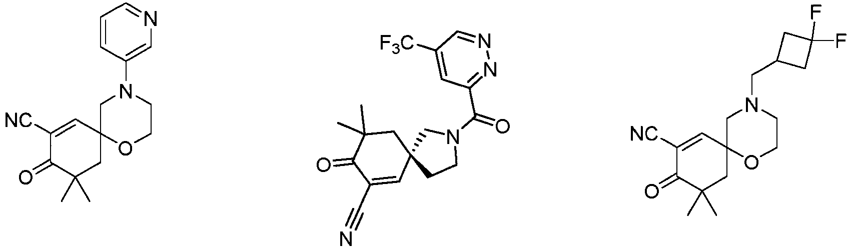 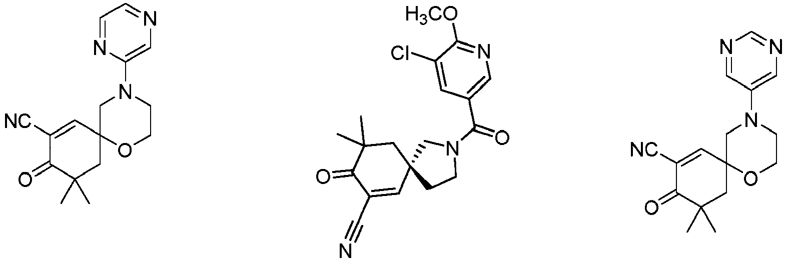 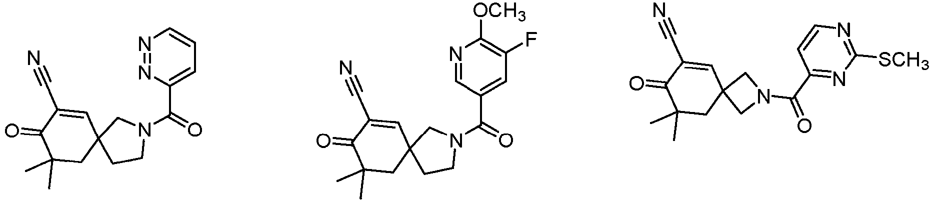 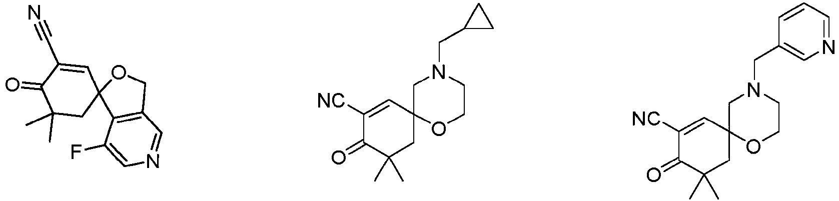 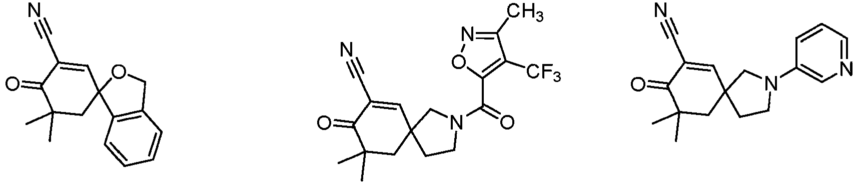 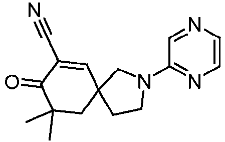 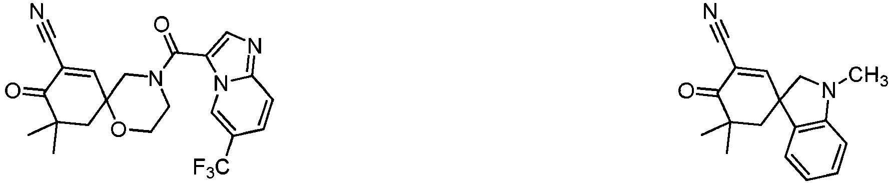 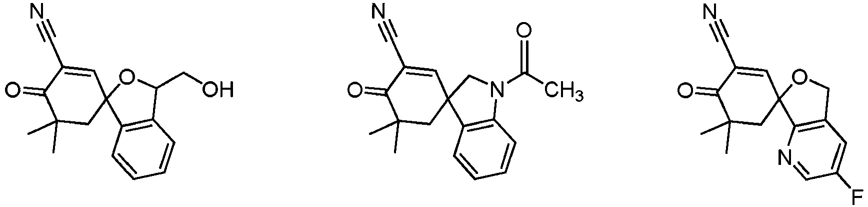 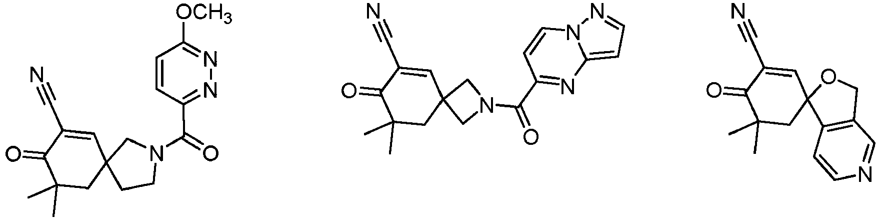 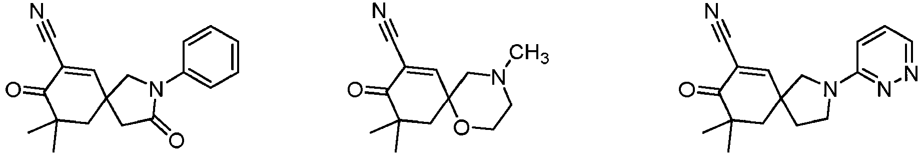 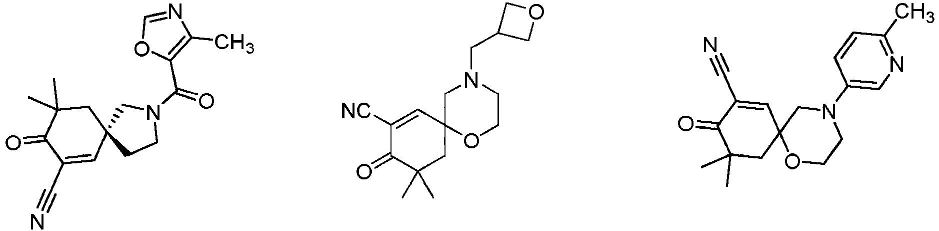 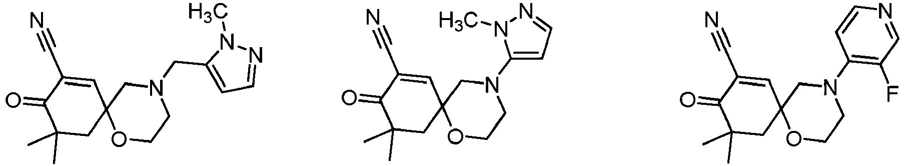 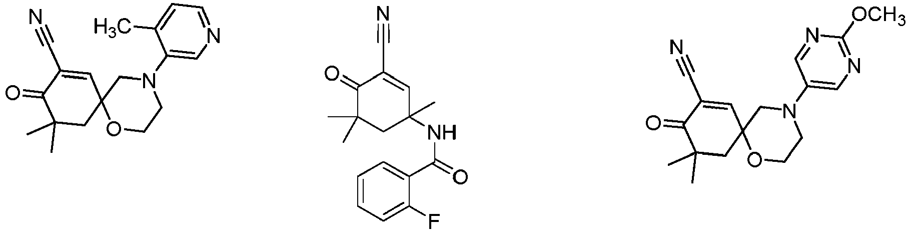 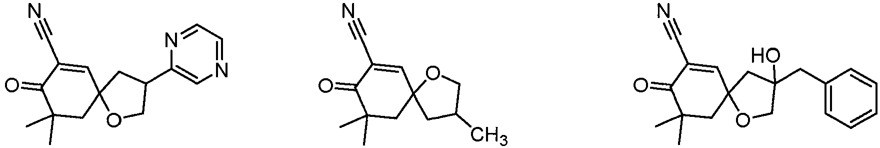 und 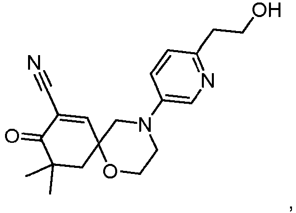 oder ein pharmazeutisch annehmbares Salz oder Solvat davon.

8. Eine pharmazeutische Zusammensetzung, die eine Verbindung gemäß einem der Ansprüche 1 - 7 oder ein pharmazeutisch annehmbares Salz oder Solvat davon zusammen mit einem oder mehreren pharmazeutisch annehmbaren Hilfsstoffen umfasst.

9. Eine Verbindung gemäß einem der Ansprüche 1 - 7 oder ein pharmazeutisch annehmbares Salz oder Solvat davon zur Verwendung in der Therapie.

10. Eine Verbindung gemäß einem der Ansprüche 1 - 7 oder ein pharmazeutisch annehmbares Salz oder Solvat davon zur Verwendung bei der Behandlung einer neurodegenerativen Erkrankung, ausgewählt aus Friedreich-Ataxie, amyotropher Lateralsklerose, Schlaganfall, Alzheimer-Krankheit, Parkinson-Krankheit, Huntington-Krankheit, peripherer Neuropathie und Hörverlust bei einem Patienten.

11. Eine Kombination, die eine Verbindung gemäß einem der Ansprüche 1 - 7 oder ein pharmazeutisch annehmbares Salz oder Solvat davon und ein oder mehrere therapeutische Mittel umfasst.

## Revendications

1. Composé selon la formule (I) : 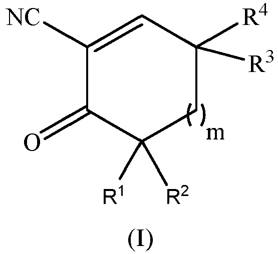
ou sel ou solvate pharmaceutiquement acceptable de celui-ci,
dans lequel :
R¹ et R² sont chacun indépendamment méthyle ;
R³ et R⁴ conjointement avec le carbone auquel ils sont liés forment un cyclobutyle, cyclopentyle ou cyclohexyle, lesdits cyclobutyle, cyclopentyle et cyclohexyle étant éventuellement substitués par 1 à 4 substituants R¹² ; ou
R³ et R⁴ conjointement avec le carbone auquel ils sont liés forment un azétidine, une pipéridine, une pyrrolidine, une morpholine, un tétrahydrofurane ou un tétrahydropyrane, lesdits azétidine, pipéridine, pyrrolidine, morpholine, tétrahydrofurane et tétrahydropyrane étant éventuellement substitués par 1 à 4 substituants R¹² ;
chaque R¹² est indépendamment choisi parmi :
(1) -C₁₋₆alkyle,
(2) halogène,
(3) oxo,
(4) - (CH₂)ₚC₃₋₆cycloalkyle,
(5) - (CH₂)ₚC₆₋₁₀aryle,
(6) - (CH₂)ₚhet, dans lequel het désigne un cycle hétérocyclique à 3 à 9 chaînons saturé ou insaturé comprenant 1 à 4 hétéroatomes indépendamment choisis parmi O, S et N,
(7) - (CH₂)ₚ-OR¹⁴,
(8) - (CH₂)ₚ-NR¹⁵R¹⁶,
(9) -(CH₂)ₚ-C(O)NR¹⁷R¹⁸,
(10) -(CH₂)ₚ-S(O)ₙNR¹⁷R¹⁸
(11) -(CH₂)ₚ-NR¹⁷C(O)R¹⁸,
(12) -(CH₂)p-NR¹⁷SO₂R¹⁸,
(13) -(CH₂)ₚ-C(O)R¹⁹,
(14) -(CH₂)ₚ-S(O)ₙR¹⁹
(15) -(CH₂)ₚ-C(O)OR²⁰ et
(16) -(CH₂)ₚ-CN ;
dans lequel lesdits C₁₋₆alkyle, C₃₋₆cycloalkyle, C₆₋₁₀aryle et cycle hétérocyclique à 3 à 9 chaînons sont chacun indépendamment substitués par un ou plusieurs substituants choisis parmi halogène, hydroxyle, C₁₋₆alkyle, -OC₁₋₆alkyle et -SC₁₋₆alkyle et dans lequel lesdits -C₁₋₆alkyle, -C₃₋₆cycloalkyle, -OC₁₋₆alkyle et -SC₁₋₆alkyle sont éventuellement substitués par un ou plusieurs halogènes ou un ou plusieurs hydroxyles ;
ou deux substituants R¹² sur des atomes adjacents du cycle cyclobutyle, cyclopentyle, cyclohexyle, azétidine, pipéridine, pyrrolidine, morpholine, tétrahydrofurane ou tétrahydropyrane conjointement avec les atomes de cycles auxquels ils sont liés forment un système cyclique bicyclique condensé qui est éventuellement substitué par un ou plusieurs substituants R²¹ ;
R¹⁴ est H, -C₁₋₆alkyle ou -C₃₋₈cycloalkyle, lesdits -C₁₋₆alkyle et -C₃₋₈cycloalkyle étant éventuellement substitués par un ou plusieurs halogènes ;
R¹⁵ est H ou -C₁₋₆alkyle éventuellement substitué par un ou plusieurs halogènes ;
R¹⁶ est H, C₁₋₆alkyle, C₃₋₆cycloalkyle, C₆₋₁₀aryle ou un hétéroaryle à 5 à 8 chaînons comprenant 1 à 3 hétéroatomes indépendamment choisis parmi O, S et N, dans lequel lesdits C₁₋₆alkyle, C₃₋₆cycloalkyle C₆₋₁₀aryle et hétéroaryle à 5 à 8 chaînons sont chacun indépendamment éventuellement substitués par un ou plusieurs substituants choisis parmi halogène, hydroxyle, C₁₋₆alkyle et -OC₁₋₆alkyle ;
R¹⁷ est H ou C₁₋₆alkyle éventuellement substitué par un ou plusieurs halogènes ;
R¹⁸, R¹⁹ et R²⁰ sont chacun indépendamment H, -C₁₋₆alkyle, -C₂₋₆alcényle, -C₂₋₆alcynyle, -C₃₋₆cycloalkyle -C₆₋₁₂aryle ou un hétéroaryle à 5 à 10 chaînons comprenant 1 à 3 hétéroatomes indépendamment choisis parmi O, S et N, dans lequel lesdits -C₁₋₆alkyle, -C₂₋₆alcényle, -C₂₋₆alcynyle, -C₃₋₆cycloalkyle -C₆₋₁₂aryle et hétéroaryle 5 à 10 chaînons sont chacun éventuellement indépendamment substitués par un ou plusieurs substituants R²⁷ ;
chaque R²¹ est indépendamment choisi parmi H, halogène, hydroxyle, -C₁₋₆alkyle et -OC₁₋₆alkyle, dans lequel lesdits -C₁₋₆alkyle et -OC₁₋₆alkyle étant chacun éventuellement indépendamment substitués par un ou plusieurs substituants choisis parmi halogène, hydroxyle et -OC₁₋₆alkyle ;
R²⁷ est halogène, hydroxyle, nitrile, -C₁₋₆alkyle, -O-C₁₋₆alkyle, -S-C₁₋₆alkyle, C(O)NHC₁₋₆alkyle, -C₃₋₆cycloalkyle, -C₆₋₁₀aryle, -O-C₆₋₁₀aryle, -O-C₁₋₂alkyle C₆₋₁₀aryle ou un hétéroaryle à 5 à 8 chaînons comprenant 1 à 3 hétéroatomes indépendamment choisis parmi O, S et N, dans lequel chaque occurrence desdits -C₁₋₆alkyle, -C₃₋₆cycloalkyle -C₆₋₁₀aryle et hétéroaryle à 5 à 8 chaînons est indépendamment substitué par un ou plusieurs substituants choisis parmi halogène, hydroxyle, nitrile, -C₁₋₆alkyle, -OC₁₋₆alkyle et -N(C₁₋₆alkyl)₂, dans lequel lesdits -C₁₋₆alkyle et -OC₁₋₆alkyle sont chacun indépendamment substitués par un ou plusieurs halogènes ;
m est 0, 1 ou 2 ;
chaque n est indépendamment 0, 1 ou 2 et
chaque p est indépendamment 0, 1 ou 2.

2. Composé selon la revendication 1, ou sel ou solvate pharmaceutiquement acceptable de celui-ci, dans lequel R³ et R⁴ conjointement avec le carbone auquel ils sont liés forment un cyclobutyle, cyclopentyle ou cyclohexyle, lesdits cyclobutyle, cyclopentyle et cyclohexyle étant éventuellement substitués par 1 à 4 substituants R¹².

3. Composé selon la revendication 1, ou sel ou solvate pharmaceutiquement acceptable de celui-ci, dans lequel R³ et R⁴ conjointement avec le carbone auquel ils sont liés forment une azétidine, une pipéridine, une pyrrolidine, une morpholine, un tétrahydrofurane ou un tétrahydropyrane, lesdits azétidine, pipéridine, pyrrolidine, morpholine, tétrahydrofurane et tétrahydropyrane étant éventuellement substitués par 1 à 4 substituants R¹².

4. Composé selon la revendication 1, ou sel ou solvate pharmaceutiquement acceptable de celui-ci, dans lequel R³ et R⁴ conjointement avec le carbone auquel ils sont liés forment un cycle spirocyclique, carbocyclique ou hétérocyclique choisi parmi : 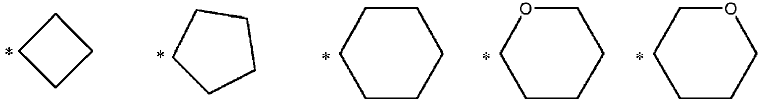 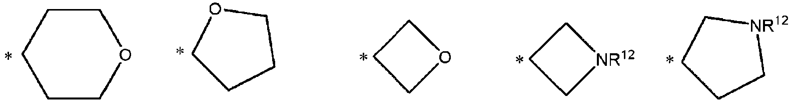 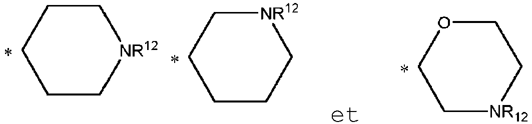 dans lequel l'astérisque désigne le point de fixation au cycle spirocyclique, carbocyclique ou hétérocyclique avec la cyanoénone, ledit cycle spirocyclique, carbocyclique ou hétérocyclique étant éventuellement substitué par 1 à 4 substituants R¹².

5. Composé selon la revendication 1, ou sel ou solvate pharmaceutiquement acceptable de celui-ci, ayant une formule Ia-Id 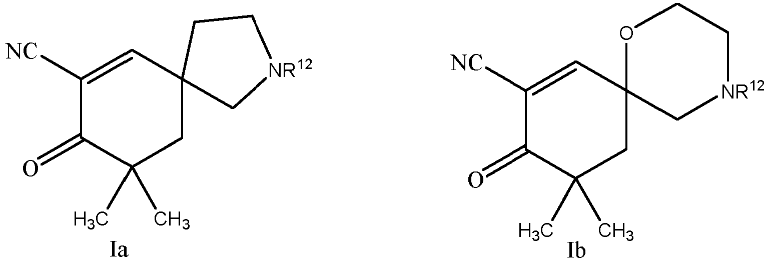 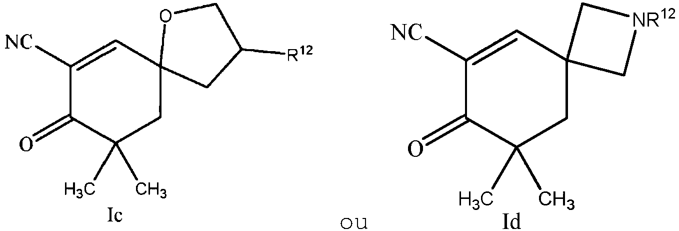 dans lequel,
R¹² est choisi parmi hydroxyle, -C₁₋₆alkyle, -C(O)O-C₁₋₆alkyle, -C (O)-C₁₋₆alkyle, -C (O)-C₃₋₆cycloalkyle, -C (O)-C₆₋₁₀aryle, -C(O)-het, -S(O)₂-C₆₋₁₀aryle, -C₆₋₁₀aryle, het, - CH₂-C₆₋₁₀aryle, -CH₂C₃₋₆cycloalkyle et -CH₂-het, dans lequel het représente un cycle hétérocyclique à 3 à 9 chaînons saturé ou insaturé comprenant 1 à 3 hétéroatomes indépendamment choisis parmi O, S et N, dans lequel chaque occurrence desdits C₁₋₆alkyle, C₃₋₆cycloalkyle, C₆₋₁₀aryle et cycle hétérocyclique à 3 à 9 chaînons est éventuellement indépendamment substitué par un ou plusieurs substituants choisis parmi halogène, hydroxyle, C₁₋₆alkyle, -C₁₋₆halogénoalkyle, -C₁₋₆hydroxyalkyle, -O-C₁₋₆alkyle et -S-C₁₋₆alkyle.

6. Composé selon la revendication 1, ou sel ou solvate pharmaceutiquement acceptable de celui-ci, ayant la formule IIa. 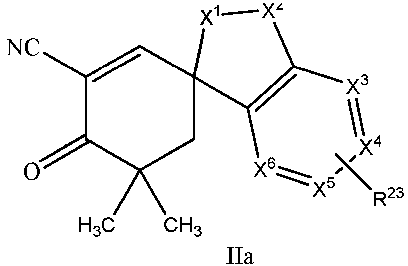 dans lequel,
X¹ est O ou CR^{12a}R^{12a} ;
X² est CR^{12a}R^{12a}, C(O) ou NR^{12a} ;
X³, X⁴, X⁵ et X⁶ sont chacun indépendamment N ou CR²³, à condition que pas plus de 2 parmi X³-X⁶ ne puissent être N ;
chaque R^{12a} est indépendamment H, -C(O)C₁₋₆alkyle, ou -C₁₋₆alkyle, éventuellement substitué par hydroxyle et chaque R²³ est indépendamment H, halogène, -C₁₋₆alkyle ou -C₁₋₆halogénoalkyle.

7. Composé selon l'une quelconque des revendications 1 à 6 qui est choisi parmi : 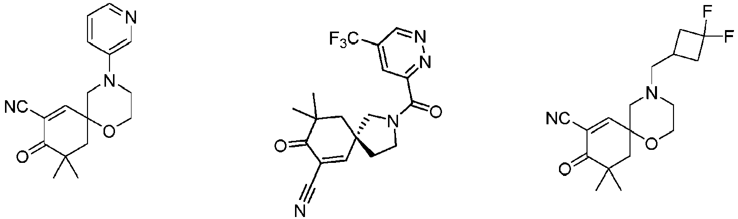 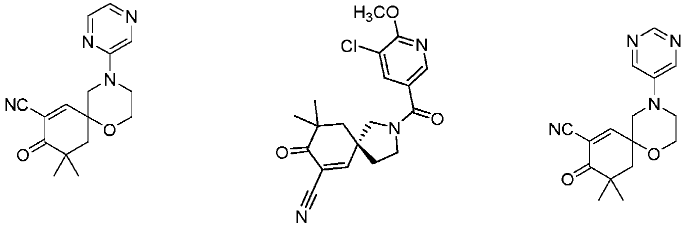 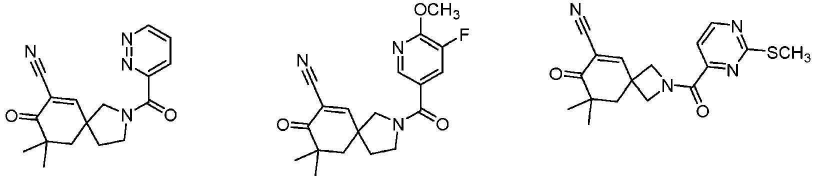 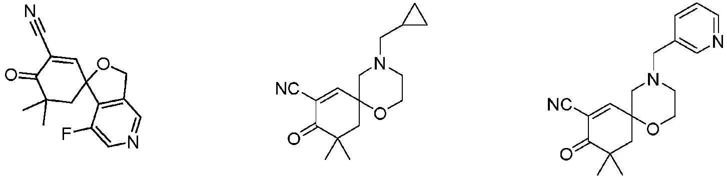 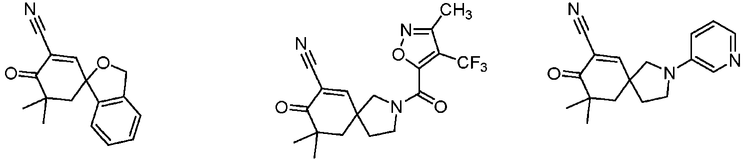 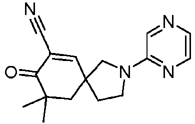 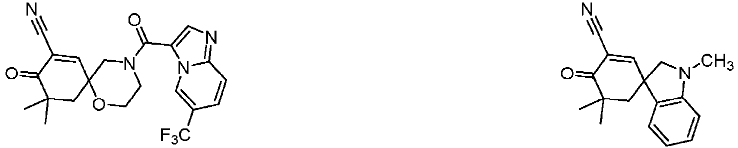 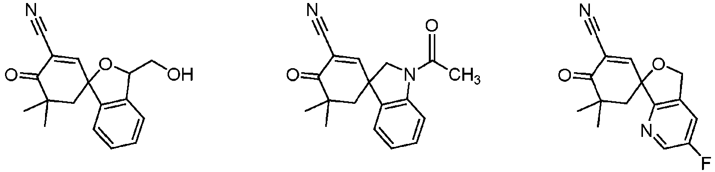 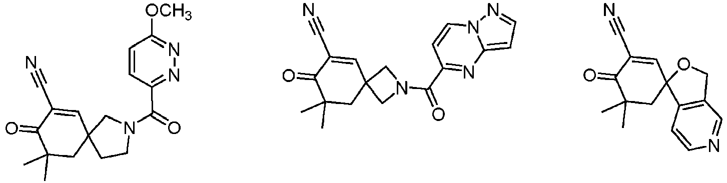 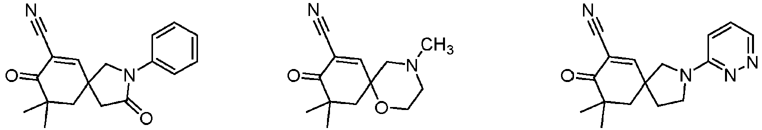 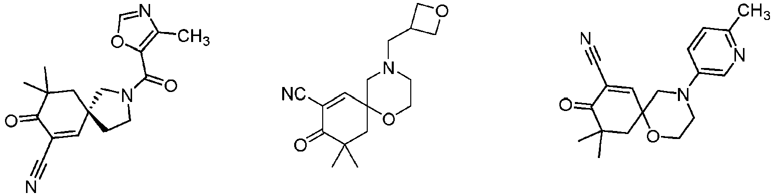 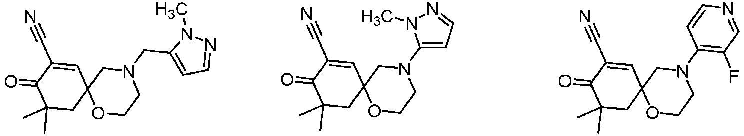 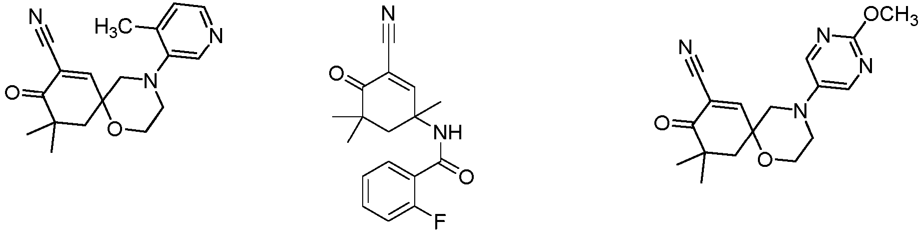 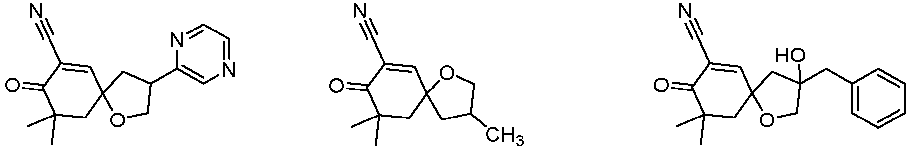 et 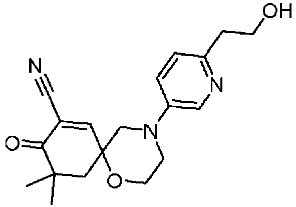 ou sel ou solvate pharmaceutiquement acceptable de celui-ci.

8. Composition pharmaceutique comprenant un composé selon l'une quelconque des revendications 1 à 7, ou un sel ou solvate pharmaceutiquement acceptable de celui-ci, conjointement avec un ou plusieurs excipients pharmaceutiquement acceptables.

9. Composé selon l'une quelconque des revendications 1 à 7, ou sel ou solvate pharmaceutiquement acceptable de celui-ci, pour une utilisation en thérapie.

10. Composé selon l'une quelconque des revendications 1 à 7, ou sel ou solvate pharmaceutiquement acceptable de celui-ci, pour une utilisation dans le traitement d'une maladie neurodégénérative choisie parmi l'ataxie de Friedreich, la sclérose latérale amyotrophique, un accident vasculaire cérébral, la maladie d'Alzheimer, la maladie de Parkinson, la maladie de Huntington, une neuropathie périphérique et une perte auditive chez un patient.

11. Combinaison comprenant un composé selon l'une quelconque des revendications 1 à 7, ou un sel ou solvate pharmaceutiquement acceptable de celui-ci, et un ou plusieurs autres agents thérapeutiques.
